# EUROPEAN PATENT APPLICATION

(11) **EP 2 208 499 A1**
(43) Date of publication of application: **21.07.2010**
(21) Application number: 08835543.3
(22) Date of filing: 03.10.2008
(51) Int. Cl.: A61K 31/7088, A61K 48/00, A61P 9/10, A61P 11/00, A61P 13/08, A61P 13/12, A61P 29/00, A61P 35/00, A61P 37/06, A61P 43/00

(54) **NUCLEIC ACID CAPABLE OF REGULATING THE PROLIFERATION OF CELL**

(30) Priority: 03.10.2007 JP 2007260393
(71) Applicant: Kyowa Hakko Kirin Co., Ltd., Tokyo 100-8185 (JP)
(72) Inventor: NAKANO, Haruo, Tokyo 194-8533 (JP); MIYAZAWA, Tatsuya, Tokyo 194-8533 (JP); YAMADA, Yoji, Shizuoka 411-8731 (JP); YOSHIDA, Tetsuo, Tokyo 194-8533 (JP)
(74) Representative: Vossius & Partner
(86) International application number: PCT/JP2008/068109
(87) International publication number: WO 2009/044899

(57) **Abstract**

Provided are an agent for suppressing or promoting cell proliferation, a diagnostic reagent or therapeutic drug for a disease resulting from a cell proliferation abnormality, an agent for inducing apoptosis, an agent for suppressing or promoting the expression of a target gene for a nucleic acid such as a microRNA, and a method of suppressing or promoting cell proliferation.

## Description

### Technical Field

The present invention relates to an agent for suppressing or promoting cell proliferation, a diagnostic reagent or therapeutic drug for a disease resulting from a cell proliferation abnormality, a method of suppressing or promoting cell proliferation, a method of suppressing or promoting the expression of a target gene for a nucleic acid, and a method of screening an gent for suppressing or promoting cell proliferation, based on a nucleic acid.

### Background Art

microRNA (miRNA), which is one kind of nucleic acids, is a small non-coding single-stranded RNA of about 22 nucleotides that is not translated into a protein, and has known as being present in many types in organisms, including humans (non-patent documents 1 and 2). A miRNA is produced from a gene transcribed to a single or clustered miRNA precursor. Specifically, first, a primary-miRNA (pri-miRNA), which is a primary transcript, is transcribed from the gene, then, in stepwise processing from the pri-miRNA to a mature type microRNA, a precursor-miRNA (pre-miRNA) of about 70 nucleotides having a characteristic hairpin structure is produced from the pri-miRNA. Furthermore, the mature type miRNA is produced from the pre-miRNA by Dicer-mediated processing (non-patent document 3).

A mature type miRNA is thought to be involved in the post-transcriptional control of gene expression by complementarily binding to a mRNA for a target to suppress the translation of the mRNA, or to degrade the mRNA.
Although the mechanism in which miRNAs suppress the expression of target mRNAs has not been clarified in full, its outline is being elucidated through recent years' research. A miRNA suppresses the expression of the mRNA for a target by binding to a partially complementary sequence in the 3'-untranslational region (3'-UTR) of the target mRNA to suppress the translation thereof, or to degrade the target mRNA. Although the complementarity may not be complete, it has been shown that the complementarity of the 2nd to 8th nucleotides from the 5'-end of the miRNA is particularly important; this region is sometimes called the "seed sequence" of the miRNA (non-patent document 4). It has been shown that miRNAs having a same seed sequence suppress the expression of a same target mRNA even if their other sequences differ. Therefore, by making a sequence complementary to a sequence present at the 3'-end of an optionally chosen mRNA as the seed sequence, an RNA having miRNA-like activity can be designed. Usually, the term miRNA, unlike siRNA, often refers exclusively to an RNA "that occurs naturally in cells", so a miRNA-like sequence designed as described above is sometimes particularly referred to as "an artificial miRNA".

As of August 2007, in the miRNA database miRBase (http://microrna.sanger.ac.uk/), 533 species of miRNAs were registered for humans, and 5,071 species for all organisms. Of the miRNAs expressed in mammals, including humans, only some have their physiological functions elucidated to date, including miR-181, which is involved in hematopoietic lineage differentiation (non-patent document 5), miR-375, which is involved in insulin secretion (non-patent document 6), and the like; many have their bioactivities unclarified. However, studies using nematodes or Drosophila have shown that miRNAs play various important roles in the development and differentiation in organisms, and a report of the relation to human diseases has been reported suggesting a profound relation to cancers (non-patent document 7).

Because miRNAs are involved in the control of the expression of a wide variety of genes, abnormalities of miRNAs are supposed to be involved in various human diseases. Particularly in cancers, research has been advanced; it has been reported that in many cancers, the expression of miRNAs differs from that in normal tissues, that classification of cancers is enabled by expression profile analyses of miRNAs, and the like (non-patent document 8). It is also known that about half of the human miRNAs that have been found so far are present in chromosome aberrations or fragile portions of chromosomes known in human cancers (non-patent document 9).

Examples of relationships between cancers and miRNAs that have been reported so far include the finding that a miR-15a/miR-16 cluster is contained in the chromosome 13q14, which is deleted in B cell chronic lymphatic leukemia (B-CLL), the deletion being supposed to be a cause of B-CLL (non-patent document 10), and the finding that in B-CLL, the expression of miR-29 and miR-181 has also been decreased, one of the targets being Tell, which is known as a carcinogenic gene (non-patent document 11). In lung cancer, it is known that the expression of Let-7, which is a miRNA, has been decreased, one of the targets thereof being Ras, which is known as a carcinogenic gene (non-patent documents 12 and 13). It is also known that the expression of miRNAs such as miR-127 and miR-124a has been decreased due to gene modification by high methylation in cancers, and Bc16 and Cdk6, which are known as carcinogenic genes, serve as targets thereof (non-patent documents 14 and 15). Many miRNAs have their expression decreased in cancer cells; conversely, however, there are some miRNAs with gene amplification or overexpression in cancer cells. For example, in regions where gene amplification is seen in malignant lymphoma, a cluster consisting of six species of miRNAs (miR-17-92) is present; it has been known that when this miRNA cluster gene is forcedly expressed in a mouse model of human B cell lymphoma, the onset of lymphoma is promoted (non-patent document 16). It has also been shown that a gene called BIC, which has been regarded as a cancer gene candidate that does not encode a protein and is overexpressed in Hodgkin lymphoma, encodes miR-155 (non-patent document 17).

As stated above, relationships between cancers and miRNAs have recently been reported in many cases, but many of them are manifested as expression abnormalities in cancer cells, with a few reports showing functions of miRNAs, which include reports that the proliferation of cancer cell lines was inhibited by forcing cancer cell lines to express Let-7, miR-143, or miR-145 (non-patent documents 18, 19, and 20), reports that the cell cycles of cancer cell lines were halted by forcing the cancer cell lines to express miR-16 or miR-34 (non-patent documents 21 and 22), and a report that cell transformation was induced by forcing the cells to express miR-372 or miR-373 (non-patent document 23). There are only a few reports of suppressing cancer growth in animal models by administering a miRNA or a precursor thereof from outside the body to increase the expression of the miRNA, or by administering an antisense oligonucleotide thereof to decrease the expression of the miRNA; these reports include a report that tumorigenesis of a transplanted colorectal cancer cell line was suppressed by administering miR-34a (non-patent document 24) and a report that tumorigenesis of a transplanted breast cancer cell line was suppressed by administering an antisense of miR-21 (non-patent document 25).
non-patent document 1: Science, 294, 853-858, (2001)
non-patent document 2: Cell, 113, 673-676, (2003)
non-patent document 3: Nature Reviews Genetics, 5, 522-531, (2004)
non-patent document 4: Current Biology, 15, R458-R460 (2005)
non-patent document 5: Science, 303, 83-86, (2004)
non-patent document 6: Nature, 432, 226-230, (2004)
non-patent document 7: Nature Reviews Cancer, 6, 259-269, (2006)
non-patent document 8: Nature, 435, 839-843 (2005)
non-patent document 9: Proc. Natl. Acad. Sci. USA, 101, 2999-3004 (2004)
non-patent document 10: Proc. Natl. Acad. Sci. USA, 99, 15524-15529 (2002)
non-patent document 11: Cancer Research, 66, 11590-11593 (2006)
non-patent document 12: Cancer Research, 64, 3753-3756, (2004)
non-patent document 13: Cell, 120, 635-647, (2005)
non-patent document 14: Cancer Cell, 9, 435-443, (2006)
non-patent document 15: Cancer Research, 67, 1424-1429, (2007)
non-patent document 16: Nature, 435, 823-833 (2005)
non-patent document 17: Proc. Natl. Acad. Sci. USA, 102, 3627-3632 (2005)
non-patent document 18: Cancer Research, 64, 3753-3756, (2004)
non-patent document 19: Oncology Reports, 16, 845-850, (2006)
non-patent document 20: Cancer Research, 67, 7713-7722, (2007)
non-patent document 21: Molecular and Cellular Biology, 27, 2240-2252, (2007)
non-patent document 22: Nature, 447, 1130-1134, (2007)
non-patent document 23: Cell, 124, 1169-1181, (2006)
non-patent document 24: Proc. Natl. Acad. Sci. USA, 104, 15472-15477 (2007)
non-patent document 25: Oncogenes, 26, 2799-2803, (2007)

### Disclosure of the Invention

### Problems to Be Solved by the Invention

It is expected that by identifying miRNAs expressed in various human organs, and analyzing the functions thereof to elucidate their relations to diseases, new therapeutic drugs and diagnostic reagents will be developed.
In particular, finding a miRNA that causes cancer cell proliferation or suppression is expected not only to help to understand the mechanisms of carcinogenesis, but also to lead to the development of diagnostic reagent and therapeutic drugs for human cancers, and new diagnostic methods and therapies for cancers based thereon. Furthermore, regarding diseases other than cancers, the same is expected to contribute to the development of diagnostic reagents and therapeutic drugs for diseases caused by abnormal proliferation of cells, tissue hyperplasia and the like, such as arteriosclerosis, rheumatoid arthritis, prostatic hyperplasia, blood vessel restenosis after percutaneous transvascular coronary angioplasty, fibroid lung, glomerulonephritis, and autoimmune diseases, and diagnostic methods and therapies based thereon.

It is an object of the present invention to provide nucleic acids that are useful in controlling cell proliferation and methods of utilizing the same.

### Means for Solving the Problems

The present invention relates to the following (1) to (32) .
(1) An agent for suppressing or promoting cell proliferation, comprising, as an active ingredient, any one of the nucleic acids (a) to (h) below:
   (a) a nucleic acid consisting of a nucleotide sequence shown by any one of SEQ ID NO:1-939, 2057-2132, 2212-2261 and 2315-2321;
   (b) a nucleic acid consisting of 17 to 28 nucleotides comprising a nucleic acid consisting of a nucleotide sequence shown by any one of SEQ ID NO:1-939, 2057-2132, 2212-2261 and 2315-2321;
   (c) a nucleic acid consisting of a nucleotide sequence having an identity of 90% or more to a nucleotide sequence shown by any one of SEQ ID NO:1-939, 2057-2132, 2212-2261 and 2315-2321;
   (d) a nucleic acid that hybridizes under stringent conditions with a complementary strand for a nucleic acid consisting of a nucleotide sequence shown by any one of SEQ ID NO:1-939, 2057-2132, 2212-2261 and 2315-2321;
   (e) a nucleic acid comprising the sequence of 2nd to 8th nucleotides of a nucleotide sequence shown by any one of SEQ ID NO:1-939, 2057-2132, 2212-2261 and 2315-2321;
   (f) a nucleic acid consisting of a nucleotide sequence shown by any one of SEQ ID NO:940-2056, 2133-2211, 2262-2314 and 2322-2328;
   (g) a nucleic acid consisting of a nucleotide sequence having an identity of 90% or more to a nucleotide sequence shown by any one of SEQ ID NO:940-2056, 2133-2211, 2262-2314 and 2322-2328;
   (h) a nucleic acid that hybridizes under stringent conditions with a complementary strand for a nucleic acid consisting of a nucleotide sequence shown by any one of SEQ ID NO:940-2056, 2133-2211, 2262-2314 and 2322-2328.
(2) The agent according to (1), wherein the nucleic acid is a miRNA or miRNA precursor.
(3) An agent for suppressing or promoting cell proliferation, comprising, as an active ingredient, a nucleic acid consisting of a nucleotide sequence complementary to the nucleotide sequence of the nucleic acid according to (1).
(4) An agent for suppressing or promoting cell proliferation, comprising, as an active ingredient, a vector that expresses the nucleic acid according to (1) or (3).
(5) An agent for suppressing or promoting cell proliferation, comprising, as an active ingredient, a substance that suppresses the expression of a gene having a target nucleotide sequence for the nucleic acid according to (1).
(6) An agent for suppressing or promoting cell proliferation, comprising, as an active ingredient, a substance that promotes the expression of a gene having a target nucleotide sequence for the nucleic acid according to (1).
(7) The agent according to (5) or (6), wherein the substance that suppresses or promotes the expression is a nucleic acid.
(8) The agent according to (7), wherein the nucleic acid is an siRNA.
(9) An agent for suppressing or promoting cell proliferation, comprising, as an active ingredient, a vector that expresses the nucleic acid according to (7).
(10) A therapeutic drug for treating a disease resulting from a cell proliferation abnormality, comprising, as an active ingredient, the agent according to any one of (1) to (9).
(11) A therapeutic drug for treating a disease resulting from a cell proliferation abnormality, comprising, as an active ingredient, the nucleic acid according to any one of (1), (3) and (7).
(12) A reagent for diagnosing a disease resulting from a cell proliferation abnormality, comprising, as an active ingredient, a reagent that detects the expression level of the nucleic acid according to (1), a mutation of the nucleic acid, or a mutation of the genome that encodes the nucleic acid.
(13) The therapeutic drug or diagnostic reagent according to any one of (10) to (12), wherein the disease resulting from a cell proliferation abnormality is a cancer.
(14) A method of treating a disease resulting from a cell proliferation abnormality, comprising administering an effective amount of the nucleic acid according to any one of (1), (3) and (7).
(15) A method of diagnosing a disease resulting from a cell proliferation abnormality, comprising detecting the expression level of the nucleic acid according to (1), a mutation of the nucleic acid, or a mutation of the genome that encodes the nucleic acid.
(16) The method of treating or diagnosing according to (14) or (15), wherein the disease resulting from a cell proliferation abnormality is a cancer.
(17) Use of the nucleic acid according to any one of (1), (3) and (7) for the production of a therapeutic drug for a disease resulting from a cell proliferation abnormality.
(18) The use according to (17), wherein the disease resulting from a cell proliferation abnormality is a cancer.
(19) An agent for suppressing the expression of a target gene for the nucleic acid according to (1), comprising the nucleic acid as an active ingredient.
(20) An agent for promoting the expression of a target gene for the nucleic acid according to (1), comprising the nucleic acid according to (3) as an active ingredient.
(21) The agent for suppressing or promoting the expression of a target gene for the nucleic acid according to (1), comprising the vector according to (4) as an active ingredient.
(22) A method of promoting or suppressing cell proliferation, comprising using the nucleic acid according to (1) or (3).
(23) A method of promoting or suppressing cell proliferation, comprising using the vector according to (4).
(24) A method of promoting or suppressing cell proliferation, comprising using a substance that suppresses the expression of a target gene for the nucleic acid according to (1).
(25) A method of promoting or suppressing cell proliferation, comprising using a substance that promotes the expression of a target gene for the nucleic acid according to (1).
(26) The method of promoting or suppressing cell proliferation according to (24) or (25), wherein the substance that suppresses or promotes the expression is a nucleic acid.
(27) The method of promoting or suppressing cell proliferation according to (26), wherein the nucleic acid is an siRNA.
(28) A method of promoting or suppressing cell proliferation, comprising using a vector that expresses the nucleic acid according to (26).
(29) A method of suppressing the expression of a target gene for the nucleic acid according to (1), comprising using the nucleic acid.
(30) A method of promoting the expression of a target gene for the nucleic acid according to (1), comprising using the nucleic acid according to (3).
(31) A method of suppressing or promoting the expression of a target gene for the nucleic acid according to (1), comprising using the vector according to (4).
(32) A method of screening for an agent for promoting or suppressing cell proliferation, comprising using promotion or suppression of the expression or function of the nucleic acid according to (1) as an index.

### Effect of the Invention

According to the present invention, an agent for suppressing or promoting cell proliferation, a diagnostic reagent or therapeutic drug for a disease resulting from a cell proliferation abnormality, an agent for inducing apoptosis, an agent for suppressing or promoting the expression of a target gene for a nucleic acid such as a miRNA, and a method of suppressing or promoting cell proliferation can be provided.

### Brief Description of the Drawings

[Figure 1] Figure 1 shows results of Western blotting for Bcl2l1. The miRNAs used for the individual lanes are shown below. 1; miR-negacon#2, 2; hsa-miR-337, 3; hsa-miR-491, 4; hsa-miR-342, 5; si-Bcl211.
[Figure 2] Figure 2 shows results of Western blotting for Birc5. The miRNAs used for the individual lanes are shown below. 1; miR-negacon#2, 2; hsa-miR-337, 3; hsa-miR-491, 4; si-Birc5.
[Figure 3] Figure 3 shows results of Western blotting for Bcl91. The miRNAs used for the individual lanes are shown below. 1; miR-negacon#2, 2; hsa-miR-491, 3; hsa-miR-147, 4; hsa-miR-518b, 5; hsa-miR-432*, 6; hsa-miR-16, 7; si-Bcl91.
[Figure 4] Figure 4 shows results of a tumorigenesis experiment by transplantation of DLD-1 forcedly expressing hsa-miR-147 or 342. Shown at each measuring point are the mean and standard deviation for 12 transplantation sites.
[Figure 5] Figure 5 shows results of a tumorigenesis experiment by transplantation of DLD-1 forcedly expressing hsa-miR-491. Shown at each measuring point are the mean and standard deviation for 12 transplantation sites. Best Mode for Carrying out the Invention

The nucleic acid to be used in the present invention may be any molecule, as far as it is a molecule resulting from polymerization of a nucleotide or a molecule functionally equivalent to the nucleotide; for example, an RNA, which is a ribonucleotide polymer, a DNA, which is a deoxyribonucleotide polymer, a mixed polymer of RNA and DNA, and a nucleotide polymer, including a nucleotide analogue, can be mentioned; furthermore, the nucleic acid may be a nucleotide polymer, including a nucleic acid derivative. In addition, the nucleic acid in the present invention may be a single-stranded nucleic acid or a double-stranded nucleic acid. Double-stranded nucleic acids include double-stranded nucleic acids wherein one strand hybridizes with the other strand under stringent conditions.

In the present invention, the nucleotide analogue may be any molecule, as far as it is a molecule prepared by modifying a ribonucleotide,'a deoxyribonucleotide, an RNA or a DNA in order to improve the nuclease resistance thereof, to stabilize the same, to increase the affinity thereof for a complementary chain nucleic acid, to increase the cell permeability thereof, or to visualize the same, compared with the RNA or DNA; for example, a nucleotide analogue modified at the sugar moiety thereof, a nucleotide analogue modified at phosphoric acid diester bond and the like can be mentioned.

The nucleotide analogue modified at the sugar moiety thereof may be any one, as far as an optionally chosen chemical structural substance has been added to, or substituted for, a portion or all of the chemical structure of the sugar of the nucleotide; for example, a nucleotide analogue substituted by 2'-O-methylribose, a nucleotide analogue substituted by 2'-O-propylribose, a nucleotide analogue substituted by 2'-methoxyethoxyribose, a nucleotide analogue substituted by 2'-O-methoxyethylribose, a nucleotide analogue substituted by 2'-O-[2-(guanidium)ethyl]ribose, a nucleotide analogue substituted by 2'-O-fluororibose, a bridged nucleic acid (BNA) having two cyclic structures as a result of introduction of a bridging structure into the sugar moiety, more specifically a locked nucleic acid (LNA) wherein the oxygen atom at the 2' position and the carbon atom at the 4' position have been bridged via methylene, and an ethylene bridged nucleic acid) (ENA) [Nucleic Acid Research, 32, e175 (2004)] can be mentioned, and a peptide nucleic acid (PNA) [Acc. Chem. Res., 32, 624 (1999)], an oxypeptide nucleic acid (OPNA) [J. Am. Chem. Soc., 123, 4653 (2001)], and a peptide ribonucleic acid (PRNA) [J. Am. Chem. Soc., 122, 6900 (2000)] and the like can also be mentioned.

The nucleotide analogue modified at phosphoric acid diester bond may be any one, as far as an optionally chosen chemical substance has been added to, or substituted for, a portion or all of the chemical structure of the phosphoric acid diester bond of the nucleotide; for example, a nucleotide analogue substituted by a phosphorothioate bond, a nucleotide analogue substituted by an N3'-P5' phosphoamidate bond, and the like can be mentioned [SAIBO KOGAKU, 16, 1463-1473 (1997)] [RNAi Method and Antisense Method, Kodansha (2005)].

The nucleic acid derivative may be any molecule, as far as it is a molecule prepared by adding another chemical substance to the nucleic acid in order to improve the nuclease resistance thereof, to stabilize the same, to increase the affinity thereof for a complementary chain nucleic acid, to increase the cell permeability thereof, or to visualize the same, compared with the nucleic acid; for example, a 5'-polyamine conjugated derivative, a cholesterol conjugated derivative, a steroid conjugated derivative, a bile acid conjugated derivative, a vitamin conjugated derivative, a Cy5 conjugated derivative, a Cy3 conjugated derivative, a 6-FAM conjugated derivative, a biotin conjugated derivative and the like can be mentioned.

As examples of nucleic acids, the following nucleic acids (a) to (k) can be mentioned.
(a) A nucleic acid consisting of a nucleotide sequence shown by any one of SEQ ID NO:1-939, 2057-2132, 2212-2261 and 2315-2321
(b) A nucleic acid of 17 to 28 nucleotides comprising a nucleic acid consisting of a nucleotide sequence shown by any one of SEQ ID NO:1-939, 2057-2132, 2212-2261 and 2315-2321
(c) A nucleic acid consisting of a nucleotide sequence having an identity of 90% or more to a nucleotide sequence shown by any one of SEQ ID NO:1-939, 2057-2132, 2212-2261 and 2315-2321
(d) A nucleic acid that hybridizes under stringent conditions with a complementary strand for a nucleic acid consisting of a nucleotide sequence shown by any one of SEQ ID NO:1-939, 2057-2132, 2212-2261 and 2315-2321
(e) A nucleic acid comprising the sequence of 2nd to 8th nucleotides of a nucleotide sequence shown by any one of SEQ ID NO:1-939, 2057-2132, 2212-2261 and 2315-2321
(f) A double-stranded nucleic acid consisting of one of the nucleic acids (a) to (e) and a nucleic acid comprising a nucleotide sequence complementary to the nucleotide sequence of the nucleic acid, or a nucleic acid comprising the double-stranded nucleic acid
(g) A double-stranded nucleic acid consisting of one of the nucleic acids (a) to (e) and a nucleic acid that hybridizes under stringent conditions with the nucleic acid, or a nucleic acid comprising the double-stranded nucleic acid
(h) A single-stranded nucleic acid having a hairpin structure wherein the double-stranded nucleic acid (f) or (g) is joined via a spacer oligonucleotide consisting of 8 to 28 nucleotides, or a nucleic acid comprising the single-stranded nucleic acid
(i) A nucleic acid consisting of a nucleotide sequence shown by any one of SEQ ID NO:940-2056, 2133-2211, 2262-2314 and 2322-2328
(j) A nucleic acid consisting of a nucleotide sequence having an identity of 90% or more to a nucleotide sequence shown by any one of SEQ ID NO:940-2056, 2133-2211, 2262-2314 and 2322-2328
(k) A nucleic acid that hybridizes under stringent conditions with a complementary strand for a nucleic acid consisting of a nucleotide sequence shown by any one of SEQ ID NO:940-2056, 2133-2211, 2262-2314 and 2322-2328.

As the above-described nucleic acid, a miRNA is preferably used. A miRNA refers to a single-stranded RNA 17 to 28 nucleotides long. The peripheral genomic sequence, including a miRNA sequence has a sequence capable of forming a hairpin structure, and the miRNA can be cut out from either one strand of the hairpin. A miRNA complementarily binds to a mRNA serving as a target therefor to suppress the translation of the mRNA or promote the degradation of the mRNA, thereby mediating the post-translational control of gene expression.

As examples of miRNAs, human miRNAs consisting of a nucleotide sequence shown by any one of SEQ ID NO:1-129, 803, 819, 916, 917, 925 and 2057-2114 can be mentioned. Furthermore, as miRNAs having the same function as the function of human miRNAs consisting of a nucleotide sequence shown by any one of 3EQ ID NO:1-129, 803, 819, 916, 917, 925 and 2057-2114, nucleic acids consisting of a nucleotide sequence shown by any one of SEQ ID NO:130-802, 822, 823, 826, 827, 829-840, 842, 843, 846-848, 850, 851, 2115-2122, 2212-2261 and 2315-2321, which are orthologues of the human miRNAs, can be mentioned. As specific examples of orthologues of the human miRNA of SEQ ID NO:1, those consisting of a nucleotide sequence shown by any one of SEQ ID NO:130-139 and 2212 can be mentioned. A table of correspondence of miRNAs consisting of a nucleotide sequence shown by any one of SEQ ID NO:1-129, 803, 819, 916, 917, 925 and 2057-2114 and orthologues thereof is shown in Table 1 (from Table 1-1 to Table 1-4). The biological species shown in the uppermost fields of Table 1 are as follows: has, Homo sapiens, human; mmu, Mus musculus, mouse; rno, Rattus norvegicus, rat; xla, Xenopus laevis, African clawed toad; xtr, Xenopus tropicalis, tropical clawed toad; gga, Gallus gallus, chicken; cfa, Canis familiaris, dog; mdo, Monodelphis domestica, gray short-tailed opossum; age, Ateles geoffroyi, black-handed spider monkey; 11a, Lagothrix lagotricha, Humboldt's wooly monkey; sla, Saguinus labiatus, red-bellied tamarin; mml, Macaca mulatta, rhesus monkey; mne, Macaca nemestrina, pig-tailed macaque; ggo, Gorilla gorilla, gorilla; ppa, Pan paniscus, bonobo; ptr, Pan troglodytes, chimpanzee; ppy, Pongo pygmaeus, orangutan; lca, Lemur catta, ring-tailed lemur; cgr, Cricetulus griseus, Chinese hamster; bta, Bos taurus, cattle; oar, Ovis aries, sheep; ssc, Sus scrofa, swine; dre, Danio rerio, zebrafish; fru, Fugu rubripes, tiger puffer; tni, Tetraodon nigroviridis, green spotted puffer. Because human-derived miRNAs and orthologues thereof have high sequence identity, they are considered to possess similar functions. Also because miRNAs in the same subgroup as classified by alphabetical figures added to the ends of their names also share high sequence identity, they are considered to possess similar functions.

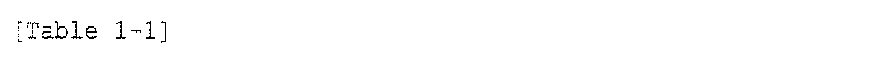

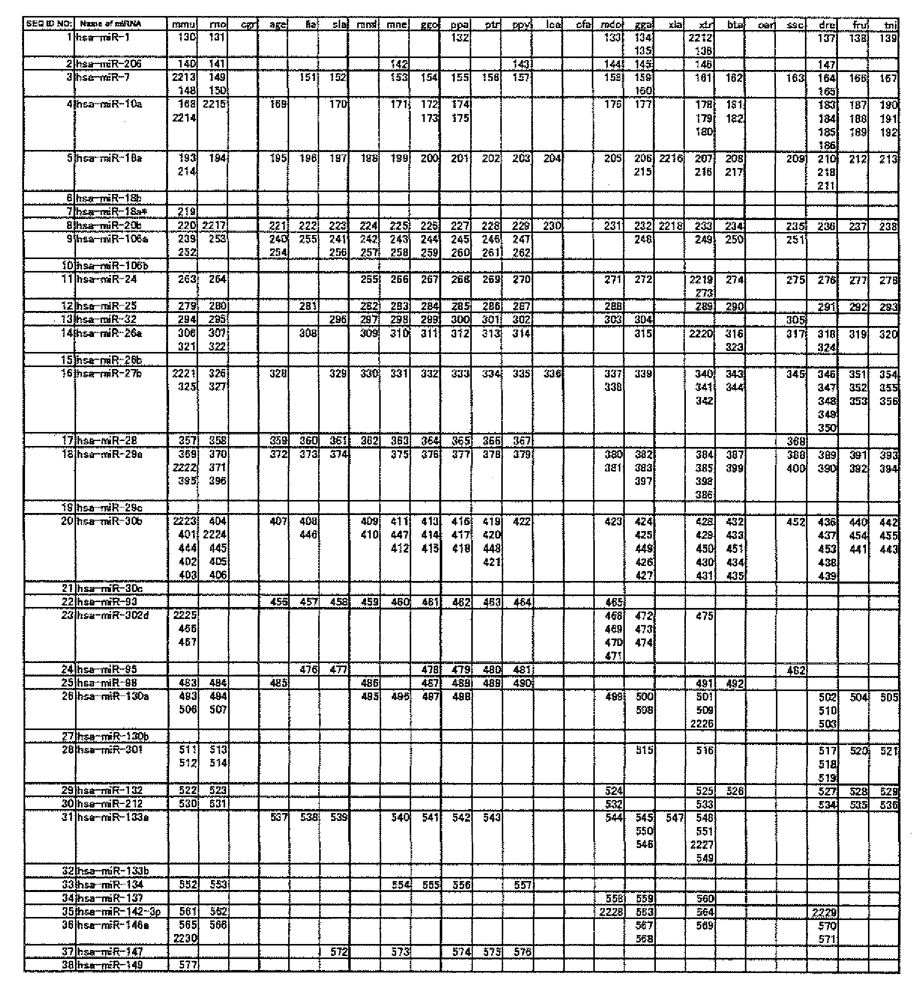

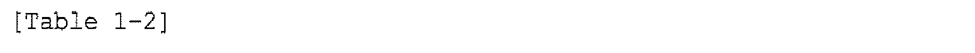

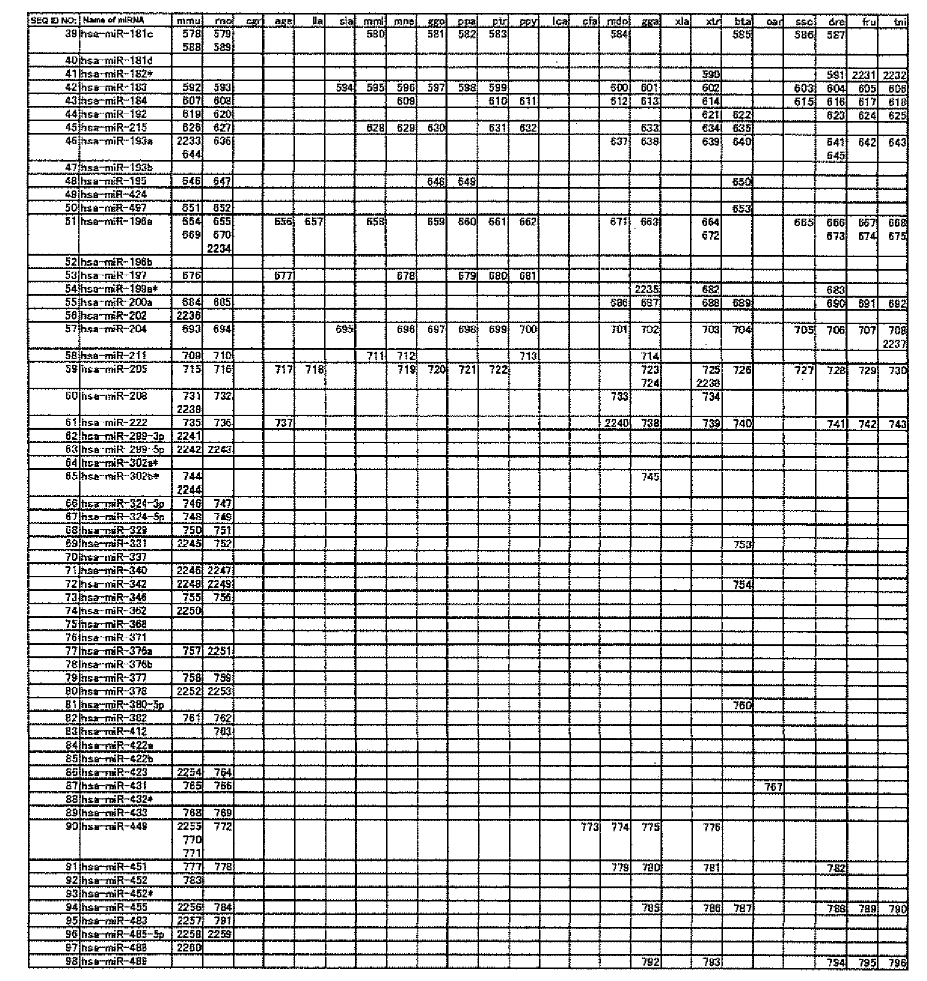

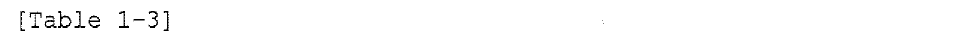

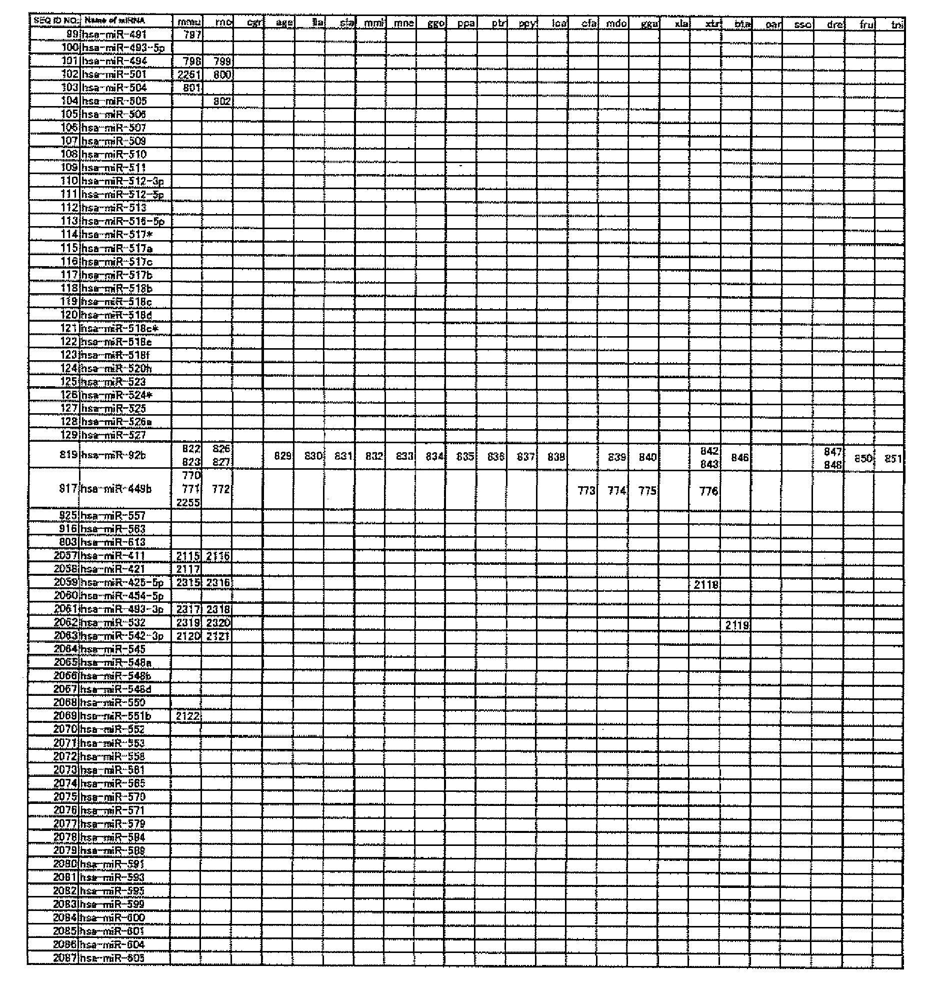

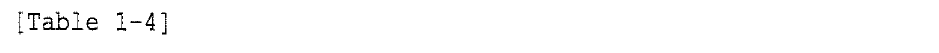

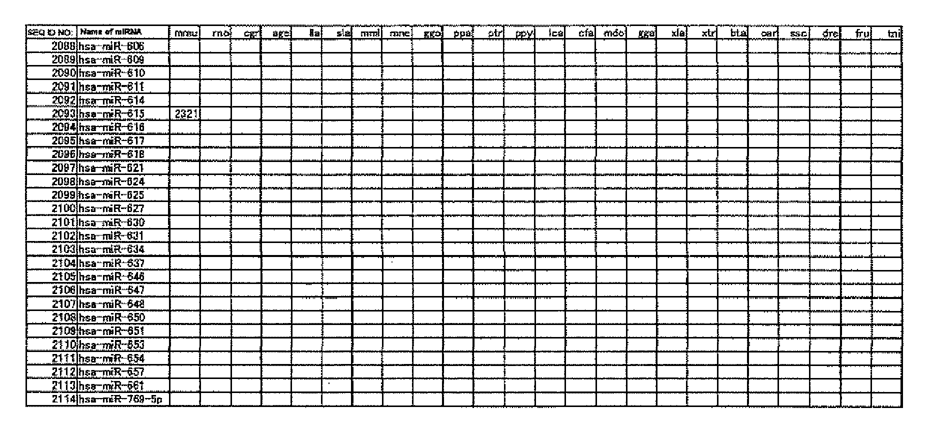

Regarding the mechanism in which a miRNA suppresses the translation of the mRNA of a target gene therefor, it is known that a mRNA having a nucleotide sequence complementary to the 2nd to 8th nucleotides from the 5'-end of the miRNA is recognized as a miRNA target gene [Current Biology, 15, R458-R460 (2005)]. By this mechanism, the expression of the mRNA is suppressed by the miRNA. Therefore, miRNAs having the same nucleotide sequence at the 2nd to 8th nucleotides from the 5'-end suppress the expression of the same mRNA to exhibit the same function. As miRNAs having the same nucleotide sequence at the 2nd to 8th nucleotides from the 5'-end as the nucleotide sequence of a miRNA consisting of a nucleotide sequence shown by any one of SEQ ID NO:1-129, 803, 819, 916, 917, 925 and 2057-2114, nucleic acids consisting of a nucleotide sequence shown by any one of SEQ ID NO:804-818, 820-915, 918-924, 926-939 and 2123-2132 can be mentioned. As a specific example of the miRNA consisting of the nucleotide sequence shown by SEQ ID NO:4, the miRNA consisting of the nucleotide sequence shown by SEQ ID NO:804 can be mentioned. Artificial miRNAs are also included in the miRNAs. A table of correspondence of miRNAs having the same nucleotide sequence at the 2nd to 8th nucleotides from the 5'-end as the nucleotide sequence of a miRNA consisting of a nucleotide sequence shown by any one of SEQ ID NO:1-129, 803, 819, 916, 917, 925 and 2057-2114 is shown in Table 2 (from Table 2-1 to Table 2-4). Because miRNAs having a same seed sequence are considered to share the same target nucleotide sequence, they are considered to possess similar functions.

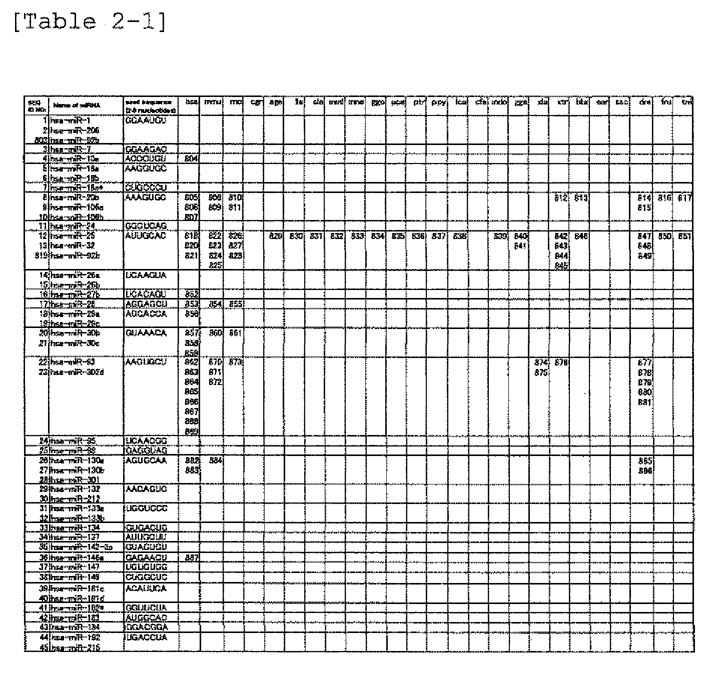

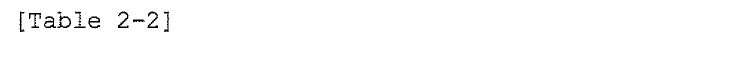

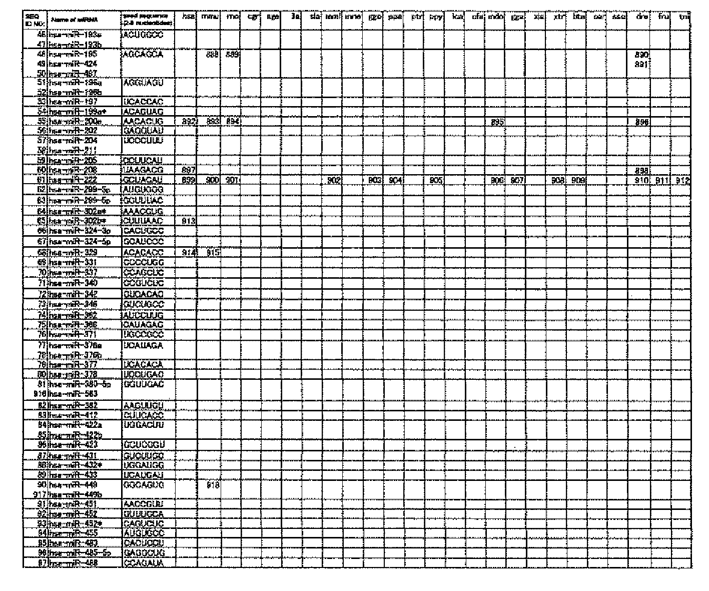

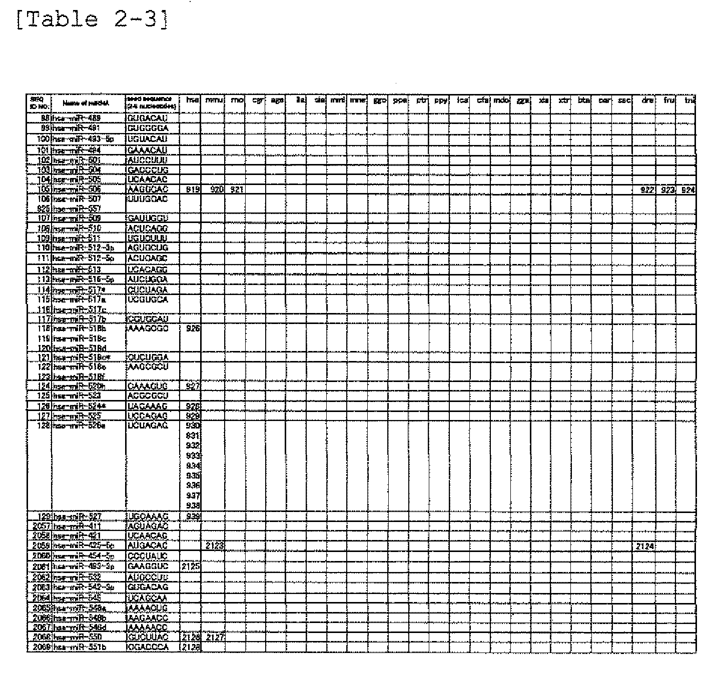

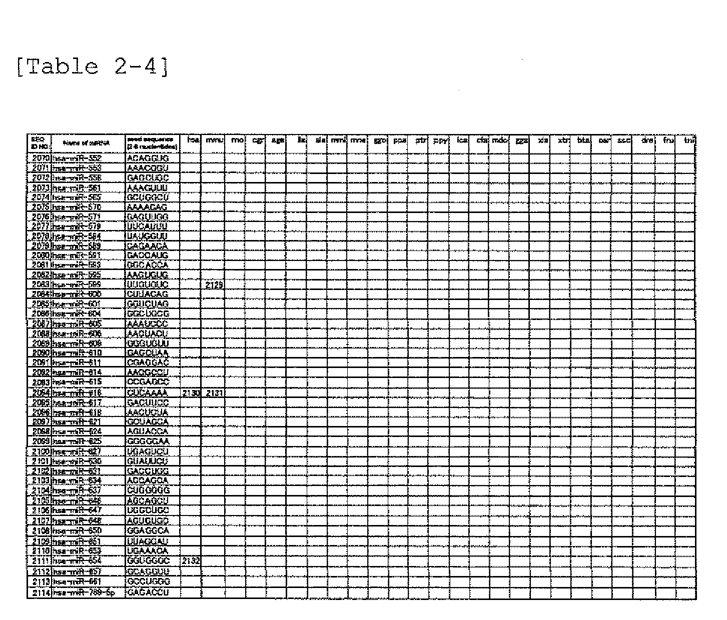

As the above-described nucleic acids, mRNA precursors can also be used preferably. A miRNA precursor is a nucleic acid about 50 to about 200 nucleotides long, more preferably about 70 to about 100 nucleotides long, including the above-described nucleic acid, and capable of forming a hairpin structure. A miRNA is produced from a miRNA precursor via processing by a protein called Dicer.

As examples of miRNA precursors for the human miRNAs of SEQ ID NO:1, nucleic acids consisting of a nucleotide sequence shown by SEQ ID NO:940 or 941 can be mentioned. For the miRNAs consisting of a nucleotide sequence shown by any one of SEQ ID NO:2-939, 2057-2132, 2212-2261 and 2315-2321, nucleic acids consisting of a nucleotide sequence shown by any one of SEQ ID NO:942-2056, 2133-2211, 2262-2314 and 2322-2328 can be mentioned. A table of correspondence of miRNAs and miRNA precursors in the present invention is shown in Table 3 (from Table 3-1 to Table 3-17). In the present invention, miRNA precursors include artificial mRNA precursors.

**[Table 3-1]**

| SEQ ID NO: | Name of mIRNA | SEQ ID NO: | Name of mIRNA precursor |
|---|---|---|---|
| 1 | hsa-miR-1 | 940 | hsa-mir-1-1 |
| | | 941 | hsa-mir-1-2 |
| 2 | hsa-miR-206 | 942 | hsa-mir-205 |
| 3 | hsa-miR-7 | 943 | hsa-mir-7-1 |
| | | 944 | hsa-mir-7-2 |
| | | 945 | hsa-mir-7-3 |
| 4 | hsa-miR-10a | 946 | hsa-mir-10a |
| 5 | hsa-miR-18a | 947 | hsa-mir-18a |
| 6 | hsa-miR-18b | 948 | hsa-mir-18b |
| 7 | hsa-miR-18a* | 947 | hse-mir-18a |
| 8 | hsa-miR-20b | 949 | hsa-mir-20b |
| 9 | hsa-miR-106a | 950 | hsa-mir-105a |
| 10 | hsa-miR-106b | 951 | hsa-mir-106b |
| 11 | hsa-miR-24 | 952 | hsa-mir-24-1 |
| | | 953 | hsa-mir-24-2 |
| 12 | hsa-miR-25 | 954 | hsa-mir-25 |
| 13 | hsa-miR-32 | 955 | hsa-mir-32 |
| 14 | hsa-miR-26a | 956 | hsa-mir-26a-1 |
| | | 957 | hsa-mir-26a-2 |
| 15 | hsa-miR-26b | 958 | hsa-mir-26b |
| 16 | hsa-miR-26b | 959 | hsa-mir-27b |
| 17 | hsa-miR-27b hsa-miR-28 | 960 | hsa-mir-28 |
| 18 | hsa-miR-29a | 961 | hsa-mir-29a |
| 19 | hsa-miR-29c | 962 | hsa-mir-29c |
| 20 | hsa-miR-30b | 963 | hsa-mir-30b |
| 21 | hsa-miR-30c | 964 | hsa-mir-30c-1 |
| | | 965 | hsa-mir-30c-2 |
| 22 | hsa-miR-93 | 966 | hsa-mir-93 |
| 23 | hsa-miR-302d | 967 | hsa-mir-302d |
| 24 | hsa-miR-95 | 968 | hsa-mir-95 |
| 25 | hsa-miR-88 | 969 | hsa-mir-98 |
| 26 | hsa-miR-130a | 970 | hsa-mir-130a |
| 27 | hsa-miR-130b | 971 | hsa-mir-130b |
| 28 | hsa-miR-301 | 972 | hsa-mir-301 |
| 29 | hsa-miR-132 | 973 | hsa-mir-132 |
| 30 | hsa-miR-212 | 974 | hsa-mir-212 |
| 31 | hsa-miR-133a | 975 | hsa-mir-133a-1 |
| | | 976 | hsa-mir-133a-2 |
| 32 | hsa-miR-133b | 977 | hsa-mir-133b |
| 33 | hsa-miR-134 | 978 | hsa-mir-134 |
| 34 | hsa-miR-137 | 979 | hsa-mir-137 |
| 35 | hsa-miR-142-30 | 980 | hsa-mir-142 |
| 36 | hsa-miR-146a | 981 | hsa-mir-146a |
| 37 | hsa-miR-147 | 982 | hsa-mir-147 |
| 38 | hsa-miR-148 | 983 | hsa-mir-149 |
| 39 | hsa-miR-181c | 984 | hsa-mir-181c |
| 40 | hsa-miR-181d | 985 | hsa-mir-181d |
| 41 | hsa-miR-182* | 986 | hsa-mir-182 |
| 42 | hsa-miR-183 | 987 | hsa-mir-83 |
| 43 | hsa-miR-184 | 988 | hsa-mir-184 |
| 44 | hsa-miR-192 | 989 | hsa-mir-192 |
| 45 | hsa-miR-215 | 990 | hsa-mir-215 |
| 46 | hsa-miR-193a | 991 | hsa-mir-193a |
| 47 | hsa-miR-193b | 992 | hsa-mir-193b |
| 49 | hsa-miR-195 | 993 | hsa-mir-195 |
| 49 | hsa-miR-424 | 994 | hsa-mir-424 |
| 50 | hsa-miR-497 | 995 | hsa-mir-497 |
| | | 996 | hsa-mir-195a-1 |
| 51 | hsa-miR-195a | 997 | hsa-mir-195a-2 |
| 52 | hsa-miR-196b | 998 | hsa-mir-196b |
| 53 | hsa-miR-197 | 999 | hsa-mir-197 |
| 54 | hsa-miR-198a* | 1000 | hsa-mir-199a-1 |
| | | 1001 | hsa-mir-199a-2 |
| 55 | hsa-miR-200a | 1002 | hsa-mir-200a |
| 56 | hsa-miR-202 | 1003 | hsa-mir-202 |
| 57 | hsa-miR-204 | 1004 | hsa-mir-204 |
| 58 | hsa-miR-211 | 1005 | hsa-mir-211 |
| 59 | hsa-miR-205 | 1006 | hsa-mir-205 |
| 60 | hsa-miR-208 | 1007 | hsa-mir-208 |
| 61 | hsa-miR-222 | 1008 | hsa-mir-222 |
| 62 | hsa-miR-298-3p | 1009 | hsa-mir-285 |
| 63 | hsa-miR-288-5c | 1009 | hse-mir-299 |
| 64 | hsa-miR-302e* | 1010 | hsa-mir-302a |
| 65 | hsa-miR-302b* | 1011 | hsa-mir-302b |
| 66 | hsa-miR-324-3p | 1012 | hsa-mir-324 |
| 67 | hsa-miR-324-5c | 1012 | hsa-mir-324 |
| 68 | hsa-miR-329 | 1013 | hsa-mir-329-1 |
| | | 1014 | hsa-mir-329-2 |
| 69 | hsa-miR-331 | 1015 | hsa-mir-331 |
| 70 | hsa-miR-337 | 1016 | hsa-mir-337 |
| 71 | hsa-miR-340 | 1017 | hsa-mir-340 |
| 72 | hsa-miR-342 | 1018 | hsa-mir-342 |

**[Table 3-2]**

| SEQ ID NO: | Name of mIRNA | SEQ ID NO: | Name of mIRNA precursor |
|---|---|---|---|
| 73 | hsa-miR-346 | 1019 | hsa-mir-346 |
| 74 | hsa-miR-362 | 1020 | hsa-mir-362 |
| 75 | hsa-miR-363 | 1021 | hsa-mir-368 |
| 76 | hsa-miR-371 | 1022 | hsa-mir-371 |
| 77 | hsa-miR-375a | 1023 | hsa-mir-376a-1 |
| | | 1024 | hsa-mir-376a-2 |
| 78 | hsa-miR-376b | 1025 | hsa-mir-376b |
| 79 | hsa-miR-377 | 1026 | hsa-mir-377 |
| 80 | hsa-miR-378 | 1027 | hsa-mir-378 |
| 81 | hsa-miR-380-5p | 1028 | hsa-mir-380 |
| 82 | hsa-miR-382 | 1029 | hsa-mir-382 |
| 83 | hsa-miR-412 | 1030 | hsa-mir-412 |
| 84 | hsa-miR-422a | 1031 | hsa-mir-422a |
| 85 | hsa-miR-422b | 1027 | hsa-mir-378 |
| 86 | hsa-miR-423 | 1032 | hsa-mir-423 |
| 87 | hsa-miR-431 | 1033 | hsa-mir-431 |
| 88 | hsa-miR-432* | 1034 | hsa-mir-432 |
| 89 | hsa-miR-433 | 1035 | hsa-mir-433 |
| 90 | hsa-miR-449 | 1036 | hsa-mir-449 |
| 91 | hsa-miR-451 | 1037 | hsa-mir-451 |
| 92 | hsa-miR-452 | 1038 | hsa-mir-452 |
| 93 | hsa-miR-452* | 1038 | hsa-mir-452 |
| 94 | hsa-miR-455 | 1039 | hsa-mir-455 |
| 95 | hsa-miR-483 | 1040 | hsa-mir-483 |
| 96 | hsa-miR-485-5p | 1041 | hsa-mir-485 |
| 97 | hsa-miR-488 | 1042 | hsa-mir-488 |
| 98 | hsa-miR-489 | 1043 | hsa-mir-488 |
| 99 | hsa-miR-491 | 1044 | hsa-mir-491 |
| 100 | hsa-miR-493-5p | 1045 | hsa-mir-493 |
| 101 | hsa-miR-494 | 1046 | hsa-mir-494 |
| 102 | hsa-miR-501 | 1047 | hsa-mir-501 |
| 103 | hsa-miR-504 | 1048 | hsa-mir-504 |
| 104 | hsa-miR-505 | 1049 | hsa-mir-505 |
| 105 | hsa-miR-506 | 1050 | hsa-mir-505 |
| 106 | hsa-miR-507 | 1051 | hsa-mir-507 |
| 107 | hsa-miR-509 | 1052 | hsa-mir-509 |
| 108 | hsa-miR-510 | 1053 | hsa-mir-510 |
| 109 | hsa-miR-511 | 1054 | hsa-mir-511-1 |
| | | 1055 | hsa-mir-5111-2 |
| 110 | hsa-miR-512-3p | 1056 | hsa-mir-512-1 |
| | | 1057 | hsa-mir-512-2 |
| 111 | hsa-miR-512-5p | 1056 | hsa-mir-512-1 |
| | | 1057 | hsa-mir-512-2 |
| 112 | hsa-miR-513 | 1058 | hsa-mir-513-1 |
| | | 1059 | hsa-mir-513-2 |
| 113 | hsa-miR-516-5p | 1060 | hsa-mir-516-1 |
| | | 1061 | hsa-mir-515-2 |
| | | 1062 | hsa-mir-516-3 |
| | | 1063 | hsa-mir-516-4 |
| 114 | hsa-miR-517* | 1064 | hsa-mir-517a |
| | | 1065 | hsa-mir-517b |
| | | 1066 | hsa-mir-517c |
| 115 | hsa-miR-517a | 1064 | hsa-mir-517a |
| 116 | hsa-miR-517c | 1065 | hsa-mir-517c |
| 117 | hsa-miR-517b | 1065 | hsa-mir-517b |
| 118 | hsa-miR-518b | 1067 | hsa-mir-518b |
| 119 | hsa-miR-518c | 1068 | hsa-mir-518c |
| 120 | hsa-miR-518d | 1069 | hsa-mir-518d |
| 121 | hsa-miR-518c* | 1068 | hsa-mir-518c |
| 122 | hsa-miR-518a | 1070 | hsa-mir-518e |
| 123 | hsa-miR-518f | 1071 | hsa-mir-518f |
| 124 | hsa-miR-520h | 1072 | hsa-mir-520h |
| 125 | hsa-miR-523 | 1073 | |
| 126 | hsa-miR-524* | 1074 | hsa-mir-523 hsa-mir-524 |
| 127 | hsa-miR-525 | 1075 | hsa-mir-525 |
| 128 | hsa-miR-525a | 1076 | hsa-mir-525a-1 |
| | | 1077 | hsa-mir-526a-2 |
| 129 | hsa-miR-527 | 1078 | hsa-mir-527 |

**[Table 3-3]**

| SEQ ID NO: | Name of mRNA | SEQ ID NO: | Name of mIRNA precursor |
|---|---|---|---|
| 130 | mmu-miR-1 | 1079 | mmu-mir-1-1 |
| | | 1080 | mmu-mir-1-2 |
| 131 | mo-miR-1 | 108 | 1081 mo-mir-1 |
| 132 | ppa-miR-1 | 1082 | ppa-mir-1 |
| 133 | mdo-miR-1 | 1083 | mdo-mir-1 |
| | | 1084 | gga-mir-1a-1 |
| 134 | gga-miR-1a | 1085 | gga-mir-1a-2 |
| 135 | gga-miR-1b | 1086 | gga-mir-1b |
| 136 | xLr-miR-1b | 1087 | xLr-mir-1b |
| 137 | dre-miR-1 | 1088 | dre-mir-1-1 |
| | | 1089 | dre-mir-1-2 |
| 138 | fru-miR-1 | 1090 | fru-mir-1 |
| 139 | tni-miR-1 | 1091 | tri-mir-1 |
| 140 | mmu-miR-206 | 1092 | mmu-mir-206 |
| 141 | mo-miR-206 | 1093 | mo-mir-206 |
| 142 | mne-miR-206 | 1094 | mne-mir-206 |
| 143 | poy-miR-206 | 1095 | ppy-mir-206 |
| 144 | mdo-miR-206 | 1096 | mdo-mir-206 |
| 145 | gge-miR-206 | 1097 | gga-mir-206 |
| 146 | xir-miR-205 | 1098 | xtr-mir-206 |
| 147 | dre-miR-206 | 1099 | dre-mir-206-1 |
| | | 1100 | dre-mir-206-2 |
| 148 | mmu-miR-7b | 1101 | mmu-mir-7b |
| 149 | mo-miR-7a | 1102 | mo-mi-7a-1 |
| | | 1103 | mo-mir-7a-2 |
| 150 | mo-miR-7b | 1104 | mo-mir-7b |
| 151 | lla-miR-7 | 1105 | lla-mir-7 |
| 152 | sla-miR-7 | 1106 | sla-mir-7 |
| 153 | mne-miR-7 | 1107 | mne-mir-7-1 |
| | | 1108 | mne-mir-7-2 |
| 154 | ggo-miR-7 | 1109 | ggo-mir-7-1 |
| | | 1110 | ggo-mir-7-2 |
| | | 1111 | ggo-mir-7-3 ppa-mir-7-1 |
| 155 | ptr-miR-7 | 1112 | |
| | | 1113 | ppa-mir-7-2 |
| | | 1114 | ppa-mir-7-3 |
| 156 | ptr-miR-7 | 1115 | ptr-mir-7 |
| 157 | ppy-miR-7 | 1116 | ppy-mir-7-1 |
| | | 1117 | ppy-mir-7-2 |
| 158 | mdo-miR-7 | 1118 | mdo-mir-7 |
| 159 | gga-miR-7 | 1119 | gga-mir-7-1 |
| | | 1120 | gga-mir-7-2 |
| | | 1121 | gga-mir-7-3 |
| 160 | gga-miR-7b | 1122 | gga-mir-7b |
| 161 | xtr-miR-7 | 1123 | xtr-mir-7-1 |
| | | 1124 | 1124 xtr-mir-7-2 |
| | | 1125 | 1125 xtr-mir-7-3 |
| 162 | bta-miR-7 | 1126 | 1126 bta-mir-7 |
| 163 | ssc-miR-7 | 1127 | sso-mir-7 |
| 164 | dre-miR-7e | 1128 | dre-mir-7a-1 |
| | | 1129 | dre-mir-7a-2 |
| | | 1130 | re-mir-7a-3 |
| 165 | dre-miR-7b | 1131 | dre-mir-7b |
| 166 | fru-miR-7 | 1132 | fru-mir-7 |
| 167 | tni-miR-7 | 1133 | tri-mir-7 |
| 168 | mmu-miR-10a | 1134 | mmu-mir-10a |
| 169 | age-miR-10a | 1135 | age-mir-10a |
| 170 | sla-miR-10a | 1136 | sla-mir-10a |
| 171 | mne-miR-10b | 1137 | 1137 mne-mir-10b |
| 172 | ggo-miR-10a | 1138 | ggo-mir-10a |
| 173 | ggo-miR-10b | 1139 | ggo-mir-10b |
| 174 | ppa-miR-10a | 1140 | ppa-mir-10e |
| 175 | ppa-miR-10b | 1141 | ppa-mir-10b |
| 176 | mdo-miR-10e | 1142 | mdo-mir-10a |
| 177 | gga-miR-10b | 1143 | gga-mir-10b |
| 178 | xtr-miR-10a | 1144 | xtr-mir-10e |
| 179 | xtr-miR-10b | 1145 | xtr-mir-10b |
| 180 | xtr-miR-10c | 1146 | xtr-mir-10c |
| 181 | bte-miR-10a | 1147 | bta-mir-10a |
| 182 | bte-miR-10b | 1148 | bta-mir-10b |
| 183 | dre-miR-10e | 1149 | dre-mir-10a |
| 184 | dre-miR-10b | 1150 | dre-mir-10b-1 |
| | | 1151 | dre-mir-10b-2 |
| 185 | dre-miR-10c | 1152 | dre-mir-10c |
| 186 | dre-miR-10d | 1153 | dre-mir-10d-1 |
| | | 1154 | dre-mir-10d-2 |

**[Table 3-4]**

| SEQ ID NO: | Name of miRNA | SEQ ID NO: | Name of mRMA precursor |
|---|---|---|---|
| 187 | fru-miR-10b | 1155 | fur-mir-10b-1 |
| | | 1156 | fur-mir-10b-2 |
| 188 | fru-miR-10c | 1157 | fur-mir-10c |
| 189 | fru-miR-10d | 1158 | fur-mir-10d |
| 190 | tni-miR-10b | 1159 | tni-mir-10b-1 |
| | | 1160 | tni-mir-10b-2 |
| 191 | tni-miR-10c | 1161 | tni-mir-10c |
| 192 | tni-miR-10d | 1162 | tni-mir-10d |
| 193 | mmu-miR-18e | 1163 | mmu-mir-18a |
| 194 | mo-miR-18a | 1164 | mo-mir-18a |
| 195 | age-miR-18 | 1165 | age-mir-18 |
| 196 | lle-miR-18 | 1166 | lla-mir-18 |
| 197 | sla-miR-18 | 1167 | sia-mir-18 |
| 198 | mml-miR-18 | 1168 | mml-mir-18 |
| 199 | mme-miR-18 | 1169 | mne-mir-18 |
| 200 | ggc-miR-18 | 1170 | ggo-mir-18 |
| 201 | ppa-miR-18 | 1171 | ppa-mir-18 |
| 202 | ptr-miR-18 | 1172 | ptr-mir-18 |
| 203 | ppy-miR-18 | 1173 | ppy-mir-18 |
| 204 | lca-miR-18 | 1174 | lca-mir-18 |
| 205 | mdo-miR-18 | 1175 | mdo-mir-18 |
| 206 | gga-miR-18a | 1176 | gge-mir-18a |
| 207 | xtr-miR-18a | 1177 | xtr-mir-18a |
| 208 | bta-miR-18a | 1178 | bta-mir-18a |
| 209 | ssc-miR-18 | 1179 | ssc-mir-18 |
| 210 | dre-miR-18a | 1180 | dre-mir-18a |
| 211 | dre-miR-18c | 1181 | dre-mir-18c |
| 212 | fru-miR-18 | 1182 | fru-mir-18 |
| 213 | tni-miR-18 | 1183 | tne-mir-18 |
| 214 | mmu-miR-18b | 1184 | mme-mir-18b |
| 215 | gga-miR-18b | 1185 | gga-mir-18b |
| 216 | xtr-miR-18b | 1186 | xtr-mir-18b |
| 217 | bta-miR-18b | 1187 | bta-mir-18b |
| 218 | dre-miR-18b | 1188 | dre-mir-18b |
| 219 | mmu-miR-18a* | 1183 | mmu-mir-18a |
| 220 | mmu-miR-20b | 1189 | mmo-mir-20b |
| 221 | age-miR-20 | 1190 | age-mir-20 |
| 222 | lla-miR-20 | 1191 | Ba-mir-20 |
| 223 | sla-miR-20 | 1192 | sle-mir-20 |
| 224 | mme-miR-20 | 1193 | mml-mir-20 |
| 225 | mme-miR-20 | 1194 | mne-mir-20 |
| 226 | ggo-miR-20 | 1195 | ggo-mir-20 |
| 227 | ppa-miR-20 | 1196 | ptr-mir-20 |
| 228 | ptr-miR-20 | 1197 | ppa-mir-20 |
| 229 | ppy-miR-20 | 1198 | ppy-mir-20 |
| 230 | lca-miR-20 | 1199 | loe-mir-20 |
| 231 | mdo-miR-20 | 1200 | mdo-mir-20 |
| 232 | gga-miR-20b | 1201 | gge-mir-20b |
| 233 | xtr-miR-20b | 1202 | xtr-mir-20b |
| 234 | bta-miR-20b | 1203 | bte-mir-20b |
| 235 | ssc-miR-20 | 1204 | ssc-mir-20 |
| 236 | dre-miR-20b | 1205 | dre-mir-20b |
| 237 | fru-miR-20 | 1206 | fru-mir-20 |
| 238 | tni-miR-20 | 1207 | tni-mir-20 |
| 239 | mmu-miR-106a | 1208 | mmu-mir-106a |
| 240 | age-miR-106a | 1209 | age-mir-106a |
| 241 | sla-miR-106a | 1210 | sla-mir-106a |
| 242 | mml-miR-106a | 1211 | mml-mir-106a |
| 243 | mne-miR-106a | 1212 | mne-mir-106a |
| 244 | ggo-miR-106a | 1213 | ggo-mir-106a |
| 245 | ppa-miR-106a | 1214 | ppe-mir-106a |
| 246 | ptr-miR-106a | 1215 | ptr-mir-106a |
| 247 | ppy-miR-106a | 1216 | ppy-mir-106a |
| 248 | gga-miR-106 | 1217 | gga-mir-106 |
| 249 | xtr-miR-106 | 1218 | xtr-mir-106 |
| 250 | bte-miR-106 | 1219 | bta-mir-106 |
| 251 | ssc-miR-106a | 1220 | ssc-mir-106a |
| 252 | mmu-miR-106b | 1221 | mmu-mir-106b |
| 253 | mo-miR-106b | 1222 | mo-mir-106b |
| 254 | age-miR-106b | 1223 | age-mir-106b |
| 255 | lla-miR-106b | 1224 | lla-mir-106b |
| 256 | sla-miR-106b | 1225 | sla-mir-106b |
| 257 | mml-miR-106b | 1226 | mml-mir-106b |
| 258 | mne-miR-106b | 1227 | mne-mir-106b |
| 259 | ggo-miR-106b | 1228 | ggo-mir-106b |
| 260 | ppa-miR-106b | 1229 | ppa-mir-106b |
| 261 | ptr-miR-106b | 1230 | ptr-mir-106b |
| 262 | ppy-miR-106b | 1231 | ppy-mir-106b |
| 263 | mmu-miR-24 | 1232 | mmu-mir-24-1 |
| | | 1233 | mmu-mir-24-2 |

**[Table 3-5]**

| SEQ ID NO: | Name of mIRNA | SEQ ID NO: | Name of mRNA precursor |
|---|---|---|---|
| 264 | mo-miR-24 | 1234 | mo-mir-24-1 |
| | | 1235 | mo-mir-24-2 |
| 265 | mml-miR-24 | 1236 | mml-mir-24-1 |
| | | 1237 | mml-mir-24-2 |
| 266 | mne-miR-24 | 1238 | mne-mir-24-1 |
| | | 1239 | mne-mir-24-2 |
| 267 | ggo-miR-24 | 1240 | ggo-mir-24 |
| 268 | ppa-miR-24 | 1241 | ppa-mir-24-1 |
| | | 1242 | ppa-mir-24-2 |
| 269 | ptr-miR-24 | 1243 | ptr-mir-24 |
| 270 | ppy-miR-24 | 1244 | ppy-mir-24-1 |
| | | 1245 | ppy-mir-24-2 |
| 271 | mdo-miR-24 | 1246 | mdo-mir-24-1 |
| | | 1247 | mdo-mir-24-2 |
| 272 | gga-miR-24 | 1248 | gga-mir-24 |
| 273 | xtr-miR-24b | 1249 | xtr-mir-24b |
| 274 | bta-miR-24 | 1250 | bta-mir-24 |
| 275 | ssc-miR-24 | 1251 | ssc-mir-24 |
| 276 | dre-miR-24 | 1252 | dre-mir-24-1 |
| | | 1253 | dre-mir-24-2 |
| | | 1254 | dre-mir-24-3 |
| | | 1255 | dre-mir-24-4 |
| 277 | fru-miR-24 | 1256 | fru-mir-24-1 |
| | | 1257 | fru-mir-24-2 |
| 278 | tni-miR-24 | 1258 | tni-mir-24-1 |
| | | 1259 | tni-mir-24-2 |
| 279 | mmu-miR-25 | 1260 | mmu-mir-25 |
| 280 | mo-miR-25 | 1261 | mo-mir-25 |
| 281 | lla-miR-25 | 1262 | lla-mir-25 |
| 282 | mml-miR-25 | 1263 | mml-mir-25 |
| 283 | mne-miR-25 | 1264 | mne-mir-25 |
| 284 | ggo-miR-25 | 1265 | ggo-mir-25 |
| 285 | ppe-miR-25 | 1266 | ppa-mir-25 |
| 286 | ptr-miR-25 | 1267 | ptr-mir-25 |
| 287 | ppy-miR-25 | 1268 | ppy-mir-25 |
| 288 | mdo-miR-25 | 1269 | mdo-mir-25 |
| 289 | xtr-miR-25 | 1270 | xtr--mir-25-1 |
| | | 1271 | xtr-mir-25-2 |
| 290 | bta-miR-25 | 1272 | bta-mir-25 |
| 291 | dre-miR-25 | 1273 | dre-mir-25 |
| 292 | fru-miR-25 | 1274 | fru-mir-25 |
| 293 | tni-miR-25 | 1275 | tni-mir-25 |
| 294 | mmu-miR-32 | 1276 | mmu-mir-32 |
| 295 | mo-miR-32 | 1277 | mo-mir-32 |
| 296 | sia-miR-32 | 1278 | sla-mir-32 |
| 297 | mml-miR-32 | 1279 | mml-mir-32 |
| 298 | mne-miR-32 | 1280 | mne-mir-32 |
| 299 | ggo-miR-32 | 1281 | ggo-mir-32 |
| 300 | ppa-miR-32 | 1282 | ppe-mir-32 |
| 301 | ptr-miR-32 | 1283 | ptr-mir-32 |
| 302 | ppy-miR-32 | 1284 | ppy-mir-32 |
| 303 | mdo-miR-32 | 1285 | mdo-mir-32 |
| 304 | gga-miR-32 | 1286 | gga-mir-32 |
| 305 | ssc-miR-32 | 1287 | ssc-mir-32 |
| 306 | mmu-miR-26a | 1288 | mmu-mir-26a-1 |
| | | 1289 | mmu-mir-26a-2 |
| 307 | mo-miR-26a | 1290 | mo-mir-26a |
| 308 | lia-miR-26a | 1291 | lla-mir-26a |
| 309 | mml-miR-26a | 1292 | mml-mir-26a |
| 310 | mne-miR-26a | 1293 | mne-mir-26a |
| 311 | ggo-miR-26a | 1294 | ggo-mir-26a |
| 312 | ppa-miR-26a | 1295 | ppa-mir-26a |
| 313 | ptr-miR-26a | 1296 | ptr-mir-26a |
| 314 | ppy-miR-26a | 1297 | ppy-mir-26a |
| 315 | gga-miR-26a | 1298 | gga-mir-26a |
| 316 | bta-miR-26a | 1299 | bta-mir-26a |
| 317 | ssc-miR-26a | 1300 | ssc-mir-26a |
| 318 | dre-miR-26a | 1301 | dre-mir-26a-1 |
| | | 1302 | dre-mir-26a-2 |
| | | 1303 | dre-mir-26a-3 |
| 319 | fru-miR-26 | 1304 | fru-mir-26 |
| 320 | tni-miR-26 | 1305 | tni-mir-26 |
| 321 | mmu-miR-26b | 1306 | mmu-mir-26b |
| 322 | mo-miR-26b | 1307 | mo-mir-26b |

**[Table 3-6]**

| SEQ ID NO: | Name of miRNA | SEQ ID NO: | Name of mRNA precursor |
|---|---|---|---|
| 323 | bta-miR-26b | 1308 | bta-mir-26b |
| 324 | dre-miR-26b | 1309 | dre-mir-26b |
| 325 | mmu-miR-27b | 1310 | mmu-mir-27b |
| 326 | mo-miR-27a | 1311 | mo-mir-27a |
| 327 | mo-miR-27b | 1312 | mo-mir-27b |
| 328 | age-miR-27a | 1313 | age-mir-27a |
| 329 | sla-miR-27a | 1314 | sla-mir-27a |
| 330 | mml-miR-27a | 1315 | mml-mir-27a |
| 331 | mne-miR-27a | 1316 | mne-mir-27a |
| 332 | ggo-miR-27a | 1317 | ggo-mir-27a |
| 333 | ppa-miR-27a | 1318 | ppa-mir-27a |
| 334 | ptr-miR-27a | 1319 | ptr-mir-27a |
| 335 | ppy-miR-27a | 1320 | ppy-mir-27a |
| 336 | lca-miR-27a | 1321 | lca-mir-27a |
| 337 | mdo-miR-27a | 1322 | mdo-mir-27a |
| 338 | mdo-miR-27b | 1323 | mdo-mir-27b |
| 339 | gga-miR-27b | 1324 | gga-mir-27b |
| 340 | xtr-miR-27a | 1325 | xtr-mir-27a |
| 341 | xtr-miR-27b | 1326 | xtr-mir-27b |
| 342 | xtr-miR-27c | 1327 | xtr-mir-27c-1 |
| | | 1328 | age-mir-27c-2 |
| 343 | bta-miR-27a | 1329 | bta-mir-27a |
| 344 | bta-miR-27b | 1330 | bta-mir-27b |
| 345 | ssc-miR-27a | 1331 | ssc-mir-27a |
| 346 | dre-miR-27a | 1332 | dre-mir-27a |
| 347 | dre-miR-27b | 1333 | dre-mir-27b |
| 348 | dre-miR-27c | 1334 | dre-mir-27c |
| 349 | dre-miR-27d | 1335 | dre-mir-27d |
| 350 | dre-miR-27e | 1336 | dre-mir-27e |
| 351 | fru-miR-27b | 1337 | fru-mir-27b |
| 352 | fru-miR-27c | 1338 | fru-mir-27c |
| 353 | fru-miR-27e | 1339 | fru-mir-27e |
| 354 | fru-miR-27b | 1340 | tni-mir-27b |
| 355 | tni-miR-27c | 1341 | tni-mir-27c |
| 356 | tni-miR-27e | 1342 | tni-mir-27e |
| 357 | mmu-miR-28 | 1343 | mmu-mir-28 |
| 358 | mo-miR-28 | 1344 | mo-mir-28 |
| 359 | age-miR-28 | 1345 | age-mir-28 |
| 360 | lia-miR-28 | 1346 | lla-mir-28 |
| 361 | sla-miR-28 | 1347 | sle-mir-28 |
| 362 | mml-miR-28 | 1348 | mml-mir-28 |
| 363 | mne-miR-28 | 1349 | mne-mir-28 |
| 364 | ggo-miR-28 | 1350 | ggo-mir-28 |
| 365 | ppa-miR-28 | 1351 | ppa-mir-28 |
| 366 | ptr-miR-28 | 1352 | ptr-mir-28 |
| 367 | ppy-miR-28 | 1353 | ppy-mir-28 |
| 368 | ssc-miR-28 | 1354 | ssc-mir-28 |
| 369 | mmu-miR-29a | 1355 | mmu-mir-29a |
| 370 | mo-miR-29a | 1356 | mo-mir-29a |
| 371 | mo-miR-29b | 1357 | mo-mir-29b-1 |
| | | 1358 | mo-mir-29b-2 |
| 372 | age-miR-29b | 1359 | age-mir-29b |
| 373 | lla-miR-29b | 1360 | lla-mir-29b |
| 374 | sia-miR-29b | 1361 | sla-mir-29b |
| 375 | mne-miR-29b | 1362 | mne-mir-29b |
| 376 | ggo-miR-29b | 1363 | ggo-mir-29b-1 |
| | | 1364 | ggo-mir-29b-2 |
| 377 | ppa-miR-29b | 1365 | ppa-mir-29b-1 |
| | | 1366 | ppa-mir-29b-2 |
| 378 | ptr-miR-29b | 1367 | ptr-mir-29b-1 |
| | | 1368 | ptr-mir-29b-2 |
| 379 | ppy-miR-29b | 1369 | ppy-mir-29b-1 |
| | | 1370 | ppy-mir-29b-2 |
| 380 | mdo-miR-29a | 1371 | mdo-mir-28a |
| 381 | mdo-miR-29b | 1372 | mdo-mir-29b |
| 382 | gga-miR-29a | 1373 | gge-mir-29a |
| 383 | gga-miR-29b | 1374 | gge-mir-29b-1 |
| | | 1375 | gge-mir-29b-2 |
| 384 | xtr-miR-29a | 1376 | xtr-mir-29e |
| 385 | xtr-miR-29b | 1377 | xtr-mir-29b |
| 386 | xtr-miR-29d | 1378 | xtr-mir-29d |
| 387 | bta-miR-29b | 1379 | bte-mir-29b |
| 388 | ssc-miR-29b | 1380 | ssc-mir-29b |
| 389 | dre-miR-29a | 1381 | dre-mir-29a-1 |
| | | 1382 | dre-mir-29a-2 |
| 390 | dre-miR-29b | 1383 | dre-mir-29b-1 |
| | | 1384 | dre-mir-29b-2 |
| | | 1385 | dre-mir-29b-3 |

**[Table 3-7]**

| SEQ ID NO: | Name of mIRNA | SEQ ID NO: | Name of mIRNA precursor |
|---|---|---|---|
| 391 | fru-miR-29a | 1386 | fru-mir-29a-1 |
| | | 1387 | fru-mir-29a-2 |
| 392 | fru-miR-29b | 1388 | fru-mir-29b-1 |
| | | 1389 | fru-mir-29b-2 |
| 393 | tni-miR-29a | 1390 | tni-mir-29a-1 |
| | | 1391 | tni-mir-29a-2 |
| 394 | tni-miR-29b | 1392 | tni-mir-29b-1 |
| | | 1393 | tni-mir-29b-2 |
| 395 | mmu-miR-28c | 1394 | mmu-mir-29c |
| 396 | mo-miR-29c | 1395 | mo-mir-29c |
| 387 | gga-miR-29c | 1396 | gge-mir-29c |
| 398 | xtr-miR-29c | 1397 | xtr-mir-29c |
| 399 | bta-miR-29c | 1398 | bta-mir-29c |
| 400 | ssc-miR-29c | 1399 | ssc-mir-29c |
| 401 | mmu-miR-30b | 1400 | mmu-mir-30b |
| 402 | mmu-miR-30d | 1401 | mmu-mir-30d |
| 403 | mmu-miR-30e | 1402 | mmu-mir-30e |
| 404 | mo-miR-30a | 1403 | mo-mir-30a |
| 405 | mo-miR-30d | 1404 | mo-mir-30d |
| 406 | mo-miR-30e | 1405 | mo-mir-30e |
| 407 | age-miR-30b | 1406 | age-mir-30b |
| 408 | lla-miR-30b | 1407 | lla-mir-30b |
| 409 | mmi-miR-30a-5p | 1408 | mml-mir-30a |
| 410 | mmi-miR-30b | 1409 | mml-mir-30b |
| 411 | mne-miR-30b | 1410 | mne-mir-30b |
| 412 | mne-miR-30d | 1411 | mne-mir-30d |
| 413 | ggo-miR-30a-5p | 1412 | ggo-mir-30a |
| 414 | ggo-miR-30b | 1413 | ggo-mir-30b |
| 415 | ggo-miR-30d | 1414 | ggo-mir-30d |
| 416 | ppa-miR-30a-5p | 1415 | ppa-mir-30a |
| 417 | ppa-miR-30b | 1416 | ppa-mir-30b |
| 418 | ppa-miR-30d | 1417 | ppa-mir-30d |
| 419 | ptr-miR-30a-5p | 1418 | ptr-mir-30a |
| 420 | ptr-miR-30b | 1419 | ptr-mir-30b |
| 421 | ptr-miR-30d | 1420 | ptr-mir-30d |
| 422 | opy-miR-30a-5p | 1421 | ppy-mir-30a |
| 423 | mdo-miR-30a | 1422 | mdo-mir-30a |
| 424 | gga-miR-30a-5p | 1423 | gge-mir-30a |
| 425 | gga-miR-30b | 1424 | gga-mir-30b |
| 426 | gga-miR-30d | 1425 | gga-mir-30d |
| 427 | gga-miR-30e | 1426 | gge-mir-30e |
| 428 | xtr-miR-30a-5p | 1427 | xtr-mir-30a |
| 429 | xtr-miR-30b | 1428 | xtr-mir-30b |
| 430 | xtr-miR-30d | 1429 | xtr-mir-30d |
| 431 | xtr-miR-30e | 1430 | xtr-mir-30e |
| 432 | bta-miR-30a-5p | 1431 | bte-mir-30a |
| 433 | bta-miR-30b | 1432 | bta-mir-30b |
| 434 | bta-miR-30d | 1433 | bta-mir-30d |
| 435 | bta-miR-30e-5p | 1434 | bta-mir-30e |
| 436 | dre-miR-30a | 1435 | bre-mir-30a |
| 437 | dre-miR-30b | 1436 | dre-mir-30b |
| 438 | dre-miR-30d | 1437 | dre-mir-30d |
| 439 | dre-miR-30e | 1438 | dre-mir-30c-2 |
| 440 | fru-miR-30b | 1439 | fru-mir-30b |
| 441 | fru-miR-30d | 1440 | fru-mir-30d |
| 442 | tni-miR-30d | 1441 | tni-mir-30b |
| 443 | tni-miR-30d | 1442 | tni-mir-30d |
| 444 | mnu-miR-30c | 1443 | mmu-mir-30c-1 |
| | | 1444 | mmu-mir-30c-2 |
| 445 | mo-miR-30c | 1445 | mo-mir-30c-1 |
| | | 1446 | mo-mir-30c-2 |
| 446 | lla-miR-30c | 1447 | lla-mir-30c |
| 447 | mo-miR-30e | 1448 | mne-mir-30c |
| 448 | ptr-miR-30c | 1449 | ptr-mir-30c |
| 449 | gga-miR-30c | 1450 | gge-mir-30c-1 |
| | | 1451 | gga-mir-30c-2 |
| 450 | xtr-miR-30c | 1452 | xtr-mir-30c-1 |
| | | 1453 | xtr-mir-30c-2 |
| 451 | ssc-miR-30c | 1454 | bta-mir-30c |
| 452 | ssc-miR-30c | 1455 | ssc-mir-30c |
| 453 | dre-miR-30c | 1456 | dre-mir-30c |
| 454 | fru-miR-30c | 1457 | fru-mir-30c |
| 455 | tni-miR-30c | 458 | tni-mir-30c |
| 456 | age-miR-93 | 1459 | age-mir-93 |
| 457 | lle-miR-93 | 1460 | lla-mir-93 |
| 456 | sie-miR-93 | 1461 | sle-mir-93 |
| 459 | mml-miR-93 | 1462 | mmi-mir-93 |
| 460 | mne-miR-93 | 1463 | mne-mir-93 |
| 461 | ggo-miR-93 | 1464 | ggo-mir-93 |
| 462 | ppa-miR-93 | 1465 | ppa-mir-93 |
| 463 | ptr-miR-93 | 1466 | ptr-mir-93 |
| 464 | ppy-miR-93 | 1467 | ppy-mir-93 |

**[Table 3-8]**

| SEQ ID NO: | Name of miRNA | SEQ ID ND: | Name of mlRNA precursor |
|---|---|---|---|
| 465 | mdo-miR-93 | 1468 | mdo-mir-93 |
| 466 | mmu-miR-302b | 1469 | mmu-mir-302b |
| 467 | mmu-miR-302d | 1470 | mmu-mir-302d |
| 468 | mdo-miR-302a | 1471 | mdo-mir-302a |
| 469 | mdo-miR-302b | 1472 | mdo-mir-302b |
| 470 | mdo-miR-302c | 1473 | mdo-mir-302c |
| 471 | mdo-miR-302d | 1474 | mdo-mir-302d |
| 472 | gga-miR-302b | 1475 | gga-mir-302b |
| 473 | gga-miR-302c | 1476 | gga-mir-302c |
| 474 | gga-miR-302d | 1477 | gga-mir-302d |
| 475 | xtr-miR-302 | 1478 | xtr-mir-302 |
| 476 | lla-miR95 | 1479 | lla-mir-95 |
| 477 | sta-miR-95 | 1480 | sla-mir-95 |
| 478 | ggo-miR-95 | 1481 | ggo-mir-95 |
| 479 | ppa-miR-95 | 1482 | ppa-mir-95 |
| 480 | ptr-miR-95 | 1483 | mmu-mir-98 |
| 481 | ppy-miR-95 | 1464 | ppy-mir-95 |
| 482 | ssc-miR-95 | 1485 | ssc-mir-95 |
| 483 | mmu-miR-98 | 1486 | mmu-mir-98 |
| 484 | rno-miR-98 | 1487 | mo-mir-98 |
| 485 | age-mir-98 | 1488 | age-mir-98 |
| 486 | mmi-miR-98 | 1689 | mml-mir-98 |
| 487 | ggo-miRR-98 | 1490 | ggo-mir-98 |
| 488 | ppa-miR-98 | 1491 | ppa-mir-98 |
| 489 | ptr-miR-98 | 1492 | ptr-mir-98 |
| 490 | ppy-miR-98 | 1493 | ppy-mir-98 |
| 491 | xtr-miR-98 | 1494 | xtr-mir-98 |
| 492 | bta-miR-98 | 1495 | bta-mir-98 |
| 493 | mmu-miR-73Da | 1496 | mmu-mir-130a |
| 494 | mo-miR-13Da | 1497 | mo-mir-130a |
| 495 | mml-miR-130a | 1498 | mml-mir-130a |
| 496 | mme-miR-130a | 1499 | mne-mir-130a |
| 497 | ggo-miR-130a | 1500 | ggo-mir-130a |
| 498 | ppa-miR-130a | 1501 | gga-mir-130a |
| 499 | mdo-miR-130a | 1502 | mdo-mir-130a |
| 500 | gga-miR-130a | 1503 | gge-mir-130a |
| 501 | xtr-mir-130a | 1504 | xtr-mir-130a |
| 502 | dre-miR-330a | 1505 | dre-mir-130a-1 |
| | | 1506 | dre-mir-130a-2 |
| 503 | dre-miR-130c | 1507 | dre-mir-130c-1 |
| | | 1508 | dre-mir-130c-2 |
| 504 | fru-miR-130 | 1509 | fru-mir-130 |
| 505 | tni-miR-130 | 1510 | tni-mir-130 |
| 506 | mma-miR-130b | 1511 | mmu-mur-130b |
| 507 | mo-miR-130b | 1512 | rno-mir-130b |
| 508 | gga-miR-130b | 1513 | gga-mir-130b |
| 509 | xtr-miR-130b | 1514 | xts-mir-130b |
| 510 | dre-miR-130b | 1515 | dre-mir-130b |
| 511 | mmu-miR-301a | 1516 | mmo-mir-301a |
| 512 | mmu-miR-301b | 1517 | mmo-mir-301b |
| 513 | mmo-miR-301a | 1518 | rno-mir-301a |
| 514 | rno-miR-301b | 1519 | rno-mir-301b |
| 515 | gge-miR-301 | 1520 | gga-mir-301 |
| 516 | xtr-miR-301 | 1521 | xtr-mir-301-1 |
| | | 1522 | xtr-mir-301-2 |
| 517 | dre-mir-301a | 1523 | dre-mir-301a-1 |
| | | 1524 | dre-mir-301a-2 |
| 518 | dre-miR-301b | 1525 | dre-mir-301b |
| 519 | dre-miR-301c | 1576 | dre-mir-301c |
| 520 | fru-mir-301 | 1527 | fru-mir-301 |
| 521 | tni-miR-301 | 1528 | tri-mir-301 |
| 522 | mmu-miR-132 | 1529 | mmu-mir-132 |
| 523 | rno-miR-132 | 1530 | rno-mir-132 |
| 524 | mdo-miR-132 | 1531 | mdo-mir-132 |
| 525 | xtr-miR-132 | 1532 | xtr-mir-132 |
| 526 | tra-miR-132 | 1533 | bta-mir-132 |
| 527 | dre-miR-132 | 1534 | dre-mir-13-1 |
| | | 1535 | dre-mir-132-2 |
| 520 | fru-miR-132 | 1536 | fru-mir-132 |
| 528 | tri-miR-132 | 1537 | tre-mir-132 |
| 530 | mmo-miR-212 | 1538 | mmu-mir-212 |
| 531 | mo-miR-212 | 1539 | rno-mir-212 |
| 532 | mdo-miR-212 | 1540 | mdo-mir-212 |
| 533 | xtr-miR-212 | 1541 | xtr-mir-212 |
| 534 | dre-miR-212 | 1542 | dre-mir-212 |
| 535 | fru-miR-212 | 1543 | fro-mir-212 |
| 536 | tni-miR-212 | 1544 | tni-mir-212 |
| 537 | age-miR-133a | 1545 | age-mir-133a |
| 538 | lla-miR-133a | 1546 | fla-mir-133a |
| 539 | sla-miR-133a | 1547 | sle-mir-133a |
| 540 | mne-miR-133a | 1548 | mne-mir-133e |
| 541 | ggo-mir-133a | 1549 | ggo-mir-133a |

**[Table 3-9]**

| SEQ ID NO: | Name of miRNA | SEQ ID NO: | Name of miRLA precursor |
|---|---|---|---|
| 542 | ppa-miR-133a | 1550 | ppa-mir-133a |
| 543 | ptr-miR-133a | 1551 | ptr-mir-133a |
| 544 | mdo-miR-133a | 1552 | mdo-mir-133a |
| 545 | gga-mir-133a | 1553 | gga-mir-133a-1 |
| | | 1554 | gga-mir-133a-2 |
| 546 | gga-miR-133c | 1555 | gga-mir-133c |
| 547 | xia-miR-133a | 1556 | xla-mir-133a |
| 548 | xtr-miR-133a | 1557 | xtr-mir-133a |
| 549 | xtr-miR-133d | 1558 | xtr-mir-133d |
| 550 | gga-miR-133b | 1559 | gga-mir-133b |
| 551 | xta-miR-133b | 1560 | xtr-mir-133b |
| 552 | mmo-miR-134 | 1561 | mmu-mir-134 |
| 553 | mo-miR-134 | 1562 | mo-mir-134 |
| 554 | mne-miR-134 | 1563 | mme-mir-134 |
| 555 | ggo-miR-134 | 1564 | ggo-mir-134 |
| 556 | ppa-miR-134 | 1565 | ppa-mir-134 |
| 557 | ppy-miR-134 | 1566 | ppy-mir-134 |
| 558 | mdo-miR-137 | 1567 | mdo-mir-137 |
| 559 | gga-miR-137 | 1568 | gga-mir-137 |
| 560 | xtr-miR-137 | 1569 | xtr-mir-137-1 |
| | | 1570 | xtr-mir-137-2 |
| | | 1571 | xtr-mir-137-3 |
| 561 | mmu-miR-142-3p | 1572 | mmu-mir-142 |
| 562 | mo-miR-142-3p | 1573 | mo-mir-142 |
| 563 | gga-miR-142-3p | 1574 | gga-mir-142 |
| 564 | xtr-miR-142-3p | 1575 | xtr-mir-142-1 |
| | | 1576 | xtr-mir-142-2 |
| 565 | mmu-miR-146a | 1577 | mmu-mir-146a |
| 566 | mo-miR-146a | 1578 | mo-mir-146a |
| 567 | gga-miR-146a | 1579 | gga-mir-146a |
| 568 | gga-miR-146b | 1580 | gga-mirr-146b |
| 569 | xtr-miR-146 | 1581 | xtr-mir-146 |
| 570 | dre-miR-146a | 1582 | dre-mir-145a |
| 571 | dre-miR-146b | 1583 | dre-mir-146b-1 |
| | | 1584 | dre-mir-146b-2 |
| 572 | sta-miR-147 | 1585 | sle-mir-147 |
| 573 | mne-miR-147 | 1586 | mne-mir-147 |
| 574 | ppa-miR-147 | 1587 | ppa-mir-147 |
| 575 | pir-miR-147 | 1588 | ptr-mir-147 |
| 576 | ppy-miR-147 | 1589 | ppy-mir-147 |
| 577 | mmu-miR-149 | 1590 | mmu-mir-149 |
| 578 | mmu-miR-181c | 1591 | mmu-mir-181c |
| 579 | mo-miR-181c | 1592 | mo-mir-181c |
| 580 | mmf-miR-181c | 1593 | mmo-mir-181c |
| 581 | ggo-miR-181c | 1594 | ggo-mir-181c |
| 582 | ppa-miR-181c | 1595 | ppa-mir-181c |
| 583 | ptr-miR-181c | 1596 | ptr-mir-181c |
| 584 | mdo-miR-181c | 1597 | mdo-mir-181c |
| 585 | bta-miR-181c | 1598 | bta-mir-282c |
| 586 | ssc-miR-181c | 1599 | ssc-mir-181c |
| 587 | dre-miR-181c | 1600 | dre-mir-181c |
| 588 | mmu-miR-181d | 1601 | mmu-mir-181d |
| 569 | mo-miR-181d | 1602 | mo-mir181d |
| 590 | xtr-miR-182* | 1603 | xtr-mir-182 |
| 591 | dre-miR-182* | 1604 | dre-mir-182 |
| 592 | mmu-mir-183 | 1605 | mmu-mir-183 |
| 593 | mo-miR-183 | 1605 | mo-mir-183 |
| 594 | sia-miR-183 | 1607 | sla-mir-183 |
| 595 | mml-miR-183 | 1608 | mnd-mir-183 |
| 596 | mne-miR-183 | 1609 | mne-mir-183 |
| 597 | ggo-miR-183 | 1610 | ggo-mir-183 |
| 598 | ppa-miR-183 | 1611 | ppa-mir-183 |
| 599 | ptr-miR-163 | 1612 | ptr-mir-183 |
| 600 | mdo-miR-183 | 1613 | mdo-mir-183 |
| 601 | gga-miR-183 | 1614 | gga-mir-183 |
| 602 | xtr-miR-183 | 1615 | xtr-mir-183 |
| 603 | ssc-miR-183 | 1616 | ssc-mir-183 |
| 604 | dre-miR-183 | 1617 | dre-mir-183 |
| 605 | fru-miR-183 | 1618 | fru-mir-183 |
| 606 | tni-miR-183 | 1619 | tni-mir-183 |
| 607 | mmu-miR-184 | 1620 | mmu-mir-184 |
| 608 | mo-miR-184 | 1621 | mo-mir-184 |
| 609 | mne-miR-184 | 1922 | mo-mir-184 |
| 610 | ptr-mir-184 | 1623 | ptr-mir-184 |
| 611 | ppy-miR-184 | 1624 | ppy-mir-184 |
| 612 | mdo-miR-184 | 1625 | mdo-mir-184 |
| 613 | gga-miR-184 | 1626 | gge-mir-184 |
| 614 | xtr-miR-184 | 1627 | xtr-mir-184 |
| 615 | ssc-miR-184 | 1628 | ssc-mir-184 |
| 616 | dre-mir-184 | 1629 | dre-mir-184 |
| | | 1630 | dre-mir-184 |
| 617 | fru-miR-184 | 1631 | fru-mir-184 |

**[Table 3-10]**

| SEQ ID NO: | Name of miRNA | SEQ ID NO: | Name of miRNA precursor |
|---|---|---|---|
| 618 | tni-miR-184 | 1632 | tni-mir-184 |
| 619 | mmu-miR-182 | 1633 | mmu-mir-192 |
| 820 | mo-miR-192 | 1634 | mo-mir-192 |
| 621 | xtr-miR-192 | 1635 | xtr-mir-192 |
| 622 | bta-miR-192 | 1636 | bta-mir-192 |
| 623 | dre-miR-192 | 1637 | dre-mir-192 |
| 624 | fru-miR-182 | 1638 | fru-mir-192 |
| 625 | tni-miR-192 | 1639 | tni-mir-192 |
| 626 | mmu-miR-215 | 1640 | mmu-mir-215 |
| 627 | mo-miR-215 | 1641 | mo-mir-215 |
| 628 | mml-miR-215 | 1642 | mmi-mir-4215 |
| 629 | mne-miR-215 | 1643 | mne-mir-215 |
| 630 | ggo-miR-215 | 1644 | ggo-mir-215 |
| 631 | tir-miR-215 | 1645 | ptr-mir-215 |
| 632 | ppy-miR-215 | 1646 | ppy-mir-215 |
| 633 | gga-miR-215 | 1647 | gga-mir-215 |
| 634 | xtr-miR-215 | 1648 | xtr-mir-215 |
| 635 | bta-miR-215 | 1649 | bta-mir-215 |
| 636 | mo-miR-193 | 1650 | mo-mir-193 |
| 637 | mdo-miR-193 | 1651 | mdo-mir-193 |
| 638 | gga-miR-193 | 1652 | gga-mir-193 |
| 639 | xtr-miR-193 | 1653 | xtr-mir-193 |
| 640 | bta-miR-193a | 1654 | bta-mir-193a |
| 641 | dre-miR-193a | 1655 | dre-mir-193-1 |
| | | 1656 | dre-mir-193-2 |
| | | 1657 | dre-mir-193a-3 |
| 642 | fru-miR-193 | 1658 | fru-mir-193 |
| 643 | tni-miR-193 | 1659 | tni-mir-193 |
| 644 | mmu-miR-193b | 1660 | mmu-mir-193b |
| 645 | dre-miR-193b | 1661 | dre-mir-193b |
| 646 | mmu-miR-195 | 1662 | mmu-mir-195 |
| 647 | mo-miR-195 | 1663 | mo-mir-195 |
| 648 | ggo-miR-193 | 1664 | ggo-mir-195 |
| 649 | ppa-miR-195 | 1665 | ppa-mir-195 |
| 650 | bta-miR-195 | 1666 | bta-mir-195 |
| 651 | mmu-miR-497 | 1667 | mmu-mir-497 |
| 652 | mo-miR-497 | 1668 | mo-mir-497 |
| 653 | bta-miR-497 | 1669 | bta-mir-487 |
| 654 | mmu-miR-196a | 1670 | mmu-mir-196a-1 |
| | | 1671 | mmu-mir-196a-2 |
| 655 | mo-miR-196a | 1672 | mo-mir-196a |
| 656 | age-miR-996a | 1673 | age-mir-196 |
| 657 | tla-miR-196 | 1674 | lla-mir-196 |
| 658 | mml-miR-196 | 1675 | mmi-mir-196 |
| 659 | ggo-miR-196 | 1676 | ggo-mir-196-1 |
| | | 1677 | ggo-mir-192-2 |
| 660 | ppa-miR-196 | 1679 | ppa-mir-196 |
| 661 | ptr-miR-196 | 1679 | ptr-mir-196 |
| 662 | ppy-miR-196196 | 1680 | ppy-mir-196-1 |
| | | 1681 | ppy-mir-196-2 |
| 663 | gga-miR-196 | 1682 | gga-mir-196-1 |
| | | 1683 | gga-mir-196-2 |
| | | 1684 | gga-mir-196-3 |
| 664 | xtr-miR-196a | 1685 | xtr-mir-196a |
| 665 | ssc-miR-196 | 1686 | ssc-mir-196 |
| 666 | dre-miR-196a | 1687 | dre-mir-196a-1 |
| | | 1688 | dre-mir-192a-2 |
| 667 | fru-miR-196a | 1989 | fru-mir-192a-1 |
| | | 1690 | fru-mir-192a-2 |
| 668 | tri-miR-195a | 1691 | tni-mir-195a-11 |
| | | 1692 | tni-mir-196a-2 |
| 669 | mmu-miR-196b | 1693 | mmu-mir-196b |
| 670 | mo-miR-196b | 1694 | mo-mir-196b |
| 671 | mdo-miR-195b | 1695 | mdo-mir-196b |
| 672 | xtr-miR-196b | 1696 | xtr-mir-196b |
| 673 | dre-miR-196b | 1697 | dre-mir-196b |
| 674 | fru-miR-196b | 1698 | fru-mir-196b |
| 675 | tni-miR-196b | 1699 | tri-mir-196b |
| 676 | mmu-miR-197 | 1700 | mmo-mir-197 |
| 677 | age-miR-197 | 1701 | age-mir-197 |
| 678 | mme-miR-197 | 1702 | mme-mir-197 |
| 679 | ppa-miR-197 | 1703 | ppe-mir-197 |
| 680 | ptr-miR-197 | 1704 | ptr-mir-197 |
| 681 | ppy-miR-197 | 1705 | ppy-mir-197 |
| 682 | xtr-miR-199a* | 1706 | xtr-mir-199a |
| 683 | dre-miR-199* | 1707 | dre-mir-199-1 |
| | | 1708 | dre-mir-199-2 |
| | | 1709 | dre-mir-199-3 |
| 684 | mmu-miR-200a | 1710 | mmu-mir-200a |
| 685 | mo-miR-200a | 1711 | mo-mir-200a |
| 686 | mdo-miR-200a | 1712 | mdo-mir-200a |
| 687 | gga-miR-200a | 1713 | ggo-mir-200a |

**[Table 3-11]**

| SEQ ID NO: | Name of miRNA | SEQ ID NO: | Name of miRNA precursor |
|---|---|---|---|
| 688 | xtr-miR-200a | 1714 | xtr-mir-200a |
| 689 | bta-miR-200a | 1715 | bta-mir-200a |
| 690 | dre-miR-200a | 1716 | dre-mir-200a |
| 691 | fru-miR-200a | 1717 | fru-mir-200a |
| 692 | tni-miR-200a | 1718 | tni-mir-200a |
| 693 | mmu-miR-204 | 1719 | mmu-mir-204 |
| 694 | mo-miR-204 | 1720 | rno-mir-204 |
| 695 | sla-miR-204 | 1721 | sla-mir-204 |
| 696 | mme-miR-204 | 1722 | nme-mir-204 |
| 697 | ggo-miR-204 | 1723 | ggo-mir-204 |
| 698 | ppa-miR-204 | 1724 | ppa-mir-204 |
| 999 | ptr-miR-204 | 1725 | ptr-mir-204 |
| 700 | ppy-miR-204 | 1726 | ppy-mir-204 |
| 701 | mdo-miR-204 | 1727 | mdo-mir-204-1 |
| 702 | gga-miR-204 | 1728 | gga-mir-204-1 |
| | | 1729 | gga-mir-204-2 |
| 703 | xtr-miR-204 | 1730 | xtr-mir-204-1 |
| | | 1731 | xtr-mir-204-2 |
| 704 | bte-miR-204 | 1732 | bta-mir-204 |
| 705 | ssc-miR-204 | 1733 | ssc-mir-204 |
| 706 | dre-miR-204 | 1734 | dre-mir-204-1 |
| | | 1735 | dre-mir-204-2 |
| | | 1736 | dre-mir-204-3 |
| 707 | fro-miR-204 | 1737 | fru-mir-204 |
| 708 | tri-miR-204 | 1738 | tni-mir-204a |
| 709 | mmu-miR-211 | 1739 | mmu-mir-211 |
| 710 | mo-miR-211 | 1740 | mo-mir-211 |
| 711 | mml-miR-211 | 1741 | mml-mir-211 |
| 712 | mme-miR-211 | 1742 | mne-mir-211 |
| 713 | ppy-miR-211 | 1743 | ppy-mir-211 |
| 714 | gga-miR-211 | 1744 | gga-mir-211 |
| 715 | mmu-miR-205 | 1745 | mmu-mir-205 |
| 716 | mo-miR-205 | 1746 | mo-mir-205 |
| 717 | age-miR-205 | 1747 | age-mir-205 |
| 718 | fla-miR-205 | 1748 | lla-mir-205 |
| 719 | mne-miR-205 | 1719 | mme-mir-205 |
| 720 | ggo-miR-205 | 1750 | ppa-mir-205 |
| 721 | ppa-miR-205 | 1751 | ggo-mir-205 |
| 722 | ptr-miR-205 | 1752 | ptr-mir-205 |
| 723 | gga-miR-205a | 1753 | gga-mir-205 |
| 724 | gga-miR-205b | 1754 | gga-mir-205 |
| 725 | xtr-miR-205a | 1755 | xir-mir-205 |
| 726 | bta-miR-205 | 1756 | bta-mir-205 |
| 727 | ssc-miR-205 | 1757 | ssc-mir-205 |
| 728 | bre-miR-205 | 1758 | dre-mir-205 |
| 729 | fru-miR-205 | 1759 | fru-mir-205 |
| 730 | tni-miR-205 | 1760 | tni-mir-205 |
| 731 | mmu-miR-208 | 1761 | mmu-mir-208 |
| 732 | mo-miR-208 | 1762 | rno-mir-208 |
| 733 | mdo-miR-208 | 1763 | mdo-mir-208 |
| 734 | xtr-miR-208 | 1764 | xtr-mir-208 |
| 735 | mmo-miR-228 | 1765 | mmu-mir-222 |
| 736 | mo-miR-222 | 1766 | mo-mir-222 |
| 737 | age-miR-222 | 1767 | age-mir-222 |
| 738 | gga-miR-222 | 1768 | gga-mir-222 |
| 739 | xtr-miR-222 | 1769 | xtr-mir-222 |
| 740 | bta-miR-222 | 1770 | bta-mir-222 |
| 741 | dre-miR-222 | 1771 | dre-mir-222 |
| 742 | fru-miR-222 | 1772 | fru-mir-222 |
| 743 | tri-miR-222 | 1773 | tni-mir-222 |
| 744 | mmo-miR-302* | 1464 | mmu-mir-302b |
| 745 | gga-miR-302* | 1475 | gga-mir-302b |
| 746 | mmu-miR-324-3p | 1774 | mmu-mir-304 |
| 747 | mo-miR-324-3p | 1775 | mo-mir-304 |
| 748 | mmu-miR-324-5p | 1774 | mmu-mir-304 |
| 749 | mo-miR-324-5p | 1775 | mo-mir-324 |
| 750 | mmu-miR-329 | 1776 | mmu-mir-328 |
| 751 | mo-miR-329 | 1777 | mmu-mir-327 |
| 752 | mo-miR-331 | 1778 | mo-mir-331 |
| 753 | bts-miR-331 | 1779 | bta-mir-331 |
| 754 | bts-miR-331 | 1780 | bta-mir-332 |
| 755 | mmu-miR-346 | 1781 | mmu-mir-346 |
| 756 | mo-miR-346 | 1782 | mo-mir-346 |
| 757 | mmu-miR-376b | 1783 | mmu-mir-376b |
| 758 | mmu-miR-377 | 1784 | mmu-mir-377 |
| 759 | mo-miR-377 | 1785 | mo-mir-377 |
| 760 | bta-miR-3480-5p | 1786 | bta-mir-380 |
| 761 | mmu-miR-382 | 1787 | mmu-mir-382 |
| 762 | rno-miR-382 | 1788 | mo-mir-382 |
| 763 | rno-miR-412 | 1789 | mo-mir-412 |
| 764 | rno-miR-423 | 1790 | mo-mir-423 |
| 765 | mmu-miR-431 | 1791 | mmu-mir-431 |

**[Table 3-12]**

| SEQ ID NO: | Name of mIRA | SEQ ID NO: | Name of mIRNA precursor |
|---|---|---|---|
| 766 | mo-miR-431 | 1792 | mo-mir-431 |
| 767 | gar-miR-431 | 1793 | mo-mir-431 |
| 768 | mmu-miR-433 | 1794 | mmu-mir-433 |
| 769 | mo-miR-433 | 1795 | mo-mir-433 |
| 770 | mmu-miR-449b | 1796 | mmu-mir-449b |
| 771 | mmu-miR-449c | 1797 | mmu-mir-449c |
| 772 | mo-miR-449a | 1798 | mo-mir-449a |
| 773 | cfa-miR-449 | 1799 | cfa-mir-449 |
| 774 | mdo-miR-449 | 1800 | mdo-mir-449 |
| 775 | gga-miR-448 | 1801 | gga-mir-449 |
| 776 | xtr-miR-449 | 1802 | xtr-mir-449 |
| 777 | mmu-miR-451 | 1803 | mmo-mir-451 |
| 778 | mo-miR-451 | 1804 | mo-mir-451 |
| 779 | mdo-miR-451 | 1805 | mdo-mir-451 |
| 780 | gga-miR-451 | 1806 | gga-mir-451 |
| 781 | xtr-miR-451 | 1807 | xtr-mir-451 |
| 782 | dre-miR-451 | 1808 | dre-mir-451 |
| 783 | mmu-miR-452 | 1809 | mmu-mir-452 |
| 784 | mo-miR-455 | 1810 | mo-mir-455 |
| 785 | gga-miR-455 | 1811 | gga-mir-455 |
| 786 | xtr-miR-455 | 1812 | xtr-mir-455 |
| 787 | bta-miR-455 | 1813 | bta-mir-455 |
| 788 | dre-miR-455 | 1814 | dre-mir-455 |
| 789 | fru-miR-455 | 1815 | fru-mir-455 |
| 790 | tni-miR-455 | 1816 | tri-mir-455 |
| 791 | mo-miR-483 | 1817 | mo-mir-483 |
| 792 | gga-miR-489 | 1818 | gga-mir-489 |
| 793 | xtr-miR-489 | 1819 | xtr-mir-489 |
| 794 | dre-miR-489 | 1820 | dre-mir-489 |
| 795 | fru-miR-489 | 1821 | fru-mir-489 |
| 796 | tni-miR-489 | 1822 | tni-mir-489 |
| 797 | mmu-miR-491 | 1823 | mmu-mir-491 |
| 798 | mmu-miR-494 | 1824 | mmu-mir-494 |
| 799 | mo-miR-494 | 1825 | mo-mir-494 |
| 800 | mo-miR-501 | 1826 | mo-mir-501 |
| 801 | mmu-miR-504 | 1827 | mmu-mir-504 |
| 802 | mo-miR-505 | 1828 | mo-mir-505 |
| 803 | hsa-miR-613 | 1829 | hsa-mir-513 |
| 804 | hsa-miR-10b | 1830 | hsa-mir-10b |
| 805 | hsa-miR-17 | 1831 | hsa-mir-17 |
| 806 | hsa-miR-519d | 1832 | hsa-mir-519d |
| 807 | hsa-miR-526b* | 1833 | hsa-mir-526b |
| 808 | mmu-miR-17 | 1834 | mmu-mir-17 |
| 809 | mmu-miR-93 | 1835 | mmu-mir-93 |
| 810 | mo-miR-17 | 1836 | mo-mir-17-1 |
| | | 1837 | mo-mir-17-2 |
| 811 | mo-miR-93 | 1838 | mo-mir-93 |
| 812 | xtr-miR-93a | 1839 | xtr-mir-93a |
| 813 | bta-miR-93 | 1840 | bta-mir-93 |
| 814 | dre-miR-17a | 1841 | dre-mir-17a-1 |
| | | 1842 | dre-mir-17a-2 |
| 815 | dre-miR-93 | 1843 | dre-mir-93 |
| 816 | fru-miR-17 | 1844 | fru-mir-17-1 |
| | | 1845 | fru-mir-17-2 |
| 817 | tni-miR-17 | 1846 | tni-mir-17-1 |
| | | 1847 | tni-mir-17-2 |
| 818 | hsa-miR-92a | 1848 | hsa-mir-92a-1 |
| | | 1849 | hsa-mir-92a-2 |
| 819 | hsa-miR-92b | 1850 | hsa-mir-92b |
| 820 | hsa-miR-363 | 1851 | hsa-mir-363 |
| 821 | hsa-miR-367 | 1852 | hsa-mir-357 |
| 822 | mmu-miR-92a | 1853 | mmu-mir-92a-1 |
| | | 1854 | mmu-mir-92a-2 |
| 823 | mmu-miR-92b | 1855 | mmu-mir-92b |
| 824 | mmu-miR-363 | 1856 | mmu-mir-363 |
| 825 | mmu-miR-367 | 1857 | mmu-mir-367 |
| 826 | mo-miR-92a | 1858 | mo-mir-92a-1 |
| | | 1859 | mo-mir-92a-2 |
| 827 | mo-miR-92b | 1860 | mo-mir-92b |
| 828 | mo-miR-363 | 1861 | mo-mir-363 |
| 829 | age-miR-92 | 1862 | age-mir-92-1 |
| | | 1893 | age-mir-92-2 |
| 830 | lla-miR-92 | 1864 | lla-mir-92-1 |
| | | 1865 | lla-mir-92-2 |
| 831 | sta-miR-92 | 1866 | sta-mir-92-1 |
| | | 1867 | sta-mir-92-2 |
| 832 | mmi-miR-92 | 1868 | mmi-mir-92 |
| 833 | mne-miR-92 | 1869 | mme-mir-92 |
| 834 | gga-miR-92 | 1870 | gga-mir-92-1 |
| | | 1871 | gga-mir-92-2 |
| 835 | ppa-miR-92 | 1872 | ppa-mir-92-1 |
| | | 1873 | ppa-mir-92-2 |

**[Table 3-13]**

| SEQ ID NO: | Name of mIRNA | SEQ ID NO: | Name of mIRNA precursor |
|---|---|---|---|
| 836 | ptr-miR-92 | 1874 | ptr-mir-92-1 |
| | | 1875 | ptr-mir-92-2 |
| 837 | ppy-miR-92 | 1876 | ppy-mir-92-1 |
| | | 1877 | ppy-mir-92-2 |
| 838 | Ica-miR-92 | 1878 | lca-mir-92-1 |
| | | 1879 | lca-mir-92-2 |
| 839 | mdo-miR-92 | 1880 | mdo-mir-92 |
| 840 | gga-miR-92 | 1881 | gga-mir-92 |
| 841 | gga-miR-357 | 1882 | gga-mir-357 |
| 842 | xtr-miR-92a | 1883 | xtr-mir-92a-1 |
| | | 1884 | xtr-mir-92a-2 |
| 843 | xtr-miR-92b | 1885 | xtr-mir-92b |
| 844 | xtr-miR-363-3p | 1886 | xtr-mir-363 |
| 845 | xtr-miR-367 | 1887 | xtr-mir-357 |
| 846 | bta-miR-92 | 1888 | bta-mir-92 |
| 847 | dre-miR-92a | 1889 | dre-mir-92a-1 |
| | | 1890 | dre-mir-92a-2 |
| 848 | dre-miR-92b | 1891 | dre-mir-92b |
| 849 | dre-miR-363 | 1892 | dre-mir-383 |
| 850 | fru-miR-92 | 1893 | fru-mir-92-1 |
| | | 1894 | fru-mir-92-2 |
| 851 | tni-miR-92 | 1895 | tni-mir-92-1 |
| | | 1896 | tni-mir-92-2 |
| 852 | hsa-miR-27a | 1897 | hsa-mir-27a |
| 853 | hsa-miR-708 | 1898 | hsa-mir-708 |
| 854 | mmu-miR-708 | 1899 | mmu-mir-708 |
| 855 | mo-miR-708 | 1900 | mo-mir-708 |
| 856 | hsa-miR-29b | 1901 | hsa-mir-29b-1 |
| | | 1902 | hsa-mir-29b-2 |
| 857 | hsa-miR-30a | 1903 | hsa-mir-30a |
| 858 | hsa-miR-30b | 1904 | hsa-mir-30d |
| 859 | hsa-miR-30c | 1905 | hsa-mir-30e |
| 860 | mmu-miR-384-5p | 1906 | mmu-mir-384 |
| 861 | mmu-miR-384-5p | 1907 | mo-mir-384 |
| 862 | hsa-miR-302a | 1010 | hsa-mir-302a |
| 863 | hsa-miR-302b | 1011 | hsa-mir-302b |
| 864 | hsa-miR-302c | 1908 | hsa-mir-302c |
| 865 | hsa-miR-520a-3p | 1909 | hsa-mir-520a |
| 866 | hsa-miR-520b | 1910 | hsa-mir-520b |
| 867 | hsa-miR-520c-3p | 1911 | hsa-mir-520c |
| 868 | hsa-miR-520d-3p | 1912 | hsa-mir-520d |
| 869 | hsa-miR-520e | 1913 | hsa-mir-520e |
| 870 | mmu-miR-291a-3p | 1914 | mmu-mir-291a |
| 871 | mmu-miR-294 | 1915 | mmu-mir-294 |
| 872 | mmu-miR-295 | 1916 | mmu-mir-295 |
| 873 | mo-miR-291a-3p | 1917 | mo-mir-291a |
| 874 | xia-miR-427 | 1918 | xia-mir-427 |
| 875 | xia-miR-428 | 1919 | xia-mir-428 |
| 876 | xtr-miR-428 | 1920 | xtr-mir-428 |

**[Table 3-14]**

| SEQ ID NO: | Name of mIRNA | SEQ ID NO: | Name of mIRNAprecursor |
|---|---|---|---|
| | | 1921 | dre-mir-430a-1 |
| | | 1922 | dre-mir-430a-2 |
| | | 1924 | dre-mir-430a-3 |
| | | 1923 | dre-mir-430a-4 |
| | | 1925 | dre-mir-430a-5 |
| | | 1926 | dre-mir-430a-6 |
| | | 1927 | dre-mir-430a-7 |
| | | 1928 | dre-mir-430a-8 |
| | | 1929 | dre-mir-430a-9 |
| | | 1930 | dre-mir-430a-10 |
| | | 1931 | dre-mir-430a-11 |
| 877 | dre-miR-430a | 1932 | dre-mir-430a-12 |
| | | 1933 | dre-mir-430a-13 |
| | | 1934 | dre-mir-430a-14 |
| | | 1935 | dre-mir-430a-15 |
| | | 1936 | dre-mir-430a-16 |
| | | 1937 | dre-mir-430a-17 |
| | | 1938 | dre-mir-430a-18 |
| | | 1939 | dre-mir-430a-19 |
| | | 1940 | dre-mir-430a-20 |
| | | 1941 | dre-mir-430a-21 |
| | | 1942 | dre-mir-430a-22 |
| | | 1943 | dre-mir-430a-23 |
| | | 1944 | dre-mir-430b-1 |
| | | 1945 | dre-mir-430b-2 |
| | | 1946 | dre-mir-430b-3 |
| | | 1947 | dre-mir-430b-4 |
| | | 1948 | dre-mir-430b-5 |
| | | 1949 | dre-mir-430b-6 |
| | | 1950 | dre-mir-430b-7 |
| | | 1951 | dre-mir-430b-8 |
| | | 1952 | dre-mir-430b-9 |
| | | 1953 | dre-mir-430b-10 |
| | | 1954 | dre-mir-430b-11 |
| 878 | dre-miR-430b | 1955 | dre-mir-430b-12 |
| | | 1956 | dre-mir-430b-13 |
| | | 1957 | dre-mir-430b-14 |
| | | 1958 | dre-mir-430b-15 |
| | | 1959 | dre-mir-430b-16 |
| | | 1960 | dre-mir-430b-17 |
| | | 1961 | dre-mir-430b-18 |
| | | 1962 | dre-mir-430b-19 |
| | | 1963 | dre-mir-430b-20 |
| | | 1964 | dre-mir-430b-21 |
| | | 1965 | dre-mir-430b-22 |
| | | 1966 | dre-mir-430b-23 |
| | | 1967 | dre-mir-430c-1 |
| | | 1968 | dre-mir-430c-2 |
| | | 1969 | dre-mir-430c-3 |
| | | 1970 | dre-mir-430c-4 |
| | | 1971 | dre-mir-430c-5 |
| | | 1972 | dre-mir-430c-6 |
| | | 1973 | dre-mir-430c-7 |
| | | 1974 | dre-mir-430c-8 |
| | | 1975 | dre-mir-430c-9 |
| | | 1976 | dre-mir-430c-10 |
| 879 | dre-miR-430c | 1977 | dre-mir-430c-11 |
| | | 1978 | dre-mir-430c-12 |
| | | 1979 | dre-mir-430c-13 |
| | | 1980 | dre-mir-430c-14 |
| | | 1981 | dre-mir-430c-15 |
| | | 1982 | dre-mir-430c-16 |
| | | 1983 | dre-mir-430c-17 |
| | | 1984 | dre-mir-430c-18 |
| | | 1985 | dre-mir-430c-19 |
| | | 1986 | dre-mir-430c-20 |
| | | 1987 | dre-mir-430c-21 |
| | | 1988 | dre-mir-430i-1 |
| 880 | dre-miR-430i | 1989 | dre-mir-430i-2 |
| | | 1990 | dre-mir-430i-3 |
| 881 | dre-miR-430j | 1991 | dre-mir-430j |
| 882 | hsa-miR-301b | 1992 | hsa-mir-301b |
| 883 | hsa-miR-454 | 1993 | hsa-mir-454 |
| 884 | mmu-miR-721 | 1994 | mmu-mir-721 |
| 885 | dre-miR-454a | 1995 | dre-mir-454a |
| 886 | dre-miR-454b | 1996 | dre-mir-454b |
| 887 | hsa-miR-148b-5p | 1997 | hsa-mir-148b |
| 888 | mmu-miR-322 | 1998 | mmu-mir-322 |
| 889 | mo-miR-322 | 1999 | mo-mir-332 |
| 890 | dre-miR-457a | 2000 | mo-mir-457a |
| 891 | dre-miR-457b | 2001 | mo-mir-457b |
| 892 | hsa-miR-141 | 2002 | hsa-mir-141 |

**[Table 3-15]**

| SEQ ID NO: | Name of mIRNA | SEQ ID NO: | Name of mIRMA precursor |
|---|---|---|---|
| 893 | mmu-miR-141 | 2003 | mmu-mir-141 |
| 894 | mo-miR-141 | 2004 | mo-mir-141 |
| 895 | mdo-miR-141 | 2005 | mdo-mir-141 |
| 896 | dre-miR-141 | 2006 | dre-mir-141 |
| 897 | hsa-miR-208b | 2007 | hsa-mir-208b |
| 898 | dre-miR-736 | 2008 | dre-mir-736 |
| 899 | hsa-miR-221 | 2009 | hsa-mir-221 |
| 900 | mmu-miR-221 | 2010 | mmu-mir-221 |
| 901 | mo-miR-221 | 2011 | mo-mir-221 |
| 902 | mml-miR-Z21 | 2012 | mml-mir-221 |
| 903 | gga-miR-221 | 2013 | ggo-mir-221 |
| 904 | ppa-miR-221 | 2014 | ppa-mir-221 |
| 905 | ppy-miR-221 | 2015 | ppy-mir-221 |
| 906 | mdo-miR-221 | 2016 | mdo-mir-221 |
| 907 | gga-miR-221 | 2017 | gga-mir-221 |
| 908 | xtr-miR-221 | 2018 | xtr-mir-221 |
| 909 | bta-miR-221 | 2019 | bta-mir-221 |
| 910 | dre-miR-221 | 2020 | dre-mir-221 |
| 911 | fru-miR-221 | 2021 | fru-mir-221 |
| 912 | tni-miR-221 | 2022 | tni-mir-221 |
| 913 | hsa-miR-302d* | 967 | hsa-mir-302d |
| 914 | hsa-miR-362-3p | 1020 | hsa-mir-362 |
| 915 | mmu-miR-362-3p | 2023 | mmu-mir-362 |
| 916 | hsa-miR-563 | 2024 | has-mir-563 |
| 917 | hsa-miR-449b | 2025 | has-mir-449b |
| 918 | mmu-miR-699 | 2026 | mmu-mir-699 |
| | | 2027 | hsa-mir-124-1 |
| 919 | hsa-miR-124 | 2028 | hsa-mir-124-2 |
| | | 2029 | hsa-mir-124-3 |
| | | 2030 | mmu-mir-124-1 |
| 920 | mmu-miR-124 | 2031 | mmu-mit-124-2 |
| | | 2032 | mmu-mir-124-3 |
| | | 2033 | mo-mir-124-1 |
| 921 | mo-miR-124 | 2034 | mo-mir134-2 |
| | | 2035 | mo-mir-124-3 |
| | | 2036 | dre-mir-124-1 |
| | | 2037 | dre-mir-124-2 |
| 922 | dre-miR-124 | 2038 | dre-mir-124-3 |
| | | 2039 | dre-mir-124-4 |
| | | 2040 | dre-mir-124-5 |
| | | 2041 | dre-mir-124-6 |
| | | 2042 | fru-mir-124-1 |
| 923 | fru-miR-124 | 2043 | fru-mir-124-2 |
| | | 2044 | fru-mir-124-3 |
| | | 2045 | tni-mir-124-1 |
| 924 | tni-miR-124 | 2046 | tni-mir-124-2 |
| | | 2047 | tni-mirv124-3 |
| 925 | hsa-miR-557 | 2048 | hsa-mir-557 |
| 926 | hsa-miR-518a-3p | 2049 | hsa-mir-518a-1 |
| | | 2050 | hsa-mir-518a-2 |
| 927 | hsa-miR-520g | 2051 | hsa-mir-520g |
| 928 | hsa-miR-520d-5p | 1912 | hsa-mir-520d |
| 929 | hsa-miR-520a-5p | 1909 | hsa-mir-520a |
| 930 | hsa-miR-518d-5p | 1069 | hsa-mir-518d |
| 931 | hsa-miR-518e* | 1070 | hsa-mir-518e |
| 932 | hsa-miR-518f* | 1071 | hse-mir-518f |
| 933 | hsa-miR-519a* | 2052 | hsa-mir-519a-1 |
| | | 2053 | hsa-mir-518a-2 |
| 934 | hsa-miR-519b-5p | 2054 | hsa-mir-519b |
| 935 | hsa-miR-519c-5p | 2055 | hsa-mir-519c |
| 936 | hsa-miR-520c-5p | 1911 | hsa-mir-520c |
| 937 | hsa-miR-522* | 2056 | has-mir-522 |
| 938 | hsa-miR-523* | 1073 | hse-mir-523 |
| 939 | hsa-miR-518a-5p | 2049 | hsa-mir-518a-1 |
| | | 2050 | hsa-mir-519a-2 |
| 2057 | hsa-miR-411 | 2133 | hsa-mir-411 |
| 2058 | hsa-miR-421 | 2134 | hsa-mir-421 |
| 2059 | hsa-miR-425-5p | 2135 | hsa-mir-425 |
| 2060 | hsa-miR-454-5p | 2136 | hsa-mir-454 |
| 2061 | hsa-miR-493-3p | 2137 | hsa-mir-483 |
| 2062 | hsa-miR-532 | 2138 | hsa-mir-532 |
| 2063 | hsa-miR-542-3p | 2139 | hsa-mir-542 |
| 2064 | hsa-miR-545 | 2140 | hsa-mir-545 |
| 2065 | hsa-miR-548a | 2141 | hsa-mir-548a-1 |
| | | 2142 | hsa-mir-548a-2 |
| | | 2143 | hsa-mir-548a-3 |
| 2066 | hsa-miR-548b | 2144 | hsa-mir-548b |
| 2067 | hsa-miR-548d | 2145 | hsa-mir-548d-1 |
| | | 2146 | hsa-mir-548d-2 |
| 2068 | hsa-miR-550 | 2147 | hsa-mir-550-1 |
| | | 2148 | hsa-mir-550-2 |
| 2069 | hsa-miR-551b | 2149 | hsa-mir-551b |

**[Table 3-16]**

| SEQ ID NO: | Name of mIRNA | SEQ ID NO: | Name of mIRNA precursor |
|---|---|---|---|
| 2070 | hsa-miR-552 | 2150 | hsa-mir-552 |
| 2071 | hsa-miR-553 | 2151 | hsa-mir-553 |
| 2072 | hsa-miR-558 | 2152 | hsa-mir-558 |
| 2073 | hsa-miR-565 | 2153 | hsa-mir-561 |
| 2074 | hsa-miR-561 | 2154 | hsa-mir-565 |
| 2075 | hsa-miR-570 | 2155 | hsa-mir-570 |
| 2076 | hsa-miR-571 | 2156 | hsa-mir-571 |
| 2077 | hsa-miR-579 | 2157 | hsa-mir-578 |
| 2078 | hsa-miR-564 | 2158 | hsa-mir-584 |
| 2079 | hsa-miR-589 | 2159 | hsa-mir-589 |
| 2080 | hsa-miR-591 | 2160 | hsa-mir-591 |
| 2081 | hsa-miR-593 | 2161 | hsa-mir-593 |
| 2082 | hsa-miR-595 | 2162 | hsa-mir-595 |
| 2083 | hsa-miR-599 | 2163 | hsa-mir-599 |
| 2084 | hsa-miR-600 | 2164 | hsa-mir-600 |
| 2085 | hsa-miR-601 | 2165 | hsa-mir-601 |
| 2086 | hsa-miR-604 | 2166 | hsa-mir-604 |
| 2087 | hsa-miR-605 | 2167 | hsa-mir-605 |
| 2088 | hsa-miR-605 | 2168 | hsa-mir-606 |
| 2089 | hsa-miR-609 | 2169 | hsa-mir-609 |
| 2090 | hsa-miR-610 | 2170 | hsa-mir-610 |
| 2091 | hsa-miR-611 | 2171 | hsa-mir-611 |
| 2092 | hsa-miR-614 | 2172 | hsa-mir-614 |
| 2093 | hsa-miR-615 | 2173 | hsa-mir-615 |
| 2094 | hsa-miR-616 | 2174 | hsa-mir-616 |
| 2095 | hsa-miR-617 | 2175 | hsa-mir-617 |
| 2096 | hsa-miR-618 | 2176 | hsa-mir-618 |
| 2097 | hsa-miR-621 | 2177 | hsa-mir-621 |
| 2098 | hsa-miR-624 | 2178 | hsa-mir-624 |
| 2099 | hsa-miR-625 | 2179 | hsa-mir-625 |
| 2100 | hsa-miR-627 | 2180 | hsa-mir-627 |
| 2101 | hsa-miR-630 | 2181 | hsa-mir-630 |
| 2102 | hsa-miR-631 | 2182 | hsa-mir-631 |
| 2103 | hsa-miR-634 | 2183 | hsa-mir-634 |
| 2104 | hsa-miR-637 | 2184 | hsa-mir-637 |
| 2105 | hsa-miR-646 | 2185 | hsa-mir-646 |
| 2106 | hsa-miR-647 | 2186 | hsa-mir-647 |
| 2107 | hsa-miR-548 | 2187 | hsa-mir-648 |
| 2108 | hsa-miR-650 | 2188 | hsa-mir-650 |
| 2109 | hsa-miR-651 | 2189 | hsa-mir-651 |
| 2110 | hsa-miR-653 | 2190 | hsa-mir-653 |
| 2111 | hsa-miR-654 | 2191 | hsa-mir-654 |
| 2112 | hsa-miR-657 | 2192 | hsa-mir-657 |
| 2113 | hsa-miR-661 | 2193 | hsa-mir-661 |
| 2114 | hsa-miR-769-5p | 2194 | hsa-mir-769 |
| 2115 | mmu-miR-411 | 2195 | mmu-mir-411 |
| 2116 | mo-miR-411 | 2196 | mo-mir-411 |
| 2117 | mmu-miR-421 | 2197 | mmu-mir-421 |
| 2118 | xtr-miR-425-5p | 2198 | xtr-mir-425 |
| 2119 | bta-miR-532 | 2199 | bta-mir-532 |
| 2120 | mmu-miR-542-3p | 2200 | mmu-mir-542 |
| 2121 | mo-miR-542-3p | 2201 | mo-mir-542 |
| 2122 | mmu-miR-551b | 2202 | mmu-mir-551b |
| 2123 | mmu-miR-489 | 2203 | mmu-mir-489 |
| 2124 | dre-miR-731 | 2204 | dre-mir-731 |
| 2125 | hsa-miR-493 | 2137 | hsa-mir-493 |
| 2126 | hsa-miR-200c* | 2205 | hsa-mir-200c |
| 2127 | mmu-miR-200c* | 2206 | mmu-mir-200c |
| 2128 | hsa-miR-551a | 2207 | hsa-mir-551a |
| 2129 | mmu-miR-592 | 2208 | mmu-mir-592 |
| 2130 | hsa-miR-373* | 2209 | hsa-mir-373 |
| 2131 | mmu-miR-294* | 2210 | mmu-mir-294 |
| 2132 | hsa-miR-541 | 2211 | hsa-mir-541 |
| 2212 | xtr-miR-1a | 2262 | xtr-mir-1a-1 |
| | | 2263 | xtr-mir-1a-2 |
| 2213 | mmu-miR-7a | 2264 | mmu-mir-7a-1 |
| | | 2255 | mmu-mir-7a-2 |
| 2214 | mmu-miR-10b | 2266 | mmu-mir-10b |
| 2215 | mo-miR-10a-5p | 2267 | mo-mir-10a |
| 2216 | xla-miR-18 | 2268 | xla-mir-18 |
| 2217 | mo-miR-20b-5p | 2269 | mo-mir-20b |
| 2218 | xta-miR-20 | 2270 | xta-mir-20 |
| 2219 | xtr-miR-24a | 2271 | xtr-mir-24a |
| 2220 | xtr-miR-26 | 2272 | xtr-mir-26-1 |
| | | 2273 | xtr-mir-26-2 |
| 2221 | mmu-miR-27a | 2274 | mmu-mir-27a |
| 2222 | mmu-miR-29b | 2275 | mmu-mir-29b-1 |
| | | 2276 | mmu-mir-29b-2 |
| 2223 | mmu-miR-30a | 2277 | mmu-mir-30a |
| 2224 | mo-miR-30b-5p | 2278 | mmu-mir-30b |
| 2225 | mmu-miR-302a | 2279 | mmu-mir-302a |
| 2226 | xtr-miR-130c | 2280 | xtr-mir-130c |

**[Table 3-17]**

| SEQ ID NO: | Name of mRNA | SEQID NO: | Name of mIRNA precursor |
|---|---|---|---|
| 2227 | xtr-miR-133c | 2281 | xtr-miR-133c |
| 2228 | mdo-miR-142 | 2282 | mdo-mir-142 |
| 2229 | dre-miR-142a-3p | 2283 | dre-mir-142a |
| 2230 | mmu-miR-146b | 2284 | mmu-mir-146b |
| 2231 | fru-miR-182 | 2285 | fru-mir-182 |
| 2232 | tni-miR-182 | 2286 | tni-mir-182 |
| 2233 | mmu-miR-183 | 2287 | mmu-mir-193 |
| 2234 | mo-miR-186c | 2288 | mo-mir-195c |
| 2235 | gga-miR-199* | 2289 | gga-mir-199-1 |
| | | 2290 | gga-mir-199-2 |
| 2236 | mmu-miR-202-3p | 2291 | mmu-mir-202 |
| 2237 | tni-miR-204b | 2292 | tri-mir-204 |
| 2238 | xtr-miR-205b | 2293 | xtr-mir-205b |
| 2239 | mmu-miR-208b | 2294 | mmu-mir-208b |
| 2240 | mdo-miR-222a | 2295 | mdo-mir-222a |
| 2241 | mmu-miR-299 | 2296 | mmu-mir-299 |
| 2242 | mmu-miR-299* | 2296 | mmu-mir-299 |
| 2243 | mo-miR-299 | 2287 | mmo-mir-299 |
| 2244 | mmu-miR-302c* | 2298 | mmu-mir-302c |
| 2245 | mmu-miR-331-3p | 2299 | mmu-mir-331 |
| 2246 | mmu-miR-340-3p | 2300 | mmu-mir-340 |
| 2247 | mo-miR-340-3p | 2301 | mo-mir-340 |
| 2248 | mmu-miR-342-3p | 2302 | mmu-mir-342 |
| 2248 | mo-miR-342-3p | 2303 | mo-mir-342 |
| 2250 | mmu-miR-362-5p | 2023 | mmu-mir-362 |
| 2251 | mo-miR-376b-3p | 2304 | mmu-mir-362 |
| 2252 | mmu-miR-378* | 2305 | mmu-mir-378 |
| 2253 | mo-miR-378* | 2306 | mo-mir-378 |
| 2254 | mmu-miR-423-3p | 2307 | mmu-mir-423 |
| 2255 | mmu-miR-449a | 2308 | mmu-mir-449a |
| 2256 | mmu-miR-455* | 2309 | mmu-mir-455 |
| 2257 | mmu-miR-483* | 2310 | mmu-mir-483 |
| 2258 | mmu-miR-485 | 2311 | mmu-mir-485 |
| 2259 | mo-miR-485 | 2312 | mmu-mir-488 |
| 2260 | mmu-miR-488* | 2313 | mo-mir-485 |
| 2261 | mmu-miR-501-5p | 2314 | mmu-mir-501 |
| 2315 | mmu-miR-425 | 2322 | mmu-mir-425 |
| 2316 | mo-miR-425 | 2323 | mo-mir-425 |
| 2317 | mmu-miR-493 | 2324 | mmu-mir-493 |
| 2318 | mo-miR-493 | 2325 | mo-mir-493 |
| 2319 | mmu-miR-532-5p | 2326 | mmu-mir-532 |
| 2320 | mo-miR-532-5p | 2327 | mo-mir-532 |
| 2321 | mmu-miR-615-3p | 2328 | mmu-mir-615 |

In the present invention, a nucleic acid having an identity of 90% or more to a nucleotide sequence shown by any one of SEQ ID NO:1-2328 means a nucleic acid having an identity of at least 90% or more, preferably 93% or more, more preferably 95% or more, still more preferably 96% or more, particularly preferably 97% or more, most preferably 98% or more, to a nucleic acid consisting of a nucleotide sequence shown by any one of SEQ ID NO:1-2328, as calculated using an analytical software program such as BLAST [J. Mol. Biol., 215, 403 (1990)] or FASTA [Methods in Enzymology, 183, 63 (1990)].

The stringent conditions in the above refer to those under which signals can be detected by adding a membrane having one chain blotted and the other chain labeled with ³²P-ATP to a Hybridization buffer consisting of 7.5 mL, 0.6 mL of 1 M Na₂HPO₄ (pH 7.2), 21 mL of 10% SDS, 0.6 mL of 50xDenhardt's solution, and 0.3 mL of 10 mg/mL sonicated salmon sperm DNA, carrying out a reaction at 50°C overnight, thereafter washing the membrane with 5xSSC/5% SDS liquid at 50°C for 10 minutes, and further washing the same with 1xSSC/1% SDS solution at 50°C for 10 minutes, thereafter taking out the membrane, and exposing it to an X-ray film.

The method of detecting the expression of nucleic acid such as a miRNA using a nucleic acid may be any method, as far as the presence of nucleic acid such as a miRNA or miRNA precursor in a sample can be detected; for example, (1) Northern hybridization, (2) dot blot hybridization, (3) in situ hybridization, (4) quantitative PCR, (5) differential hybridization, (6) microarray, (7) ribonuclease protection assay and the like can be mentioned.

The method of detecting a mutation of nucleic acid such as a miRNA using a nucleic acid may be any method, as far as it enables detection of a mutation of the nucleotide sequence of nucleic acid such as a miRNA or miRNA precursor in a sample; for example, a method wherein a hetero-double-strand formed by hybridization of a nucleic acid having a non-mutated nucleotide sequence and a nucleic acid having a mutated nucleotide sequence are detected, or a method wherein a sample-derived nucleotide sequence is directly sequenced to detect the presence or absence of a mutation and the like can be mentioned.

A vector that expresses a nucleic acid may be any vector, as far as it is designed to biosynthesize a nucleic acid such as miRNA when introduced to, and transcribed in, a cell. Specifically, as examples of vectors capable of expressing a nucleic acid such as miRNA in a cell, pcDNA6.2-GW/miR (manufactured by Invitrogen), pSilencer 4.1-CMV (manufactured by Ambion), pSINsi-hH1 DNA (manufactured by Takara Bio Inc.), pSINsi-hU6 DNA (manufactured by Takara Bio Inc.), pENTR/U6 (manufactured by Invitrogen) and the like can be mentioned.

The method of suppressing the expression of a gene having a target nucleotide sequence of nucleic acid such as miRNA (hereinafter to be referred to as target gene) may be any method, as far as it suppresses the expression of a gene having a target nucleotide sequence by means of the activity to suppress the expression of a mRNA having the target nucleotide sequence of nucleic acid such as miRNA. Here, to suppress the expression encompasses a case where the translation of a mRNA is suppressed, and a case where cleavage or degradation of a mRNA results in a decreased amount of protein translated from the mRNA. Specifically, as substances that suppress the expression of a mRNA having the target nucleotide sequence, nucleic acids such as an siRNA and an antisense oligonucleotides can be mentioned. The siRNA can be prepared on the basis of information on the continuous sequence of the mRNA [Genes Dev., 13, 3191 (1999)]. The number of nucleotide residues constituting one strand of the siRNA is preferably 17 to 30 residues, more preferably 18 to 25 residues, still more preferably 19 to 23 residues.

MiRNAs include artificial miRNAs that are single-stranded RNAs 17 to 28 nucleotides long comprising as the 2nd to 8th nucleotides a sequence that is complementary to a consecutive, 7-nucleotide sequence present in the 3'-untranslational region of the mRNA of the target gene. In the case of an RNA comprising a sequence extended anteriorly and posteriorly that forms a hairpin structure, wherein the miRNA sequence can be cut out in cells from either one strand of the hairpin structure by the biosynthetic pathway of the miRNA, the extended sequence is called an artificial miRNA precursor. With a gene whose expression is to be suppressed as the target gene, an artificial miRNA and artificial miRNA precursor can be designed using the method described above.

A target nucleotide sequence of a nucleic acid such as miRNA refers to a nucleotide sequence consisting of several nucleotides recognized by a nucleic acid such as miRNA and the like of the present invention, wherein the expression of a mRNA having the nucleotide sequence is suppressed by a nucleic acid such as miRNA of the present invention. Because a mRNA having a nucleotide sequence complementary to the 2nd to 8th nucleotides on the 5' terminal side of a miRNA undergoes suppression of the translation thereof by the miRNA [Current Biology, 15, R458-R460 (2005)], a nucleotide sequence complementary to the nucleotide sequence of the 2nd to 8th nucleotides on the 5' terminal side of a nucleic acid such as miRNA of the present invention can be mentioned as a target nucleotide sequence. For example, by providing a target sequence complementary to the 2nd to 8th nucleotides on the 5' terminal side of a miRNA, and selecting a mRNA comprising a sequence completely identical to a set of 3' UTR nucleotide sequences of human mRNAs by a method such as character string search, the target nucleotide sequence can be determined. A set of 3' UTR nucleotide sequences of human mRNAs can be prepared using information on genomic sequences and gene positions that can be acquired from "UCSC Human Genome Browser Gateway (http://genome.ucsc.edu/cgi-bin/hgGateway)". As specific examples of genes having a target nucleotide sequence of miRNA consisting of the nucleotide sequence shown by any of SEQ ID NOs: 1-939, 2057-2132, 2212-2261 and 2315-2321, the genes shown in Table 4 (Table 4-1 to Table 4-157), represented by names (Official Symbols and Gene IDs) used in the EntreGene database (http://www.ncbi.nlm.nih.gov/Entrez/) of US National Center for Biotechnology Information (NCBI), can be mentioned.

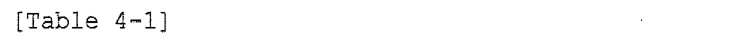

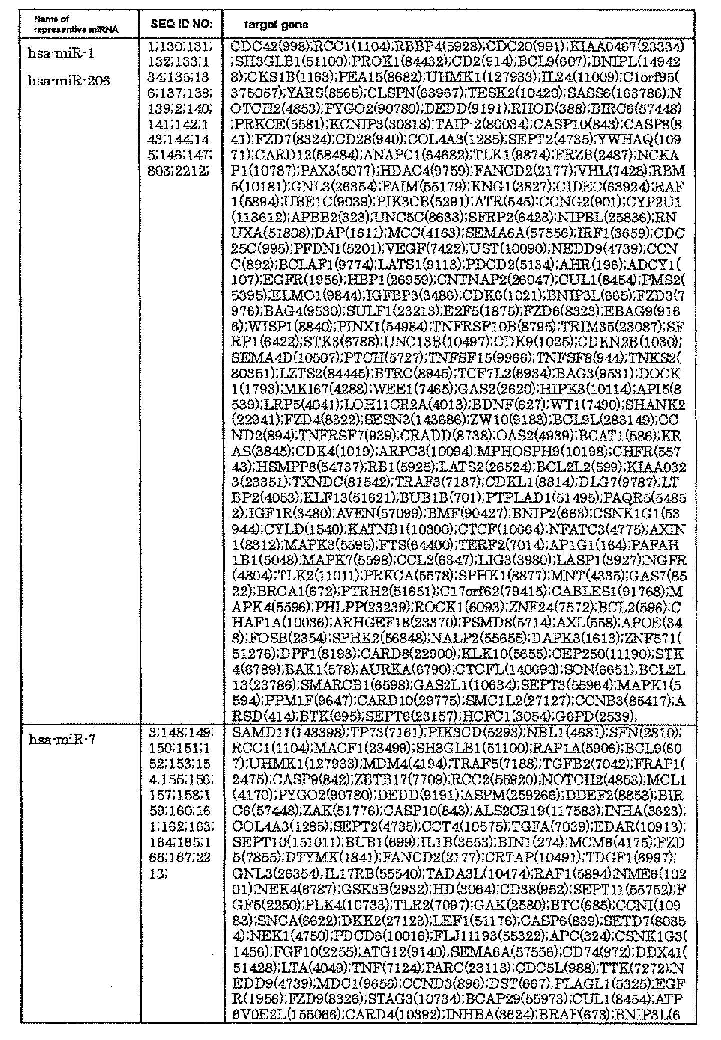

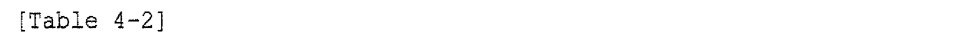

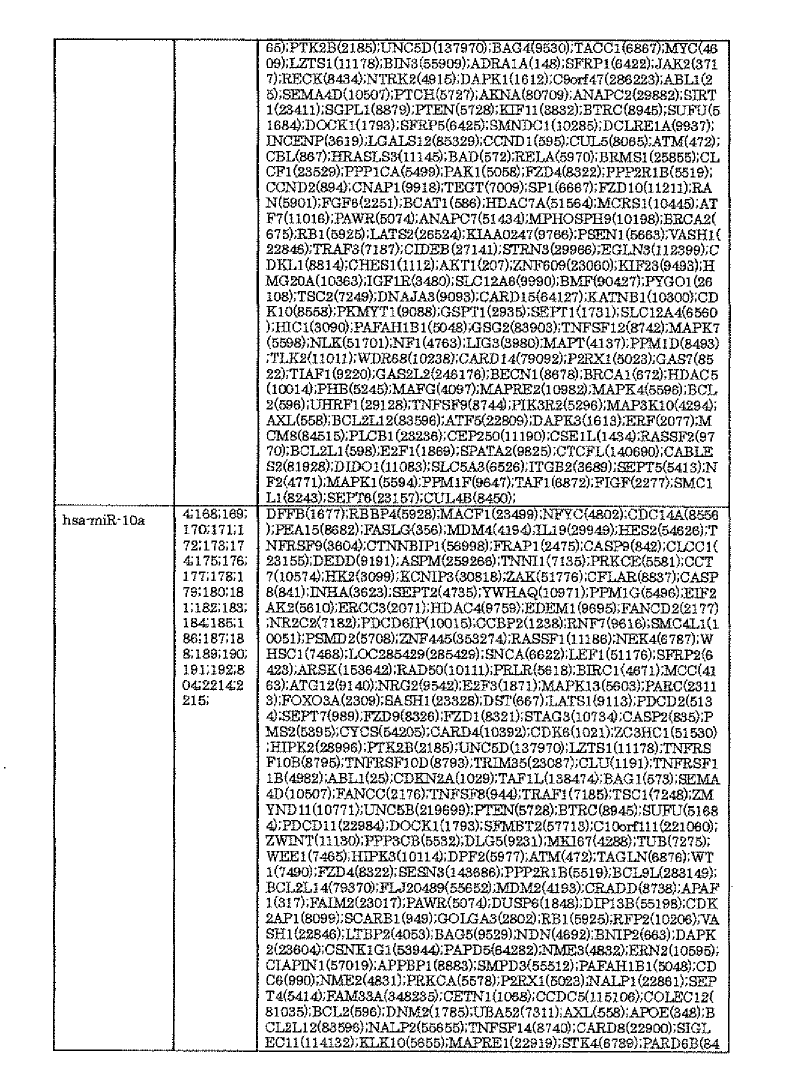

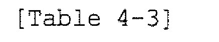

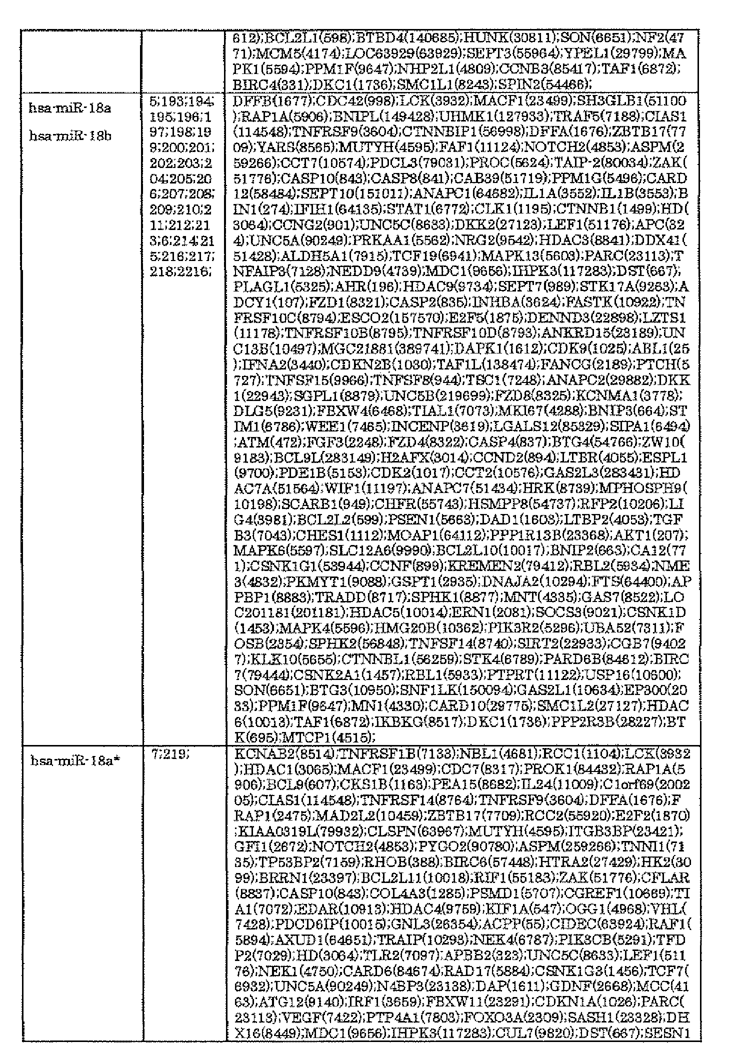

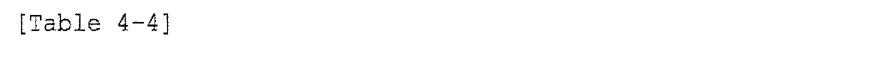

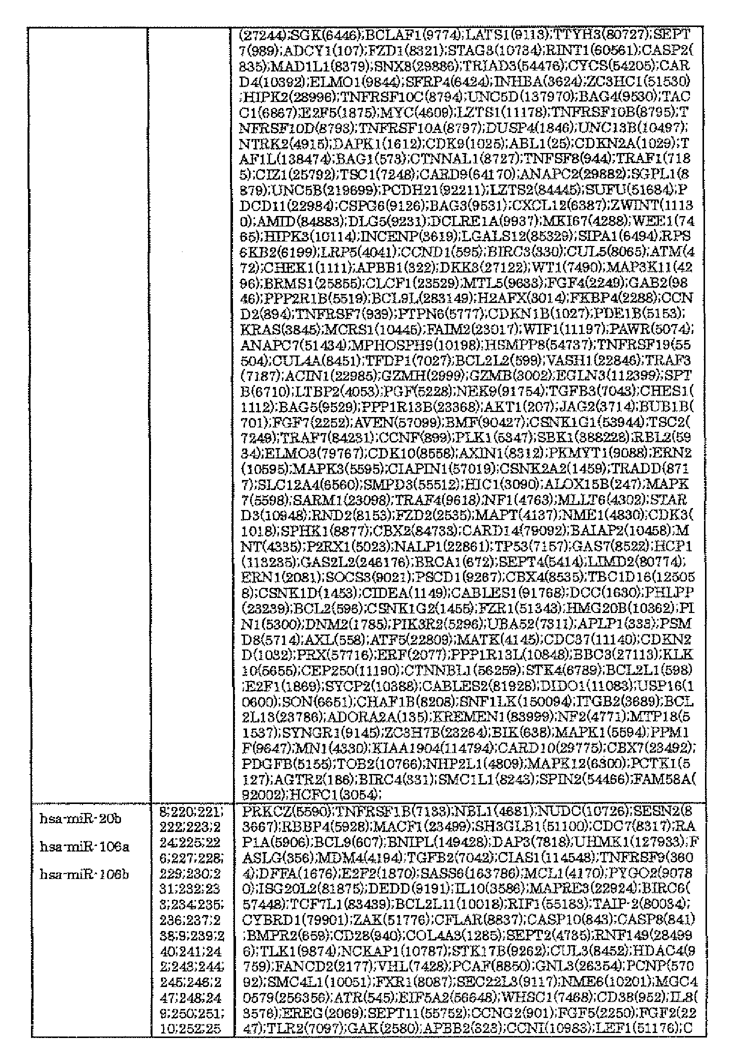

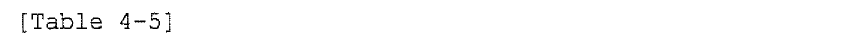

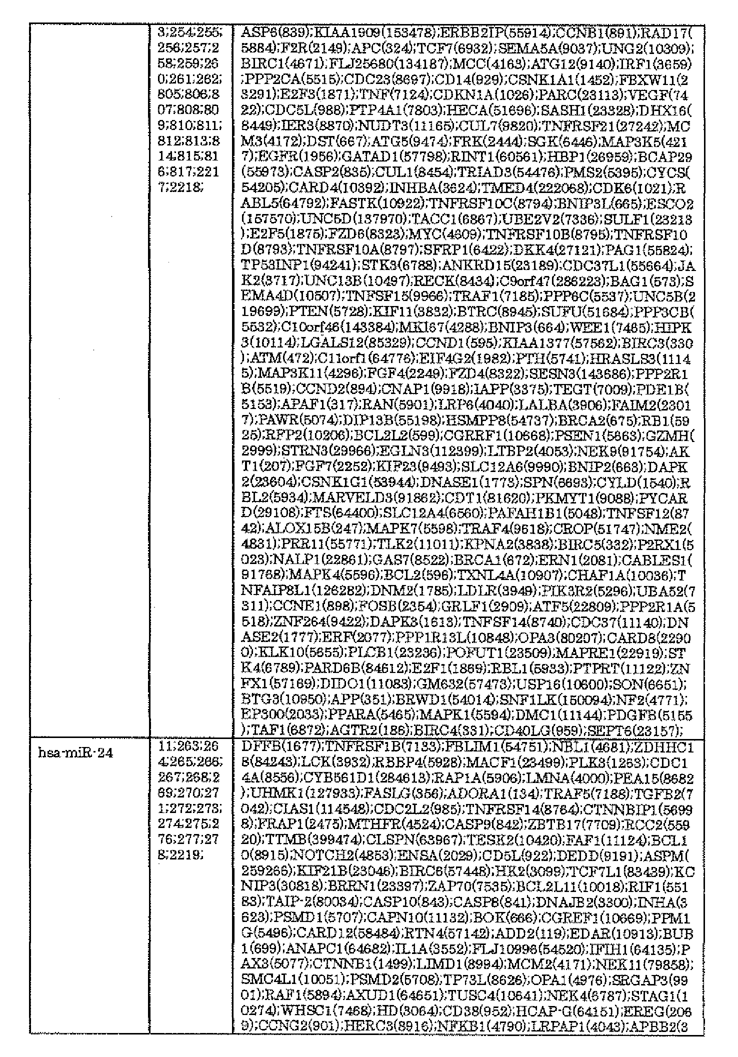

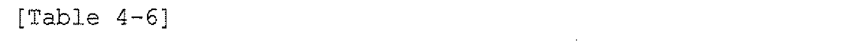

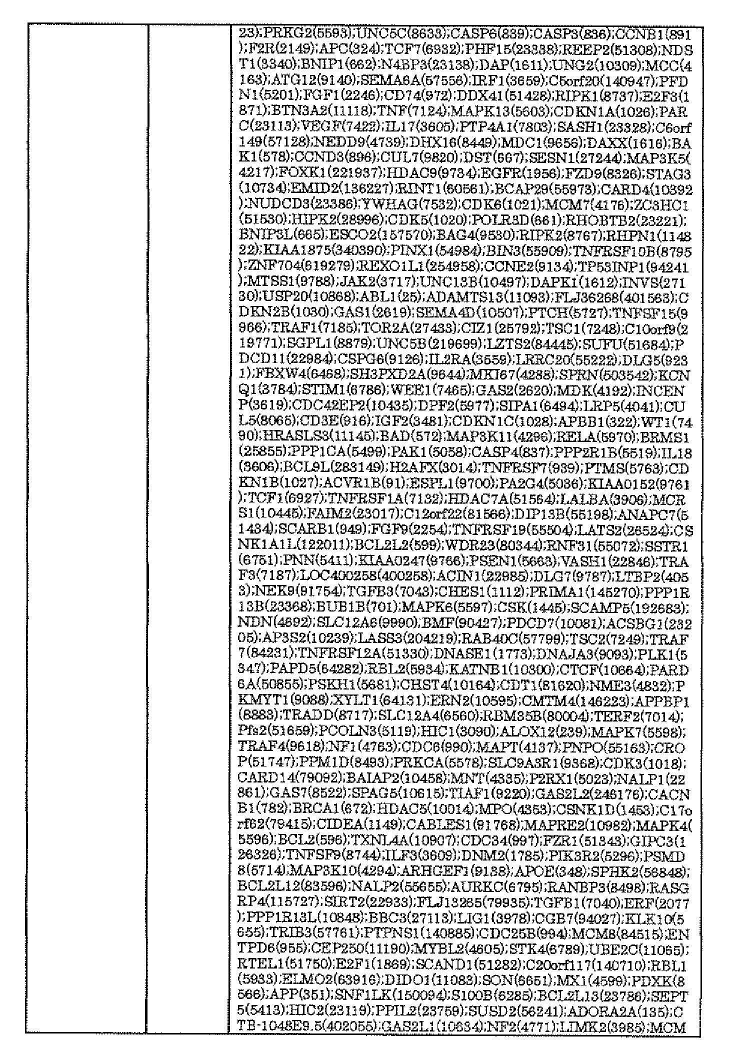

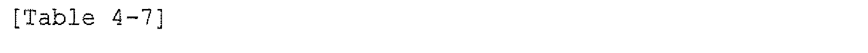

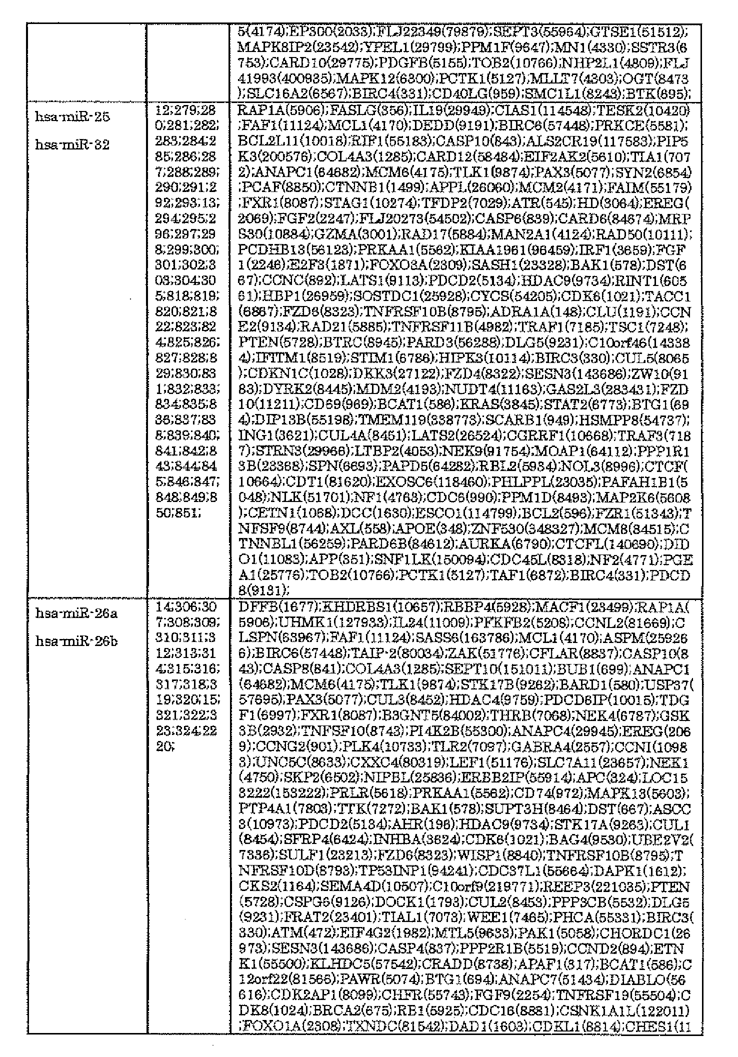

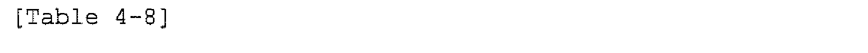

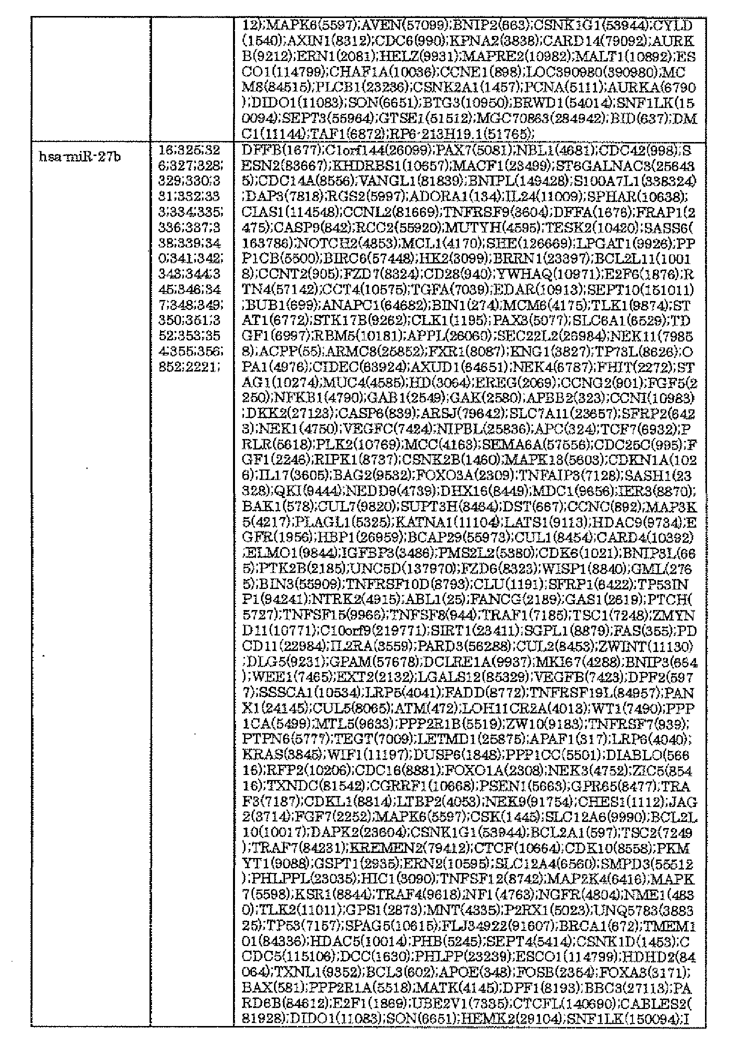

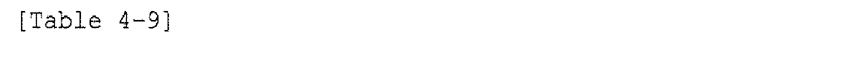

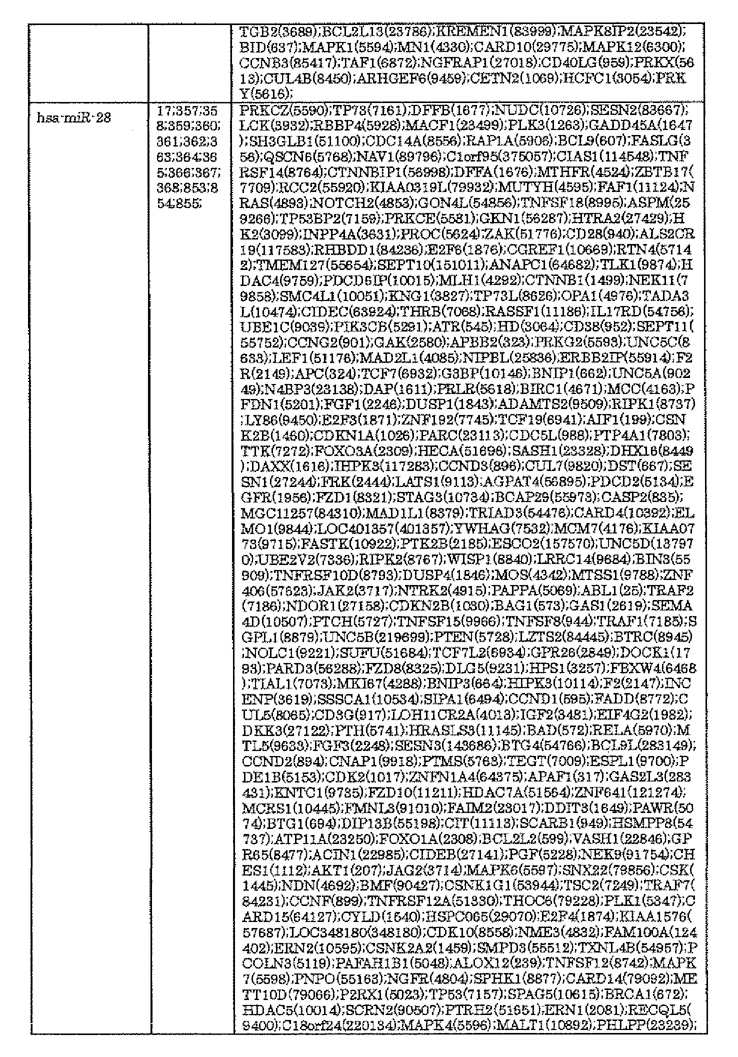

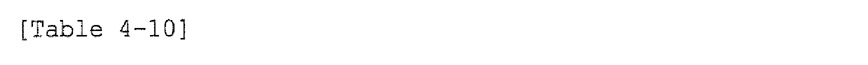

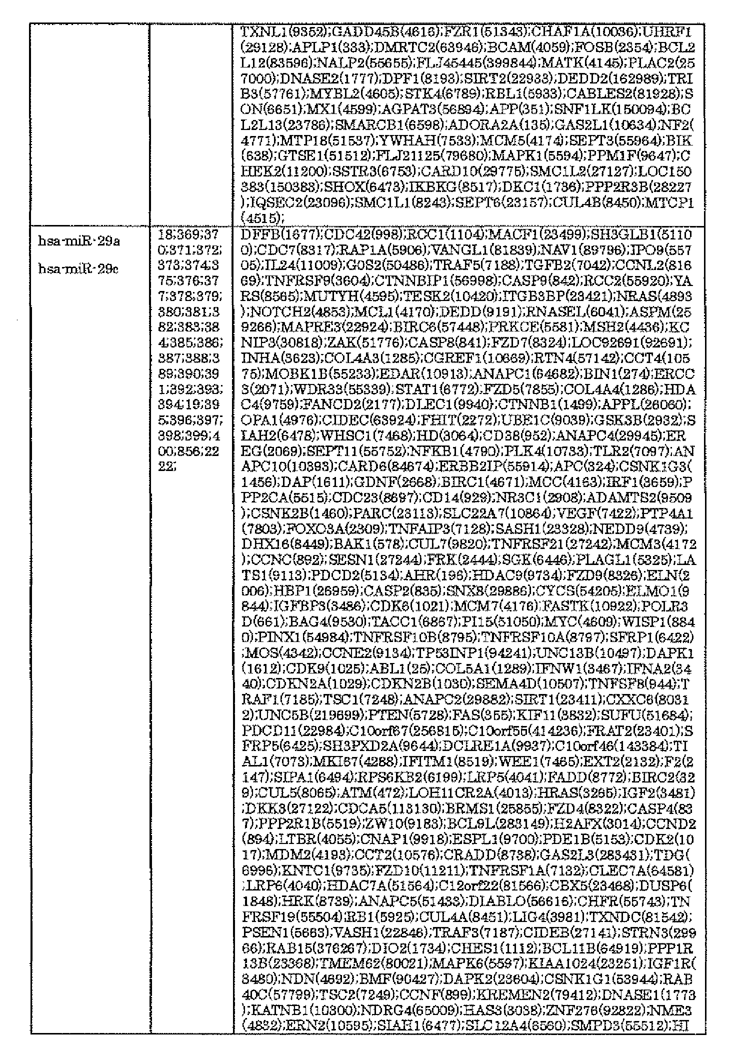

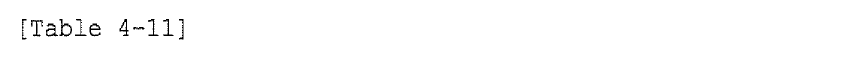

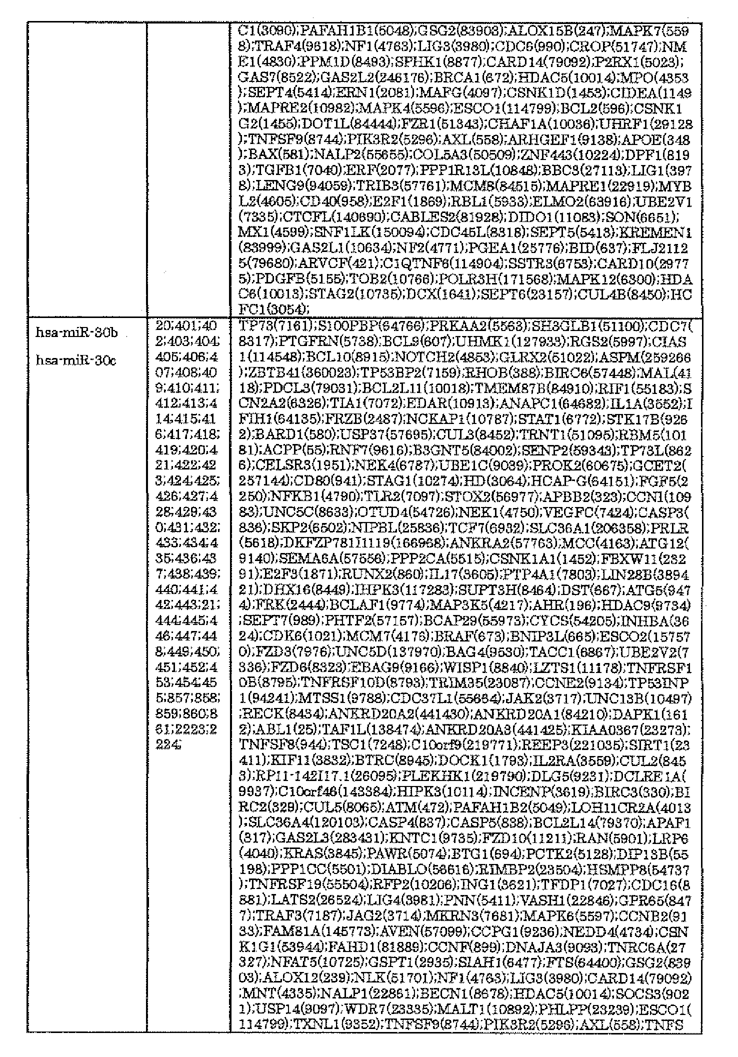

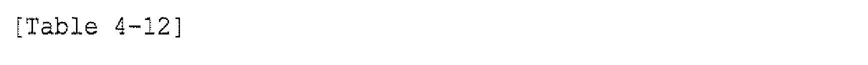

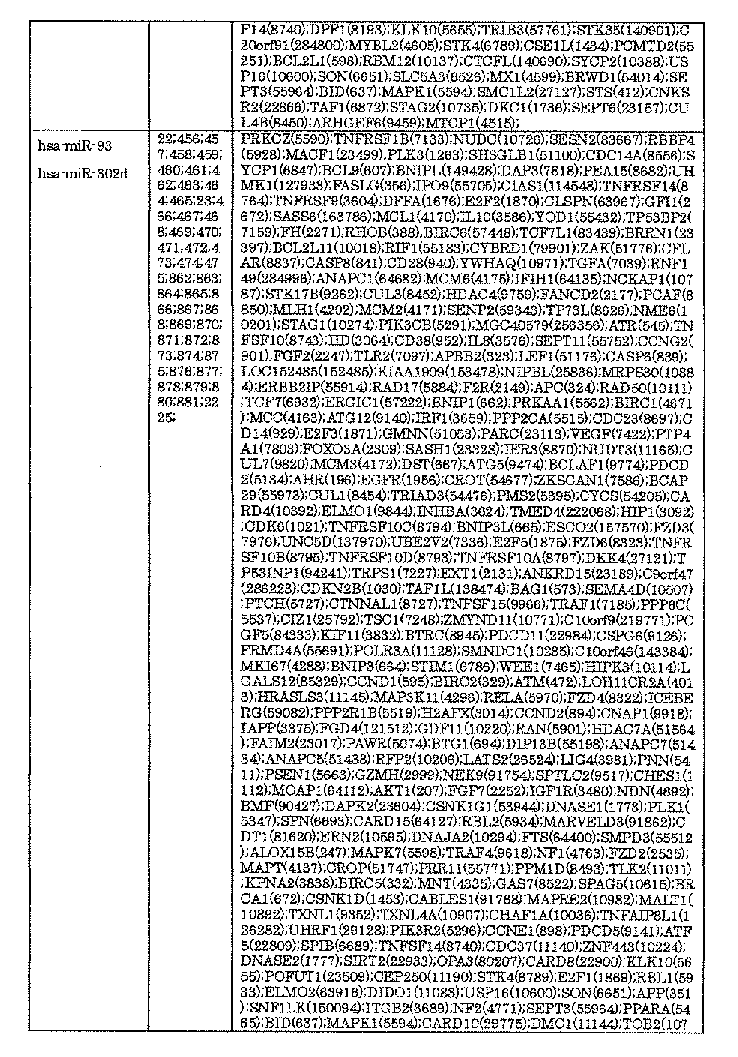

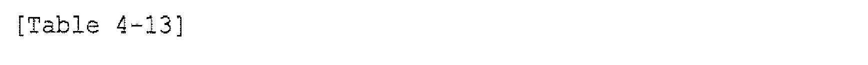

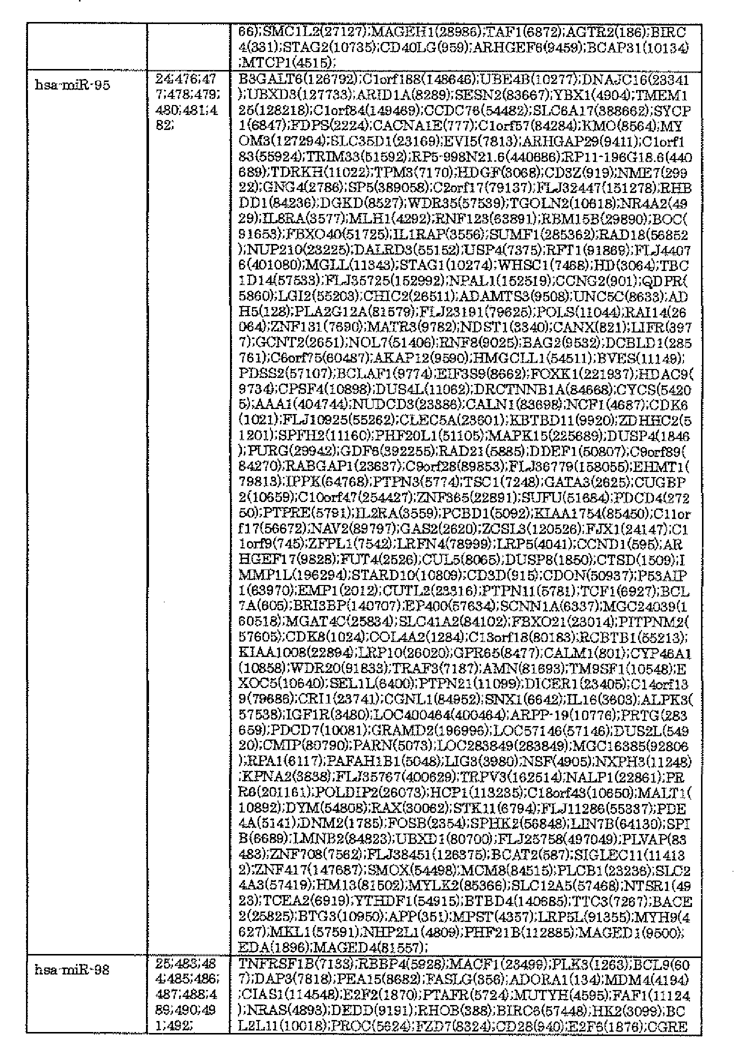

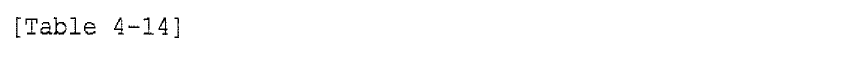

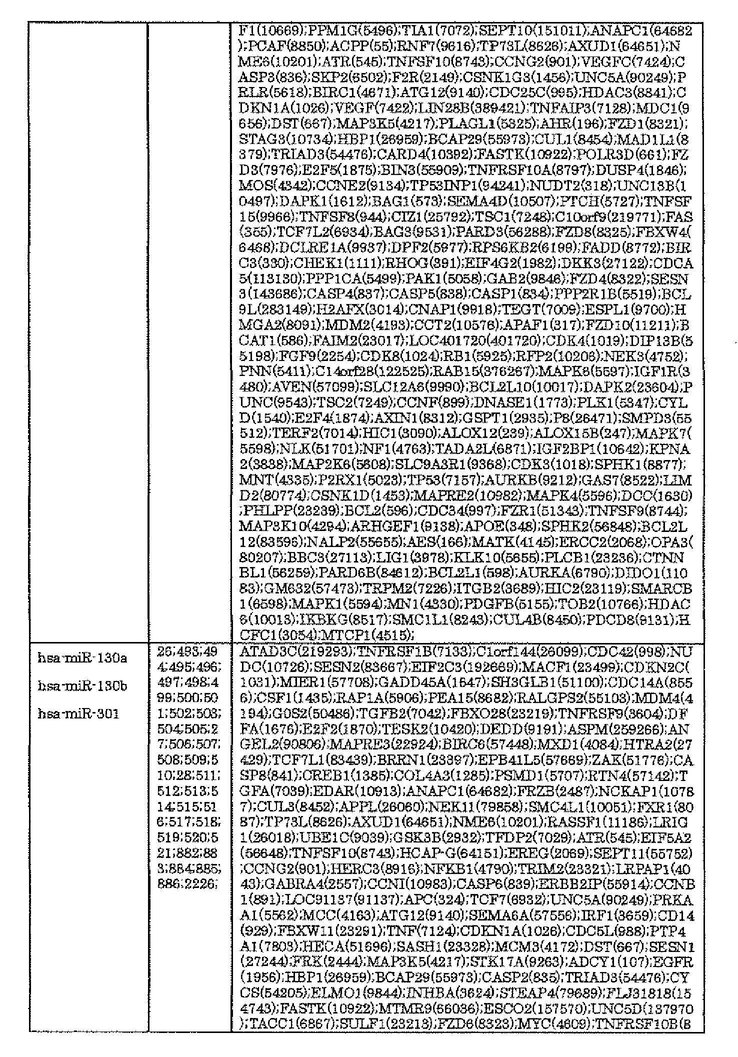

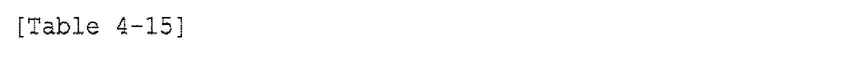

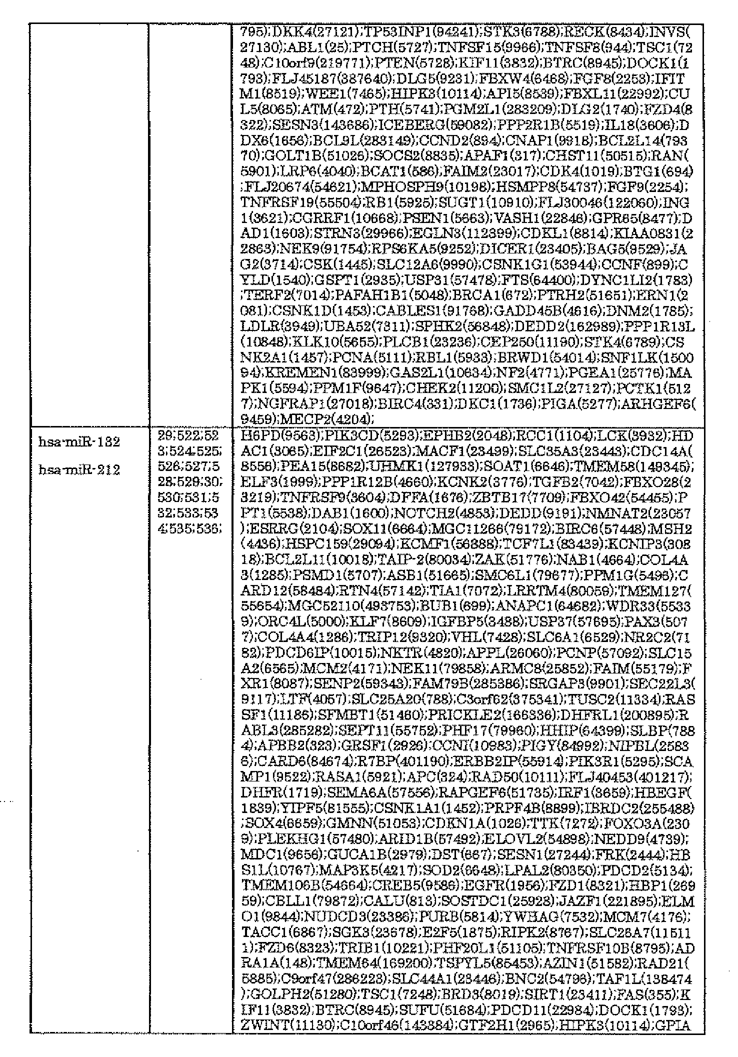

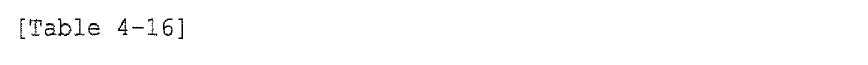

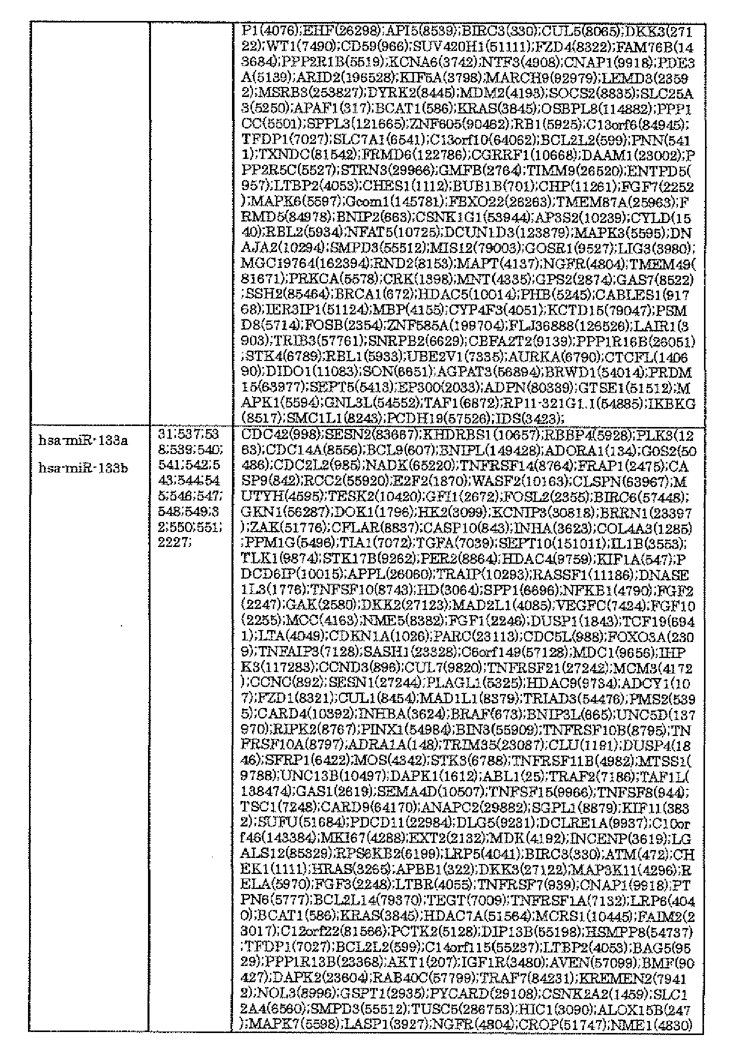

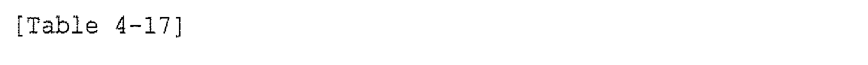

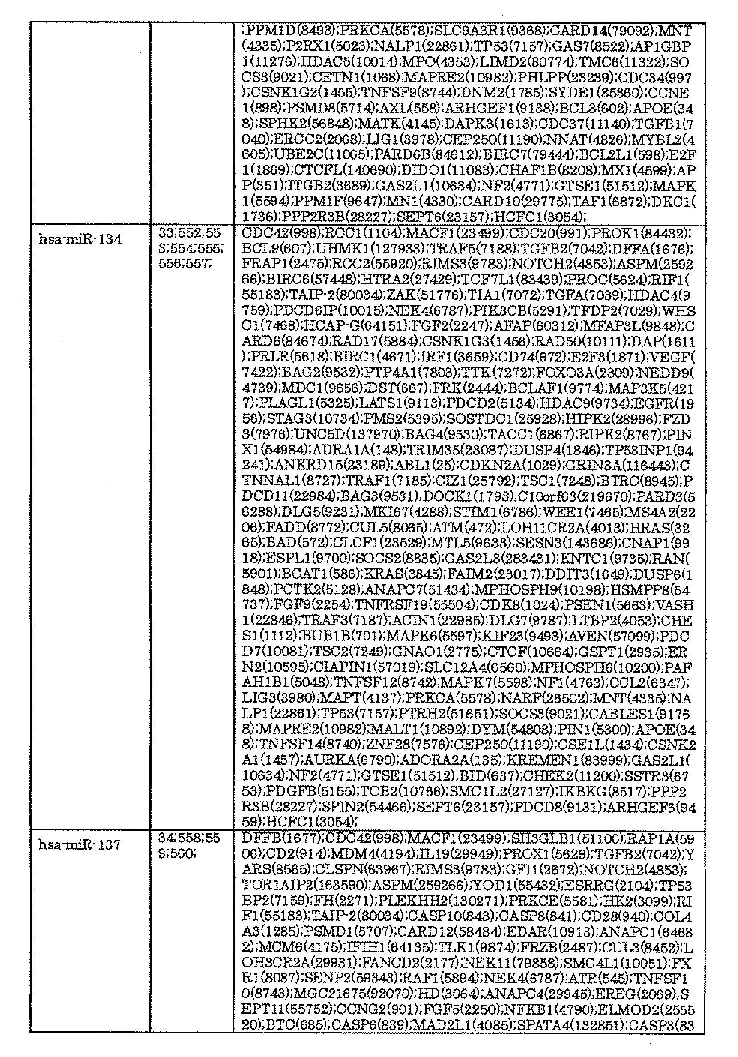

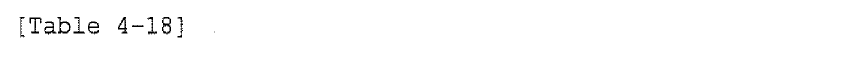

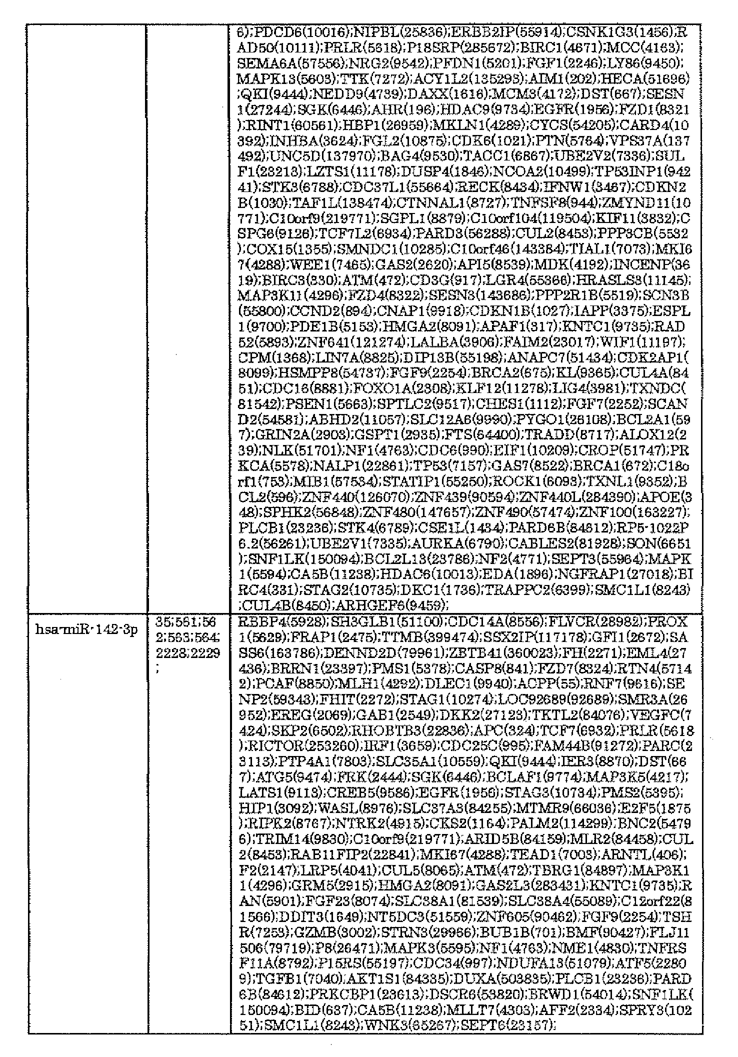

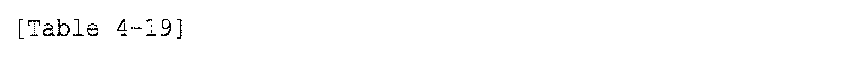

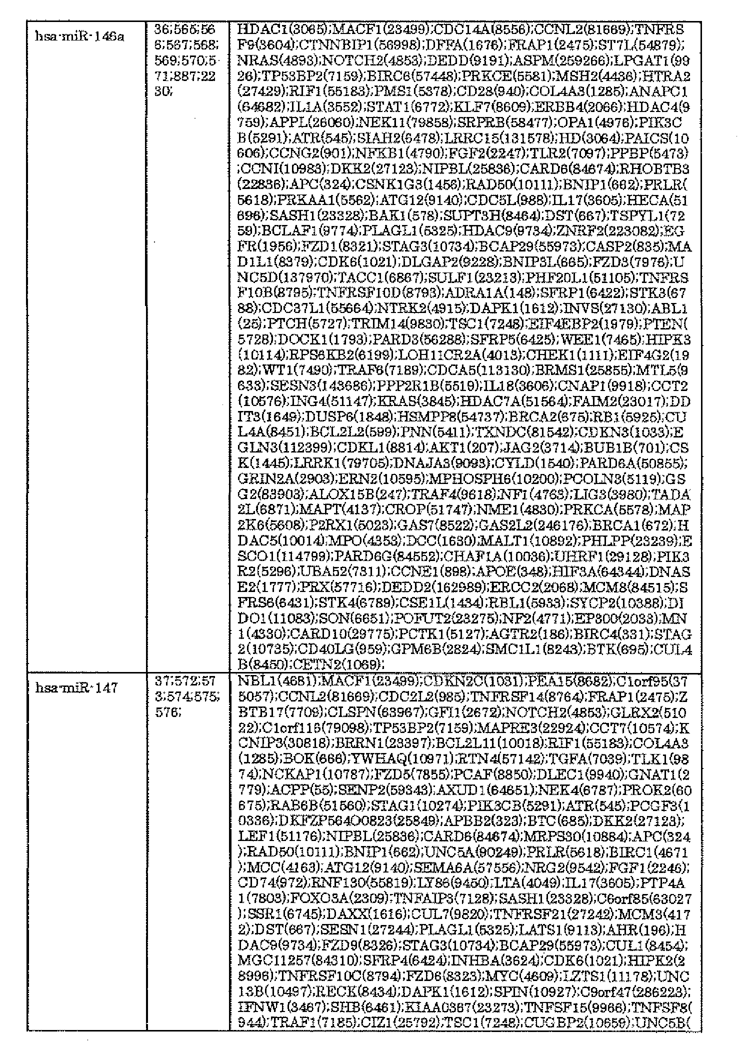

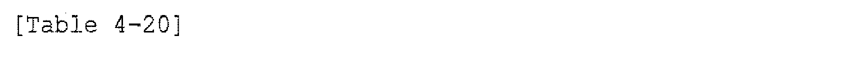

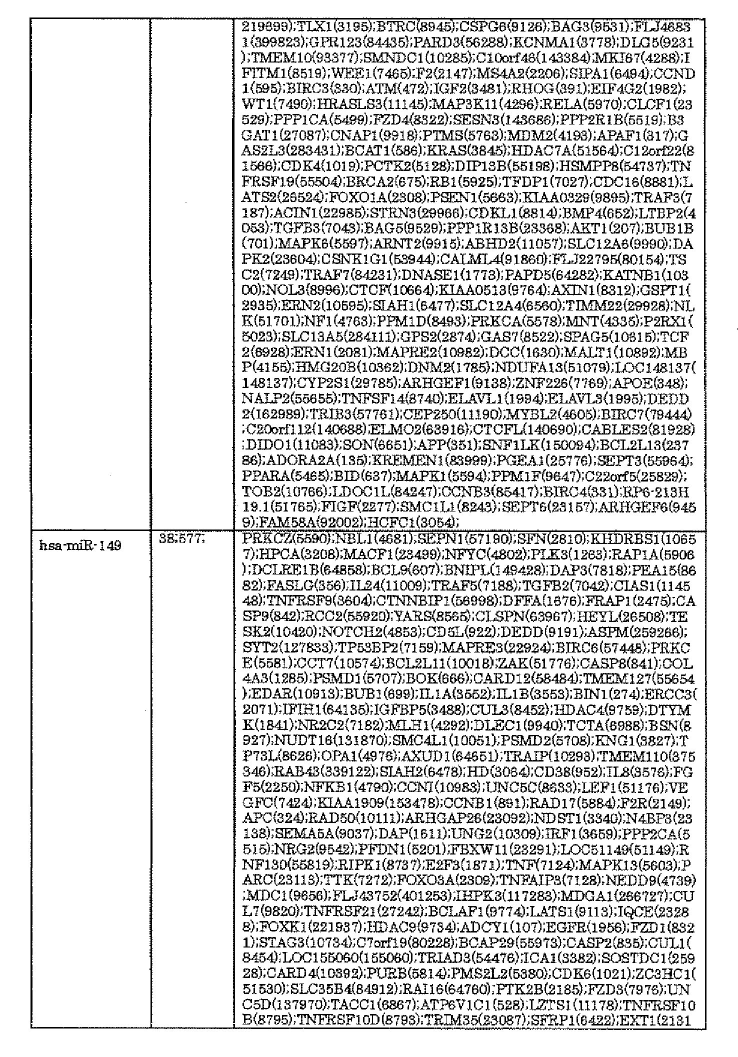

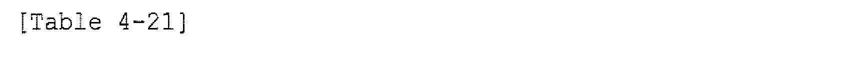

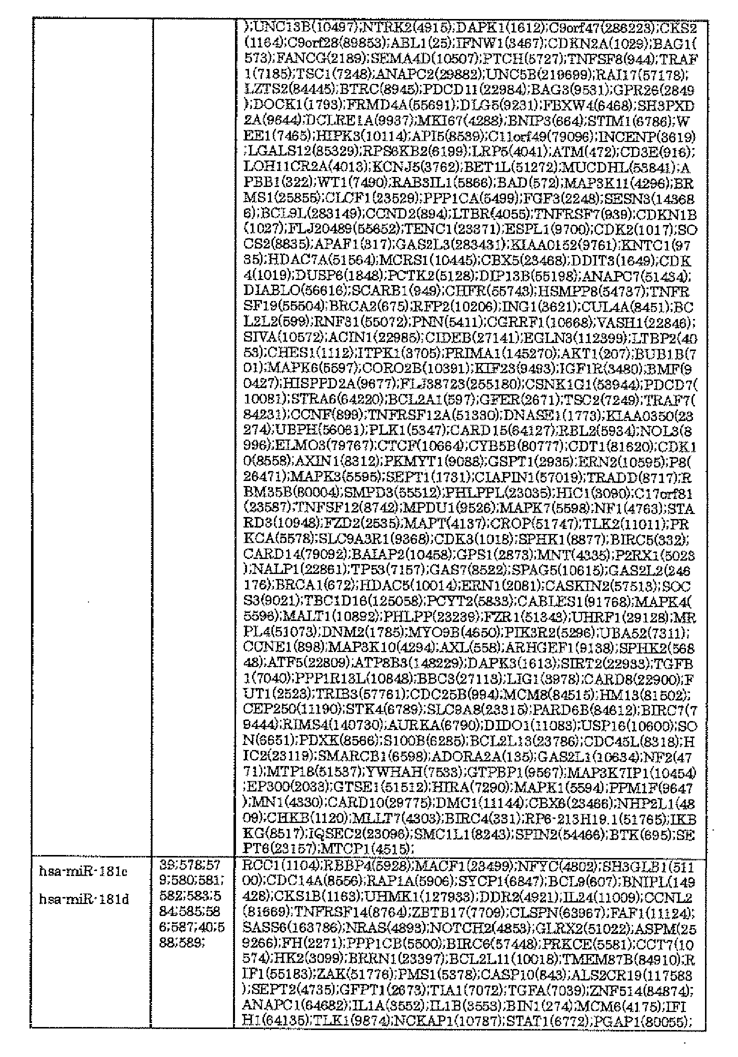

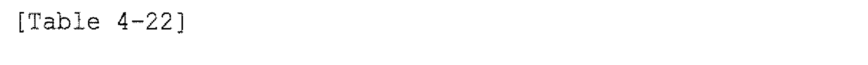

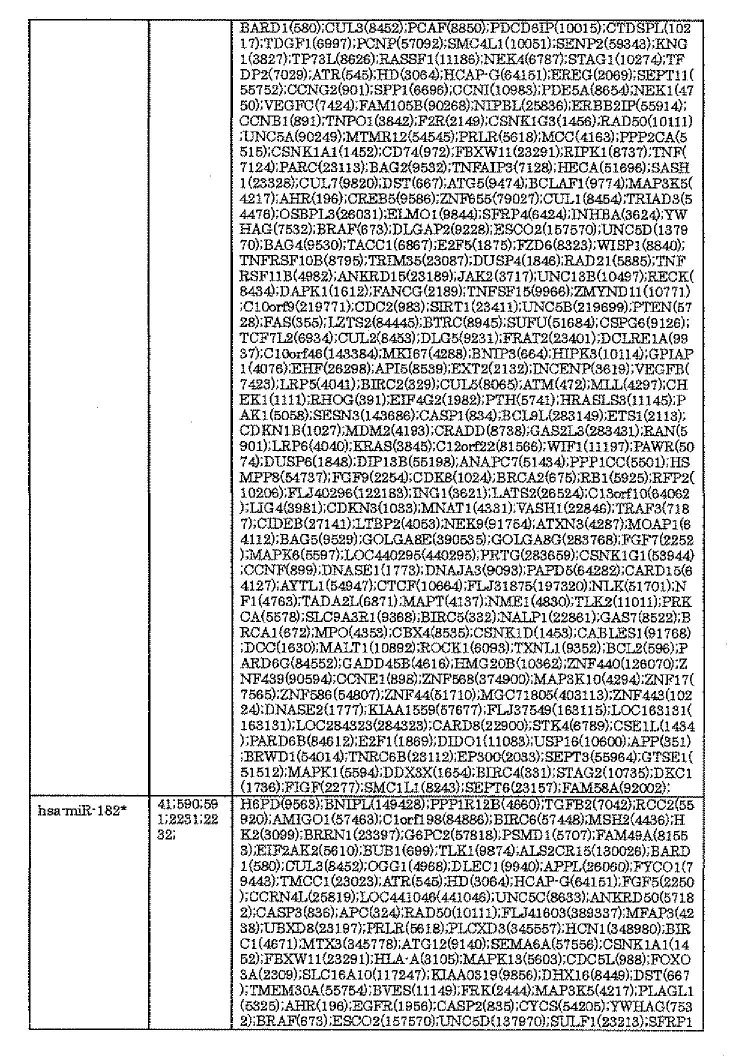

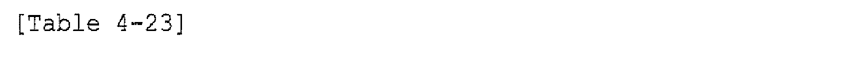

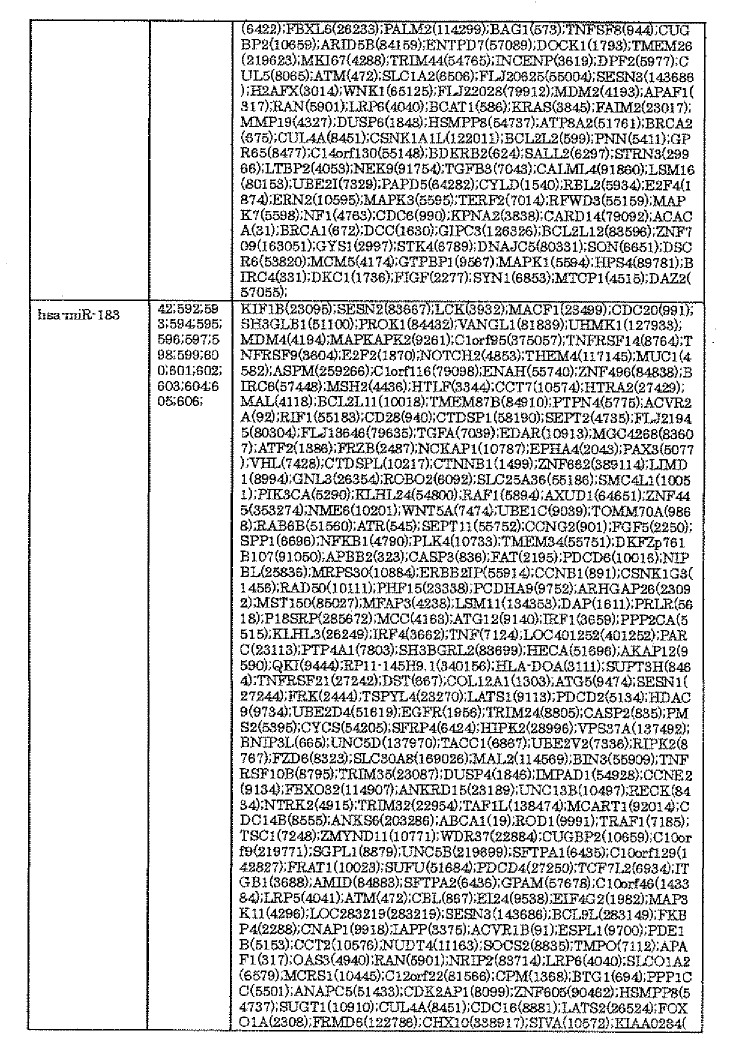

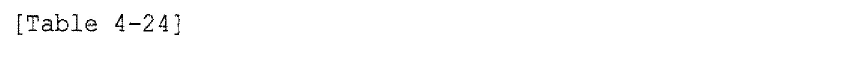

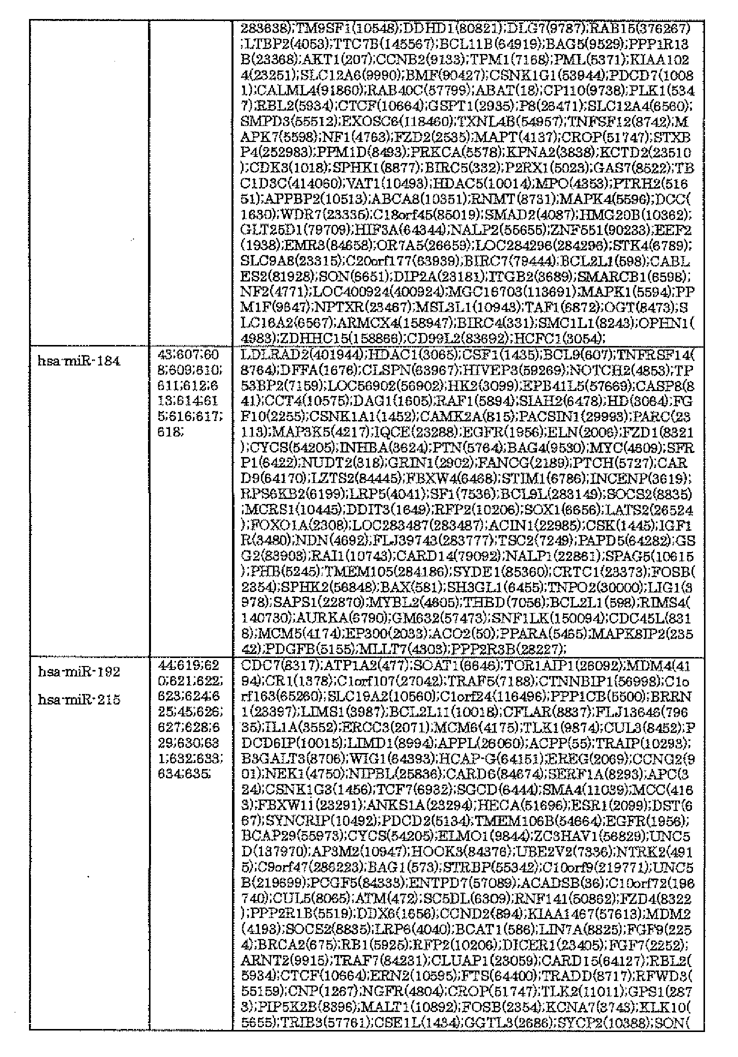

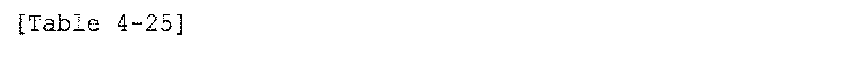

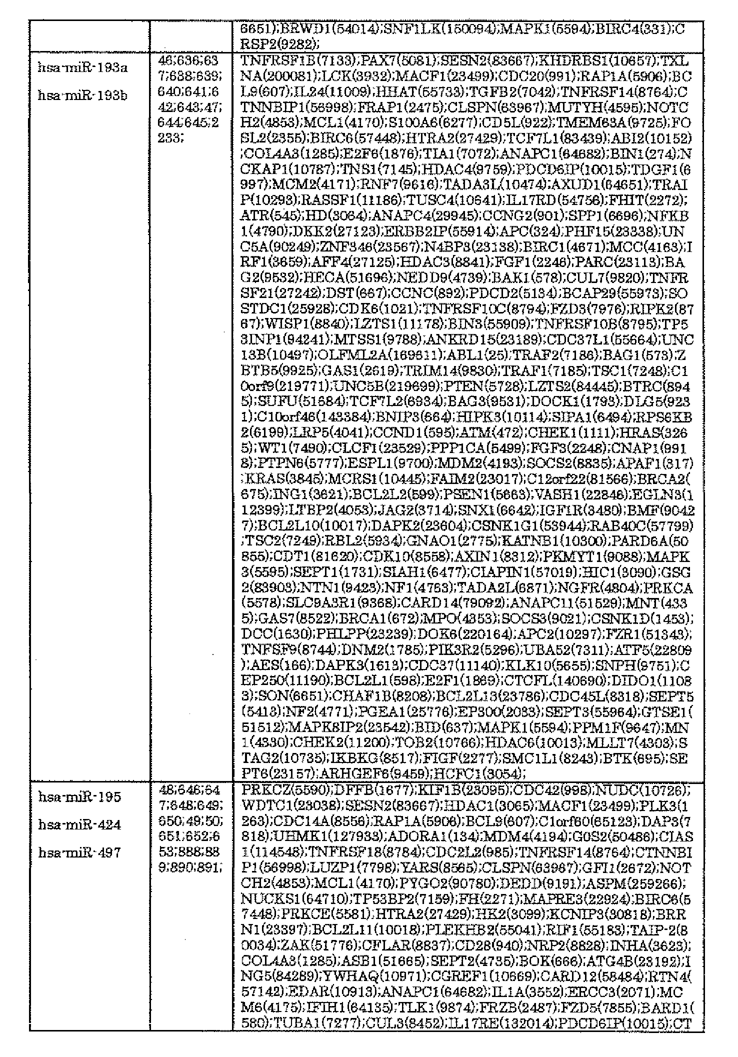

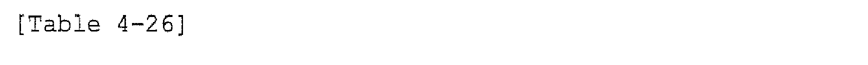

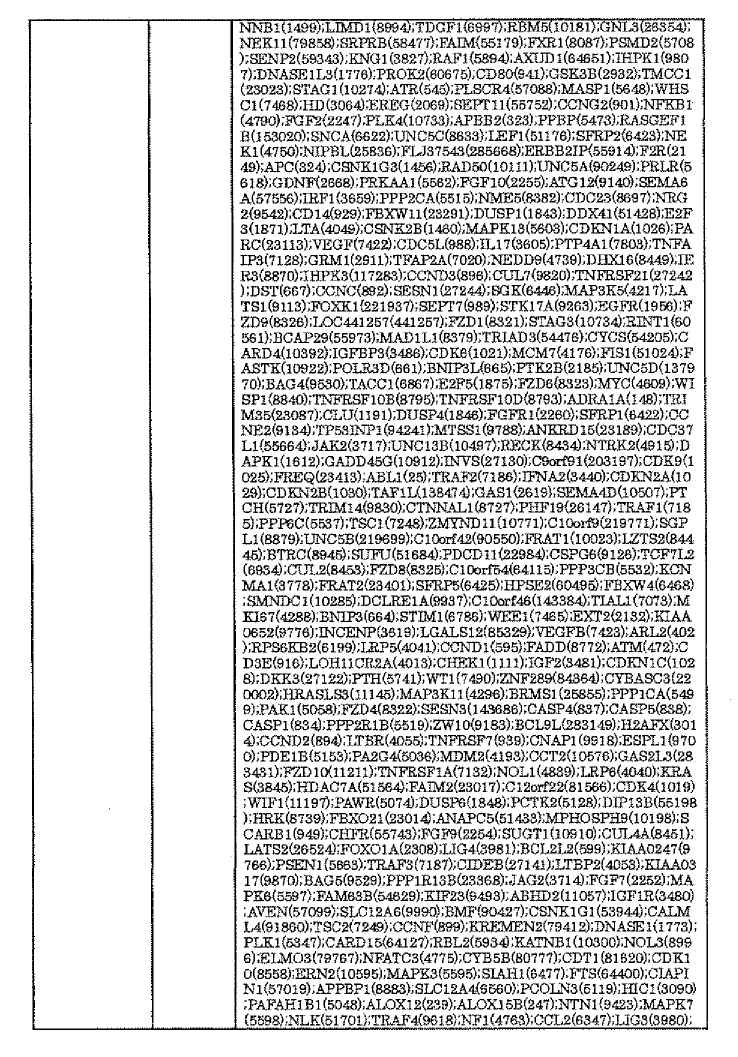

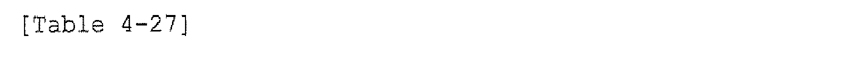

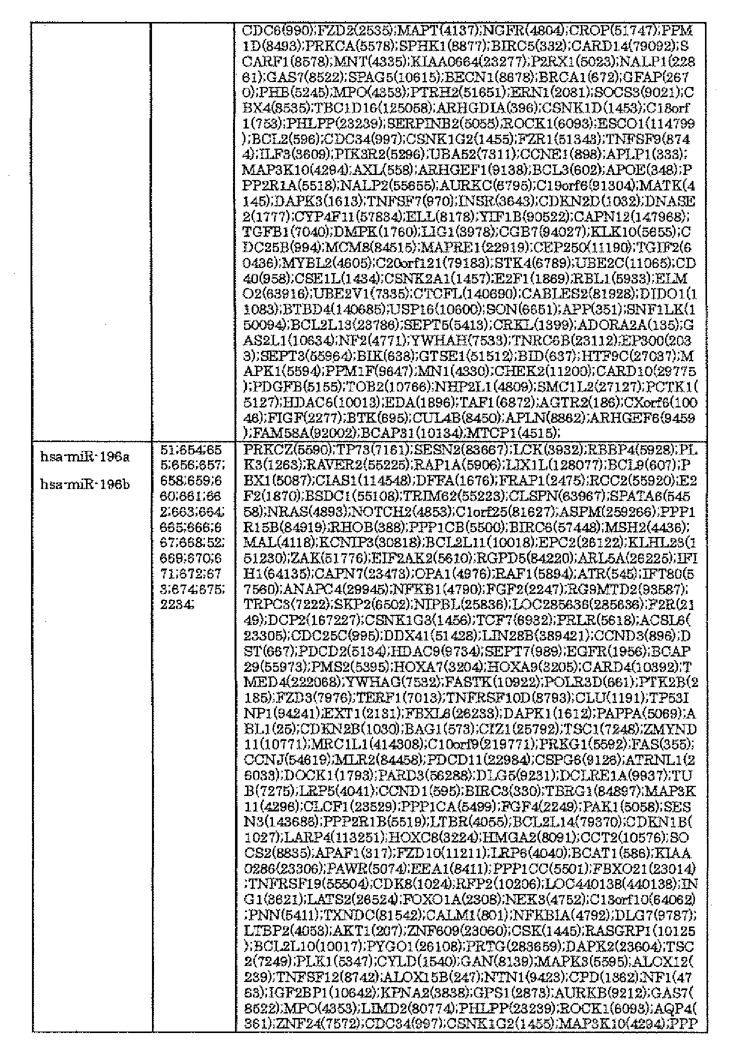

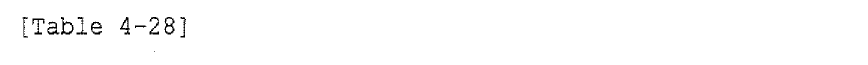

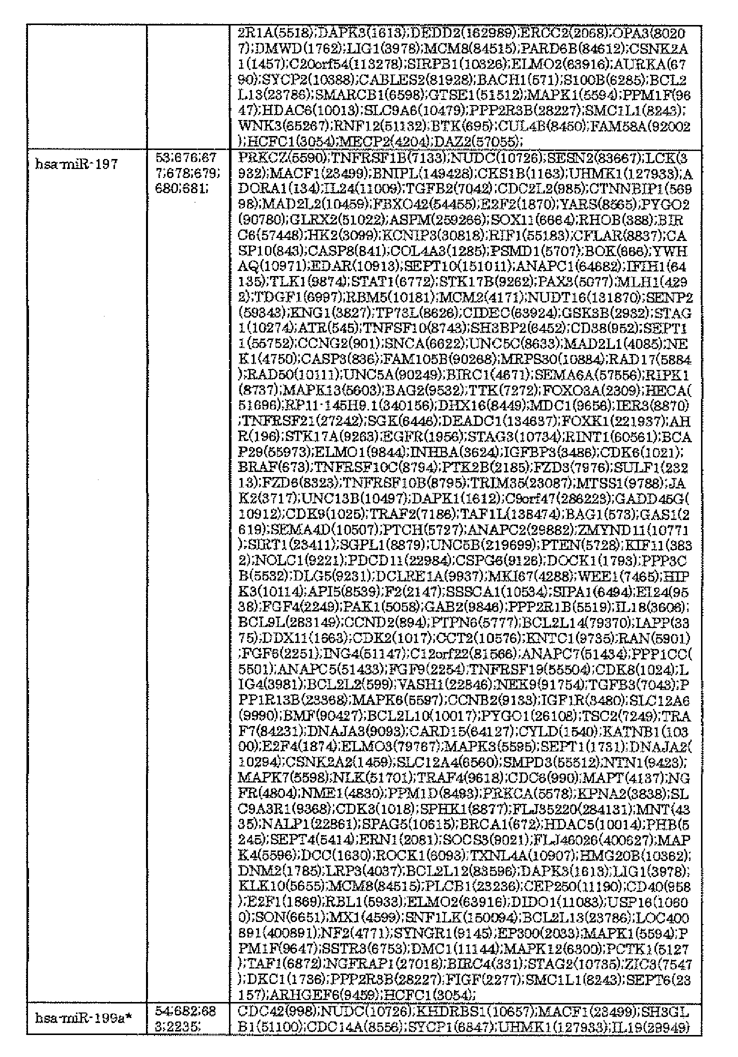

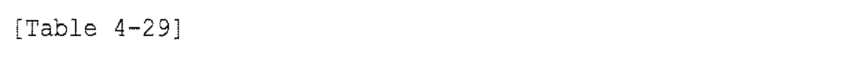

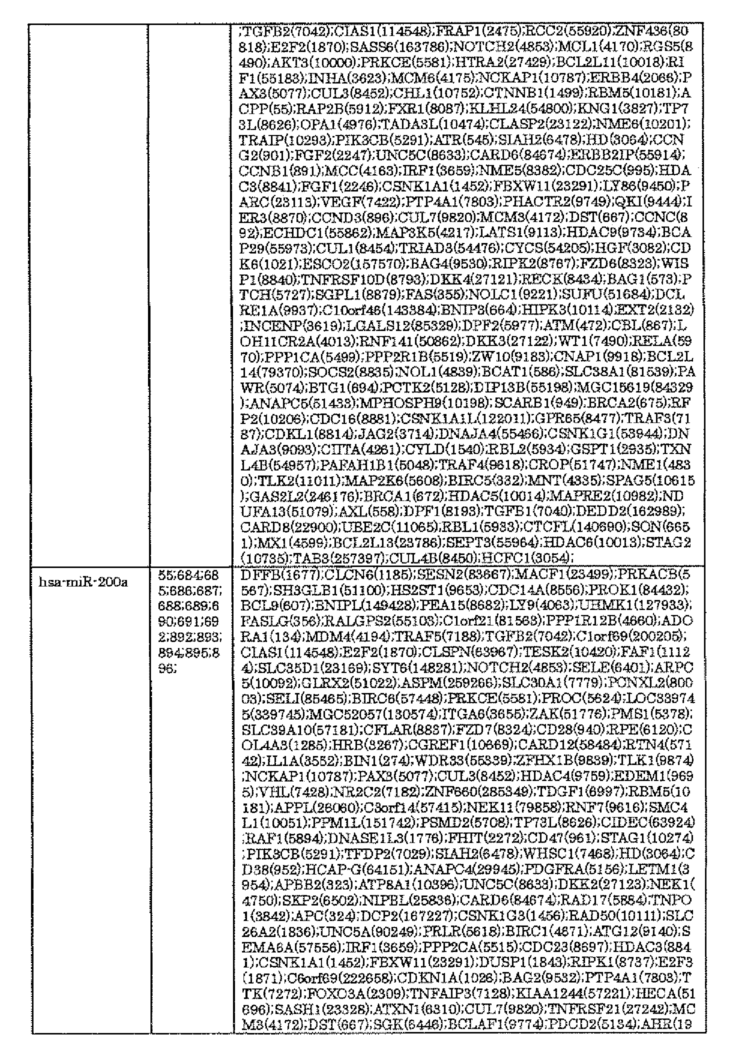

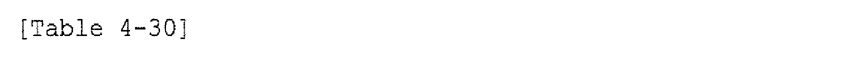

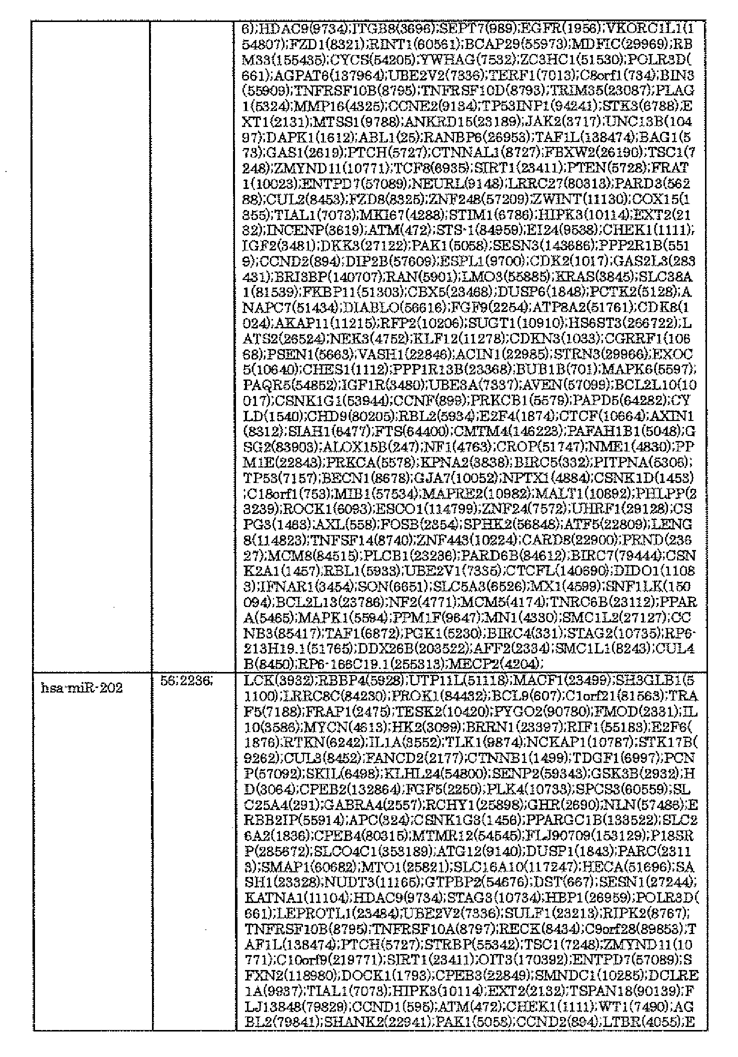

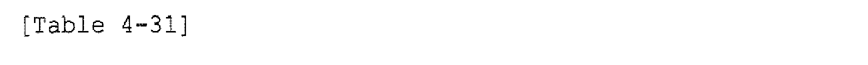

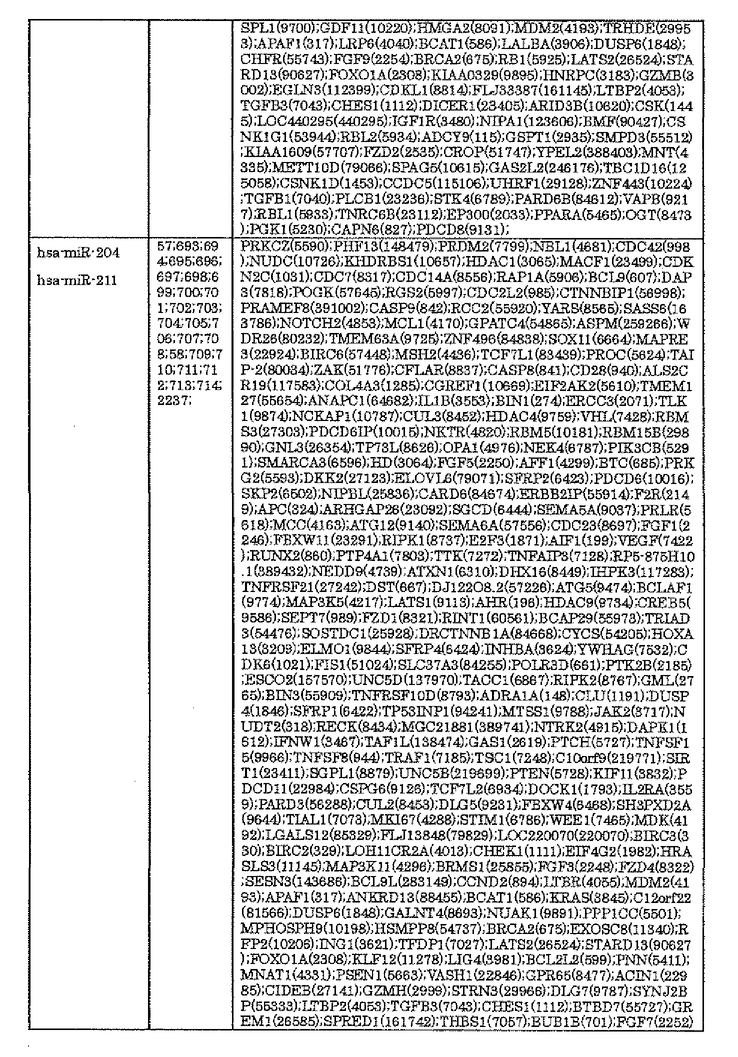

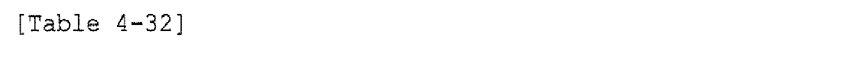

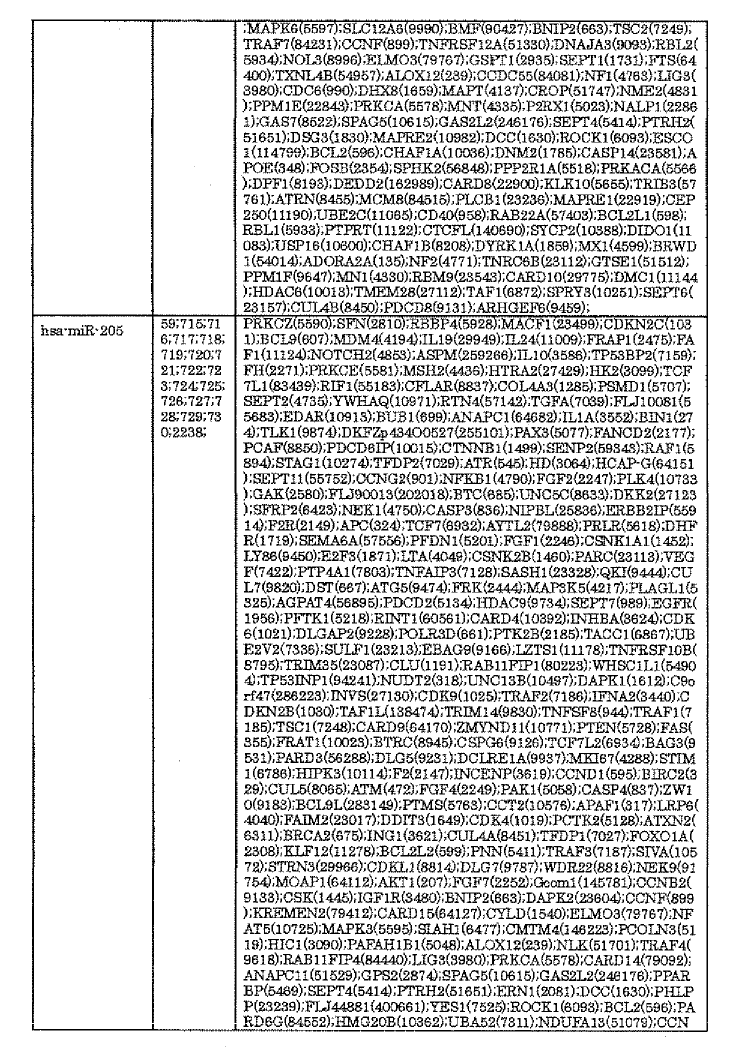

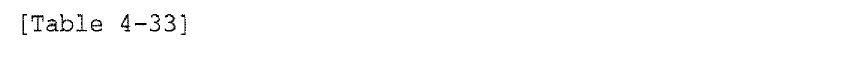

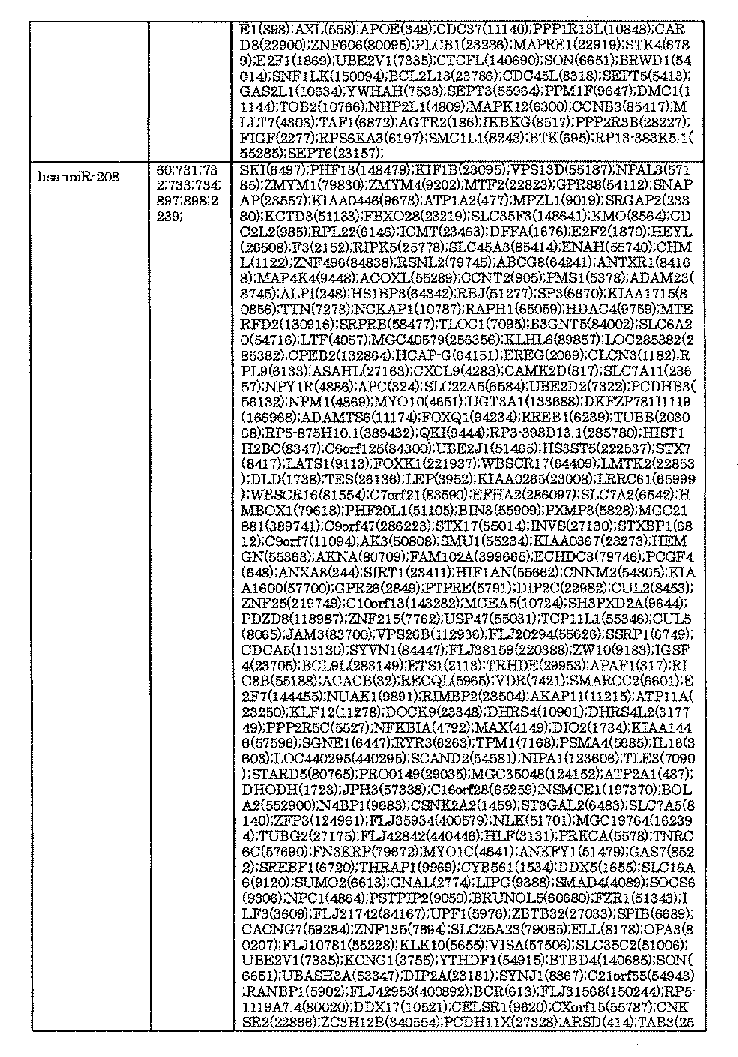

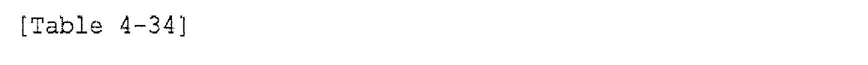

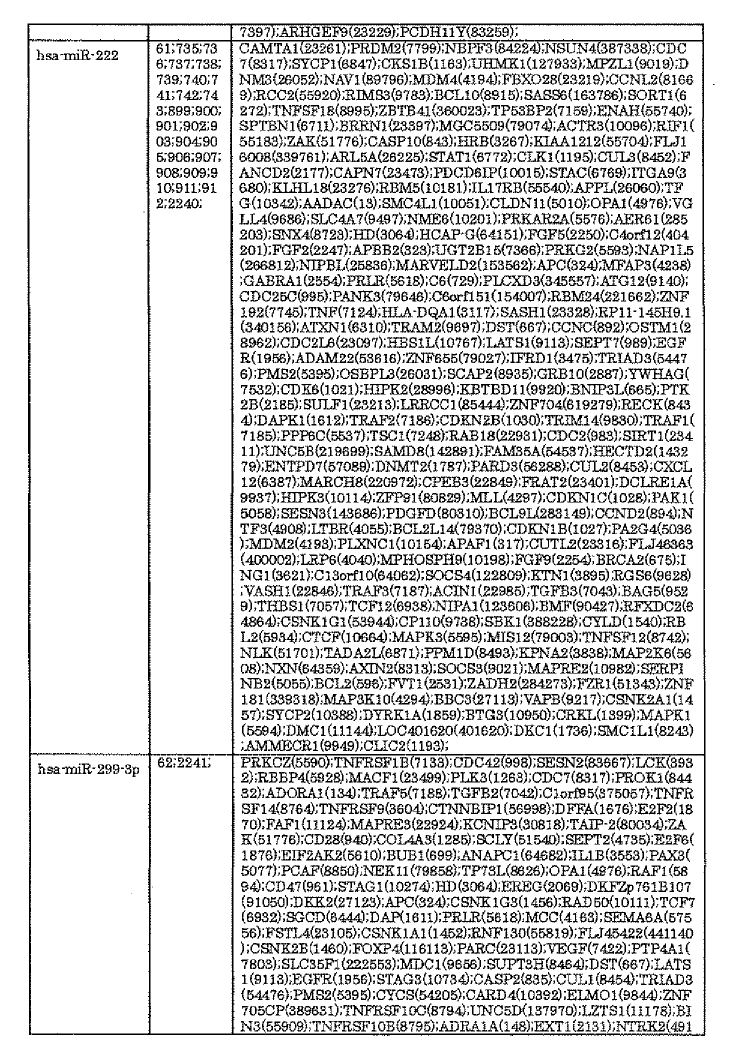

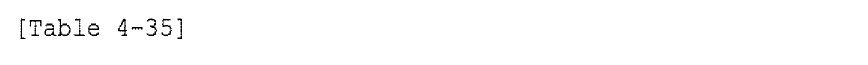

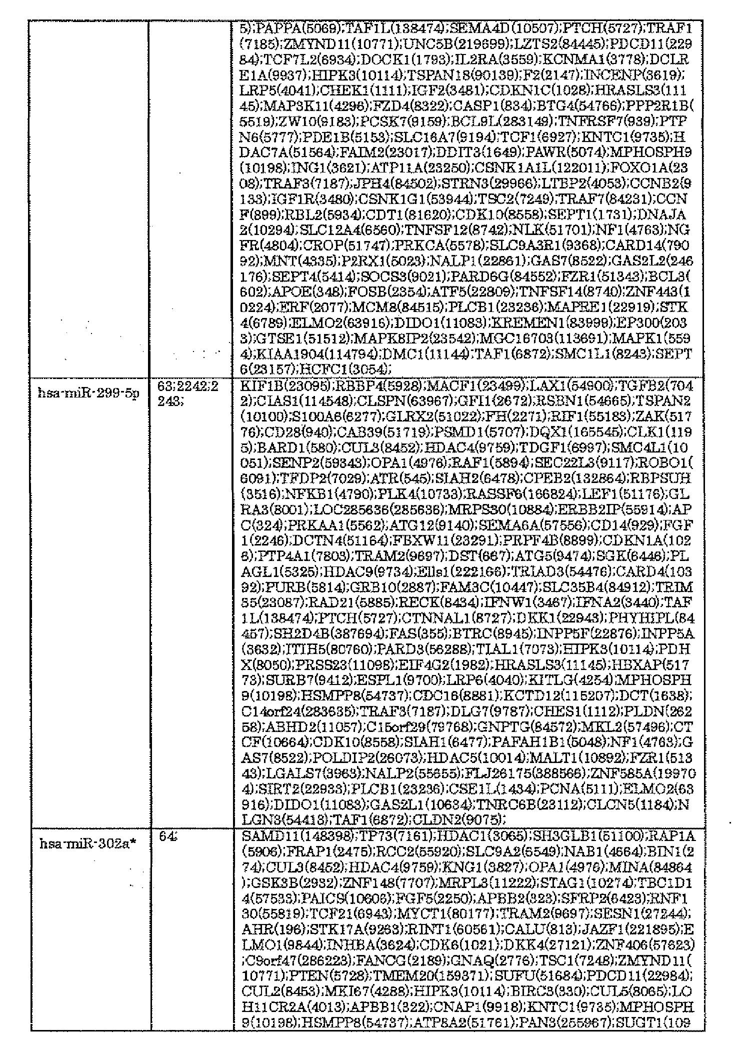

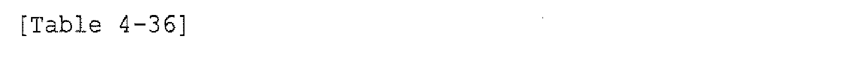

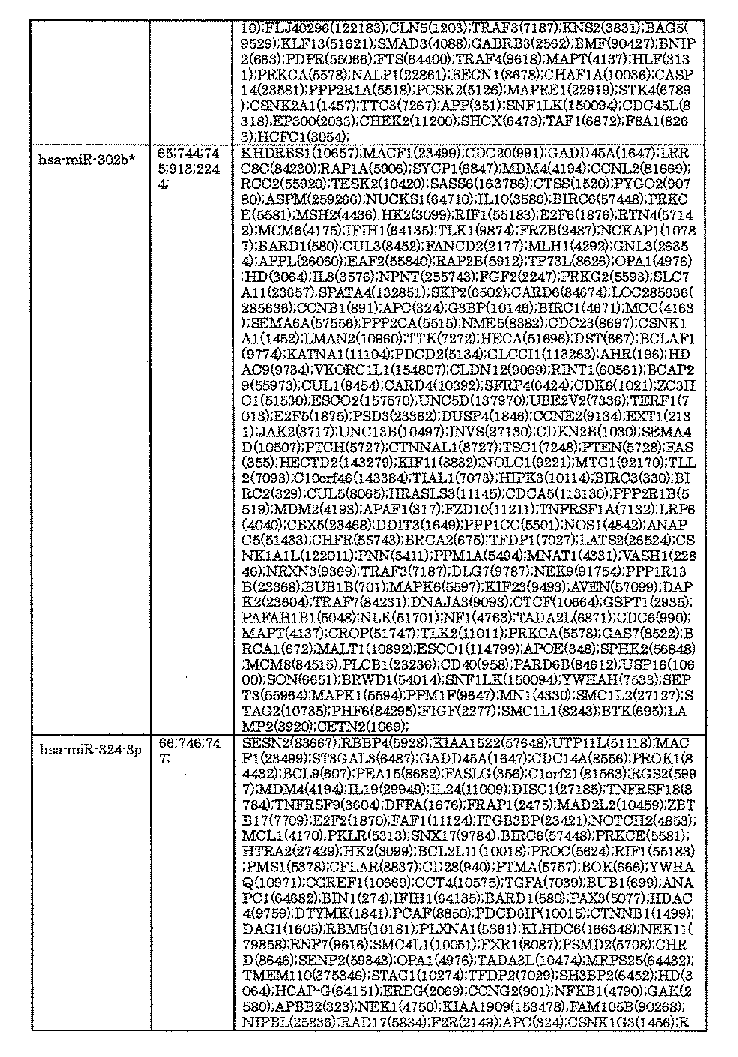

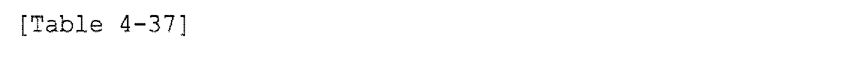

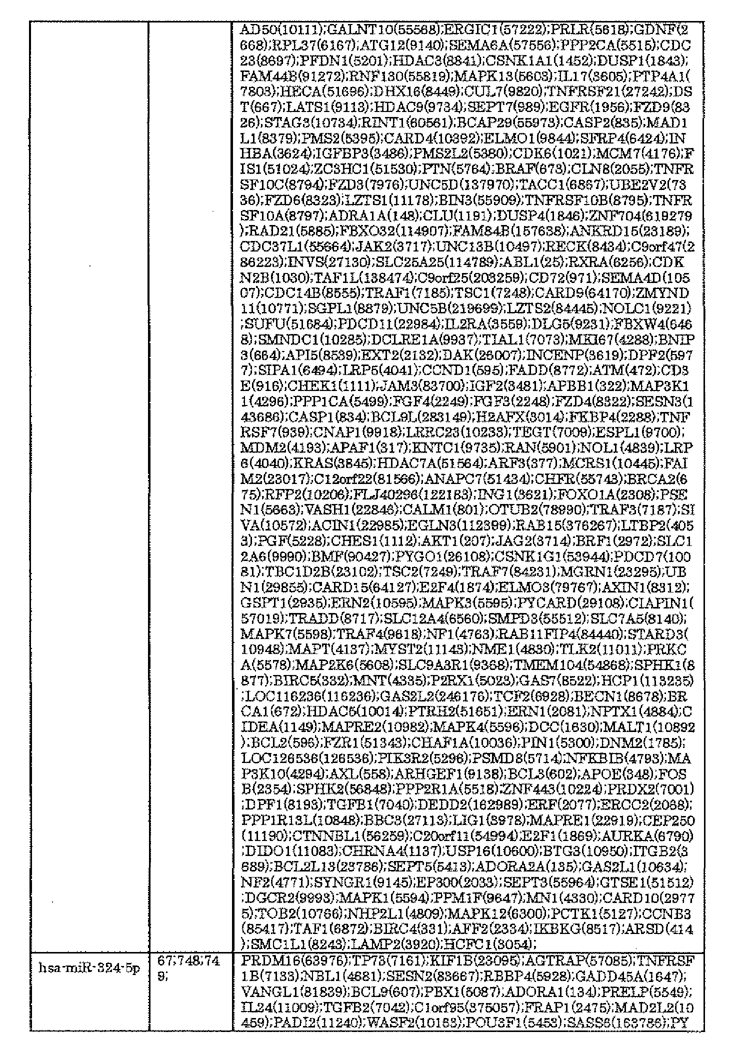

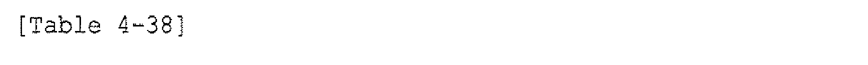

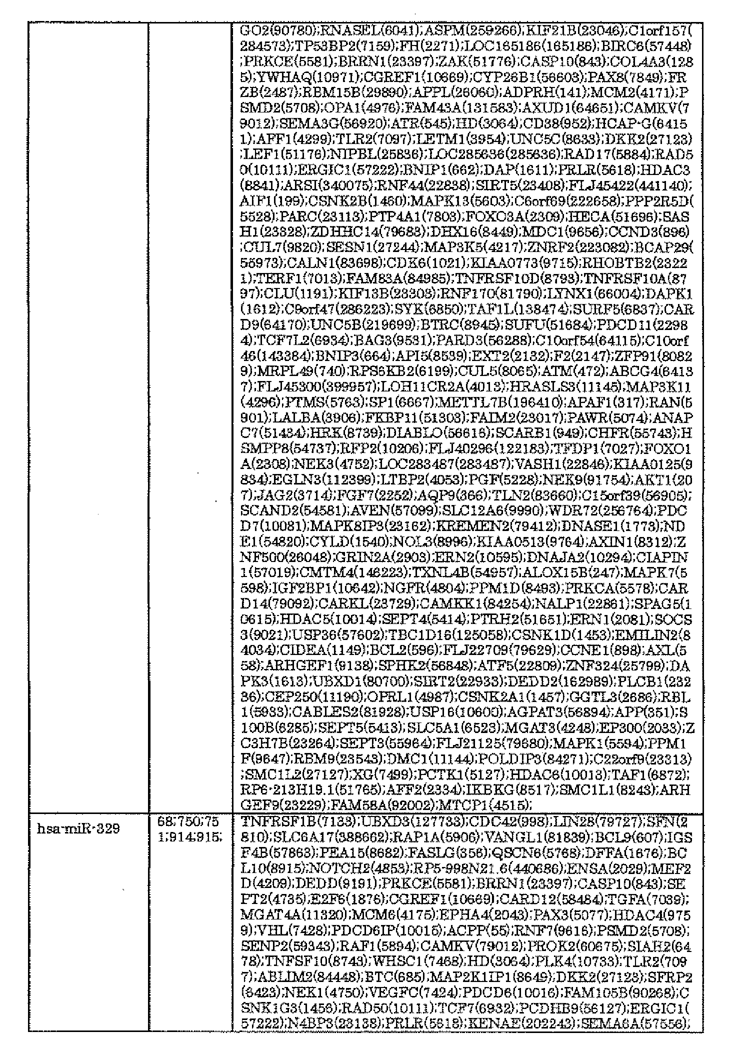

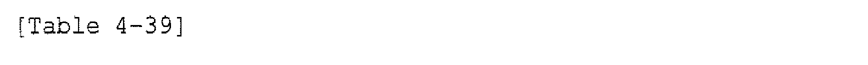

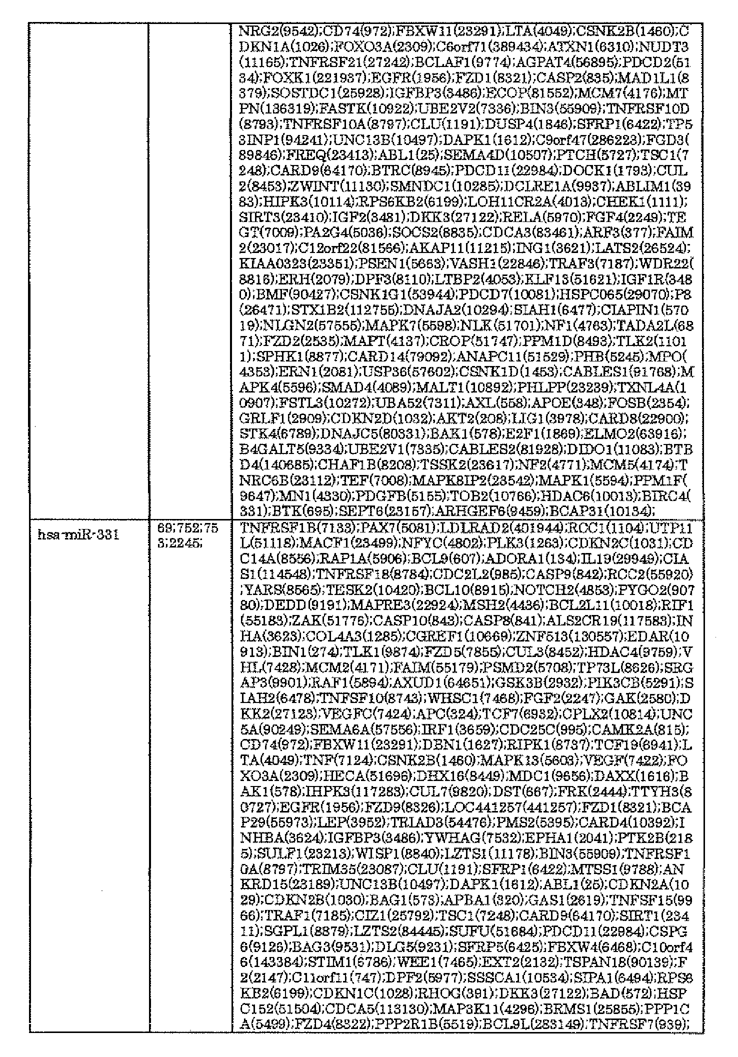

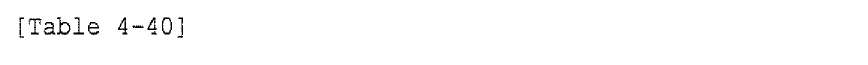

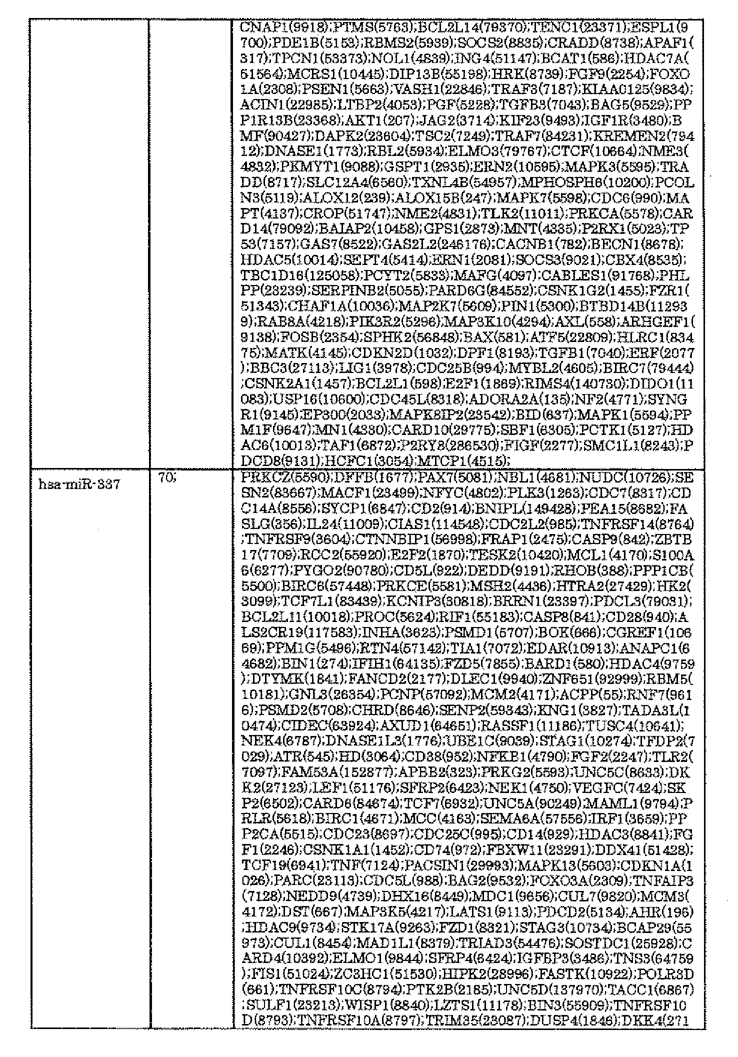

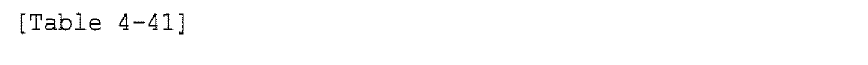

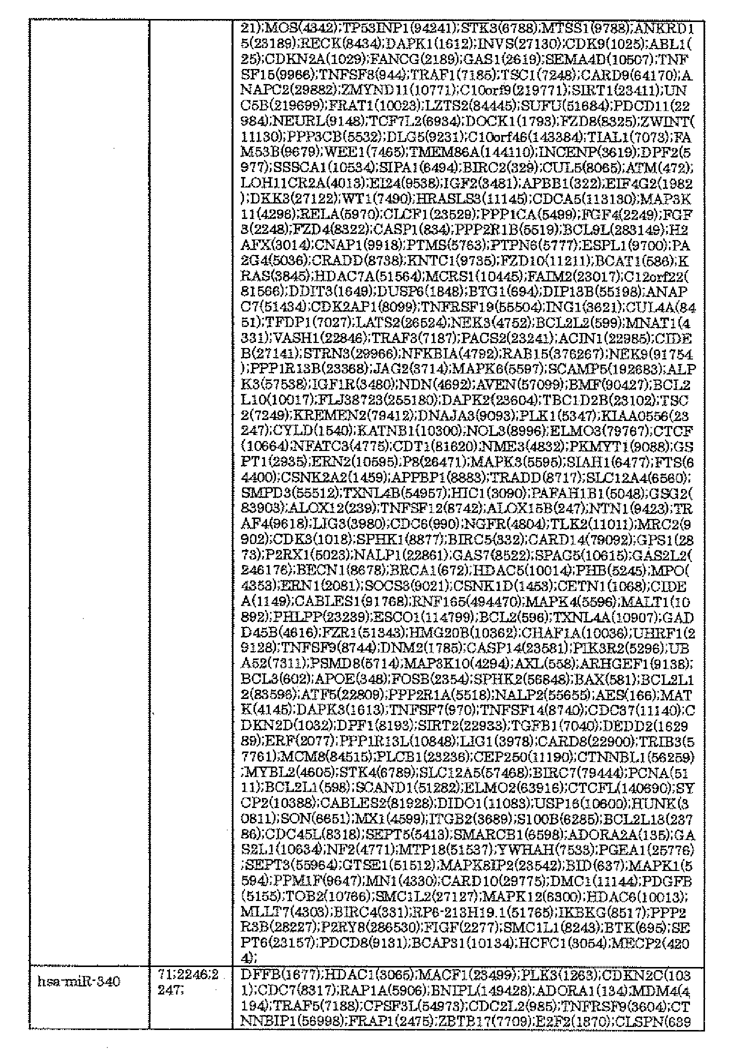

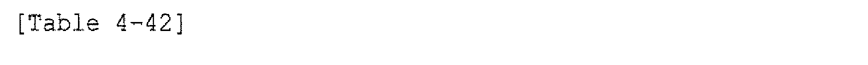

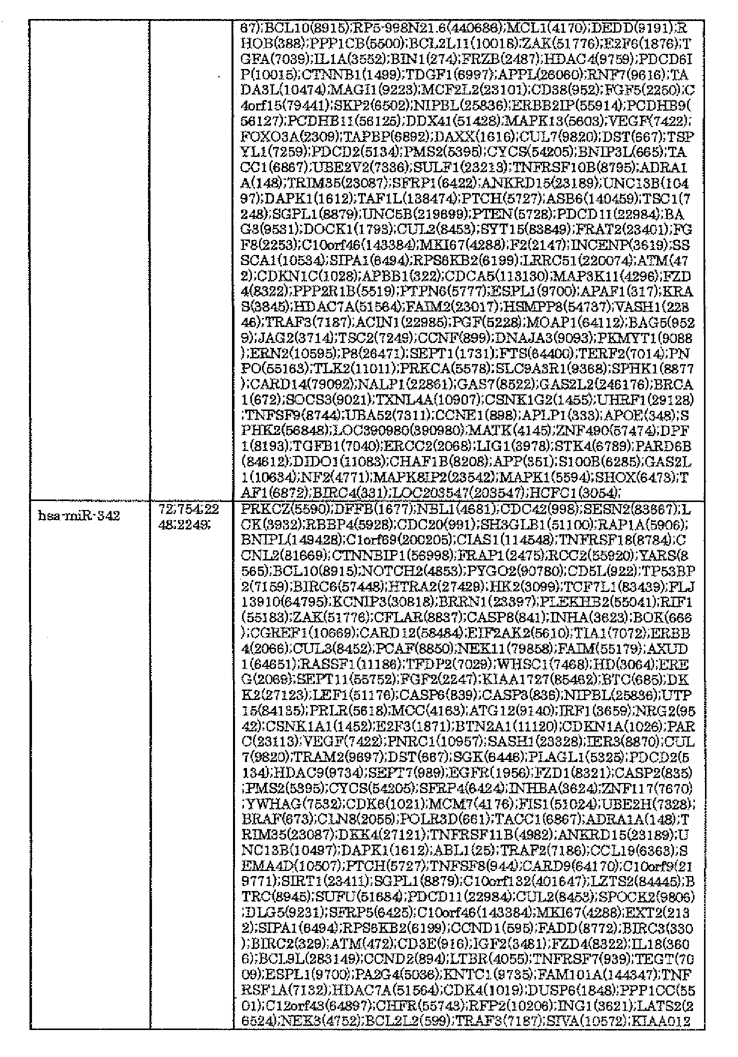

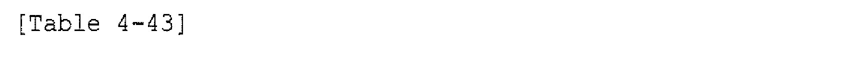

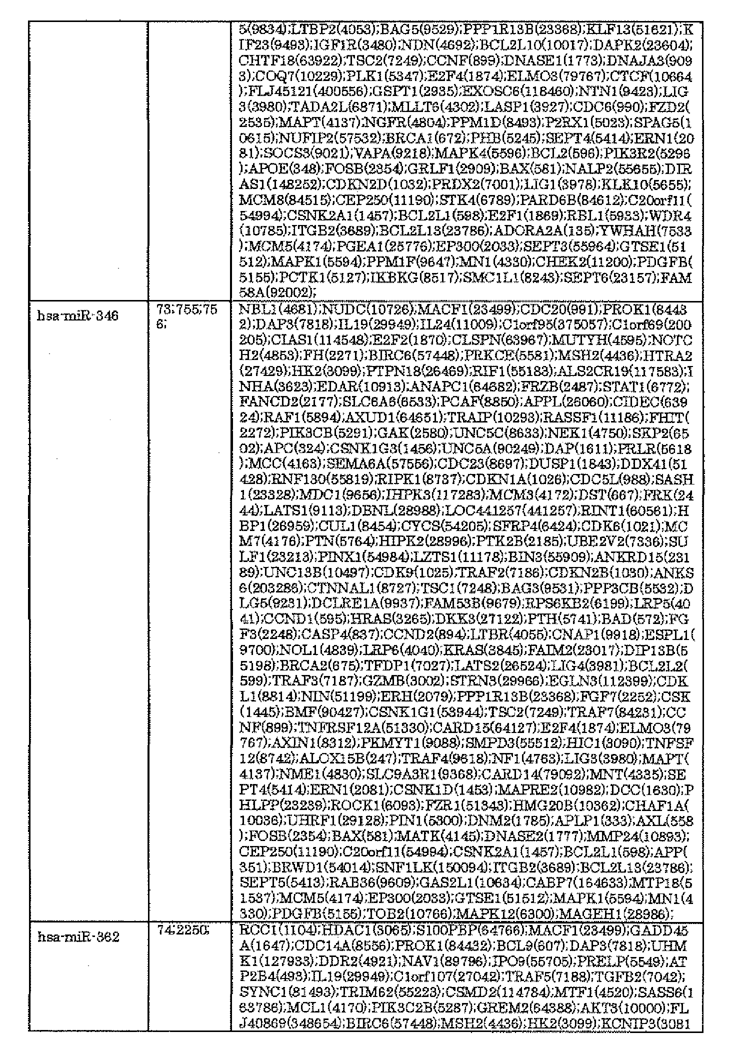

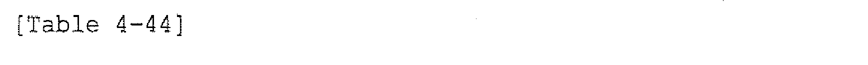

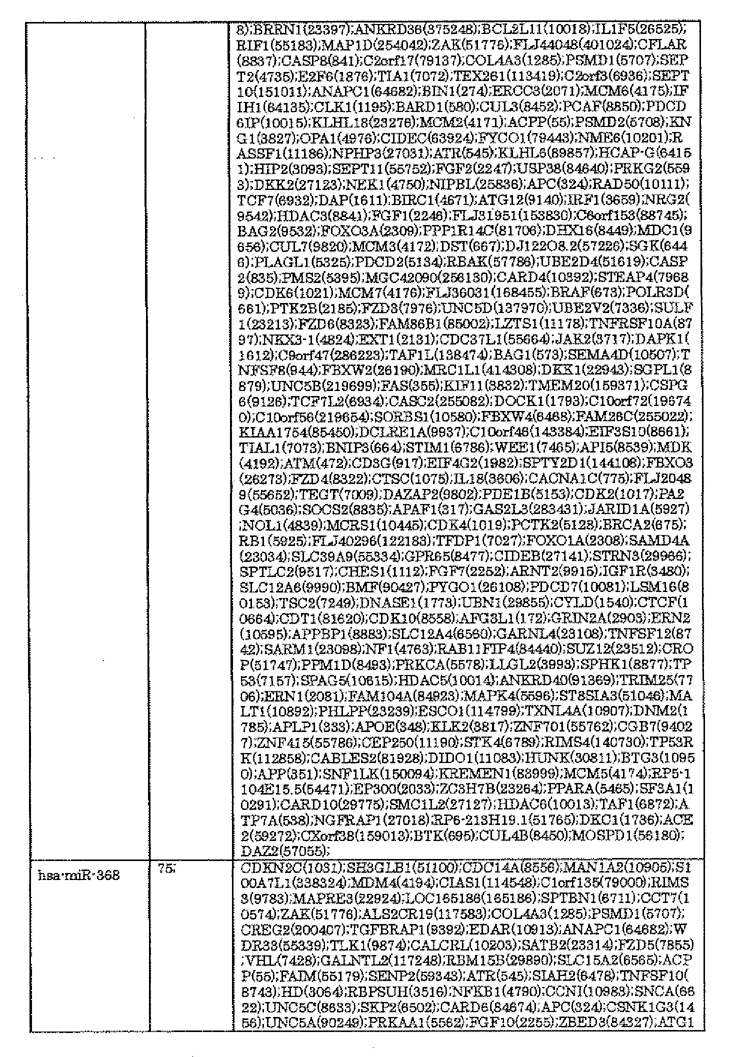

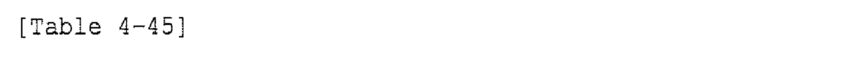

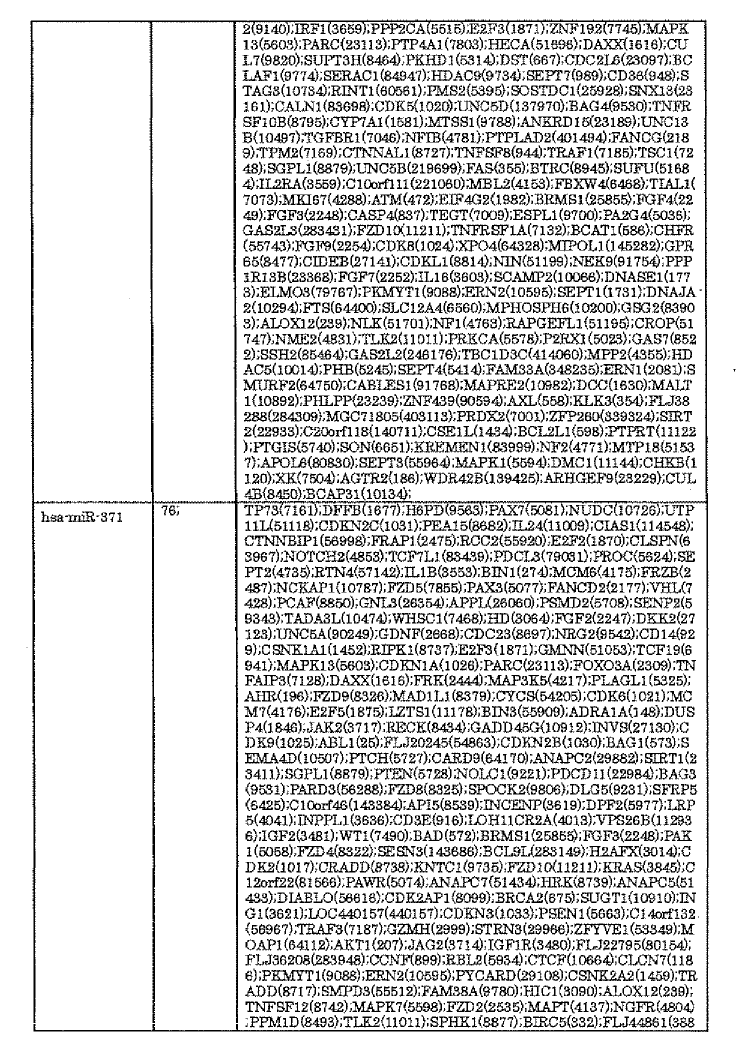

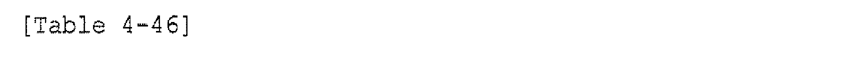

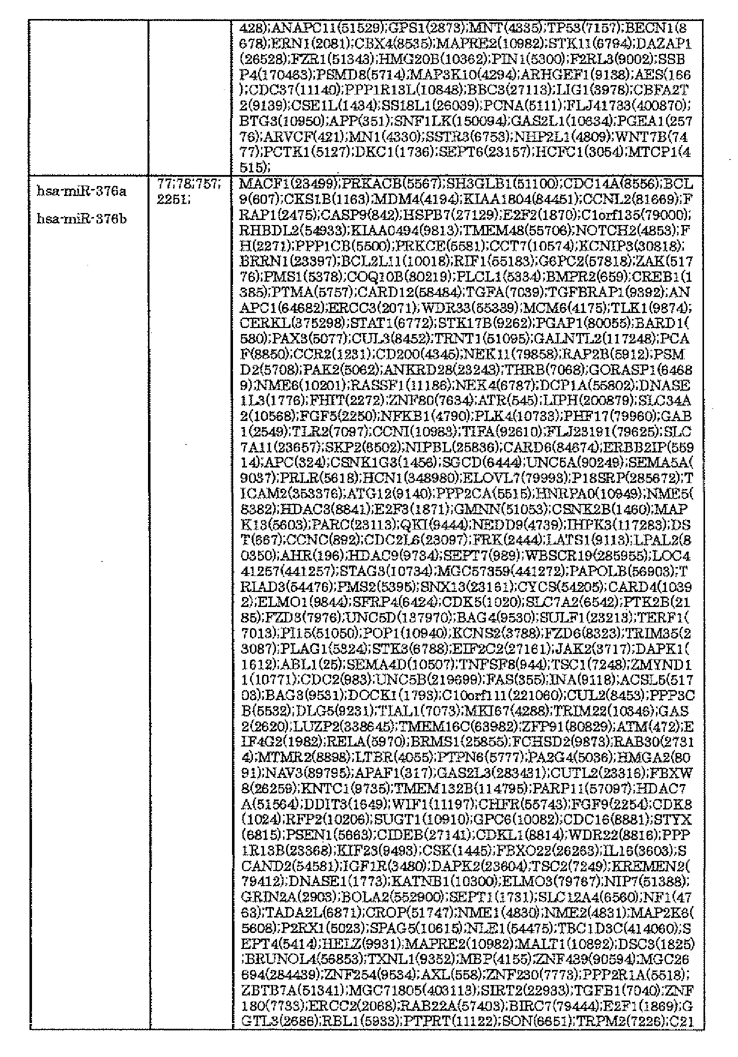

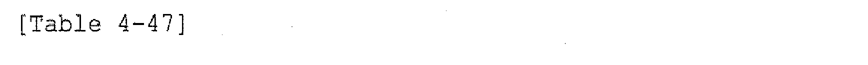

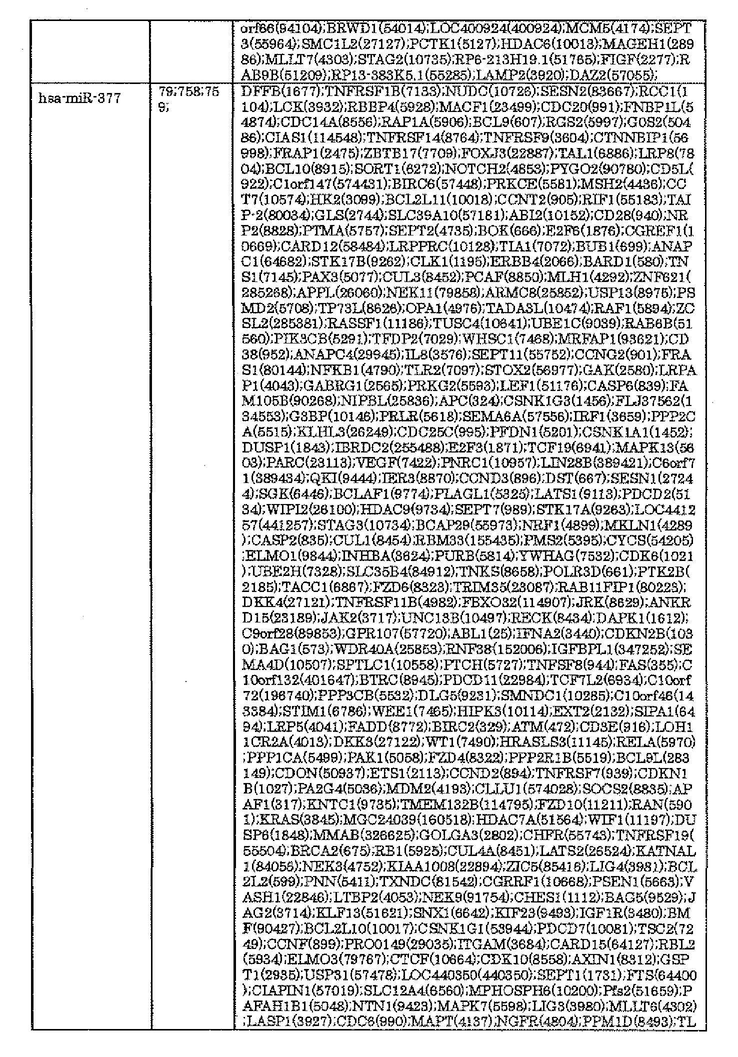

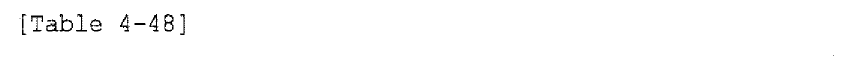

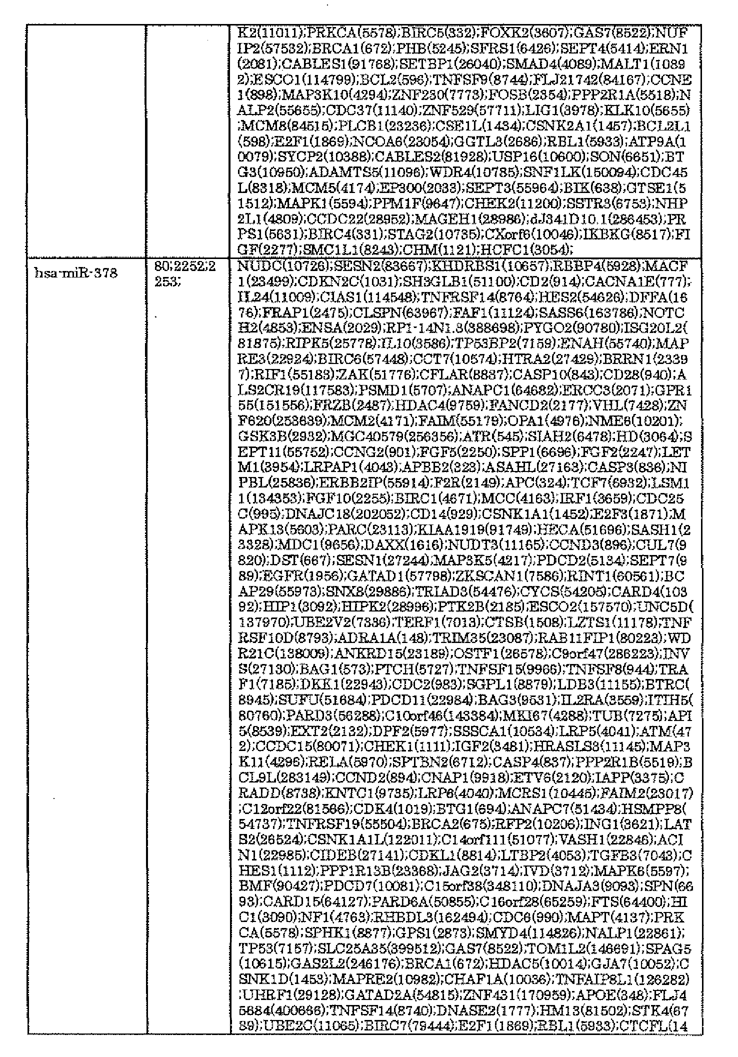

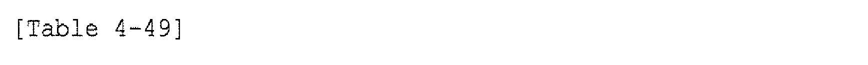

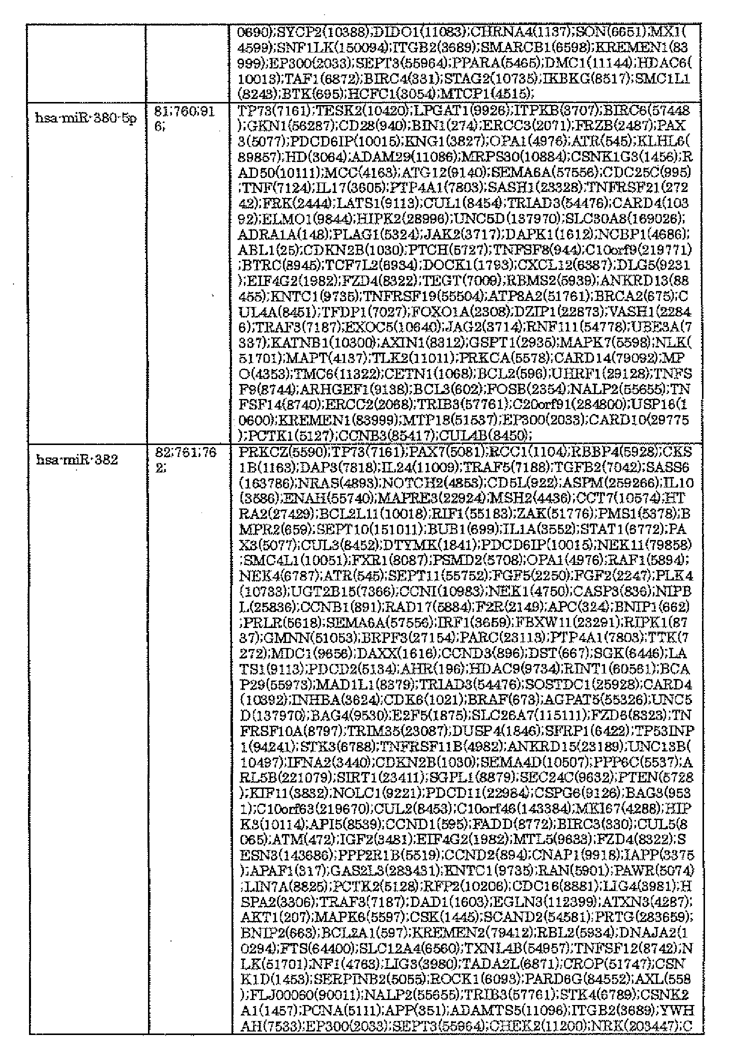

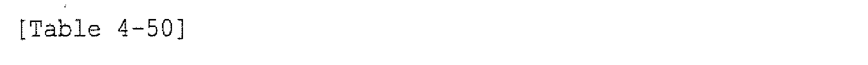

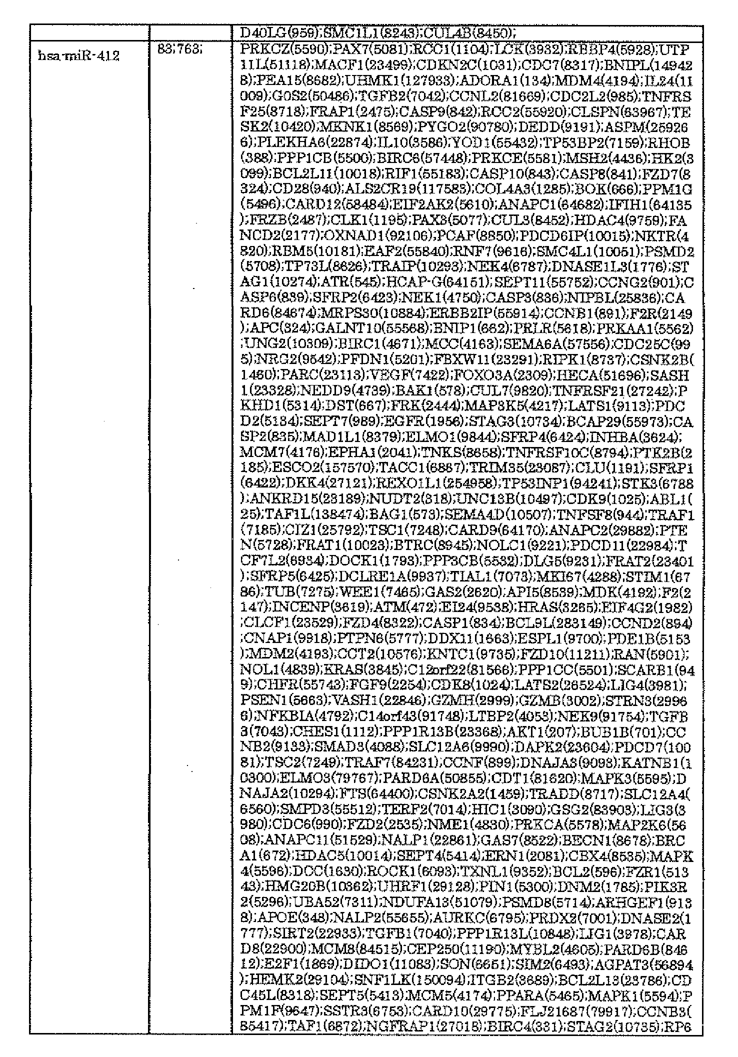

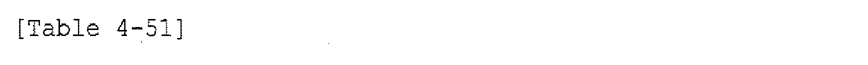

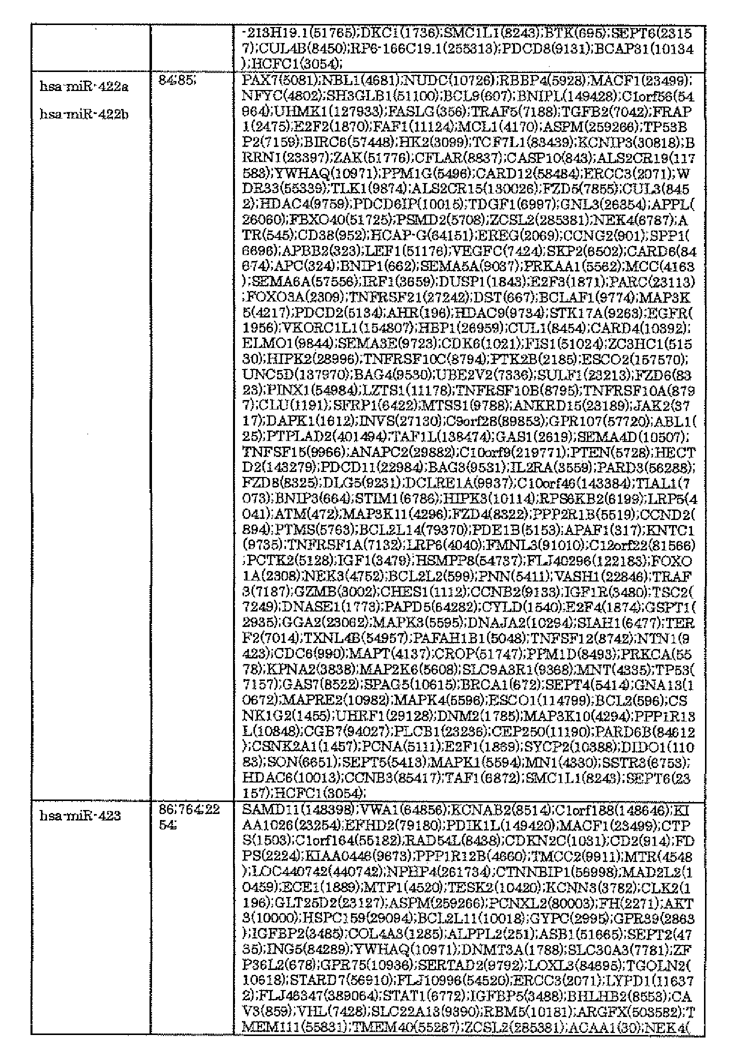

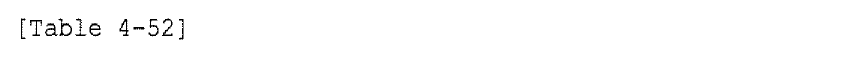

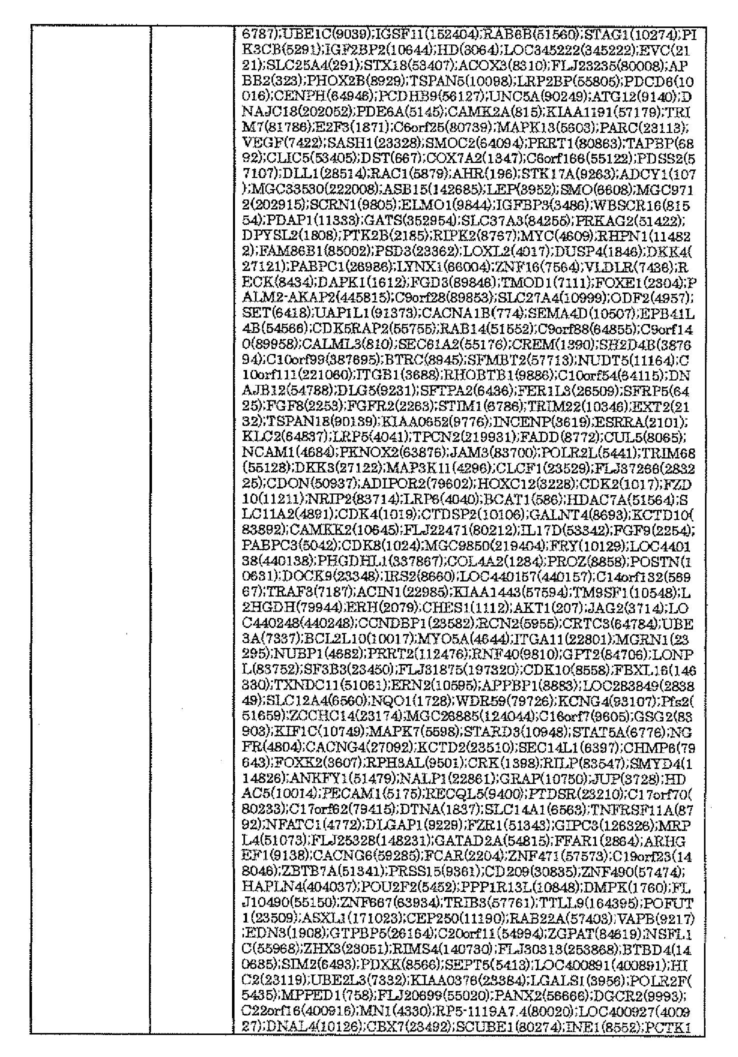

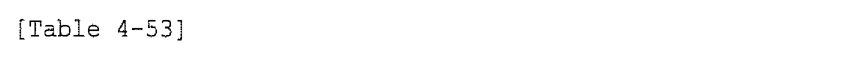

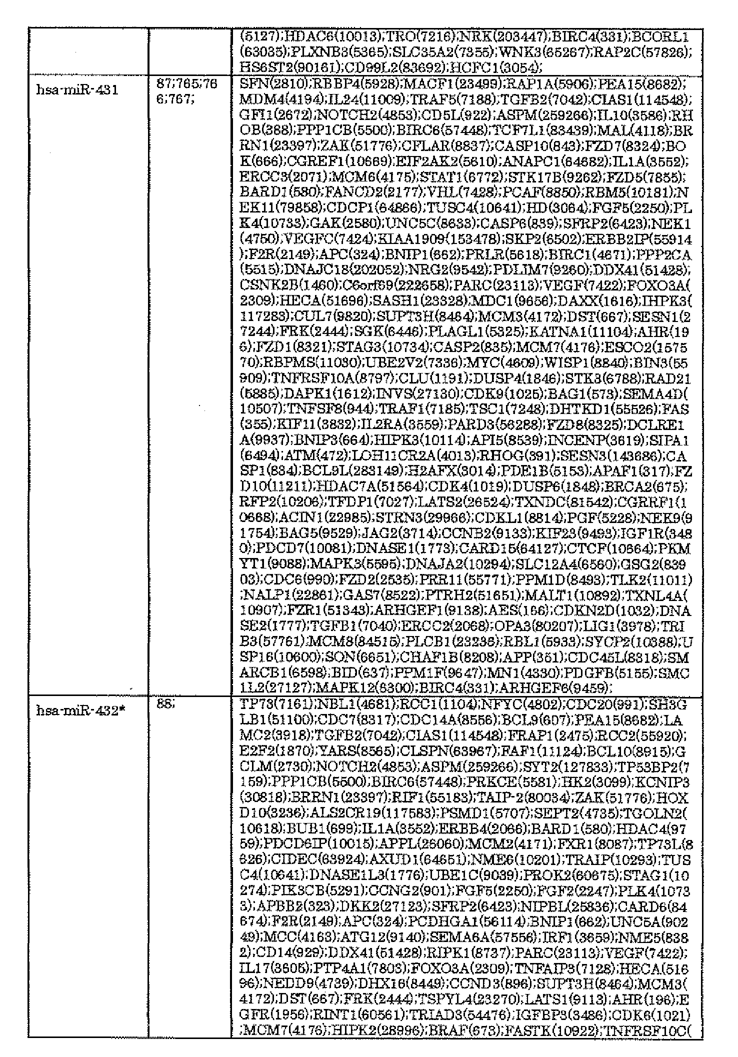

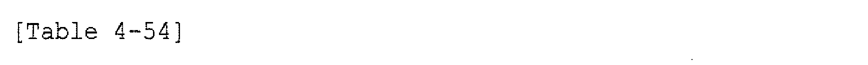

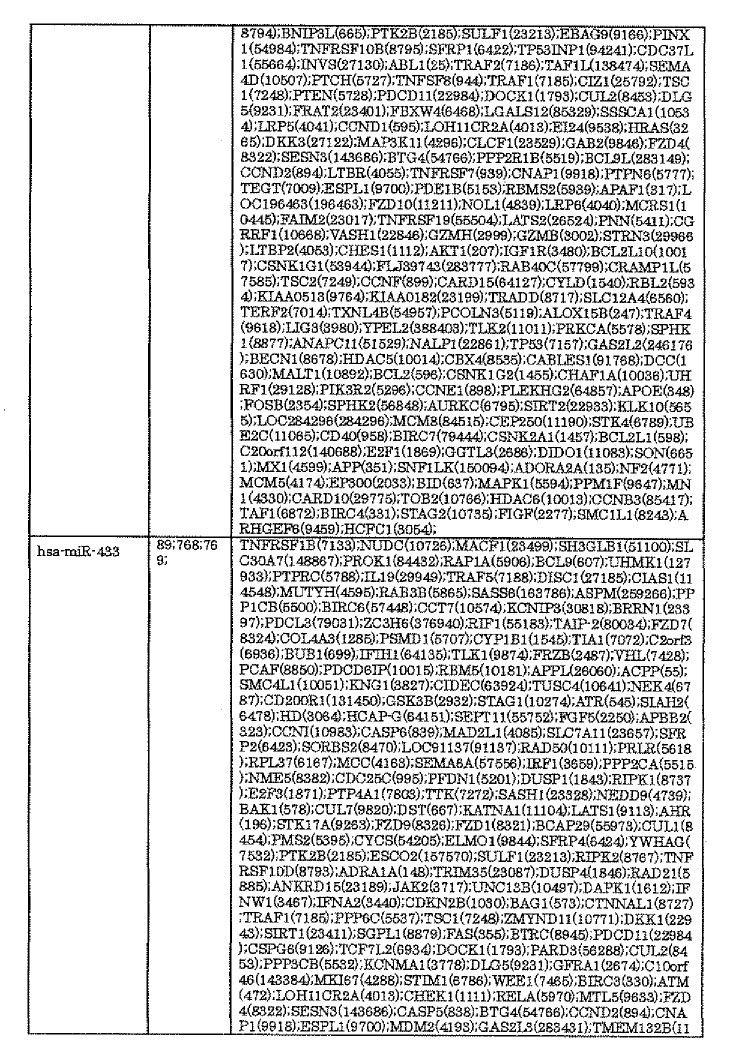

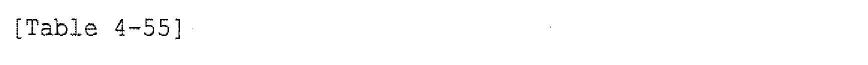

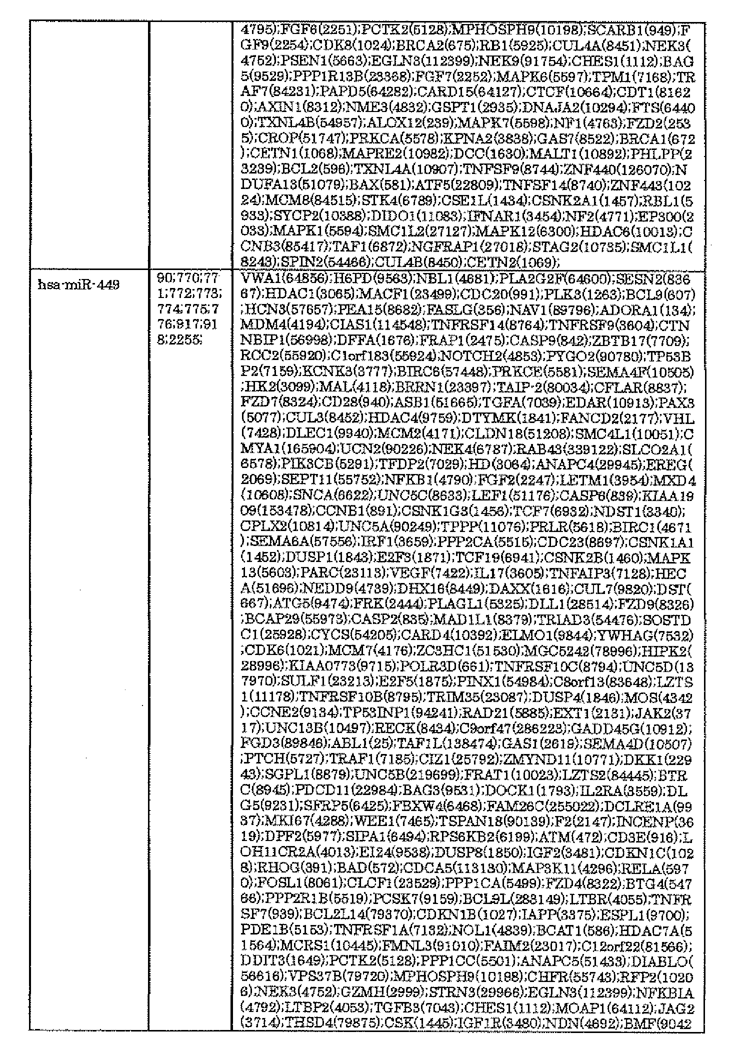

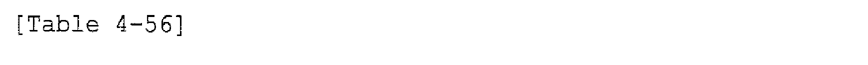

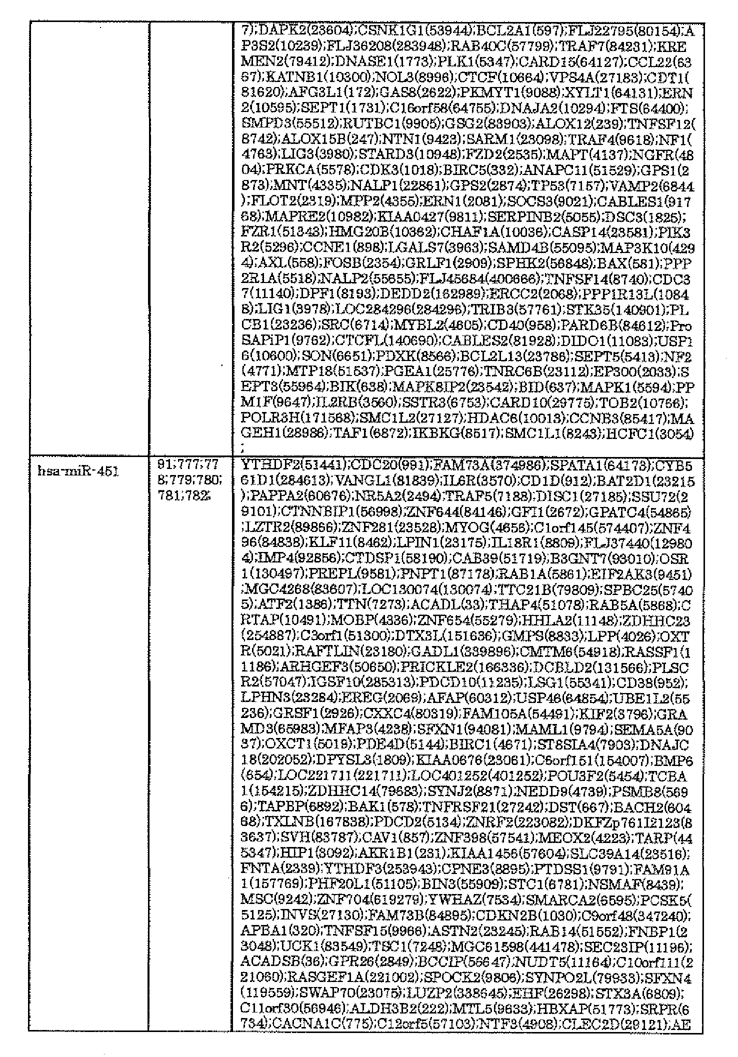

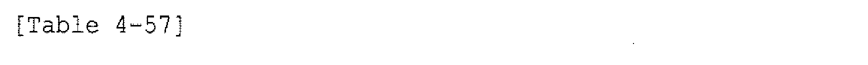

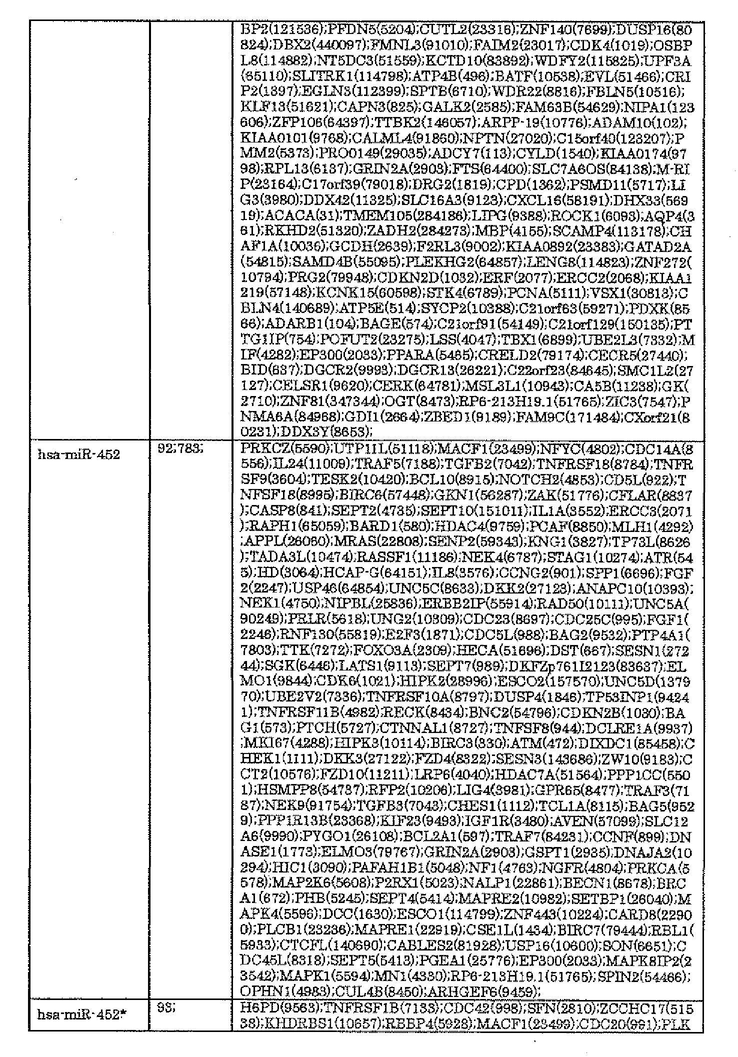

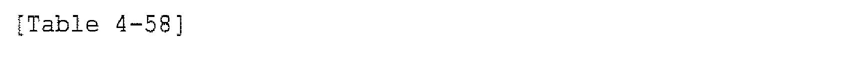

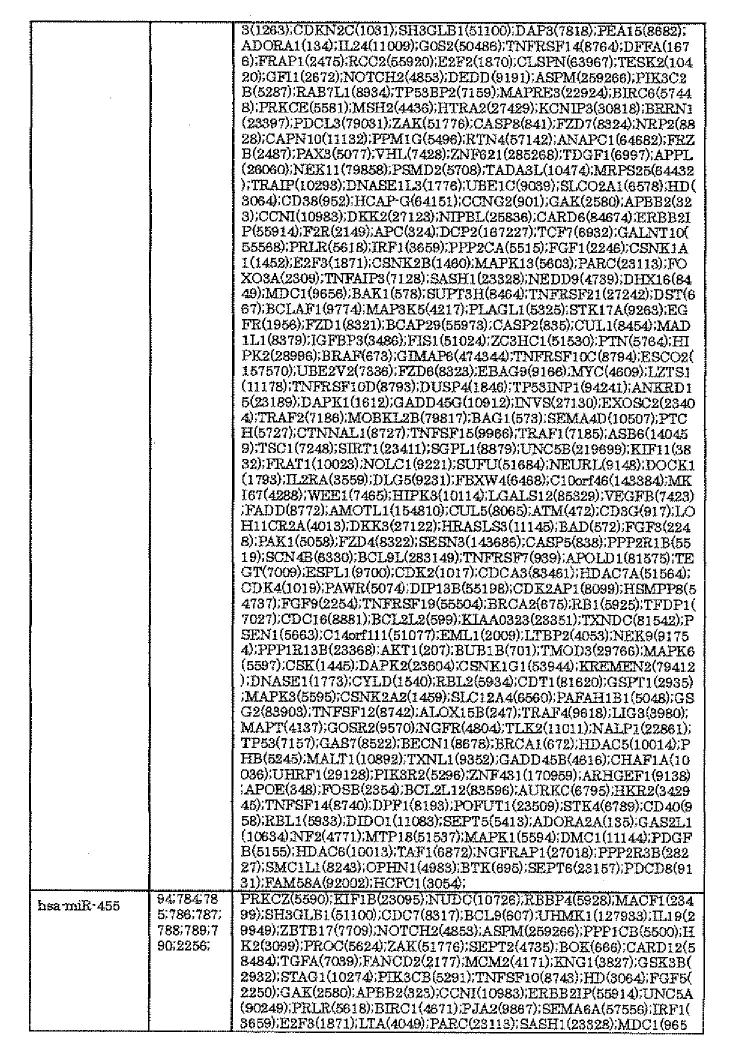

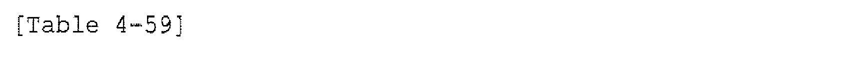

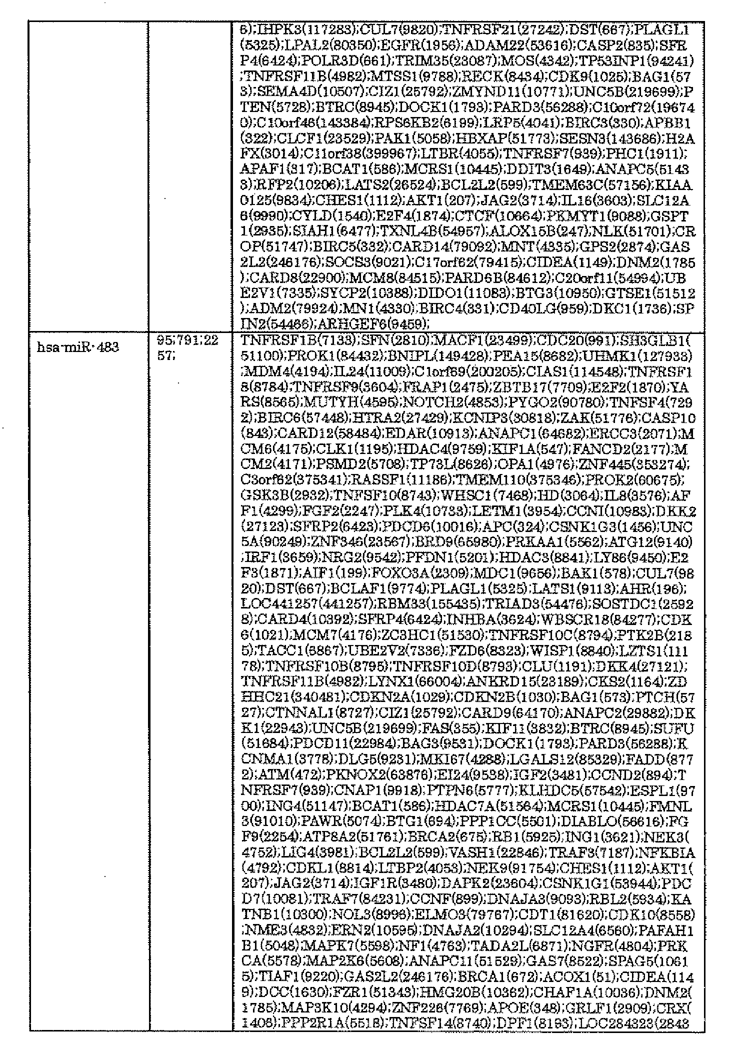

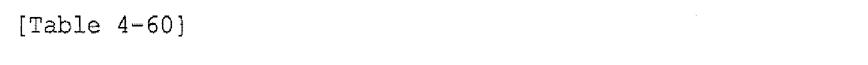

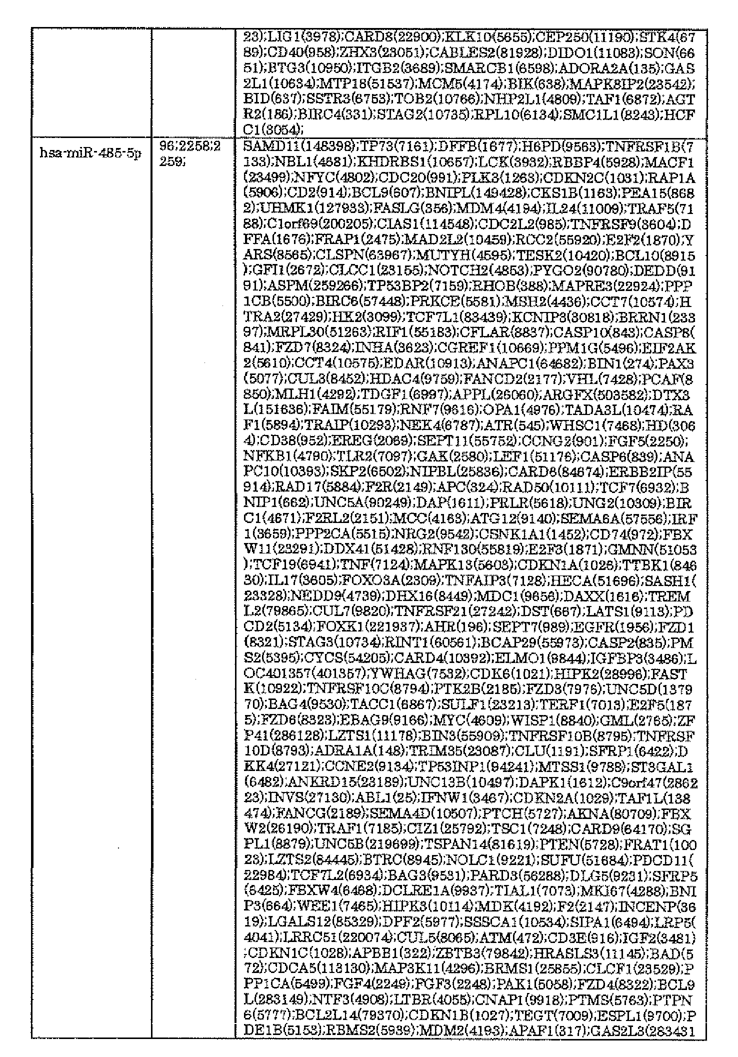

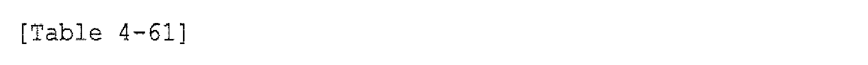

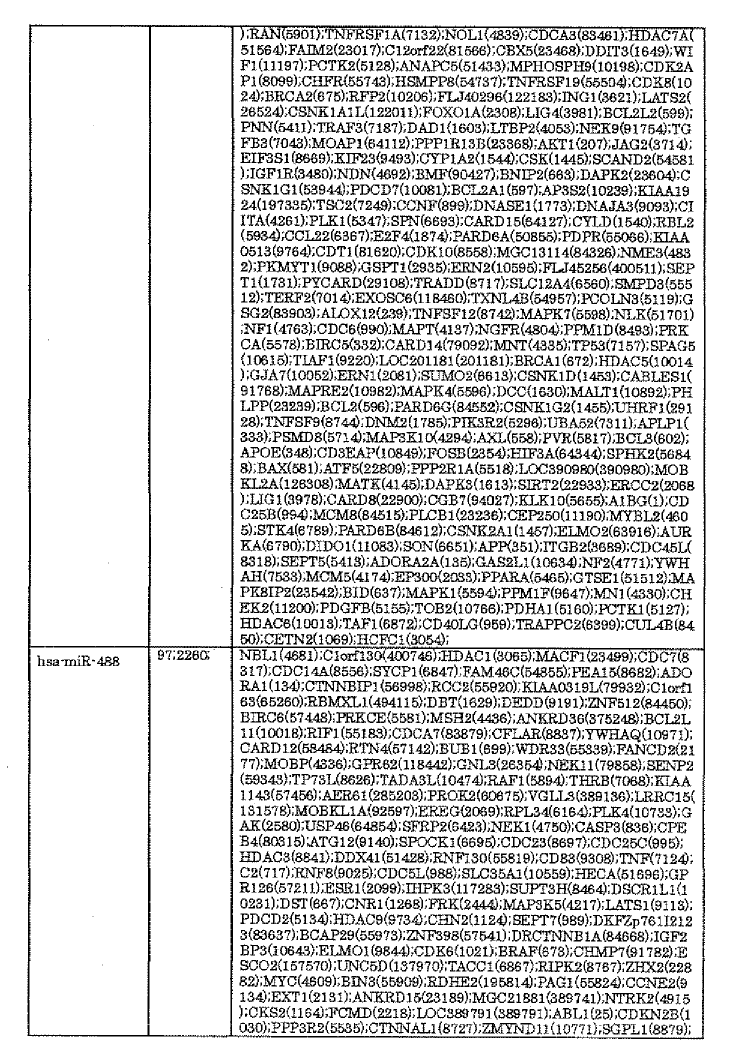

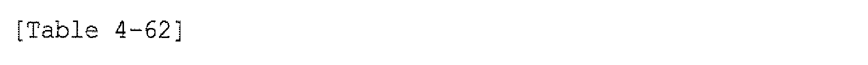

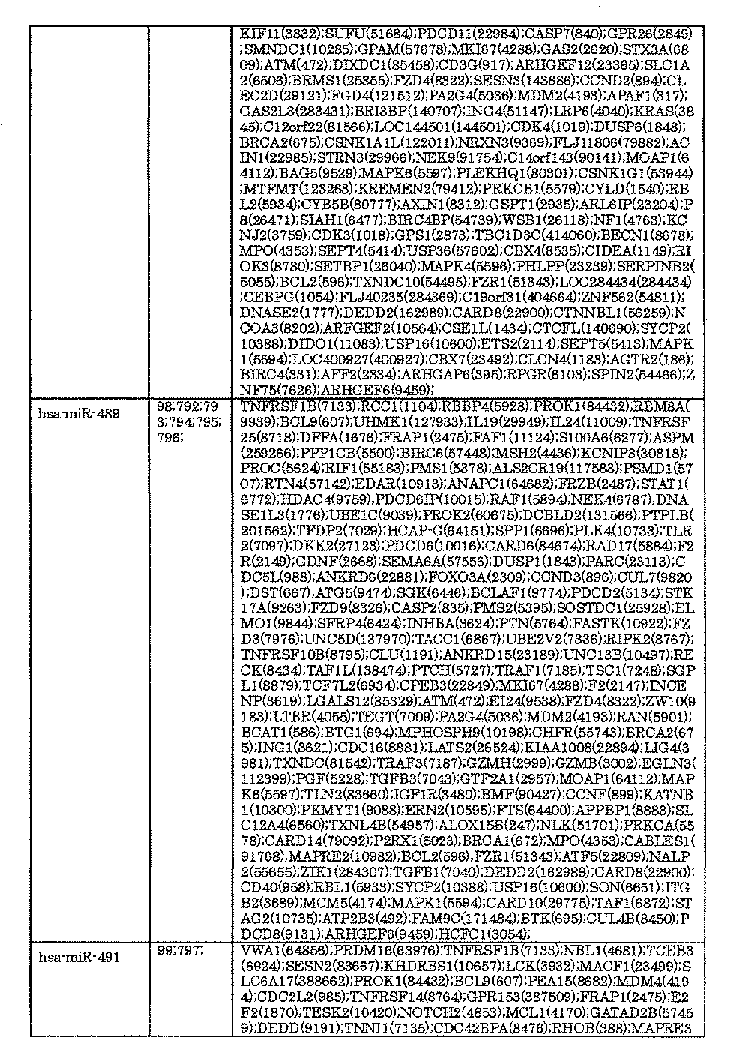

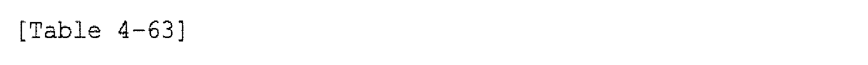

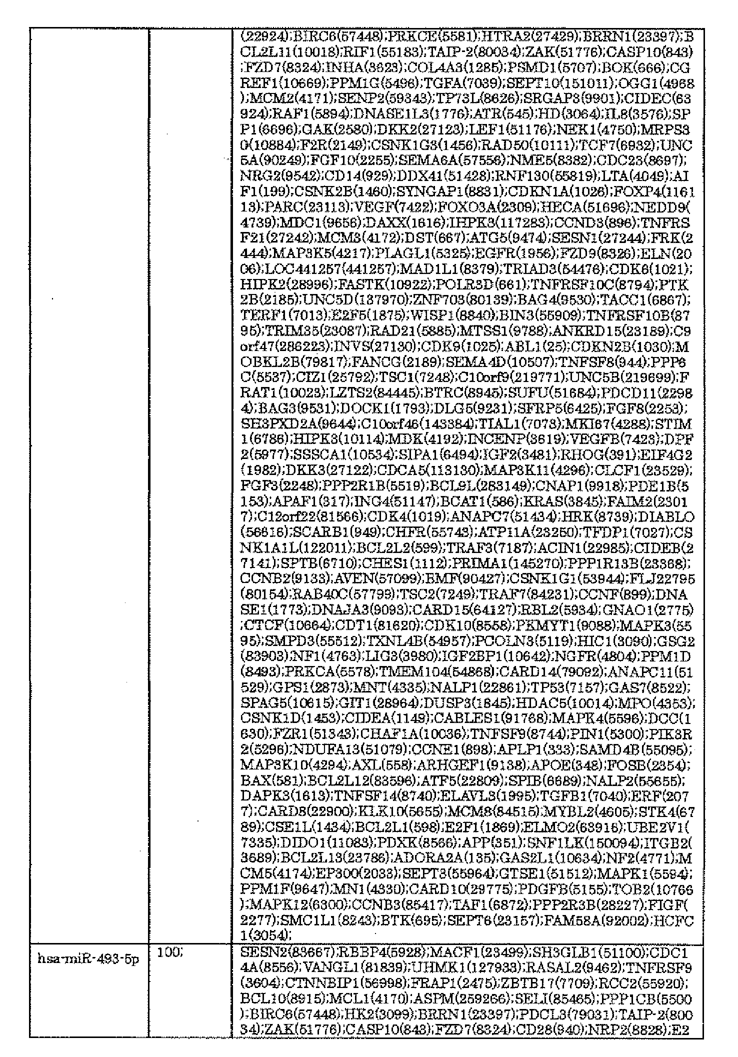

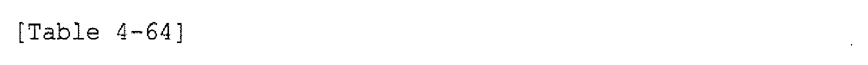

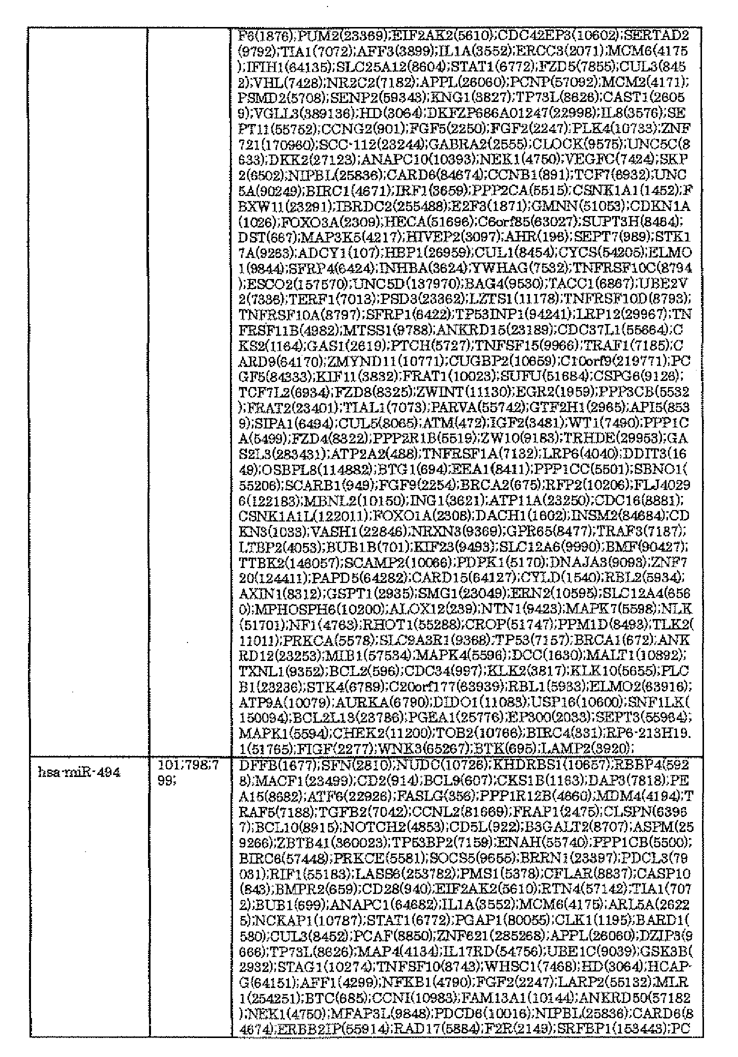

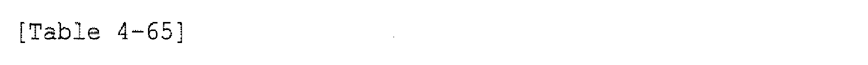

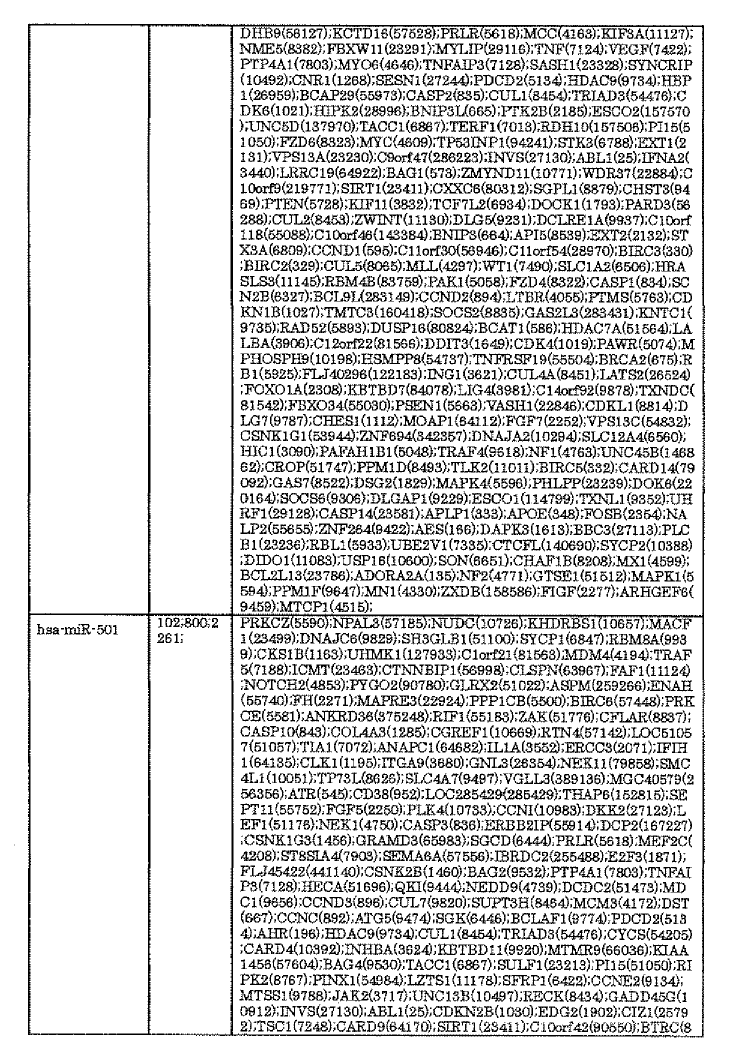

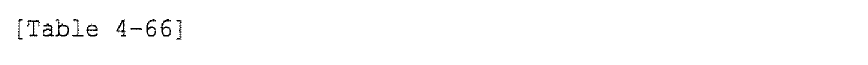

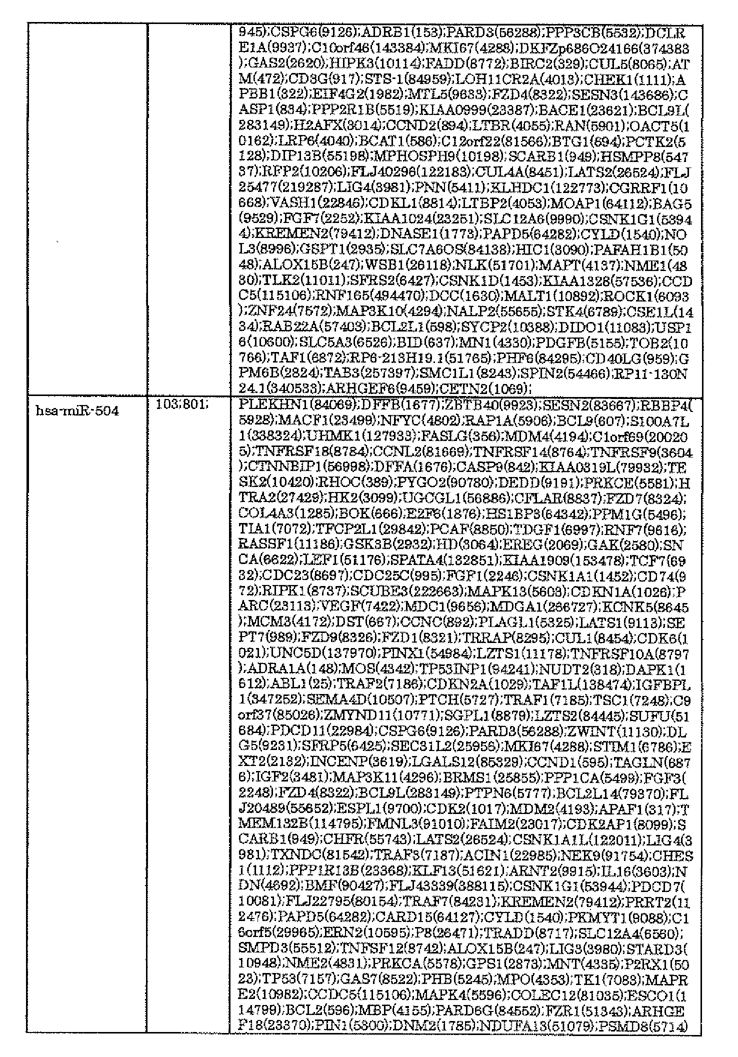

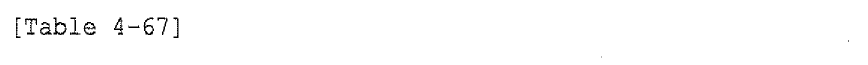

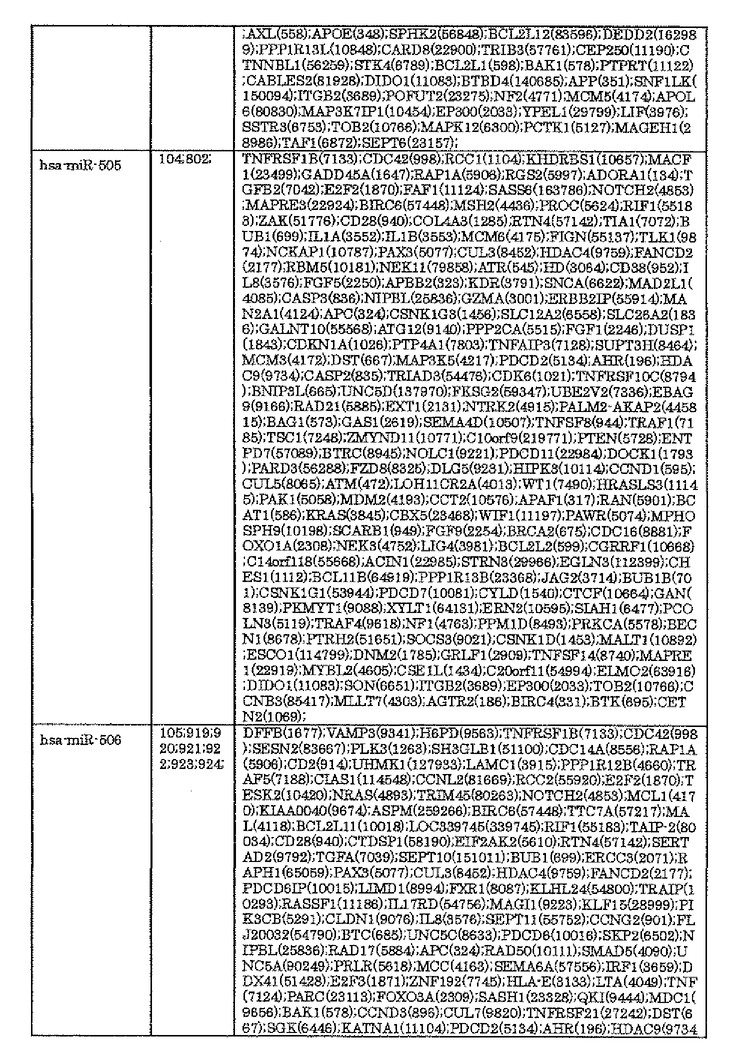

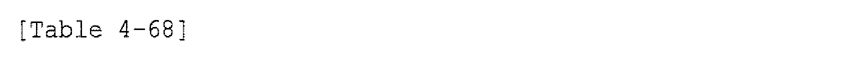

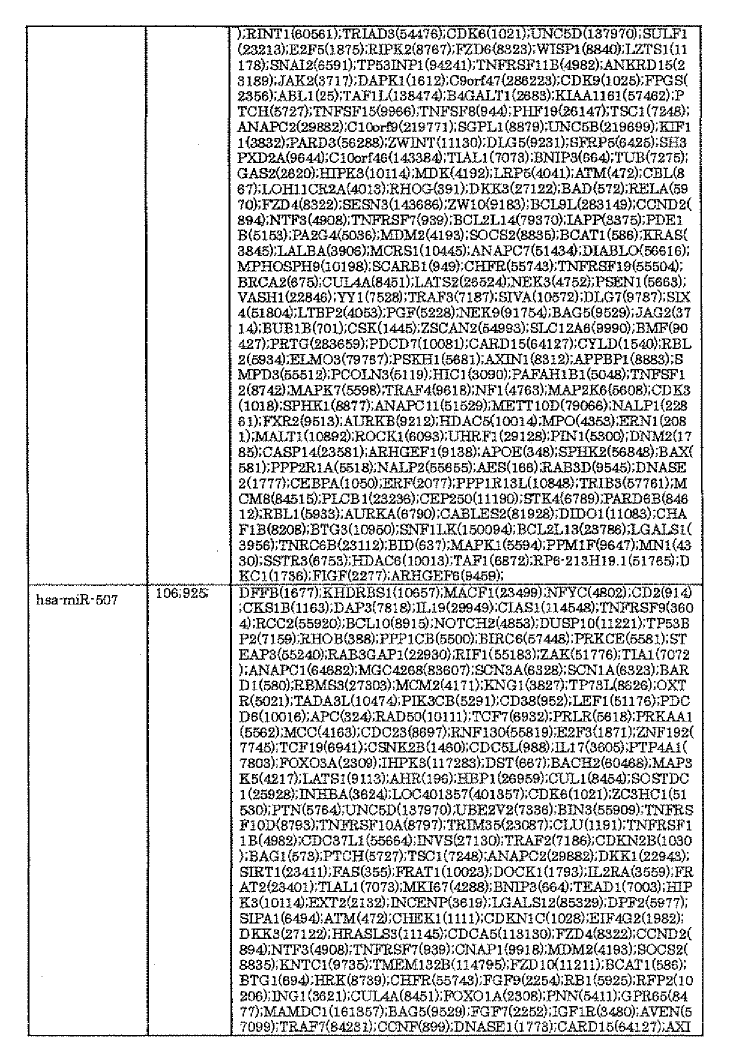

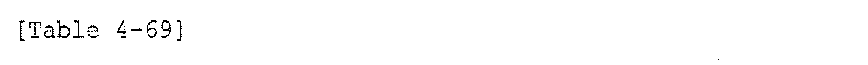

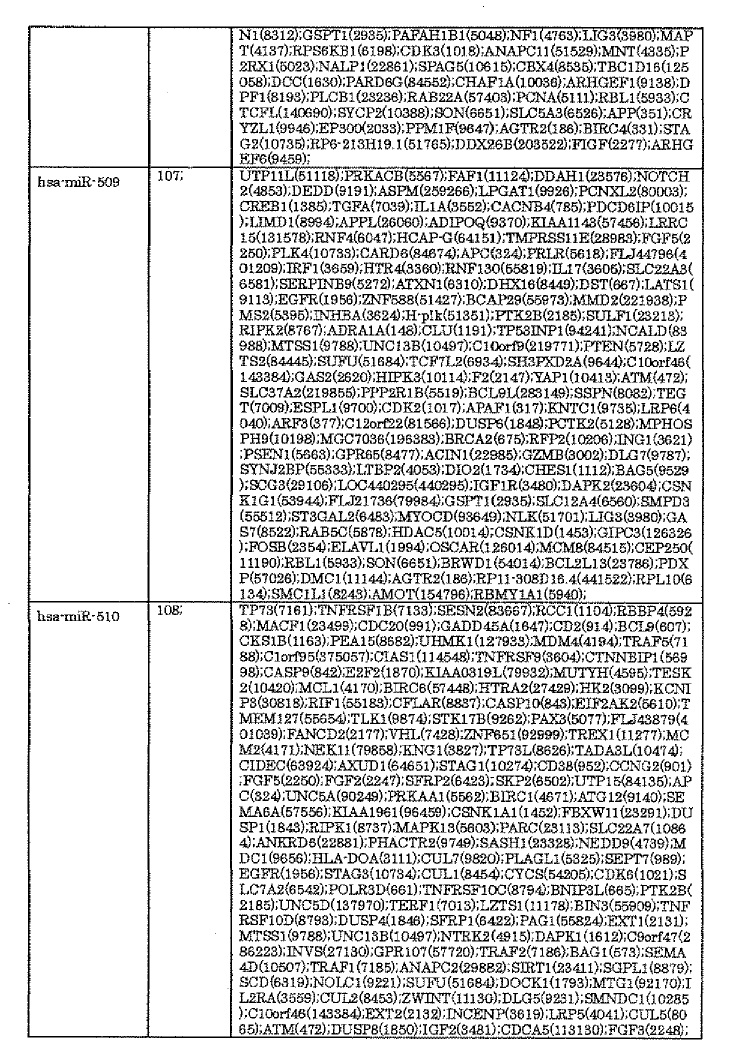

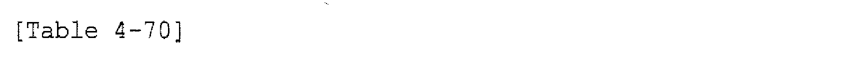

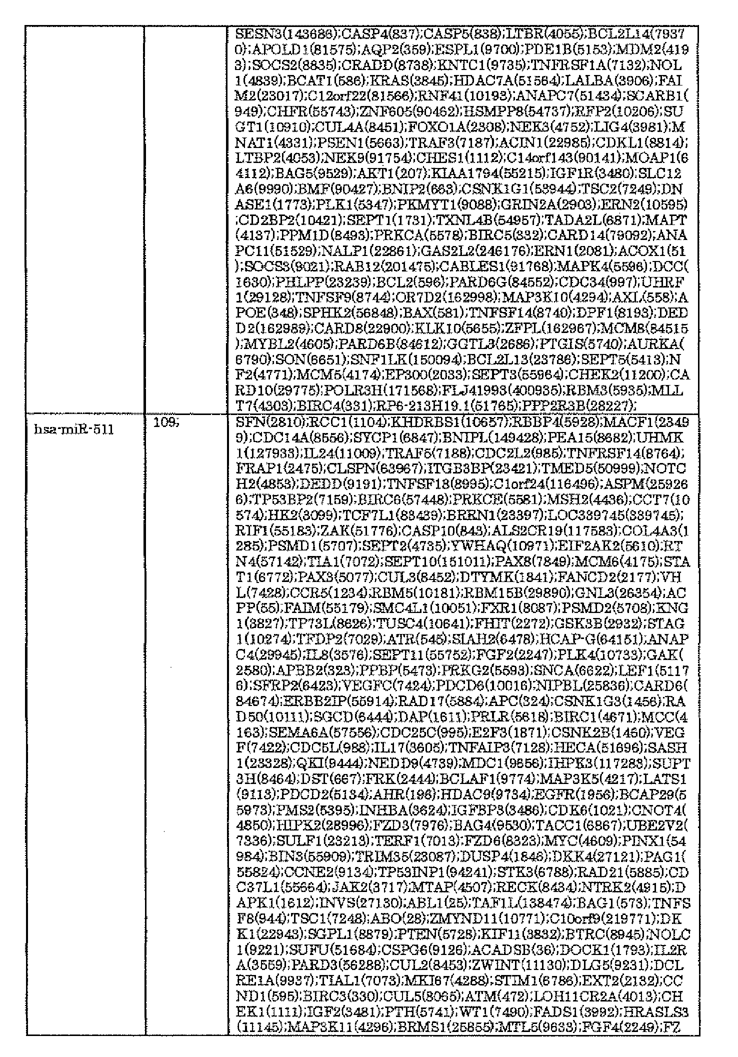

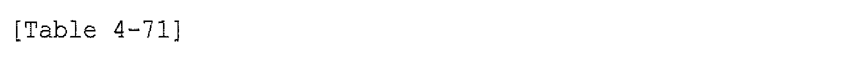

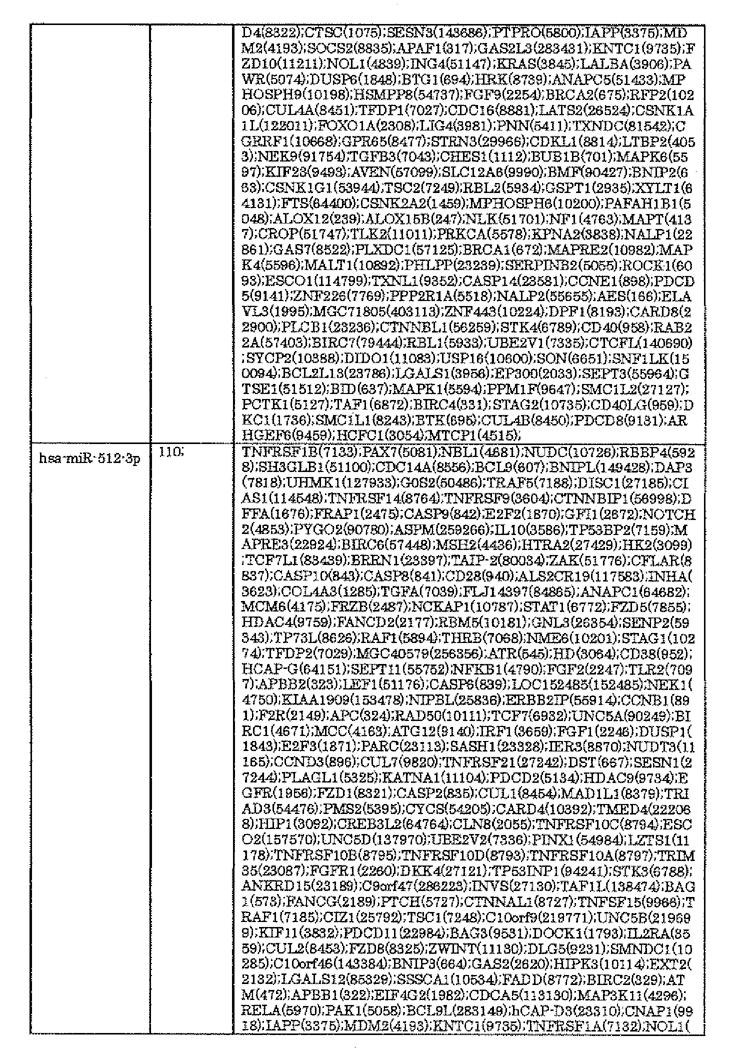

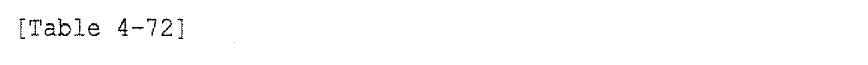

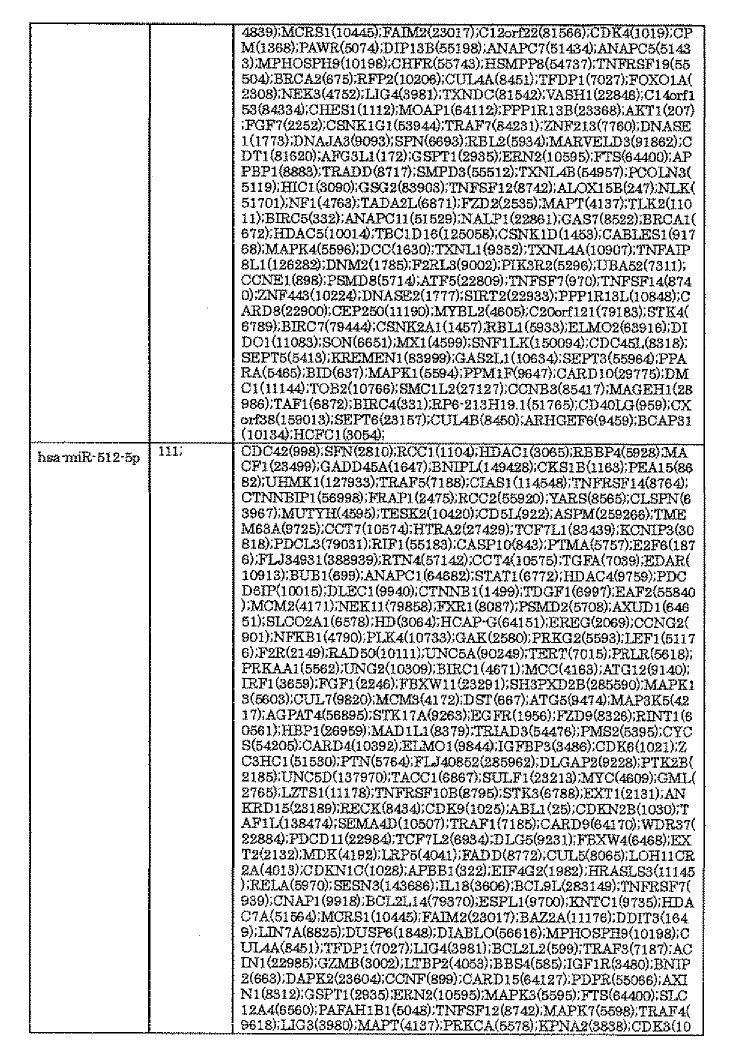

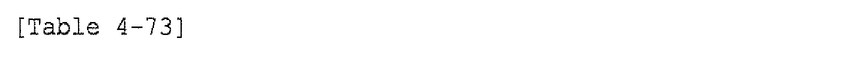

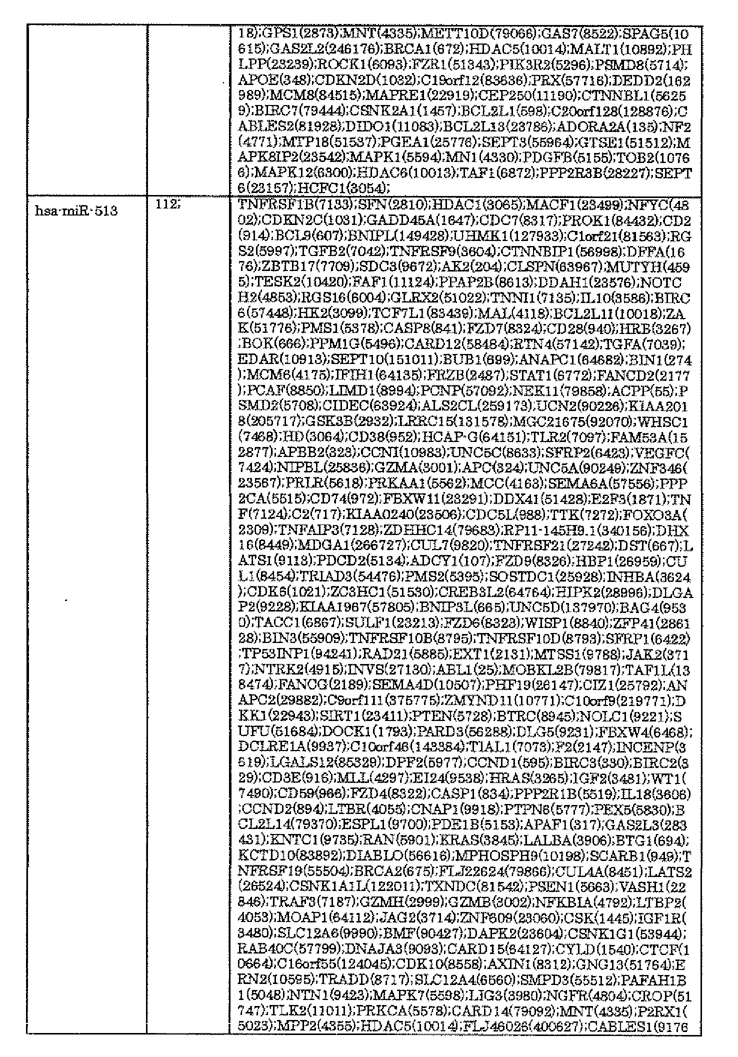

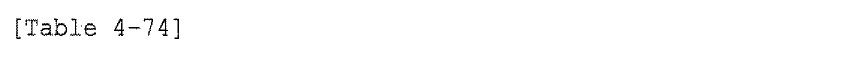

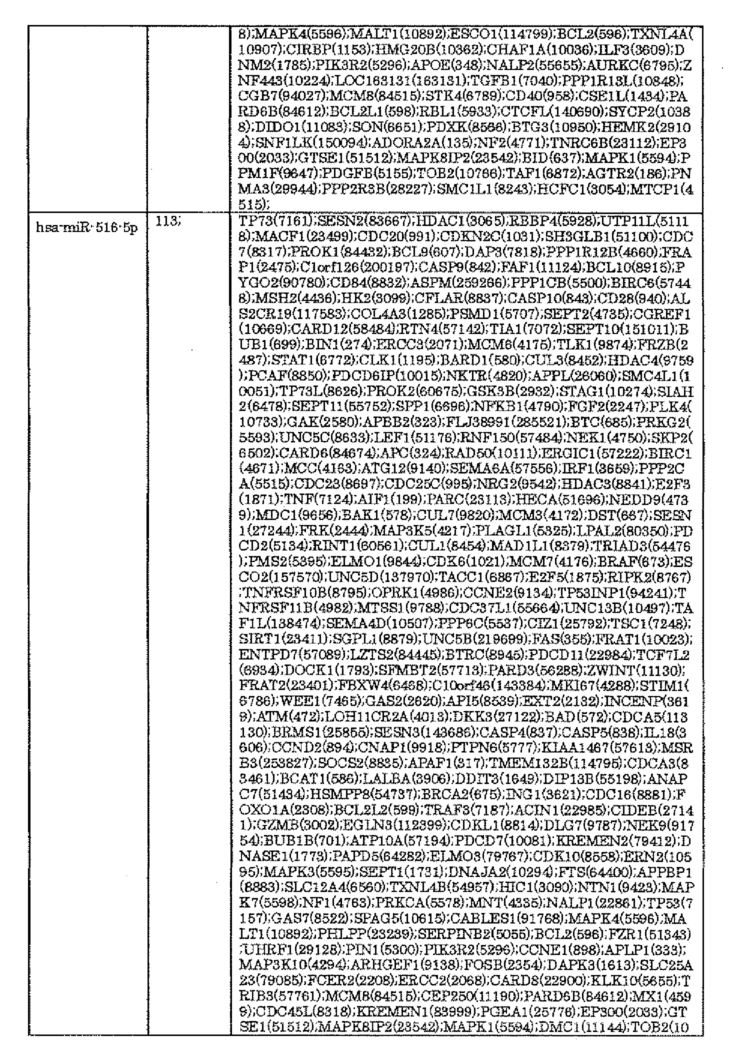

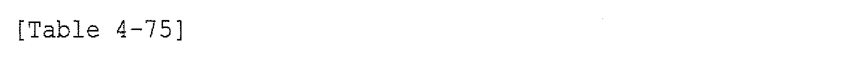

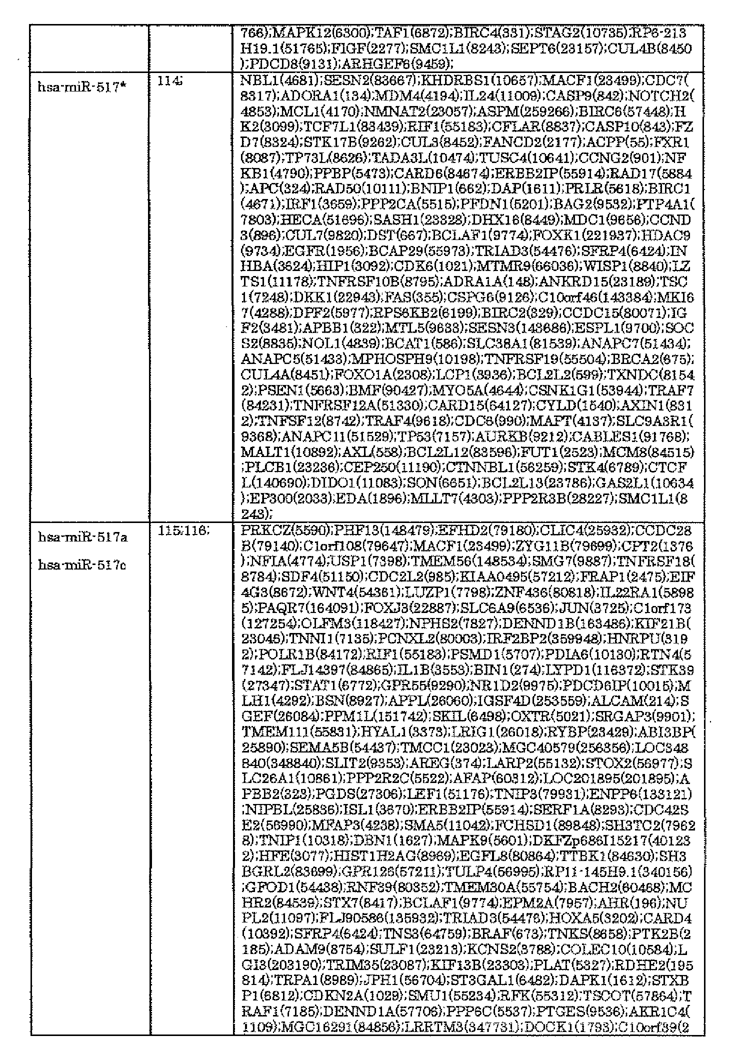

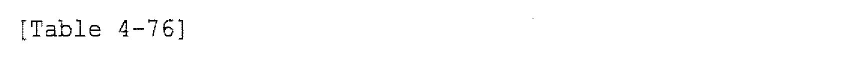

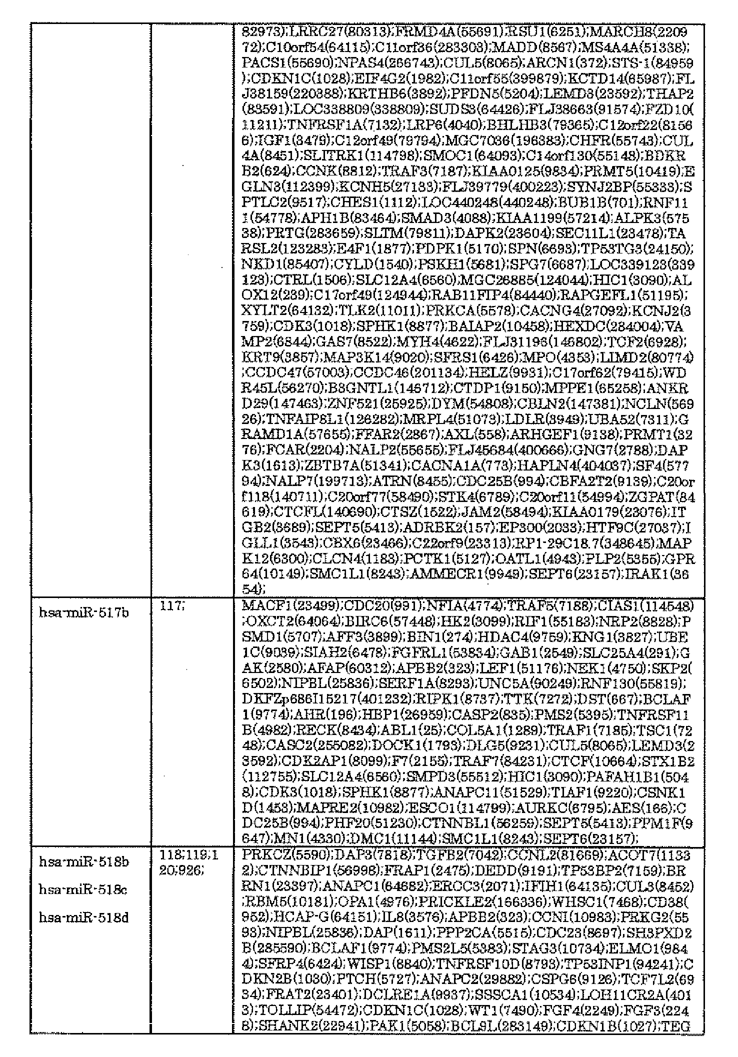

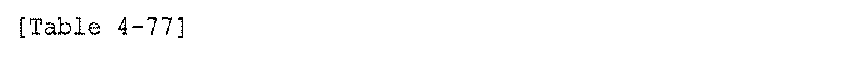

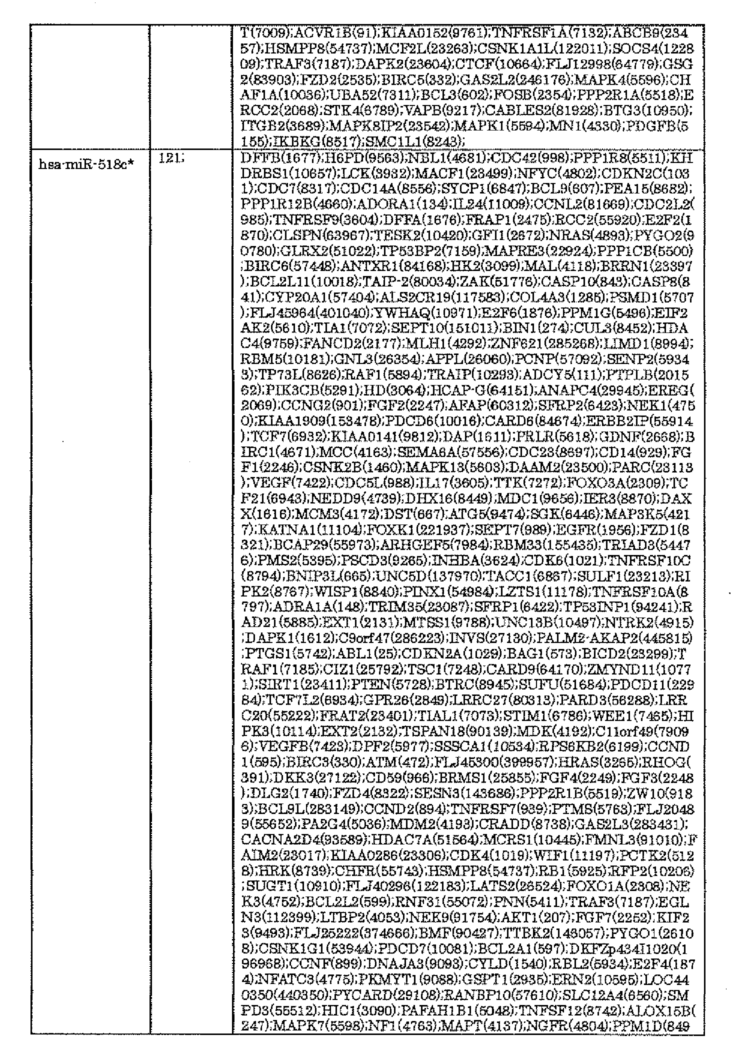

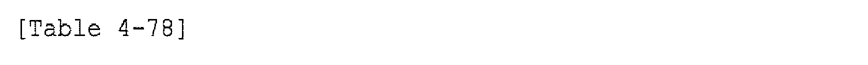

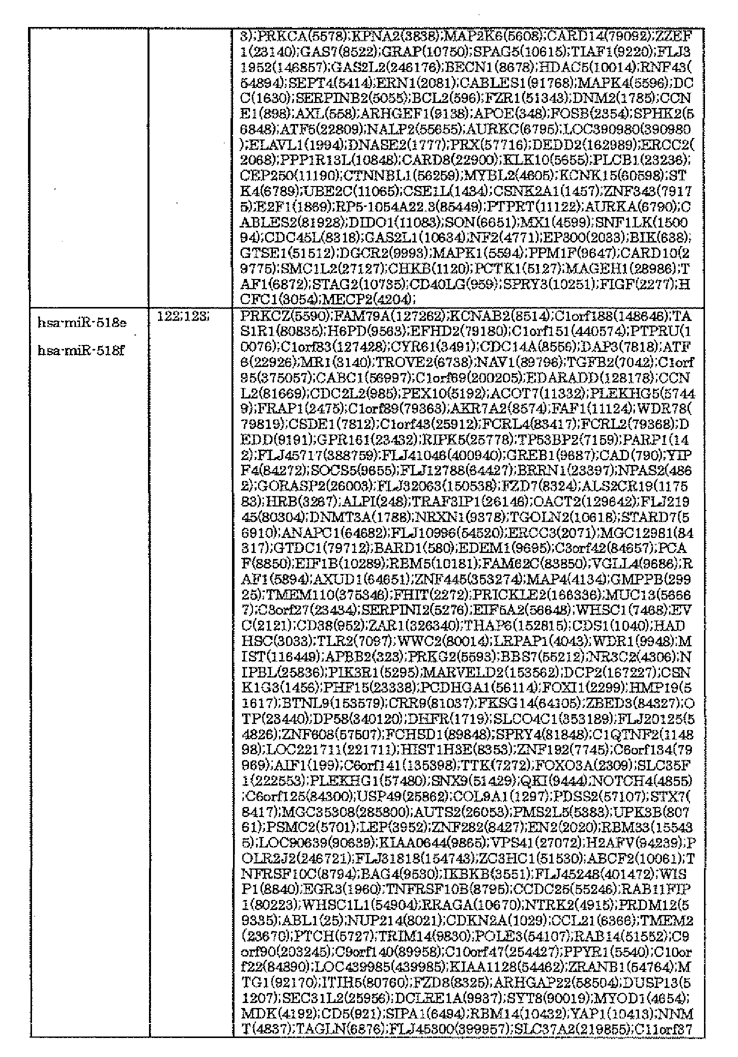

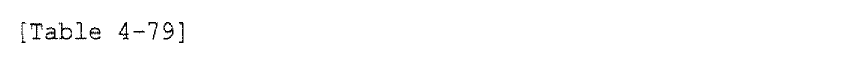

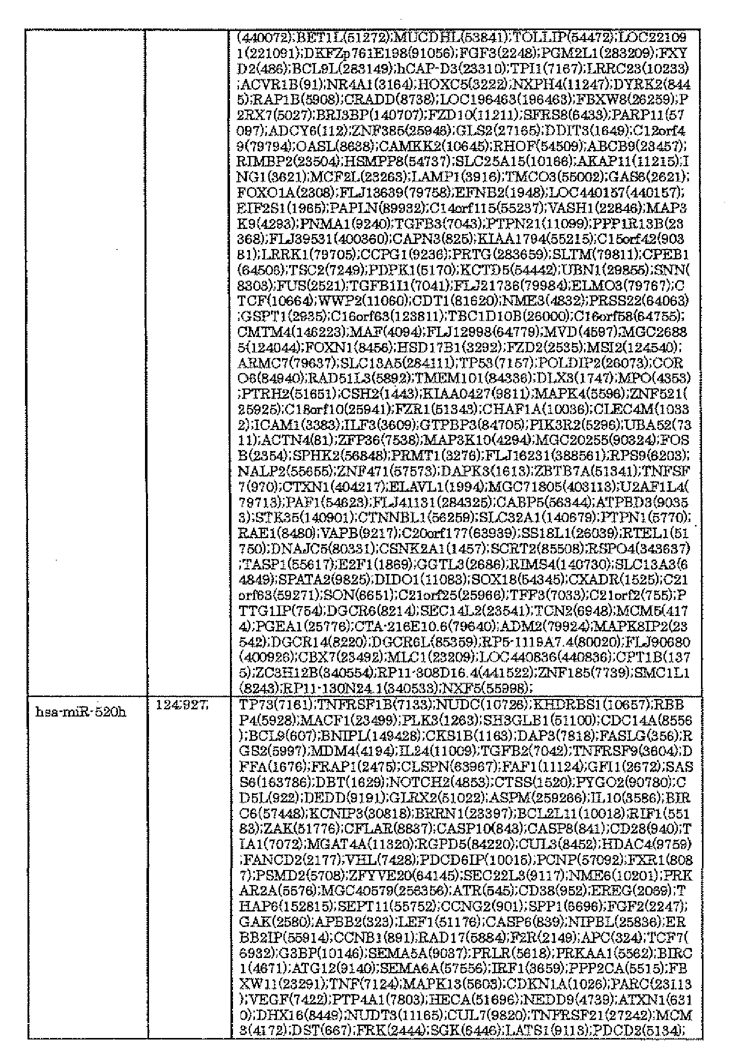

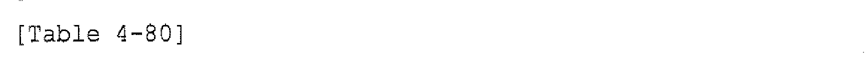

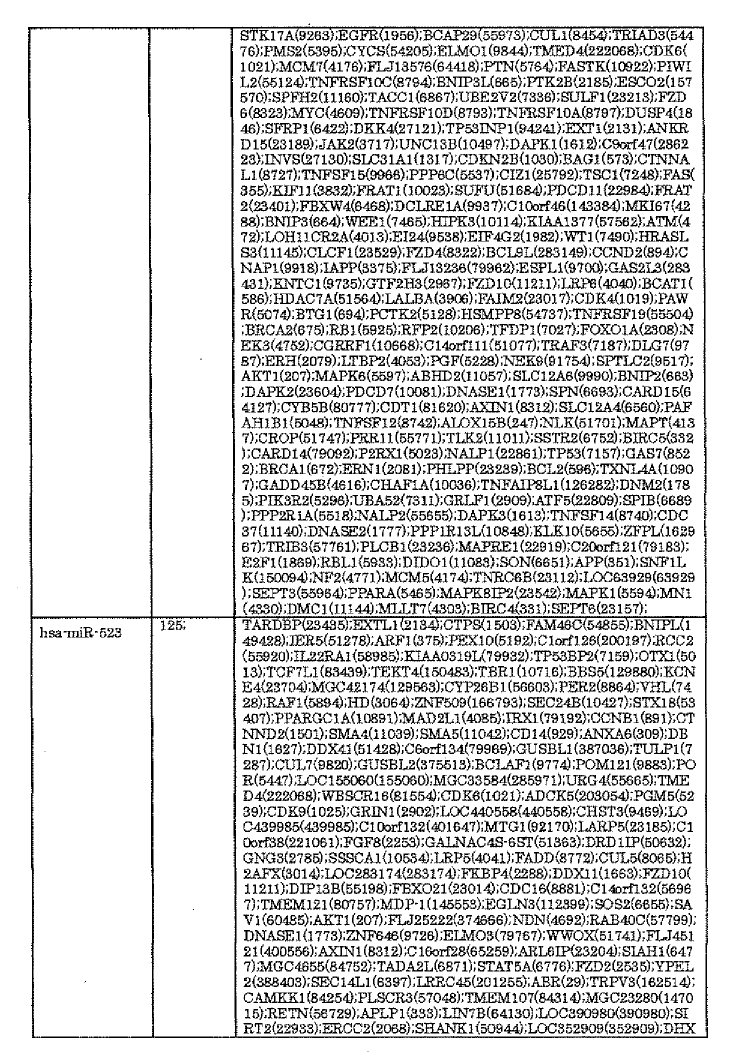

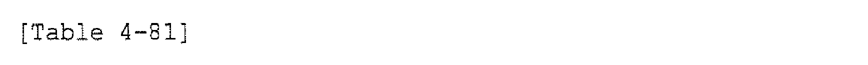

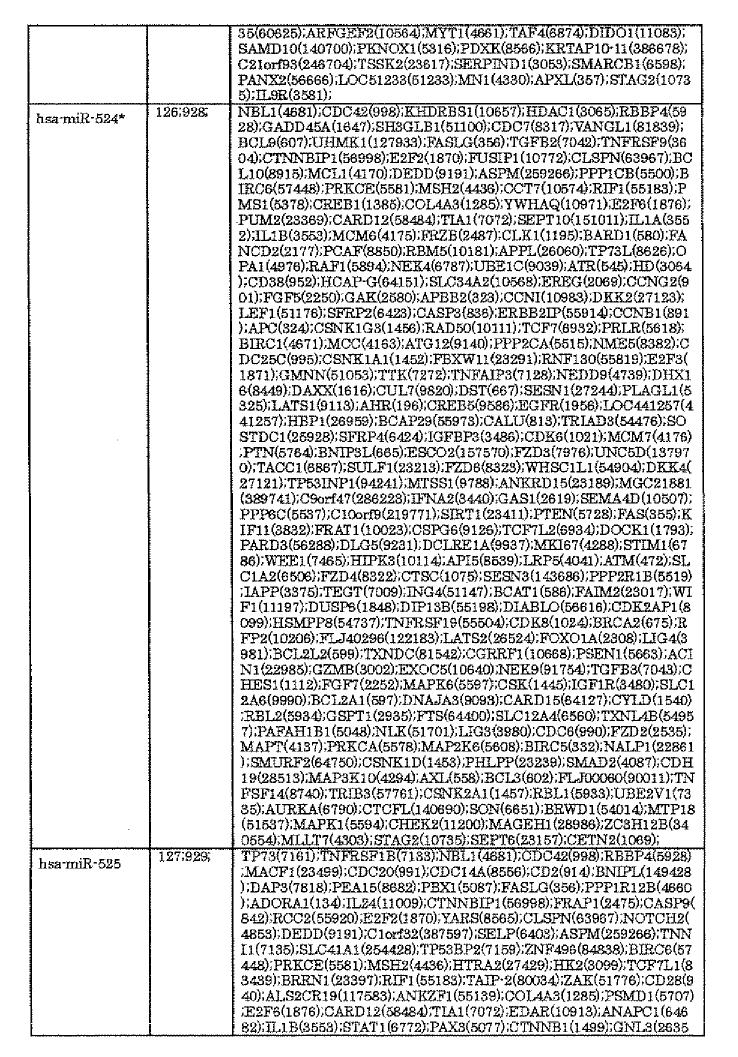

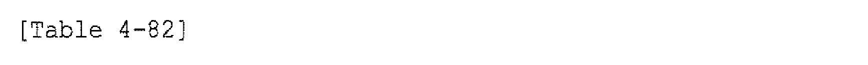

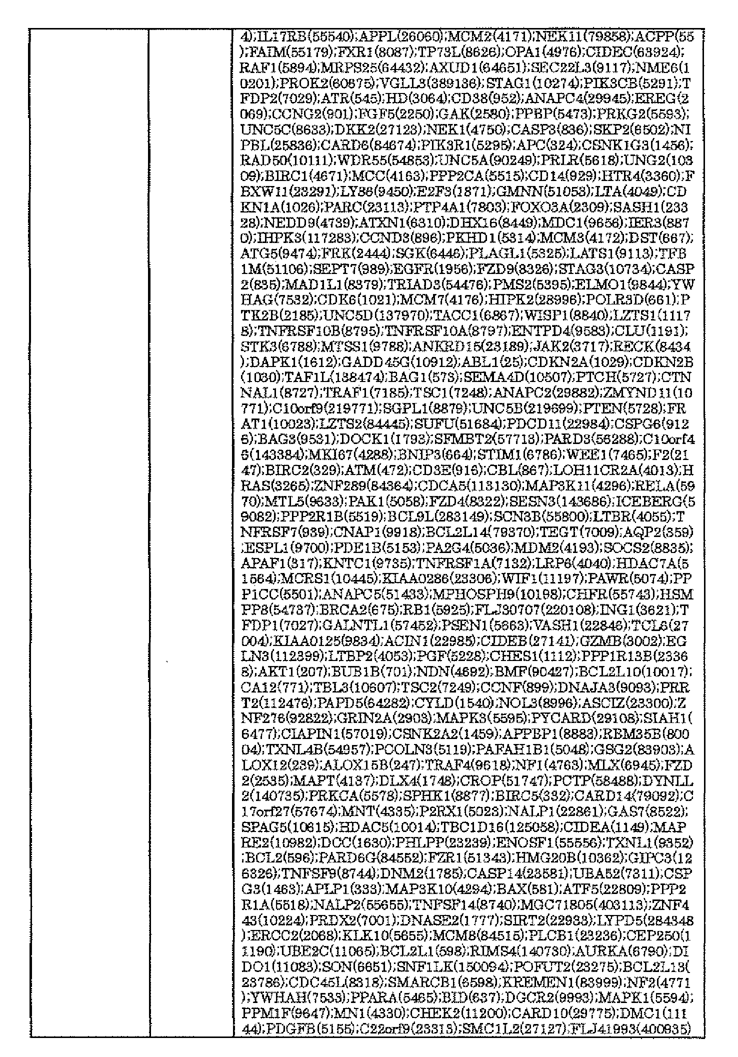

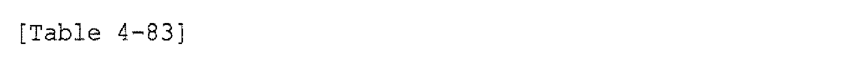

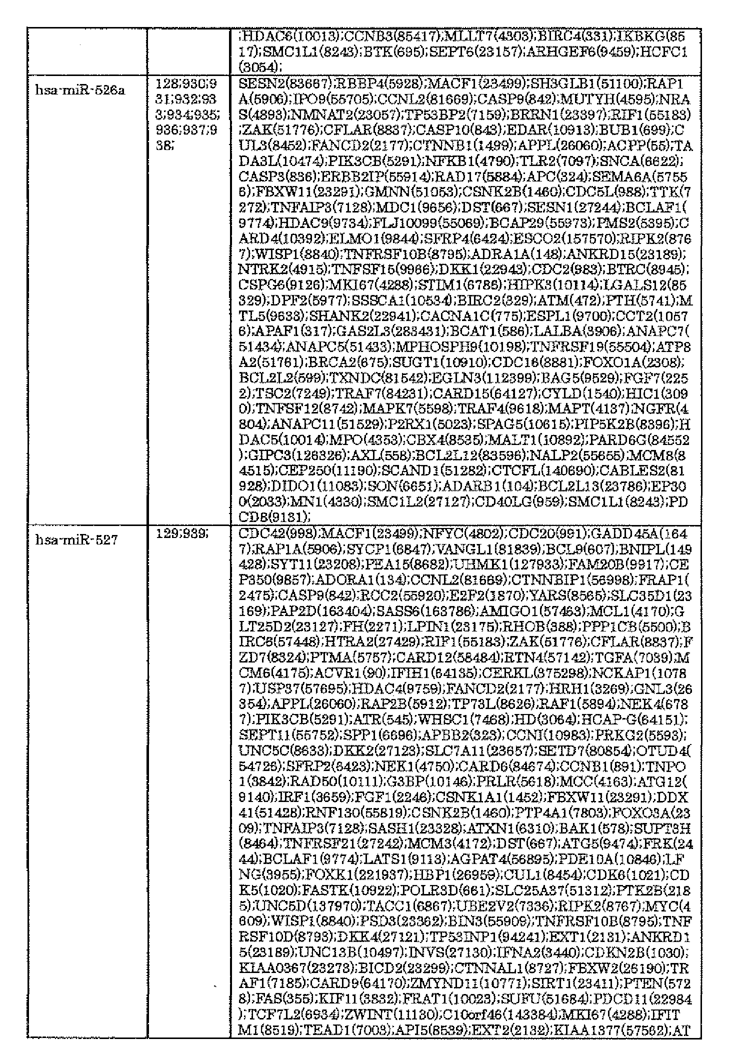

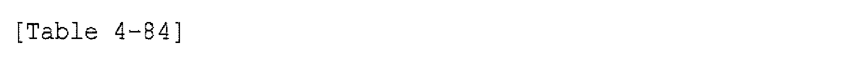

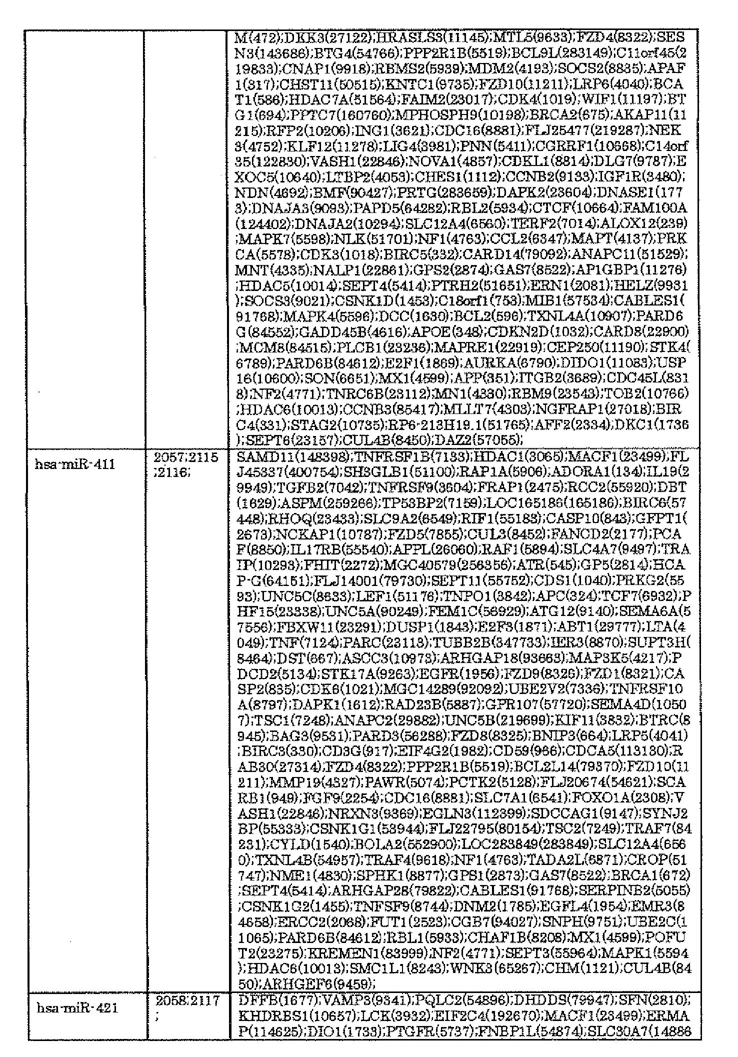

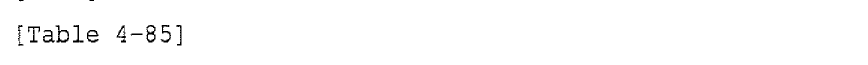

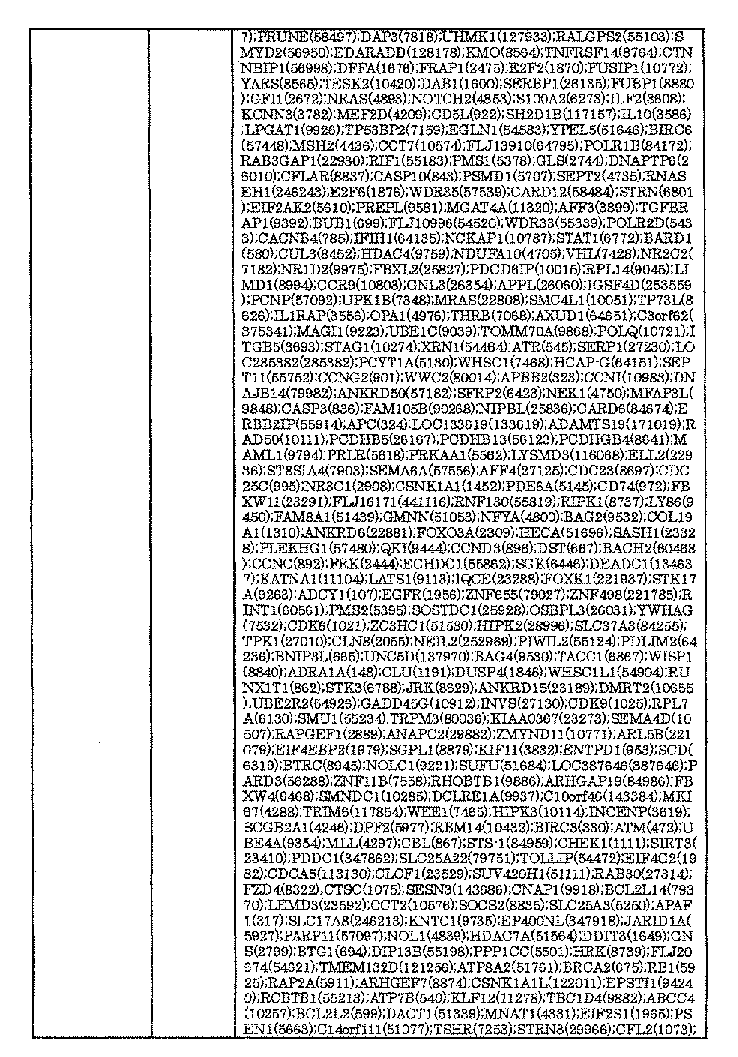

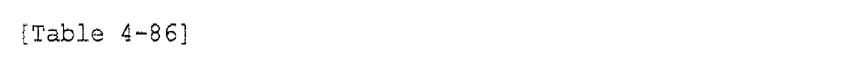

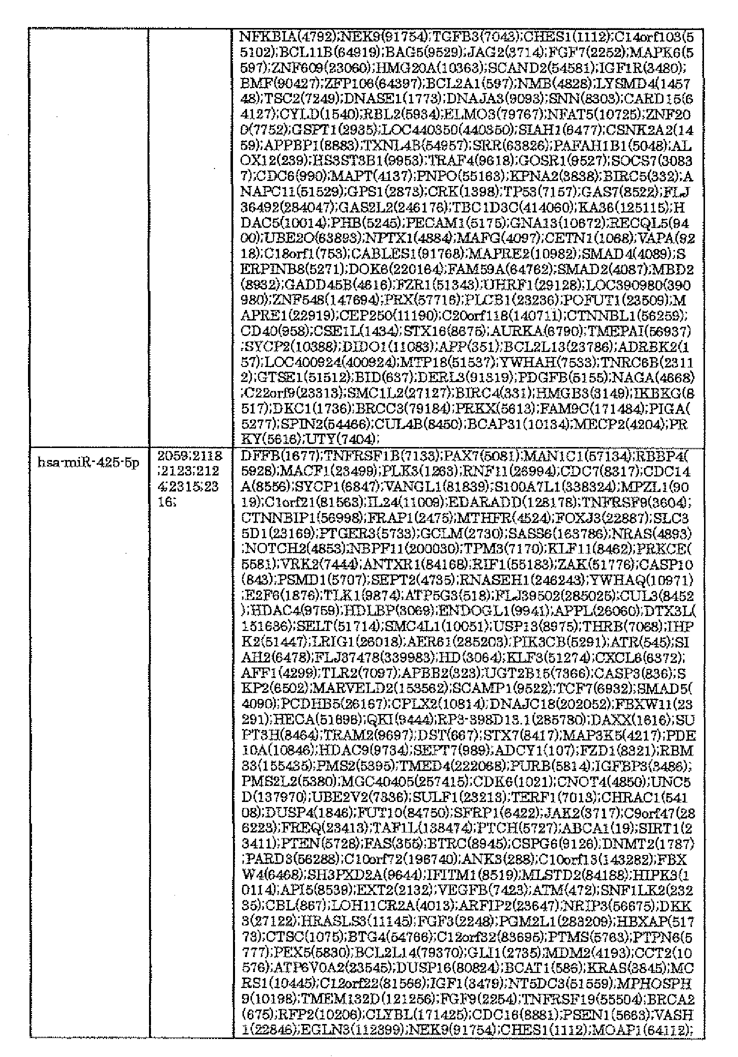

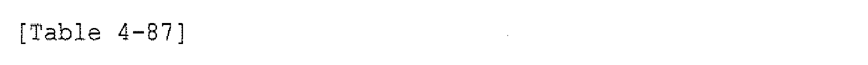

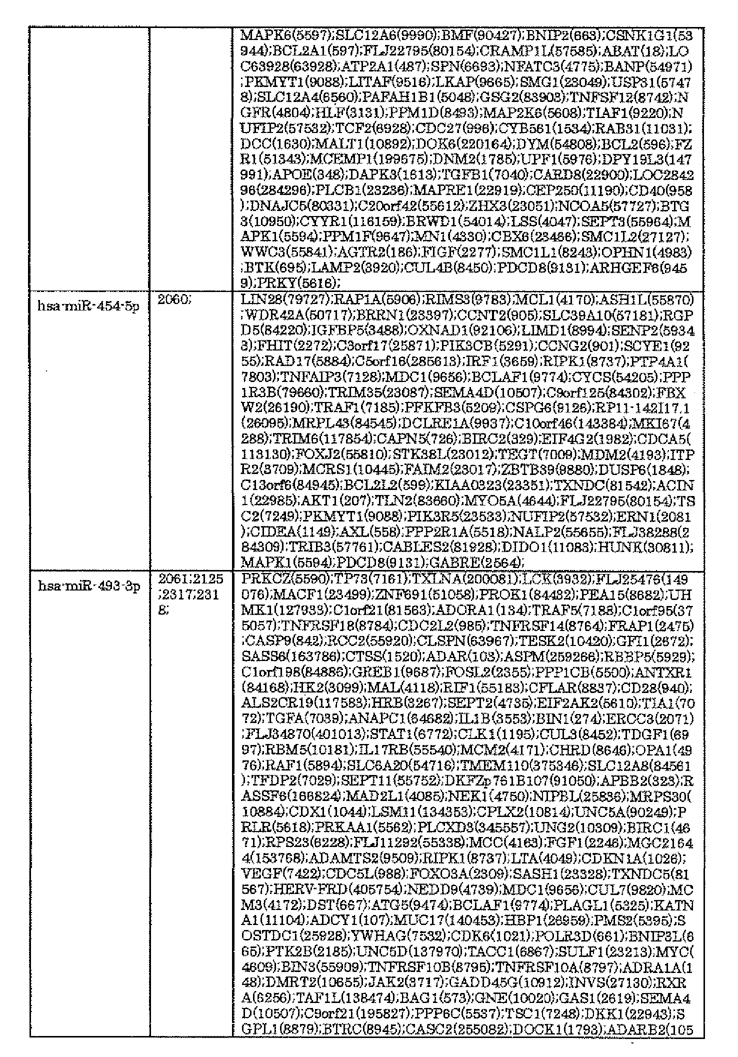

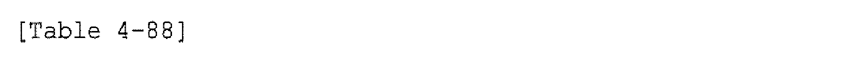

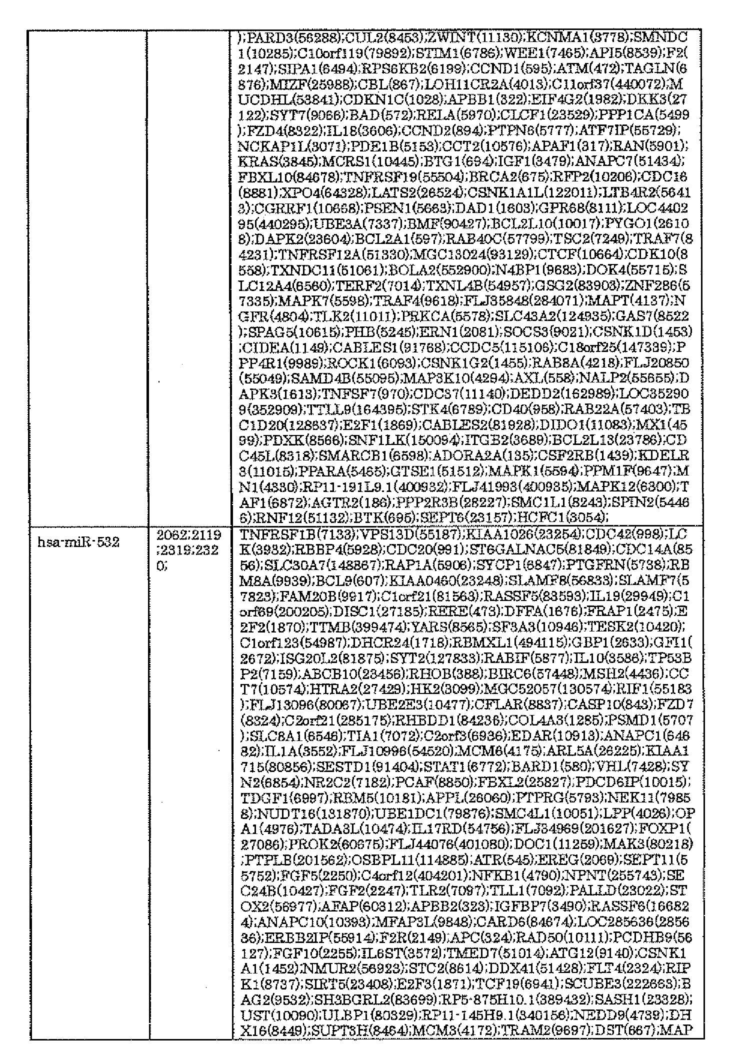

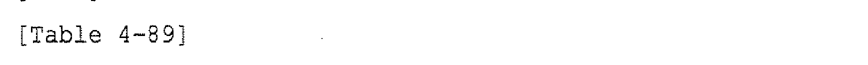

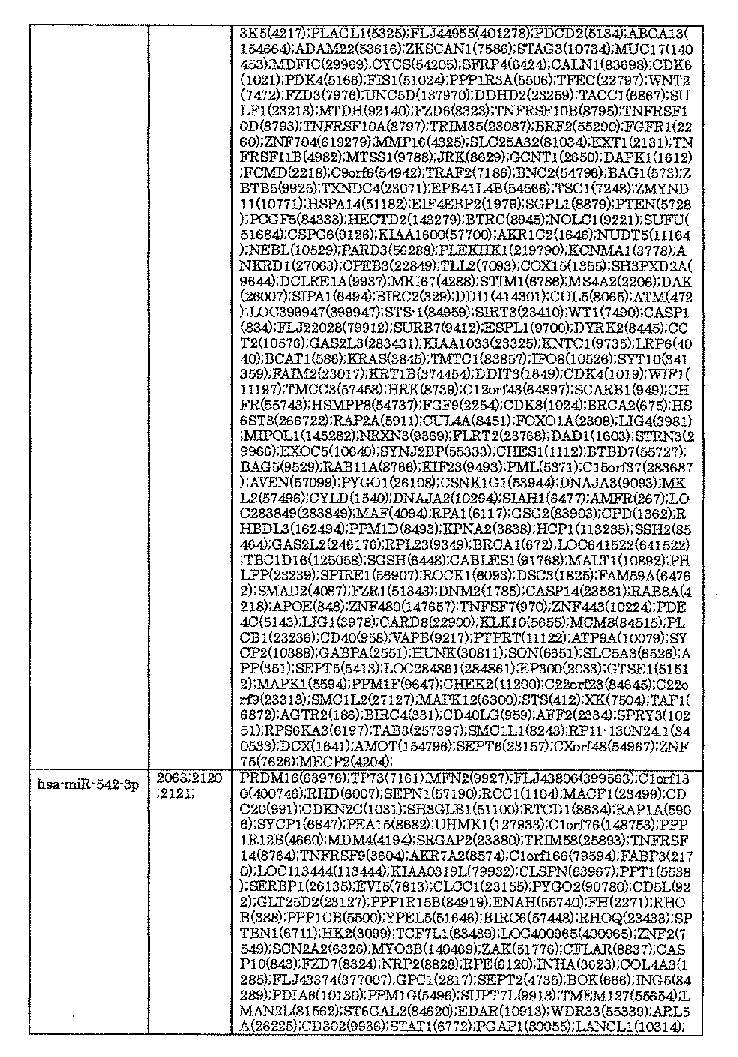

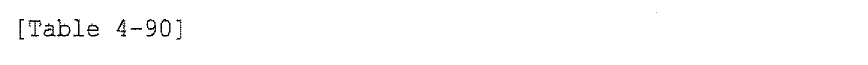

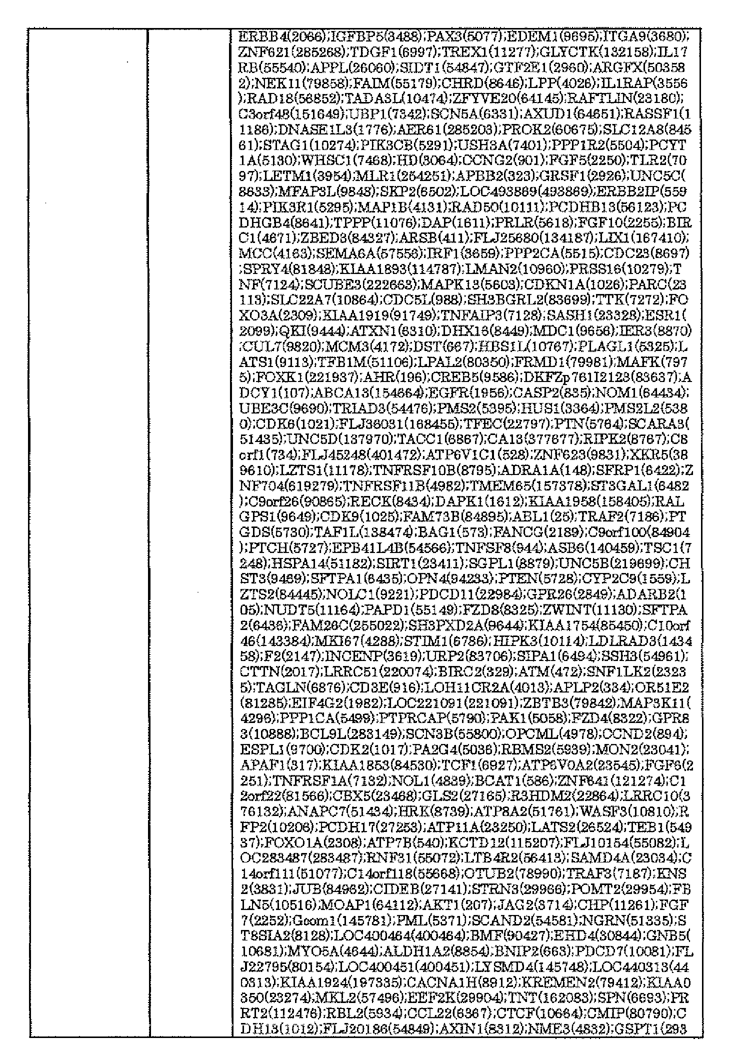

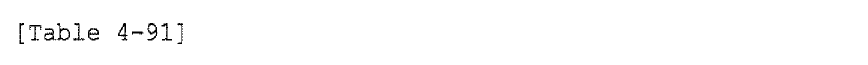

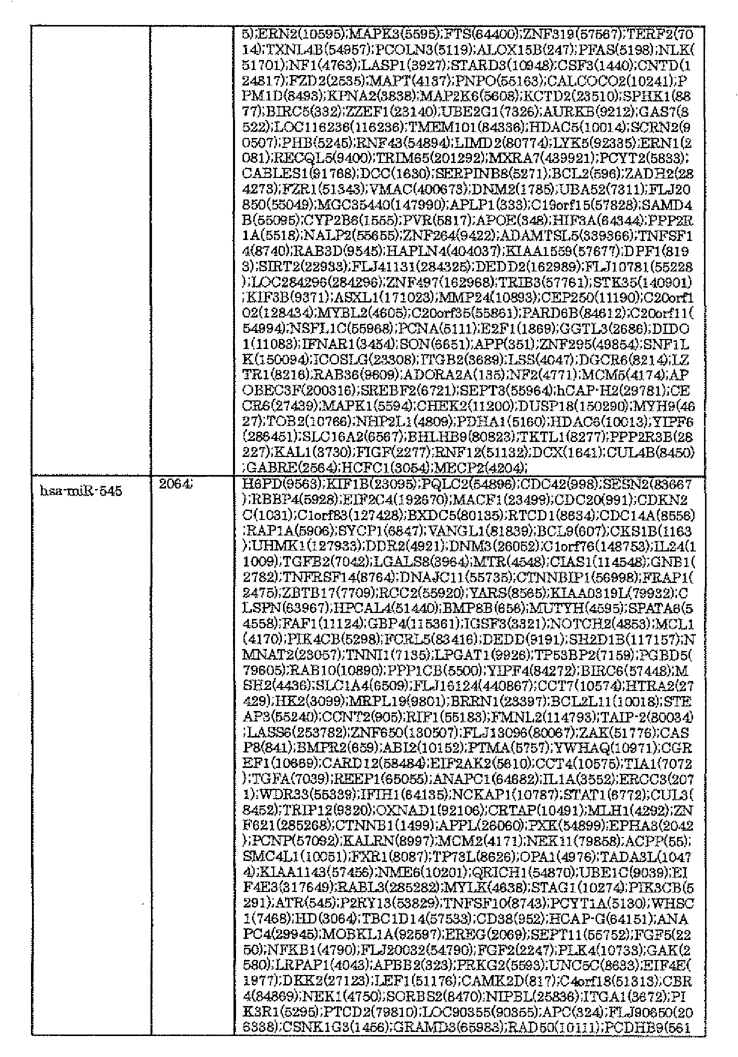

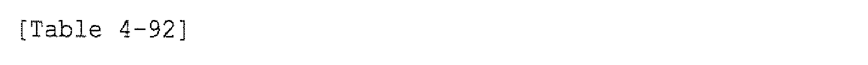

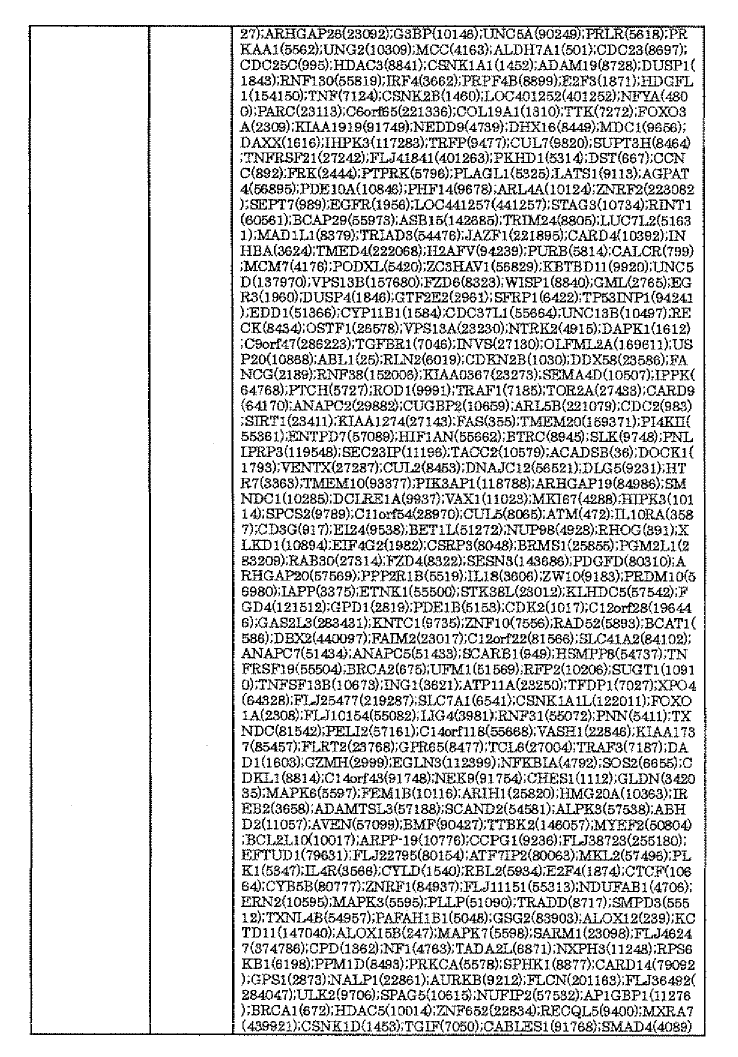

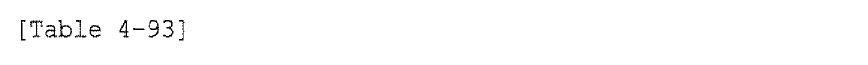

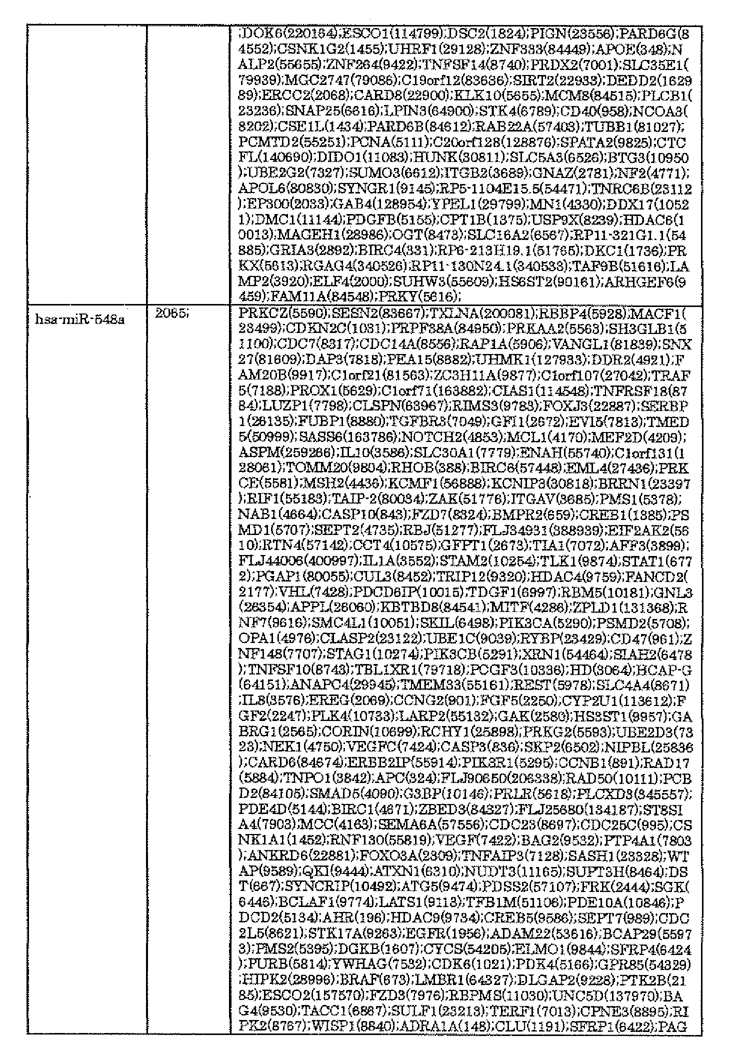

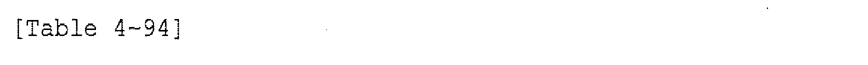

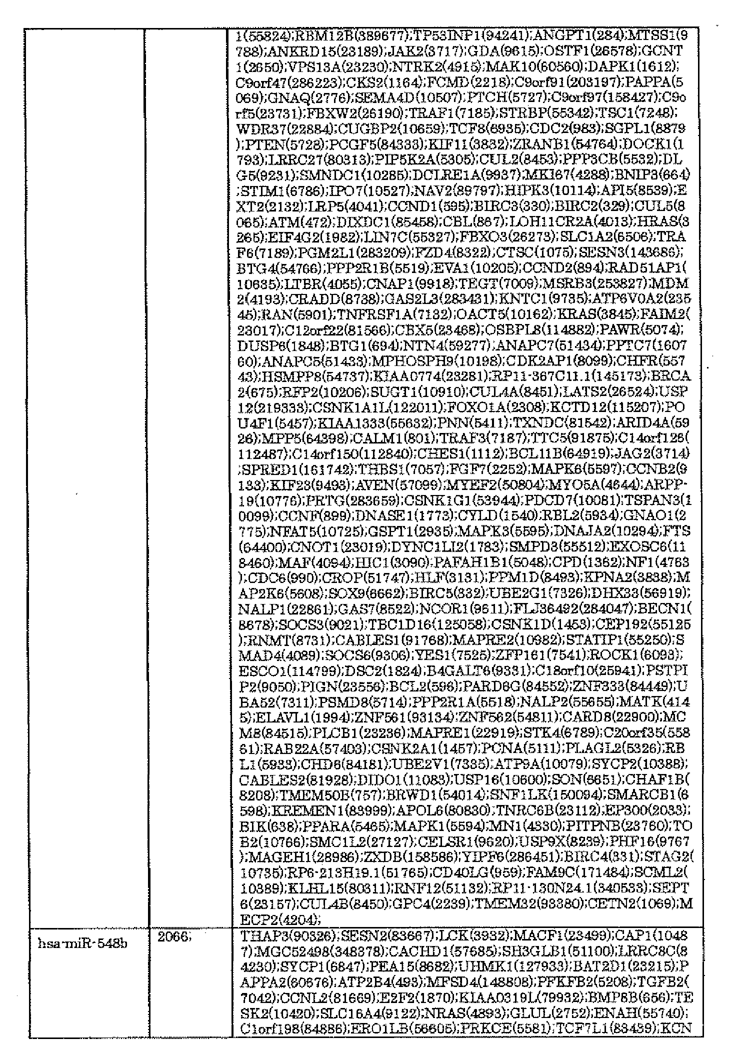

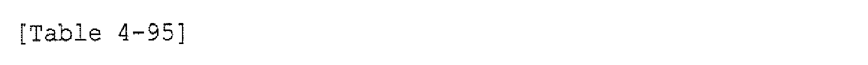

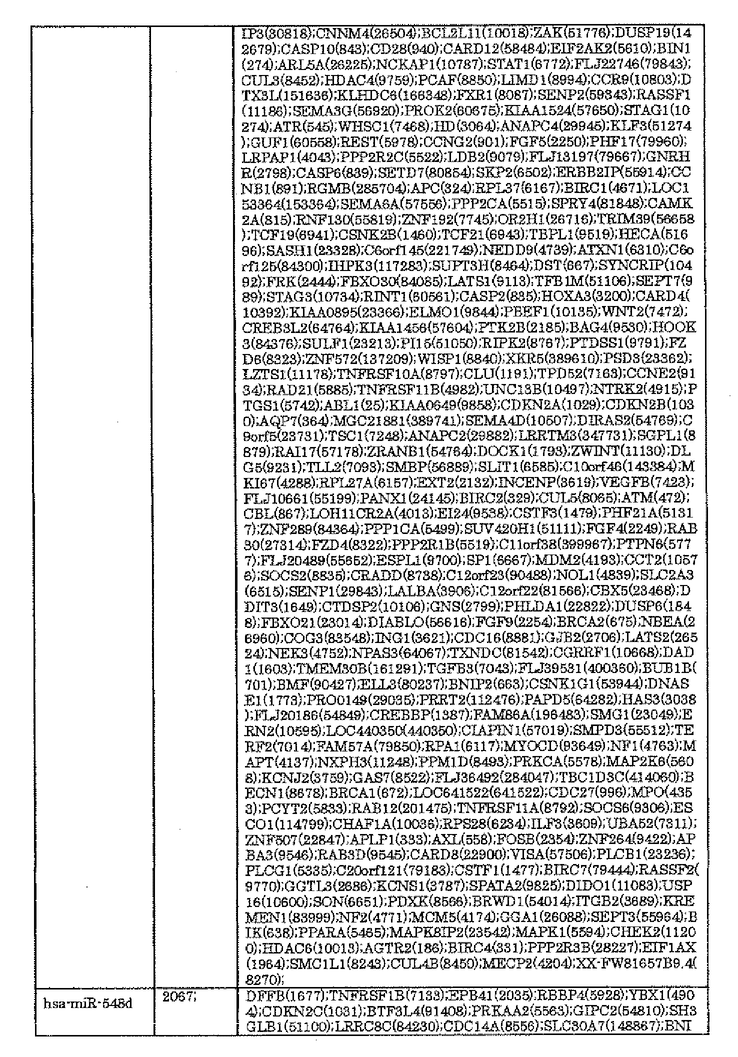

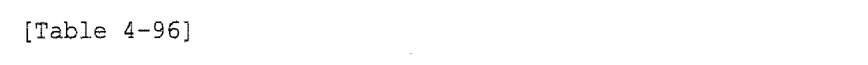

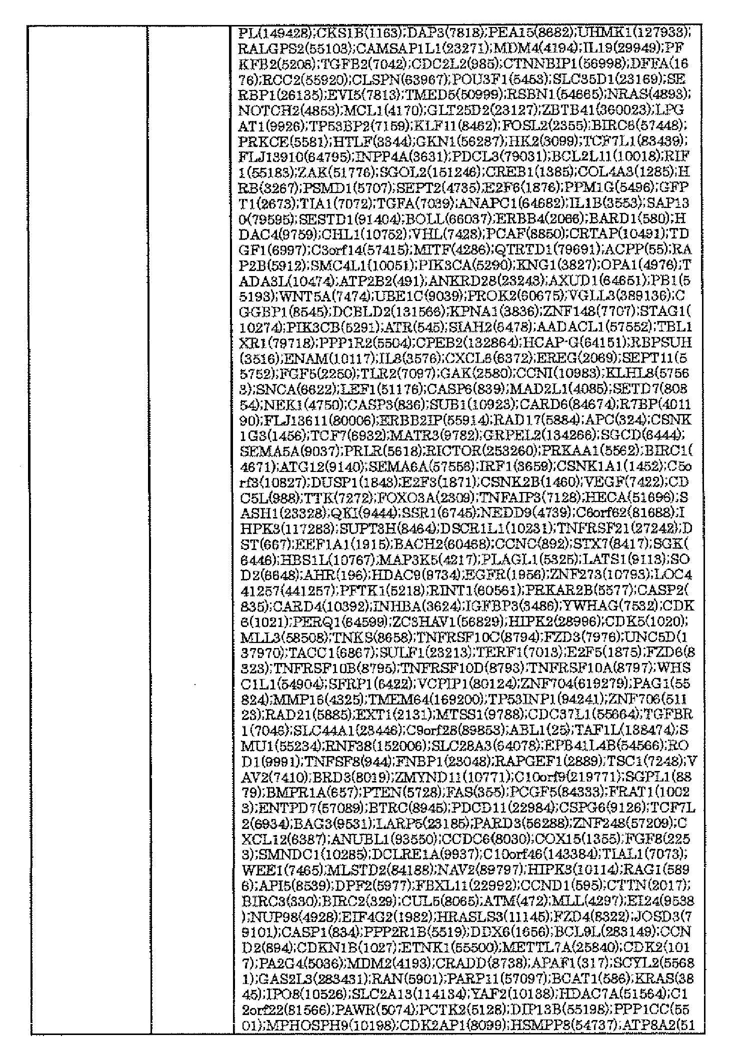

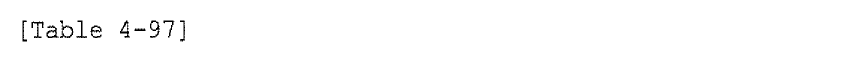

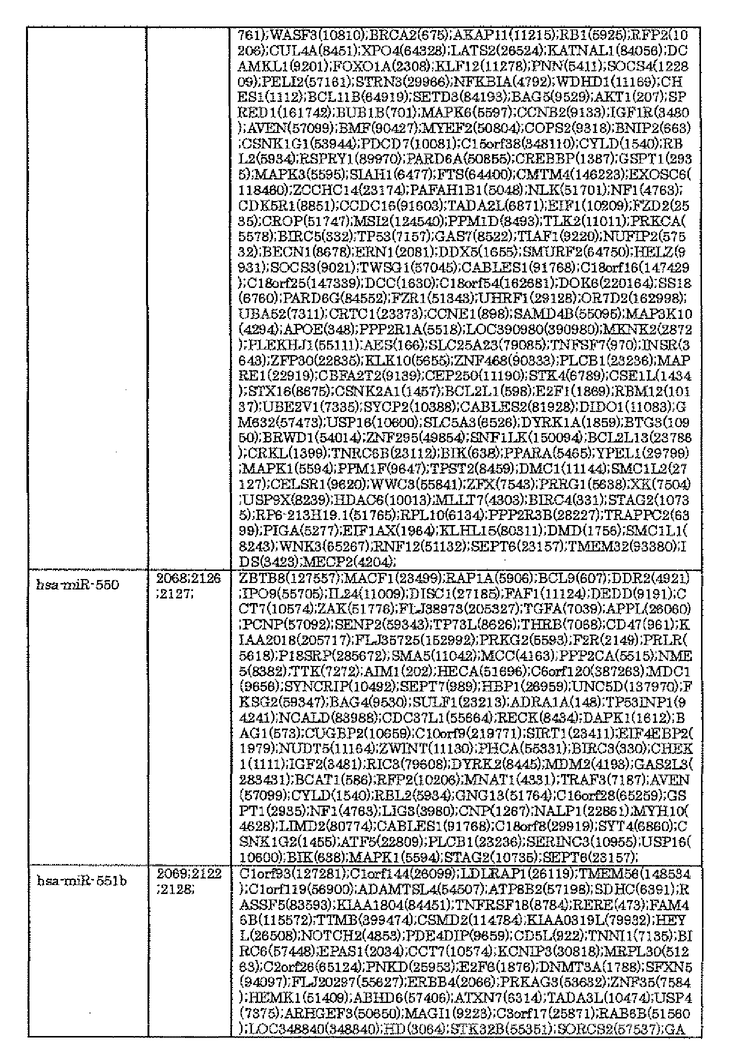

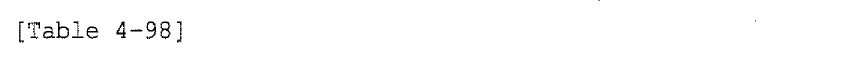

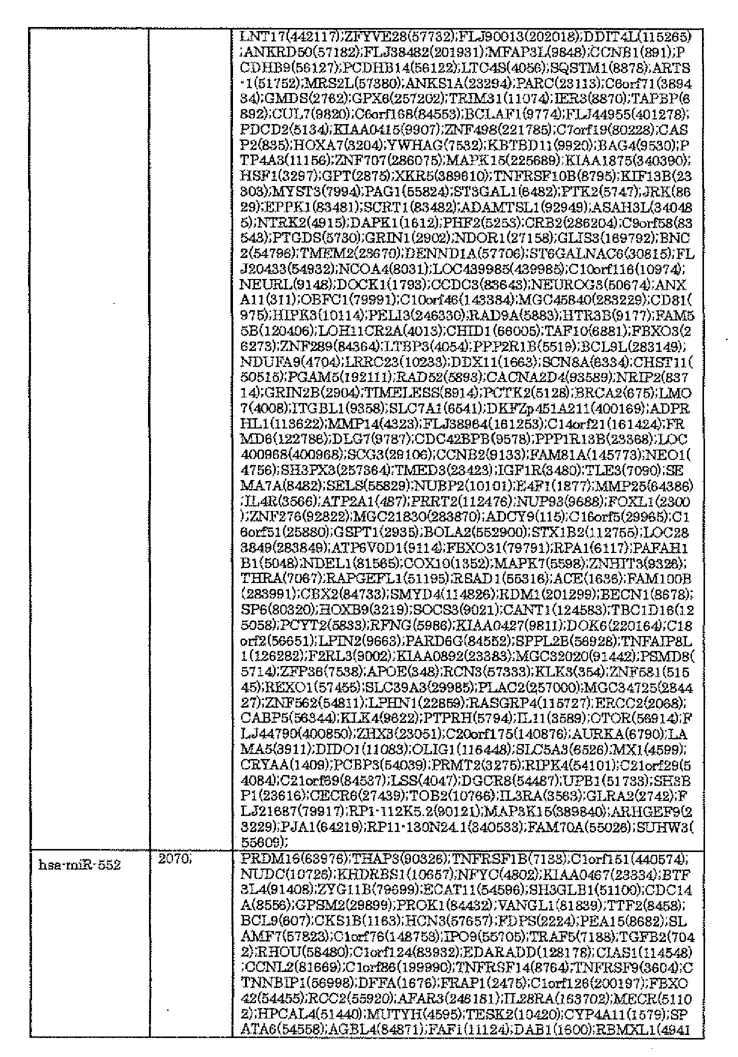

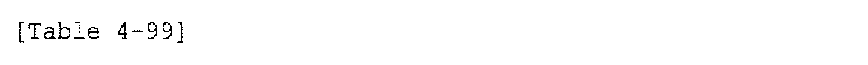

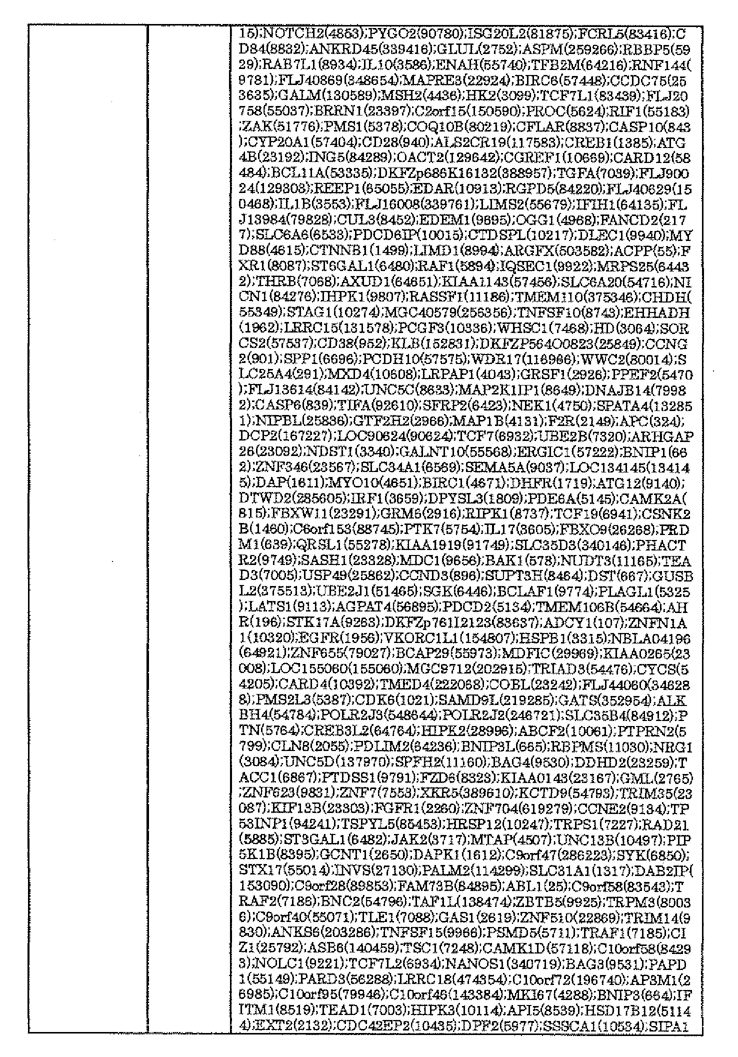

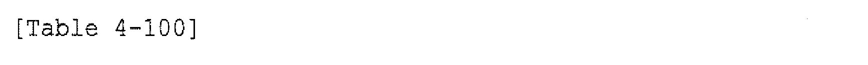

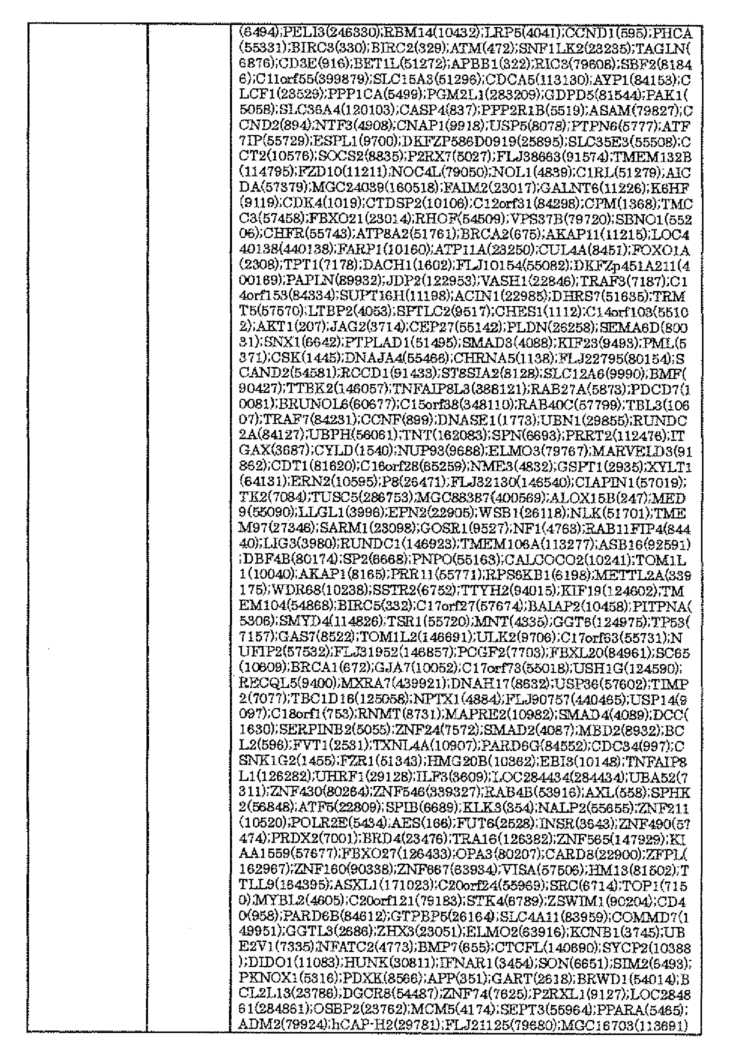

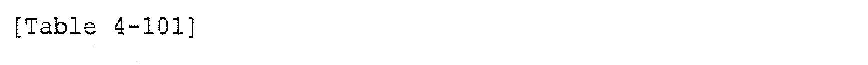

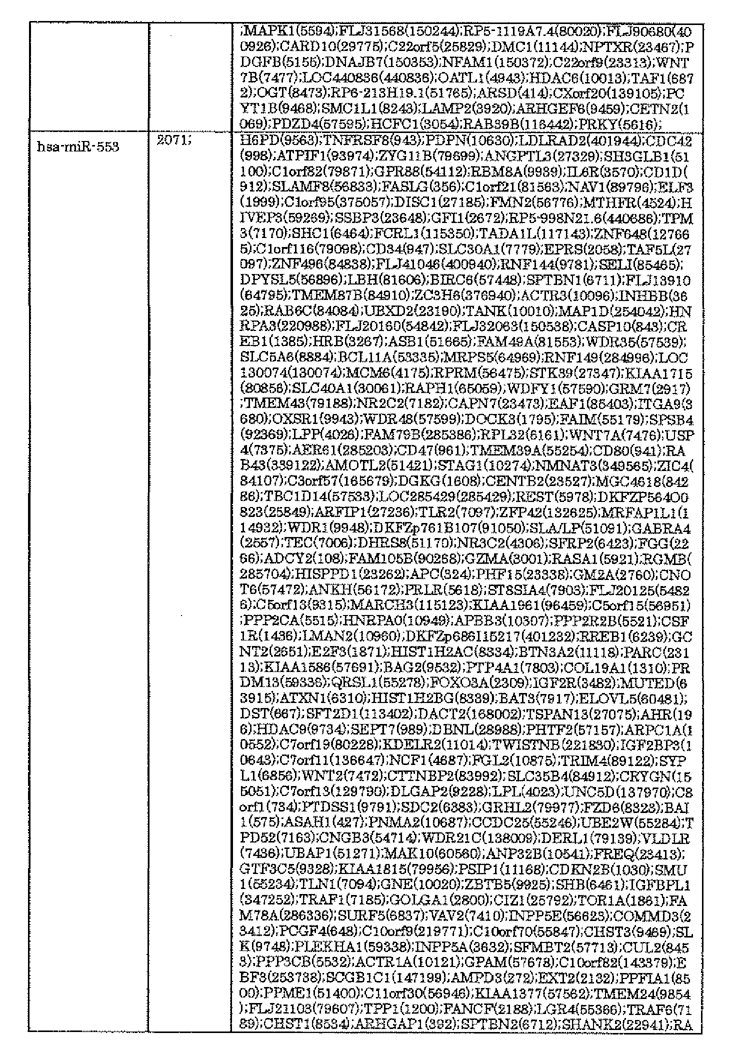

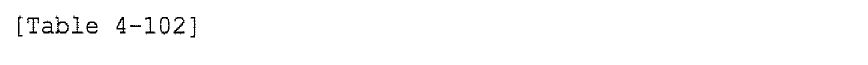

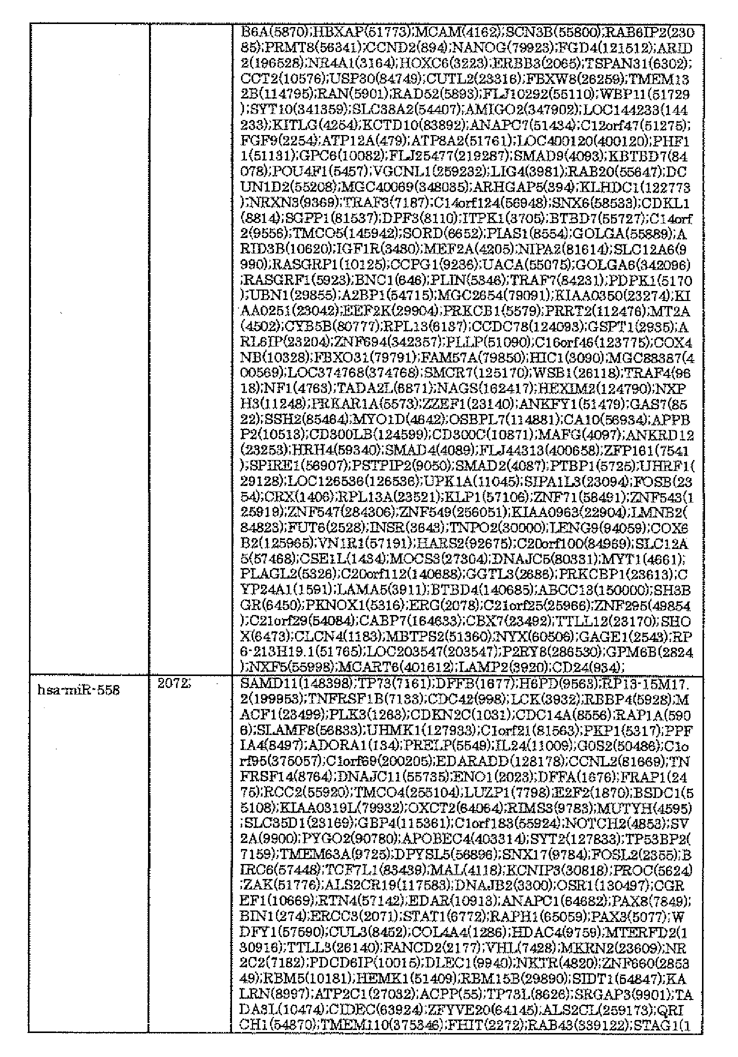

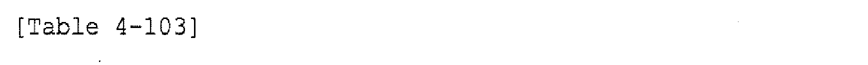

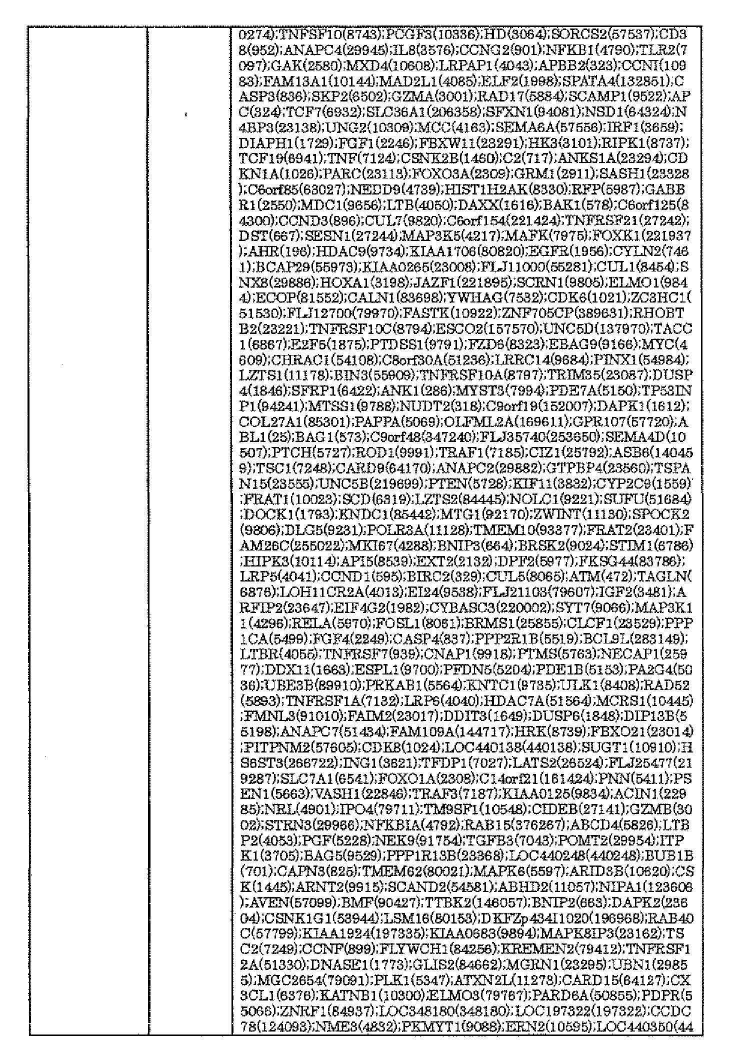

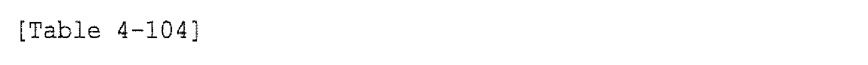

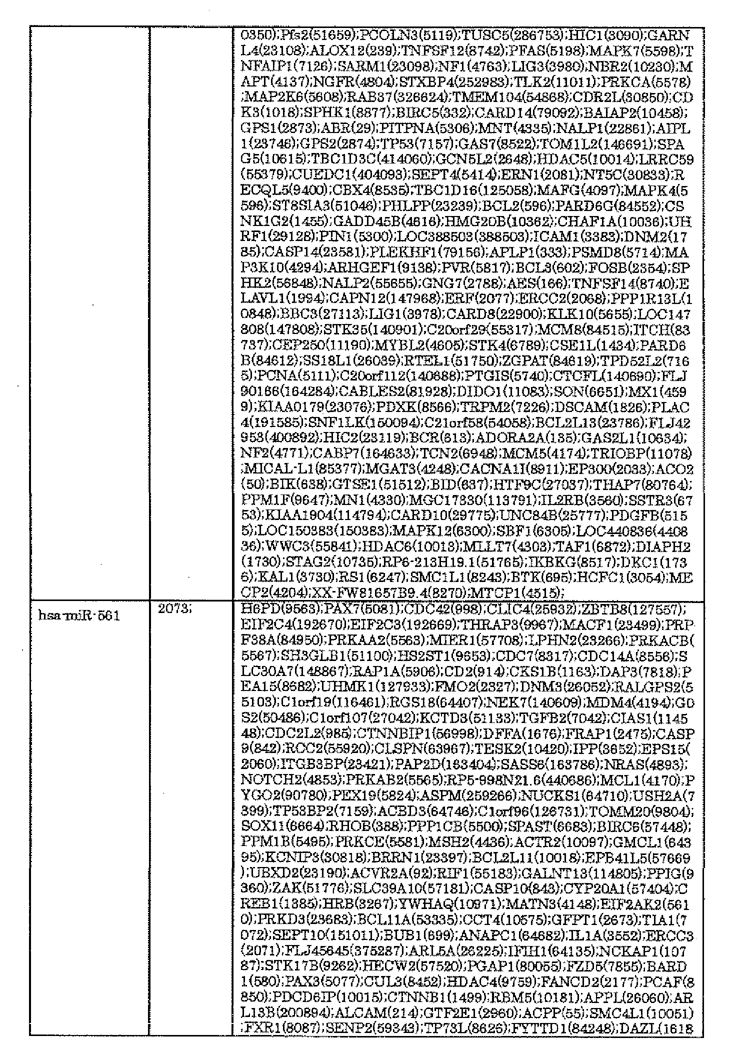

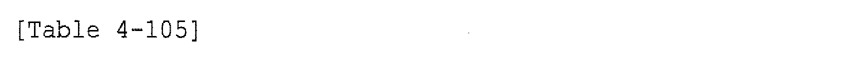

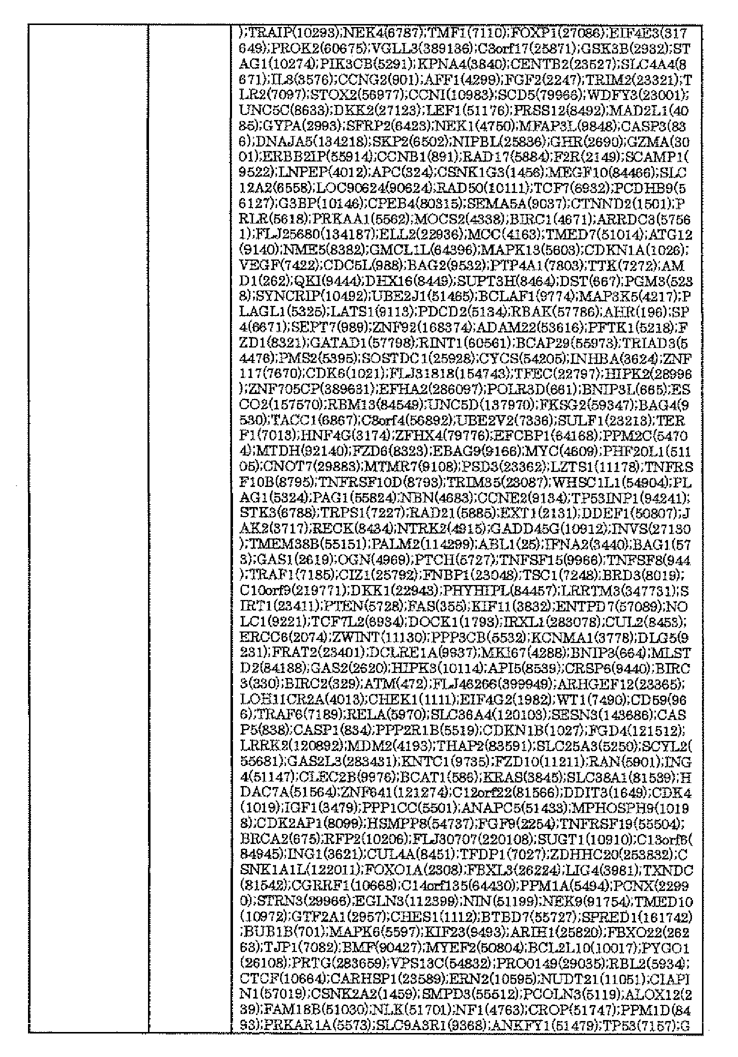

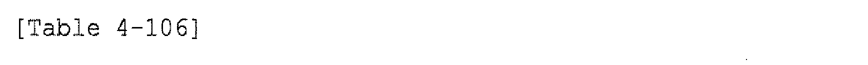

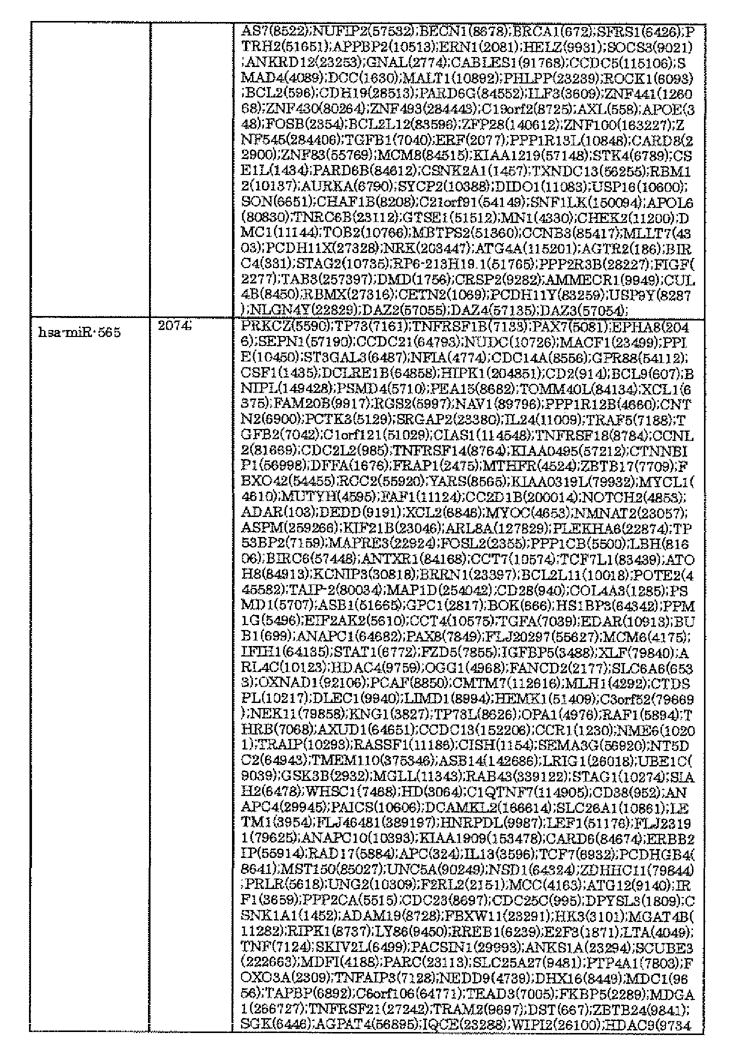

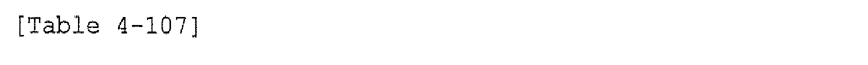

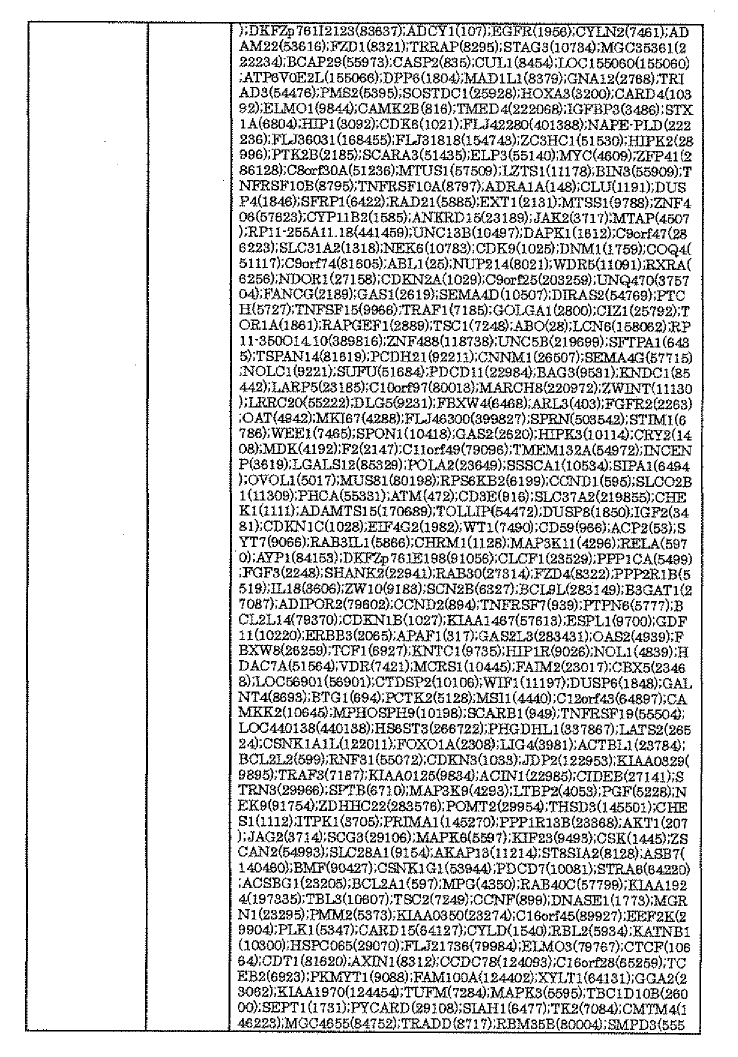

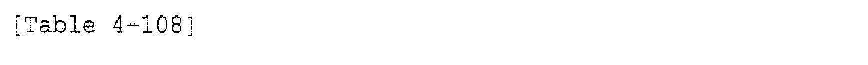

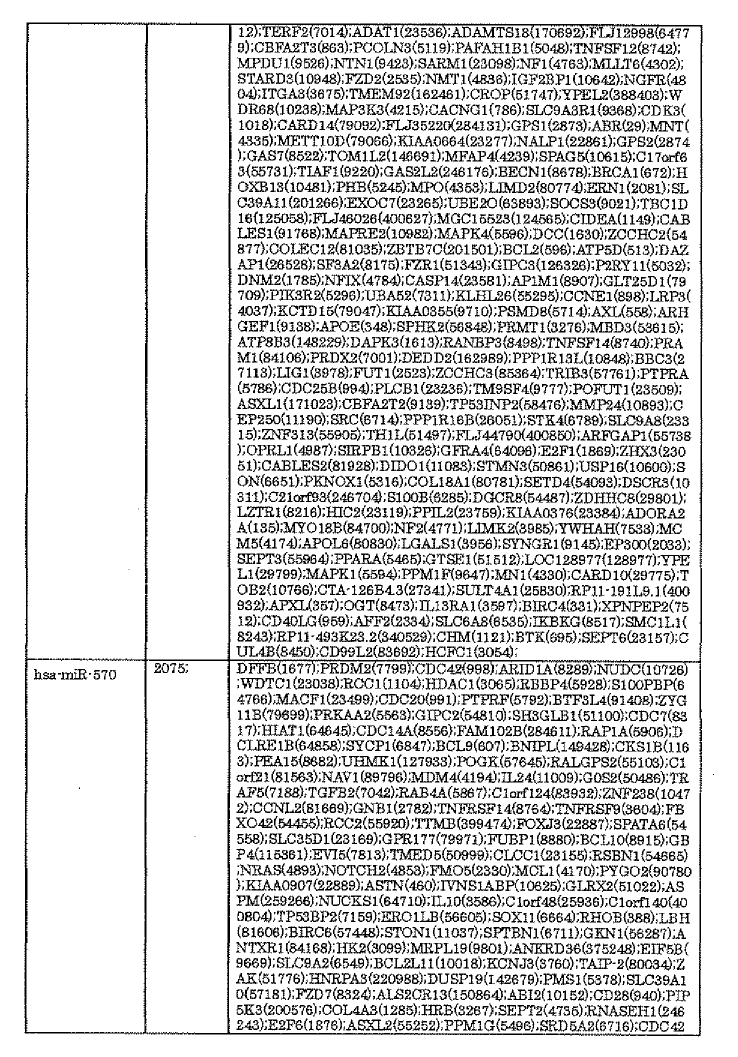

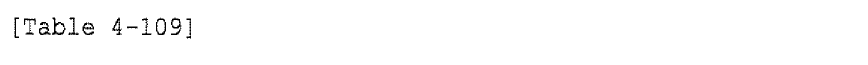

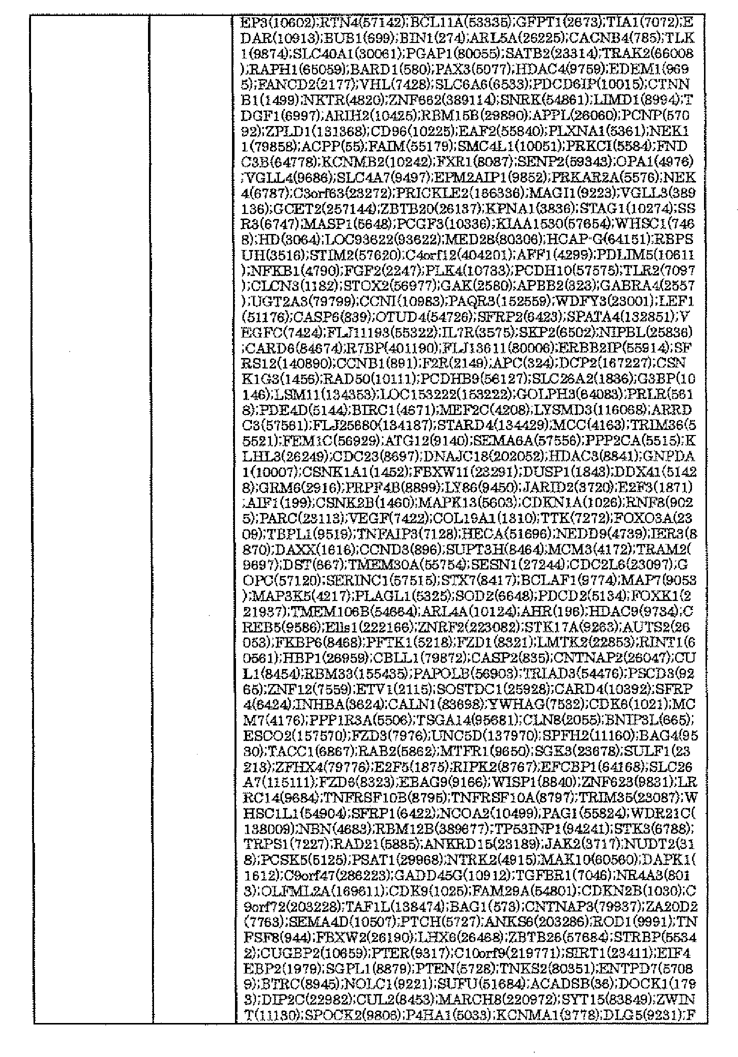

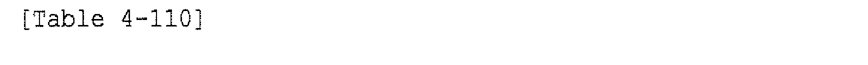

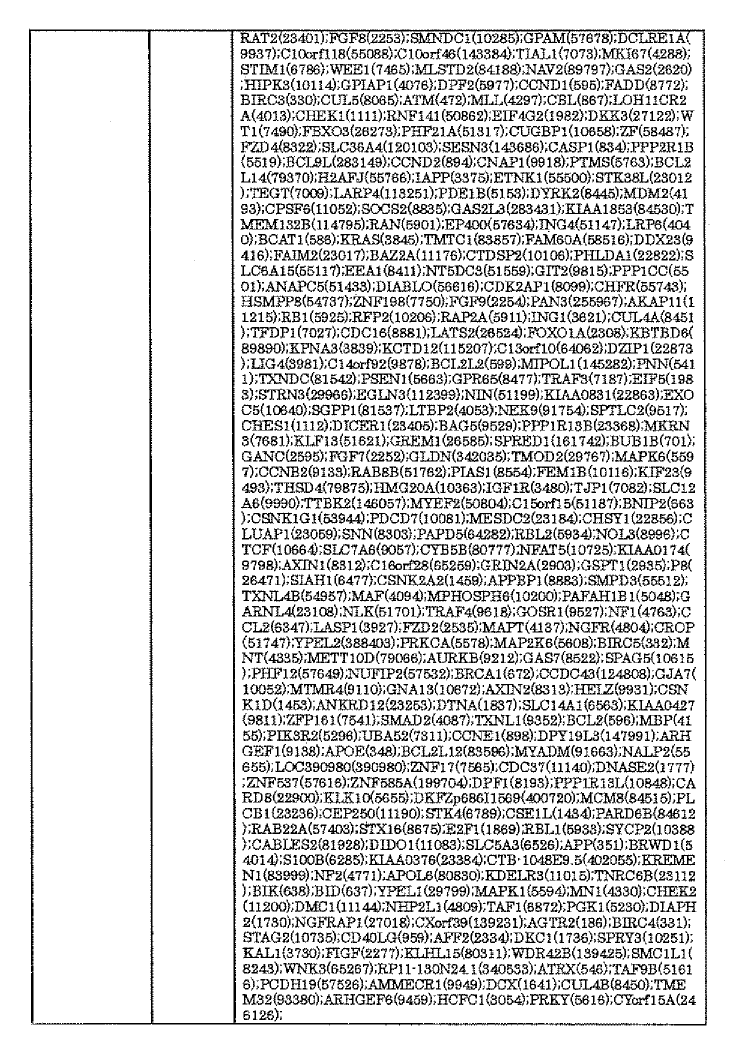

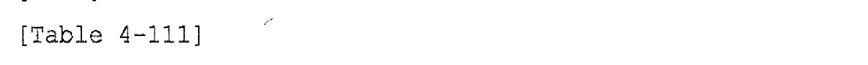

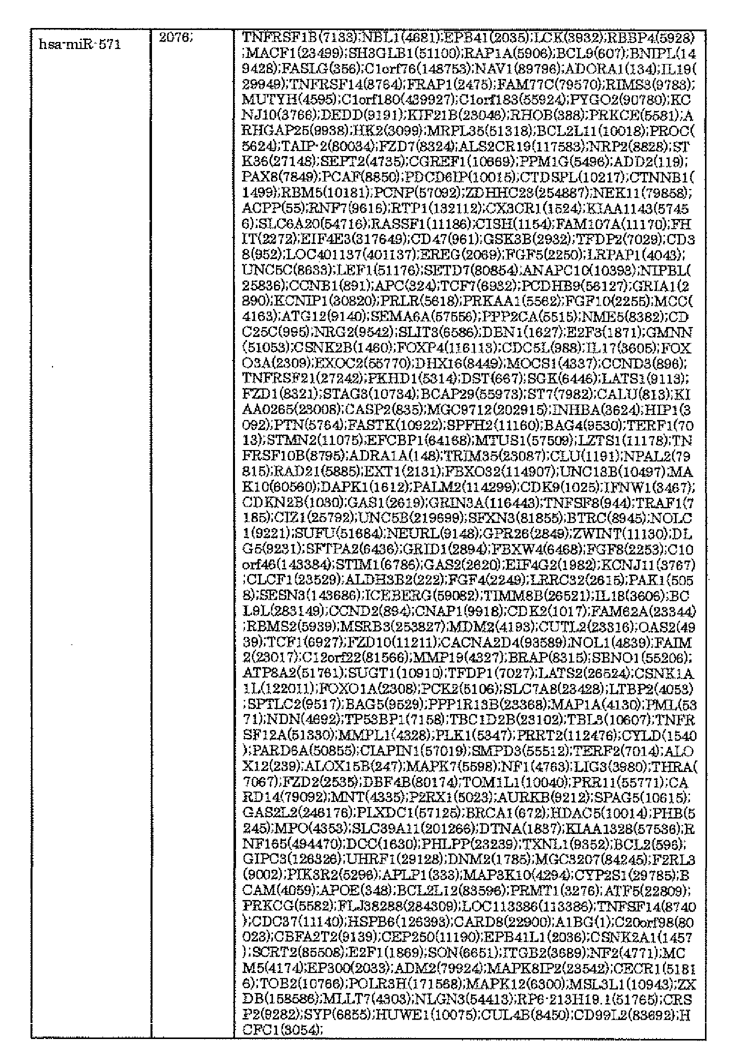

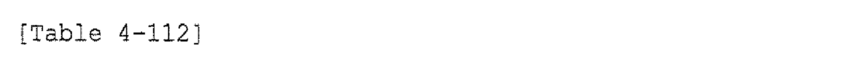

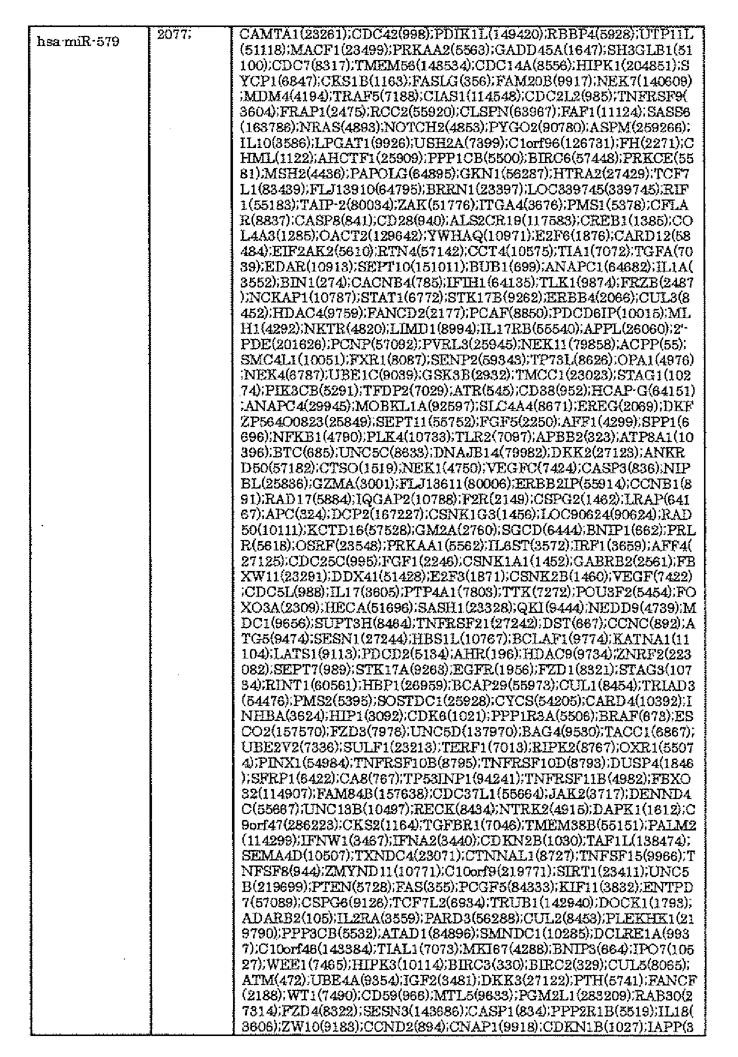

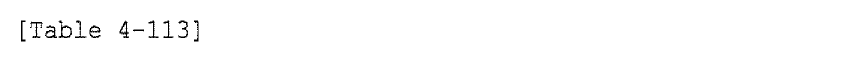

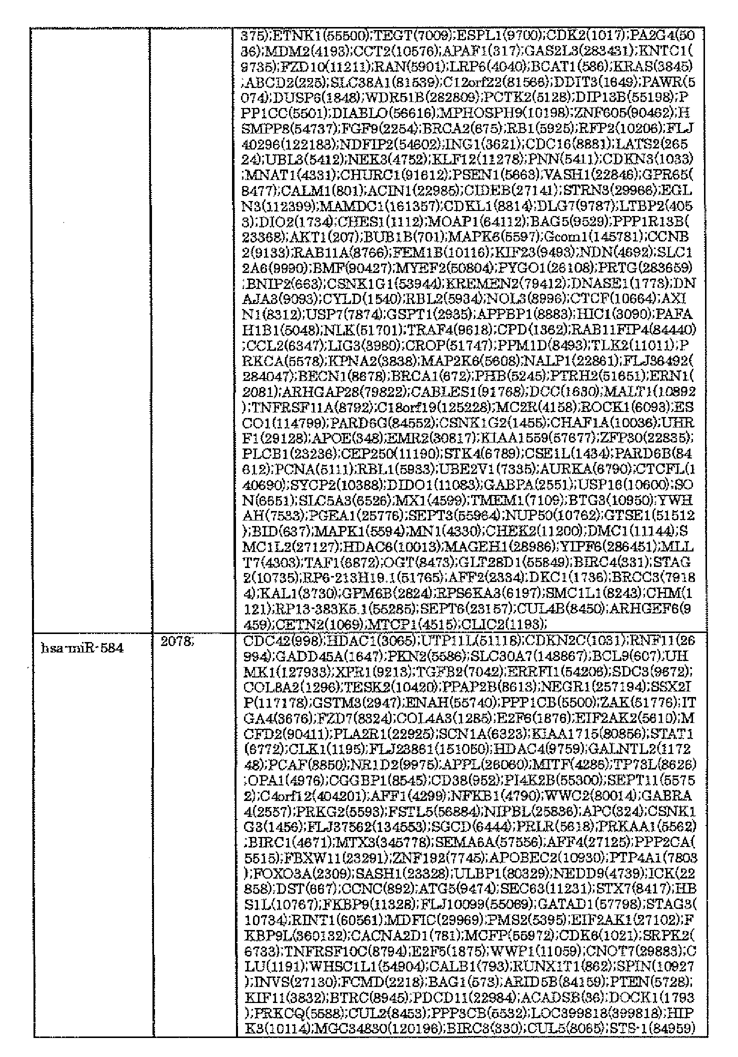

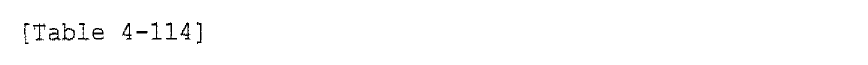

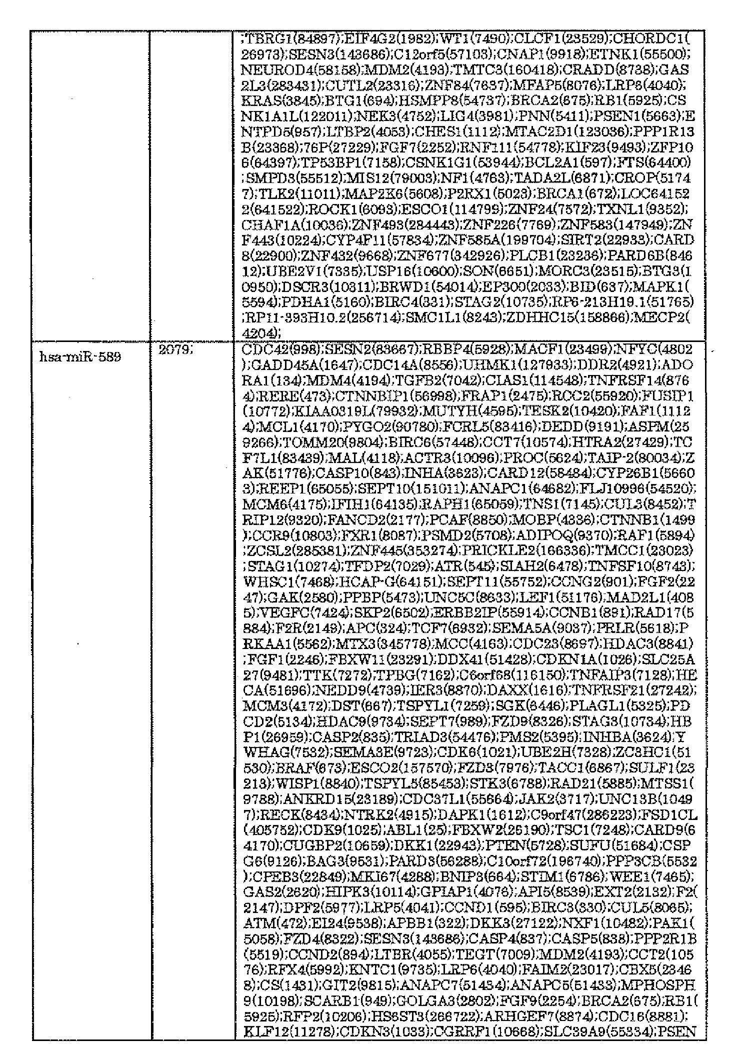

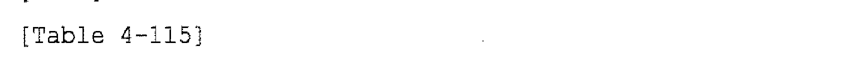

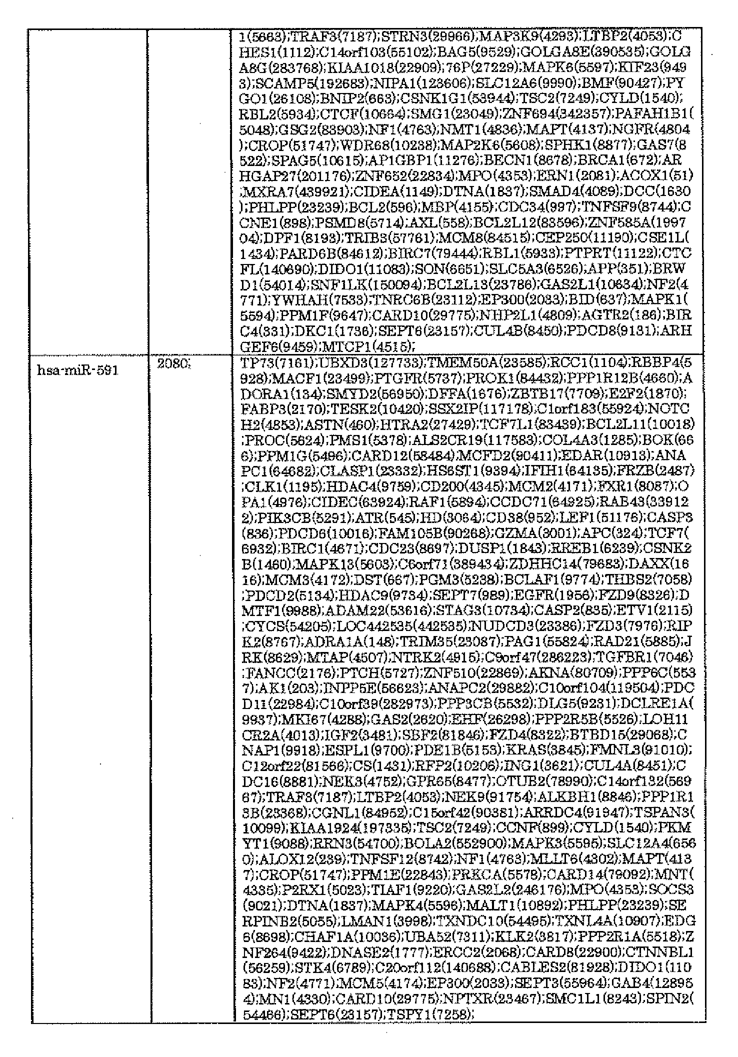

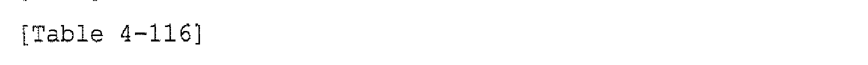

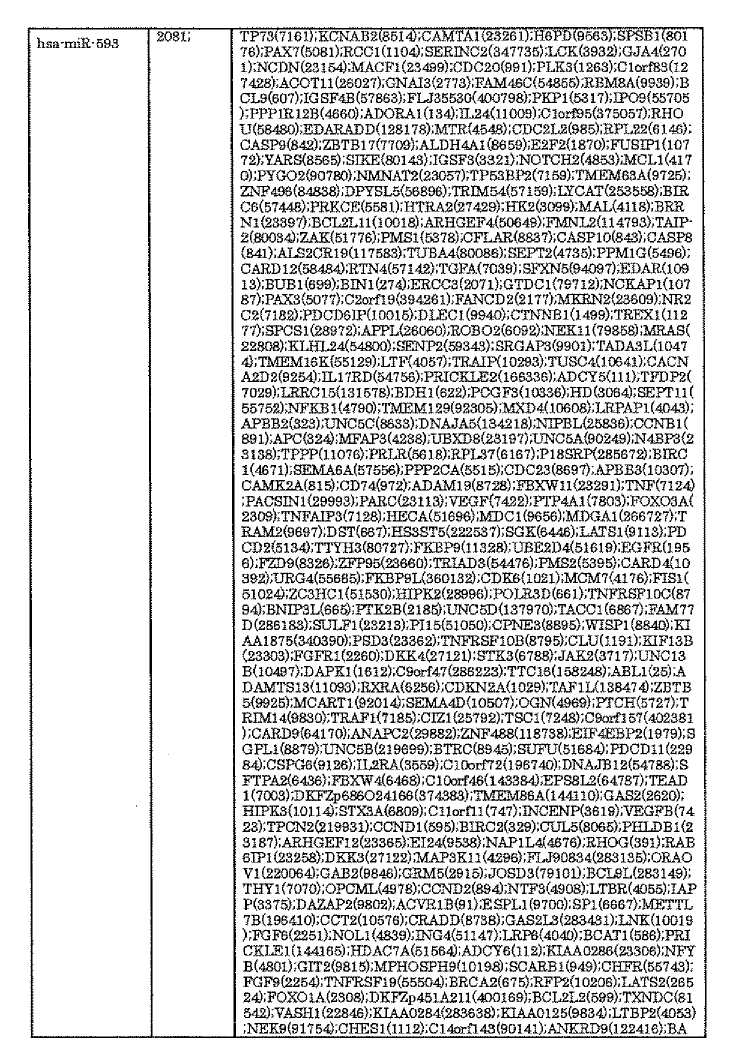

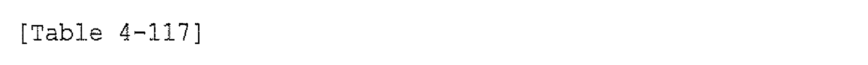

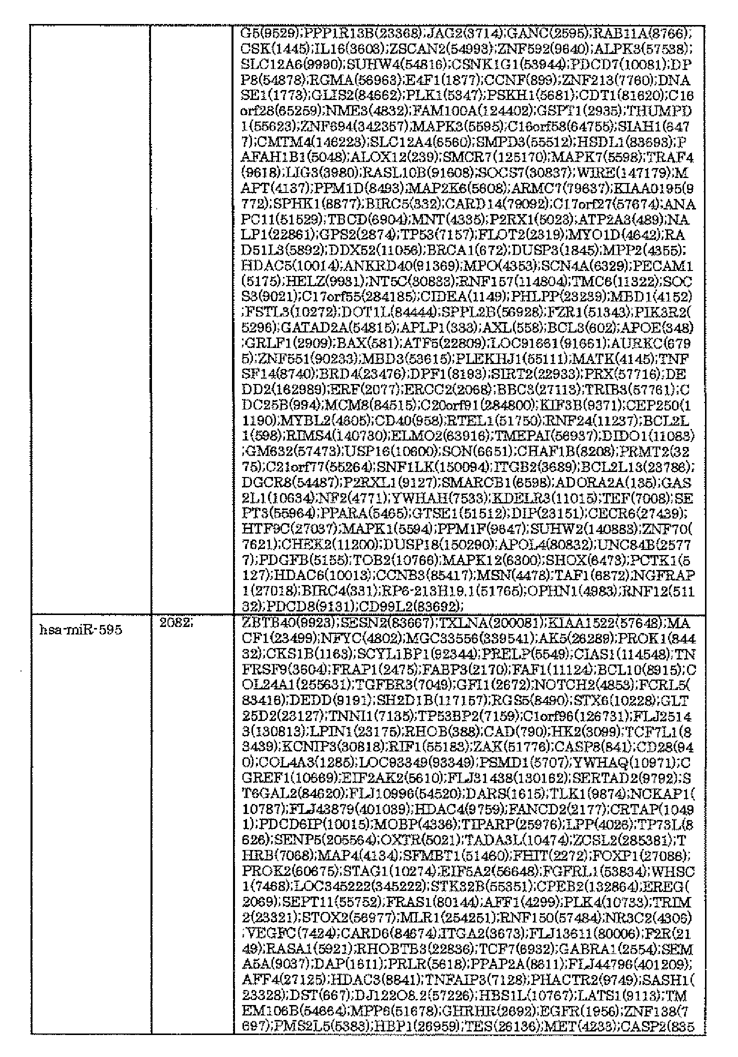

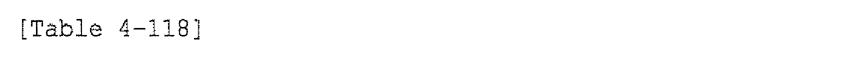

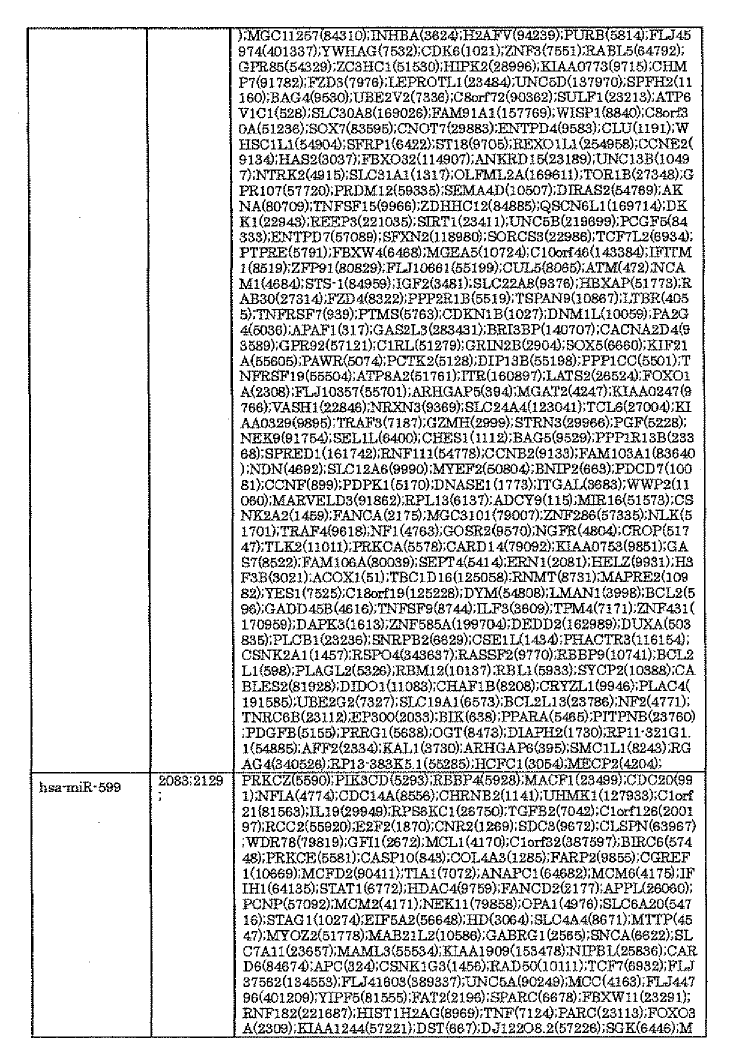

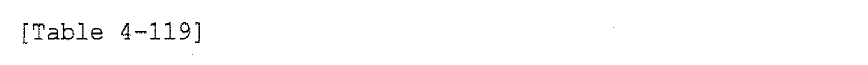

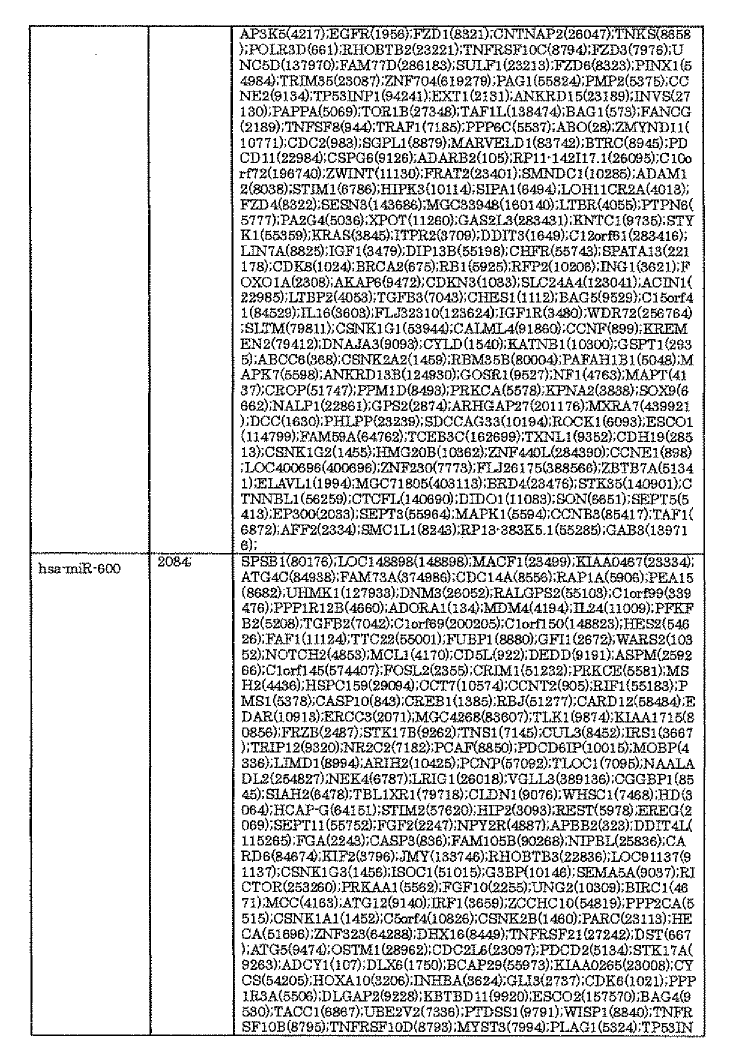

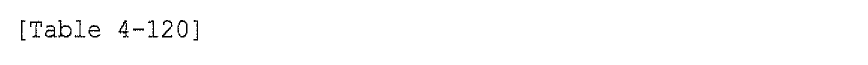

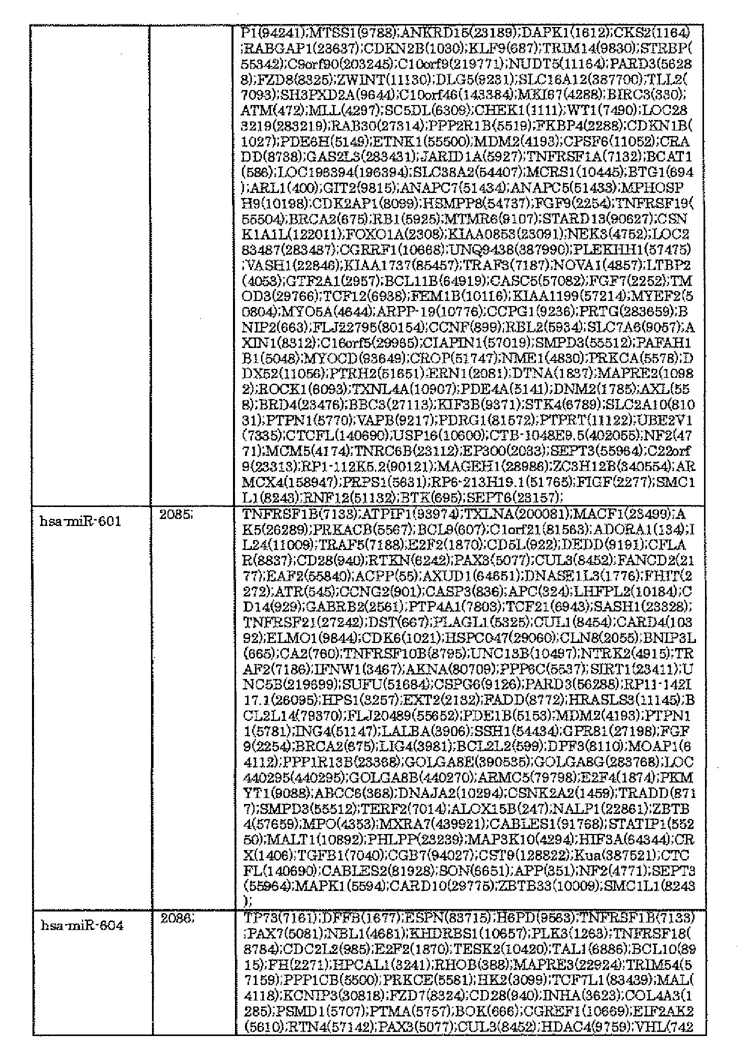

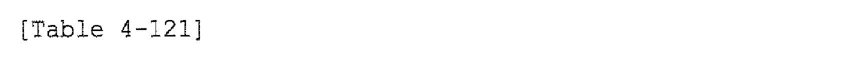

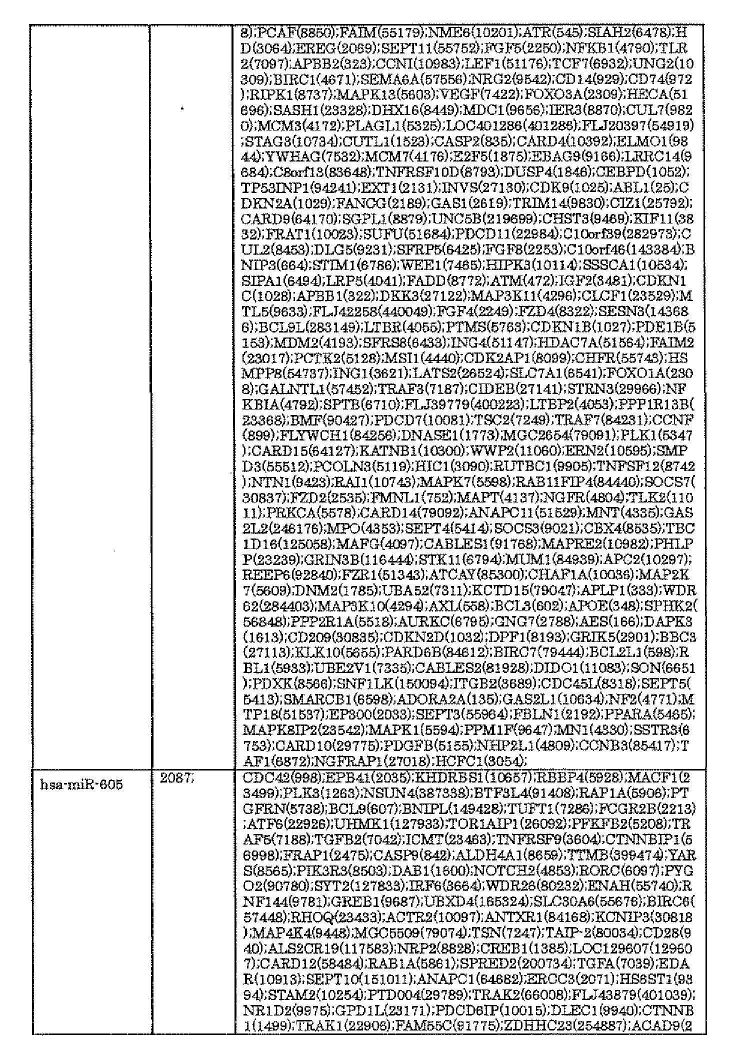

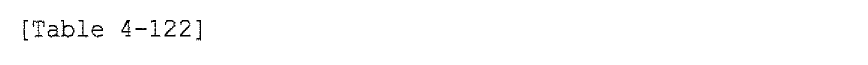

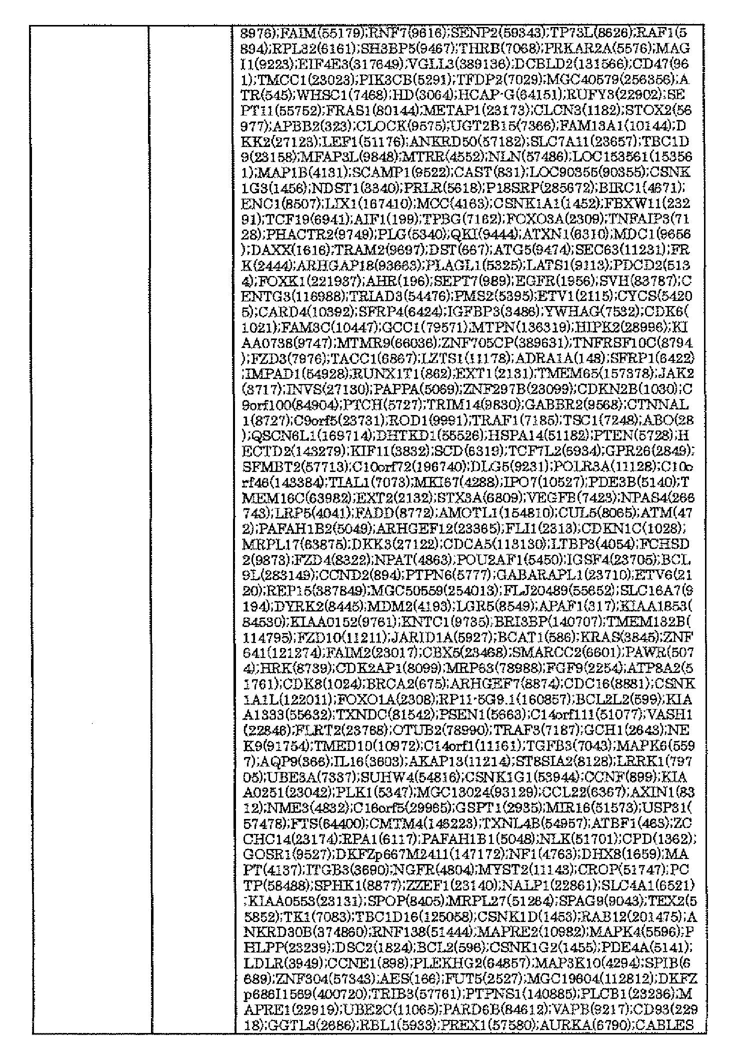

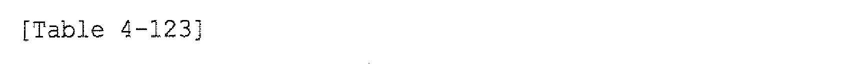

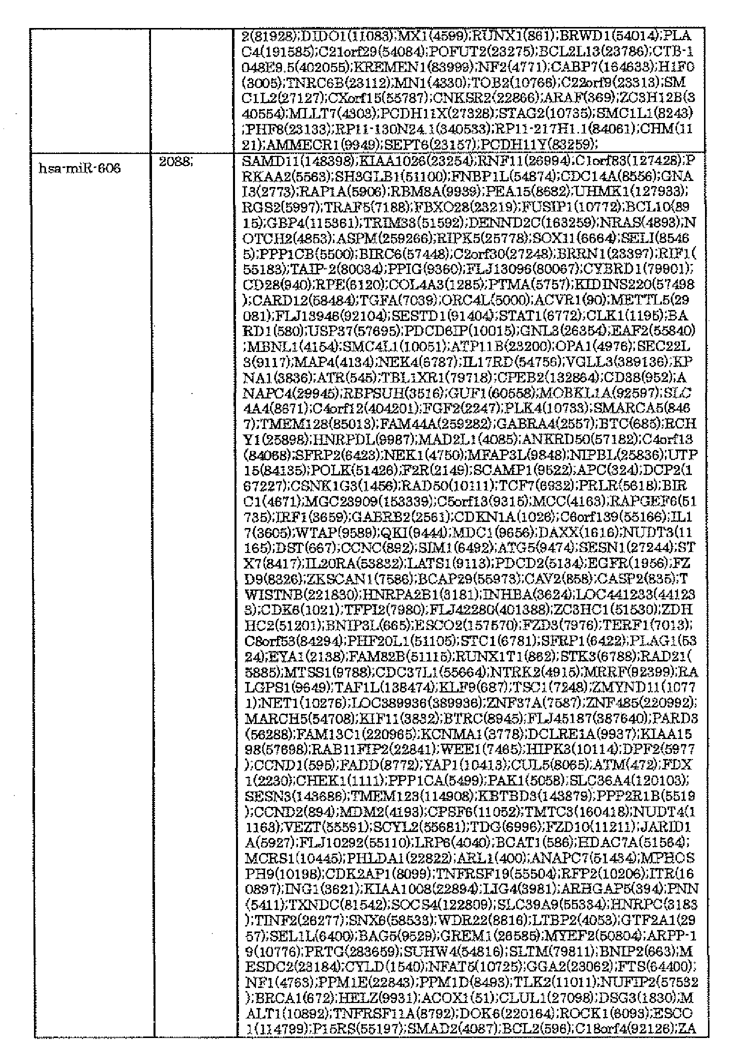

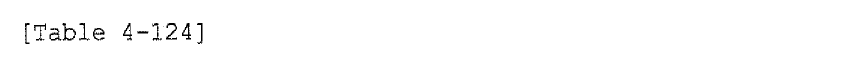

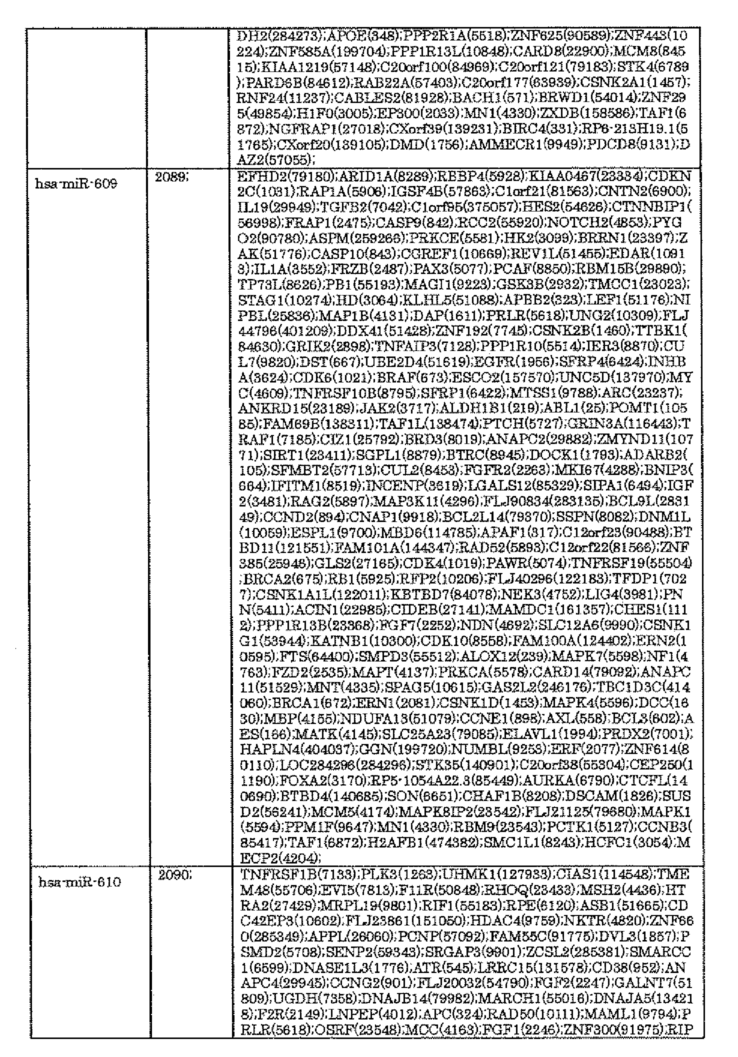

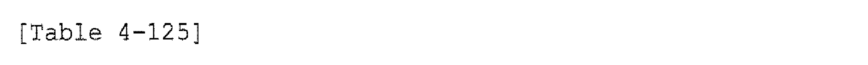

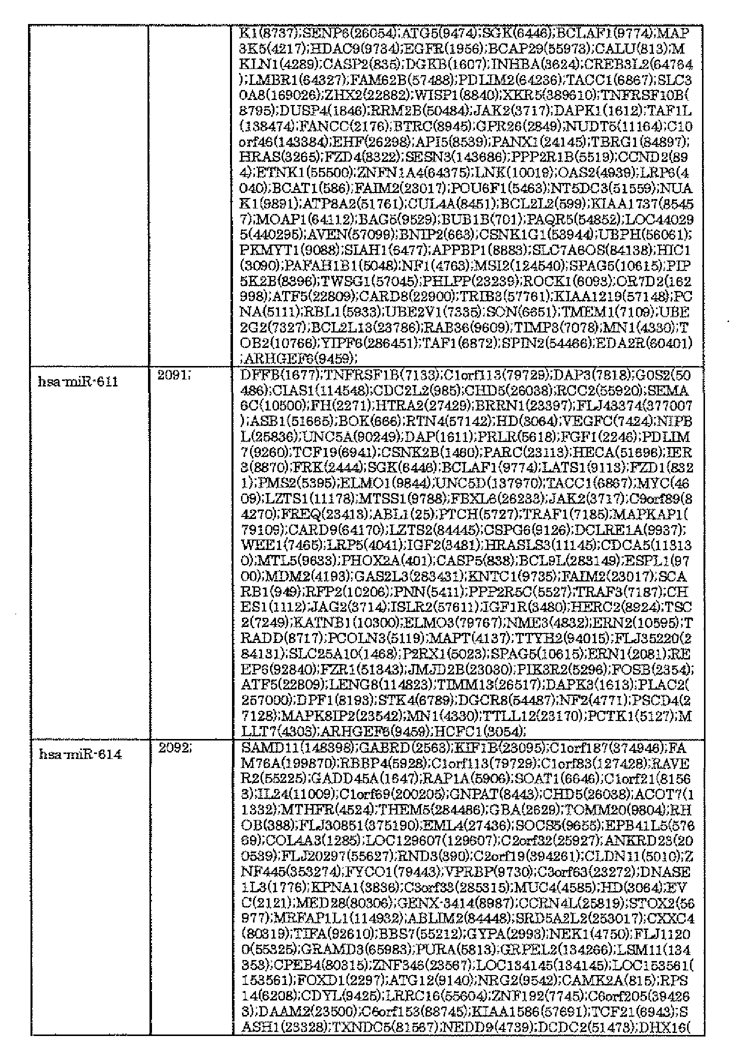

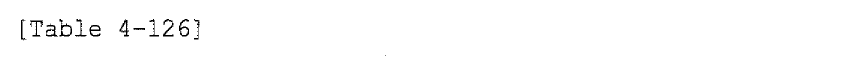

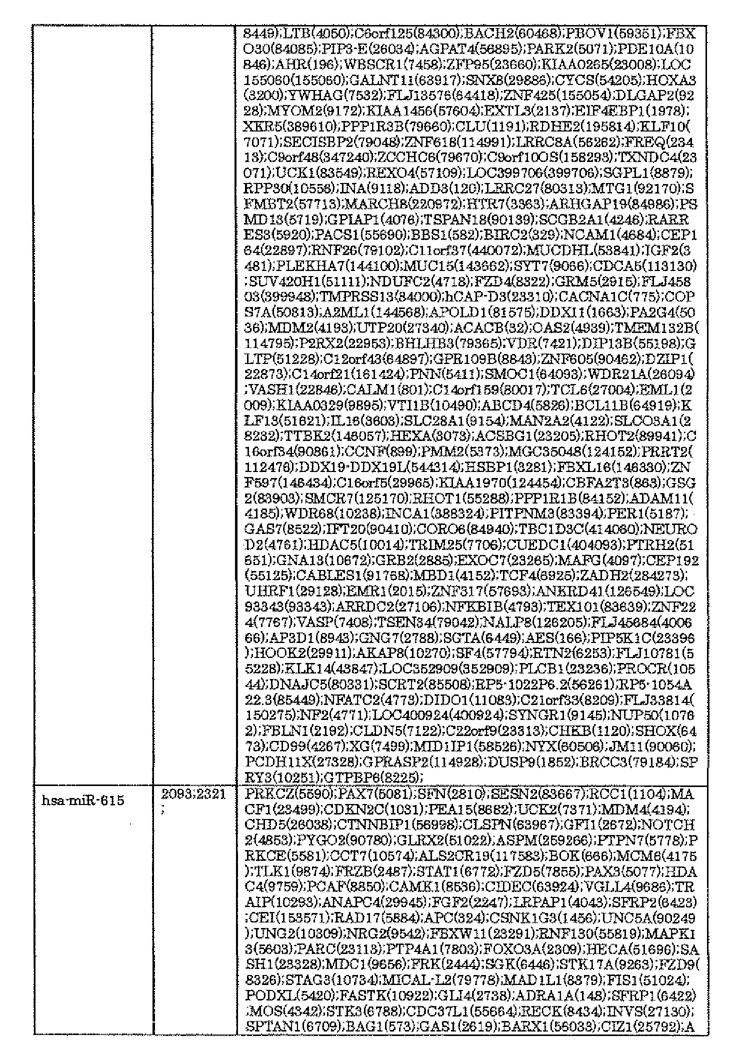

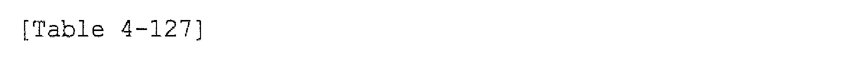

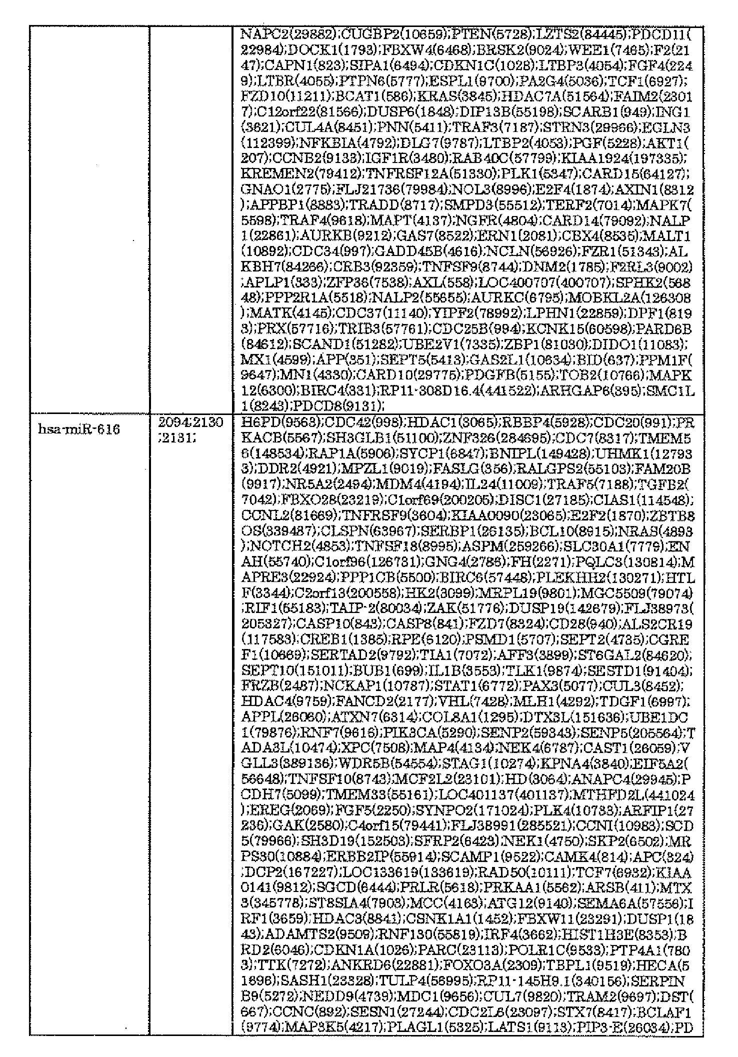

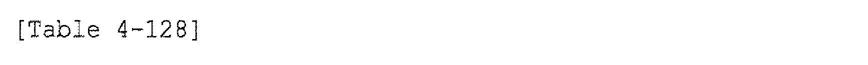

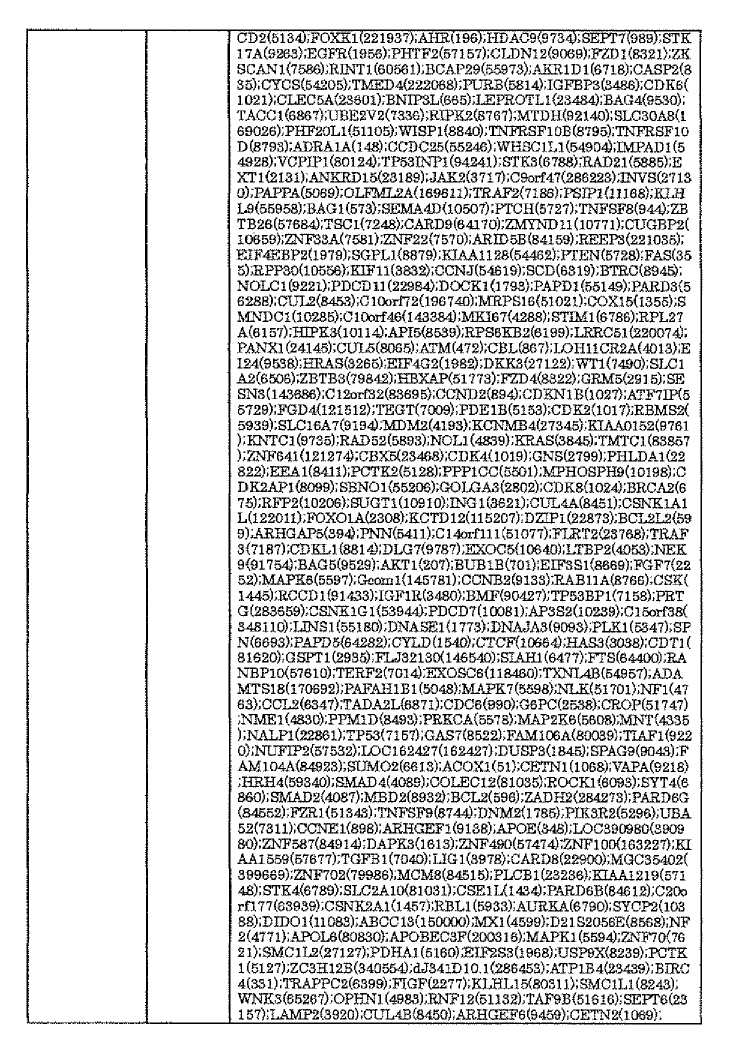

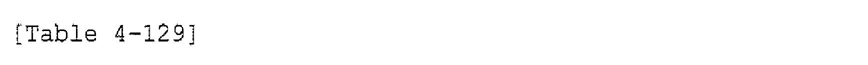

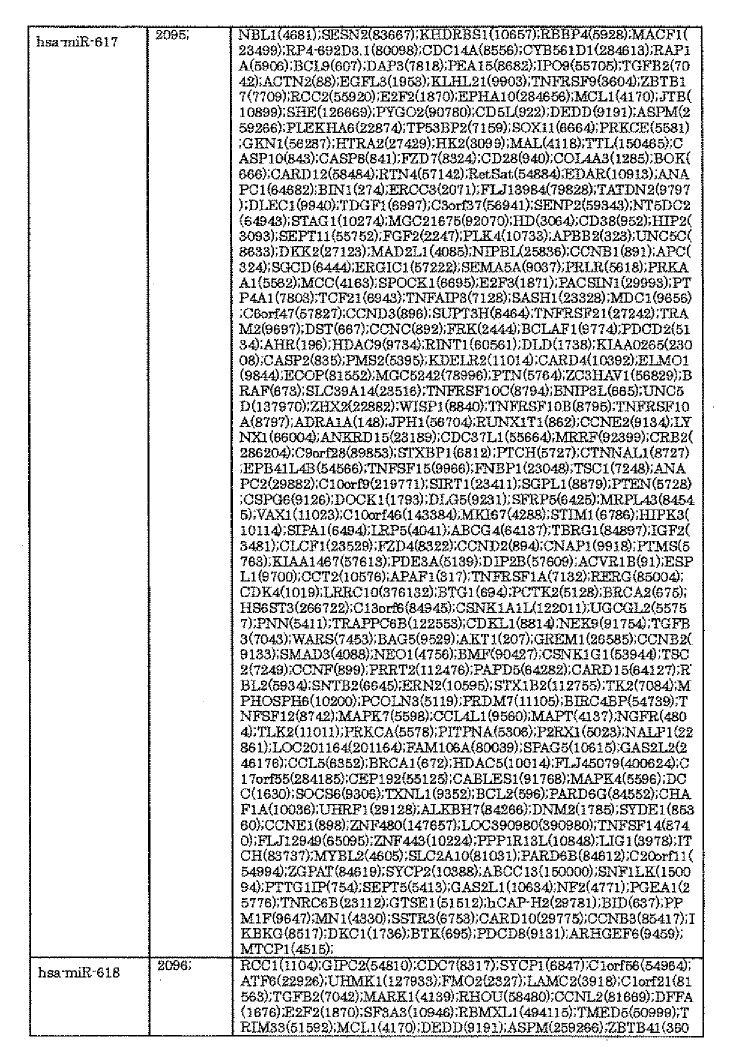

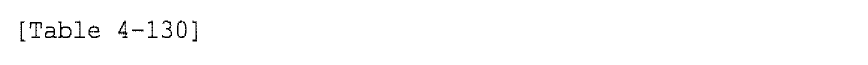

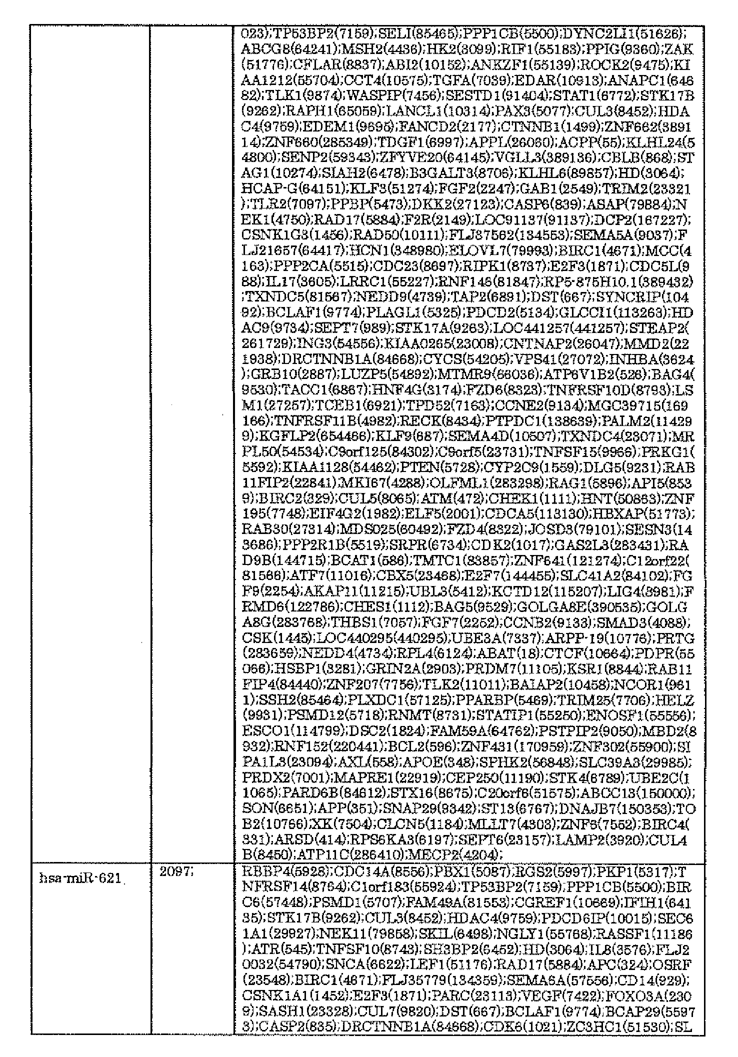

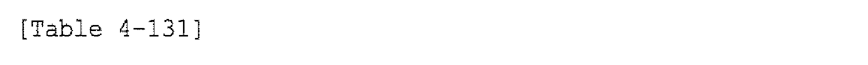

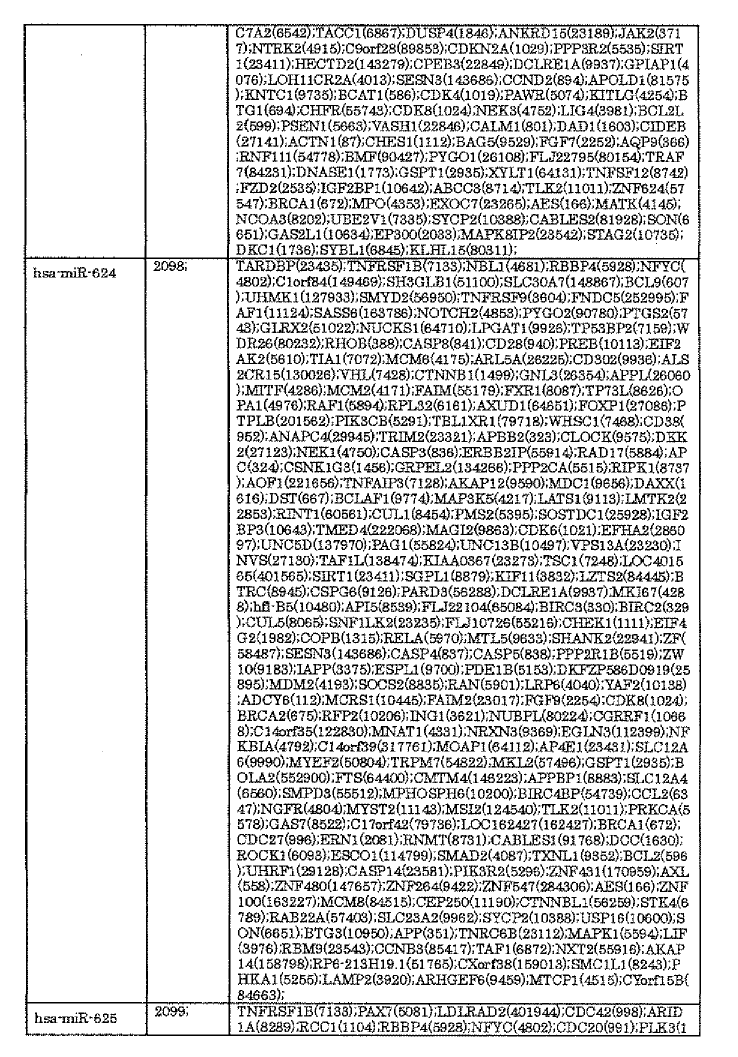

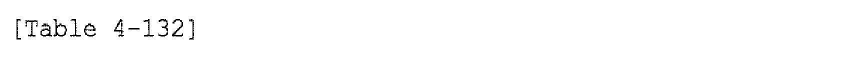

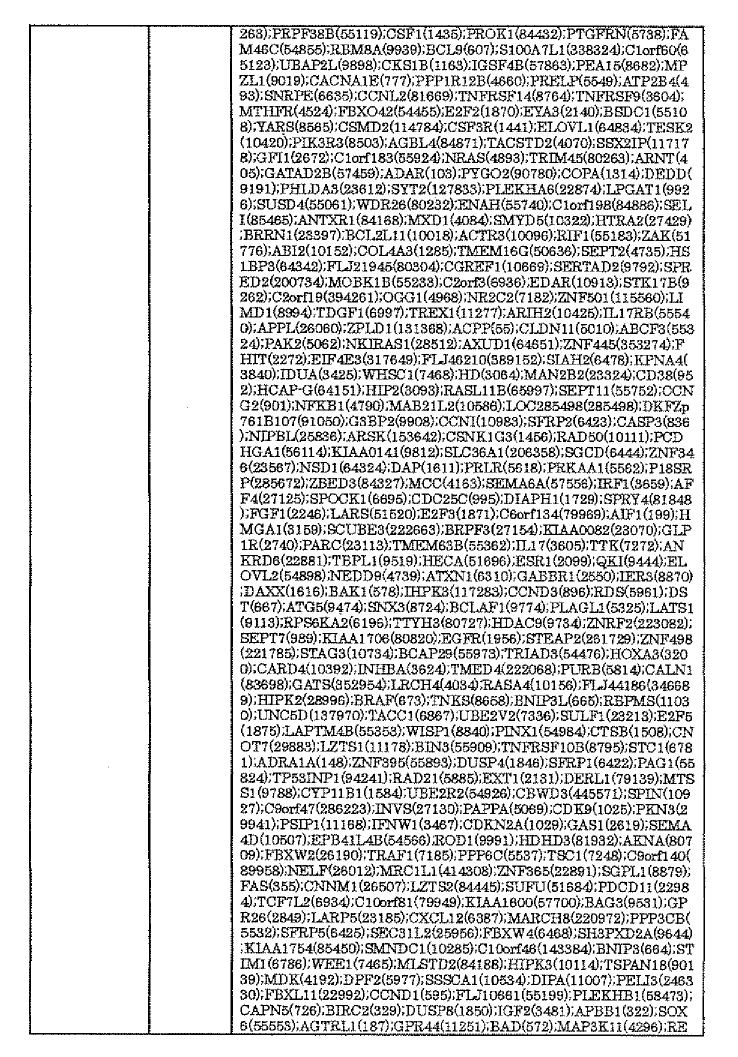

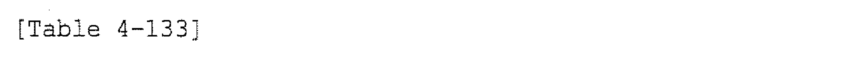

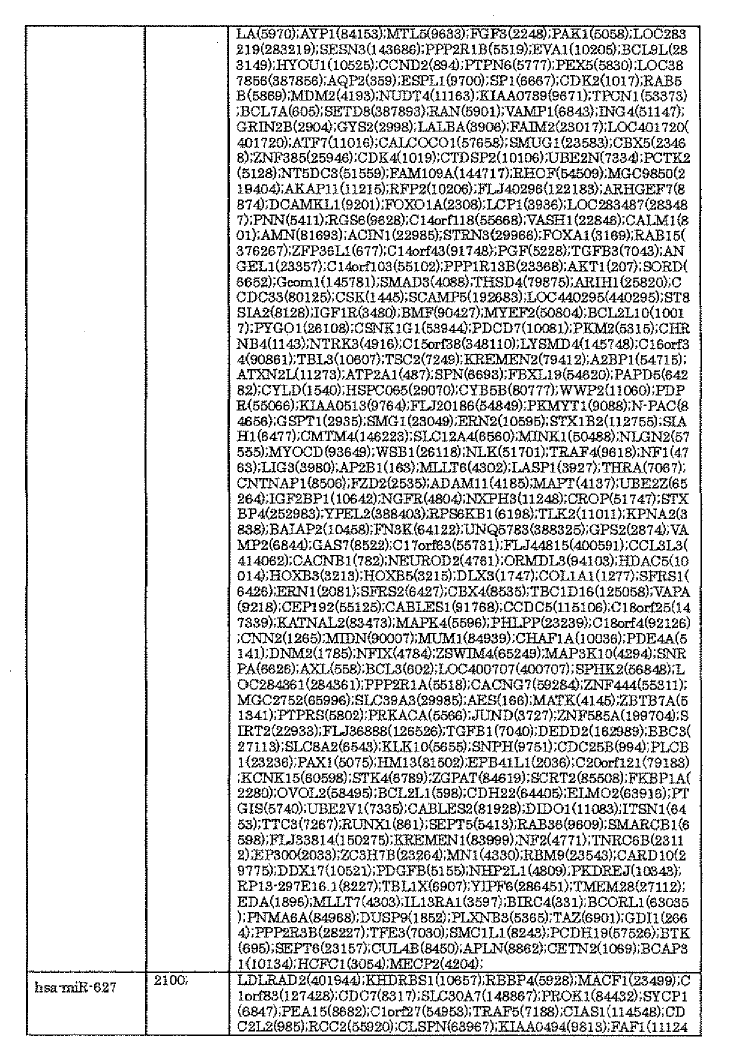

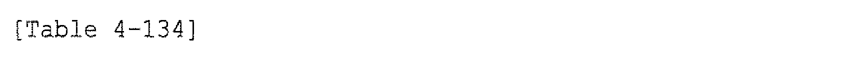

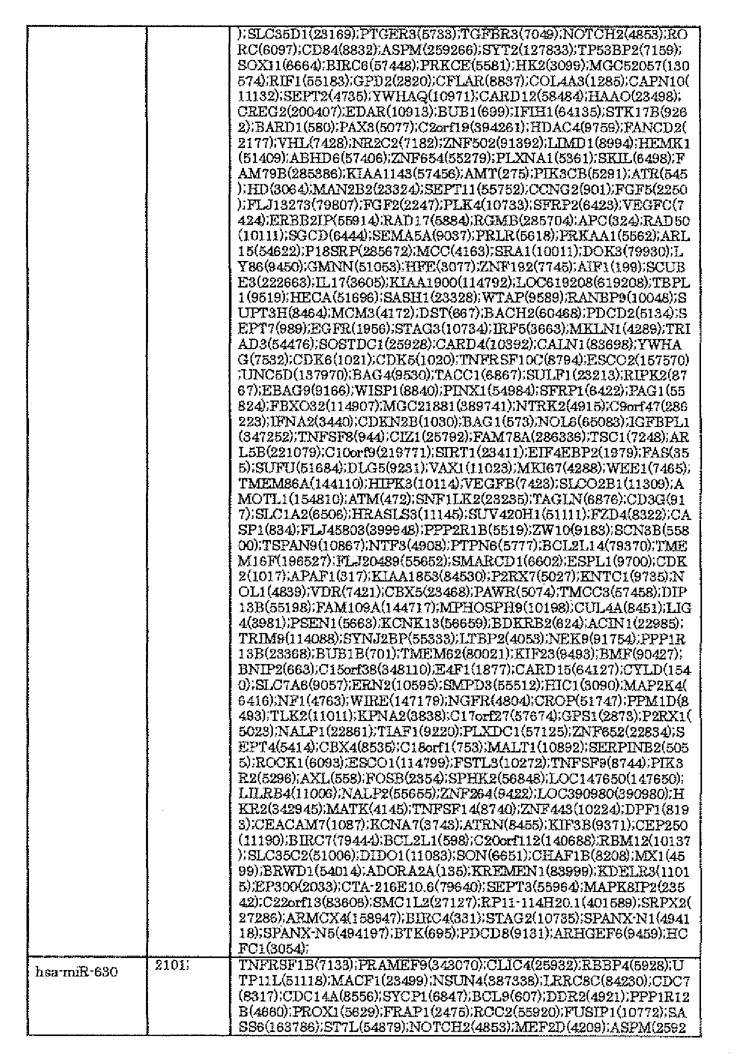

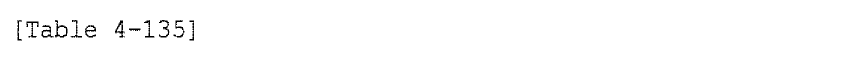

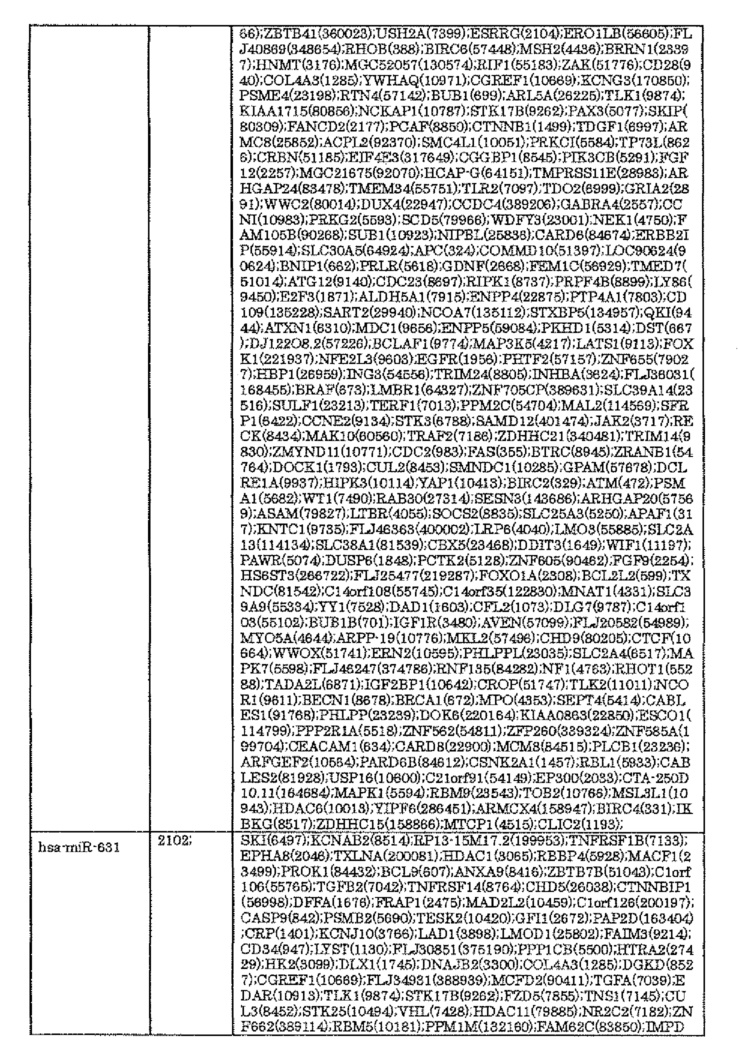

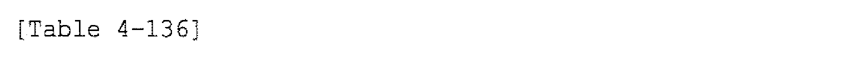

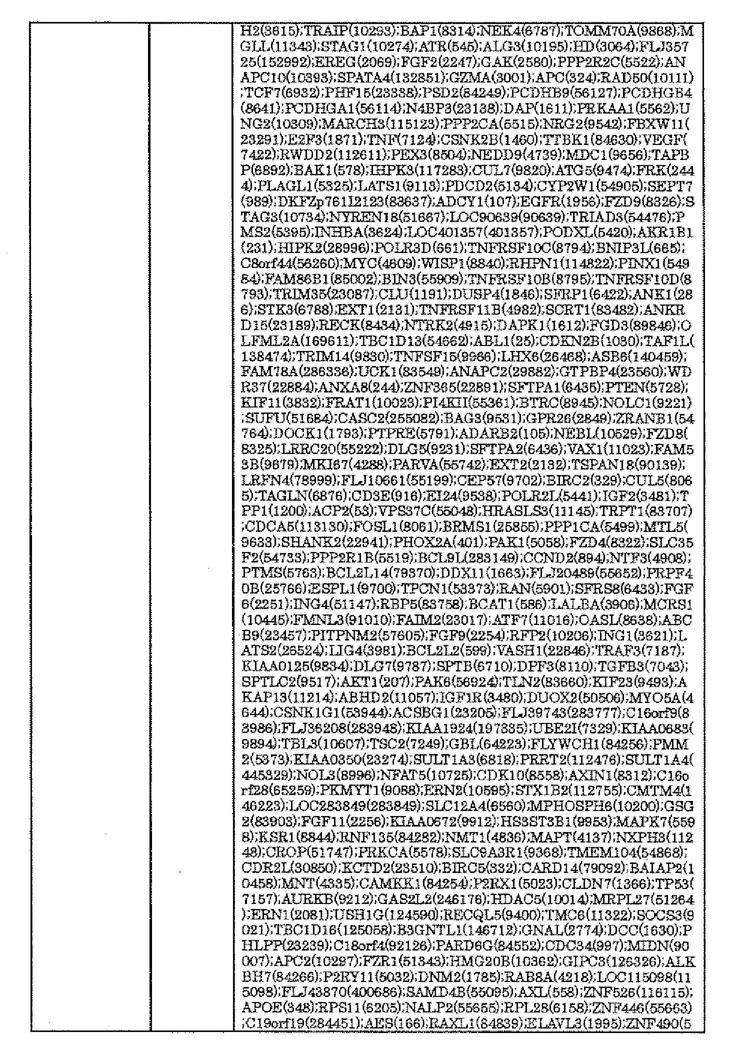

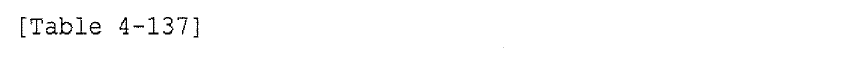

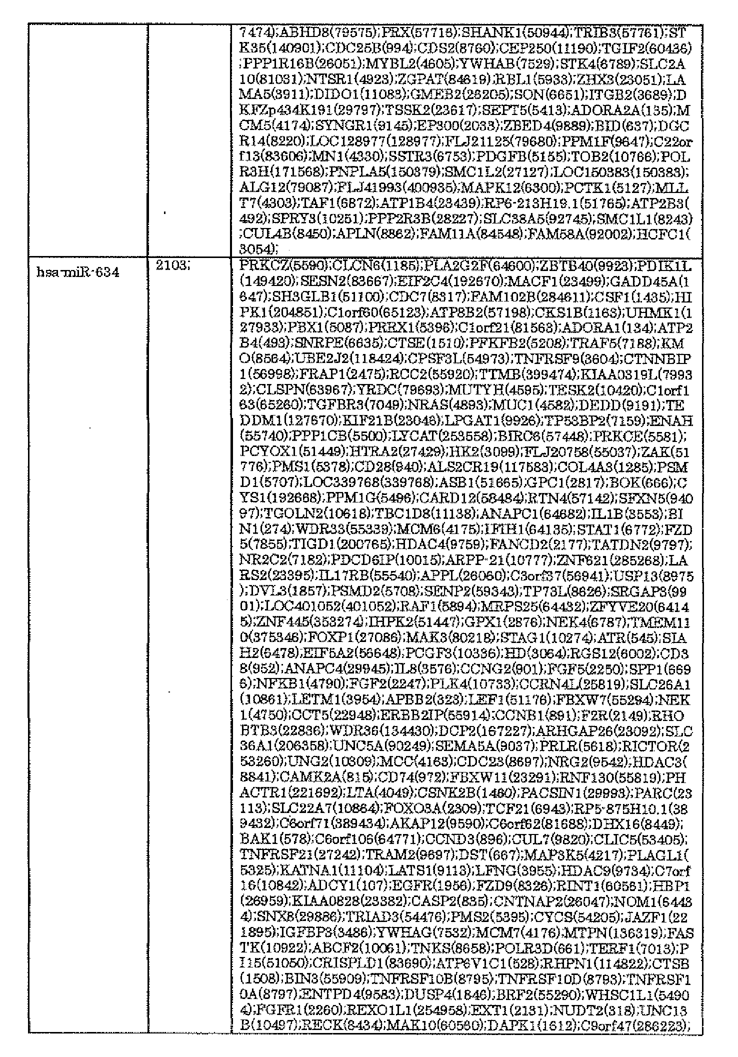

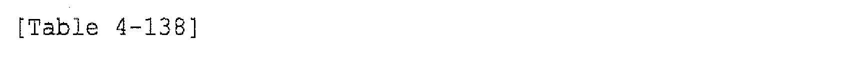

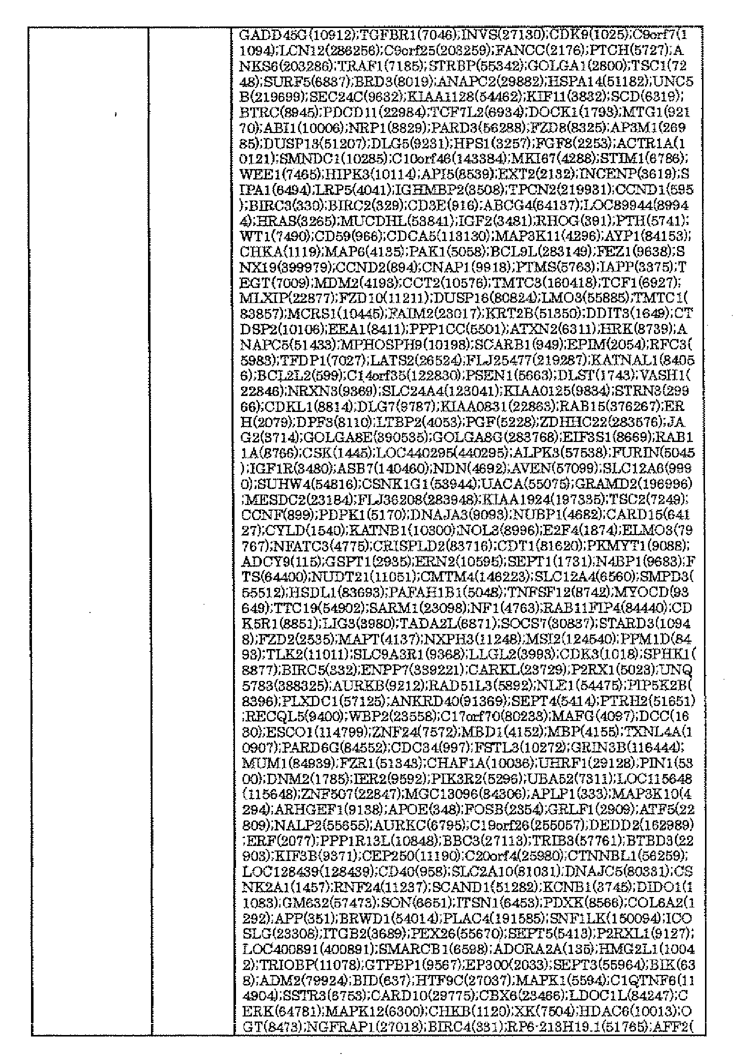

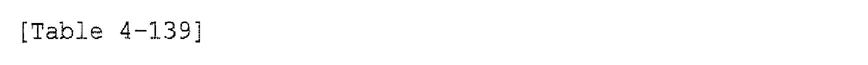

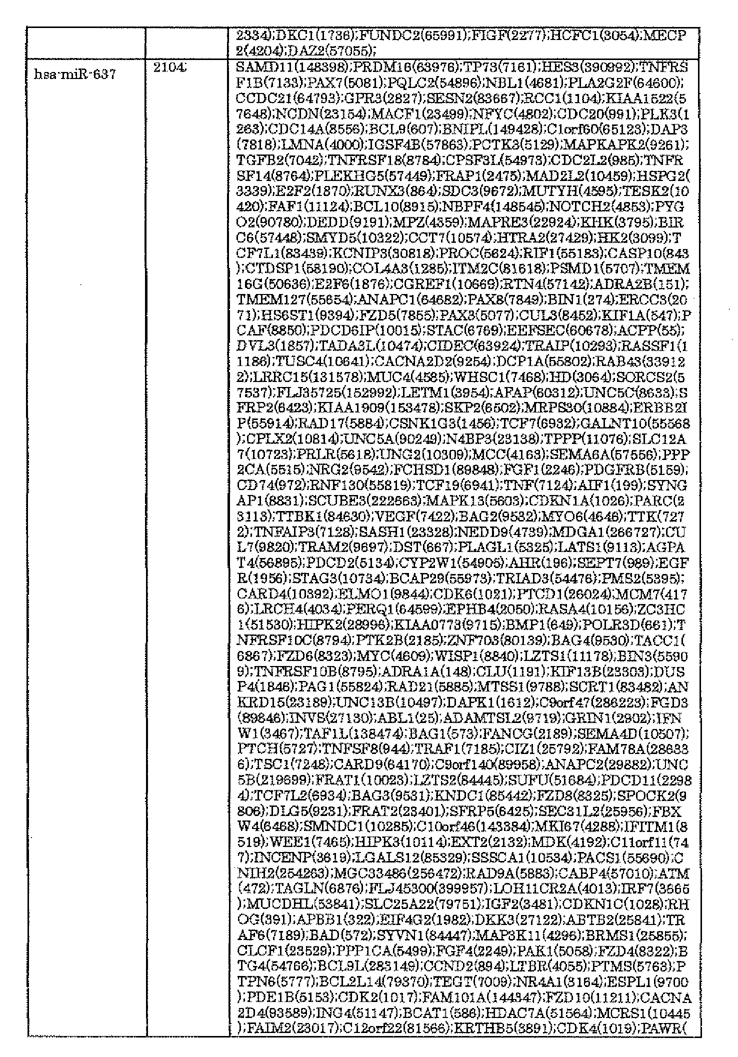

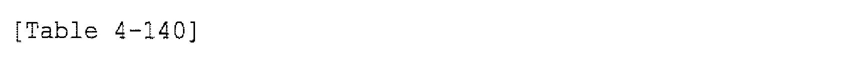

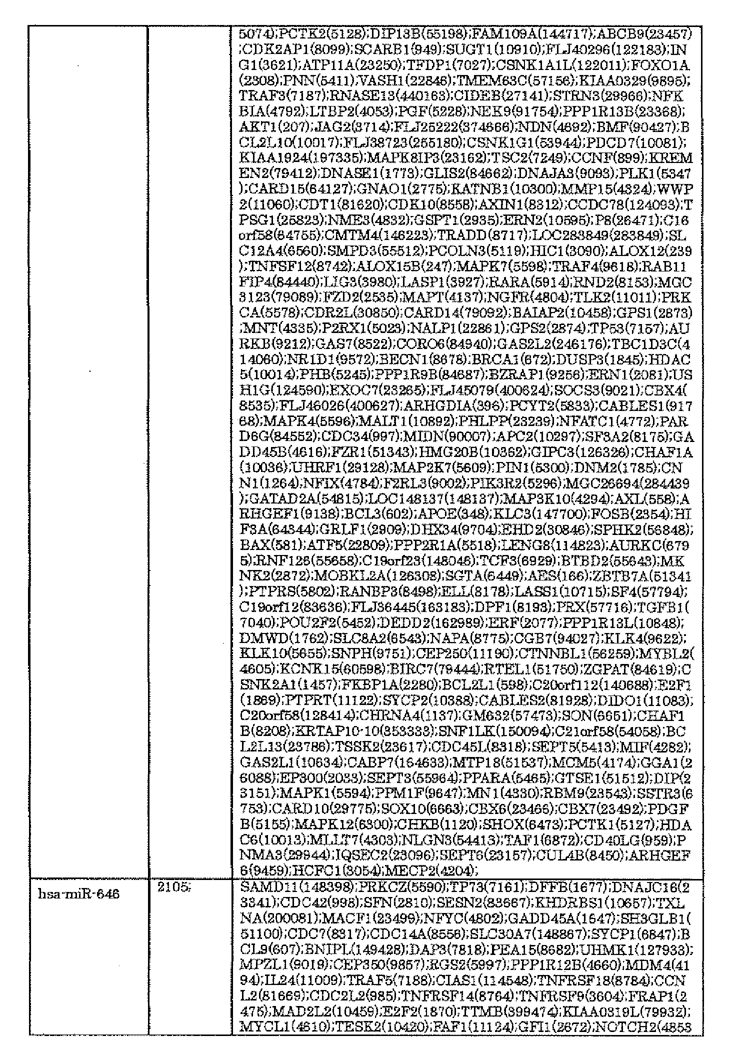

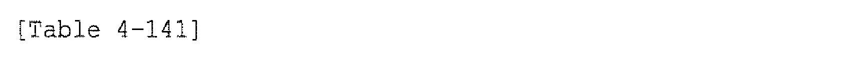

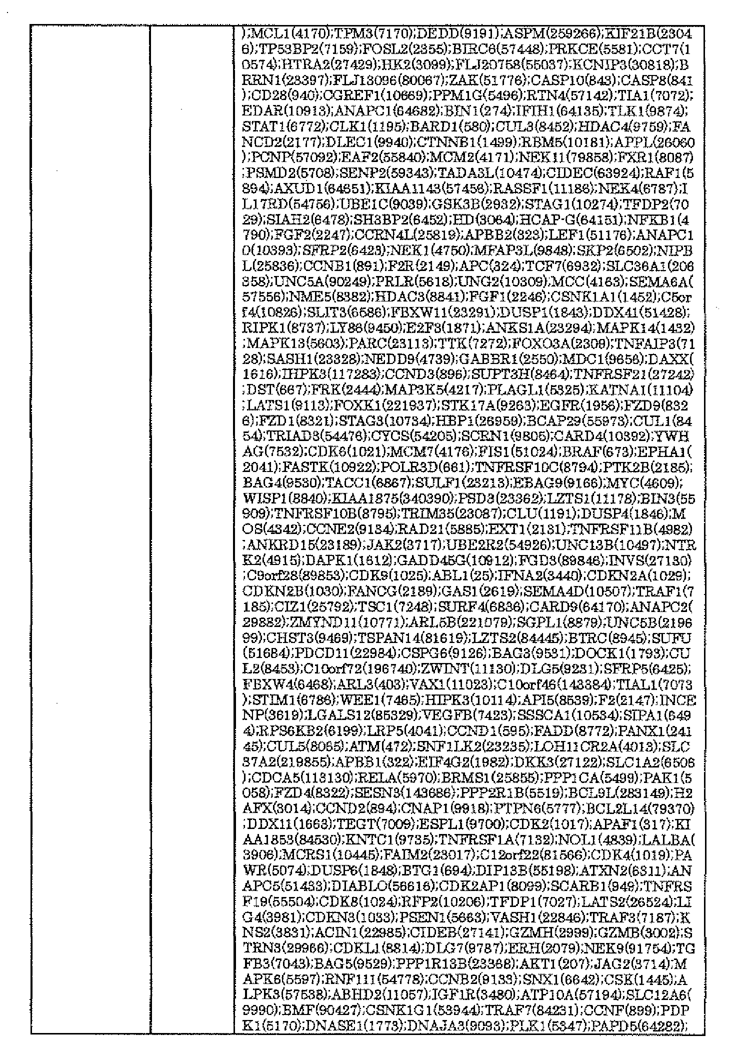

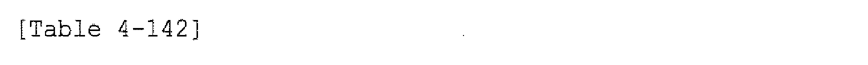

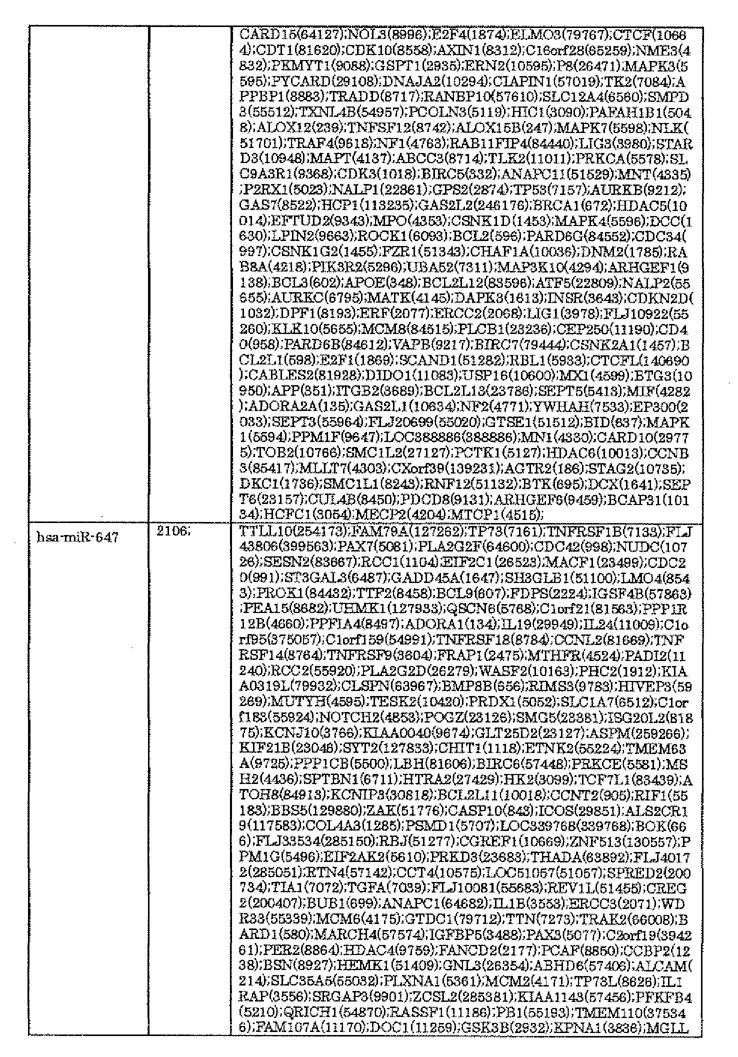

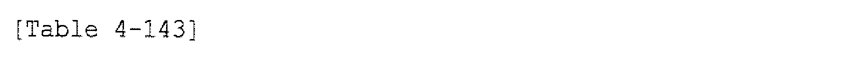

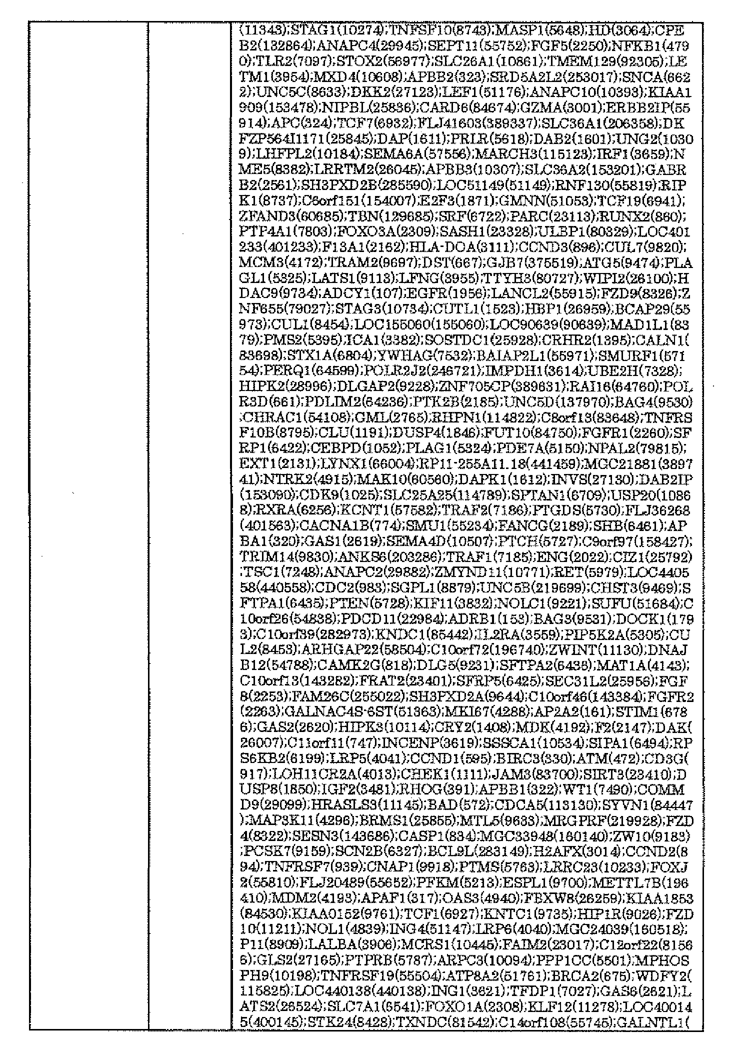

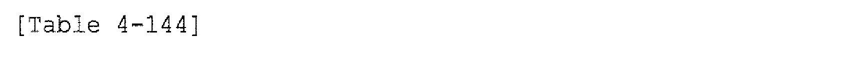

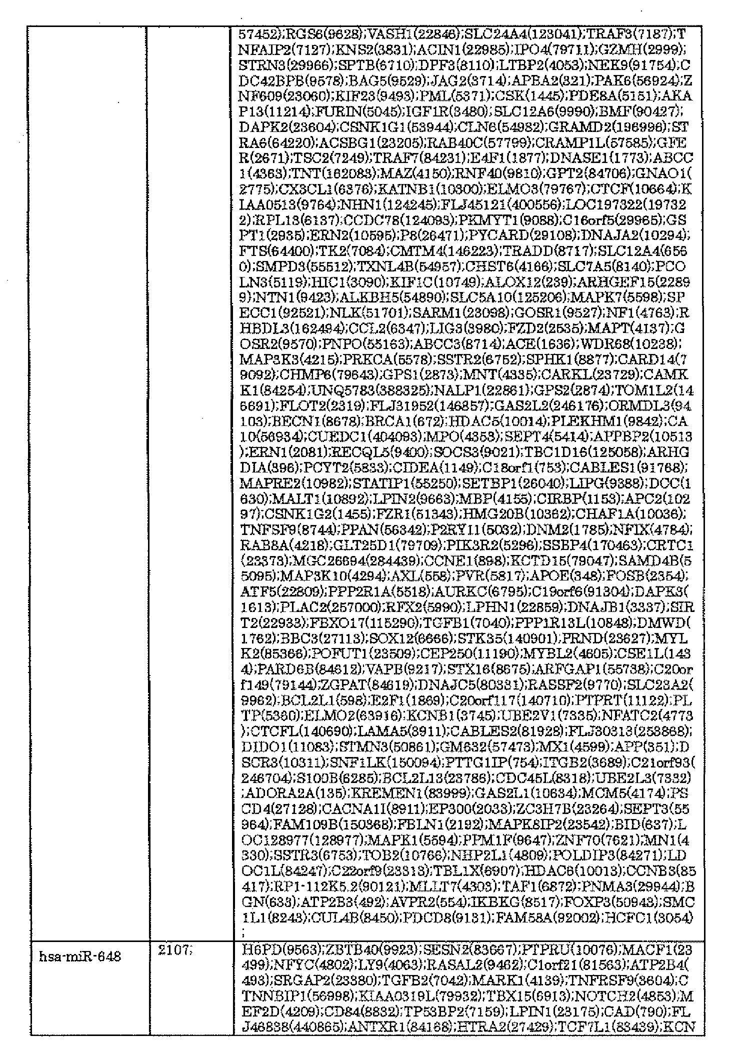

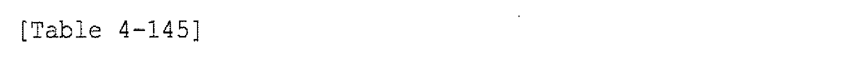

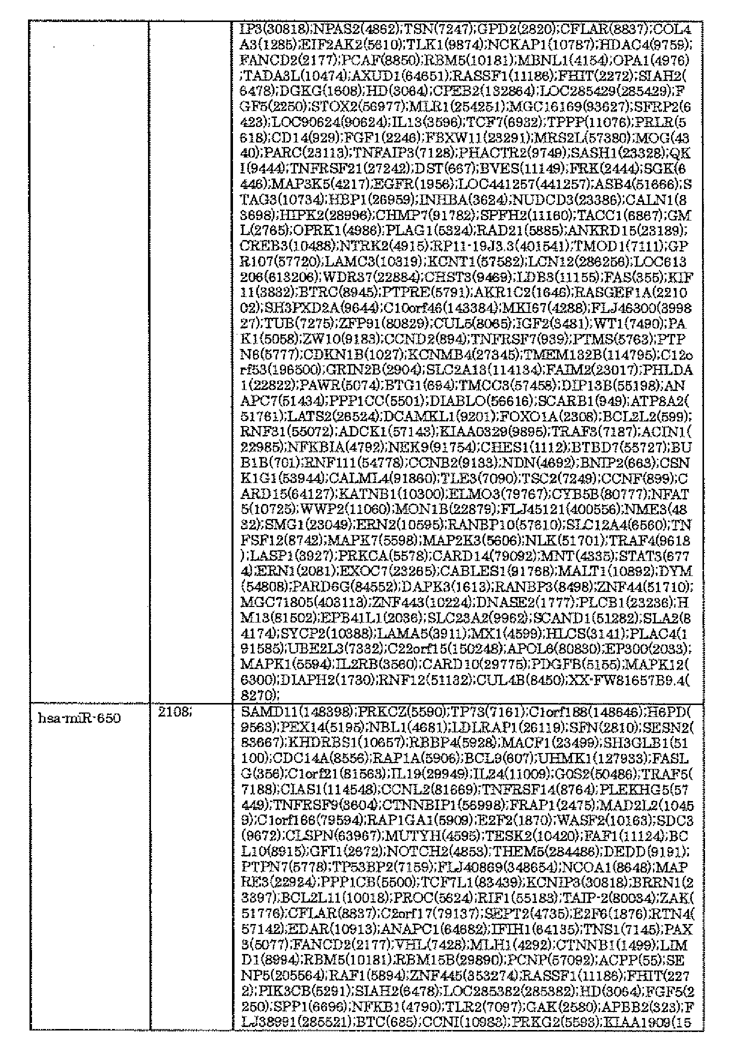

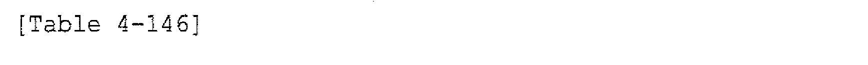

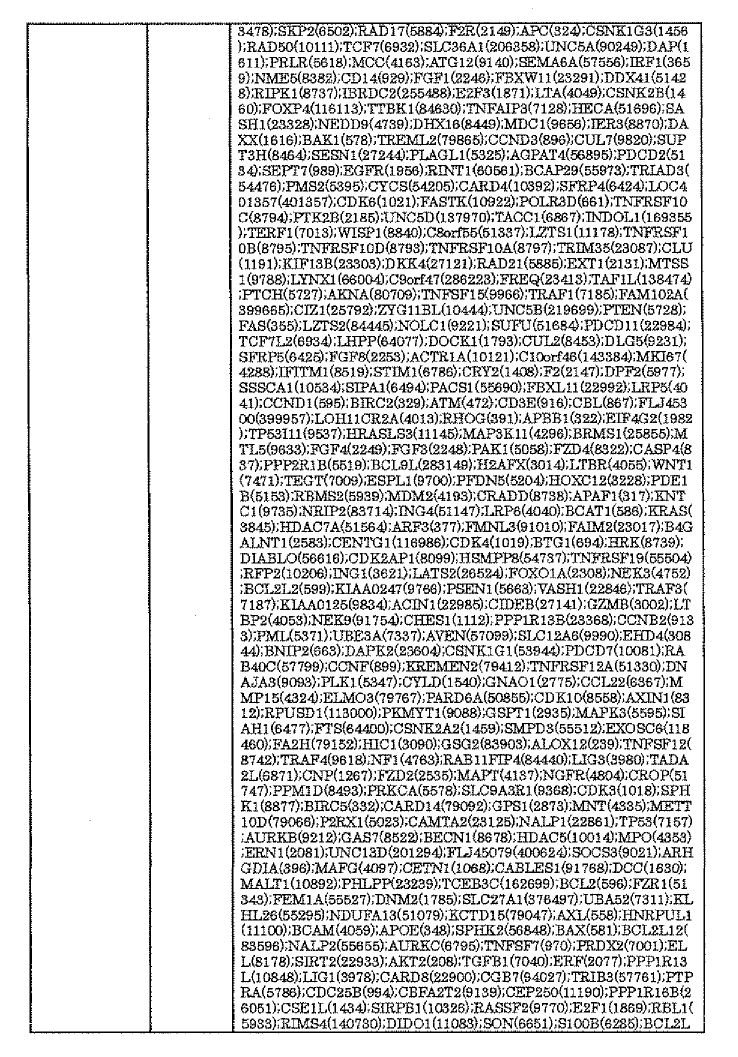

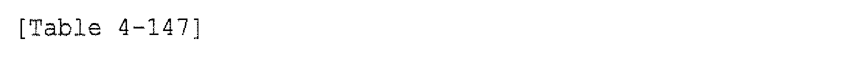

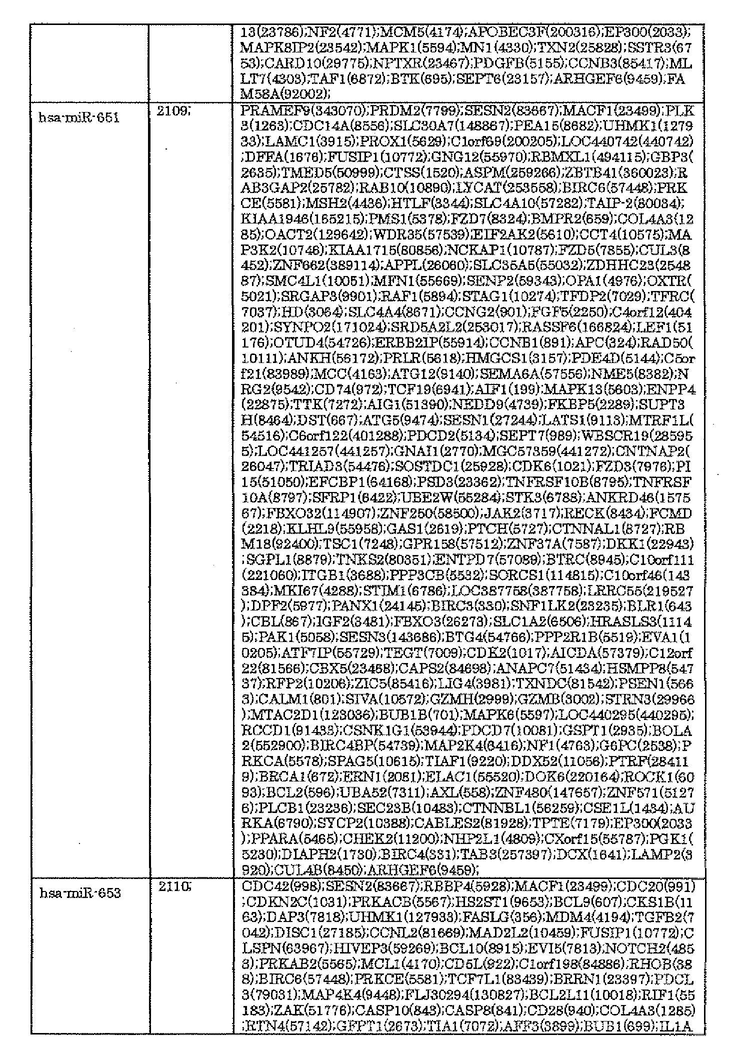

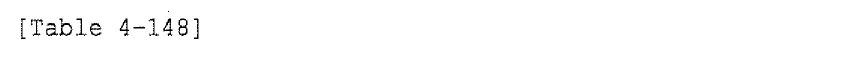

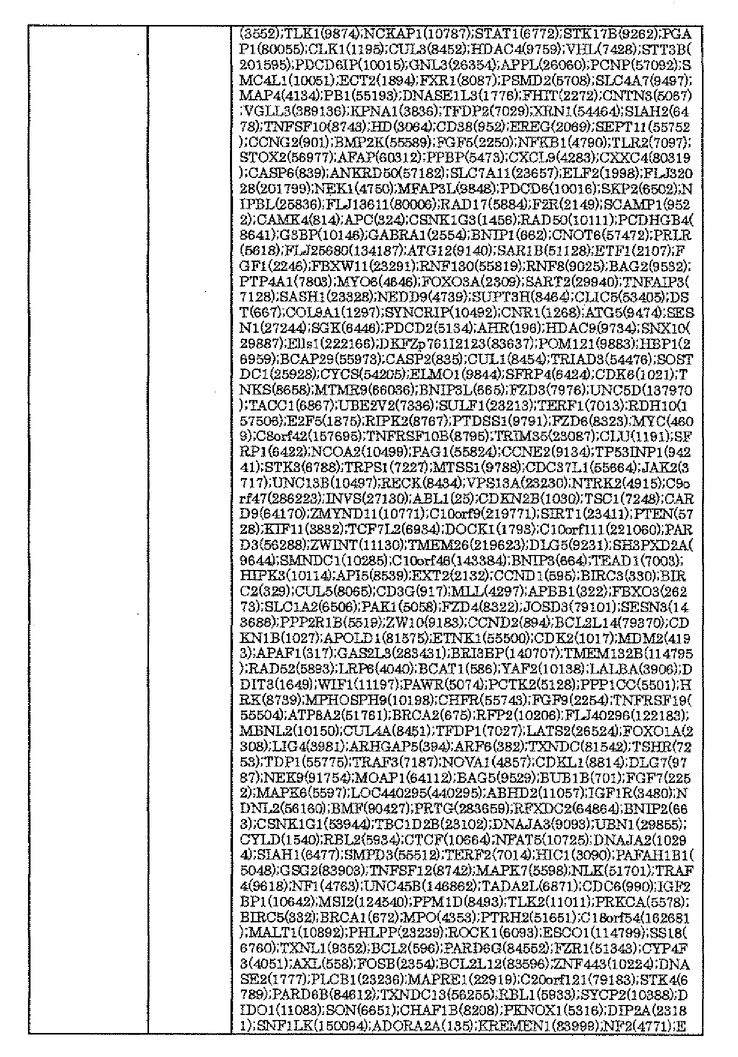

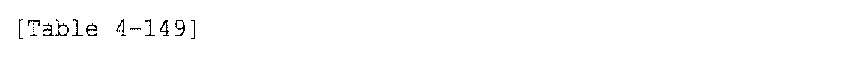

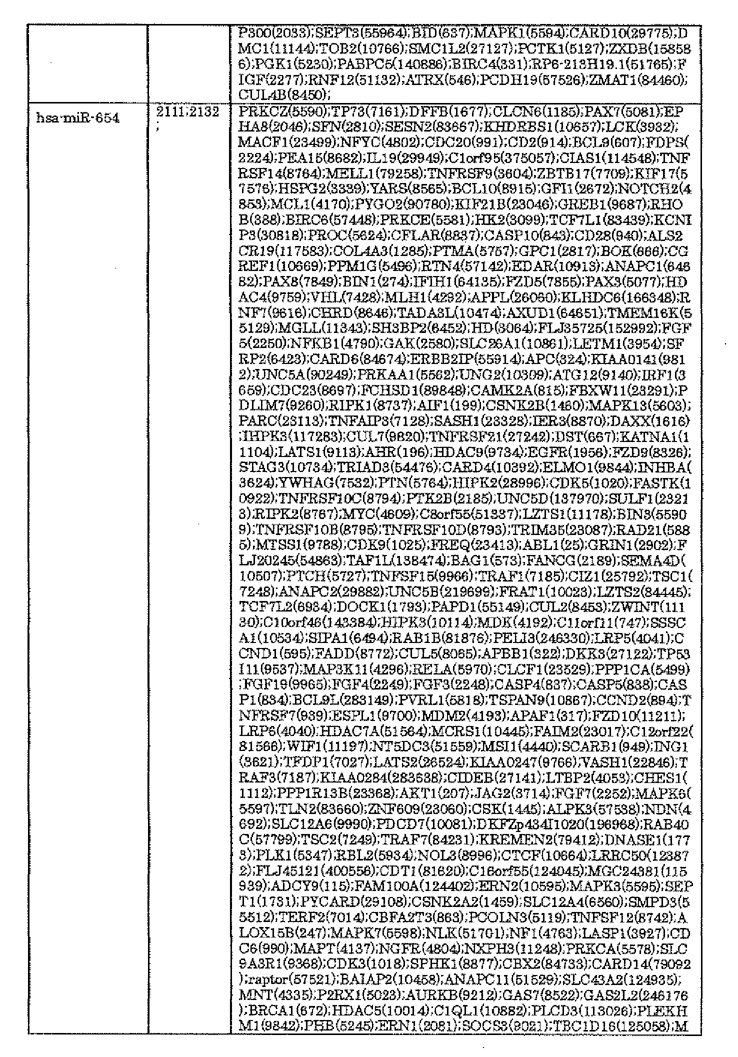

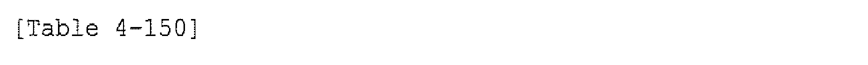

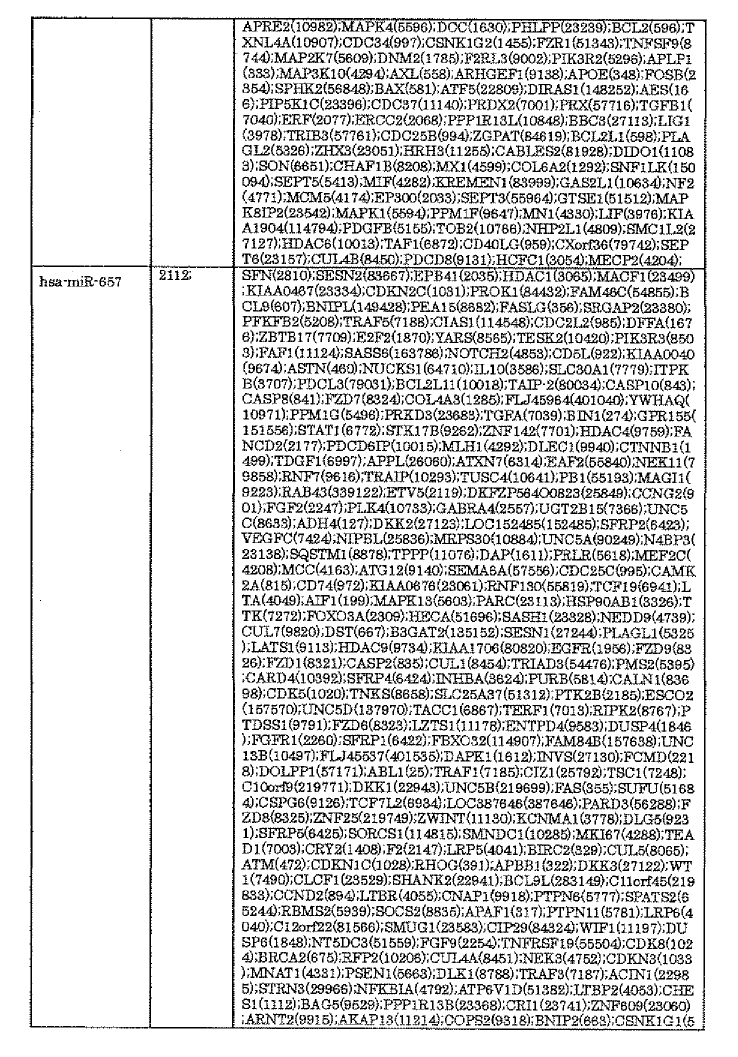

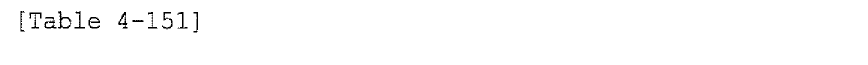

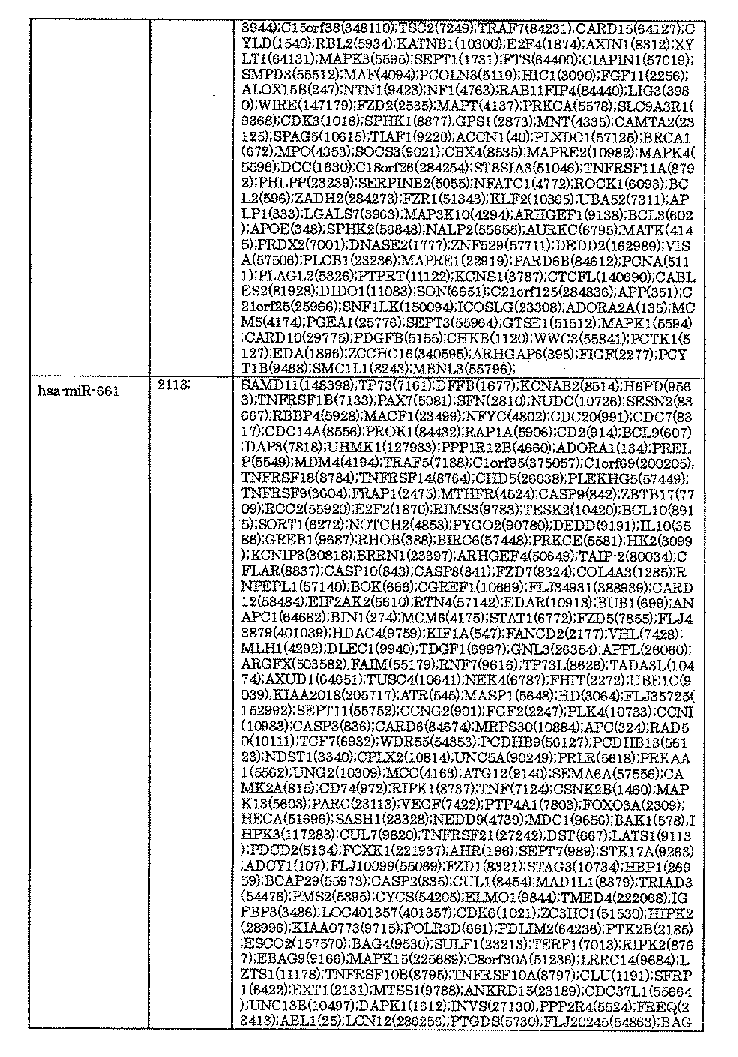

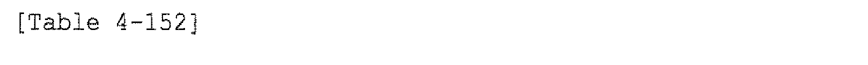

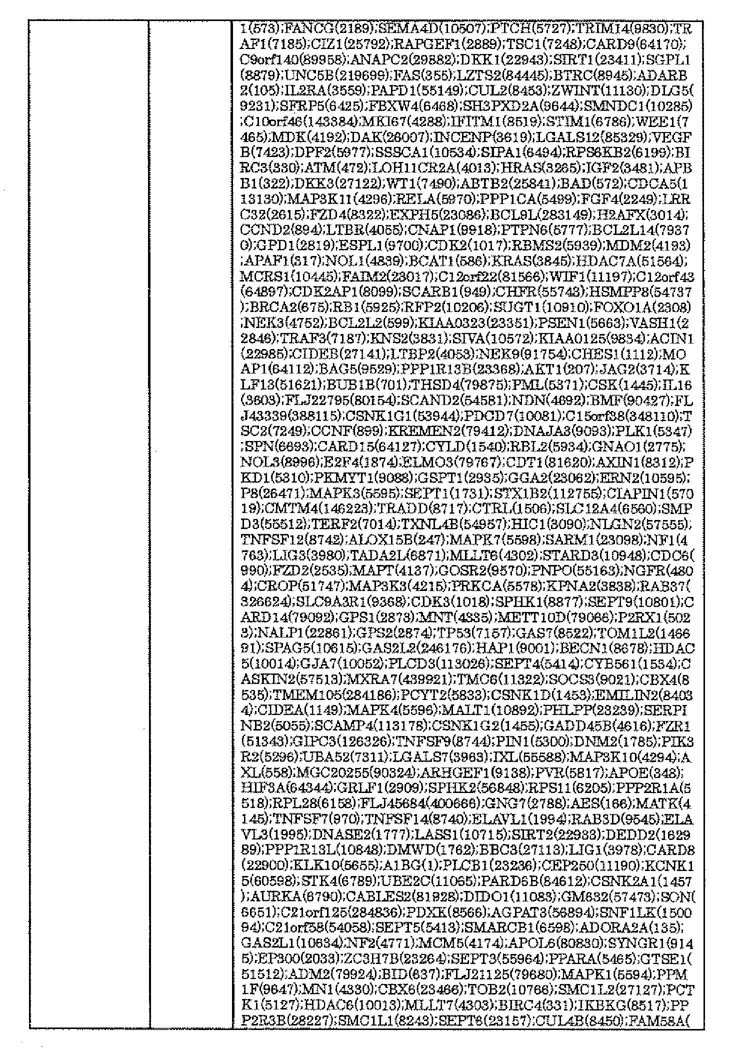

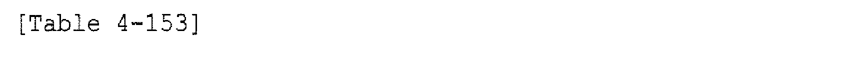

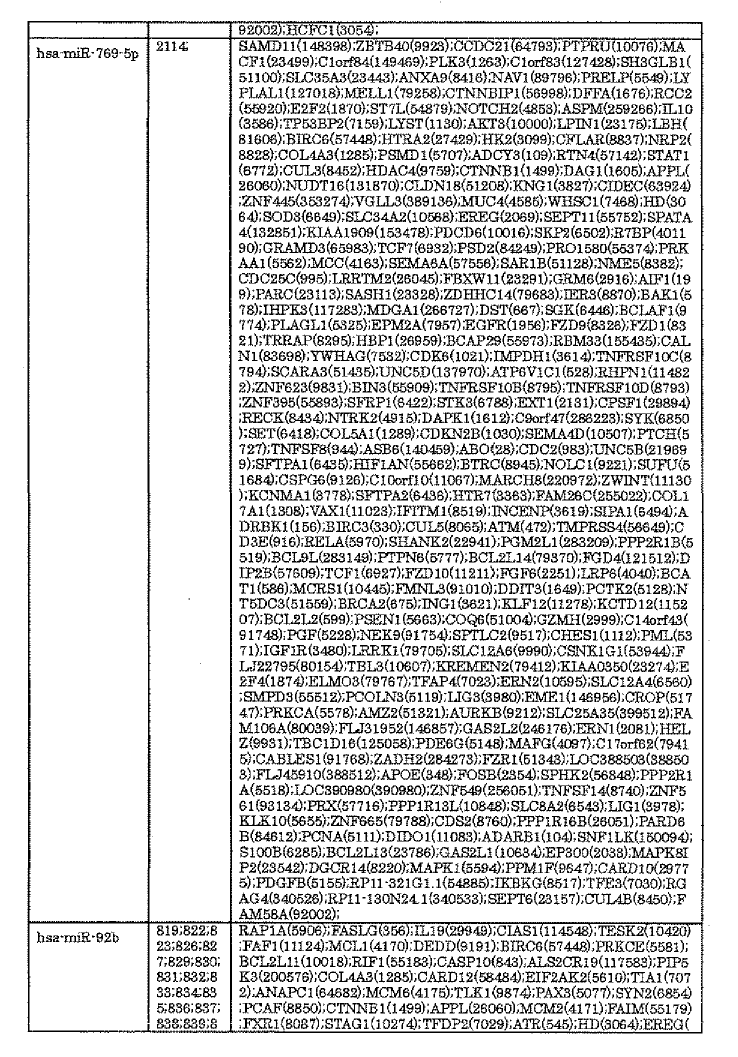

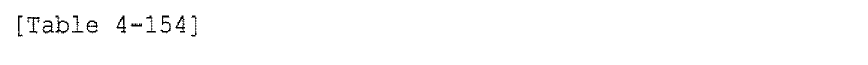

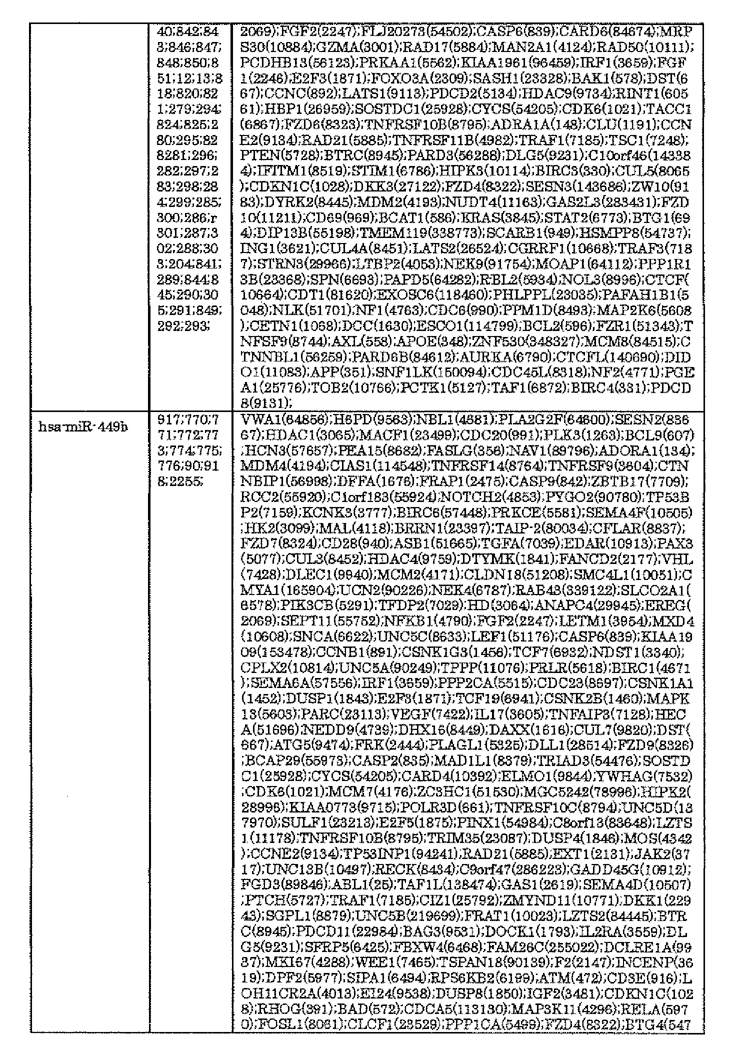

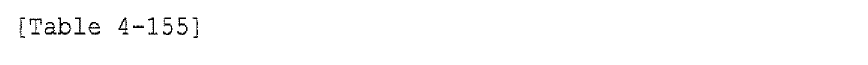

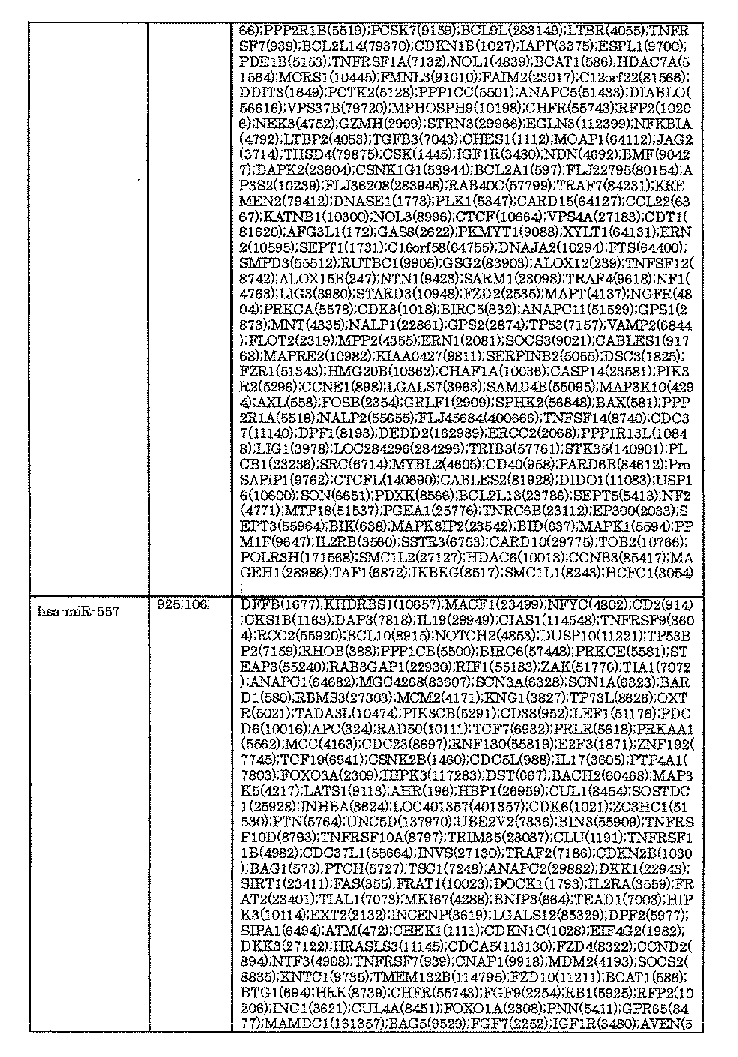

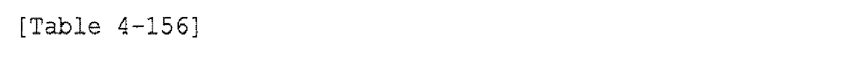

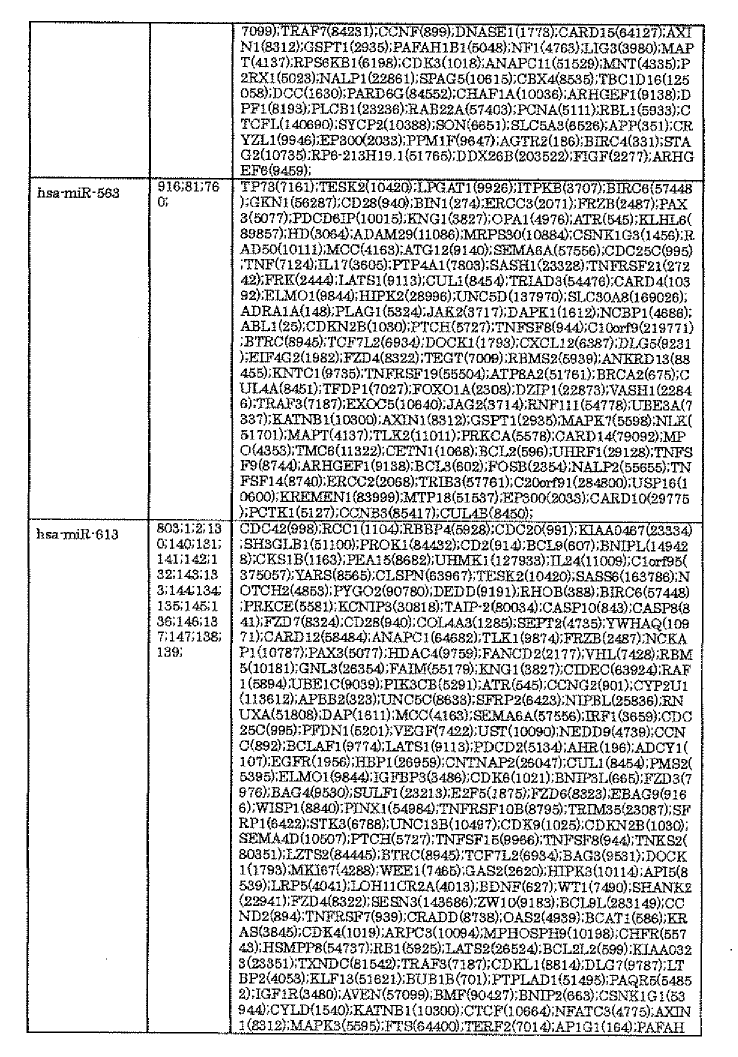

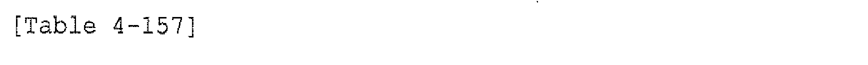

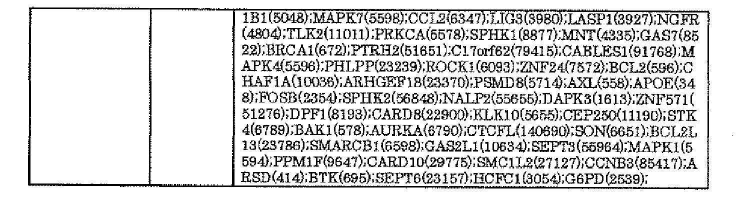

As a method of expressing a nucleic acid such as a miRNA in cells in the present invention, a method using a nucleic acid that causes the expression of a gene that encodes the miRNA and the like when introduced into the cells can be mentioned. As the nucleic acid, a DNA, an RNA, or a nucleotide analogue, as well as a chimeric molecule thereof, or a derivative of the nucleic acid can also be used. Specifically, the nucleic acid can be designed in the same way as with Pre-miR^{™} miRNA Precursor Molecules (manufactured by Ambion) or miRIDIAN microRNA Mimics (manufactured by GE Healthcare), and the nucleic acid such as the miRNA can be expressed in cells. Any method can be used to express a miRNA, as far as it allows the miRNA to be finally formed in the cells; for example, (1)
a method wherein a single-stranded RNA that is a precursor of the miRNA is introduced, as well as (2) a method wherein an RNA consisting of a 100% matched double-strand consisting of the miRNA as it is and a complementary strand for the miRNA, is introduced, and (3) a method wherein a double-stranded RNA assumed to have resulted from cleavage of a miRNA with a Dicer is introduced, can be mentioned. As examples of commercial products based on these methods, miCENTURY OX Precursor (manufactured by B-Bridge), miCENTURY OX siMature (manufactured by B-Bridge), and miCENTURY OX miNatural (manufactured by B-Bridge), respectively, can be mentioned.

The method of synthesizing a nucleic acid to be used in the present invention is not particularly limited; the same can be produced by a method using a publicly known chemical synthesis, or an enzymatic transcription method and the like. As methods using a publicly known chemical synthesis, the phosphoroamidite method, the phosphorothioate method, the phosphotriester method and the like can be mentioned; for example, the same can be synthesized using the ABI3900 high throughput nucleic acid synthesizer (manufactured by Applied Biosystems). As an enzymatic transcription method, transcription with a plasmid or DNA having a desired nucleotide sequence as the template using a typical phage RNA polymerase, for example, T7, T3, or SP6RNA polymerase, can be mentioned.

As an example of a method of screening a substance that promotes or suppresses the expression or a function of a nucleic acid used in the present invention by means of the nucleic acid, a method can be mentioned wherein a vector that expresses the nucleic acid is introduced into cells, and a substance that promotes or suppresses the expression or a function of a mRNA having a target nucleotide sequence therefor is screened for.
A pharmaceutical with a nucleic acid to be used in the present invention as an active ingredient can be used to diagnose or treat a disease caused by an abnormality of cell proliferation and the like, tissue hyperplasia and the like. In addition, a nucleic acid can also be used as an agent for suppressing or promoting cell proliferation. Here, an abnormality of cell proliferation refers to a condition wherein cells are proliferating at a rate that is not a normal proliferation rate in a living organism.

As diseases caused by a cell proliferation abnormality, tissue hyperplasia, and the like, specifically, cancers, arteriosclerosis, rheumatoid arthritis, prostatic hyperplasia, blood vessel restenosis after percutaneous transvascular coronary angioplasty, fibroid lung, glomerulonephritis, autoimmune diseases and the like, preferably cancer, can be mentioned.
Hereinafter, the present invention is described in detail.

### 1. Method of detecting the expression of a nucleic acid such as miRNA or miRNA precursor expressed in cancer cells

### (1-1) Identification of miRNA

Whether or not the small RNA sequence is a miRNA can be determined on the basis of whether or not the criteria described in RNA, 9, 277-279 (2003) are met. For example, in cases where the small RNA was newly acquired and the nucleotide sequence thereof was determined, this can be performed as described below.

A surrounding genomic sequence wherein a DNA sequence corresponding to the nucleotide sequence of the small RNA acquired is extended by about 50 nt toward the 5' terminal side and the 3' terminal side, respectively, is acquired, and the secondary structure of the RNA expected to be transcribed from the genomic sequence is predicted. If the result shows that a hairpin structure is present and the nucleotide sequence of the small RNA is located in one chain of the hairpin, the small RNA can be judged to be a miRNA. Genomic sequences are open to the general public, and are available from, for example, UCSC Genome Bioinformatics (http://genome-ucsc.edu/). For prediction of secondary structures, various programs are open; for example, RNAfold [Nucleic Acids Research 31, 3429-3431 (2003)], Mfold [Nucleic Acids Research 31, 3406-3415 (2003)] and the like can be used. Existing miRNA sequences are registered in the database called miRBase in Sanger Institute, UK; whether or not a miRNA is identical to an existing miRNA can be determined on the basis of whether or not the sequence thereof is identical to one of the sequences listed therein.

### (1-2) Acquisition of sequence information on small RNA

Total RNA is extracted from a human cancer cell line or a cancer patient-derived sample, and a small RNA comprising a miRNA expressed in cancer cells or cancer tissue can be acquired using the total RNA as described below.

As a method of acquiring a small RNA, specifically, a method wherein separation and cutting out of a small RNA by 15% polyacrylamide gel electrophoresis as described in Genes & Development 15, 188-200 (2000), can be mentioned. Then, 5' terminal dephosphorylation, 3'-adapter ligation, phosphorylation, 5'-adapter ligation, reverse transcription, PCR amplification, concatemerization, and ligation to a vector are performed sequentially, thereafter the small RNA is cloned, and the nucleotide sequence of the clone is determined. Alternatively, for example, a method wherein 5'-adenylation 3'-adapter ligation, 5'-adapter ligation, reverse transcription, PCR amplification, concatemerization, and ligation to a vector are performed sequentially, thereafter the small RNA is cloned, and the nucleotide sequence of the clone is also determined, as described in Science 294, 858-862 (2001).

Alternatively, 5' terminal dephosphorylation, 3'-adapter ligation, phosphorylation, 5'-adapter ligation, reverse transcription, PCR amplification, and ligation to a microbead vector are performed sequentially, thereafter the small RNA is cloned, and the nucleotide sequence of the microbeads is read to determine the nucleotide sequence, whereby nucleotide sequence information of the small RNA can also be acquired, as described in Nucleic Acids Research 34, 1765-1771 (2006).

As another method for acquiring a small RNA, a method using a small RNA Cloning Kit (manufactured by Takara Bio Inc.) can be mentioned.

### (1-3) Method of detecting the expression level of a nucleic acid such as a MRNA

As examples of methods of detecting the expression level of a nucleic acid such as miRNA, a miRNA precursor and the like include (1) Northern hybridization, (2) dot blot hybridization, (3) in situ hybridization, (4) quantitative PCR, (5) differential hybridization, (6) microarray, (7) ribonuclease protection assay and the like.

The Northern blot method is a method wherein a sample-derived RNA is separated by gel electrophoresis, then transferred to a solid support such as a nylon filter, and an appropriately-labeled probe is prepared on the basis of the nucleotide sequence of a nucleic acid, and hybridization and washing are performed, whereby a band specifically bound to the nucleic acid is detected; specifically, for example, this method can be performed as described in Science 294, 853-858 (2001) and the like.

A labeled probe can be prepared by incorporating a radioisotope, biotin, digoxigenin, a fluorescent group, a chemiluminescent group and the like in a DNA, RNA, or LNA having a sequence complementary to the nucleotide sequence of a nucleic acid to be used in the present invention by a method, for example, nick translation, random priming or 5'-terminal phosphorylation. Because the amount of labeled probe bound reflects the expression level of the nucleic acid, the expression level of the nucleic acid can be quantified by quantifying the amount of labeled probe bound. Electrophoresis, membrane transfer, probe preparation, hybridization, and nucleic acid detection can be achieved by a method described in Molecular Cloning, 3rd edition [Cold Spring Harbor Press, (2001) Cold Spring Harbor, NY].

Dot blot hybridization is a method wherein an RNA extracted from a tissue or a cell is spotted in dot forms and immobilized on a membrane, and hybridized with a labeled polynucleotide to be a probe, and an RNA that specifically hybridizes with the probe is detected. The probe used may be the same as that used for Northern hybridization. RNA preparation, RNA spotting, hybridization, and RNA detection can be achieved by a method described in Molecular Cloning, 3rd edition.

In situ hybridization is a method wherein a paraffin-embedded or cryostat-treated section of a tissue acquired from a living organism, or a cell fixed, is used as a sample and subjected to steps for hybridization with a labeled probe and washing, and the distribution and localization of a nucleic acid in the tissue or cell are examined by microscopic examination [Methods in Enzymology, 254, 419 (1995)]. The probe used may be the same as that used for Northern hybridization. Specifically, a miRNA can be detected in accordance with a method described in Nature Method 3, 27 (2006).

In quantitative PCR, a cDNA synthesized from a sample-derived RNA using a primer for reverse transcription and a reverse transcriptase (hereunder, this cDNA is referred to as a sample-derived cDNA) is used for the measurement. As a primer for reverse transcription to be supplied for cDNA synthesis, a random primer or a specific RT primer and the like can be used. A specific RT primer refers to a primer having a sequence complementary to a nucleotide sequence corresponding to a nucleic acid and a genomic sequence therearound.

For example, a sample-derived cDNA is synthesized, after which a PCR is performed with this cDNA as the template, using a template-specific primer designed from a nucleotide sequence corresponding to a nucleic acid such as miRNA or miRNA precursor and a genomic sequence therearound, or from a nucleotide sequence corresponding to a primer for reverse transcription, to amplify a cDNA fragment containing the nucleic acid, and the amount of the nucleic acid contained in the sample-derived RNA is detected from the number of cycles for reach to a given amount of the fragment. As the template-specific primer, an appropriate region corresponding to a nucleic acid and a genomic sequence therearound is selected, and a pair of a DNA or LNA consisting of a sequence of 15 to 40 nucleotides at the 5'terminus, preferably 20 to 30 nucleotides of the nucleotide sequence of the region, and a DNA or LNA consisting of a sequence complementary to a sequence of 15 to 40 nucleotides at the 3' terminus, preferably 20 to 30 nucleotides can be used. Specifically, this can be performed in accordance with a method described in Nucleic Acids Research, 32, e43 (2004) and the like.

Alternatively, as the primer for reverse transcription to be supplied for cDNA synthesis, a specific RT primer having a stem-loop structure can also be used. Specifically, this can be performed using a method described in Nucleic Acid Research, 33, e179 (2005), or TaqMan MiRNA Assays (manufactured by Applied Biasystems).
As still another method of synthesizing a sample-derived cDNA, a polyA sequence is added to a sample-derived RNA by means of polyA polymerase, and a nucleotide sequence comprising an oligodT sequence is used as a primer for reverse transcription, whereby a reverse transcription reaction can be performed. Specifically, this can be performed using the miScript System (manufactured by QIAGEN) or the QuantiMir RT Kit (manufactured by System Biosciences). Furthermore, by hybridizing a sample-derived RNA or cDNA to a DNA or LNA corresponding to a nucleotide sequence comprising at least one or more of a nucleic acid immobilized on a substrate such as a filter, glass slide, or silicone having, and performing washing, a change in the amount of the nucleic acid can be detected.

As such methods based on hybridization, methods using differential hybridization [Trends Genet., 7, 314 (1991)] or a microarray [Genome Res., 6, 639 (1996)] can be mentioned. Both methods enable accurate detection of a difference in the amount of a nucleic acid between a control sample and a target sample by immobilizing an internal control, such as a nucleotide sequence corresponding to U6RNA, on a filter or a substrate. Also, by synthesizing labeled cDNAs using differently labeled dNTPs (mixtures of dATP, dGTP, dCTP, and dTTP) on the basis of RNAs derived from a control sample and a target sample, and simultaneously hybridizing the two labeled cDNAs to a single filter or a single substrate, accurate quantitation of a nucleic acid can be performed. Furthermore, quantitation of a nucleic acid can also be performed by directly labeling and hybridizing an RNA derived from a control sample and/or a target sample. For example, a miRNA can be detected using a microarray described in Proc. Natl. Acad. Sci. USA, 101, 9740-9744 (2004), Nucleic Acid Research, 32, e188 (2004), RNA, 13, 151-159 (2007) and the like. Specifically, a miRNA can be detected or quantified in the same manner as mirVana miRNA Bioarray (manufactured by Ambion) and miRNA microarray kit (manufactured by Agilent Technologies).

In ribonuclease protection assay, first, a promoter sequence such as the T7 promoter or the SP6 promoter is bound to the 3' terminus of a nucleotide sequence corresponding to a nucleic acid or a genomic sequence therearound, and a labeled antisense RNA is synthesized with an in vitro transcription system using a labeled NTP (a mixture of ATP, GTP, CTP, and UTP) and an RNA polymerase. The labeled antisense RNA is bound to a sample-derived RNA to form an RNA-RNA hybrid, after which the hybrid is digested with ribonuclease A, which degrades single-stranded RNAs only. The digest is subjected to gel electrophoresis to detect or quantify an RNA fragment protected against the digestion by forming the RNA-RNA hybrid, as a nucleic acid. Specifically, the fragment can be detected or quantified using the mirVana miRNA Detection Kit (manufactured by Ambion).

### 2. Synthesis of nucleic acid

After a nucleic acid such as a miRNA or miRNA precursor expressed in cancer cells or cancer tissue, or exhibiting increased or decreased expression compared with normal tissue, has been identified as described in 1 above, not only an RNA, which is a polymer of a ribonucleotide, but also a DNA, which is a polymer of a deoxyribonucleotide, can be synthesized on the basis of the nucleotide sequences. For example, on the basis of the nucleotide sequence of the miRNA identified in 1 above, the nucleotide sequence of a DNA can be determined. The nucleotide sequence of a DNA corresponding to the nucleotide sequence of an RNA can be determined, without exception, by reading the U (uracil) contained in the sequence of the RNA as T (thymine). A polymer being a mixture of a ribonucleotide and a deoxyribonucleotide and a polymer comprising a nucleotide analogue and a derivative of a nucleic acid can also be synthesized in the same manner.

The method of synthesizing a nucleic acid is not particularly limited; the same can be produced by a method using a publicly known chemical synthesis, or an enzymatic transcription method and the like. As methods using a publicly known chemical synthesis, the phosphoroamidite method, the phosphorothioate method, the phosphotriester method and the like can be mentioned; for example, a nucleic acid can be synthesized using the ABI3900 high throughput nucleic acid synthesizer (manufactured by Applied Biosystems). As an enzymatic transcription method, a method by transcription with a plasmid or DNA having a desired nucleotide sequence as the template using a typical phage RNA polymerase, for example, T7, T3, or SP6RNA polymerase, can be mentioned.

### 3. Method of detecting a function of a nucleic acid such as miRNA or miRNA precursor

As a method of detecting a function of a nucleic acid such as a miRNA, a method can be mentioned wherein the function is detected on the basis of whether or not the translation of the mRNA having a target nucleotide sequence is suppressed.

MiRNAs are known to suppress the translation of a mRNA comprising a target nucleotide sequence thereof in the untranslated region on the 3' side (3'UTR) [Current Biology, 15, R458-R460 (2005)]. Hence, a DNA wherein a target nucleotide sequence for the single-stranded RNA to be measured is inserted into the 3'UTR of an appropriate reporter gene expression vector is prepared and introduced into a host cell suitable for the expression vector, and the expression of the reporter gene is measured when the cell is allowed to express the single-stranded RNA, whereby whether or not a function of a miRNA is possessed can be determined.

The reporter gene expression vector may be any one, as far as it has a promoter upstream of a reporter gene, and is capable of expressing the reporter gene in the host cell. Any reporter gene can be used; for example, the firefly luciferase gene, the Renilla luciferase gene, the chloramphenicol acetyltransferase gene, the β-glucuronidase gene, the β-galactosidase gene, the β-lactamase gene, the aequorin gene, the green fluorescent protein gene, the DsRed fluorescence gene and the like can be utilized. As examples of reporter gene expression vectors having these properties, psiCHECK-1 (manufactured by Promega), psiCHECK-2 (manufactured by Promega), pGL3-Cantrol (manufactured by Promega), pGL4 (manufactured by Promega), pRNAi-GL (manufactured by Takara Bio Inc.), pCMV-DsRed-Express (manufactured by CLONTECH) and the like can be mentioned. A single-stranded RNA can be expressed by the method described in 6 below.

As the function of a miRNA of a single-stranded RNA, specifically, detection can be achieved as described bellow. First, host cells are cultured on a multi-well plate and the like, and a reporter gene expression vector having a target sequence and a single-stranded RNA are expressed. Thereafter, reporter activity is measured both when the single-stranded RNA is and is not expressed, based on which the function as the miRNA of the single-stranded RNA can be detected.

### 4. Method of detecting a mutation of a nucleic acid such as miRNA or mRNA precursor

As a method of detecting a mutation of a nucleic acid such as a miRNA or miRNA precursor, a method can be used wherein a hetero-double-strand formed by hybridization of a normal type nucleic acid and a mutated type nucleic acid are detected.

As methods of detecting a hetero-double-strand, (1) detection of a hetero-double-strand by polyacrylamide gel electrophoresis [Trends genet., 7, 5 (1991)], (2) single-strand conformation polymorphism analysis [Genomics, 16, 325-332 (1993)], (3) chemical cleavage of mismatches (CCM) [Human Genetics (1996), Tom Strachan and Andrew P. Read, BIOS Scientific Publishers Limited], (4) enzymatic cleavage of mismatches [Nature Genetics, 9, 103-104 (1996)], (5) denatured gel electrophoresis [Mutat. Res., 288, 103-112 (1993)] and the like can be mentioned.

Detection of a hetero-double-strand by polyacrylamide gel electrophoresis is, for example, performed as described below. First, with a sample-derived DNA or a sample-derived cDNA as the template, and using a primer designed on the basis of a genomic nucleotide sequence comprising the nucleotide sequence of a nucleic acid, a fragment smaller than 200 bp is amplified. Hetero-double-strands, if formed, are slower in mobility than mutation-free homo-double-strands, and can be detected as extra bands. In the case of search for a fragment smaller than 200 bp, almost any insertions, deletions, and substitutions of one or more nucleotides can be detected. It is desirable that hetero-double-strand analysis be performed using a single gel in combination with the single strand conformation analysis described below.

In single strand conformation polymorphism analysis (SSCP analysis), a DNA amplified as a fragment smaller than 200 bp with a sample-derived DNA or sample-derived cDNA as the template, using a primer designed on the basis of a genomic nucleotide sequence comprising the nucleotide sequence of a nucleic acid, is denatured, after which it is electrophoresed in non-denatured polyacrylamide gel. By labeling the primer with an isotope or a fluorescent dye at the time of DNA amplification, or by silver-staining the nfln-labeled amplification product, the amplified DNA can be detected as a band. To clarify a difference from the wild type pattern, a control sample may be electrophoresed simultaneously, whereby a fragment with a mutation can be detected on the basis of a difference in mobility.

In chemical cleavage of mismatches (CCM method), a DNA fragment amplified with a sample-derived DNA or sample-derived cDNA as the template, using a primer designed on the basis of a genomic nucleotide sequence comprising the nucleotide sequence of a nucleic acid is hybridized to a labeled nucleic acid prepared by allowing a nucleic acid to incorporate an isotope or a fluorescent target, and treated with osmium tetraoxide to cleave one strand of the DNA at the mismatched portion, whereby a mutation can be detected. CCM is one of the most sensitive methods of detection, and can be applied to samples of kilobase length.

In place of osmium tetraoxide used above, T4 phage resolvase and an enzyme involved in mismatch repair in cells, such as endonuclease VII, and RNaseA may be used in combination to enzymatically cleave a mismatch. In denaturing gradient gel electrophoresis (DGGE method), a DNA amplified with a sample-derived DNA or sample-derived cDNA as the template, using a primer designed on the basis of a genomic nucleotide sequence comprising the nucleotide sequence of a nucleic acid is electrophoresed using a gel having a chemical denaturant density gradient or a temperature gradient. The DNA fragment amplified migrates in the gel to a position where it denatures to a single strand, and no longer migrates after the denaturation. Because the migration of the amplified DNA in the gel differs between the presence and absence of a mutation, the presence of the mutation can be detected. To increase the detection sensitivity, the addition of a poly (G:C) end to each primer is effective.

By directly determining and analyzing the nucleotide sequence of a sample-derived DNA or sample-derived cDNA, a mutation of a nucleic acid can also be detected.

### 5. Methods of expressing a nucleic acid such as miRNA or miRNA precursor

Existing miRNA sequences and sequences of precursors thereof are registered with a database known as miRBase at the Sanger Institute in UK; nucleic acids such as miRNAs and miRNA precursors can be prepared by utilizing such sequences. Such nucleic acids can also be prepared using miRNA sequences acquired by the method described in 1.

A nucleic acid can be expressed by using a vector such that a nucleic acid is transcribed and hence biosynthesized when the vector is introduced into a cell. Specifically, by preparing a DNA fragment comprising a hairpin portion on the basis of the nucleotide sequence of a nucleic acid or a genomic nucleotide sequence comprising the foregoing nucleotide sequence, and inserting the fragment downstream of a promoter in the expression vector to construct an expression plasmids, and then introducing the expression plasmid into a host cell suitable for the expression vector, whereby a nucleic acid can be expressed.

As an expression vector, one capable of self-replication in the host cell, and capable of being incorporated in the chromosome, which comprises a promoter at a position enabling the transcription of a gene comprising the nucleotide sequence of a nucleic acid is used. The promoter may be any one, as far as it is capable of expressing in the host cell; for example,
a RNA polymerase II (pol II) system promoter, a RNA polymerase III (pol III) system promoter being a U6RNA and HIRNA transcription system and the like can be mentioned. As examples of pol II system promoters, the promoter of the cytomegalovirus (human CMV) IE (immediate early) gene, the early promoter of SV40 and the like can be mentioned. As examples of expression vectors using them, pCDNA6.2-GW/miR (manufactured by Invitrogen), pSilencer 4.1-CMV (manufactured by Ambion) and the like can be mentioned. As pol III system promoters, U6RNA, H1RNA or tRNA gene promoters can be mentioned. As examples of expression vectors using them, pSINsi-hH1 DNA (manufactured by Takara Bio Inc.), pSINsi-hU6 DNA (manufactured by Takara Bio Inc.), pENTR/U6 (manufactured by Invitrogen) and the like can be mentioned.

Alternatively, by inserting a gene comprising the nucleotide sequence of a nucleic acid into the downstream of the promoter in a viral vector to construct a recombinant viral vector, and introducing the vector into a packaging cell to produce a recombinant virus, the gene comprising the nucleic acid can be expressed.
The packaging cell may be any cell, as far as it is capable of supplementing a recombinant viral vector deficient in any one of the genes that encode the proteins necessary for the packaging of the virus with the lacked protein; for example, human kidney-derived HEK293 cells, mouse fibroblasts NIH3T3-derived cells and the like can be used. As the protein supplemented by the packaging cell, in the case of a retrovirus vector, proteins derived from a mouse retrovirus, such as gag, pol, and env, can be used; in the case of a lentivirus vector, proteins derived from a HIV virus, such as gag, pol, env, vpr, vpu, vif, tat, rev, and nef, can be used; in the case of an adenovirus vector, proteins derived from an adenovirus, such as EIA and E1B, can be used; in the case of an adeno-associated viral vector, proteins such as Rep (p5, p19, p40) and Vp(Cap), can be used.

In addition to using an expression vector, a nucleic acid to be used in the present invention can also be introduced directly into a cell, without using a vector. As the nucleic acid used in this technique, a DNA, an RNA, or a nucleotide analogue, as well as a chimeric molecule thereof, or a derivative of the nucleic acid can also be used. Specifically, in the same way as with Pre-miR^{™} miRNA Precursor Molecules (manufactured by Ambion) or miRIDIAN mRNA Mimics (manufactured by GE Healthcare), a nucleic acid such as miRNA or mRNA precursor can be expressed. Any method can be used to express a miRNA, as far as it allows the miRNA to be finally formed in the cells; for example, (1) a method wherein a single-stranded RNA that is a precursor of the mRNA is introduced, as well as (2) a method wherein an RNA consisting of a 100% matched double-strand consisting of the miRNA as it is and a complementary strand for the miRNA, is introduced, and (3) a method wherein a double-stranded RNA assumed to have resulted from cleavage of the mRNA with a Dicer is introduced, can be mentioned. As commercial products based on these methods, miCENTURY OX Precursor (manufactured by B-Bridge), miCENTURY OX siMature (manufactured by B-Bridge), and miCENTURY OX miNatural (manufactured by B-Bridge), respectively, can be mentioned.

### 6. Methods of suppressing the activity of a nucleic acid such as miRNA or miRNA precursor

The activation of a nucleic acid such as miRNA or miRNA precursor can be suppressed using an antisense technology [Bioscience and Industry, 50, 322 (1992); Kagaku, 46, 681 (1991), Biotechnology, 9, 358 (1992), Trends in Biotechnology, 10, 87 (1992), Trends in Biotechnology, 10, 152 (1992); Cell Technology, 16, 1463 (1997)], triple helix technology [Trends in Biotechnology, 10, 132 (1992)], ribozyme technology [Current Opinion in Chemical Biology, 3, 274 (1999), FEMS Microbiology Reviews, 23, 257 (1999), Frontiers in Bioscience, 4, Do7 (1999), Chemistry & Biology, 6, R33 (1999), Nucleic Acids Research, 26, 5237 (1998), Trends In Biotechnology, 16, 438 (1998)], decoy DNA method [Nippon Rinsho - Japanese Journal of Clinical Medicine, 56, 563 (1998), Circulation Research, 82, 1023 (1998), Experimental Nephrology, 5, 429 (1997), Nippon Rinsho - Japanese Journal of Clinical Medicine, 54, 2583 (1996)], or a siRNA (short interfering RNA).

An antisense refers to one that allows nucleotide sequence-specific hybridization of a nucleic acid having a nucleotide sequence complementary to a certain target nucleic acid to enable the suppression of the expression of the target nucleic acid. As the nucleic acid used as the antisense, a DNA, an RNA or a nucleotide analogue, as well as a chimeric molecule thereof, or a derivative of the nucleic acid can also be used. Specifically, by preparing an antisense in accordance with a method described in Nature 432, 226 (2004) and the like, the expression can be suppressed. In addition, in the same way as with Anti-miR^{™} miRNA Inhibitors (manufactured by Ambion) and miRIDIAN miRNA Inhibitors (manufactured by GE Healthcare), the expression of a nucleic acid such as miRNA or miRNA precursor can be suppressed.

An siRNA refers to a short double-stranded RNA comprising the nucleotide sequence of a certain target nucleic acid, that is capable of suppressing the expression of the target nucleic acid by RNA interference (RNAi). The sequence of an siRNA can be designed as appropriate from the target nucleotide sequence on the basis of conditions shown in the literature [Genes Dev., 13, 3191 (1999)]. For example, by synthesizing two RNA having a 19-nucleotide sequence selected and a complementary sequence, with TT added to the 3' terminus of each thereof using a nucleic acid synthesizer, and performing annealing, an siRNA can be prepared. By inserting a DNA corresponding to the above-described 19-nucleotide sequence selected into an siRNA expression vector such as pSilencer 1.0-U6 (manufactured by Ambion) or pSUPER (manufactured by OligoEngine), a vector that expresses an siRNA capable of suppressing the expression of the gene can be prepared. Although any siRNA can be used to suppress a nucleic acid such as a miRNA used in the present invention, as far as it is capable of suppressing the activity of the nucleic acid, preference is given to siRNAs designed on the basis of continuous sequence information on the 9th nucleotide and beyond of each nucleotide sequence shown by any one of SEQ ID NO:1-939, 2057-2132, 2212-2261 and 2315-2321.
The number of nucleotide residues constituting one strand of the siRNA is preferably 17 to 30 residues, more preferably 18 to 25 residues, still more preferably 19 to 23 residues.

Using an antisense or siRNA specific for a nucleic acid such as miRNA expressed in cancer cells, or a precursor of the miRNA, the expression of a miRNA expressed in cancer cells, a precursor of the miRNA and the like can be suppressed. For example, by administering an antisense DNA or siRNA specific for the miRNA or the miRNA precursor, the activation of the mRNA can be suppressed, and the action of the miRNA or MRNA precursor in cancer cells can be controlled.

In addition, in the case of a patient affected by an abnormality of the expression of a miRNA expressed in cancer cells or a precursor thereof, by administering an antisense oligonucleotide or siRNA specific for the miRNA or precursor thereof to the patient, it is possible to control a function of cancer cells to treat a disease that develops as a result of the above-described expression abnormality. Hence, an antisense oligonucleotide or siRNA that is specific for the mRNA or precursor thereof is useful as a therapeutic agent for a disease caused by a cell proliferation abnormality of cancer cells.

When an antisense oligonucleotide or siRNA specific for a nucleic acid such as a miRNA or a precursor thereof is used as the above-described therapeutic agent, the antisense oligonucleotide or siRNA alone, or a nucleic acid that encodes the same after being inserted into an appropriate vector such as a retrovirus vector, adenovirus vector, or adeno-associated virus vector, may be prepared as a pharmaceutical preparation according to the conventional method described in 9 below, and administered.

### 7. Methods of suppressing functions of genes using a nucleic acid such as mRNA or miRNA precursor

Any method can be used to suppress a function or the expression of the target gene for a nucleic acid, as far as it is based on an activity of a nucleic acid such as a mRNA to suppress the expression of the RNA having the target nucleotide sequence. For example, a method can be mentioned wherein a nucleic acid such as a miRNA is expressed to increase the amount of the nucleic acid such as the miRNA in cells, whereby the translation of the mRNA having the target sequence is suppressed to suppress the expression of the gene. The expression of a nucleic acid can be achieved by the method described in 5 above. As examples of mRNAs having a target nucleotide sequence for a nucleic acid consisting of a nucleotide sequence shown by any one of SEQ LID NO:1-939, 2057-2132, 2212-2261 and 2315-2321, the respective sets of genes shown in the foregoing Table 4 can be mentioned.

Using an siRNA against a target gene shown in Table 4, it is possible to suppress a function of the target gene.

### 8. Methods of screening for substances that promote or suppress the expression or a function of a nucleic acid such as a miRNA or mRNA precursor using the nucleic acid

A substance that promotes or suppresses the expression or a function of a nucleic acid such as a miRNA or precursor thereof can be screened for using a nucleic acid. For example, a nucleotide sequence to be targeted for screening is chosen from the nucleotide sequence of a nucleic acid, and by means of cells that express a nucleic acid having the nucleotide sequence, a substance that promotes or suppresses the expression or a function of the chosen miRNA or precursor thereof can be screened for.

As cells that express a miRNA or miRNA precursor and the like, used for screening, cancer cells, as well as transformant cells obtained by introducing a vector that expresses a nucleic acid having the nucleotide sequence into a host cell such as an animal cell or yeast, cells incorporating a nucleic acid having the nucleotide sequence introduced directly without using a vector and the like as described in 5 above can also be used.
As specific screening methods, a method comprising using a change in the expression level of a nucleic acid such as an miRNA or precursor thereof to be targeted in the screening as an index, as well as a method comprising using a change in the expression level of a mRNA having a target sequence for a nucleic acid such as a miRNA or a gene product encoded thereby as an index can be mentioned.

### (a) Screening method comprising using a change in the expression level of a nucleic acid such as a miRNA or precursor thereof to be targeted in the screening as an index

A test substance is brought into contact with a cell that expresses a nucleic acid, and with a change in the expression level of the nucleic acid selected as an index, a substance that promotes or suppresses the nucleic acid of expression such as a miRNA and precursor thereof is obtained. The expression level of a nucleic acid can be detected by the method described in 3 above.

### (b) Screening method comprising using a change in the expression level of a mRNA having a target sequence for a nucleic acid such as a miRNA to be targeted in the screening or a gene product encoded thereby as an index

A test substance is brought into contact with a cell that expresses the mRNA, and with a change in the expression level of a mRNA having a target sequence of the nucleic acid selected or a gene product encoded thereby as an index, a substance that promotes or suppresses the expression or a function of a nucleic acid such as a miRNA and a precursor thereof is obtained. Alternatively, a DNA having a target sequence of a nucleic acid such as miRNA inserted into the 3' UTR of an appropriate reporter gene expression vector is prepared and introduced into a host cell suitable for the expression vector, a test substance is brought into contact with the cell, and with a change in the expression level of the reporter gene as an index, a substance that promotes or suppresses the expression or a function of a nucleic acid such as a miRNA and a precursor thereof is obtained.

Choice of a target sequence can be achieved by the method described in 7 above; as examples of a mRNA having a target sequence of a nucleic acid such as a mRNA consisting of the nucleotide sequence of any one of SEQ ID NO:1 to 939, 2057 to 2132, 2212 to 2261 and 2315 to 2321, the above-described genes shown in Table 4 above can be exemplified.

### 9. Agent for suppressing or promoting cell proliferation using a nucleic acid such as miRNA or miRNA precursor

Nucleic acids such as mRNAs and miRNA precursors, and nucleic acids having nucleotide sequences complementary to the nucleotide sequences thereof can be utilized as agents for suppressing or promoting cell proliferation because they control the expression of genes having target sequences.

As agents for suppressing cell proliferation, the nucleic acids (a) to (h) below can be mentioned.
(a) A nucleic acid consisting of a nucleotide sequence shown by any one of SEQ ID NO:1-3, 5-7, 11, 14, 15, 17, 24, 25, 29-33, 35, 37, 39-54, 56-58, 61-63, 66-68, 70, 72-82, 84, 85, 87-112, 114-121, 125, 128-167, 193-219, 263-278, 306-324, 357-368, 476-492, 522-557, 561-564, 572-576, 578-683, 693-714, 735-743, 746-751, 754-762, 765-803, 853-855, 888-891, 899-912, 914-926, 930-939, 2058, 2060, 2061, 2063-2065, 2067-2071, 2073-2078, 2080, 2082, 2084-2092, 2096-2108, 2110, 2111, 2113, 2117, 2120-2122, 2125-2128, 2132, 2212, 2213, 2216, 2219, 2220, 2227-2229, 2231-2237, 2240-2243, 2248-2253, 2255-2261, 2317 and 2318;
(b) a nucleic acid of 17 to 28 nucleotides comprising a nucleic acid consisting of a nucleotide sequence shown by any one of SEQ ID NO:1-3, 5-7, 11, 14, 15, 17, 24, 25, 29-33, 35, 37, 39-54, 56-58, 61-63, 66-68, 70, 72-82, 84, 85, 87-112, 114-121, 125, 128-167, 193-219, 263-278, 306-324, 357-368, 476-492, 522-557, 561-564, 572-576, 578-683, 693-714, 735-743, 746-751, 754-762, 765-803, 853-855, 888-891, 899-912, 914-926, 930-939, 2058, 2060, 2061, 2063-2065, 2067-2071, 2073-2078, 2080, 2082, 2084-2092, 2096-2108, 2110, 2111, 2113, 2117, 2120-2122, 2125-2128, 2132, 2212, 2213, 2216, 2219, 2220, 2227-2229, 2231-2237, 2240-2243, 2248-2253, 2255-2261, 2317 and 2318;
(c) a nucleic acid consisting of a nucleotide sequence having an identity of 90% or more to a nucleotide sequence shown by any one of SEQ ID NO:1-3, 5-7, 11, 14, 15, 17, 24, 25, 29-33, 35, 37, 39-54, 56-58, 61-63, 66-68, 70, 72-82, 84, 85, 87-112, 114-121, 125, 128-167, 193-219, 263-278, 306-324, 357-368, 476-492, 522-557, 561-564, 572-576, 578-683, 693-714, 735-743, 746-751, 754-762, 765-803, 853-855, 888-891, 899-912, 914-926, 930-939, 2058, 2060, 2061, 2063-2065, 2067-2071, 2073-2078, 2080, 2082, 2084-2092, 2096-2108, 2110, 2111, 2113, 2117, 2120-2122, 2125-2128, 2132, 2212, 2213, 2216, 2219, 2220, 2227-2229, 2231-2237, 2240-2243, 2248-2253, 2255-2261, 2317 and 2318;
(d) a nucleic acid that hybridizes under stringent conditions with a complementary strand for a nucleic acid consisting of a nucleotide sequence shown by any one of SEQ ID NO:1-3, 5-7, 11, 14, 15, 17, 24, 25, 29-33, 35, 37, 39-54, 56-58, 61-63, 66-68, 70, 72-82, 84, 85, 87-112, 114-121, 125, 128-167, 193-219, 263-278, 306-324, 357-368, 476-492, 522-557, 561-564, 572-576, 578-683, 693-714, 735-743, 746-751, 754-762, 765-803, 853-855, 888-891, 899-912, 914-926, 930-939, 2058, 2060, 2061, 2063-2065, 2067-2071, 2073-2078, 2080, 2082, 2084-2092, 2096-2108, 2110, 2111, 2113, 2117, 2120-2122, 2125-2128, 2132, 2212, 2213, 2216, 2219, 2220, 2227-2229, 2231-2237, 2240-2243, 2248-2253, 2255-2261, 2317 and 2318;
(e) a nucleic acid comprising the sequence of 2nd to 8th nucleotides of a nucleotide sequence shown by any one of SEQ ID NO:1-3, 5-7, 11, 14, 15, 17, 24, 25, 29-33, 35, 37, 39-54, 56-58, 61-63, 66-68, 70, 72-82, 84, 85, 87-112, 114-121, 125, 128-167, 193-219, 263-278, 306-324, 357-368, 476-492, 522-557, 561-564, 572-576, 578-683, 693-714, 735-743, 746-751, 754-762, 765-803, 853-855, 888-891, 899-912, 914-926, 930-939, 2058, 2060, 2061, 2063-2065, 2067-2071, 2073-2078, 2080, 2082, 2084-2092, 2096-2108, 2110, 2111, 2113, 2117, 2120-2122, 2125-2128, 2132, 2212, 2213, 2216, 2219, 2220, 2227-2229, 2231-2237, 2240-2243, 2248-2253, 2255-2261, 2317 and 2318;
(f) a nucleic acid consisting of a nucleotide sequence shown by any one of SEQ ID NO:940-945, 947, 948, 952, 953, 956-958, 960, 968, 969, 973-978, 980, 982, 984-1001, 1003-1005, 1008-1009, 1012-1014, 1016, 1018-1029, 1031, 1033-1059, 1064-1069, 1073, 1076-1133, 1163-1188, 1232-1259, 1288-1309, 1343-1354, 1479-1495, 1529-1566, 1572-1576, 1585-1589, 1591-1709, 1719-1744, 1765-1777, 1780-1788, 1791-1829, 1898-1900, 1998-2001, 2009-2050, 2052-2056, 2134, 2136, 2137, 2139-2143, 2145-2151, 2153-2158, 2160, 2162, 2164-2172, 2176-2188, 2190, 2191, 2193, 2197, 2200-2202, 2205-2207, 2211, 2262-2265, 2268, 2271-2273, 2281-2283, 2285-2292, 2295-2297, 2302-2306, 2308-2314, 2324 and 2325;
(g) a nucleic acid consisting of a nucleotide sequence having an identity of 90% or more to a nucleotide sequence shown by any one of SEQ ID NO:940-945, 947, 948, 952, 953, 956-958, 960, 968, 969, 973-978, 980, 982, 984-1001, 1003-1005, 1008-1009, 1012-1014, 1016, 1018-1029, 1031, 1033-1059, 1064-1069, 1073, 1076-1133, 1163-1188, 1232-1259, 1288-1309, 1343-1354, 1479-1495, 1529-1566, 1572-1576, 1585-1589, 1591-1709, 1719-1744, 1765-1777, 1780-1788, 1791-1829, 1898-1900, 1998-2001, 2009-2050, 2052-2056, 2134, 2136, 2137, 2139-2143, 2145-2151, 2153-2158, 2160, 2162, 2164-2172, 2176-2188, 2190, 2191, 2193, 2197, 2200-2202, 2205-2207, 2211, 2262-2265, 2268, 2271-2273, 2281-2283, 2285-2292, 2295-2297, 2302-2306, 2308-2314, 2324 and 2325; and
(h) a nucleic acid that hybridizes under stringent conditions with a complementary strand for a nucleic acid consisting of a nucleotide sequence shown by any one of SEQ ID NO:940-945, 947, 948, 952, 953, 956-958, 960, 968, 969, 973-978, 980, 982, 984-1001, 1003-1005, 1008-1009, 1012-1014, 1016, 1018-1029, 1031, 1033-1059, 1064-1069, 1073, 1076-1133, 1163-1188, 1232-1259, 1288-1309, 1343-1354, 1479-1495, 1529-1566, 1572-1576, 1585-1589, 1591-1709, 1719-1744, 1765-1777, 1780-1788, 1791-1829, 1898-1900, 1998-2001, 2009-2050, 2052-2056, 2134, 2136, 2137, 2139-2143, 2145-2151, 2153-2158, 2160, 2162, 2164-2172, 2176-2188, 2190, 2191, 2193, 2197, 2200-2202, 2205-2207, 2211, 2262-2265, 2268, 2271-2273, 2281-2283, 2285-2292, 2295-2297, 2302-2306, 2308-2314, 2324 and 2325.

As agents for promoting cell proliferation, the following nucleic acids (i)-(p) can be mentioned.
(i) A nucleic acid consisting of a nucleotide sequence shown by any one of SEQ ID NO:4, 8-10, 12, 13, 16, 18-23, 26-28, 34, 36, 38, 55, 59, 60, 64, 65, 69, 71, 83, 86, 113, 122-124, 126, 127, 168-192, 220-262, 279-305, 325-356, 369-475, 493-521, 558-560, 565-571, 577, 684-692, 715-734, 744, 745, 752, 753, 763, 764, 804-852, 856-887, 892-898, 913, 927-929, 2057, 2059, 2062, 2066, 2072, 2079, 2081, 2083, 2093-2095, 2109, 2112, 2114-2116, 2118, 2119, 2123, 2124, 2129-2131, 2214, 2215, 2218, 2221-2226, 2230, 2238, 2239, 2244-2247, 2254, 2315, 2316 and 2319-2321;
(j) a nucleic acid of 17-28 nucleotides comprising a nucleic acid consisting of a nucleotide sequence shown by any one of SEQ ID NO:4, 8-10, 12, 13, 16, 18-23, 26-28, 34, 36, 38, 55, 59, 60, 64, 65, 69, 71, 83, 86, 113, 122-124, 126, 127, 168-192, 220-262, 279-305, 325-356, 369-475, 493-521, 558-560, 565-571, 577, 684-692, 715-734, 744, 745, 752, 753, 763, 764, 804-852, 856-887, 892-898, 913, 927-929, 2057, 2059, 2062, 2066, 2072, 2079, 2081, 2083, 2093-2095, 2109, 2112, 2114-2116, 2118, 2119, 2123, 2124, 2129-2131, 2214, 2215, 2218, 2221-2226, 2230, 2238, 2239, 2244-2247, 2254, 2315, 2316 and 2319-2321;
(k) a nucleic acid consisting of a nucleotide sequence having an identity of 90% or more to a nucleotide sequence shown by any one of SEQ ID NO:4, 8-10, 12, 13, 16, 18-23, 26-28, 34, 36, 38, 55, 59, 60, 64, 65, 69, 71, 83, 86, 113, 122-124, 126, 127, 168-192, 220-262, 279-305, 325-356, 369-475, 493-521, 558-560, 565-571, 577, 684-692, 715-734, 744, 745, 752, 753, 763, 764, 804-852, 856-887, 892-898, 913, 927-929, 2057, 2059, 2062, 2066, 2072, 2079, 2081, 2083, 2093-2095, 2109, 2112, 2114-2116, 2118, 2119, 2123, 2124, 2129-2131, 2214, 2215, 2218, 2221-2226, 2230, 2238, 2239, 2244-2247, 2254, 2315, 2316 and 2319-2321;
(1) a nucleic acid that hybridizes under stringent conditions with a complementary strand for a nucleic acid consisting of a nucleotide sequence shown by any one of SEQ ID NO:4, 8-10, 12, 13, 16, 18-23, 26-28, 34, 36, 38, 55, 59, 60, 64, 65, 69, 71, 83, 86, 113, 122-124, 126, 127, 168-192, 220-262, 279-305, 325-356, 369-475, 493-521, 558-560, 565-571, 577, 684-692, 715-734, 744, 745, 752, 753, 763, 764, 804-852, 856-887, 892-898, 913, 927-929, 2057, 2059, 2062, 2066, 2072, 2079, 2081, 2083, 2093-2095, 2109, 2112, 2114-2116, 2118, 2119, 2123, 2124, 2129-2131, 2214, 2215, 2218, 2221-2226, 2230, 2238, 2239, 2244-2247, 2254, 2315, 2316 and 2319-2321;
(m) a nucleic acid comprising the sequence of 2nd to 8th nucleotides of a nucleotide sequence shown by any one of SEQ ID NO:4, 8-10, 12, 13, 16, 18-23, 26-28, 34, 36, 38, 55, 59, 60, 64, 65, 69, 71, 83, 86, 113, 122-124, 126, 127, 168-192, 220-262, 279-305, 325-356, 369-475, 493-521, 558-560, 565-571, 577, 684-692, 715-734, 744, 745, 752, 753, 763, 764, 804-852, 856-887, 892-898, 913, 927-929, 2057, 2059, 2062, 2066, 2072, 2079, 2081, 2083, 2093-2095, 2109, 2112, 2114-2116, 2118, 2119, 2123, 2124, 2129-2131, 2214, 2215, 2218, 2221-2226, 2230, 2238, 2239, 2244-2247, 2254, 2315, 2316 and 2319-2321;
(n) a nucleic acid consisting of a nucleotide sequence shown by any one of SEQ ID NO:946, 949-951, 954, 955, 959, 961-967, 970-972, 979, 981, 983, 1002, 1006, 1007, 1010, 1011, 1015, 1017, 1030, 1032, 1060-1063, 1070-1072, 1074, 1075, 1134-1162, 1189-1231, 1260-1287, 1310-1342, 1355-1478, 1496-1528, 1567-1571, 1577-1584, 1590, 1710-1718, 1745-1764, 1778, 1779, 1789, 1790, 1830-1897, 1901-1997, 2002-2008, 2051, 2133, 2135, 2138, 2144, 2152, 2159, 2161, 2163, 2173-2175, 2189, 2192, 2194-2196, 2198, 2199, 2203, 2204, 2208-2210, 2266, 2267, 2270, 2274-2280, 2284, 2293, 2294, 2298-2301, 2307, 2322, 2323 and 2326-2328;
(o) a nucleic acid consisting of a nucleotide sequence having an identity of 90% or more to a nucleotide sequence shown by any one of SEQ ID NO:946, 949-951, 954, 955, 959, 961-967, 970-972, 979, 981, 983, 1002, 1006, 1007, 1010, 1011, 1015, 1017, 1030, 1032, 1060-1063, 1070-1072, 1074, 1075, 1134-1162, 1189-1231, 1260-1287, 1310-1342, 1355-1478, 1496-1528, 1567-1571, 1577-1584, 1590, 1710-1718, 1745-1764, 1778, 1779, 1789, 1790, 1830-1897, 1901-1997, 2002-2008, 2051, 2133, 2135, 2138, 2144, 2152, 2159, 2161, 2163, 2173-2175, 2189, 2192, 2194-2196, 2198, 2199, 2203, 2204, 2208-2210, 2266, 2267, 2270, 2274-2280, 2284, 2293, 2294, 2298-2301, 2307, 2322, 2323 and 2326-2328; and
(p) a nucleic acid that hybridizes under stringent conditions with a complementary strand for a nucleic acid consisting of a nucleotide sequence shown by any one of SEQ ID NO:946, 949-951, 954, 955, 959, 961-967, 970-972, 979, 981, 983, 1002, 1006, 1007, 1010, 1011, 1015, 1017, 1030, 1032, 1060-1063, 1070-1072, 1074, 1075, 1134-1162, 1189-1231, 1260-1287, 1310-1342, 1355-1478, 1496-1528, 1567-1571, 1577-1584, 1590, 1710-1718, 1745-1764, 1778, 1779, 1789, 1790, 1830-1897, 1901-1997, 2002-2008, 2051, 2133, 2135, 2138, 2144, 2152, 2159, 2161, 2163, 2173-2175, 2189, 2192, 2194-2196, 2198, 2199, 2203, 2204, 2208-2210, 2266, 2267, 2270, 2274-2280, 2284, 2293, 2294, 2298-2301, 2307, 2322, 2323 and 2326-2328.

The above-described nucleic acids are suitably miRNAs or miRNA precursors.
As agents for promoting cell proliferation, nucleic acids consisting of a nucleotide sequence complementary to the nucleotide sequence of one of the nucleic acids (a) to (h) above can be mentioned; as agents for suppressing cell proliferation, nucleic acids consisting of a nucleotide sequence complementary to the nucleotide sequence of one of the nucleic acids (i) to (p) above can be mentioned. Vectors that express the above-described nucleic acids and nucleic acids that are complementary thereto can also be used as agents for suppressing or promoting cell proliferation.

Meanwhile, substances that suppress the expression of target genes for nucleic acids such as the above-described miRNAs, and substances that promote the expression of the target genes can also be used as agents for suppressing or promoting cell proliferation. As these substances, nucleic acids or vectors that express the same can also be used. As substances that suppress the expression of a target gene, an siRNA against the mRNA of the target gene and an antisense against the target gene can be mentioned; as substances that promote the expression of the target gene, an siRNA against a miRNA specific for the target gene and an antisense against the target gene can be mentioned.

Regarding the preparation forms, methods of administration and the like for an agent for suppressing or promoting cell proliferation, the same applies as with the diagnostic reagent and therapeutic drug containing a nucleic acid such as a miRNA or miRNA precursor described in 10 below.

### 10. Diagnostic reagent and therapeutic drug containing a nucleic acid such as a miRNA or miRNA precursor

Nucleic acids such as miRNAs and miRNA precursors can be utilized as drugs for treating diseases resulting from a cell proliferation abnormality and the like, such as cancers, because they control the expression of genes having a target sequence, or control the expression of nucleic acids such as miRNAs. Furthermore, siRNAs against the target gene for the nucleic acid can be utilized as drugs for treating diseases resulting from a cell proliferation or differentiation abnormality and the like because they control the expression of the gene. As diseases resulting from a cell proliferation abnormality and the like, cancers, arteriosclerosis, rheumatoid arthritis, prostatic hyperplasia, blood vessel restenosis after percutaneous transvascular coronary angioplasty, fibroid lung, glomerulonephritis, autoimmune diseases and the like can be mentioned.

By quantifying a nucleic acid or detecting a mutation therein, a disease resulting from a cell proliferation or differentiation abnormality and the like, such as a cancer, can be diagnosed.
A diagnostic reagent comprising a nucleic acid, according to the desired diagnostic method, may comprise reagents necessary for quantitation or detection of mutation of a nucleic acid, for example, buffering agents, salts, reaction enzymes, labeled proteins that bind to a nucleic acid, and a color developer for detection and the like.

Although a therapeutic agent containing a nucleic acid as an active ingredient can be administered alone, the same is normally desirably administered as a pharmaceutical preparation produced by an optionally chosen method known well in the technical field of pharmaceutical making with one or more pharmacologically acceptable carriers blended therein.
The route of administration used is desirably the most effective one in treatment; oral administration, or parenteral administration such as intraoral administration, airway administration, intrarectal administration, subcutaneous administration, intramuscular administration and intravenous administration can be mentioned, and desirably intravenous administration can be mentioned.

As dosage forms, sprays, capsules, tablets, granules, syrups, emulsions, suppositories, injection formulations, ointments, tapes and the like can be mentioned.
As preparations appropriate for oral administration, emulsions, syrups, capsules, tablets, powders, granules and the like can be mentioned.
Liquid preparations like emulsions and syrups can be produced using water, saccharides such as sucrose, sorbitol, and fructose, glycols such as polyethylene glycol and propylene glycol, oils such as sesame oil, olive oil, and soybean oil, antiseptics such as p-hydroxybenzoic acid esters, flavors such as strawberry flavor and peppermint and the like as additives.

Capsules, tablets, powders, granules and the like can be produced using excipients such as lactose, glucose, sucrose, and mannitol, disintegrants such as starch and sodium alginate, lubricants such as magnesium stearate and talc, binders such as polyvinyl alcohol, hydroxypropylcellulose, and gelatin, surfactants such as fatty acid esters, plasticizers such as glycerin and the like as additives.

As appropriate preparations for parenteral administration, injection formulations, suppositories, sprays and the like can be mentioned.
An injection formulation is prepared using a carrier consisting of a salt solution, a glucose solution or a mixture of both and the like. A suppository is prepared using a carrier such as cacao butter, hydrogenated fat or carboxylic acid. A spray is prepared using a carrier that does not stimulate the recipient's oral cavity and airway mucosa, and that disperses the active ingredients as fine particles to facilitate the absorption thereof, and the like.

As examples of the carrier, specifically, lactose, glycerin and the like can be exemplified. Depending on the nature of the nucleic acid or miRNA precursor, and of the carrier used, preparations such as aerosols and dry powders are possible. In these parenteral preparations, components exemplified as additives for oral preparations can also be added.
The dose or frequency of administration varies depending on desired therapeutic effect, method of administration, duration of treatment, age, body weight and the like, and is normally 10 µg/kg to 20 mg/kg per day for an adult.

A therapeutic drug comprising a nucleic acid as an active ingredient can also be produced by blending a vector that expresses a nucleic acid and a nucleotide used in a nucleic acid therapeutic drug [Nature Genet., 8, 42(1994)].
The base used in the therapeutic drug may be any base for ordinary use in injection formulations; distilled water, solutions of salts such as sodium chloride or a mixture of sodium chloride and an inorganic salt, solutions of mannitol, lactose, dextran, glucose and the like, solutions of amino acids such as glycine and arginine, mixed solutions of organic acid solutions or salt solutions and glucose solution and the like can be mentioned. In accordance with a conventional method, using auxiliary agents such as an osmoregulator, a pH adjuster, a vegetable oil such as sesame oil or soybean oil, lecithin, and a surfactant such as a non-ionic surfactant in these bases, an injection formulation may be prepared as a solution, suspension, or dispersion. These injection formulations can also be prepared as preparations for dissolution before use, by procedures such as powdering and lyophilization. A therapeutic agent can be used for treatment as is in the case of a liquid, or after being dissolved in a base described above, optionally sterilized, in the case of a solid, just before treatment.

As a vector expressing a nucleic acid, the recombinant viral vector prepared in the method of the above-mentioned 5 can be mentioned, more specifically, retrovirus vector and lentivirus vector and the like can be mentioned.
For example, by combining a nucleic acid with a polylysine-conjugated antibody that is specific for adenovirus hexon protein to prepare a complex, and binding the complex obtained to an adenovirus vector, a viral vector can be prepared. The viral vector is capable of stably reaching the desired cell, being incorporated into cells by endosome, being decomposed in the cells, and efficiently expressing the nucleic acid.

A viral vector based on Sendai virus, which is a (-) strand RNA virus, has been developed (WO97/16538, WO97/16539), a Sendai virus incorporating a nucleic acid can be prepared using the Sendai virus.
A nucleic acid can also be transferred by a non-viral nucleic acid transfer method. The same can be transferred by, for example, calcium phosphate co-precipitation [Virology, 52, 456-467 (1973); Science, 209, 1414-1422 (1980)], microinjection method [Proc. Natl. Acad. Sci. USA, 77, 5399-5403 (1980); Proc. Natl. Acad. Sci. USA, 77, 7380-7384 (1980); Cell, 27, 223-231 (1981); Nature, 294, 92-94 (1981)], membrane fusion-mediated transfer mediated by liposome [Proc. Natl. Acad. Sci. URSA, 84, 7413-7417 (1987); Biochemistry, 28, 9508-9514 (1989); J. Biol. Chem., 264, 12126-12129 (1989); Hum. Gene Ther., 3, 267-275, (1992); Science, 249, 1285-1288 (1990); Circulation, 83, 2007-2011 (1992)] or direct DNA uptake and receptor-mediated DNA transfer method [Science, 247, 1465-1468 (1990); J. Biol. Chem., 266, 14338-14342 (1991); Proc. Natl. Acad. Sci. USA, 87, 3655-3659 (1991); J. Biol. Chem., 264, 16985-16987 (1989); BioTechniques, 11, 474-485 (1991); Proc. Natl. Acad. Sci. USA, 87, 3410-3414 (1990); Proc. Natl. Acad. Sci. URSA, 88, 4255-4259 (1991); Proc. Natl. Acad. Sci. USA, 87, 4033-4037 (1998); Proc. Natl. Acad. Sci. USA, 88, 8850-8854 (1991); Hum. Gene Ther., 3, 147-154 (1991)] and the like.

Membrane fusion-mediated transfer mediated by liposome allows a nucleic acid to be incorporated locally in the tissue, and to be expressed, by administering a liposome preparation directly to the target tissue [Hum. Gene Ther., 3, 399 (1992)]. For direct targeting of a DNA to a focus, direct DNA uptake technology is preferable.
For receptor-mediated DNA transfer, for example, a method performed by binding a DNA (usually assuming the form of a covalently cyclized supercoiled plasmid) to a protein ligand via polylysine can be mentioned. A ligand is chosen on the basis of the presence of a corresponding ligand receptor on the cell surface of the desired cell or tissue. The ligand-DNA conjugate can be injected directly into a blood vessel as desired, and can be directed to a target tissue wherein receptor binding and DNA-protein complex internalization occur. To prevent the destruction of the DNA in a cell, an adenovirus may be infected simultaneously to destroy the endosome function.

### 11. Method of measuring proliferation of cancer cells and other cells

The method of measuring cell proliferation is not particularly limited, as far as it enables a measurement of an index that reflects cell count or cell proliferation rate. Viable cell counting, DNA synthesis rate measurements, total protein content measurements and the like can be used. As a method of evaluating viable cell counts, a method wherein the ATP content in cells is measured can be mentioned. It is known that the ATP content in cells is proportional to the number of cells in culture (J. Immunol. Meth. 160, 81-88 (1993)). As more specific methods of measuring the ATP content in cells, the MTT method, the XTT method and the like can be mentioned (J. Immunol. Meth. 65, 55-63 (1983)). A method can also be mentioned wherein ATP is measured by luminescence of a luciferin substrate by the ATP-dependent enzyme luciferase. As a kit for measuring the ATP content in cells, for example, CellTite-Glo^{R} Luminescent Cell viability Assay (manufactured by Promega) and the like may be used.

### 12. Methods of measuring the degree of cell death of cancer cells

As methods of measuring the degree of cell death, a method wherein dead cells are stained with a dye such as Propium Iodide, a method wherein the activity of an enzyme leaked extracellularly as a result of cell death is measured, and the like can be used. For the latter, for example, a method wherein the enzyme activity of extracellularly leaked adenylate kinase is measured can be utilized. More specifically, ToxiLight(R) Non-Destructive Cytotoxicity BioAssay Kit (manufactured by Lonza) and the like may be used.

It is also possible to measure the degree of apoptosis (programmed cell death), which is important in relation to cancers, out of the various types of cell death. As methods of evaluating cell apoptosis, a method wherein the degree of DNA fragmentation is measured, a method wherein changes in cell membrane constituent lipids are measured, and a method wherein the activity of caspase 3/7, an intracellular protease induced upon apoptosis, is measured, can be mentioned. As a more specific method of measuring caspase 3/7 activity in cells, a method wherein a luciferin substrate liberated by caspase 3/7 activity is measured by luciferin luminescence by a luciferase enzyme reaction can be mentioned. As kits for measuring caspase 3/7 activity in cells, for example, Caspase-Glo^{R} 3/7 Assay (manufactured by Promega) and the like may be used.

The present invention is hereinafter described specifically by means of the following Examples. However, the present invention is not limited to these Examples.

### Example 1

Cell viability and apoptotic activity in colorectal cancer-derived cell line with a miRNA expressed forcedly therein
Pre-miR^{™}miRNA Precursor Molecules (manufactured by Ambion) were introduced into a colorectal cancer-derived cell line, and the influences of the miRNAs produced from the molecules on cell viability and apoptotic activity were examined.
The DLD-1 human colorectal cancer-derived cell line (hereinafter referred to as DLD-1) was obtained from the American Type Culture Collection (hereinafter referred to as ATCC). Cells were cultured with an RPMI1640 medium (manufactured by Invitrogen) containing 10% fetal bovine serum (FBS) (manufactured by JRH Biosciences) in a 37°C 5% CO₂ concentration incubator.

DLD-1 was sown to a 96-well plate at 2500 cells per well, and cultured in an RPMI medium containing 10% FBS overnight. One day later, Pre-miRwmiRNA Precursor Molecules that produce hsa-miR-7 (SEQ ID NO:3), 15a, 15b, 16, 18a (SEQ ID NO:5), 18a* (SEQ ID NO:7), 28 (SEQ ID NO:17), 95 (SEQ ID NO:24), 124a, 132 (SEQ ID NO:29), 133a (SEQ ID NO:31), 133b (SEQ ID NO:32), 147 (SEQ ID NO:37), 181b, 181c (SEQ ID NO:39), 181d (SEQ ID NO:40), 184 (SEQ ID NO:43), 192 (SEQ ID NO:44), 193a (SEQ ID NO:46), 193b (SEQ ID NO:47), 195 (SEQ ID NO:48), 196a (SEQ ID NO:51), 196b (SEQ ID NO:52), 197 (SEQ ID NO:53), 202 (SEQ ID NO:56), 204 (SEQ ID NO:57), 211 (SEQ ID NO:58), 212 (SEQ ID NO:30), 215 (SEQ ID NO:45), 299-3p (SEQ ID NO:62), 299-5p (SEQ ID NO:63), 324-3p (SEQ ID NO:66), 337 (SEQ ID NO:70), 342 (SEQ ID NO:72), 362 (SEQ ID NO:74), 368 (SEQ ID NO:75), 376a (SEQ ID NO:77), 376b (SEQ ID NO:78), 380-5p (SEQ ID NO:81), 422a (SEQ ID NO:84), 422b (SEQ ID NO:85), 424 (SEQ ID NO:49), 432* (SEQ ID NO:88), 452 (SEQ ID NO:92), 452* (SEQ ID NO:93), 455 (SEQ ID NO:94), 483 (SEQ ID NO:95), 489 (SEQ ID NO:98), 491 (SEQ ID NO:99), 493-5p (SEQ ID NO:100), 494 (SEQ ID NO:101), 497 (SEQ ID NO:50), 501 (SEQ ID NO:102), 504 (SEQ ID ND:103), 506 (SEQ ID NO:105), 512-3p (SEQ ID N0:110), 512-5p (SEQ ID N0:113), 517* (SEQ ID NO:114), 517b (SEQ ID NO:117), 517c (SEQ ID NO:116), 518b (SEQ ID NO:118), 518c* (SEQ ID NO:121), 518d (SEQ ID NO:120), 523 (SEQ ID NO:125), 527 (SEQ ID NO:129), let-7g, and let-7i were introduced into the DLD-1 to obtain a final concentration of 5 nM or 25 nM by a lipofection method, specifically a method using Lipofectamine2000 (manufactured by Invitrogen). Pre-miR^{™}iniRNA Precursor Molecules-Negative Control #2 (hereinafter, referred to as miR-negacon#2) (manufactured by Ambion) was also introduced into DLD-1, and this was used as the negative control. The lipofection was performed per the directions attached to the product.

Three days after introduction of the molecules by a lipofection method, cell viability was measured using CellTiter-Glo^{™}Lumine scent Cell Viability Assay (manufactured by Promega) according to the method described in the protocol attached to the product. Taking the cell viability of DLD-1 in a control plot (Lipofectamine2000 only) as 1.0, the relative cell viability of each was calculated. As a result, as shown in Table 5, with introduction of molecules that produce hsa-miR-7, 28, 95, 147, 181d, 192, 193a, 193b, 195, 196a, 197, 211, 215, 299-3p, 299-5p, 337, 342, 362, 368, 376a, 376b, 380-5p, 422b, 432*, 452, 483, 489, 491, 493-5p, 494, 501, 506, 517c, 518b, and 518d, decreases of 40% or more in cell viability were observed. With introduction of molecules that produce hsa-miR-15b and 124a, which are known to be associated with cancers, decreases of 40% or more in cell viability were observed.

**[Table 5]**

| **Introduced miRNA** | **cell viability (25 nM)** | **cell viability (5 nM)** |
|---|---|---|
| miR-362 | 0.31 | 0.83 |
| miR-197 | 0.31 | 0.39 |
| miR-342 | 0.32 | 0.67 |
| miR-299-3p | 0.33 | 0.51 |
| miR-376b | 0.34 | 0.62 |
| miR-192 | 0.35 | 0.62 |
| miR-147 | 0.37 | 0.53 |
| miR-452 | 0.39 | 0.54 |
| emir-491 | 0.40 | 0.71 |
| miR-124a | 0.44 | 0.70 |
| miR-493-5p | 0.44 | 0.74 |
| mir-215 | 0.44 | 0.47 |
| miR-483 | 0.47 | 0.73 |
| miR-432* | 0.47 | 0.68 |
| miR-95 | 0.47 | 0.59 |
| miR-376a | 0.48 | 0.71 |
| miR-506 | 0.50 | 0.60 |
| miR-337 | 0.50 | 0.78 |
| miR-195 | 0.51 | 0.79 |
| miR-181d | 0.51 | 0.88 |
| miR-7 | 0.51 | 0.62 |
| miR-368 | 0.51 | 0.73 |
| miR-489 | 0.52 | 0.74 |
| miR-494 | 0.53 | 0.80 |
| miR-28 | 0.53 | 0.67 |
| miR-518d | 0.54 | 0.57 |
| miR-422b | 0.55 | 0.76 |
| miR-517c | 0.57 | 0.69 |
| miR-193a | 0.57 | 0.78 |
| miR-193b | 0.57 | 0.76 |
| miR-299-5p | 0.57 | 0.75 |
| miR-15b | 0.58 | 0.77 |
| miR-501 | 0.59 | 0.70 |
| miR-211 | 0.59 | 0.73 |
| miR-196a | 0.60 | 0.82 |
| miR-380-5p | 0.60 | 0.65 |
| miR-518b | 0.61 | 0.60 |
| miR-negacon#2 | 0.96 | 1.00 |

Two days after introduction of the molecules (final concentration 25 nM) by a lipofection method, caspase 3/7 activity was measured using Caspase-Glo^{R} 3/7 assay (manufactured by Promega) per the directions described in the protocol attached to the product. Taking the caspase 3/7 activity value of DLD-1 in a control plot (Lipofectamine2000 only) as 1.0, the relative caspase 3/7 activity value of each was calculated. As a result, as shown in Table 6, with introduction of molecules that produce hsa-miR-18a, 18a*, 132, 133a, 133b, 181c, 181d, 189, 192, 193a, 195, 195a, 196b, 197, 202, 204, 211, 212, 215, 299-3p, 299-5p, 324-3p, 337, 342, 362, 368, 376a, 380-5p, 422a, 422b, 424, 432*, 452*, 455, 483, 491, 493-5p, 497, 501, 504, 512-3p, 512-5p, 517*, 517b, 517c, 518c*, 523, and 527, increases of 50% or more in caspase 3/7 activity were observed. With introduction of molecules that produce hsa-miR-15a, 15b, 16, 181b, let-7g, and let-7i, which are known to be associated with cancers, increases of 50% or more in caspase 3/7 activity were observed.

**[Table 6]**

| **introduced miRNA** | caspase 3/7 activity |
|---|---|
| miR-337 | 3.90 |
| miR-452* | 3.61 |
| miR-491 | 3.33 |
| miR-133a | 3.26 |
| miR-299-3p | 3.19 |
| miR-422b | 2.94 |
| miR-422a | 2.65 |
| miR-512-3p | 2.57 |
| miR-299-5p | 2.50 |
| miR-368 | 2.44 |
| miR-432* | 2.43 |
| miR-197 | 2.39 |
| miR-195 | 2.30 |
| miR-133b | 2.25 |
| miR-527 | 2.12 |
| miR-380-5p | 2.07 |
| miR-181b | 2. 06 |
| miR-15b | 2.04 |
| miR-132 | 2.01 |
| miR-455 | 1.97 |
| miR-192 | 1.97 |
| miR-501 | 1.93 |
| miR-181d | 1.93 |
| miR-193a | 1.93 |
| miR-181c | 1.93 |
| miR-512-5p | 1.90 |
| miR-215 | 1.89 |
| miR-362 | 1.89 |
| miR-15a | 1.83 |
| miR-493-5p | 1.83 |
| miR-517* | 1.81 |
| miR-324-3p | 1.80 |
| let-7g | 1.78 |
| miR-517b | 1.77 |
| miR-342 | 1.71 |
| miR-504 | 1.69 |
| miR-196a | 1.69 |
| miR-518c* | 1.67 |
| let-7i | 1.66 |
| miR-196b | 1.65 |
| miR-212 | 1.65 |
| miR-424 | 1.64 |
| miR-204 | 1.64 |
| miR-483 | 1.63 |
| miR-202 | 1.60 |
| miR-523 | 1.59 |
| miR-497 | 1.59 |
| miR-517c | 1.58 |
| miR-376a | 1.58 |
| miR-184 | 1.56 |
| miR-211 | 1.52 |
| miR-16 | 1.51 |
| miR-18a | 1.51 |
| miR-18a* | 1.51 |
| miR-negacon#2 | 0.96 |

In another test, DLD-1 was sown to a 96-well plate at 2500 cells per well, and cultured in an RPMI medium containing 10% FBS overnight. One day later, Pre-miR^{™}iRNA Precursor Molecules (manufactured by Ambion) that produce hsa-miR-7 (SEQ 10 NO:3), 24 (SEQ ID NO:11), 28 (SEQ ID NO:17), 34c, 95 (SEQ ID NO:24), 124a, 132 (SEQ ID NO:29), 134 (SEQ ID NO:33), 142-3p (SEQ ID NO:35), 147 (SEQ ID NO:37), 181a, 182* (SEQ ID NO:41), 183 (SEQ ID NO:42), 192 (SEQ ID NO:44), 197 (SEQ ID NO:53), 211 (SEQ ID NO:58), 222 (SEQ ID NO:61), 299-3p (SEQ ID NO:62), 329 (SEQ ID NO:68), 337 (SEQ ID NO:70), 342 (SEQ ID NO:72), 362 (SEQ ID NO:74), 368 (SEQ ID NO:75), 376a (SEQ ID NO:77), 376b (SEQ ID NO:78), 377 (SEQ ID NO:79), 380-5p (SEQ ID NO:81), 422b (SEQ ID NO:85), 424 (SEQ ID NO:49), 431 (SEQ ID NO:87), 432* (SEQ ID NO:88), 452 (SEQ ID NO:92), 452* (SEQ ID NO:93), 455 (SEQ ID NO:94), 483 (SEQ ID NO:95), 485-5p (SEQ ID NO:96), 489 (SEQ ID NO:98), 491 (SEQ ID NO:99), 493-5p (SEQ ID NO:100), 494 (SEQ ID NO:101), 505 (SEQ ID NO:104), 506 (SEQ ID NO:105), 509 (SEQ 10 NO:107), 510 (SEQ ID NO:108), 517a (SEQ ID NO:115), and 517c (SEQ ID NO:116) were introduced into the DLD-1 to obtain a final concentration of 50 nM by a lipofection method, specifically a method using Lipofectamine2000 (manufactured by Invitrogen). miR-negacon#2 (manufactured by Ambion) was also introduced into DLD-1, and this was used as the negative control. The lipofection was performed per the directions attached to the product.

Three or five days after introduction of the molecules by the lipofection method, cell viability was measured using CellTiter-Glo^{™}Luminescent Cell Viability Assay (manufactured by Promega) per the directions attached to the product. Taking the cell viability for the DLD-1 in the miR-negacon#2 introduction plot as 1.0, respective relative cell viability was calculated. As a result, as shown in Table 7, with introduction of molecules that produce hsa-miR-7, 24, 28, 95, 132, 134, 142-3p, 147, 182*, 183, 192, 197, 211, 222, 299-3p, 329, 337, 342, 362, 368, 376a, 376b, 377, 380-5p, 422b, 424, 431, 432*, 452, 452*, 455, 483, 485-5p, 489, 491, 493-5p, 494, 505, 506, 509, 510, 517a, and 517c, decreases of 50% or more in viable cell activity were observed. With introduction of molecules that produce hsa-miR-34c, 181a, and 124a, which are known to be associated with cancers, a decrease of 50% or more in cell viability was observed.

**[Table 7]**

| **Introduced miRNA** | cell viability (Day 3) | **cell viability (Day 5)** |
|---|---|---|
| miR-362 | 0.17 | 0.06 |
| miR-491 | 0.29 | 0.14 |
| miR-132 | 0.31 | 0.30 |
| miR-299-3p | 0.34 | 0.15 |
| miR-376a | 0.34 | 0.27 |
| miR-483 | 0.34 | 0.20 |
| miR-493-5p | 0.35 | 0.18 |
| miR-368 | 0.37 | 0.42 |
| miR-124a | 0.38 | 0.18 |
| miR-181a | 0.38 | 0.27 |
| miR-376b | 0.38 | 0.25 |
| miR-377 | 0.39 | 0.65 |
| miR-432* | 0.41 | 0.24 |
| miR-342 | 0.43 | 0.18 |
| miR-197 | 0.43 | 0.08 |
| miR-494 | 0.45 | 0.21 |
| miR-183 | 0.46 | 0.50 |
| miR-211 | 0.47 | 0.45 |
| miR-489 | 0.48 | 0.23 |
| miR-424 | 0.49 | 0.44 |
| miR-28 | 0.50 | 0.47 |
| miR-95 | 0.50 | 0.28 |
| miR-517c | 0.51 | 0.33 |
| miR-452* | 0.52 | 0.25 |
| miR-192 | 0.53 | 0.46 |
| miR-7 | 0.53 | 0.42 |
| miR-380-5p | 0.55 | 0.37 |
| miR-182* | 0.55 | 0.41 |
| miR-142-3p | 0.55 | 0.37 |
| miR-329 | 0.56 | 0.42 |
| miR-506 | 0.56 | 0.39 |
| miR-452 | 0.57 | 0.28 |
| miR-505 | 0.57 | 0.46 |
| miR-422b | 0.57 | 0.42 |
| miR-147 | 0.58 | 0.36 |
| miR-455 | 0.62 | 0.38 |
| miR-510 | 0.63 | 0.49 |
| miR-509 | 0.65 | 0.46 |
| miR-222 | 0.67 | 0.45 |
| miR-34c | 0.68 | 0.45 |
| miR-431 | 0.69 | 0.29 |
| miR-134 | 0.71 | 0.42 |
| miR-485 | 0.72 | 0.44 |
| miR-337 | 0.75 | 0.47 |
| miR-24 | 0.76 | 0.50 |
| miR-517a | 0.84 | 0.43 |

Antisense oligonucleotides that are complementary to miRNAs were introduced into DLD-1 to obtain a final concentration of 50 nM using a lipofection method. As the antisense oligonucleotides that are complementary to the miRNAs, Anti-miR^{™} miRNA Inhibitors of hsa-miR-197 (SEQ ID NO:53) and 483 (SEQ ID NO:95) (manufactured by Ambion) were used. Anti-miR^{™}miRNA Inhibitors-Negative Control#1 (hereinafter, referred to as anti-miR-negacon#1) (manufactured by Ambion) was also introduced into DLD-1, and this was used as the negative control. The lipofection was performed per the directions attached to the product.

Three days after introduction of the antisense oligonucleotides by the lipofection method, cell viability was measured using CellTiter-Glo^{™}Luminescent Cell Viability Assay (manufactured by Promega) per the directions attached to the product. Taking the cell viability for the DLD-1 in the anti-miR-negacon#1 introduction plot as 1.0, respective relative cell viability was calculated. As a result, as shown in Table 8, with introduction of antisense oligonucleotides that are complementary to hsa-miR-197 and 483, decreases of 50% or more in cell viability were observed.

**[Table 8]**

| **introduced antisense** | **cell viability (50 nM)** |
|---|---|
| anti-miR-483 | 0.43 |
| anti-miR-197 | 0.44 |

### Example 2

### Cell viability and apoptotic activity in ovarian cancer-derived cell line with miRNA expressed forcedly therein

Pre-miR^{™}miRNA Precursor Molecules (manufactured by Ambion) were introduced into an ovarian cancer-derived cell line, and the influences of the miRNAs produced from the molecules on cell viability and apoptotic activity were examined.
The A2780 human ovarian cancer-derived cell line (Nature, 295, 116-119 (1982); Science, 224, 994-996 (1984); Semin. Oncol., 11, 285-298 (1984); hereinafter to be referred to as A2780) was cultured with an RPMI1640 medium (manufactured by Invitrogen) containing 5% FBS (manufactured by JRH Biosciences) in a 37°C 5% CO₂ concentration incubator.

The A2780 was sown to a 96-well plate at 2500 cells per well, and cultured with an RPMI medium containing 10% FBS overnight. One day later, Pre-miR^{™} miRNA Precursor Molecules that generate hsa-miR-1 (SEQ ID NO:1), 7 (SEQ ID NO:3), 15a, 15b, 16, 18a (SEQ ID NO:5), 18a* (SEQ ID NO:7), 18b (SEQ ID NO:6), 28 (SEQ ID NO:17), 34b, 95 (SEQ ID NO:24), 98 (SEQ ID NO:25), 124a, 133a (SEQ ID NO:31), 133b (SEQ ID NO:32), 143, 145, 147 (SEQ ID NO:37), 181c (SEQ ID NO:39), 192 (SEQ ID NO:44), 193a (SEQ ID NO:46), 193b (SEQ ID NO:47), 195 (SEQ ID NO:48), 196a (SEQ ID NO:51), 196b (SEQ ID NO:52), 197 (SEQ ID NO:53), 199a* (SEQ ID NO:54), 202 (SEQ ID NO:56), 204 (SEQ ID NO:57), 211 (SEQ ID NO:58), 212 (SEQ ID NO:30), 215 (SEQ ID NO:45), 299-3p (SEQ ID NO:62), 324-3p (SEQ ID NO:66), 324-5p (SEQ ID NO:67), 337 (SEQ ID NO:70), 342 (SEQ ID NO:72), 346 (SEQ ID NO:73), 362 (SEQ ID NO:74), 368 (SEQ ID NO:75), 376a (SEQ ID NO:77), 376b (SEQ ID NO:78), 378 (SEQ ID NO:80), 380-5p (SEQ ID NO:81), 382 (SEQ ID NO:82), 422a (SEQ ID NO:84), 422b (SEQ ID NO:85), 424 (SEQ ID NO:49), 432* (SEQ ID NO:88), 451 (SEQ ID NO:91), 452 (SEQ ID NO:92), 452* (SEQ ID NO:93), 455 (SEQ ID NO:94), 483 (SEQ ID NO:95), 489 (SEQ ID NO:98), 491 (SEQ ID NO:99), 493-5p (SEQ ID NO:100), 494 (SEQ ID NO:101), 497 (SEQ ID NO:50), 501 (SEQ ID NO:102), 504 (SEQ ID NO:103), 506 (SEQ ID NO:105), 511 SEQ ID NO:109), 512-5p (SEQ ID NO:111), 517a (SEQ ID NO:115), 517c (SEQ ID NO:116), 518b (SEQ ID NO:118), 518c (SEQ ID NO:119), 518c* (SEQ ID NO:121), 518d (SEQ ID NO:120), 527 (SEQ ID NO:129), let-7a, let-7b, let-7d, let-7d, let-7e, let-7f, let-7g, and let-7i were introduced into the A2780 to obtain a final concentration of 5 nM or 25 nM using a lipofection method, specifically Lipofectamine2000 (manufactured by Invitrogen). miR-negacon#2 (manufactured by Ambion) was also introduced into A2780, and this was used as the negative control. The lipofection was performed per the directions attached to the product.

Three days after introduction of the molecules by the lipofection method, cell viability was measured using CellTiter-Glo^{™}Luminescent Cell Viability Assay (manufactured by Promega) per the directions attached to the product. Taking the cell viability for the A2780 in the control plot (Lipofectamine2000 only) as 1.0, respective relative cell viability was calculated. As a result, as shown in Table 9 (Table 9-1 and Table 9-2), with introduction of molecules that produce hsa-miR-1, 7, 18a, 18a*, 18b, 28, 95, 98, 133a, 133b, 147, 181c, 192, 193a, 193b, 195, 196a, 196b, 197, 199a*, 202, 204, 211, 212, 215, 299-3p, 324-3p, 324-5p, 342, 346, 362, 368, 376a, 376b, 378, 380-5p, 382, 422a, 422b, 424, 432*, 451, 452, 452*, 455, 483, 489, 491, 493-5p, 494, 497, 501, 504, 506, 511, 512-5p, 517a, 517c, 518b, 518c, 518c*, 518d, and 527, decreases of 40% or more in viable cell activity were observed. With introduction of molecules that produce hsa-miR-15a, 15b, 16, 34b, 124a, 143, 145, let-7a, let-7b, let-7d, let-7e, let-7f, let-7g, and let-7i, which are known to be associated with cancers, decreases of 40% or more in cell viability were observed.

**[Table 9-1]**

| **Introduced miRNA** | **cell viability (25 nM)** | **cell viability (5 nM)** |
|---|---|---|
| miR-124a | 0. 13 | 0. 24 |
| miR-192 | 0. 21 | 0. 32 |
| miR-193b | 0. 22 | 0. 35 |
| miR-193a | 0. 23 | 0. 33 |
| miR-491 | 0. 25 | 0. 41 |
| miR-506 | 0. 26 | 0. 35 |
| miR-195 | 0. 29 | 0. 44 |
| miR-497 | 0. 29 | 0. 37 |
| miR-34b | 0. 29 | 0. 36 |
| miR-493-5p | 0. 30 | 0. 47 |
| miR-196b | 0. 30 | 0. 43 |
| miR-197 | 0. 31 | 0. 43 |
| miR-489 | 0. 31 | 0. 48 |
| miR-452 | 0. 31 | 0. 45 |
| miR-147 | 0. 32 | 0. 40 |
| miR-324-3p | 0. 32 | 0. 50 |
| miR-15b | 0. 32 | 0. 42 |
| miR-518d | 0. 32 | 0. 40 |
| miR-215 | 0. 32 | 0. 33 |
| miR-378 | 0.33 | 0. 48 |
| miR-518b | 0. 33 | 0. 37 |
| miR-196a | 0.34 | 0. 48 |
| miR-517c | 0.34 | 0. 72 |
| miR-517a | 0. 34 | 0. 58 |
| miR-432* | 0. 34 | 0. 51 |
| miR-7 | 0. 34 | 0. 44 |
| miR-376b | 0. 35 | 0. 53 |
| miR-18a* | 0. 35 | 0. 58 |
| let-7b | 0. 36 | 0. 49 |
| miR-346 | 0. 36 | 0. 49 |
| miR-527 | 0. 36 | 0. 64 |
| miR-518c | 0. 37 | 0. 46 |
| let-7f | 0. 37 | 0. 49 |
| let-7g | 0. 37 | 0. 49 |
| let-7i | 0. 38 | 0. 57 |
| miR-98 | 0. 39 | 0. 51 |
| miR-15a | 0. 40 | 0. 54 |
| miR-299-3p | 0. 41 | 0. 70 |
| miR-511 | 0. 42 | 0.59 |
| miR-212 | 0.42 | 0. 64 |
| miR-424 | 0. 42 | 0. 61 |
| miR-483 | 0. 43 | 0. 62 |
| miR-28 | 0. 44 | 0. 53 |
| let-7d | 0. 44 | 0. 57 |
| let-7e | 0. 45 | 0. 59 |
| let-7a | 0. 46 | 0. 68 |
| miR-211 | 0. 46 | 0. 61 |
| miR-422a | 0. 46 | 0. 65 |

**[Table 9-2]**

| **introduced MRNA** | **cell viability (25 nM)** | **cell viability (5 nM)** |
|---|---|---|
| miR-324-5p | 0. 46 | 0. 68 |
| miR-504 | 0. 47 | 0. 61 |
| miR-204 | 0. 47 | 0. 66 |
| miR-368 | 0. 48 | 0. 59 |
| miR-199a* | 0. 48 | 0. 73 |
| miR-16 | 0. 48 | 0. 37 |
| miR-362 | 0. 49 | 0. 76 |
| miR-376a | 0. 49 | 0. 62 |
| miR-451 | 0. 49 | 0. 65 |
| miR-202 | 0. 49 | 0. 70 |
| miR-18a | 0. 49 | 0. 70 |
| miR-501 | 0. 50 | 0. 69 |
| miR-133a | 0. 51 | 0. 78 |
| miR-18b | 0. 51 | 0. 69 |
| miR-494 | 0. 52 | 0. 71 |
| miR-422b | 0. 52 | 0. 66 |
| miR-518c* | 0. 52 | 0. 78 |
| miR-512-5p | 0. 52 | 0. 72 |
| miR-382 | 0. 53 | 0. 77 |
| miR-342 | 0. 53 | 0. 78 |
| miR-95 | 0. 54 | 0. 79 |
| miR-181c | 0. 55 | 0. 72 |
| miR-380-5p | 0. 56 | 0. 53 |
| miR-145 | 0. 57 | 0. 86 |
| miR-1 | 0. 58 | 0. 78 |
| miR-452* | 0. 58 | 0. 79 |
| miR-143 | 0. 59 | 0. 75 |
| miR-133b | 0. 59 | 0. 75 |
| miR-455 | 0. 60 | 0. 76 |
| miR-negacon#2 | 0. 79 | 0. 96 |

Two days after introduction of the molecules (final concentration 25 nM) by the lipofection method, caspase 3/7 activity was measured using Caspase-Glo^{R}3/7 assay (manufactured by Promega) per the directions attached to the product. Taking the caspase 3/7 activity value for the A2780 in the control plot (Lipofectamine2000 only) as 1.0, respective relative caspase 3/7 activity values were calculated. As a result, as shown in Table 10, with introduction of molecules that produce hsa-miR-18a*, 133a, 193a, 193b, 197, 199a*, 212, 324-3p, 337, 432*, 452*, 489, 491, 493-5p, 512-5p, 517a, 517c, and 527, increases of 50% or more in caspase 3/7 activity were observed. With introduction of a molecule that produces hsa-miR-124a, which is known to be associated with cancers, an increase of 50% or more in caspase 3/7 activity was observed.

**[Table 10]**

| **introduced miRNA** | **caspase 3/7 activity** |
|---|---|
| miR-527 | 2. 88 |
| miR-193b | 2. 70 |
| miR-193a | 2. 63 |
| miR-199a* | 2. 18 |
| miR-517c | 2. 12 |
| miR-517a | 2. 02 |
| miR-512-5p | 1. 98 |
| miR-18a* | 1. 96 |
| miR-489 | 1. 91 |
| miR-493-5p | 1. 89 |
| miR-432* | 1. 84 |
| miR-197 | 1. 80 |
| miR-337 | 1. 76 |
| miR-133a | 1. 68 |
| miR-452* | 1. 68 |
| miR-124a | 1. 60 |
| miR-491 | 1. 59 |
| miR-212 | 1. 58 |
| miR-324-3p | 1. 56 |
| miR-negacon#2 | 1. 25 |

In another test, A2780 was sown to a 96-well plate at 2500 cells per well, and cultured in an RPMI medium containing 10% FBS overnight. One day later, Pre-miR^{™}miRNA Precursor Molecules of hsa-miR-7 (SEQ ID NO:3), 15a, 15b, 18a* (SEQ ID NO:7), 24 (SEQ ID NO:11), 26a (SEQ ID NO:14), 26b (SEQ ID NO:15), 34a, 34b, 34c, 124a, 130a (SEQ ID NO:26), 130b (SEQ ID NO:27), 134 (SEQ ID NO:33), 147 (SEQ ID NO:37), 192 (SEQ ID NO:44), 193a (SEQ ID NO:46), 193b (SEQ ID NO:47), 196a (SEQ ID NO:51), 196b (SEQ ID NO:52), 197 (SEQ ID NO:53), 199a* (SEQ ID NO:54), 204 (SEQ ID NO:57), 206 (SEQ ID NO:2), 211 (SEQ ID NO:58), 212 (SEQ ID NO:30), 215 (SEQ ID NO:45), 301 (SEQ ID NO:28), 346 (SEQ ID NO:73), 368 (SEQ ID NO:75), 371 (SEQ ID NO:76), 376b (SEQ ID NO:78), 378 (SEQ ID NO:80), 422a (SEQ ID NO:84), 424 (SEQ ID NO:49), 431 (SEQ ID NO:87), 432* (SEQ ID NO:88), 433 (SEQ ID NO:89), 449 (SEQ ID NO:90), 452 (SEQ ID NO:92), 483 (SEQ ID NO:95), 485-5p (SEQ ID NO:96), 488 (SEQ ID NO:97), 489 (SEQ ID NO:98), 491 (SEQ ID NO:99), 493-5p (SEQ ID NO:100), 497 (SEQ ID NO:50), 504 (SEQ ID NO:103), 505 (SEQ ID NO:104), 506 (SEQ ID NO:105), 507 (SEQ ID NO:106), 510 (SEQ ID NO:108), 511 (SEQ ID NO:109), 512-5p (SEQ ID NO:111), 513 (SEQ ID NO:112), 517a (SEQ ID NO:115), 517c (SEQ ID NO:116), 518b (SEQ ID NO:118), 518c (SEQ ID NO:119), 518d (SEQ ID NO:120), 526a (SEQ ID NO:128), 527 (SEQ ID NO:129), let-7b, let-7c, and let-7i were introduced into the A2780 to obtain a final concentration of 50 nM using a lipofection method, specifically Lipofectamine2000 (manufactured by Invitrogen). miR-negacon#2 (manufactured by Ambion) was also introduced into A2780, and this was used as the negative control. The lipofection was performed per the directions attached to the product.

Three or five days after introduction of the molecules by the lipofection method, cell viability was measured using CellTiter-Glo^{™}Luminescent Cell Viability Assay (manufactured by Promega) per the directions attached to the product. Taking the cell viability for the A2780 in the miR-negacon#2 introduction plot as 1.0, respective relative cell viability was calculated. As a result, as shown in Table 11 (Table 11-1 and Table 11-2), with introduction of molecules that produce hsa-miR-7, 18a*, 24, 26a, 26b, 134, 147, 192, 193a, 193b, 196a, 196b, 197, 199a*, 204, 206, 211, 212, 215, 346, 368, 371, 376b, 378, 422a, 424, 431, 432*, 433, 449, 452, 483, 485-5p, 488, 489, 491, 493-5p, 497, 504, 505, 506, 507, 510, 511, 512-5p, 513, 517a, 517c, 518b, 518c, 518d, 526a, and 527, decreases of 50% or more in cell viability were observed. With introduction of miRNA precursors of molecules that produce hsa-miR-15a, 15b, 34a, 34b, 34c, 124a, let-7b, let-7c, and let-7i, which are known to be associated with cancers, decreases of 50% or more in cell viability were observed. Conversely, with introduction of molecules that produce hsa-miR-130a, 130b, and 301, increases in cell viability were observed.

**[Table 11-1]**

| **introduced miRNA** | **cell viability (Day 3)** | **cell viability (Day 5)** |
|---|---|---|
| miR-124a | 0. 28 | 0. 09 |
| miR-517c | 0. 29 | 0. 20 |
| miR-193a | 0. 30 | 0. 45 |
| miR-506 | 0. 32 | 0. 36 |
| miR-199a* | 0. 35 | 0. 44 |
| miR-192 | 0. 37 | 0. 31 |
| miR-7 | 0. 37 | 0. 46 |
| miR-193b | 0. 38 | 0. 44 |
| miR-432* | 0. 38 | 0. 19 |
| miR-497 | 0. 38 | 0. 33 |
| miR-518b | 0. 39 | 0. 17 |
| miR-34b | 0. 39 | 0. 29 |
| miR-527 | 0. 39 | 0. 40 |
| miR-197 | 0. 39 | 0. 20 |
| miR-510 | 0. 41 | 0. 48 |
| miR-15b | 0. 41 | 0. 73 |
| miR-518c | 0. 41 | 0. 18 |
| miR-505 | 0. 42 | 0. 53 |
| miR-196b | 0. 43 | 0. 68 |
| miR-493-5p | 0. 43 | 0. 37 |
| miR-215 | 0. 43 | 0. 47 |
| miR-196a | 0. 43 | 0. 67 |
| miR-518d | 0. 44 | 0. 34 |
| miR-424 | 0. 46 | 0. 31 |
| miR-526a | 0. 46 | 0. 65 |
| miR-511 | 0. 46 | 0. 45 |
| miR-346 | 0. 46 | 0. 35 |
| miR-507 | 0. 47 | 0. 64 |
| miR-15a | 0. 47 | 0. 53 |
| miR-26a | 0. 49 | 0. 57 |
| miR-26b | 0. 49 | 0. 58 |
| miR-147 | 0. 49 | 0. 40 |
| miR-489 | 0. 50 | 0. 21 |
| miR-491 | 0. 50 | 0. 38 |
| let-7i | 0. 50 | 0. 47 |
| let-7c | 0. 50 | 0. 65 |
| miR-422a | 0. 51 | 0. 14 |
| let-7b | 0. 51 | 0. 48 |
| miR-488 | 0. 51 | 0. 44 |
| miR-18a* | 0. 51 | 0. 44 |
| miR-376b | 0. 51 | 0. 48 |
| miR-378 | 0. 52 | 0. 31 |
| miR-134 | 0. 53 | 0. 45 |
| miR-517a | 0. 54 | 0. 39 |
| miR-512-5p | 0. 57 | 0. 48 |
| miR-431 | 0. 58 | 0. 39 |
| miR-368 | 0. 59 | 0. 50 |
| miR-433 | 0. 59 | 0. 49 |

**[Table 11-2]**

| **introduced miRNA** | **cell viability (Day 3)** | **cell viability (Day 5)** |
|---|---|---|
| miR-206 | 0. 60 | 0. 49 |
| miR-24 | 0. 60 | 0. 46 |
| miR-449 | 0. 61 | 0. 26 |
| miR-212 | 0. 62 | 0. 45 |
| miR-513 | 0. 62 | 0. 39 |
| miR-485 | 0. 63 | 0. 36 |
| miR-483 | 0. 64 | 0. 42 |
| miR-504 | 0. 64 | 0. 33 |
| miR-452 | 0. 65 | 0. 42 |
| miR-34a | 0. 66 | 0. 42 |
| miR-211 | 0. 68 | 0. 41 |
| miR-34c | 0. 73 | 0. 50 |
| miR-204 | 0. 74 | 0. 41 |
| miR-371 | 0. 97 | 0. 49 |
| miR-130b | 1. 62 | 0. 99 |
| miR-130a | 1. 73 | 1. 18 |
| miR-301 | 2. 18 | 1. 23 |

Antisense oligonucleotides that are complementary to miRNAs were introduced into A2780 to obtain a final concentration of 50 nM using a lipofection method. As the antisense oligonucleotides that are complementary to the miRNAs, Anti-miR^{™} miRNA Inhibitors (manufactured by Ambion) of hsa-miR-10a (SEQ ID NO:4), 20b (SEQ ID NO:8), 25 (SEQ ID NO:12), 27b (SEQ ID NO:16), 29a (SEQ ID NO:18), 29c (SEQ ID NO:19), 30b (SEQ ID NO:20), 30c (SEQ ID NO:21), 32 (SEQ ID NO:13), 93 (SEQ ID NO:22), 106a (SEQ ID NO:9); 106b (SEQ ID NO:10), 137 (SEQ ID NO:34), 146a (SEQ ID NO:36), 149 (SEQ ID NO:38), 191, 195 (SEQ ID NO:48), 200a (SEQ ID NO:55), 204 (SEQ ID NO:57), 205 (SEQ ID NO:59), 208 (SEQ ID NO:60), 212 (SEQ ID NO:30), 215 (SEQ ID NO:45), 301 (SEQ ID NO:28), 302a* (SEQ ID NO:64), 302b* (SEQ ID NO:65), 302d (SEQ ID NO:23), 324-3p (SEQ ID NO:66), 331 (SEQ ID NO:69), 340 (SEQ ID NO:71), 362 (SEQ ID NO:74), 412 (SEQ ID NO:83), 423 (SEQ ID NO:86), 451 (SEQ ID NO:91), 452 (SEQ ID NO:92), 483 (SEQ ID NO:95), 516-5p (SEQ ID NO:113), 518e (SEQ ID NO:122), 518f (SEQ ID NO:123), 520h (SEQ ID NO:124), 524* (SEQ ID NO:126), and 525 (SEQ ID NO:127) were used. Anti-miR-negacon#1 (manufactured by Ambion) was also introduced into A2780, and this was used as the negative control. The lipofection was performed per the directions attached to the product.

Three days after introduction of the antisense oligonucleotides by the lipofection method, cell viability was measured using CellTiter-Glo^{™}Luminescent Cell Viability Assay (manufactured by Promega) per the directions attached to the product. Taking the cell viability for the A2780 in the anti-miR-negacon#1 introduction plot as 1.0, respective relative cell viability was calculated. As a result, as shown in Table 12, with introduction of the antisense oligonucleotides that are complementary to hsa-miR-10a, 20b, 25, 27b, 29a, 29c, 30b, 30c, 32, 93, 106a, 106b, 137, 146a, 149, 191, 195, 200a, 204, 205, 208, 212, 215, 301, 302a*, 302b*, 302d, 324-3p, 331, 340, 362, 412, 423, 451, 452, 483, 516-5p, 518e, 518f, 520h, 524*, and 525, decreases of 40% or more in cell viability were observed.

**[Table 12]**

| **introduced antisense** | **cell viability (50 nM)** |
|---|---|
| anti-miR-29a | 0. 39 |
| anti-miR-146 | 0. 39 |
| anti-miR-200a | 0. 41 |
| anti-miR-30c | 0. 42 |
| anti-miR-412 | 0. 43 |
| anti-miR-340 | 0. 44 |
| anti-miR-25 | 0. 44 |
| anti-miR-212 | 0. 47 |
| anti-miR-331 | 0. 48 |
| anti-miR-191 | 0. 48 |
| anti-miR-451 | 0. 48 |
| anti-miR-518f | 0. 49 |
| anti-miR-518e | 0. 49 |
| anti-miR-10a | 0. 49 |
| anti-miR-204 | 0. 49 |
| anti-miR-215 | 0. 53 |
| anti-miR-106a | 0. 52 |
| anti-miR-520h | 0. 52 |
| anti-miR-208 | 0. 53 |
| anti-miR-149 | 0. 54 |
| anti-miR-205 | 0. 54 |
| anti-miR-106b | 0. 54 |
| anti-miR-302b* | 0. 55 |
| anti-miR-483 | 0. 55 |
| anti-miR-30b | 0.56 |
| anti-miR-362 | 0. 57 |
| anti-miR-524* | 0. 57 |
| anti-miR-27b | 0. 57 |
| anti-miR-423 | 0. 57 |
| anti-miR-93 | 0. 58 |
| anti-miR-302a* | 0. 58 |
| anti-miR-525 | 0. 58 |
| anti-miR-324-3p | 0. 58 |
| anti-miR-301 | 0. 58 |
| anti-miR-302d | 0. 59 |
| anti-miR-20b | 0. 59 |
| anti-miR-195 | 0. 59 |
| anti-miR-137 | 0. 60 |
| anti-miR-516-5p | 0. 60 |
| anti-miR-452 | 0. 60 |
| anti-miR-29c | 0. 60 |
| anti-miR-32 | 0. 60 |

### Example 3

### Cell viability and apoptotic activity in prostatic cancer-derived cell line with miRNA expressed forcedly therein

Pre-miR^{™} miRNA Precursor Molecules (manufactured by Ambion) were introduced into a prostatic cancer-derived cell line, and the influences of the miRNAs produced from the molecules on cell viability and apoptotic activity were examined.

The PC-3 human prostatic cancer-derived cell line (hereinafter referred to as PC-3) was obtained from ATCC, and cultured with F-12 Kaighn's medium (manufactured by Invitrogen) containing 10% fetal bovine serum (FBS) (manufactured by JRH Biosciences) in an incubator at 37°C and a 5% CO₂ concentration.
The PC-3 was sown to a 96-well plate at 3000 cells per well, and cultured in F-12 Kaighn's containing 10% FBS overnight. One day later, Pre-miR^{™} miRNA Precursor Molecules that produce hsa-miR-15a, 15b, 16, 18a* (SEQ ID NO:7), 34b, 124a, 133b (SEQ ID NO:32), 181b, 192 (SEQ ID NO:44), 195 (SEQ ID NO:48), 196b (SEQ ID NO:52), 324-3p (SEQ ID NO:66), 337 (SEQ ID NO:70), 362 (SEQ ID NO:74), 368 (SEQ ID NO:75), 376b (SEQ ID NO:78), 380-5p (SEQ ID NO:81), 432* (SEQ ID NO:88), 452 (SEQ ID NO:92), 452* (SEQ ID NO:93), 489 (SEQ ID NO:98), 491 (SEQ ID NO:99), 493-5p (SEQ ID NO:100), and 494 (SEQ ID NO:101) were introduced into the PC-3 to obtain a final concentration of 25 nM using a lipofection method, specifically Lipofectamine2000 (manufactured by Invitrogen). miR-negacon#2 (manufactured by Ambion) was also introduced into PC-3, and this was used as the negative control. The lipofection was performed per the directions attached to the product.

Three days after introduction of the molecules by the lipofection method, cell viability was measured using CellTiter-Glo^{™}Luminescent Cell Viability Assay (manufactured by Promega) per the directions attached to the product. Taking the cell viability for the PC-3 in the control plot (Lipofectamine2000 only) as 1.0, respective relative cell viability was calculated. As a result, as shown in Table 13, with introduction of the molecules that produce hsa-miR-18a*, 133b, 192, 195, 196b, 324-3p, 337, 362, 368, 376b, 380-5p, 432*, 452, 452*, 489, 491, 493-5p, and 494, decreases of 40% or more in cell viability were observed. With the molecules that produce hsa-miR-15a, 15b, 16, 34b, 124a, and 181b, which are known to be associated with cancers, decreases of 40% or more in cell viability were observed.

**[Table 13]**

| **introduced miRNA** | **cell viability (25 nM)** |
|---|---|
| miR-362 | 0. 06 |
| miR-181b | 0. 19 |
| miR-491 | 0. 20 |
| miR-494 | 0. 36 |
| miR-489 | 0. 42 |
| miR-432* | 0. 42 |
| miR-452 | 0. 44 |
| miR-493-5p | 0. 45 |
| miR-124a | 0. 47 |
| miR-376b | 0. 51 |
| miR-15a | 0. 51 |
| miR-133b | 0. 52 |
| miR-16 | 0. 53 |
| miR-368 | 0. 53 |
| miR-15b | 0. 54 |
| miR-195 | 0. 55 |
| miR-452* | 0. 56 |
| miR-380-5p | 0. 56 |
| miR-196b | 0. 56 |
| miR-192 | 0. 57 |
| miR-337 | 0. 57 |
| miR-34b | 0. 57 |
| miR-324-3p | 0. 58 |
| miR-18a* | 0. 60 |
| miR-negacon#2 | 0. 95 |

Two days after introduction of the molecules by the lipofection method, caspase 3/7 activity was measured using Gaspase-Glo^{R} 3/7 assay (manufactured by Promega) per the directions attached to the product. Taking the caspase 3/7 activity value for the PC-3 in the control plot (Lipofectamine2000 only) as 1.0, respective relative caspase 3/7 activity values were calculated. As a result, as shown in Table 14, with introduction of the molecules that produce hsa-miR-133b, 337, 362, 432*, 452*, 491, and 493-5p, increases of 50% or more in caspase 3/7 activity were observed. With introduction of the molecule that produces hsa-miR-181b, which is known to be associated with cancers, an increase of 50% or more in caspase 3/7 activity was observed.

**[Table 14]**

| **introduced miRNA** | **caspase 3/7 activity** |
|---|---|
| miR-491 | 5. 12 |
| miR-362 | 3. 27 |
| miR-181b | 2. 76 |
| miR-432* | 2. 38 |
| miR-452* | 2. 13 |
| miR-133b | 1. 77 |
| miR-493-5p | 1. 65 |
| miR-337 | 1. 62 |
| miR-negacon#2 | 1. 11 |

### Example 4

### BrdU uptake activity in colorectal cancer-derived cell line (DLD-1) with miRNAs expressed forcedly therein

Pre-miR^{™}miRNA Precursor Molecules (manufactured by Ambion) were introduced into DLD-1, and the influences of the miRNAs produced from the molecules on BrdU uptake activity were examined.
The DLD-1 was sown to a 96-well plate at 2500 cells per well, and cultured in an RPMI medium containing 10% FBS overnight. One day later, Pre-miR^{™} miRNA Precursor Molecules that produce hsa-miR-147 (SEQ ID NO:37), 342 (SEQ ID NO:72), 362 (SEQ ID NO:74), 376b (SEQ ID NO:78), 483 (SEQ ID NO:95), and 494 (SEQ ID NO:101) were introduced into the DLD-1 to obtain a final concentration of 25 nM using a lipofection method, specifically Lipofectamine2000 (manufactured by Invitrogen). miR-negacon#2 (manufactured by Ambion) was also introduced into DLD-1, and this was used as the negative control. The lipofection was performed per the directions attached to the product.

One day after introduction of the molecules by the lipofection method, BrdU uptake activity was measured using a BrdU labeling & detection kit III (manufactured by Roche) per the directions attached to the product. Taking the BrdU uptake activity for the DLD-1 in the miR-negacon#2 introduction plot as 1.0, respective relative BrdU uptake activities were calculated. As a result, as shown in Table 15, with introduction of the molecules that produce hsa-miR-147, 342, 362, 376b, 483, and 494, decreases of 40% or more in BrdU uptake activity were observed.

**[Table 15]**

| **introduced MRNA** | **BrdU uptake activity** |
|---|---|
| miR-342 | 0. 22 |
| miR-362 | 0. 48 |
| miR-147 | 0. 52 |
| miR-376b | 0. 56 |
| miR-494 | 0. 57 |
| miR-483 | 0. 59 |

### BrdU uptake activity in ovarian cancer-derived cell line (A2780) with miRNAs expressed forcedly therein

Pre-miR^{™}miRNA Precursor Molecules (manufactured by Ambion) were introduced into A2780, and the influences of the miRNAs produced from the molecules on BrdU uptake activity were examined.

A2780 was sown to a 96-well plate at 4000 cells per well, and cultured in an RPMI medium containing 10% FBS overnight. One day later, the Pre-miR^{™} miRNA Precursor Molecules of hsa-miR-15a, 16, 124a, 147 (SEQ ID NO:37), 192 (SEQ ID NO:44), 193a (SEQ ID NO:46), 193b (SEQ ID NO:47), 215 (SEQ ID NO:45), 378 (SEQ ID NO:80), 432* (SEQ ID NO:88), 506 (SEQ ID NO:105), and 518c (SEQ ID NO:119) were introduced into the A2780 to obtain a final concentration of 25 nM using a lipofection method, specifically Lipofectamine2000 (manufactured by Invitrogen). miR-negacon#2 (manufactured by Ambion) was also introduced into A2780, and this was used as the negative control. The lipofection was performed per the directions attached to the product.

One day after introduction of the molecules by the lipofection method, BrdU uptake activity was measured using the BrdU labeling & detection kit III (manufactured by Roche) per the directions attached to the product. Taking the BrdU uptake activity for the A2780 in the miR-negacon#2 introduction plot as 1.0, respective relative BrdU uptake activities were calculated. As a result, as shown in Table 16, with introduction of the molecules that produce hsa-miR-hsa-miR-147, 192, 193a, 193b, 215, 378, 432*, 506, and 518c, decreases of 40% or more in BrdU uptake activity were observed. With introduction of the molecules that produce hsa-miR-15a, 16, and 124a, which are known to be associated with cancers, decreases of 40% or more in BrdU uptake activity were observed.

**[Table 16]**

| | |
|---|---|
| | |

| **introduced miRNA** | **BrdU uptake activity** |
|---|---|
| miR-124a miR-518c | 0. 32 |
| | 0. 37 |
| miR-506 | 0. 42 |
| miR-15a | 0. 45 |
| miR-192 | 0. 48 |
| miR-193a | 0. 48 |
| miR-215 | 0. 49 |
| miR-193b | 0. 51 |
| miR-16 | 0. 52 |
| miR-432* | 0. 54 |
| miR-378 | 0. 54 |
| miR-147 | 0. 56 |

### Example 5

### Analysis of the expression level of proteins encoded by target genes for hsa-miR-337, 342, 432*, 491, and 518b

Molecules that produce hsa-miR-337, 342, 432*, 491, and 518b, which exhibited DLD-1 proliferation suppression or caspase activity in Example 1, were introduced into DLD-1, and changes in the amounts of proteins encoded by the target genes were examined.

Pre-miR^{™}miRNA Precursor Molecules (manufactured by Ambion) that produce hsa-miR-16, 147, 337, 342, 432*, 491, and 518b were introduced into previously sown 1x10⁵ cells of DLD-1 to obtain a final concentration of 25 nM, using Lipofectamine2000. miR-negacon#2 was also introduced, and this was used as the negative control. Also, si-Bc1211, si-Birc5, and si-Bc191 (manufactured by QIAGEN) were introduced to obtain a final concentration of 25 nM, and these were used as the positive controls. Two days after the introduction, the cells were recovered in solution in an RIPA buffer [50mM Tris-HCL (pH 8.0), 150mM NaCl, 1% Nonident P-40, 0.5% sodium deoxycholate, 0.1% sodium dodecyl sulfate, 1% Protease Inhibitor Cocktail SetIII (manufactured by Calbiochem)]. After separation by a conventional method of SDS-PAGE, the amounts of the proteins of Bc1211, Birc5 and Bc191 were detected by a Western blotting method. For detection of Bc1211, an anti-Bc1211 antibody (manufactured by BD Biosciences) was used; for detection of Birc5, an anti-Birc5 antibody (manufactured by R&D Systems) was used; for detection of Bc191, an anti-Bc191 antibody (manufactured by Abnova) was used. Also detected was the amount of the alpha-tubulin protein; this was used as the control for the amount of sample supplied. To detect alpha-tubulin, an anti-alpha-tubulin antibody (manufactured by Sigma) was used.

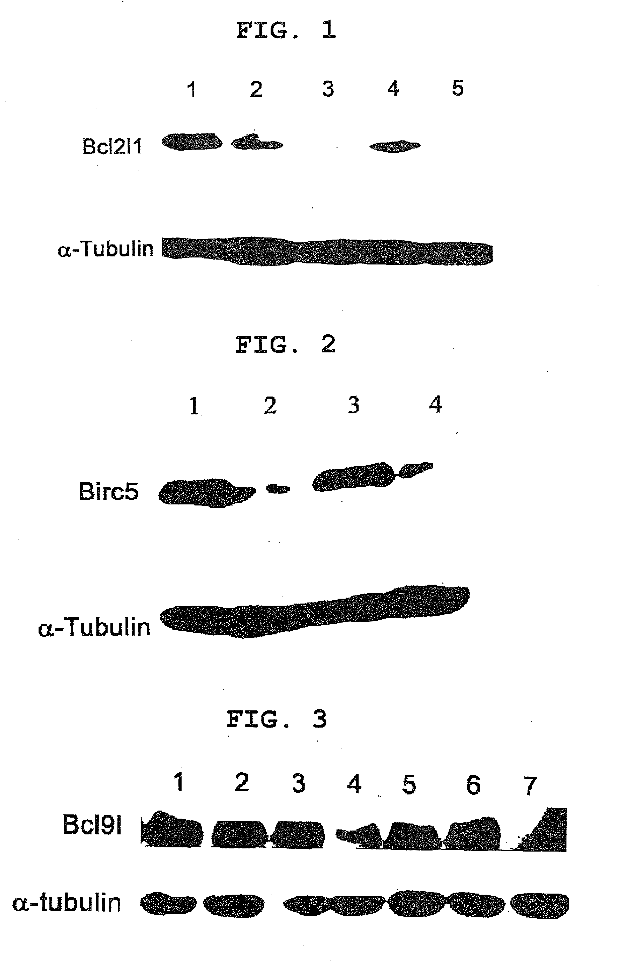

As a result, as shown in Figure 1, the DLD-1 with hsa-miR-337, 342, and 491 expressed forcedly therein exhibited decreased amounts of the protein of Bc1211 compared with the negative control DLD-1, showing that the Bc1211 gene is the target gene for hsa-miR-337, 342, and 491, and has its expression regulated. As shown in Figure 2, the DLD-1 with hsa-miR-337 expressed forcedly therein exhibited a decreased amount of the protein of Birc5 compared with the negative control DLD-1, showing that the Birc5 gene is the target gene for hsa-miR-337, and has its expression regulated. Furthermore, as shown in Figure 3, the DLD-1 with hsa-miR-432* or 518b expressed forcedly therein exhibited decreased amounts of the protein of Bc191 compared with the negative control DLD-1, showing that the Bc191 gene is a target gene for hsa-miR-432* and 518b, and has its expression regulated.

### Example 6

### Cell viability and apoptotic activity in colorectal cancer-derived cell line with mRNAs expressed forcedly therein

DLD-1 was sown to a 96-well plate at 2500 cells per well, and cultured in an RPMI medium containing 10% FBS overnight. One day later, Pre-miR^{™}miRNA Precursor Molecules that produce hsa-miR-493-3p (SEQ ID NO:2061), 542-3p (SEQ ID NO:2063), 571 (SEQ ID NO:2076), 579 (SEQ ID NO:2077), 625 (SEQ ID NO:2099), 630 (SEQ ID NO:2101), 634 (SEQ ID NO:2103), 650 (SEQ ID NO:2108), and 653 (SEQ ID NO:2110) were introduced into the DLD-1 to obtain a final concentration of 50 nM using a lipofection method, specifically Lipofectamine2000 (manufactured by Invitrogen). miR-negacon#2 (manufactured by Ambion) was also introduced into DLD-1, and this was used as the negative control. The lipofection was performed per the directions attached to the product.

Three days after introduction of the miRNA precursors by the lipofection method, cell viability was measured using CellTiter-Glo^{™}Luminescent Cell Viability Assay (manufactured by Promega) per the directions attached to the product. Taking the cell viability for the DLD-1 in the miR-negacon#2 introduction plot as 1.0, respective relative cell viability was calculated. As a result, as shown in Table 17, with introduction of the molecules that produce hsa-miR-493-3p, 542-3p, 571, 579, 625, 630, 634, 650, and 653, decreases of 50% or more in cell viability were observed.

In another test, two days after introduction of the Pre-miR^{™} miRNA Precursor Molecules that produces hsa-miR-634 by the lipofection method, caspase 3/7 activity was measured using Caspase-Glo^{R} 3/7 assay (manufactured by Promega) per the directions attached to the product. Taking the caspase activity for the DLD-1 in the miR-negacon#2 introduction plot as 1.0, relative caspase activity was calculated. As a result, with introduction of the molecule that produces hsa-miR-634, an increase of 50% or more in caspase activity was observed.

**[Table 17]**

| | |
|---|---|
| **introduced miRNA** | **cell viability (Day 3)** |
| miR-634 | 0. 37 |
| miR-653 | 0. 41 |
| miR-650 | 0. 43 |
| miR-493-3p | 0. 44 |
| miR-542-3p | 0. 45 |
| miR-571 | 0. 45 |
| miR-625 | 0. 46 |
| miR-630 | 0. 48 |
| miR-579 | 0. 49 |

Antisense oligonucleotides that are complementary to miRNAs were introduced into DLD-1 to obtain a final concentration of 50 nM using a lipofection method. As the antisense oligonucleotides that are complementary to the miRNAs, Anti-miR^{™} miRNA Inhibitors (manufactured by Ambion) of hsa-miR-411 (SEQ ID NO:2057), 425-5p (SEQ ID NO:2059), 449b (SEQ ID NO:917), 532 (SEQ ID NO:2062), 548b (SEQ ID NO:2066), 558 (SEQ ID NO:2072), 589 (SEQ ID NO:2079), 593 (SEQ ID NO:2081), 599 (SEQ ID NO:2083), 615 (SEQ ID NO:2093), 637 (SEQ ID NO:2104), 657 (SEQ ID NO:2112), 769-5p (SEQ ID NO:2114), and 92b (SEQ ID NO:819) were used. Anti-miR-negacon#1 (manufactured by Ambion) was also introduced into DLD-1, and this was used as the negative control. The lipofection was performed per the directions attached to the product.

Three days after introduction of the antisense oligonucleotides by the lipofection method, cell viability was measured using CellTiter-Glo^{™}Luminescent Cell Viability Assay (manufactured by Promega) per the directions attached to the product. Taking the cell viability for the DLD-1 in the anti-miR-negacon#1 introduction plot as 1.0, respective relative cell viability was calculated. As a result, as shown in Table 18, with introduction of the antisense oligonucleotides complementary to hsa-miR-411, 425-5p, 449b, 532, 548b, 558, 589, 593, 599, 615, 637, 657, 769-5p, and 92b, decreases of 40% or more in cell viability were observed.

**[Table 18]**

| **introduced antisense RNA** | **cell viability (Day 3)** |
|---|---|
| anti-miR-593 | 0. 52 |
| anti-miR-411 | 0. 53 |
| anti-miR-589 | 0. 55 |
| anti-miR-558 | 0. 56 |
| anti-miR-92b | 0. 57 |
| anti-miR-657 | 0. 57 |
| anti-miR-548b | 0. 57 |
| anti-miR-615 | 0. 58 |
| anti-miR-532 | 0. 58 |
| anti-miR-425-5p | 0. 59 |
| anti-miR-599 | 0. 59 |
| anti-miR-637 | 0. 59 |
| anti-miR-769-5p | 0. 60 |
| anti-miR-449b | 0. 60 |

### Example 7

### Cell viability in ovarian cancer-derived cell line with miRNAs expressed forcedly therein

A2780 was sown to a 96-well plate at 2500 cells per well, and cultured in an RPMI medium containing 10% FBS overnight. One day later, Pre-miR^{™} miRNA Precursor Molecules that produce hsa-miR-421 (SEQ ID NO:2058), 454-5p (SEQ ID NO:2060), 542-3p (SEQ ID NO:2063), 545 (SEQ ID NO:2064), 548a (SEQ ID NO:2065), 548d (SEQ ID NO:2067), 550 (SEQ ID NO:2068), 551b (SEQ ID NO:2069), 552 (SEQ ID NO:2070), 553 (SEQ ID NO:2071), 557 (SEQ ID NO:925), 561 (SEQ ID NO:2073), 563 (SEQ ID NO:916), 565 (SEQ ID NO:2074), 570 (SEQ ID NO:2075), 571 (SEQ ID NO:2076), 584 (SEQ ID NO:2078), 591 (SEQ ID NO:2080), 595 (SEQ ID NO:2082), 600 (SEQ ID NO:2084), 601 (SEQ ID NO:2085), 604 (SEQ ID NO:2086), 605 (SEQ ID NO:2087), 606 (SEQ ID NO:2088), 609 (SEQ ID NO:2089), 610 (SEQ ID NO:2090), 611 (SEQ ID NO:2091), 613 (SEQ ID NO:803), 614 (SEQ ID NO:2092), 618 (SEQ ID NO:2096), 621 (SEQ ID NO:2097), 624 (SEQ ID NO:2098), 625 (SEQ ID NO:2099), 627 (SEQ ID NO:2100), 630 (SEQ ID NO:2101), 631 (SEQ ID NO:2102), 634 (SEQ ID NO:2103), 637 (SEQ ID NO:2104), 646 (SEQ ID NO:2105), 647 (SEQ ID NO:2106), 648 (SEQ ID NO:2107), 653 (SEQ ID NO:2110), 654 (SEQ ID NO:2111), and 661 (SEQ ID NO:2113) were introduced into the A2780 to obtain a final concentration of 50 nM using a lipofection method, specifically Lipofectamine2000 (manufactured by Invitrogen). miR-negacon#2 (manufactured by Ambion) was also introduced into A2780, and this was used as the negative control. The lipofection was performed per the directions attached to the product.

Three days after introduction of the molecules by the lipofection method, cell viability was measured using CellTiter-Glo^{™}Luminescent Cell Viability Assay (manufactured by Promega) per the directions attached to the product. Taking the cell viability for the A2780 in the miR-negacon#2 introduction plot as 1.0, respective relative cell viability was calculated. As a result, as shown in Table 19, with introduction of the molecules that produce hsa-miR-421, 454-5p, 542-3p, 545, 548a, 548d, 550, 551b, 552, 553, 557, 561, 563, 565, 570, 571, 584, 591, 595, 600, 601, 604, 605, 606, 609, 610, 611, 613, 614, 618, 621, 624, 625, 627, 630, 631, 634, 637, 646, 647, 648, 653, 654, and 661, decreases of 50% or more in cell viability were observed.

**[Table 19]**

| **introduced miRNA** | **cell viability (Day 3)** |
|---|---|
| miR-634 | 0. 21 |
| miR-552 | 0. 21 |
| miR-611 | 0. 22 |
| miR-604 | 0. 26 |
| miR-646 | 0. 28 |
| miR-630 | 0. 30 |
| miR-421 | 0. 30 |
| miR-571 | 0. 32 |
| miR-647 | 0. 33 |
| miR-605 | 0. 33 |
| miR-627 | 0. 34 |
| miR-614 | 0. 37 |
| miR-637 | 0. 37 |
| miR-631 | 0. 38 |
| miR-609 | 0. 38 |
| miR-654 | 0. 38 |
| miR-454-5p | 0. 39 |
| miR-542-3p | 0. 40 |
| miR-601 | 0. 42 |
| miR-561 | 0. 42 |
| miR-600 | 0. 42 |
| miR-618 | 0. 42 |
| miR-653 | 0. 43 |
| miR-591 | 0. 43 |
| miR-548a | 0. 44 |
| miR-565 | 0. 44 |
| miR-584 | 0. 45 |
| miR-563 | 0. 45 |
| miR-548d | 0. 46 |
| miR-570 | 0. 46 |
| miR-606 | 0. 47 |
| miR-550 | 0. 47 |
| miR-557 | 0. 47 |
| miR-648 | 0. 48 |
| miR-621 | 0. 48 |
| miR-545 | 0. 49 |
| miR-551b | 0. 49 |
| miR-625 | 0. 49 |
| miR-595 | 0. 49 |
| miR-624 | 0. 49 |
| miR-661 | 0. 49 |
| miR-610 | 0. 50 |
| miR-613 | 0. 50 |
| miR-553 | 0. 50 |

Antisense oligonucleotides that are complementary to miRNAs were introduced into A2780 to obtain a final concentration of 50 nM using a lipofection method. As the antisense oligonucleotides that are complementary to the miRNAs, Anti-miR^{™} miRNA Inhibitors (manufactured by Ambion) of hsa-miR-611 (SEQ ID NO:2091), 616 (SEQ ID NO:2094), 617 (SEQ ID NO:2095), 651 (SEQ ID NO:2109), and 657 (SEQ ID NO:2112) were used. Anti-miR-negacon#1 (manufactured by Ambion) was also introduced into A2780, and this was used as the negative control. The lipofection was performed per the directions attached to the product.

Three days after introduction of the antisense oligonucleotides by the lipofection method, cell viability was measured using CellTiter-Glo^{™}Luminescent Cell Viability Assay (manufactured by Promega) per the directions attached to the product. Taking the cell viability for the A2780 in the anti-miR-negacon#1 introduction plot as 1.0, respective relative cell viability was calculated. As a result, as shown in Table 20, with introduction of the antisense oligonucleotides that are complementary to hsa-miR-611, 616, 617, 651, and 657, decreases of 40% or more in cell viability were observed.

**[Table 20]**

| **introduced antisense RNA** | **cell viability (Day 3)** |
|---|---|
| anti-miR-617 | 0. 58 |
| anti-miR-611 | 0. 59 |
| anti-miR-651 | 0. 59 |
| anti-miR-657 | 0. 59 |
| anti-miR-616 | 0. 60 |

### Example 8

### Tumorigenesis by transplantation of colorectal cancer-derived cell line with miRNAs expressed forcedly therein to nude mice

DLD-1 incorporating molecules that produce hsa-miR-147, 342, and 491, which exhibited DLD-1 proliferation suppression or caspase activity in Example 1, was transplanted to nude mice, and the influences of miRNAs on tumorigenesis were examined.

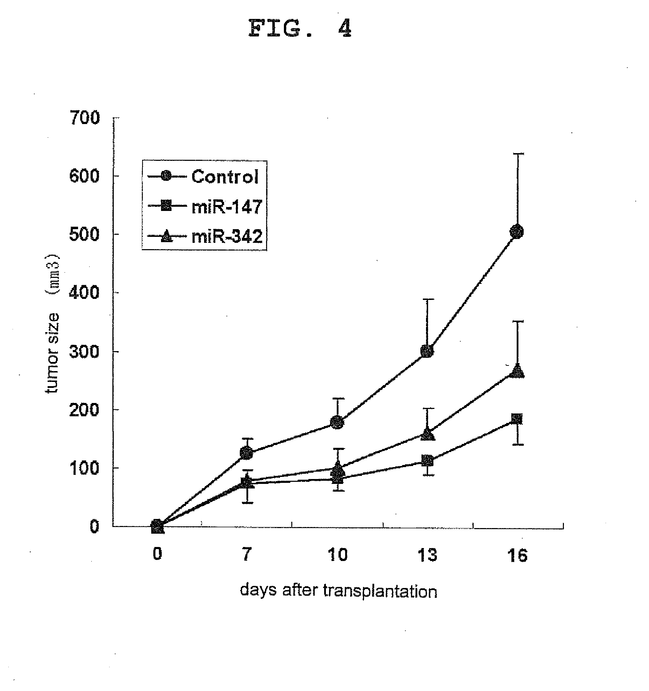

Pre-miR^{™} miRNA Precursor Molecules (manufactured by Ambion) that produce hsa-miR-147, 342, and 491 were introduced into previously sown 1x10⁶ cells of DLD-1 to obtain a final concentration of 50 nM using Lipofectoamine 2000. miR-negacon#2 was also introduced, and this was used as the negative control. One day after the introduction, cells were detached using 0.05% trypsin solution (manufactured by Gibco). The detached cells were mixed with Matrigel (manufactured by BD Bioscience), and 2.5×10⁵ cells were subcutaneously transplanted to the flanks of nude mice at 6 weeks of age. The major diameters and minor diameters of the tumors formed after 7, 10, 13, and 16 days of transplantation were measured. Tumor size was calculated as major diameter×(minor diameter)2×0.5. As shown in Figure 4, with transplantation of the DLD-1 with hsa-miR-147 or 342 expressed forcedly therein, tumorigenesis was suppressed compared with the negative control.

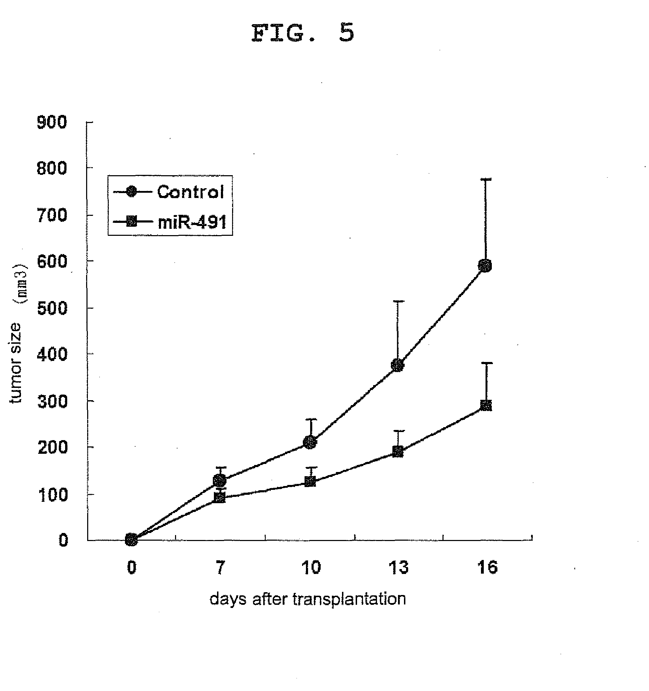

As shown in Figure 5, with transplantation of the DLD-1 with hsa-miR-491 expressed forcedly therein, tumorigenesis was suppressed, compared with the negative control.

### Industrial Applicability

An agent for suppressing or promoting cell proliferation, a diagnostic reagent or therapeutic drug for a disease resulting from a cell proliferation abnormality, an agent for inducing apoptosis, an agent for suppressing or promoting the expression of a target gene for a nucleic acid such as a miRNA, and a method of suppressing or promoting cell proliferation can be provided.

### Sequence Listing Free Text

### SEQ ID NO: 1399-n is a, c, g or u

## Claims

1. An agent for suppressing or promoting cell proliferation, comprising, as an active ingredient, any one of the nucleic acids (a) to (h) below:
(a) a nucleic acid consisting of a nucleotide sequence shown by any one of SEQ ID NO:1-939, 2057-2132, 2212-2261 and 2315-2321;
(b) a nucleic acid consisting of 17 to 28 nucleotides comprising a nucleic acid consisting of a nucleotide sequence shown by any one of SEQ ID NO:1-939, 2057-2132, 2212-2261 and 2315-2321;
(c) a nucleic acid consisting of a nucleotide sequence having an identity of 90% or more to a nucleotide sequence shown by any one of SEQ ID NO:1-939, 2057-2132, 2212-2261 and 2315-2321;
(d) a nucleic acid that hybridizes under stringent conditions with a complementary strand for a nucleic acid consisting of a nucleotide sequence shown by any one of SEQ ID NO:1-939, 2057-2132, 2212-2261 and 2315-2321;
(e) a nucleic acid comprising the sequence of 2nd to 8th nucleotides of a nucleotide sequence shown by any one of SEQ ID NO:1-939, 2057-2132, 2212-2261 and 2315-2321;
(f) a nucleic acid consisting of a nucleotide sequence shown by any one of SEQ ID NO:940-2056, 2133-2211, 2262-2314 and 2322-2328;
(g) a nucleic acid consisting of a nucleotide sequence having an identity of 90% or more to a nucleotide sequence shown by any one of SEQ ID NO:940-2056, 2133-2211, 2262-2314 and 2322-2328;
(h) a nucleic acid that hybridizes under stringent conditions with a complementary strand for a nucleic acid consisting of a nucleotide sequence shown by any one of SEQ ID NO:940-2056, 2133-2211, 2262-2314 and 2322-2328.

2. The agent according to claim 1 , wherein the nucleic acid is a microRNA or microRNA precursor.

3. An agent for suppressing or promoting cell proliferation, comprising, as an active ingredient, a nucleic acid consisting of a nucleotide sequence complementary to the nucleotide sequence of the nucleic acid according to claim 1.

4. An agent for suppressing or promoting cell proliferation, comprising, as an active ingredient, a vector that expresses the nucleic acid according to claim 1 or 3.

5. An agent for suppressing or promoting cell proliferation, comprising, as an active ingredient, a substance that suppresses the expression of a gene having a target nucleotide sequence for the nucleic acid according to claim 1.

6. An agent for suppressing or promoting cell proliferation, comprising, as an active ingredient, a substance that promotes the expression of a gene having a target nucleotide sequence for the nucleic acid according to claim 1.

7. The agent according to claim 5 or 6, wherein the substance that suppresses or promotes the expression is a nucleic acid.

8. The agent according to claim 7, wherein the nucleic acid is an siRNA.

9. An agent for suppressing or promoting cell proliferation, comprising, as an active ingredient, a vector that expresses the nucleic acid according to claim 7.

10. A therapeutic drug for treating a disease resulting from a cell proliferation abnormality, comprising, as an active ingredient, the agent according to any one of claims 1 to 9.

11. A therapeutic drug for treating a disease resulting from a cell proliferation abnormality, comprising, as an active ingredient, the nucleic acid according to any one of claims 1), (3) and (7).

12. A reagent for diagnosing a disease resulting from a cell proliferation abnormality, comprising, as an active ingredient, a reagent that detects the expression level of the nucleic acid according to claim 1, a mutation of the nucleic acid, or
a mutation of the genome that encodes the nucleic acid.

13. The therapeutic drug or diagnostic reagent according to any one of claims 10 to 12, wherein the disease resulting from a cell proliferation abnormality is a cancer.

14. A method of treating a disease resulting from a cell proliferation abnormality, comprising administering an effective amount of the nucleic acid according to any one of claims 1, 3 and 7.

15. A method of diagnosing a disease resulting from a cell proliferation abnormality, comprising detecting the expression level of the nucleic acid according to claim 1, a mutation of the nucleic acid, or a mutation of the genome that encodes the nucleic acid.

16. The method of treating or diagnosing according to claim 14 or 15, wherein the disease resulting from a cell proliferation abnormality is a cancer.

17. Use of the nucleic acid according to any one of claims 1, 3 and 7 for the production of a therapeutic drug for a disease resulting from a cell proliferation abnormality.

18. The use according to claim 17, wherein the disease resulting from a cell proliferation abnormality is a cancer.

19. An agent for suppressing the expression of a target gene for the nucleic acid according to claim 1, comprising the nucleic acid as an active ingredient.

20. An agent for promoting the expression of a target gene for the nucleic acid according to claim 1, comprising the nucleic acid according to claim 3 as an active ingredient.

21. The agent for suppressing or promoting the expression of a target gene for the nucleic acid according to claim 1, comprising the vector according to claim 4 as an active ingredient.

22. A method of promoting or suppressing cell proliferation, comprising using the nucleic acid according to claim 1 or 3.

23. A method of promoting or suppressing cell proliferation, comprising using the vector according to claim 4.

24. A method of promoting or suppressing cell proliferation, comprising using a substance that suppresses the expression of a target gene for the nucleic acid according to claim 1.

25. A method of promoting or suppressing cell proliferation, comprising using a substance that promotes the expression of a target gene for the nucleic acid according to claim 1.

26. The method of promoting or suppressing cell proliferation according to claim 24 or 25, wherein the substance that suppresses or promotes the expression is a nucleic acid.

27. The method of promoting or suppressing cell proliferation according to claim 26, wherein the nucleic acid is an siRNA.

28. A method of promoting or suppressing cell proliferation, comprising using a vector that expresses the nucleic acid according to claim 26.

29. A method of suppressing the expression of a target gene for the nucleic acid according to claim 1, comprising using the nucleic acid.

30. A method of promoting the expression of a target gene for the nucleic acid according to claim 1, comprising using the nucleic acid according to claim 3.

31. A method of suppressing or promoting the expression of a target gene for the nucleic acid according to claim 1, comprising using the vector according to claim 4.

32. A method of screening for an agent for promoting or suppressing cell proliferation, comprising using promotion or suppression of the expression or function of the nucleic acid according to claim 1 as an index.


SEQUENCE LISTING

<110>  Kyowa Hakko Kirin Co., Ltd.

<120>  Nucleic Acid Capable of Regulating the Proliferation of Cell

<130>  S1667 EP

<140>  EP 08 83 5543.3
<141>  2008-10-03

<150>  JP2007-260393
<151>  2007-10-03

<160>  2328

<170>  PatentIn version 3.3

<210>  1
<211>  21
<212>  RNA
<213>  Homo sapiens

<400>  1
uggaauguaa agaaguaugu a                                                21


<210>  2
<211>  22
<212>  RNA
<213>  Homo sapiens

<400>  2
uggaauguaa ggaagugugu gg                                               22


<210>  3
<211>  22
<212>  RNA
<213>  Homo sapiens

<400>  3
uggaagacua gugauuuugu ug                                               22


<210>  4
<211>  23
<212>  RNA
<213>  Homo sapiens

<400>  4
uacccuguag auccgaauuu gug                                              23


<210>  5
<211>  22
<212>  RNA
<213>  Homo sapiens

<400>  5
uaaggugcau cuagugcaga ua                                               22


<210>  6
<211>  22
<212>  RNA
<213>  Homo sapiens

<400>  6
uaaggugcau cuagugcagu ua                                              22

<210>  7
<211>  21
<212>  RNA
<213>  Homo sapiens

<400>  7
acugcccuaa gugcuccuuc u                                               21

<210>  8
<211>  23
<212>  RNA
<213>  Homo sapiens

<400>  8
caaagugcuc auagugcagg uag                                             23

<210>  9
<211>  24
<212>  RNA
<213>  Homo sapiens

<400>  9
aaaagugcuu acagugcagg uagc                                            24

<210>  10
<211>  21
<212>  RNA
<213>  Homo sapiens

<400>  10
uaaagugcug acagugcaga u                                               21

<210>  11
<211>  22
<212>  RNA
<213>  Homo sapiens

<400>  11
uggcucaguu cagcaggaac ag                                              22

<210>  12
<211>  22
<212>  RNA
<213>  Homo sapiens

<400>  12
cauugcacuu gucucggucu ga                                              22

<210>  13
<211>  21
<212>  RNA
<213>  Homo sapiens

<400>  13
uauugcacau uacuaaguug c                                               21

<210>  14

<211>  21
<212>  RNA
<213>  Homo sapiens

<400>  14
uucaaguaau ccaggauagg c                                                21


<210>  15
<211>  22
<212>  RNA
<213>  Homo sapiens

<400>  15
uucaaguaau ucaggauagg uu                                               22


<210>  16
<211>  21
<212>  RNA
<213>  Homo sapiens

<400>  16
uucacagugg cuaaguucug c                                                21


<210>  17
<211>  22
<212>  RNA
<213>  Homo sapiens

<400>  17
aaggagcuca cagucuauug ag                                               22


<210>  18
<211>  21
<212>  RNA
<213>  Homo sapiens

<400>  18
uagcaccauc ugaaaucggu u                                                21


<210>  19
<211>  20
<212>  RNA
<213>  Homo sapiens

<400>  19
uagcaccauu ugaaaucggu                                                  20


<210>  20
<211>  22
<212>  RNA
<213>  Homo sapiens

<400>  20
uguaaacauc cuacacucag cu                                               22


<210>  21
<211>  23
<212>  RNA
<213>  Homo sapiens

<400>  21

uguaaacauc cuacacucuc agc                                                23

<210>  22
<211>  22
<212>  RNA
<213>  Homo sapiens

<400>  22
aaagugcugu ucgugcaggu ag                                                    22

<210>  23
<211>  23
<212>  RNA
<213>  Homo sapiens

<400>  23
uaagugcuuc cauguuugag ugu                                                   23

<210>  24
<211>  22
<212>  RNA
<213>  Homo sapiens

<400>  24
uucaacgggu auuuauugag ca                                                    22

<210>  25
<211>  22
<212>  RNA
<213>  Homo sapiens

<400>  25
ugagguagua aguuguauug uu                                                    22

<210>  26
<211>  22
<212>  RNA
<213>  Homo sapiens

<400>  26
cagugcaaug uuaaaagggc au                                                    22

<210>  27
<211>  22
<212>  RNA
<213>  Homo sapiens

<400>  27
cagugcaaug augaaagggc au                                                    22

<210>  28
<211>  23
<212>  RNA
<213>  Homo sapiens

<400>  28
cagugcaaua guauugucaa agc                                                   23

<210>  29
<211>  22

<212>  RNA
<213>  Homo sapiens

<400>  29
uaacagucua cagccauggu cg                                              22


<210>  30
<211>  21
<212>  RNA
<213>  Homo sapiens

<400>  30
uaacagucuc cagucacggc c                                               21


<210>  31
<211>  22
<212>  RNA
<213>  Homo sapiens

<400>  31
uugguccccu ucaaccagcu gu                                              22


<210>  32
<211>  21
<212>  RNA
<213>  Homo sapiens

<400>  32
uugguccccu ucaaccagcu a                                               21


<210>  33
<211>  21
<212>  RNA
<213>  Homo sapiens

<400>  33
ugugacuggu ugaccagagg g                                               21


<210>  34
<211>  22
<212>  RNA
<213>  Homo sapiens

<400>  34
uauugcuuaa gaauacgcgu ag                                              22


<210>  35
<211>  23
<212>  RNA
<213>  Homo sapiens

<400>  35
uguaguguuu ccuacuuuau gga                                             23


<210>  36
<211>  22
<212>  RNA
<213>  Homo sapiens

<400>  36
ugagaacuga auuccauggg uu                                              22

<210>  37
<211>  20
<212>  RNA
<213>  Homo sapiens

<400>  37
guguguggaa augcuucugc                                                    20


<210>  38
<211>  22
<212>  RNA
<213>  Homo sapiens

<400>  38
ucuggcuccg ugucuucacu cc                                                 22


<210>  39
<211>  22
<212>  RNA
<213>  Homo sapiens

<400>  39
aacauucaac cugucgguga gu                                                 22


<210>  40
<211>  24
<212>  RNA
<213>  Homo sapiens

<400>  40
aacauucauu guugucggug gguu                                               24


<210>  41
<211>  21
<212>  RNA
<213>  Homo sapiens

<400>  41
ugguucuaga cuugccaacu a                                                  21


<210>  42
<211>  23
<212>  RNA
<213>  Homo sapiens

<400>  42
uauggcacug guagaauuca cug                                                23


<210>  43
<211>  22
<212>  RNA
<213>  Homo sapiens

<400>  43
uggacggaga acugauaagg gu                                                 22


<210>  44
<211>  21
<212>  RNA

<213>  Homo sapiens

<400>  44
cugaccuaug aauugacagc c                                                21


<210>  45
<211>  21
<212>  RNA
<213>  Homo sapiens

<400>  45
augaccuaug aauugacaga c                                                21


<210>  46
<211>  21
<212>  RNA
<213>  Homo sapiens

<400>  46
aacuggccua caaaguccca g                                                21


<210>  47
<211>  24
<212>  RNA
<213>  Homo sapiens

<400>  47
aacuggcccu caaagucccg cuuu                                             24


<210>  48
<211>  21
<212>  RNA
<213>  Homo sapiens

<400>  48
uagcagcaca gaaauauugg c                                                21


<210>  49
<211>  22
<212>  RNA
<213>  Homo sapiens

<400>  49
cagcagcaau ucauguuuug aa                                               22


<210>  50
<211>  21
<212>  RNA
<213>  Homo sapiens

<400>  50
cagcagcaca cugugguuug u                                                21


<210>  51
<211>  21
<212>  RNA
<213>  Homo sapiens

<400>  51
uagguaguuu cauguuguug g                                                21

<210>  52
<211>  21
<212>  RNA
<213>  Homo sapiens

<400>  52
uagguaguuu ccuguuguug g                                                21


<210>  53
<211>  22
<212>  RNA
<213>  Homo sapiens

<400>  53
uucaccaccu ucuccaccca gc                                               22


<210>  54
<211>  22
<212>  RNA
<213>  Homo sapiens

<400>  54
uacaguaguc ugcacauugg uu                                               22


<210>  55
<211>  22
<212>  RNA
<213>  Homo sapiens

<400>  55
uaacacuguc ugguaacgau gu                                               22


<210>  56
<211>  22
<212>  RNA
<213>  Homo sapiens

<400>  56
agagguauag ggcaugggaa aa                                               22


<210>  57
<211>  22
<212>  RNA
<213>  Homo sapiens

<400>  57
uucccuuugu cauccuaugc cu                                               22


<210>  58
<211>  22
<212>  RNA
<213>  Homo sapiens

<400>  58
uucccuuugu cauccuucgc cu                                               22


<210>  59
<211>  22
<212>  RNA
<213>  Homo sapiens

<400>  59
uccuucauuc caccggaguc ug                                                22

<210>  60
<211>  22
<212>  RNA
<213>  Homo sapiens

<400>  60
auaagacgag caaaaagcuu gu                                                22

<210>  61
<211>  24
<212>  RNA
<213>  Homo sapiens

<400>  61
agcuacaucu ggcuacuggg ucuc                                              24

<210>  62
<211>  22
<212>  RNA
<213>  Homo sapiens

<400>  62
uaugugggau gguaaaccgc uu                                                22

<210>  63
<211>  22
<212>  RNA
<213>  Homo sapiens

<400>  63
ugguuuaccg ucccacauac au                                                22

<210>  64
<211>  22
<212>  RNA
<213>  Homo sapiens

<400>  64
uaaacgugga uguacuugcu uu                                                22

<210>  65
<211>  23
<212>  RNA
<213>  Homo sapiens

<400>  65
acuuuaacau ggaagugcuu ucu                                               23

<210>  66
<211>  22
<212>  RNA
<213>  Homo sapiens

<400>  66
ccacugcccc aggugcugcu gg                                                22

<210>  67
<211>  23
<212>  RNA
<213>  Homo sapiens

<400>  67
   cgcauccccu agggcauugg ugu                                              23

<210>  68
<211>  22
<212>  RNA
<213>  Homo sapiens

<400>  68
aacacaccug guuaaccucu uu                                              22

<210>  69
<211>  21
<212>  RNA
<213>  Homo sapiens

<400>  69
gccccugggc cuauccuaga a                                               21

<210>  70
<211>  23
<212>  RNA
<213>  Homo sapiens

<400>  70
uccagcuccu auaugaugcc uuu                                             23

<210>  71
<211>  23
<212>  RNA
<213>  Homo sapiens

<400>  71
uccgucucag uuacuuuaua gcc                                             23

<210>  72
<211>  24
<212>  RNA
<213>  Homo sapiens

<400>  72
ucucacacag aaaucgcacc cguc                                            24

<210>  73
<211>  23
<212>  RNA
<213>  Homo sapiens

<400>  73
ugucugcccg caugccugcc ucu                                             23

<210>  74
<211>  24
<212>  RNA
<213>  Homo sapiens

<400>  74
aauccuugga accuaggugu gagu                                            24

<210>  75
<211>  22
<212>  RNA
<213>  Homo sapiens

<400>  75
acauagagga aauuccacgu uu                                              22

<210>  76
<211>  21
<212>  RNA
<213>  Homo sapiens

<400>  76
gugccgccau cuuuugagug u                                               21

<210>  77
<211>  21
<212>  RNA
<213>  Homo sapiens

<400>  77
aucauagagg aaaauccacg u                                               21

<210>  78
<211>  22
<212>  RNA
<213>  Homo sapiens

<400>  78
aucauagagg aaaauccaug uu                                              22

<210>  79
<211>  22
<212>  RNA
<213>  Homo sapiens

<400>  79
aucacacaaa ggcaacuuuu gu                                              22

<210>  80
<211>  22
<212>  RNA
<213>  Homo sapiens

<400>  80
cuccugacuc cagguccugu gu                                              22

<210>  81
<211>  22
<212>  RNA
<213>  Homo sapiens

<400>  81
ugguugacca uagaacaugc gc                                              22

<210>  82

<211>  22
<212>  RNA
<213>  Homo sapiens

<400>  82
gaaguuguuc gugguggauu cg                                                22

<210>  83
<211>  23
<212>  RNA
<213>  Homo sapiens

<400>  83
acuucaccug guccacuagc cgu                                               23

<210>  84
<211>  22
<212>  RNA
<213>  Homo sapiens

<400>  84
cuggacuuag ggucagaagg cc                                                22

<210>  85
<211>  22
<212>  RNA
<213>  Homo sapiens

<400>  85
cuggacuugg agucagaagg cc                                                22

<210>  86
<211>  22
<212>  RNA
<213>  Homo sapiens

<400>  86
agcucggucu gaggccccuc ag                                                22

<210>  87
<211>  21
<212>  RNA
<213>  Homo sapiens

<400>  87
ugucuugcag gccgucaugc a                                                 21

<210>  88
<211>  21
<212>  RNA
<213>  Homo sapiens

<400>  88
cuggauggcu ccuccauguc u                                                 21

<210>  89
<211>  22
<212>  RNA
<213>  Homo sapiens

<400>  89

aucaugaugg gcuccucggu gu                                                22

<210>  90
<211>  22
<212>  RNA
<213>  Homo sapiens

<400>  90
uggcagugua uuguuagcug gu                                                22

<210>  91
<211>  23
<212>  RNA
<213>  Homo sapiens

<400>  91
aaaccguuac cauuacugag uuu                                               23

<210>  92
<211>  22
<212>  RNA
<213>  Homo sapiens

<400>  92
uguuugcaga ggaaacugag ac                                                22

<210>  93
<211>  21
<212>  RNA
<213>  Homo sapiens

<400>  93
ucagucucau cugcaaagaa g                                                 21

<210>  94
<211>  22
<212>  RNA
<213>  Homo sapiens

<400>  94
uaugugccuu uggacuacau cg                                                22

<210>  95
<211>  23
<212>  RNA
<213>  Homo sapiens

<400>  95
ucacuccucu ccucccgucu ucu                                               23

<210>  96
<211>  22
<212>  RNA
<213>  Homo sapiens

<400>  96
agaggcuggc cgugaugaau uc                                                22

<210>  97
<211>  21

<212>  RNA
<213>  Homo sapiens

<400>  97
cccagauaau ggcacucuca a                                                21


<210>  98
<211>  23
<212>  RNA
<213>  Homo sapiens

<400>  98
agugacauca cauauacggc agc                                              23


<210>  99
<211>  23
<212>  RNA
<213>  Homo sapiens

<400>  99
aguggggaac ccuuccauga gga                                              23


<210>  100
<211>  22
<212>  RNA
<213>  Homo sapiens

<400>  100
uuguacaugg uaggcuuuca uu                                               22


<210>  101
<211>  24
<212>  RNA
<213>  Homo sapiens

<400>  101
ugaaacauac acgggaaacc ucuu                                             24


<210>  102
<211>  22
<212>  RNA
<213>  Homo sapiens

<400>  102
aauccuuugu cccuggguga ga                                               22


<210>  103
<211>  21
<212>  RNA
<213>  Homo sapiens

<400>  103
agacccuggu cugcacucua u                                                21


<210>  104
<211>  22
<212>  RNA
<213>  Homo sapiens

<400>  104
gucaacacuu gcugguuucc uc                                               22

<210>  105
<211>  21
<212>  RNA
<213>  Homo sapiens

<400>  105
uaaggcaccc uucugaguag a                                                21


<210>  106
<211>  21
<212>  RNA
<213>  Homo sapiens

<400>  106
uuuugcaccu uuuggaguga a                                                21


<210>  107
<211>  23
<212>  RNA
<213>  Homo sapiens

<400>  107
ugauugguac gucugugggu aga                                              23


<210>  108
<211>  23
<212>  RNA
<213>  Homo sapiens

<400>  108
uacucaggag aguggcaauc aca                                              23


<210>  109
<211>  21
<212>  RNA
<213>  Homo sapiens

<400>  109
gugucuuuug cucugcaguc a                                                21


<210>  110
<211>  22
<212>  RNA
<213>  Homo sapiens

<400>  110
aagugcuguc auagcugagg uc                                               22


<210>  111
<211>  23
<212>  RNA
<213>  Homo sapiens

<400>  111
cacucagccu ugagggcacu uuc                                              23


<210>  112
<211>  22
<212>  RNA

<213>  Homo sapiens

<400>  112
uucacaggga ggugucauuu au                                              22


<210>  113
<211>  23
<212>  RNA
<213>  Homo sapiens

<400>  113
caucuggagg uaagaagcac uuu                                             23


<210>  114
<211>  22
<212>  RNA
<213>  Homo sapiens

<400>  114
ccucuagaug gaagcacugu cu                                              22


<210>  115
<211>  23
<212>  RNA
<213>  Homo sapiens

<400>  115
aucgugcauc ccuuuagagu guu                                             23


<210>  116
<211>  22
<212>  RNA
<213>  Homo sapiens

<400>  116
aucgugcauc cuuuuagagu gu                                              22


<210>  117
<211>  22
<212>  RNA
<213>  Homo sapiens

<400>  117
ucgugcaucc cuuuagagug uu                                              22


<210>  118
<211>  22
<212>  RNA
<213>  Homo sapiens

<400>  118
caaagcgcuc cccuuuagag gu                                              22


<210>  119
<211>  22
<212>  RNA
<213>  Homo sapiens

<400>  119
caaagcgcuu cucuuuagag ug                                              22

<210>  120
<211>  21
<212>  RNA
<213>  Homo sapiens

<400>  120
caaagcgcuu cccuuuggag c                                                21


<210>  121
<211>  23
<212>  RNA
<213>  Homo sapiens

<400>  121
ucucuggagg gaagcacuuu cug                                              23


<210>  122
<211>  22
<212>  RNA
<213>  Homo sapiens

<400>  122
aaagcgcuuc ccuucagagu gu                                               22


<210>  123
<211>  21
<212>  RNA
<213>  Homo sapiens

<400>  123
aaagcgcuuc ucuuuagagg a                                                21


<210>  124
<211>  22
<212>  RNA
<213>  Homo sapiens

<400>  124
acaaagugcu ucccuuuaga gu                                               22


<210>  125
<211>  21
<212>  RNA
<213>  Homo sapiens

<400>  125
aacgcgcuuc ccuauagagg g                                                21


<210>  126
<211>  22
<212>  RNA
<213>  Homo sapiens

<400>  126
cuacaaaggg aagcacuuuc uc                                               22


<210>  127
<211>  21
<212>  RNA
<213>  Homo sapiens

<400>  127
cuccagaggg augcacuuuc u                                                21

<210>  128
<211>  21
<212>  RNA
<213>  Homo sapiens

<400>  128
cucuagaggg aagcacuuuc u                                                21

<210>  129
<211>  21
<212>  RNA
<213>  Homo sapiens

<400>  129
cugcaaaggg aagcccuuuc u                                                21

<210>  130
<211>  22
<212>  RNA
<213>  Mus musculus

<400>  130
uggaauguaa agaaguaugu au                                               22

<210>  131
<211>  22
<212>  RNA
<213>  Rattus norvegicus

<400>  131
uggaauguaa agaagugugu au                                               22

<210>  132
<211>  21
<212>  RNA
<213>  Pan paniscus

<400>  132
uggaauguaa agaaguaugu a                                                21

<210>  133
<211>  21
<212>  RNA
<213>  Monodelphis domestica

<400>  133
uggaauguaa agaaguaugu a                                                21

<210>  134
<211>  21
<212>  RNA
<213>  Gallus gallus

<400>  134
uggaauguaa agaaguaugu a                                                21

<210>  135
<211>  21
<212>  RNA
<213>  Gallus gallus

<400>  135
uggaauguua agaaguaugu a                                                21


<210>  136
<211>  21
<212>  RNA
<213>  Xenopus tropicalis

<400>  136
uggaauguua agaaguaugu a                                                21


<210>  137
<211>  22
<212>  RNA
<213>  Danio rerio

<400>  137
uggaauguaa agaaguaugu au                                               22


<210>  138
<211>  22
<212>  RNA
<213>  Fugu rubripes

<400>  138
uggaauguaa agaaguaugu au                                               22


<210>  139
<211>  22
<212>  RNA
<213>  Tetraodon nigroviridis

<400>  139
uggaauguaa agaaguaugu au                                               22


<210>  140
<211>  22
<212>  RNA
<213>  Mus musculus

<400>  140
uggaauguaa ggaagugugu gg                                               22


<210>  141
<211>  22
<212>  RNA
<213>  Rattus norvegicus

<400>  141
uggaauguaa ggaagugugu gg                                               22


<210>  142
<211>  22
<212>  RNA
<213>  Macaca nemestrina

<400>  142
uggaauguaa ggaagugugu gg                                              22

<210>  143
<211>  22
<212>  RNA
<213>  Pongo pygmaeus

<400>  143
uggaauguaa ggaagugugu gg                                              22

<210>  144
<211>  21
<212>  RNA
<213>  Monodelphis domestica

<400>  144
uggaauguaa ggaagugugu g                                               21

<210>  145
<211>  22
<212>  RNA
<213>  Gallus gallus

<400>  145
uggaauguaa ggaagugugu gg                                              22

<210>  146
<211>  22
<212>  RNA
<213>  Xenopus tropicalis

<400>  146
uggaauguaa ggaagugugu gg                                              22

<210>  147
<211>  22
<212>  RNA
<213>  Danio rerio

<400>  147
uggaauguaa ggaagugugu gg                                              22

<210>  148
<211>  23
<212>  RNA
<213>  Mus musculus

<400>  148
uggaagacuu gugauuuugu ugu                                             23

<210>  149
<211>  23
<212>  RNA
<213>  Rattus norvegicus

<400>  149
uggaagacua gugauuuugu ugu                                             23

<210>  150

<211>  23
<212>  RNA
<213>  Rattus norvegicus

<400>  150
uggaagacuu gugauuuugu ugu                                              23


<210>  151
<211>  21
<212>  RNA
<213>  Lagothrix lagotricha

<400>  151
uggaagacua gugauuuugu u                                                21


<210>  152
<211>  21
<212>  RNA
<213>  Saguinus labiatus

<400>  152
uggaagacua gugauuuugu u                                                21


<210>  153
<211>  21
<212>  RNA
<213>  Macaca nemestrina

<400>  153
uggaagacua gugauuuugu u                                                21


<210>  154
<211>  21
<212>  RNA
<213>  Gorilla gorilla

<400>  154
uggaagacua gugauuuugu u                                                21


<210>  155
<211>  21
<212>  RNA
<213>  Pan paniscus

<400>  155
uggaagacua gugauuuugu u                                                21


<210>  156
<211>  21
<212>  RNA
<213>  Pan troglodytes

<400>  156
uggaagacua gugauuuugu u                                                21


<210>  157
<211>  21
<212>  RNA
<213>  Pongo pygmaeus

<400>  157

uggaagacua gugauuuugu u                                                21

<210>  158
<211>  22
<212>  RNA
<213>  Monodelphis domestica

<400>  158
uggaagacua gugauuuugu ug                                               22

<210>  159
<211>  22
<212>  RNA
<213>  Gallus gallus

<400>  159
uggaagacua gugauuuugu ug                                               22

<210>  160
<211>  22
<212>  RNA
<213>  Gallus gallus

<400>  160
uggaagacua gugauuuuug uu                                               22

<210>  161
<211>  22
<212>  RNA
<213>  Xenopus tropicalis

<400>  161
uggaagacua gugauuuugu ug                                               22

<210>  162
<211>  24
<212>  RNA
<213>  Bos taurus

<400>  162
uggaagacua gugauuuugu uguu                                             24

<210>  163
<211>  21
<212>  RNA
<213>  Sus scrofa

<400>  163
uggaagacua gugauuuugu u                                                21

<210>  164
<211>  23
<212>  RNA
<213>  Danio rerio

<400>  164
uggaagacua gugauuuugu ugu                                              23

<210>  165
<211>  21

<212>  RNA
<213>  Danio rerio

<400>  165
uggaagacuu gugauuuugu u                                                21


<210>  166
<211>  21
<212>  RNA
<213>  Fugu rubripes

<400>  166
uggaagacua gugauuuugu u                                                21


<210>  167
<211>  23
<212>  RNA
<213>  Tetraodon nigroviridis

<400>  167
uggaagacua gugauuuugu ugu                                              23


<210>  168
<211>  23
<212>  RNA
<213>  Mus musculus

<400>  168
uacccuguag auccgaauuu gug                                              23


<210>  169
<211>  23
<212>  RNA
<213>  Ateles geoffroyi

<400>  169
uacccuguag auccgaauuu gug                                              23


<210>  170
<211>  23
<212>  RNA
<213>  Saguinus labiatus

<400>  170
uacccuguag auccgaauuu gug                                              23


<210>  171
<211>  22
<212>  RNA
<213>  Macaca nemestrina

<400>  171
uacccuguag aaccgaauuu gu                                               22


<210>  172
<211>  23
<212>  RNA
<213>  Gorilla gorilla

<400>  172
uacccuguag auccgaauuu gug                                              23

<210>  173
<211>  22
<212>  RNA
<213>  Gorilla gorilla

<400>  173
uacccuguag aaccgaauuu gu                                                22


<210>  174
<211>  23
<212>  RNA
<213>  Pan paniscus

<400>  174
uacccuguag auccgaauuu gug                                               23


<210>  175
<211>  22
<212>  RNA
<213>  Pan paniscus

<400>  175
uacccuguag aaccgaauuu gu                                                22


<210>  176
<211>  23
<212>  RNA
<213>  Monodelphis domestica

<400>  176
uacccuguag auccgaauuu gug                                               23


<210>  177
<211>  22
<212>  RNA
<213>  Gallus gallus

<400>  177
uacccuguag aaccgaauuu gu                                                22


<210>  178
<211>  23
<212>  RNA
<213>  Xenopus tropicalis

<400>  178
uacccuguag auccgaauuu gug                                               23


<210>  179
<211>  22
<212>  RNA
<213>  Xenopus tropicalis

<400>  179
uacccuguag aaccgaauuu gu                                                22


<210>  180
<211>  22
<212>  RNA

<213>  Xenopus tropicalis

<400>  180
cacccuguag aaucgaauuu gu                                                22

<210>  181
<211>  23
<212>  RNA
<213>  Bos taurus

<400>  181
uacccuguag auccgaauuu gug                                               23

<210>  182
<211>  23
<212>  RNA
<213>  Bos taurus

<400>  182
uacccuguag aaccgaauuu gug                                               23

<210>  183
<211>  22
<212>  RNA
<213>  Danio rerio

<400>  183
uacccuguag auccgaauuu gu                                                22

<210>  184
<211>  23
<212>  RNA
<213>  Danio rerio

<400>  184
uacccuguag aaccgaauuu gug                                               23

<210>  185
<211>  22
<212>  RNA
<213>  Danio rerio

<400>  185
uacccuguag auccggauuu gu                                                22

<210>  186
<211>  23
<212>  RNA
<213>  Danio rerio

<400>  186
uacccuguag aaccgaaugu gug                                               23

<210>  187
<211>  23
<212>  RNA
<213>  Fugu rubripes

<400>  187
uacccuguag aaccgaauuu gug                                               23

<210>  188
<211>  22
<212>  RNA
<213>  Fugu rubripes

<400>  188
uacccuguag auccggauuu gu                                                22

<210>  189
<211>  25
<212>  RNA
<213>  Fugu rubripes

<400>  189
uacccuguag aaccgaaugu gugug                                             25

<210>  190
<211>  23
<212>  RNA
<213>  Tetraodon nigroviridis

<400>  190
uacccuguag aaccgaauuu gug                                               23

<210>  191
<211>  22
<212>  RNA
<213>  Tetraodon nigroviridis

<400>  191
uacccuguag auccggauuu gu                                                22

<210>  192
<211>  25
<212>  RNA
<213>  Tetraodon nigroviridis

<400>  192
uacccuguag aaccgaaugu gugug                                             25

<210>  193
<211>  23
<212>  RNA
<213>  Mus musculus

<400>  193
uaaggugcau cuagugcaga uag                                               23

<210>  194
<211>  23
<212>  RNA
<213>  Rattus norvegicus

<400>  194
uaaggugcau cuagugcaga uag                                               23

<210>  195
<211>  22
<212>  RNA
<213>  Ateles geoffroyi

<400>  195
uaaggugcau cuagugcaga ua                                              22

<210>  196
<211>  22
<212>  RNA
<213>  Lagothrix lagotricha

<400>  196
uaaggugcau cuagugcaga ua                                              22

<210>  197
<211>  22
<212>  RNA
<213>  Saguinus labiatus

<400>  197
uaaggugcau cuagugcaga ua                                              22

<210>  198
<211>  22
<212>  RNA
<213>  Macaca mulatta

<400>  198
uaaggugcau cuagugcaga ua                                              22

<210>  199
<211>  22
<212>  RNA
<213>  Macaca nemestrina

<400>  199
uaaggugcau cuagugcaga ua                                              22

<210>  200
<211>  22
<212>  RNA
<213>  Gorilla gorilla

<400>  200
uaaggugcau cuagugcaga ua                                              22

<210>  201
<211>  22
<212>  RNA
<213>  Pan paniscus

<400>  201
uaaggugcau cuagugcaga ua                                              22

<210>  202
<211>  22
<212>  RNA
<213>  Pan troglodytes

<400>  202
uaaggugcau cuagugcaga ua                                              22

<210>  203
<211>  22
<212>  RNA
<213>  Pongo pygmaeus

<400>  203
uaaggugcau cuagugcaga ua                                                22


<210>  204
<211>  22
<212>  RNA
<213>  Lemur catta

<400>  204
uaaggugcau cuagugcaga ua                                                22


<210>  205
<211>  22
<212>  RNA
<213>  Monodelphis domestica

<400>  205
uaaggugcau cuagugcaga ua                                                22


<210>  206
<211>  22
<212>  RNA
<213>  Gallus gallus

<400>  206
uaaggugcau cuagugcaga ua                                                22


<210>  207
<211>  22
<212>  RNA
<213>  Xenopus tropicalis

<400>  207
uaaggugcau cuagugcaga ua                                                22


<210>  208
<211>  22
<212>  RNA
<213>  Bos taurus

<400>  208
uaaggugcau cuagugcaga ua                                                22


<210>  209
<211>  22
<212>  RNA
<213>  Sus scrofa

<400>  209
uaaggugcau cuagugcaga ua                                                22


<210>  210
<211>  22
<212>  RNA
<213>  Danio rerio

<400>  210
uaaggugcau cuagugcaga ua                                              22


<210>  211
<211>  22
<212>  RNA
<213>  Danio rerio

<400>  211
uaaggugcau cuuguguagu ua                                              22


<210>  212
<211>  22
<212>  RNA
<213>  Fugu rubripes

<400>  212
uaaggugcau cuagugcaga ua                                              22


<210>  213
<211>  22
<212>  RNA
<213>  Tetraodon nigroviridis

<400>  213
uaaggugcau cuagugcaga ua                                              22


<210>  214
<211>  23
<212>  RNA
<213>  Mus musculus

<400>  214
uaaggugcau cuagugcugu uag                                             23


<210>  215
<211>  22
<212>  RNA
<213>  Gallus gallus

<400>  215
uaaggugcau cuagugcagu ua                                              22


<210>  216
<211>  23
<212>  RNA
<213>  Xenopus tropicalis

<400>  216
uaaggugcau cuagugcagu uag                                             23


<210>  217
<211>  22
<212>  RNA
<213>  Bos taurus

<400>  217
uaaggugcau cuagugcagu ua                                              22


<210>  218

<211>  22
<212>  RNA
<213>  Danio rerio

<400>  218
uaaggugcau uuagugcaga ua                                                22


<210>  219
<211>  22
<212>  RNA
<213>  Mus musculus

<400>  219
acugcccuaa gugcuccuuc ug                                                22


<210>  220
<211>  23
<212>  RNA
<213>  Mus musculus

<400>  220
caaagugcuc auagugcagg uag                                               23


<210>  221
<211>  22
<212>  RNA
<213>  Ateles geoffroyi

<400>  221
uaaagugcuu auagugcagg ua                                                22


<210>  222
<211>  22
<212>  RNA
<213>  Lagothrix lagotricha

<400>  222
uaaagugcuu auagugcagg ua                                                22


<210>  223
<211>  22
<212>  RNA
<213>  Saguinus labiatus

<400>  223
uaaagugcuu auagugcagg ua                                                22


<210>  224
<211>  22
<212>  RNA
<213>  Macaca mulatta

<400>  224
uaaagugcuu auagugcagg ua                                                22


<210>  225
<211>  22
<212>  RNA
<213>  Macaca nemestrina

<400>  225

uaaagugcuu auagugcagg ua                                                22

<210>  226
<211>  22
<212>  RNA
<213>  Gorilla gorilla

<400>  226
uaaagugcuu auagugcagg ua                                                22

<210>  227
<211>  22
<212>  RNA
<213>  Pan paniscus

<400>  227
uaaagugcuu auagugcagg ua                                                22

<210>  228
<211>  22
<212>  RNA
<213>  Pan troglodytes

<400>  228
uaaagugcuu auagugcagg ua                                                22

<210>  229
<211>  22
<212>  RNA
<213>  Pongo pygmaeus

<400>  229
uaaagugcuu auagugcagg ua                                                22

<210>  230
<211>  22
<212>  RNA
<213>  Lemur catta

<400>  230
uaaagugcuu auagugcagg ua                                                22

<210>  231
<211>  23
<212>  RNA
<213>  Monodelphis domestica

<400>  231
uaaagugcuu auagugcagg uag                                               23

<210>  232
<211>  23
<212>  RNA
<213>  Gallus gallus

<400>  232
caaagugcuc auagugcagg uag                                               23

<210>  233
<211>  23

<212>  RNA
<213>  Xenopus tropicalis

<400>  233
caaagugcuc auagugcagg uag                                              23


<210>  234
<211>  22
<212>  RNA
<213>  Bos taurus

<400>  234
caaagugcuc acagugcagg ua                                               22


<210>  235
<211>  22
<212>  RNA
<213>  Sus scrofa

<400>  235
uaaagugcuu auagugcagg ua                                               22


<210>  236
<211>  23
<212>  RNA
<213>  Danio rerio

<400>  236
caaagugcuc acagugcagg uag                                              23


<210>  237
<211>  23
<212>  RNA
<213>  Fugu rubripes

<400>  237
uaaagugcuu auagugcagg uag                                              23


<210>  238
<211>  23
<212>  RNA
<213>  Tetraodon nigroviridis

<400>  238
uaaagugcuu auagugcagg uag                                              23


<210>  239
<211>  23
<212>  RNA
<213>  Mus musculus

<400>  239
caaagugcua acagugcagg uag                                              23


<210>  240
<211>  24
<212>  RNA
<213>  Ateles geoffroyi

<400>  240
aaaagugcuu acagugcagg uagc                                             24

<210>  241
<211>  24
<212>  RNA
<213>  Saguinus labiatus

<400>  241
aaaagugcuu acagugcagg uagc                                              24


<210>  242
<211>  24
<212>  RNA
<213>  Macaca mulatta

<400>  242
aaaagugcuu acagugcagg uagc                                              24


<210>  243
<211>  24
<212>  RNA
<213>  Macaca nemestrina

<400>  243
aaaagugcuu acagugcagg uagc                                              24


<210>  244
<211>  24
<212>  RNA
<213>  Gorilla gorilla

<400>  244
aaaagugcuu acagugcagg uagc                                              24


<210>  245
<211>  24
<212>  RNA
<213>  Pan paniscus

<400>  245
aaaagugcuu acagugcagg uagc                                              24


<210>  246
<211>  24
<212>  RNA
<213>  Pan troglodytes

<400>  246
aaaagugcuu acagugcagg uagc                                              24


<210>  247
<211>  24
<212>  RNA
<213>  Pongo pygmaeus

<400>  247
aaaagugcuu acagugcagg uagc                                              24


<210>  248
<211>  22
<212>  RNA

<213>  Gallus gallus

<400>  248
aaaagugcuu acagugcagg ua                                                22


<210>  249
<211>  24
<212>  RNA
<213>  Xenopus tropicalis

<400>  249
aaaagugcuu auagugcagg uaga                                              24


<210>  250
<211>  22
<212>  RNA
<213>  Bos taurus

<400>  250
aaaagugcuu acagugcagg ua                                                22


<210>  251
<211>  24
<212>  RNA
<213>  Sus scrofa

<400>  251
aaaagugcuu acagugcagg uagc                                              24


<210>  252
<211>  21
<212>  RNA
<213>  Mus musculus

<400>  252
uaaagugcug acagugcaga u                                                 21


<210>  253
<211>  21
<212>  RNA
<213>  Rattus norvegicus

<400>  253
uaaagugcug acagugcaga u                                                 21


<210>  254
<211>  21
<212>  RNA
<213>  Ateles geoffroyi

<400>  254
uaaagugcug acagugcaga u                                                 21


<210>  255
<211>  21
<212>  RNA
<213>  Lagothrix lagotricha

<400>  255
uaaagugcug acagugcaga u                                                 21

<210>  256
<211>  21
<212>  RNA
<213>  Saguinus labiatus

<400>  256
uaaagugcug acagugcaga u                                                21

<210>  257
<211>  21
<212>  RNA
<213>  Macaca mulatta

<400>  257
uaaagugcug acagugcaga u                                                21

<210>  258
<211>  21
<212>  RNA
<213>  Macaca nemestrina

<400>  258
uaaagugcug acagugcaga u                                                21

<210>  259
<211>  21
<212>  RNA
<213>  Gorilla gorilla

<400>  259
uaaagugcug acagugcaga u                                                21

<210>  260
<211>  21
<212>  RNA
<213>  Pan paniscus

<400>  260
uaaagugcug acagugcaga u                                                21

<210>  261
<211>  21
<212>  RNA
<213>  Pan troglodytes

<400>  261
uaaagugcug acagugcaga u                                                21

<210>  262
<211>  21
<212>  RNA
<213>  Pongo pygmaeus

<400>  262
uaaagugcug acagugcaga u                                                21

<210>  263
<211>  22
<212>  RNA
<213>  Mus musculus

<400>  263
uggcucaguu cagcaggaac ag                                                22

<210>  264
<211>  22
<212>  RNA
<213>  Rattus norvegicus

<400>  264
uggcucaguu cagcaggaac ag                                                22

<210>  265
<211>  22
<212>  RNA
<213>  Macaca mulatta

<400>  265
uggcucaguu cagcaggaac ag                                                22

<210>  266
<211>  22
<212>  RNA
<213>  Macaca nemestrina

<400>  266
uggcucaguu cagcaggaac ag                                                22

<210>  267
<211>  22
<212>  RNA
<213>  Gorilla gorilla

<400>  267
uggcucaguu cagcaggaac ag                                                22

<210>  268
<211>  22
<212>  RNA
<213>  Pan paniscus

<400>  268
uggcucaguu cagcaggaac ag                                                22

<210>  269
<211>  22
<212>  RNA
<213>  Pan troglodytes

<400>  269
uggcucaguu cagcaggaac ag                                                22

<210>  270
<211>  22
<212>  RNA
<213>  Pongo pygmaeus

<400>  270
uggcucaguu cagcaggaac ag                                                22

<210>  271
<211>  16
<212>  RNA
<213>  Monodelphis domestica

<400>  271
uggcucaguu cagcag                                                    16


<210>  272
<211>  22
<212>  RNA
<213>  Gallus gallus

<400>  272
uggcucaguu cagcaggaac ag                                             22


<210>  273
<211>  21
<212>  RNA
<213>  Xenopus tropicalis

<400>  273
uggcucaguu cagcaggaca g                                              21


<210>  274
<211>  22
<212>  RNA
<213>  Bos taurus

<400>  274
uggcucaguu cagcaggaac ag                                             22


<210>  275
<211>  22
<212>  RNA
<213>  Sus scrofa

<400>  275
uggcucaguu cagcaggaac ag                                             22


<210>  276
<211>  22
<212>  RNA
<213>  Danio rerio

<400>  276
uggcucaguu cagcaggaac ag                                             22


<210>  277
<211>  22
<212>  RNA
<213>  Fugu rubripes

<400>  277
uggcucaguu cagcaggaac ag                                             22


<210>  278
<211>  22
<212>  RNA
<213>  Tetraodon nigroviridis

<400>  278
uggcucaguu cagcaggaac ag                                              22


<210>  279
<211>  22
<212>  RNA
<213>  Mus musculus

<400>  279
cauugcacuu gucucggucu ga                                              22


<210>  280
<211>  22
<212>  RNA
<213>  Rattus norvegicus

<400>  280
cauugcacuu gucucggucu ga                                              22


<210>  281
<211>  22
<212>  RNA
<213>  Lagothrix lagotricha

<400>  281
cauugcacuu gucucggucu ga                                              22


<210>  282
<211>  22
<212>  RNA
<213>  Macaca mulatta

<400>  282
cauugcacuu gucucggucu ga                                              22


<210>  283
<211>  22
<212>  RNA
<213>  Macaca nemestrina

<400>  283
cauugcacuu gucucggucu ga                                              22


<210>  284
<211>  22
<212>  RNA
<213>  Gorilla gorilla

<400>  284
cauugcacuu gucucggucu ga                                              22


<210>  285
<211>  22
<212>  RNA
<213>  Pan paniscus

<400>  285
cauugcacuu gucucggucu ga                                              22


<210>  286

<211>  21
<212>  RNA
<213>  Pan troglodytes

<400>  286
cauugcacuu gucucggcug a                                                21


<210>  287
<211>  22
<212>  RNA
<213>  Pongo pygmaeus

<400>  287
cauugcacuu gucucggucu ga                                               22


<210>  288
<211>  22
<212>  RNA
<213>  Monodelphis domestica

<400>  288
cauugcacuu gucucggucu ga                                               22


<210>  289
<211>  22
<212>  RNA
<213>  Xenopus tropicalis

<400>  289
cauugcacuu gucucggucu ga                                               22


<210>  290
<211>  22
<212>  RNA
<213>  Bos taurus

<400>  290
cauugcacuu gucucggucu ga                                               22


<210>  291
<211>  22
<212>  RNA
<213>  Danio rerio

<400>  291
cauugcacuu gucucggucu ga                                               22


<210>  292
<211>  22
<212>  RNA
<213>  Fugu rubripes

<400>  292
cauugcacuu gucucggucu ga                                               22


<210>  293
<211>  22
<212>  RNA
<213>  Tetraodon nigroviridis

<400>  293

cauugcacuu gucucggucu ga                                                22

<210>  294
<211>  22
<212>  RNA
<213>  Mus musculus

<400>  294
uauugcacau uacuaaguug ca                                                22

<210>  295
<211>  22
<212>  RNA
<213>  Rattus norvegicus

<400>  295
uauugcacau uacuaaguug ca                                                22

<210>  296
<211>  21
<212>  RNA
<213>  Saguinus labiatus

<400>  296
uauugcacau uacuaaguug c                                                 21

<210>  297
<211>  21
<212>  RNA
<213>  Macaca mulatta

<400>  297
uauugcacau uacuaaguug c                                                 21

<210>  298
<211>  21
<212>  RNA
<213>  Macaca nemestrina

<400>  298
uauugcacau uacuaaguug c                                                 21

<210>  299
<211>  21
<212>  RNA
<213>  Gorilla gorilla

<400>  299
uauugcacau uacuaaguug c                                                 21

<210>  300
<211>  21
<212>  RNA
<213>  Pan paniscus

<400>  300
uauugcacau uacuaaguug c                                                 21

<210>  301
<211>  21

<212>  RNA
<213>  Pan troglodytes

<400>  301
uauugcacau uacuaaguug c                                                21

<210>  302
<211>  21
<212>  RNA
<213>  Pongo pygmaeus

<400>  302
uauugcacau uacuaaguug c                                                21

<210>  303
<211>  21
<212>  RNA
<213>  Monodelphis domestica

<400>  303
uauugcacau uacuaaguug c                                                21

<210>  304
<211>  21
<212>  RNA
<213>  Gallus gallus

<400>  304
uauugcacau uacuaaguug c                                                21

<210>  305
<211>  21
<212>  RNA
<213>  Sus scrofa

<400>  305
uauugcacau uacuaaguug c                                                21

<210>  306
<211>  22
<212>  RNA
<213>  Mus musculus

<400>  306
uucaaguaau ccaggauagg cu                                               22

<210>  307
<211>  22
<212>  RNA
<213>  Rattus norvegicus

<400>  307
uucaaguaau ccaggauagg cu                                               22

<210>  308
<211>  22
<212>  RNA
<213>  Lagothrix lagotricha

<400>  308
uucaaguaau ccaggauagg cu                                               22

<210>  309
<211>  22
<212>  RNA
<213>  Macaca mulatta

<400>  309
uucaaguaau ccaggauagg cu                                                22

<210>  310
<211>  22
<212>  RNA
<213>  Macaca nemestrina

<400>  310
uucaaguaau ccaggauagg cu                                                22

<210>  311
<211>  22
<212>  RNA
<213>  Gorilla gorilla

<400>  311
uucaaguaau ccaggauagg cu                                                22

<210>  312
<211>  22
<212>  RNA
<213>  Pan paniscus

<400>  312
uucaaguaau ccaggauagg cu                                                22

<210>  313
<211>  22
<212>  RNA
<213>  Pan troglodytes

<400>  313
uucaaguaau ccaggauagg cu                                                22

<210>  314
<211>  22
<212>  RNA
<213>  Pongo pygmaeus

<400>  314
uucaaguaau ccaggauagg cu                                                22

<210>  315
<211>  21
<212>  RNA
<213>  Gallus gallus

<400>  315
uucaaguaau ccaggauagg c                                                 21

<210>  316
<211>  22
<212>  RNA

<213>  Bos taurus

<400>  316
uucaaguaau ccaggauagg cu                                              22


<210>  317
<211>  22
<212>  RNA
<213>  Sus scrofa

<400>  317
uucaaguaau ccaggauagg cu                                              22


<210>  318
<211>  22
<212>  RNA
<213>  Danio rerio

<400>  318
uucaaguaau ccaggauagg cu                                              22


<210>  319
<211>  22
<212>  RNA
<213>  Fugu rubripes

<400>  319
uucaaguaau ccaggauagg cu                                              22


<210>  320
<211>  22
<212>  RNA
<213>  Tetraodon nigroviridis

<400>  320
uucaaguaau ccaggauagg cu                                              22


<210>  321
<211>  21
<212>  RNA
<213>  Mus musculus

<400>  321
uucaaguaau ucaggauagg u                                               21


<210>  322
<211>  21
<212>  RNA
<213>  Rattus norvegicus

<400>  322
uucaaguaau ucaggauagg u                                               21


<210>  323
<211>  22
<212>  RNA
<213>  Bos taurus

<400>  323
uucaaguaau ucaggauagg uu                                              22

<210>  324
<211>  22
<212>  RNA
<213>  Danio rerio

<400>  324
uucaaguaau ccaggauagg uu                                                22


<210>  325
<211>  21
<212>  RNA
<213>  Mus musculus

<400>  325
uucacagugg cuaaguucug c                                                 21


<210>  326
<211>  21
<212>  RNA
<213>  Rattus norvegicus

<400>  326
uucacagugg cuaaguuccg c                                                 21


<210>  327
<211>  21
<212>  RNA
<213>  Rattus norvegicus

<400>  327
uucacagugg cuaaguucug c                                                 21


<210>  328
<211>  22
<212>  RNA
<213>  Ateles geoffroyi

<400>  328
uucacagugg cuaaguuccg cc                                                22


<210>  329
<211>  22
<212>  RNA
<213>  Saguinus labiatus

<400>  329
uucacagugg cuaaguuccg cc                                                22


<210>  330
<211>  22
<212>  RNA
<213>  Macaca mulatta

<400>  330
uucacagugg cuaaguuccg cc                                                22


<210>  331
<211>  22
<212>  RNA
<213>  Macaca nemestrina

<400>  331
uucacagugg cuaaguuccg cc                                                22

<210>  332
<211>  22
<212>  RNA
<213>  Gorilla gorilla

<400>  332
uucacagugg cuaaguuccg cc                                                22

<210>  333
<211>  22
<212>  RNA
<213>  Pan paniscus

<400>  333
uucacagugg cuaaguuccg cc                                                22

<210>  334
<211>  22
<212>  RNA
<213>  Pan troglodytes

<400>  334
uucacagugg cuaaguuccg cc                                                22

<210>  335
<211>  22
<212>  RNA
<213>  Pongo pygmaeus

<400>  335
uucacagugg cuaaguuccg cc                                                22

<210>  336
<211>  22
<212>  RNA
<213>  Lemur catta

<400>  336
uucacagugg cuaaguuccg cc                                                22

<210>  337
<211>  21
<212>  RNA
<213>  Monodelphis domestica

<400>  337
uucacagugg cuaaguuccg c                                                 21

<210>  338
<211>  21
<212>  RNA
<213>  Monodelphis domestica

<400>  338
uucacagugg cuaaguucug c                                                 21

<210>  339
<211>  21
<212>  RNA
<213>  Gallus gallus

<400>  339
uucacagugg cuaaguucug c                                                21


<210>  340
<211>  21
<212>  RNA
<213>  Xenopus tropicalis

<400>  340
uucacagugg cuaaguuccg c                                                21


<210>  341
<211>  21
<212>  RNA
<213>  Xenopus tropicalis

<400>  341
uucacagugg cuaaguucug c                                                21


<210>  342
<211>  21
<212>  RNA
<213>  Xenopus tropicalis

<400>  342
uucacagugg cuaaguucca c                                                21


<210>  343
<211>  20
<212>  RNA
<213>  Bos taurus

<400>  343
uucacagugg cuaaguuccg                                                  20


<210>  344
<211>  21
<212>  RNA
<213>  Bos taurus

<400>  344
uucacagugg cuaaguucug c                                                21


<210>  345
<211>  21
<212>  RNA
<213>  Sus scrofa

<400>  345
uucacagugg cuaaguuccg c                                                21


<210>  346
<211>  22
<212>  RNA
<213>  Danio rerio

<400>  346
uucacagugg cuaaguuccg cu                                              22

<210>  347
<211>  22
<212>  RNA
<213>  Danio rerio

<400>  347
uucacagugg cuaaguucug ca                                              22

<210>  348
<211>  21
<212>  RNA
<213>  Danio rerio

<400>  348
uucacagugg uuaaguucug c                                               21

<210>  349
<211>  22
<212>  RNA
<213>  Danio rerio

<400>  349
uucacagugg cuaaguucuu ca                                              22

<210>  350
<211>  22
<212>  RNA
<213>  Danio rerio

<400>  350
uucacagugg cuaaguucag ug                                              22

<210>  351
<211>  22
<212>  RNA
<213>  Fugu rubripes

<400>  351
uucacagugg cuaaguucug ca                                              22

<210>  352
<211>  21
<212>  RNA
<213>  Fugu rubripes

<400>  352
uucacagugg uuaaguucug c                                               21

<210>  353
<211>  22
<212>  RNA
<213>  Fugu rubripes

<400>  353
uucacagugg cuaaguucag ug                                              22

<210>  354

<211>  22
<212>  RNA
<213>  Tetraodon nigroviridis

<400>  354
uucacagugg cuaaguucug ca                                              22


<210>  355
<211>  21
<212>  RNA
<213>  Tetraodon nigroviridis

<400>  355
uucacagugg uuaaguucug c                                               21


<210>  356
<211>  22
<212>  RNA
<213>  Tetraodon nigroviridis

<400>  356
uucacagugg cuaaguucag ug                                              22


<210>  357
<211>  22
<212>  RNA
<213>  Mus musculus

<400>  357
aaggagcuca cagucuauug ag                                              22


<210>  358
<211>  22
<212>  RNA
<213>  Rattus norvegicus

<400>  358
aaggagcuca cagucuauug ag                                              22


<210>  359
<211>  22
<212>  RNA
<213>  Ateles geoffroyi

<400>  359
aaggagcuca cagucuauug ag                                              22


<210>  360
<211>  22
<212>  RNA
<213>  Lagothrix lagotricha

<400>  360
aaggagcuca cagucuauug ag                                              22


<210>  361
<211>  22
<212>  RNA
<213>  Saguinus labiatus

<400>  361

aaggagcuca cagucuauug ag                                                22

<210>  362
<211>  22
<212>  RNA
<213>  Macaca mulatta

<400>  362
aaggagcuca cagucuauug ag                                                22

<210>  363
<211>  22
<212>  RNA
<213>  Macaca nemestrina

<400>  363
aaggagcuca cagucuauug ag                                                22

<210>  364
<211>  22
<212>  RNA
<213>  Gorilla gorilla

<400>  364
aaggagcuca cagucuauug ag                                                22

<210>  365
<211>  22
<212>  RNA
<213>  Pan paniscus

<400>  365
aaggagcuca cagucuauug ag                                                22

<210>  366
<211>  22
<212>  RNA
<213>  Pan troglodytes

<400>  366
aaggagcuca cagucuauug ag                                                22

<210>  367
<211>  22
<212>  RNA
<213>  Pongo pygmaeus

<400>  367
aaggagcuca cagucuauug ag                                                22

<210>  368
<211>  22
<212>  RNA
<213>  Sus scrofa

<400>  368
aaggagcuca cacucuauug ag                                                22

<210>  369
<211>  22

<212>  RNA
<213>  Mus musculus

<400>  369
uagcaccauc ugaaaucggu ua                                                22


<210>  370
<211>  22
<212>  RNA
<213>  Rattus norvegicus

<400>  370
uagcaccauc ugaaaucggu ua                                                22


<210>  371
<211>  23
<212>  RNA
<213>  Rattus norvegicus

<400>  371
uagcaccauu ugaaaucagu guu                                               23


<210>  372
<211>  20
<212>  RNA
<213>  Ateles geoffroyi

<400>  372
uagcaccauu ugaaaucagu                                                   20


<210>  373
<211>  20
<212>  RNA
<213>  Lagothrix lagotricha

<400>  373
uagcaccauu ugaaaucagu                                                   20


<210>  374
<211>  20
<212>  RNA
<213>  Saguinus labiatus

<400>  374
uagcaccauu ugaaaucagu                                                   20


<210>  375
<211>  20
<212>  RNA
<213>  Macaca nemestrina

<400>  375
uagcaccauu ugaaaucagu                                                   20


<210>  376
<211>  20
<212>  RNA
<213>  Gorilla gorilla

<400>  376
uagcaccauu ugaaaucagu                                                   20

<210>  377
<211>  20
<212>  RNA
<213>  Pan paniscus

<400>  377
uagcaccauu ugaaaucagu                                                    20


<210>  378
<211>  20
<212>  RNA
<213>  Pan troglodytes

<400>  378
uagcaccauu ugaaaucagu                                                    20


<210>  379
<211>  20
<212>  RNA
<213>  Pongo pygmaeus

<400>  379
uagcaccauu ugaaaucagu                                                    20


<210>  380
<211>  20
<212>  RNA
<213>  Monodelphis domestica

<400>  380
uagcaccauu ugaaaucggu                                                    20


<210>  381
<211>  20
<212>  RNA
<213>  Monodelphis domestica

<400>  381
uagcaccauu ugaaaucagu                                                    20


<210>  382
<211>  21
<212>  RNA
<213>  Gallus gallus

<400>  382
uagcaccauu ugaaaucggu u                                                  21


<210>  383
<211>  23
<212>  RNA
<213>  Gallus gallus

<400>  383
uagcaccauu ugaaaucagu guu                                                23


<210>  384
<211>  21
<212>  RNA

<213>  Xenopus tropicalis

<400>  384
uagcaccauu ugaaaucggu u                                                21


<210>  385
<211>  23
<212>  RNA
<213>  Xenopus tropicalis

<400>  385
uagcaccauu ugaaaucagu guu                                              23


<210>  386
<211>  23
<212>  RNA
<213>  Xenopus tropicalis

<400>  386
uagcaccaua ugaaaucagu guc                                              23


<210>  387
<211>  24
<212>  RNA
<213>  Bos taurus

<400>  387
uagcaccauu ugaaaucagu guuu                                             24


<210>  388
<211>  20
<212>  RNA
<213>  Sus scrofa

<400>  388
uagcaccauu ugaaaucagu                                                  20


<210>  389
<211>  22
<212>  RNA
<213>  Danio rerio

<400>  389
uagcaccauu ugaaaucggu ua                                               22


<210>  390
<211>  22
<212>  RNA
<213>  Danio rerio

<400>  390
uagcaccauu ugaaaucagu gu                                               22


<210>  391
<211>  22
<212>  RNA
<213>  Fugu rubripes

<400>  391
uagcaccauu ugaaaucggu ua                                               22

<210>  392
<211>  22
<212>  RNA
<213>  Fugu rubripes

<400>  392
uagcaccauu ugaaaucagu gu                                                22

<210>  393
<211>  22
<212>  RNA
<213>  Tetraodon nigroviridis

<400>  393
uagcaccauu ugaaaucggu ua                                                22

<210>  394
<211>  22
<212>  RNA
<213>  Tetraodon nigroviridis

<400>  394
uagcaccauu ugaaaucagu gu                                                22

<210>  395
<211>  22
<212>  RNA
<213>  Mus musculus

<400>  395
uagcaccauu ugaaaucggu ua                                                22

<210>  396
<211>  22
<212>  RNA
<213>  Rattus norvegicus

<400>  396
uagcaccauu ugaaaucggu ua                                                22

<210>  397
<211>  20
<212>  RNA
<213>  Gallus gallus

<400>  397
uagcaccauu ugaaaucggu                                                   20

<210>  398
<211>  20
<212>  RNA
<213>  Xenopus tropicalis

<400>  398
uagcaccauu ugaaaucggu                                                   20

<210>  399
<211>  22
<212>  RNA
<213>  Bos taurus

<400>  399
uagcaccauu ugaaaucggu ua                                              22

<210>  400
<211>  22
<212>  RNA
<213>  Sus scrofa

<400>  400
uagcaccauu ugaaaucggu ua                                              22

<210>  401
<211>  22
<212>  RNA
<213>  Mus musculus

<400>  401
uguaaacauc cuacacucag cu                                              22

<210>  402
<211>  22
<212>  RNA
<213>  Mus musculus

<400>  402
uguaaacauc cccgacugga ag                                              22

<210>  403
<211>  22
<212>  RNA
<213>  Mus musculus

<400>  403
uguaaacauc cuugacugga ag                                              22

<210>  404
<211>  22
<212>  RNA
<213>  Rattus norvegicus

<400>  404
uguaaacauc cucgacugga ag                                              22

<210>  405
<211>  22
<212>  RNA
<213>  Rattus norvegicus

<400>  405
uguaaacauc cccgacugga ag                                              22

<210>  406
<211>  22
<212>  RNA
<213>  Rattus norvegicus

<400>  406
uguaaacauc cuugacugga ag                                              22

<210> 407
<211> 21
<212> RNA
<213> Ateles geoffroyi

<400> 407
uguaaacauc cuacacucag c 21

<210> 408
<211> 21
<212> RNA
<213> Lagothrix lagotricha

<400> 408
uguaaacauc cuacacucag c 21

<210> 409
<211> 22
<212> RNA
<213> Macaca mulatta

<400> 409
uguaaacauc cucgacugga ag 22

<210> 410
<211> 21
<212> RNA
<213> Macaca mulatta

<400> 410
uguaaacauc cuacacucag c 21

<210> 411
<211> 21
<212> RNA
<213> Macaca nemestrina

<400> 411
uguaaacauc cuacacucag c 21

<210> 412
<211> 22
<212> RNA
<213> Macaca nemestrina

<400> 412
uguaaacauc cccgacugga ag 22

<210> 413
<211> 22
<212> RNA
<213> Gorilla gorilla

<400> 413
uguaaacauc cucgacugga ag 22

<210> 414
<211> 21
<212> RNA
<213> Gorilla gorilla

<400>  414
uguaaacauc cuacacucag c                                                21

<210>  415
<211>  22
<212>  RNA
<213>  Gorilla gorilla

<400>  415
uguaaacauc cccgacugga ag                                               22

<210>  416
<211>  22
<212>  RNA
<213>  Pan paniscus

<400>  416
uguaaacauc cucgacugga ag                                               22

<210>  417
<211>  21
<212>  RNA
<213>  Pan paniscus

<400>  417
uguaaacauc cuacacucag c                                                21

<210>  418
<211>  22
<212>  RNA
<213>  Pan paniscus

<400>  418
uguaaacauc cccgacugga ag                                               22

<210>  419
<211>  22
<212>  RNA
<213>  Pan troglodytes

<400>  419
uguaaacauc cucgacugga ag                                               22

<210>  420
<211>  21
<212>  RNA
<213>  Pan troglodytes

<400>  420
uguaaacauc cuacacucag c                                                21

<210>  421
<211>  22
<212>  RNA
<213>  Pan troglodytes

<400>  421
uguaaacauc cccgacugga ag                                               22

<210>  422

<211>  22
<212>  RNA
<213>  Pongo pygmaeus

<400>  422
uguaaacauc cucgacugga ag                                                22


<210>  423
<211>  22
<212>  RNA
<213>  Monodelphis domestica

<400>  423
uguaaacauc cucgacugga ag                                                22


<210>  424
<211>  22
<212>  RNA
<213>  Gallus gallus

<400>  424
uguaaacauc cucgacugga ag                                                22


<210>  425
<211>  22
<212>  RNA
<213>  Gallus gallus

<400>  425
uguaaacauc cuacacucag cu                                                22


<210>  426
<211>  22
<212>  RNA
<213>  Gallus gallus

<400>  426
uguaaacauc cccgacugga ag                                                22


<210>  427
<211>  19
<212>  RNA
<213>  Gallus gallus

<400>  427
uguaaacauc cuugacugg                                                    19


<210>  428
<211>  22
<212>  RNA
<213>  Xenopus tropicalis

<400>  428
uguaaacauc cucgacugga ag                                                22


<210>  429
<211>  22
<212>  RNA
<213>  Xenopus tropicalis

<400>  429

uguaaacauc cuacacucag cu                                                22

<210>  430
<211>  22
<212>  RNA
<213>  Xenopus tropicalis

<400>  430
uguaaacauc cccgacugga ag                                                22

<210>  431
<211>  20
<212>  RNA
<213>  Xenopus tropicalis

<400>  431
uguaaacauc cuugacugga                                                   20

<210>  432
<211>  24
<212>  RNA
<213>  Bos taurus

<400>  432
uguaaacauc cucgacugga agcu                                              24

<210>  433
<211>  22
<212>  RNA
<213>  Bos taurus

<400>  433
uguaaacauc cuacacucag cu                                                22

<210>  434
<211>  24
<212>  RNA
<213>  Bos taurus

<400>  434
uguaaacauc cccgacugga agcu                                              24

<210>  435
<211>  24
<212>  RNA
<213>  Bos taurus

<400>  435
uguaaacauc cuugacugga agcu                                              24

<210>  436
<211>  22
<212>  RNA
<213>  Danio rerio

<400>  436
uguaaacauu cccgacugga ag                                                22

<210>  437
<211>  22

<212>  RNA
<213>  Danio rerio

<400>  437
uguaaacauc cuacacucag cu                                              22


<210>  438
<211>  22
<212>  RNA
<213>  Danio rerio

<400>  438
uguaaacauc cccgacugga ag                                              22


<210>  439
<211>  22
<212>  RNA
<213>  Danio rerio

<400>  439
uguaaacauc cuugacugga ag                                              22


<210>  440
<211>  22
<212>  RNA
<213>  Fugu rubripes

<400>  440
uguaaacauc cuacacucag cu                                              22


<210>  441
<211>  22
<212>  RNA
<213>  Fugu rubripes

<400>  441
uguaaacauc cccgacugga ag                                              22


<210>  442
<211>  22
<212>  RNA
<213>  Tetraodon nigroviridis

<400>  442
uguaaacauc cuacacucag cu                                              22


<210>  443
<211>  22
<212>  RNA
<213>  Tetraodon nigroviridis

<400>  443
uguaaacauc cccgacugga ag                                              22


<210>  444
<211>  23
<212>  RNA
<213>  Mus musculus

<400>  444
uguaaacauc cuacacucuc agc                                             23

<210>  445
<211>  23
<212>  RNA
<213>  Rattus norvegicus

<400>  445
uguaaacauc cuacacucuc agc                                              23


<210>  446
<211>  23
<212>  RNA
<213>  Lagothrix lagotricha

<400>  446
uguaaacauc cuacacucuc agc                                              23


<210>  447
<211>  23
<212>  RNA
<213>  Macaca nemestrina

<400>  447
uguaaacauc cuacacucuc agc                                              23


<210>  448
<211>  23
<212>  RNA
<213>  Pan troglodytes

<400>  448
uguaaacauc cuacacucuc agc                                              23


<210>  449
<211>  24
<212>  RNA
<213>  Gallus gallus

<400>  449
uguaaacauc cuacacucuc agcu                                             24


<210>  450
<211>  23
<212>  RNA
<213>  Xenopus tropicalis

<400>  450
uguaaacauc cuacacucuc agc                                              23


<210>  451
<211>  23
<212>  RNA
<213>  Bos taurus

<400>  451
uguaaacauc cuacacucuc agc                                              23


<210>  452
<211>  23
<212>  RNA

<213>  Sus scrofa

<400>  452
uguaaacauc cuacacucuc agc                                              23


<210>  453
<211>  22
<212>  RNA
<213>  Danio rerio

<400>  453
uguaaacauc cuacacucuc ag                                               22


<210>  454
<211>  22
<212>  RNA
<213>  Fugu rubripes

<400>  454
uguaaacauc cuacacucuc gg                                               22


<210>  455
<211>  22
<212>  RNA
<213>  Tetraodon nigroviridis

<400>  455
uguaaacauc cuacacucuc gg                                               22


<210>  456
<211>  22
<212>  RNA
<213>  Ateles geoffroyi

<400>  456
aaagugcugu ucgugcaggu ag                                               22


<210>  457
<211>  22
<212>  RNA
<213>  Lagothrix lagotricha

<400>  457
aaagugcugu ucgugcaggu ag                                               22


<210>  458
<211>  22
<212>  RNA
<213>  Saguinus labiatus

<400>  458
aaagugcugu ucgugcaggu ag                                               22


<210>  459
<211>  22
<212>  RNA
<213>  Macaca mulatta

<400>  459
aaagugcugu ucgugcaggu ag                                               22

<210>  460
<211>  22
<212>  RNA
<213>  Macaca nemestrina

<400>  460
aaagugcugu ucgugcaggu ag                                                22


<210>  461
<211>  22
<212>  RNA
<213>  Gorilla gorilla

<400>  461
aaagugcugu ucgugcaggu ag                                                22


<210>  462
<211>  22
<212>  RNA
<213>  Pan paniscus

<400>  462
aaagugcugu ucgugcaggu ag                                                22


<210>  463
<211>  22
<212>  RNA
<213>  Pan troglodytes

<400>  463
aaagugcugu ucgugcaggu ag                                                22


<210>  464
<211>  22
<212>  RNA
<213>  Pongo pygmaeus

<400>  464
aaagugcugu ucgugcaggu ag                                                22


<210>  465
<211>  22
<212>  RNA
<213>  Monodelphis domestica

<400>  465
aaagugcugu ucgugcaggu ag                                                22


<210>  466
<211>  23
<212>  RNA
<213>  Mus musculus

<400>  466
uaagugcuuc cauguuuuag uag                                               23


<210>  467
<211>  23
<212>  RNA
<213>  Mus musculus

<400>  467
uaagugcuuc cauguuugag ugu                                              23

<210>  468
<211>  20
<212>  RNA
<213>  Monodelphis domestica

<400>  468
uaagugcuuc cauguuuuag                                                  20

<210>  469
<211>  20
<212>  RNA
<213>  Monodelphis domestica

<400>  469
uaagugcuuc cauguuuugg                                                  20

<210>  470
<211>  21
<212>  RNA
<213>  Monodelphis domestica

<400>  470
uaagugcuuc cauguuucag u                                                21

<210>  471
<211>  21
<212>  RNA
<213>  Monodelphis domestica

<400>  471
uaagugcuuc cauguuugag u                                                21

<210>  472
<211>  23
<212>  RNA
<213>  Gallus gallus

<400>  472
uaagugcuuc cauguuuuag uag                                              23

<210>  473
<211>  23
<212>  RNA
<213>  Gallus gallus

<400>  473
uaagugcuuc cauguuucag ugg                                              23

<210>  474
<211>  23
<212>  RNA
<213>  Gallus gallus

<400>  474
uaagugcuuc cauguuuuag uug                                              23

<210>  475
<211>  23
<212>  RNA
<213>  Xenopus tropicalis

<400>  475
uaagugcucc aauguuuuag ugg                                              23


<210>  476
<211>  22
<212>  RNA
<213>  Lagothrix lagotricha

<400>  476
uucaacgggu auuuauugag ca                                               22


<210>  477
<211>  22
<212>  RNA
<213>  Saguinus labiatus

<400>  477
uucaacgggu auuuauugag ca                                               22


<210>  478
<211>  22
<212>  RNA
<213>  Gorilla gorilla

<400>  478
uucaacgggu auuuauugag ca                                               22


<210>  479
<211>  22
<212>  RNA
<213>  Pan paniscus

<400>  479
uucaacgggu auuuauugag ca                                               22


<210>  480
<211>  22
<212>  RNA
<213>  Pan troglodytes

<400>  480
uucaacgggu auuuauugag ca                                               22


<210>  481
<211>  22
<212>  RNA
<213>  Pongo pygmaeus

<400>  481
uucaacgggu auuuauugag ca                                               22


<210>  482
<211>  22
<212>  RNA
<213>  Sus scrofa

<400>  482
uucaacgggu auuuauugag ca                                                22

<210>  483
<211>  22
<212>  RNA
<213>  Mus musculus

<400>  483
ugagguagua aguuguauug uu                                                22

<210>  484
<211>  22
<212>  RNA
<213>  Rattus norvegicus

<400>  484
ugagguagua aguuguauug uu                                                22

<210>  485
<211>  22
<212>  RNA
<213>  Ateles geoffroyi

<400>  485
ugagguagua aguuguauug uu                                                22

<210>  486
<211>  22
<212>  RNA
<213>  Macaca mulatta

<400>  486
ugagguagua aguuguauug uu                                                22

<210>  487
<211>  22
<212>  RNA
<213>  Gorilla gorilla

<400>  487
ugagguagua aguuguauug uu                                                22

<210>  488
<211>  22
<212>  RNA
<213>  Pan paniscus

<400>  488
ugagguagua aguuguauug uu                                                22

<210>  489
<211>  22
<212>  RNA
<213>  Pan troglodytes

<400>  489
ugagguagua aguuguauug uu                                                22

<210>  490

<211>  22
<212>  RNA
<213>  Pongo pygmaeus

<400>  490
ugagguagua aguuguauug uu                                                22


<210>  491
<211>  22
<212>  RNA
<213>  Xenopus tropicalis

<400>  491
ugagguagua aguuguauug uu                                                22


<210>  492
<211>  22
<212>  RNA
<213>  Bos taurus

<400>  492
ugagguagua aguuguauug uu                                                22


<210>  493
<211>  22
<212>  RNA
<213>  Mus musculus

<400>  493
cagugcaaug uuaaaagggc au                                                22


<210>  494
<211>  22
<212>  RNA
<213>  Rattus norvegicus

<400>  494
cagugcaaug uuaaaagggc au                                                22


<210>  495
<211>  20
<212>  RNA
<213>  Macaca mulatta

<400>  495
cagugcaaug uuaaaagggc                                                   20


<210>  496
<211>  20
<212>  RNA
<213>  Macaca nemestrina

<400>  496
cagugcaaug uuaaaagggc                                                   20


<210>  497
<211>  20
<212>  RNA
<213>  Gorilla gorilla

<400>  497

cagugcaaug uuaaaagggc                                                    20

<210>  498
<211>  20
<212>  RNA
<213>  Pan paniscus

<400>  498
cagugcaaug uuaaaagggc                                                    20

<210>  499
<211>  22
<212>  RNA
<213>  Monodelphis domestica

<400>  499
cagugcaaug uaaaaagggc au                                                 22

<210>  500
<211>  22
<212>  RNA
<213>  Gallus gallus

<400>  500
cagugcaaua uuaaaagggc au                                                 22

<210>  501
<211>  22
<212>  RNA
<213>  Xenopus tropicalis

<400>  501
cagugcaaug uuaaaagggc au                                                 22

<210>  502
<211>  22
<212>  RNA
<213>  Danio rerio

<400>  502
cagugcaaug uuaaaagggc au                                                 22

<210>  503
<211>  22
<212>  RNA
<213>  Danio rerio

<400>  503
cagugcaaua uuaaaagggc au                                                 22

<210>  504
<211>  22
<212>  RNA
<213>  Fugu rubripes

<400>  504
cagugcaaua uuaaaagggc au                                                 22

<210>  505
<211>  22

<212>  RNA
<213>  Tetraodon nigroviridis

<400>  505
cagugcaaua uuaaaagggc au                                              22


<210>  506
<211>  22
<212>  RNA
<213>  Mus musculus

<400>  506
cagugcaaug augaaagggc au                                              22


<210>  507
<211>  22
<212>  RNA
<213>  Rattus norvegicus

<400>  507
cagugcaaug augaaagggc au                                              22


<210>  508
<211>  22
<212>  RNA
<213>  Gallus gallus

<400>  508
cagugcaaua augaaagggc gu                                              22


<210>  509
<211>  22
<212>  RNA
<213>  Xenopus tropicalis

<400>  509
cagugcaaug augaaagggc au                                              22


<210>  510
<211>  22
<212>  RNA
<213>  Danio rerio

<400>  510
cagugcaaua augaaagggc au                                              22


<210>  511
<211>  23
<212>  RNA
<213>  Mus musculus

<400>  511
cagugcaaua guauugucaa agc                                             23


<210>  512
<211>  23
<212>  RNA
<213>  Mus musculus

<400>  512
cagugcaaug guauugucaa agc                                             23

<210>  513
<211>  23
<212>  RNA
<213>  Rattus norvegicus

<400>  513
cagugcaaua guauugucaa agc                                              23


<210>  514
<211>  23
<212>  RNA
<213>  Rattus norvegicus

<400>  514
cagugcaaug guauugucaa agc                                              23


<210>  515
<211>  25
<212>  RNA
<213>  Gallus gallus

<400>  515
cagugcaaua auauugucaa agcau                                            25


<210>  516
<211>  23
<212>  RNA
<213>  Xenopus tropicalis

<400>  516
cagugcaaua guauugucaa agc                                              23


<210>  517
<211>  22
<212>  RNA
<213>  Danio rerio

<400>  517
cagugcaaua guauugucaa ag                                               22


<210>  518
<211>  22
<212>  RNA
<213>  Danio rerio

<400>  518
cagugcaaua guauugucau ug                                               22


<210>  519
<211>  22
<212>  RNA
<213>  Danio rerio

<400>  519
cagugcaaua guauugucau ag                                               22


<210>  520
<211>  22
<212>  RNA

<213>  Fugu rubripes

<400>  520
cagugcaaua guauugucau ag                                                22


<210>  521
<211>  22
<212>  RNA
<213>  Tetraodon nigroviridis

<400>  521
cagugcaaua guauugucau ag                                                22


<210>  522
<211>  22
<212>  RNA
<213>  Mus musculus

<400>  522
uaacagucua cagccauggu cg                                                22


<210>  523
<211>  22
<212>  RNA
<213>  Rattus norvegicus

<400>  523
uaacagucua cagccauggu cg                                                22


<210>  524
<211>  22
<212>  RNA
<213>  Monodelphis domestica

<400>  524
uaacagucua cagccauggu cg                                                22


<210>  525
<211>  22
<212>  RNA
<213>  Xenopus tropicalis

<400>  525
uaacagucua cagccauggu cg                                                22


<210>  526
<211>  22
<212>  RNA
<213>  Bos taurus

<400>  526
uaacagucua cagccauggu cg                                                22


<210>  527
<211>  22
<212>  RNA
<213>  Danio rerio

<400>  527
uaacagucua cagccauggu cg                                                22

<210>  528
<211>  22
<212>  RNA
<213>  Fugu rubripes

<400>  528
uaacagucua cagccauggu cg                                                22


<210>  529
<211>  22
<212>  RNA
<213>  Tetraodon nigroviridis

<400>  529
uaacagucua cagccauggu cg                                                22


<210>  530
<211>  22
<212>  RNA
<213>  Mus musculus

<400>  530
uaacagucuc cagucacggc ca                                                22


<210>  531
<211>  22
<212>  RNA
<213>  Rattus norvegicus

<400>  531
uaacagucuc cagucacggc ca                                                22


<210>  532
<211>  21
<212>  RNA
<213>  Monodelphis domestica

<400>  532
uaacagucuc cagucacggc c                                                 21


<210>  533
<211>  21
<212>  RNA
<213>  Xenopus tropicalis

<400>  533
uaacagucua cagucauggc u                                                 21


<210>  534
<211>  21
<212>  RNA
<213>  Danio rerio

<400>  534
uaacagucua cagucauggc u                                                 21


<210>  535
<211>  21
<212>  RNA
<213>  Fugu rubripes

<400>  535
uaacagucua cagucauggc u                                                21

<210>  536
<211>  21
<212>  RNA
<213>  Tetraodon nigroviridis

<400>  536
uaacagucua cagucauggc u                                                21

<210>  537
<211>  22
<212>  RNA
<213>  Ateles geoffroyi

<400>  537
uugguccccu ucaaccagcu gu                                               22

<210>  538
<211>  22
<212>  RNA
<213>  Lagothrix lagotricha

<400>  538
uugguccccu ucaaccagcu gu                                               22

<210>  539
<211>  22
<212>  RNA
<213>  Saguinus labiatus

<400>  539
uugguccccu ucaaccagcu gu                                               22

<210>  540
<211>  22
<212>  RNA
<213>  Macaca nemestrina

<400>  540
uugguccccu ucaaccagcu gu                                               22

<210>  541
<211>  22
<212>  RNA
<213>  Gorilla gorilla

<400>  541
uugguccccu ucaaccagcu gu                                               22

<210>  542
<211>  22
<212>  RNA
<213>  Pan paniscus

<400>  542
uugguccccu ucaaccagcu gu                                               22

<210>  543
<211>  21
<212>  RNA
<213>  Pan troglodytes

<400>  543
uugguccccu ucaaccagcu g                                                21


<210>  544
<211>  22
<212>  RNA
<213>  Monodelphis domestica

<400>  544
uugguccccu ucaaccagcu gu                                               22


<210>  545
<211>  22
<212>  RNA
<213>  Gallus gallus

<400>  545
uugguccccu ucaaccagcu gu                                               22


<210>  546
<211>  22
<212>  RNA
<213>  Gallus gallus

<400>  546
uugguccccu ucaaccagcu gc                                               22


<210>  547
<211>  22
<212>  RNA
<213>  Xenopus laevis

<400>  547
uugguccccu ucaaccagcu gu                                               22


<210>  548
<211>  22
<212>  RNA
<213>  Xenopus tropicalis

<400>  548
uugguccccu ucaaccagcu gu                                               22


<210>  549
<211>  22
<212>  RNA
<213>  Xenopus tropicalis

<400>  549
uugguccccu ucaaccagcc gc                                               22


<210>  550
<211>  21
<212>  RNA
<213>  Gallus gallus

<400>  550
   uugguccccu ucaaccagcu a                                                21

<210>  551
<211>  21
<212>  RNA
<213>  Xenopus tropicalis

<400>  551
   uugguccccu ucaaccagcu a                                                21

<210>  552
<211>  22
<212>  RNA
<213>  Mus musculus

<400>  552
ugugacuggu ugaccagagg gg                                              22

<210>  553
<211>  22
<212>  RNA
<213>  Rattus norvegicus

<400>  553
ugugacuggu ugaccagagg gg                                              22

<210>  554
<211>  21
<212>  RNA
<213>  Macaca nemestrina

<400>  554
ugugacuggu ugaccagagg g                                               21

<210>  555
<211>  21
<212>  RNA
<213>  Gorilla gorilla

<400>  555
ugugacuggu ugaccagagg g                                               21

<210>  556
<211>  21
<212>  RNA
<213>  Pan paniscus

<400>  556
ugugacuggu ugaccagagg g                                               21

<210>  557
<211>  21
<212>  RNA
<213>  Pongo pygmaeus

<400>  557
ugugacuggu ugaccagagg g                                               21

<210>  558

<211>  22
<212>  RNA
<213>  Monodelphis domestica

<400>  558
uauugcuuaa gaauacgcgu ag                                                22


<210>  559
<211>  22
<212>  RNA
<213>  Gallus gallus

<400>  559
uauugcuuaa gaauacgcgu ag                                                22


<210>  560
<211>  22
<212>  RNA
<213>  Xenopus tropicalis

<400>  560
uauugcuuaa gaauacgcgu ag                                                22


<210>  561
<211>  23
<212>  RNA
<213>  Mus musculus

<400>  561
uguaguguuu ccuacuuuau gga                                               23


<210>  562
<211>  23
<212>  RNA
<213>  Rattus norvegicus

<400>  562
uguaguguuu ccuacuuuau gga                                               23


<210>  563
<211>  22
<212>  RNA
<213>  Gallus gallus

<400>  563
uguaguguuu ccuacuuuau gg                                                22


<210>  564
<211>  23
<212>  RNA
<213>  Xenopus tropicalis

<400>  564
uguaguguuu ccuacuuuau gga                                               23


<210>  565
<211>  22
<212>  RNA
<213>  Mus musculus

<400>  565

ugagaacuga auuccauggg uu                                                22

<210>  566
<211>  22
<212>  RNA
<213>  Rattus norvegicus

<400>  566
ugagaacuga auuccauggg uu                                                22

<210>  567
<211>  22
<212>  RNA
<213>  Gallus gallus

<400>  567
ugagaacuga auuccauggg uu                                                22

<210>  568
<211>  22
<212>  RNA
<213>  Gallus gallus

<400>  568
ugagaacuga auuccauagg cg                                                22

<210>  569
<211>  22
<212>  RNA
<213>  Xenopus tropicalis

<400>  569
ugagaacuga auuccauagg uu                                                22

<210>  570
<211>  23
<212>  RNA
<213>  Danio rerio

<400>  570
ugagaacuga auuccauaga ugg                                               23

<210>  571
<211>  22
<212>  RNA
<213>  Danio rerio

<400>  571
ugagaacuga auuccaaggg ug                                                22

<210>  572
<211>  20
<212>  RNA
<213>  Saguinus labiatus

<400>  572
guguguggaa augcuucugc                                                   20

<210>  573
<211>  20

<212>  RNA
<213>  Macaca nemestrina

<400>  573
guguguggaa augcuucugc                                                    20


<210>  574
<211>  20
<212>  RNA
<213>  Pan paniscus

<400>  574
guguguggaa augcuucugc                                                    20


<210>  575
<211>  20
<212>  RNA
<213>  Pan troglodytes

<400>  575
guguguggaa augcuucugc                                                    20


<210>  576
<211>  20
<212>  RNA
<213>  Pongo pygmaeus

<400>  576
guguguggaa augcuucugc                                                    20


<210>  577
<211>  23
<212>  RNA
<213>  Mus musculus

<400>  577
ucuggcuccg ugucuucacu ccc                                                23


<210>  578
<211>  22
<212>  RNA
<213>  Mus musculus

<400>  578
aacauucaac cugucgguga gu                                                 22


<210>  579
<211>  22
<212>  RNA
<213>  Rattus norvegicus

<400>  579
aacauucaac cugucgguga gu                                                 22


<210>  580
<211>  22
<212>  RNA
<213>  Macaca mulatta

<400>  580
aacauucaac cugucgguga gu                                                 22

<210>  581
<211>  22
<212>  RNA
<213>  Gorilla gorilla

<400>  581
aacauucaac cugucgguga gu                                                22

<210>  582
<211>  22
<212>  RNA
<213>  Pan paniscus

<400>  582
aacauucaac cugucgguga gu                                                22

<210>  583
<211>  22
<212>  RNA
<213>  Pan troglodytes

<400>  583
aacauucaac cugucgguga gu                                                22

<210>  584
<211>  23
<212>  RNA
<213>  Monodelphis domestica

<400>  584
aacauucaac gcugucggug agu                                               23

<210>  585
<211>  24
<212>  RNA
<213>  Bos taurus

<400>  585
aacauucaac cugucgguga guuu                                              24

<210>  586
<211>  22
<212>  RNA
<213>  Sus scrofa

<400>  586
aacauucaac cugucgguga gu                                                22

<210>  587
<211>  22
<212>  RNA
<213>  Danio rerio

<400>  587
cacauucauu gcugucggug gg                                                22

<210>  588
<211>  23
<212>  RNA

<213>  Mus musculus

<400>  588
aacauucauu guugucggug ggu                                              23


<210>  589
<211>  23
<212>  RNA
<213>  Rattus norvegicus

<400>  589
aacauucauu guugucggug ggu                                              23


<210>  590
<211>  21
<212>  RNA
<213>  Xenopus tropicalis

<400>  590
ugguucuaga cuugccaacu a                                                21


<210>  591
<211>  21
<212>  RNA
<213>  Danio rerio

<400>  591
ugguucuaga cuugccaacu a                                                21


<210>  592
<211>  22
<212>  RNA
<213>  Mus musculus

<400>  592
uauggcacug guagaauuca cu                                               22


<210>  593
<211>  22
<212>  RNA
<213>  Rattus norvegicus

<400>  593
uauggcacug guagaauuca cu                                               22


<210>  594
<211>  23
<212>  RNA
<213>  Saguinus labiatus

<400>  594
uauggcacug guagaauuca cug                                              23


<210>  595
<211>  23
<212>  RNA
<213>  Macaca mulatta

<400>  595
uauggcacug guagaauuca cug                                              23

<210>  596
<211>  23
<212>  RNA
<213>  Macaca nemestrina

<400>  596
uauggcacug guagaauuca cug                                                23


<210>  597
<211>  23
<212>  RNA
<213>  Gorilla gorilla

<400>  597
uauggcacug guagaauuca cug                                                23


<210>  598
<211>  23
<212>  RNA
<213>  Pan paniscus

<400>  598
uauggcacug guagaauuca cug                                                23


<210>  599
<211>  23
<212>  RNA
<213>  Pan troglodytes

<400>  599
uauggcacug guagaauuca cug                                                23


<210>  600
<211>  23
<212>  RNA
<213>  Monodelphis domestica

<400>  600
uauggcacug guagaauuca cug                                                23


<210>  601
<211>  23
<212>  RNA
<213>  Gallus gallus

<400>  601
uauggcacug guagaauuca cug                                                23


<210>  602
<211>  23
<212>  RNA
<213>  Xenopus tropicalis

<400>  602
uauggcacug guagaauuca cug                                                23


<210>  603
<211>  23
<212>  RNA
<213>  Sus scrofa

<400>  603
uauggcacug guagaauuca cug                                              23

<210>  604
<211>  23
<212>  RNA
<213>  Danio rerio

<400>  604
uauggcacug guagaauuca cug                                              23

<210>  605
<211>  23
<212>  RNA
<213>  Fugu rubripes

<400>  605
uauggcacug guagaauuca cug                                              23

<210>  606
<211>  23
<212>  RNA
<213>  Tetraodon nigroviridis

<400>  606
uauggcacug guagaauuca cug                                              23

<210>  607
<211>  22
<212>  RNA
<213>  Mus musculus

<400>  607
uggacggaga acugauaagg gu                                               22

<210>  608
<211>  22
<212>  RNA
<213>  Rattus norvegicus

<400>  608
uggacggaga acugauaagg gu                                               22

<210>  609
<211>  22
<212>  RNA
<213>  Macaca nemestrina

<400>  609
uggacggaga acugauaagg gu                                               22

<210>  610
<211>  22
<212>  RNA
<213>  Pan troglodytes

<400>  610
uggacggaga acugauaagg gu                                               22

<210>  611
<211>  22
<212>  RNA
<213>  Pongo pygmaeus

<400>  611
uggacggaga acugauaagg gu                                                22


<210>  612
<211>  22
<212>  RNA
<213>  Monodelphis domestica

<400>  612
uggacggaga acugauaagg gu                                                22


<210>  613
<211>  22
<212>  RNA
<213>  Gallus gallus

<400>  613
uggacggaga acugauaagg gu                                                22


<210>  614
<211>  22
<212>  RNA
<213>  Xenopus tropicalis

<400>  614
uggacggaga acugauaagg cu                                                22


<210>  615
<211>  22
<212>  RNA
<213>  Sus scrofa

<400>  615
uggacggaga acugauaagg gu                                                22


<210>  616
<211>  22
<212>  RNA
<213>  Danio rerio

<400>  616
uggacggaga acugauaagg gc                                                22


<210>  617
<211>  21
<212>  RNA
<213>  Fugu rubripes

<400>  617
uggacggaga acugauaagg g                                                 21


<210>  618
<211>  21
<212>  RNA
<213>  Tetraodon nigroviridis

<400>  618
uggacggaga acugauaagg g                                                21

<210>  619
<211>  21
<212>  RNA
<213>  Mus musculus

<400>  619
cugaccuaug aauugacagc c                                                21

<210>  620
<211>  21
<212>  RNA
<213>  Rattus norvegicus

<400>  620
cugaccuaug aauugacagc c                                                21

<210>  621
<211>  21
<212>  RNA
<213>  Xenopus tropicalis

<400>  621
augaccuaug aauugacagc c                                                21

<210>  622
<211>  23
<212>  RNA
<213>  Bos taurus

<400>  622
cugaccuaug aauugacagc cag                                              23

<210>  623
<211>  21
<212>  RNA
<213>  Danio rerio

<400>  623
augaccuaug aauugacagc c                                                21

<210>  624
<211>  21
<212>  RNA
<213>  Fugu rubripes

<400>  624
augaccuaug aauugacagc c                                                21

<210>  625
<211>  21
<212>  RNA
<213>  Tetraodon nigroviridis

<400>  625
augaccuaug aauugacagc c                                                21

<210>  626

<211>  21
<212>  RNA
<213>  Mus musculus

<400>  626
augaccuaug auuugacaga c                                                21


<210>  627
<211>  21
<212>  RNA
<213>  Rattus norvegicus

<400>  627
augaccuaug auuugacaga c                                                21


<210>  628
<211>  21
<212>  RNA
<213>  Macaca mulatta

<400>  628
augaccuaug aauugacaga c                                                21


<210>  629
<211>  21
<212>  RNA
<213>  Macaca nemestrina

<400>  629
augaccuaug aauugacaga c                                                21


<210>  630
<211>  21
<212>  RNA
<213>  Gorilla gorilla

<400>  630
augaccuaug aauugacaga c                                                21


<210>  631
<211>  21
<212>  RNA
<213>  Pan troglodytes

<400>  631
augaccuaug aauugacaga c                                                21


<210>  632
<211>  21
<212>  RNA
<213>  Pongo pygmaeus

<400>  632
augaccuaug aauugacaga c                                                21


<210>  633
<211>  21
<212>  RNA
<213>  Gallus gallus

<400>  633

augaccuaug aauugacaga c                                                21

<210>  634
<211>  21
<212>  RNA
<213>  Xenopus tropicalis

<400>  634
augaccuaug aaaugacagc c                                                21

<210>  635
<211>  22
<212>  RNA
<213>  Bos taurus

<400>  635
augaccuaug aauugacaga ca                                               22

<210>  636
<211>  22
<212>  RNA
<213>  Rattus norvegicus

<400>  636
aacuggccua caaaguccca gu                                               22

<210>  637
<211>  21
<212>  RNA
<213>  Monodelphis domestica

<400>  637
aacuggccua caaaguccca g                                                21

<210>  638
<211>  24
<212>  RNA
<213>  Gallus gallus

<400>  638
aacuggccca caaagucccg cuuu                                             24

<210>  639
<211>  24
<212>  RNA
<213>  Xenopus tropicalis

<400>  639
aacuggcccg caaagucccg cuuc                                             24

<210>  640
<211>  22
<212>  RNA
<213>  Bos taurus

<400>  640
aacuggccua caaaguccca gu                                               22

<210>  641
<211>  22

<212>  RNA
<213>  Danio rerio

<400>  641
aacuggccua caaaguccca gu                                                22


<210>  642
<211>  22
<212>  RNA
<213>  Fugu rubripes

<400>  642
aacuggccua caaaguccca gu                                                22


<210>  643
<211>  22
<212>  RNA
<213>  Tetraodon nigroviridis

<400>  643
aacuggccua caaaguccca gu                                                22


<210>  644
<211>  22
<212>  RNA
<213>  Mus musculus

<400>  644
aacuggccca caaagucccg cu                                                22


<210>  645
<211>  22
<212>  RNA
<213>  Danio rerio

<400>  645
aacuggcccg caaagucccg cu                                                22


<210>  646
<211>  21
<212>  RNA
<213>  Mus musculus

<400>  646
uagcagcaca gaaauauugg c                                                 21


<210>  647
<211>  21
<212>  RNA
<213>  Rattus norvegicus

<400>  647
uagcagcaca gaaauauugg c                                                 21


<210>  648
<211>  21
<212>  RNA
<213>  Gorilla gorilla

<400>  648
uagcagcaca gaaauauugg c                                                 21

<210>  649
<211>  21
<212>  RNA
<213>  Pan paniscus

<400>  649
uagcagcaca gaaauauugg c                                                21


<210>  650
<211>  22
<212>  RNA
<213>  Bos taurus

<400>  650
uagcagcaca gaaauauugg ca                                               22


<210>  651
<211>  22
<212>  RNA
<213>  Mus musculus

<400>  651
cagcagcaca cugugguuug ua                                               22


<210>  652
<211>  22
<212>  RNA
<213>  Rattus norvegicus

<400>  652
cagcagcaca cugugguuug ua                                               22


<210>  653
<211>  22
<212>  RNA
<213>  Bos taurus

<400>  653
cagcagcaca cugugguuug ua                                               22


<210>  654
<211>  22
<212>  RNA
<213>  Mus musculus

<400>  654
uagguaguuu cauguuguug gg                                               22


<210>  655
<211>  22
<212>  RNA
<213>  Rattus norvegicus

<400>  655
uagguaguuu cauguuguug gg                                               22


<210>  656
<211>  22
<212>  RNA

<213>  Ateles geoffroyi

<400>  656
uagguaguuu cauguuguug gg                                                22


<210>  657
<211>  22
<212>  RNA
<213>  Lagothrix lagotricha

<400>  657
uagguaguuu cauguuguug gg                                                22


<210>  658
<211>  21
<212>  RNA
<213>  Macaca mulatta

<400>  658
uagguaguuu cauguuguug g                                                 21


<210>  659
<211>  21
<212>  RNA
<213>  Gorilla gorilla

<400>  659
uagguaguuu cauguuguug g                                                 21


<210>  660
<211>  22
<212>  RNA
<213>  Pan paniscus

<400>  660
uagguaguuu cauguuguug gg                                                22


<210>  661
<211>  22
<212>  RNA
<213>  Pan troglodytes

<400>  661
uagguaguuu cauguuguug gg                                                22


<210>  662
<211>  21
<212>  RNA
<213>  Pongo pygmaeus

<400>  662
uagguaguuu cauguuguug g                                                 21


<210>  663
<211>  21
<212>  RNA
<213>  Gallus gallus

<400>  663
uagguaguuu cauguuguug g                                                 21

<210>  664
<211>  21
<212>  RNA
<213>  Xenopus tropicalis

<400>  664
uagguaguuu cauguuguug g                                                21


<210>  665
<211>  21
<212>  RNA
<213>  Sus scrofa

<400>  665
uagguaguuu cauguuguug g                                                21


<210>  666
<211>  22
<212>  RNA
<213>  Danio rerio

<400>  666
uagguaguuu cauguuguug gg                                               22


<210>  667
<211>  22
<212>  RNA
<213>  Fugu rubripes

<400>  667
uagguaguuu cauguuguug gg                                               22


<210>  668
<211>  22
<212>  RNA
<213>  Tetraodon nigroviridis

<400>  668
uagguaguuu cauguuguug gg                                               22


<210>  669
<211>  22
<212>  RNA
<213>  Mus musculus

<400>  669
uagguaguuu ccuguuguug gg                                               22


<210>  670
<211>  22
<212>  RNA
<213>  Rattus norvegicus

<400>  670
uagguaguuu ccuguuguug gg                                               22


<210>  671
<211>  21
<212>  RNA
<213>  Monodelphis domestica

<400>  671
uagguaguuu ccuguuguug g                                                21

<210>  672
<211>  21
<212>  RNA
<213>  Xenopus tropicalis

<400>  672
uagguaguuu uauguuguug g                                                21

<210>  673
<211>  22
<212>  RNA
<213>  Danio rerio

<400>  673
uagguaguuu caaguuguug gg                                               22

<210>  674
<211>  22
<212>  RNA
<213>  Fugu rubripes

<400>  674
uagguaguuu caaguuguug gg                                               22

<210>  675
<211>  22
<212>  RNA
<213>  Tetraodon nigroviridis

<400>  675
uagguaguuu caaguuguug gg                                               22

<210>  676
<211>  22
<212>  RNA
<213>  Mus musculus

<400>  676
uucaccaccu ucuccaccca gc                                               22

<210>  677
<211>  22
<212>  RNA
<213>  Ateles geoffroyi

<400>  677
uucaccaccu ucuccaccca gc                                               22

<210>  678
<211>  22
<212>  RNA
<213>  Macaca nemestrina

<400>  678
uucaccaccu ucuccacccg gc                                               22

<210>  679
<211>  22
<212>  RNA
<213>  Pan paniscus

<400>  679
uucaccaccu ucuccaccca gc                                                22

<210>  680
<211>  22
<212>  RNA
<213>  Pan troglodytes

<400>  680
uucaccaccu ucuccaccca gc                                                22

<210>  681
<211>  22
<212>  RNA
<213>  Pongo pygmaeus

<400>  681
uucaccaccu ucuccaccca gc                                                22

<210>  682
<211>  22
<212>  RNA
<213>  Xenopus tropicalis

<400>  682
uacaguaguc ugcacauugg uu                                                22

<210>  683
<211>  22
<212>  RNA
<213>  Danio rerio

<400>  683
uacaguaguc ugcacauugg uu                                                22

<210>  684
<211>  22
<212>  RNA
<213>  Mus musculus

<400>  684
uaacacuguc ugguaacgau gu                                                22

<210>  685
<211>  22
<212>  RNA
<213>  Rattus norvegicus

<400>  685
uaacacuguc ugguaacgau gu                                                22

<210>  686
<211>  22
<212>  RNA
<213>  Monodelphis domestica

<400>  686
uaacacuguc ugguaacgau gu                                              22


<210>  687
<211>  22
<212>  RNA
<213>  Gallus gallus

<400>  687
uaacacuguc ugguaacgau gu                                              22


<210>  688
<211>  22
<212>  RNA
<213>  Xenopus tropicalis

<400>  688
uaacacuguc ugguaacgau gu                                              22


<210>  689
<211>  23
<212>  RNA
<213>  Bos taurus

<400>  689
uaacacuguc ugguaacgau guu                                             23


<210>  690
<211>  22
<212>  RNA
<213>  Danio rerio

<400>  690
uaacacuguc ugguaacgau gu                                              22


<210>  691
<211>  22
<212>  RNA
<213>  Fugu rubripes

<400>  691
uaacacuguc ugguaacgau gu                                              22


<210>  692
<211>  22
<212>  RNA
<213>  Tetraodon nigroviridis

<400>  692
uaacacuguc ugguaacgau gu                                              22


<210>  693
<211>  22
<212>  RNA
<213>  Mus musculus

<400>  693
uucccuuugu cauccuaugc cu                                              22


<210>  694

<211>  22
<212>  RNA
<213>  Rattus norvegicus

<400>  694
uucccuuugu cauccuaugc cu                                                22


<210>  695
<211>  22
<212>  RNA
<213>  Saguinus labiatus

<400>  695
uucccuuugu cauccuaugc cu                                                22


<210>  696
<211>  22
<212>  RNA
<213>  Macaca nemestrina

<400>  696
uucccuuugu cauccuaugc cu                                                22


<210>  697
<211>  22
<212>  RNA
<213>  Gorilla gorilla

<400>  697
uucccuuugu cauccuaugc cu                                                22


<210>  698
<211>  22
<212>  RNA
<213>  Pan paniscus

<400>  698
uucccuuugu cauccuaugc cu                                                22


<210>  699
<211>  22
<212>  RNA
<213>  Pan troglodytes

<400>  699
uucccuuugu cauccuaugc cu                                                22


<210>  700
<211>  22
<212>  RNA
<213>  Pongo pygmaeus

<400>  700
uucccuuugu cauccuaugc cu                                                22


<210>  701
<211>  22
<212>  RNA
<213>  Monodelphis domestica

<400>  701

uucccuuugu cauccuaugc cu                                                22

<210>  702
<211>  22
<212>  RNA
<213>  Gallus gallus

<400>  702
uucccuuugu cauccuaugc cu                                                22

<210>  703
<211>  22
<212>  RNA
<213>  Xenopus tropicalis

<400>  703
uucccuuugu cauccuaugc cu                                                22

<210>  704
<211>  22
<212>  RNA
<213>  Bos taurus

<400>  704
uucccuuugu cauccuaugc cu                                                22

<210>  705
<211>  22
<212>  RNA
<213>  Sus scrofa

<400>  705
uucccuuugu cauccuaugc cu                                                22

<210>  706
<211>  22
<212>  RNA
<213>  Danio rerio

<400>  706
uucccuuugu cauccuaugc cu                                                22

<210>  707
<211>  22
<212>  RNA
<213>  Fugu rubripes

<400>  707
uucccuuugu cauccuaugc cu                                                22

<210>  708
<211>  22
<212>  RNA
<213>  Tetraodon nigroviridis

<400>  708
uucccuuugu cauccuaugc cu                                                22

<210>  709
<211>  22

<212>  RNA
<213>  Mus musculus

<400>  709
uucccuuugu cauccuuugc cu                                                22

<210>  710
<211>  22
<212>  RNA
<213>  Rattus norvegicus

<400>  710
uucccuuugu cauccuuugc cu                                                22

<210>  711
<211>  22
<212>  RNA
<213>  Macaca mulatta

<400>  711
uucccuuugu cauccuuugc cu                                                22

<210>  712
<211>  22
<212>  RNA
<213>  Macaca nemestrina

<400>  712
uucccuuugu cauccuucgc cu                                                22

<210>  713
<211>  22
<212>  RNA
<213>  Pongo pygmaeus

<400>  713
uucccuuugu cauccuucgc cu                                                22

<210>  714
<211>  22
<212>  RNA
<213>  Gallus gallus

<400>  714
uucccuuugu cauccuaugc cu                                                22

<210>  715
<211>  22
<212>  RNA
<213>  Mus musculus

<400>  715
uccuucauuc caccggaguc ug                                                22

<210>  716
<211>  22
<212>  RNA
<213>  Rattus norvegicus

<400>  716
uccuucauuc caccggaguc ug                                                22

<210>  717
<211>  22
<212>  RNA
<213>  Ateles geoffroyi

<400>  717
uccuucauuc caccggaguc ug                                                22

<210>  718
<211>  22
<212>  RNA
<213>  Lagothrix lagotricha

<400>  718
uccuucauuc caccggaguc ug                                                22

<210>  719
<211>  22
<212>  RNA
<213>  Macaca nemestrina

<400>  719
uccuucauuc caccggaguc ug                                                22

<210>  720
<211>  22
<212>  RNA
<213>  Gorilla gorilla

<400>  720
uccuucauuc caccggaguc ug                                                22

<210>  721
<211>  22
<212>  RNA
<213>  Pan paniscus

<400>  721
uccuucauuc caccggaguc ug                                                22

<210>  722
<211>  22
<212>  RNA
<213>  Pan troglodytes

<400>  722
uccuucauuc caccggaguc ug                                                22

<210>  723
<211>  22
<212>  RNA
<213>  Gallus gallus

<400>  723
uccuucauuc caccggaguc ug                                                22

<210>  724
<211>  22
<212>  RNA

<213>  Gallus gallus

<400>  724
cccuucauuc caccggaauc ug                                                22


<210>  725
<211>  22
<212>  RNA
<213>  Xenopus tropicalis

<400>  725
uccuucauuc caccggaguc ug                                                22


<210>  726
<211>  22
<212>  RNA
<213>  Bos taurus

<400>  726
uccuucauuc caccggaguc ug                                                22


<210>  727
<211>  22
<212>  RNA
<213>  Sus scrofa

<400>  727
uccuucauuc caccggaguc ug                                                22


<210>  728
<211>  22
<212>  RNA
<213>  Danio rerio

<400>  728
uccuucauuc caccggaguc ug                                                22


<210>  729
<211>  22
<212>  RNA
<213>  Fugu rubripes

<400>  729
uccuucauuc caccggaguc ug                                                22


<210>  730
<211>  22
<212>  RNA
<213>  Tetraodon nigroviridis

<400>  730
uccuucauuc caccggaguc ug                                                22


<210>  731
<211>  22
<212>  RNA
<213>  Mus musculus

<400>  731
auaagacgag caaaaagcuu gu                                                22

<210>  732
<211>  22
<212>  RNA
<213>  Rattus norvegicus

<400>  732
auaagacgag caaaaagcuu gu                                                22


<210>  733
<211>  22
<212>  RNA
<213>  Monodelphis domestica

<400>  733
auaagacgag caaaaagcuc gu                                                22


<210>  734
<211>  22
<212>  RNA
<213>  Xenopus tropicalis

<400>  734
auaagacgag cauaaagcuu gu                                                22


<210>  735
<211>  21
<212>  RNA
<213>  Mus musculus

<400>  735
agcuacaucu ggcuacuggg u                                                 21


<210>  736
<211>  21
<212>  RNA
<213>  Rattus norvegicus

<400>  736
agcuacaucu ggcuacuggg u                                                 21


<210>  737
<211>  24
<212>  RNA
<213>  Ateles geoffroyi

<400>  737
agcuacaucu ggcuacuggg ucuc                                              24


<210>  738
<211>  24
<212>  RNA
<213>  Gallus gallus

<400>  738
agcuacaucu ggcuacuggg ucuc                                              24


<210>  739
<211>  24
<212>  RNA
<213>  Xenopus tropicalis

<400>  739
agcuacaucu ggcuacuggg ucuc                                          24

<210>  740
<211>  21
<212>  RNA
<213>  Bos taurus

<400>  740
agcuacaucu ggcuacuggg u                                             21

<210>  741
<211>  24
<212>  RNA
<213>  Danio rerio

<400>  741
agcuacaucu ggcuacuggg ucuc                                          24

<210>  742
<211>  24
<212>  RNA
<213>  Fugu rubripes

<400>  742
agcuacaucu ggcuacuggg ucuc                                          24

<210>  743
<211>  24
<212>  RNA
<213>  Tetraodon nigroviridis

<400>  743
agcuacaucu ggcuacuggg ucuc                                          24

<210>  744
<211>  23
<212>  RNA
<213>  Mus musculus

<400>  744
acuuuaacau gggaaugcuu ucu                                           23

<210>  745
<211>  23
<212>  RNA
<213>  Gallus gallus

<400>  745
acuuuaacau ggaggugcuu ucu                                           23

<210>  746
<211>  20
<212>  RNA
<213>  Mus musculus

<400>  746
ccacugcccc aggugcugcu                                               20

<210>  747
<211>  22
<212>  RNA
<213>  Rattus norvegicus

<400>  747
ccacugcccc aggugcugcu gg                                                22

<210>  748
<211>  23
<212>  RNA
<213>  Mus musculus

<400>  748
cgcauccccu agggcauugg ugu                                               23

<210>  749
<211>  23
<212>  RNA
<213>  Rattus norvegicus

<400>  749
cgcauccccu agggcauugg ugu                                               23

<210>  750
<211>  22
<212>  RNA
<213>  Mus musculus

<400>  750
aacacaccca gcuaaccuuu uu                                                22

<210>  751
<211>  22
<212>  RNA
<213>  Rattus norvegicus

<400>  751
aacacaccca gcuaaccuuu uu                                                22

<210>  752
<211>  21
<212>  RNA
<213>  Rattus norvegicus

<400>  752
gccccugggc cuauccuaga a                                                 21

<210>  753
<211>  21
<212>  RNA
<213>  Bos taurus

<400>  753
gccccugggc cuauccuaga a                                                 21

<210>  754
<211>  25
<212>  RNA
<213>  Bos taurus

<400>  754
ucucacacag aaaucgcacc caucu                                          25


<210>  755
<211>  23
<212>  RNA
<213>  Mus musculus

<400>  755
ugucugcccg agugccugcc ucu                                            23


<210>  756
<211>  23
<212>  RNA
<213>  Rattus norvegicus

<400>  756
ugucugccug agugccugcc ucu                                            23


<210>  757
<211>  21
<212>  RNA
<213>  Mus musculus

<400>  757
aucauagagg aacauccacu u                                              21


<210>  758
<211>  22
<212>  RNA
<213>  Mus musculus

<400>  758
aucacacaaa ggcaacuuuu gu                                             22


<210>  759
<211>  22
<212>  RNA
<213>  Rattus norvegicus

<400>  759
aucacacaaa ggcaacuuuu gg                                             22


<210>  760
<211>  22
<212>  RNA
<213>  Bos taurus

<400>  760
ugguugacca uagaacaugc gc                                             22


<210>  761
<211>  22
<212>  RNA
<213>  Mus musculus

<400>  761
gaaguuguuc gugguggauu cg                                             22


<210>  762

<211>  22
<212>  RNA
<213>  Rattus norvegicus

<400>  762
gaaguuguuc gugguggauu cg                                                22

<210>  763
<211>  23
<212>  RNA
<213>  Rattus norvegicus

<400>  763
acuucaccug guccacuagc cgu                                               23

<210>  764
<211>  23
<212>  RNA
<213>  Rattus norvegicus

<400>  764
agcucggucu gaggccccuc agu                                               23

<210>  765
<211>  21
<212>  RNA
<213>  Mus musculus

<400>  765
ugucuugcag gccgucaugc a                                                 21

<210>  766
<211>  21
<212>  RNA
<213>  Rattus norvegicus

<400>  766
ugucuugcag gccgucaugc a                                                 21

<210>  767
<211>  23
<212>  RNA
<213>  Ovis aries

<400>  767
ugucuugcag gccgucaugc agg                                               23

<210>  768
<211>  22
<212>  RNA
<213>  Mus musculus

<400>  768
aucaugaugg gcuccucggu gu                                                22

<210>  769
<211>  22
<212>  RNA
<213>  Rattus norvegicus

<400>  769

aucaugaugg gcuccucggu gu                                                22

<210>  770
<211>  20
<212>  RNA
<213>  Mus musculus

<400>  770
aggcaguguu guuagcuggc                                                    20

<210>  771
<211>  21
<212>  RNA
<213>  Mus musculus

<400>  771
aggcagugca uugcuagcug g                                                  21

<210>  772
<211>  22
<212>  RNA
<213>  Rattus norvegicus

<400>  772
uggcagugua uuguuagcug gu                                                 22

<210>  773
<211>  22
<212>  RNA
<213>  Canis familiaris

<400>  773
uggcagugua uuguuagcug gu                                                 22

<210>  774
<211>  22
<212>  RNA
<213>  Monodelphis domestica

<400>  774
uggcagugua uuguuagcug gu                                                 22

<210>  775
<211>  21
<212>  RNA
<213>  Gallus gallus

<400>  775
uggcagugua uguuagcugg u                                                  21

<210>  776
<211>  22
<212>  RNA
<213>  Xenopus tropicalis

<400>  776
aggcagugua auguuagcug gu                                                 22

<210>  777
<211>  22

<212>  RNA
<213>  Mus musculus

<400>  777
aaaccguuac cauuacugag uu                                                22

<210>  778
<211>  22
<212>  RNA
<213>  Rattus norvegicus

<400>  778
aaaccguuac cauuacugag uu                                                22

<210>  779
<211>  22
<212>  RNA
<213>  Monodelphis domestica

<400>  779
aaaccguuac cauuacugug uu                                                22

<210>  780
<211>  23
<212>  RNA
<213>  Gallus gallus

<400>  780
aaaccguuac cauuacugag uuu                                               23

<210>  781
<211>  22
<212>  RNA
<213>  Xenopus tropicalis

<400>  781
aaaccguuac cauuacugag uu                                                22

<210>  782
<211>  22
<212>  RNA
<213>  Danio rerio

<400>  782
aaaccguuac cauuacugag uu                                                22

<210>  783
<211>  22
<212>  RNA
<213>  Mus musculus

<400>  783
uguuugcaga ggaaacugag ac                                                22

<210>  784
<211>  22
<212>  RNA
<213>  Rattus norvegicus

<400>  784
uaugugccuu uggacuacau cg                                                22

<210>  785
<211>  22
<212>  RNA
<213>  Gallus gallus

<400>  785
uaugugcccu uggacuacau cg                                                22

<210>  786
<211>  22
<212>  RNA
<213>  Xenopus tropicalis

<400>  786
uaugugcccu uggacuacau cg                                                22

<210>  787
<211>  21
<212>  RNA
<213>  Bos taurus

<400>  787
uaugugccuu uggacuacau c                                                 21

<210>  788
<211>  22
<212>  RNA
<213>  Danio rerio

<400>  788
uaugugcccu uggacuacau cg                                                22

<210>  789
<211>  22
<212>  RNA
<213>  Fugu rubripes

<400>  789
uaugugcccu uggacuacau cg                                                22

<210>  790
<211>  22
<212>  RNA
<213>  Tetraodon nigroviridis

<400>  790
uaugugcccu uggacuacau cg                                                22

<210>  791
<211>  23
<212>  RNA
<213>  Rattus norvegicus

<400>  791
ucacuccucc ccucccgucu ugu                                               23

<210>  792
<211>  23
<212>  RNA

<213>  Gallus gallus

<400>  792
agugacauca uauguacggc ugc                                              23


<210>  793
<211>  23
<212>  RNA
<213>  Xenopus tropicalis

<400>  793
agugacauca uauguacggc ugc                                              23


<210>  794
<211>  23
<212>  RNA
<213>  Danio rerio

<400>  794
agugacauca uauguacggc ugc                                              23


<210>  795
<211>  23
<212>  RNA
<213>  Fugu rubripes

<400>  795
agugacauca uauguacggc ugc                                              23


<210>  796
<211>  23
<212>  RNA
<213>  Tetraodon nigroviridis

<400>  796
agugacauca uauguacggc ugc                                              23


<210>  797
<211>  22
<212>  RNA
<213>  Mus musculus

<400>  797
aguggggaac ccuuccauga gg                                               22


<210>  798
<211>  22
<212>  RNA
<213>  Mus musculus

<400>  798
ugaaacauac acgggaaacc uc                                               22


<210>  799
<211>  21
<212>  RNA
<213>  Rattus norvegicus

<400>  799
ugaaacauac acgggaaacc u                                                21

<210>  800
<211>  22
<212>  RNA
<213>  Rattus norvegicus

<400>  800
aauccuuugu cccuggguga aa                                                22

<210>  801
<211>  22
<212>  RNA
<213>  Mus musculus

<400>  801
agacccuggu cugcacucua uc                                                22

<210>  802
<211>  20
<212>  RNA
<213>  Rattus norvegicus

<400>  802
gucaacacuu gcugguuucc                                                   20

<210>  803
<211>  20
<212>  RNA
<213>  Homo sapiens

<400>  803
aggaauguuc cuucuuugcc                                                   20

<210>  804
<211>  23
<212>  RNA
<213>  Homo sapiens

<400>  804
uacccuguag aaccgaauuu gug                                               23

<210>  805
<211>  23
<212>  RNA
<213>  Homo sapiens

<400>  805
caaagugcuu acagugcagg uag                                               23

<210>  806
<211>  22
<212>  RNA
<213>  Homo sapiens

<400>  806
caaagugccu cccuuuagag ug                                                22

<210>  807
<211>  22
<212>  RNA
<213>  Homo sapiens

<400>  807
gaaagugcuu ccuuuuagag gc                                              22

<210>  808
<211>  23
<212>  RNA
<213>  Mus musculus

<400>  808
caaagugcuu acagugcagg uag                                             23

<210>  809
<211>  23
<212>  RNA
<213>  Mus musculus

<400>  809
caaagugcug uucgugcagg uag                                             23

<210>  810
<211>  23
<212>  RNA
<213>  Rattus norvegicus

<400>  810
caaagugcuu acagugcagg uag                                             23

<210>  811
<211>  23
<212>  RNA
<213>  Rattus norvegicus

<400>  811
caaagugcug uucgugcagg uag                                             23

<210>  812
<211>  23
<212>  RNA
<213>  Xenopus tropicalis

<400>  812
caaagugcug uucgugcagg uag                                             23

<210>  813
<211>  22
<212>  RNA
<213>  Bos taurus

<400>  813
caaagugcug uucgugcagg ua                                              22

<210>  814
<211>  22
<212>  RNA
<213>  Danio rerio

<400>  814
caaagugcuu acagugcagg ua                                              22

<210>  815
<211>  22
<212>  RNA
<213>  Danio rerio

<400>  815
aaaagugcug uuugugcagg ua                                                22


<210>  816
<211>  22
<212>  RNA
<213>  Fugu rubripes

<400>  816
caaagugcuu acagugcagg ua                                                22


<210>  817
<211>  22
<212>  RNA
<213>  Tetraodon nigroviridis

<400>  817
caaagugcuu acagugcagg ua                                                22


<210>  818
<211>  22
<212>  RNA
<213>  Homo sapiens

<400>  818
uauugcacuu gucccggccu gu                                                22


<210>  819
<211>  22
<212>  RNA
<213>  Homo sapiens

<400>  819
uauugcacuc gucccggccu cc                                                22


<210>  820
<211>  22
<212>  RNA
<213>  Homo sapiens

<400>  820
aauugcacgg uauccaucug ua                                                22


<210>  821
<211>  22
<212>  RNA
<213>  Homo sapiens

<400>  821
aauugcacuu uagcaauggu ga                                                22


<210>  822
<211>  21
<212>  RNA
<213>  Mus musculus

<400>  822
uauugcacuu gucccggccu g                                                21

<210>  823
<211>  22
<212>  RNA
<213>  Mus musculus

<400>  823
uauugcacuc gucccggccu cc                                               22

<210>  824
<211>  22
<212>  RNA
<213>  Mus musculus

<400>  824
aauugcacgg uauccaucug ua                                               22

<210>  825
<211>  22
<212>  RNA
<213>  Mus musculus

<400>  825
aauugcacuu uagcaauggu ga                                               22

<210>  826
<211>  21
<212>  RNA
<213>  Rattus norvegicus

<400>  826
uauugcacuu gucccggccu g                                                21

<210>  827
<211>  22
<212>  RNA
<213>  Rattus norvegicus

<400>  827
uauugcacuc gucccggccu cc                                               22

<210>  828
<211>  22
<212>  RNA
<213>  Rattus norvegicus

<400>  828
aauugcacgg uauccaucug ua                                               22

<210>  829
<211>  22
<212>  RNA
<213>  Ateles geoffroyi

<400>  829
uauugcacuu gucccggccu gu                                               22

<210>  830

<211>  22
<212>  RNA
<213>  Lagothrix lagotricha

<400>  830
uauugcacuu gucccggccu gu                                                22


<210>  831
<211>  22
<212>  RNA
<213>  Saguinus labiatus

<400>  831
uauugcacuu gucccggccu gu                                                22


<210>  832
<211>  22
<212>  RNA
<213>  Macaca mulatta

<400>  832
uauugcacuu gucccggccu gu                                                22


<210>  833
<211>  22
<212>  RNA
<213>  Macaca nemestrina

<400>  833
uauugcacuu gucccggccu gu                                                22


<210>  834
<211>  22
<212>  RNA
<213>  Gorilla gorilla

<400>  834
uauugcacuu gucccggccu gu                                                22


<210>  835
<211>  22
<212>  RNA
<213>  Pan paniscus

<400>  835
uauugcacuu gucccggccu gu                                                22


<210>  836
<211>  22
<212>  RNA
<213>  Pan troglodytes

<400>  836
uauugcacuu gucccggccu gu                                                22


<210>  837
<211>  22
<212>  RNA
<213>  Pongo pygmaeus

<400>  837

uauugcacuu gucccggccu gu                                                22

<210>  838
<211>  22
<212>  RNA
<213>  Lemur catta

<400>  838
uauugcacuu gucccggccu gu                                                22

<210>  839
<211>  21
<212>  RNA
<213>  Monodelphis domestica

<400>  839
uauugcacuu gucccggccu g                                                 21

<210>  840
<211>  21
<212>  RNA
<213>  Gallus gallus

<400>  840
uauugcacuu gucccggccu g                                                 21

<210>  841
<211>  21
<212>  RNA
<213>  Gallus gallus

<400>  841
aauugcacuu uagcaauggu g                                                 21

<210>  842
<211>  21
<212>  RNA
<213>  Xenopus tropicalis

<400>  842
uauugcacuu gucccggccu g                                                 21

<210>  843
<211>  21
<212>  RNA
<213>  Xenopus tropicalis

<400>  843
uauugcacuc gucccggccu c                                                 21

<210>  844
<211>  21
<212>  RNA
<213>  Xenopus tropicalis

<400>  844
aauugcacgg uauccaucug u                                                 21

<210>  845
<211>  22

<212>  RNA
<213>  Xenopus tropicalis

<400>  845
aauugcacug uagcaauggu ga                                              22


<210>  846
<211>  22
<212>  RNA
<213>  Bos taurus

<400>  846
uauugcacuu gucccggccu gu                                              22


<210>  847
<211>  22
<212>  RNA
<213>  Danio rerio

<400>  847
uauugcacuu gucccggccu gu                                              22


<210>  848
<211>  22
<212>  RNA
<213>  Danio rerio

<400>  848
uauugcacuc gucccggccu cc                                              22


<210>  849
<211>  22
<212>  RNA
<213>  Danio rerio

<400>  849
aauugcacgg uauccaucug ua                                              22


<210>  850
<211>  22
<212>  RNA
<213>  Fugu rubripes

<400>  850
uauugcacuu gucccggccu gu                                              22


<210>  851
<211>  22
<212>  RNA
<213>  Tetraodon nigroviridis

<400>  851
uauugcacuu gucccggccu gu                                              22


<210>  852
<211>  21
<212>  RNA
<213>  Homo sapiens

<400>  852
uucacagugg cuaaguuccg c                                               21

<210>  853
<211>  23
<212>  RNA
<213>  Homo sapiens

<400>  853
aaggagcuua caaucuagcu ggg                                                23

<210>  854
<211>  23
<212>  RNA
<213>  Mus musculus

<400>  854
aaggagcuua caaucuagcu ggg                                                23

<210>  855
<211>  23
<212>  RNA
<213>  Rattus norvegicus

<400>  855
aaggagcuua caaucuagcu ggg                                                23

<210>  856
<211>  23
<212>  RNA
<213>  Homo sapiens

<400>  856
uagcaccauu ugaaaucagu guu                                                23

<210>  857
<211>  22
<212>  RNA
<213>  Homo sapiens

<400>  857
uguaaacauc cucgacugga ag                                                 22

<210>  858
<211>  22
<212>  RNA
<213>  Homo sapiens

<400>  858
uguaaacauc cccgacugga ag                                                 22

<210>  859
<211>  22
<212>  RNA
<213>  Homo sapiens

<400>  859
uguaaacauc cuugacugga ag                                                 22

<210>  860
<211>  23
<212>  RNA

<213>  Mus musculus

<400>  860
uguaaacaau uccuaggcaa ugu                                              23


<210>  861
<211>  23
<212>  RNA
<213>  Rattus norvegicus

<400>  861
uguaaacaau uccuaggcaa ugu                                              23


<210>  862
<211>  23
<212>  RNA
<213>  Homo sapiens

<400>  862
uaagugcuuc cauguuuugg uga                                              23


<210>  863
<211>  23
<212>  RNA
<213>  Homo sapiens

<400>  863
uaagugcuuc cauguuuuag uag                                              23


<210>  864
<211>  23
<212>  RNA
<213>  Homo sapiens

<400>  864
uaagugcuuc cauguuucag ugg                                              23


<210>  865
<211>  22
<212>  RNA
<213>  Homo sapiens

<400>  865
aaagugcuuc ccuuuggacu gu                                               22


<210>  866
<211>  21
<212>  RNA
<213>  Homo sapiens

<400>  866
aaagugcuuc cuuuuagagg g                                                21


<210>  867
<211>  22
<212>  RNA
<213>  Homo sapiens

<400>  867
aaagugcuuc cuuuuagagg gu                                               22

<210>  868
<211>  22
<212>  RNA
<213>  Homo sapiens

<400>  868
aaagugcuuc ucuuuggugg gu                                                22

<210>  869
<211>  21
<212>  RNA
<213>  Homo sapiens

<400>  869
aaagugcuuc cuuuuugagg g                                                 21

<210>  870
<211>  22
<212>  RNA
<213>  Mus musculus

<400>  870
aaagugcuuc cacuuugugu gc                                                22

<210>  871
<211>  22
<212>  RNA
<213>  Mus musculus

<400>  871
aaagugcuuc ccuuuugugu gu                                                22

<210>  872
<211>  23
<212>  RNA
<213>  Mus musculus

<400>  872
aaagugcuac uacuuuugag ucu                                               23

<210>  873
<211>  23
<212>  RNA
<213>  Rattus norvegicus

<400>  873
aaagugcuuc cacuuugugu gcc                                               23

<210>  874
<211>  22
<212>  RNA
<213>  Xenopus laevis

<400>  874
aaagugcuuu cuguuuuggg cg                                                22

<210>  875
<211>  22
<212>  RNA
<213>  Xenopus laevis

<400>  875
uaagugcucu cuaguucggu ug                                              22


<210>  876
<211>  22
<212>  RNA
<213>  Xenopus tropicalis

<400>  876
uaagugcucu cuaguucggu ug                                              22


<210>  877
<211>  22
<212>  RNA
<213>  Danio rerio

<400>  877
   uaagugcuau uuguuggggu ag                                               22


<210>  878
<211>  23
<212>  RNA
<213>  Danio rerio

<400>  878
aaagugcuau caaguugggg uag                                             23


<210>  879
<211>  22
<212>  RNA
<213>  Danio rerio

<400>  879
uaagugcuuc ucuuuggggu ag                                              22


<210>  880
<211>  22
<212>  RNA
<213>  Danio rerio

<400>  880
uaagugcuau uuguuggcgu ag                                              22


<210>  881
<211>  22
<212>  RNA
<213>  Danio rerio

<400>  881
aaagugcuau caaauugggg ua                                              22


<210>  882
<211>  23
<212>  RNA
<213>  Homo sapiens

<400>  882
cagugcaaug auauugucaa agc                                             23

<210>  883
<211>  23
<212>  RNA
<213>  Homo sapiens

<400>  883
uagugcaaua uugcuuauag ggu                                              23


<210>  884
<211>  21
<212>  RNA
<213>  Mus musculus

<400>  884
cagugcaauu aaaaggggga a                                                21


<210>  885
<211>  22
<212>  RNA
<213>  Danio rerio

<400>  885
uagugcaaua uugcuaauag gg                                               22


<210>  886
<211>  22
<212>  RNA
<213>  Danio rerio

<400>  886
uagugcaaua uugcuuauag gg                                               22


<210>  887
<211>  22
<212>  RNA
<213>  Homo sapiens

<400>  887
ugagaacuga auuccauagg cu                                               22


<210>  888
<211>  22
<212>  RNA
<213>  Mus musculus

<400>  888
cagcagcaau ucauguuuug ga                                               22


<210>  889
<211>  22
<212>  RNA
<213>  Rattus norvegicus

<400>  889
cagcagcaau ucauguuuug ga                                               22


<210>  890
<211>  22
<212>  RNA
<213>  Danio rerio

<400>  890
aagcagcaca ucaauauugg ca                                              22


<210>  891
<211>  22
<212>  RNA
<213>  Danio rerio

<400>  891
aagcagcaca uaaauacugg ag                                              22


<210>  892
<211>  22
<212>  RNA
<213>  Homo sapiens

<400>  892
uaacacuguc ugguaaagau gg                                              22


<210>  893
<211>  22
<212>  RNA
<213>  Mus musculus

<400>  893
uaacacuguc ugguaaagau gg                                              22


<210>  894
<211>  22
<212>  RNA
<213>  Rattus norvegicus

<400>  894
uaacacuguc ugguaaagau gg                                              22


<210>  895
<211>  22
<212>  RNA
<213>  Monodelphis domestic

<400>  895
uaacacuguc ugguaaagau gc                                              22


<210>  896
<211>  22
<212>  RNA
<213>  Danio rerio

<400>  896
uaacacuguc ugguaacgau gc                                              22


<210>  897
<211>  22
<212>  RNA
<213>  Homo sapiens

<400>  897
auaagacgaa caaaagguuu gu                                              22


<210>  898

<211>  21
<212>  RNA
<213>  Danio rerio

<400>  898
guaagacgaa caaaaaguuu u                                                21


<210>  899
<211>  23
<212>  RNA
<213>  Homo sapiens

<400>  899
agcuacauug ucugcugggu uuc                                              23


<210>  900
<211>  23
<212>  RNA
<213>  Mus musculus

<400>  900
agcuacauug ucugcugggu uuc                                              23


<210>  901
<211>  23
<212>  RNA
<213>  Rattus norvegicus

<400>  901
agcuacauug ucugcugggu uuc                                              23


<210>  902
<211>  23
<212>  RNA
<213>  Macaca mulatta

<400>  902
agcuacauug ucugcugggu uuc                                              23


<210>  903
<211>  23
<212>  RNA
<213>  Gorilla gorilla

<400>  903
agcuacauug ucugcugggu uuc                                              23


<210>  904
<211>  23
<212>  RNA
<213>  Pan paniscus

<400>  904
agcuacauug ucugcugggu uuc                                              23


<210>  905
<211>  23
<212>  RNA
<213>  Pongo pygmaeus

<400>  905

agcuacauug ucugcugggu uuc                                                23

<210>  906
<211>  23
<212>  RNA
<213>  Monodelphis domestica

<400>  906
agcuacauug ucugcugggu uuc                                                23

<210>  907
<211>  23
<212>  RNA
<213>  Gallus gallus

<400>  907
agcuacauug ucugcugggu uuc                                                23

<210>  908
<211>  23
<212>  RNA
<213>  Xenopus tropicalis

<400>  908
agcuacauug ucugcugggu uuc                                                23

<210>  909
<211>  22
<212>  RNA
<213>  Bos taurus

<400>  909
agcuacauug ucugcugggu uu                                                 22

<210>  910
<211>  23
<212>  RNA
<213>  Danio rerio

<400>  910
agcuacauug ucugcugggu uuc                                                23

<210>  911
<211>  23
<212>  RNA
<213>  Fugu rubripes

<400>  911
agcuacauug ucugcugggu uuc                                                23

<210>  912
<211>  23
<212>  RNA
<213>  Tetraodon nigroviridis

<400>  912
agcuacauug ucugcugggu uuc                                                23

<210>  913
<211>  22

<212>  RNA
<213>  Homo sapiens

<400>  913
acuuuaacau ggaggcacuu gc                                              22


<210>  914
<211>  22
<212>  RNA
<213>  Homo sapiens

<400>  914
aacacaccua uucaaggauu ca                                              22


<210>  915
<211>  22
<212>  RNA
<213>  Mus musculus

<400>  915
aacacaccug uucaaggauu ca                                              22


<210>  916
<211>  19
<212>  RNA
<213>  Homo sapiens

<400>  916
agguugacau acguuuccc                                                  19


<210>  917
<211>  22
<212>  RNA
<213>  Homo sapiens

<400>  917
aggcagugua uuguuagcug gc                                              22


<210>  918
<211>  20
<212>  RNA
<213>  Mus musculus

<400>  918
aggcagugcg accuggcucg                                                 20


<210>  919
<211>  20
<212>  RNA
<213>  Homo sapiens

<400>  919
uaaggcacgc ggugaaugcc                                                 20


<210>  920
<211>  20
<212>  RNA
<213>  Mus musculus

<400>  920
uaaggcacgc ggugaaugcc                                                 20

<210>  921
<211>  20
<212>  RNA
<213>  Rattus norvegicus

<400>  921
uaaggcacgc ggugaaugcc                                                    20


<210>  922
<211>  22
<212>  RNA
<213>  Danio rerio

<400>  922
uaaggcacgc ggugaaugcc aa                                                 22


<210>  923
<211>  22
<212>  RNA
<213>  Fugu rubripes

<400>  923
uaaggcacgc ggugaaugcc aa                                                 22


<210>  924
<211>  22
<212>  RNA
<213>  Tetraodon nigroviridis

<400>  924
uaaggcacgc ggugaaugcc aa                                                 22


<210>  925
<211>  23
<212>  RNA
<213>  Homo sapiens

<400>  925
guuugcacgg gugggccuug ucu                                                23


<210>  926
<211>  22
<212>  RNA
<213>  Homo sapiens

<400>  926
gaaagcgcuu cccuuugcug ga                                                 22


<210>  927
<211>  24
<212>  RNA
<213>  Homo sapiens

<400>  927
acaaagugcu ucccuuuaga gugu                                               24


<210>  928
<211>  20
<212>  RNA

<213>  Homo sapiens

<400>  928
cuacaaaggg aagcccuuuc                                                    20


<210>  929
<211>  21
<212>  RNA
<213>  Homo sapiens

<400>  929
cuccagaggg aaguacuuuc u                                                  21


<210>  930
<211>  22
<212>  RNA
<213>  Homo sapiens

<400>  930
cucuagaggg aagcacuuuc ug                                                 22


<210>  931
<211>  22
<212>  RNA
<213>  Homo sapiens

<400>  931
cucuagaggg aagcgcuuuc ug                                                 22


<210>  932
<211>  22
<212>  RNA
<213>  Homo sapiens

<400>  932
cucuagaggg aagcacuuuc uc                                                 22


<210>  933
<211>  22
<212>  RNA
<213>  Homo sapiens

<400>  933
cucuagaggg aagcgcuuuc ug                                                 22


<210>  934
<211>  22
<212>  RNA
<213>  Homo sapiens

<400>  934
cucuagaggg aagcgcuuuc ug                                                 22


<210>  935
<211>  22
<212>  RNA
<213>  Homo sapiens

<400>  935
cucuagaggg aagcgcuuuc ug                                                 22

<210>  936
<211>  22
<212>  RNA
<213>  Homo sapiens

<400>  936
cucuagaggg aagcacuuuc ug                                                22

<210>  937
<211>  22
<212>  RNA
<213>  Homo sapiens

<400>  937
cucuagaggg aagcgcuuuc ug                                                22

<210>  938
<211>  22
<212>  RNA
<213>  Homo sapiens

<400>  938
cucuagaggg aagcgcuuuc ug                                                22

<210>  939
<211>  20
<212>  RNA
<213>  Homo sapiens

<400>  939
cugcaaaggg aagcccuuuc                                                   20

<210>  940
<211>  71
<212>  RNA
<213>  Homo sapiens

<400>  940
ugggaaacau acuucuuuau augcccauau ggaccugcua agcuauggaa uguaaagaag      60

uauguaucuc a                                                            71

<210>  941
<211>  85
<212>  RNA
<213>  Homo sapiens

<400>  941
accuacucag aguacauacu ucuuuaugua cccauaugaa cauacaaugc uauggaaugu      60

aaagaaguau guauuuuugg uaggc                                             85

<210>  942
<211>  86
<212>  RNA
<213>  Homo sapiens

<400>  942
ugcuucccga ggccacaugc uucuuuauau ccccauaugg auuacuuugc uauggaaugu      60

aaggaagugu gugguuucgg caagug                                            86

<210> 943
<211> 110
<212> RNA
<213> Homo sapiens

<400> 943
uuggauguug gccuaguucu guguggaaga cuagugauuu uguuguuuuu agauaacuaa 60

aucgacaaca aaucacaguc ugccauaugg cacaggccau gccucuacag 110


<210> 944
<211> 110
<212> RNA
<213> Homo sapiens

<400> 944
cuggauacag aguggaccgg cuggccccau cuggaagacu agugauuuug uuguugucuu 60

acugcgcuca acaacaaauc ccagucuacc uaauggugcc agccaucgca 110


<210> 945
<211> 110
<212> RNA
<213> Homo sapiens

<400> 945
agauuagagu ggcugugguc uagugcugug uggaagacua gugauuuugu uguucugaug 60

uacuacgaca acaagucaca gccggccuca uagcgcagac ucccuucgac 110


<210> 946
<211> 110
<212> RNA
<213> Homo sapiens

<400> 946
gaucugucug ucuucuguau auacccugua gauccgaauu uguguaagga auuuuguggu 60

cacaaauucg uaucuagggg aauauguagu ugacauaaac acuccgcucu 110


<210> 947
<211> 71
<212> RNA
<213> Homo sapiens

<400> 947
uguucuaagg ugcaucuagu gcagauagug aaguagauua gcaucuacug cccuaagugc 60

uccuucuggc a 71


<210> 948
<211> 71
<212> RNA
<213> Homo sapiens

<400> 948
uguguuaagg ugcaucuagu gcaguuagug aagcagcuua gaaucuacug cccuaaaugc 60

cccuucuggc a 71

<210>  949
<211>  69
<212>  RNA
<213>  Homo sapiens

<400>  949
aguaccaaag ugcucauagu gcagguaguu uuggcaugac ucuacuguag uaugggcacu      60

uccaguacu                                                              69


<210>  950
<211>  81
<212>  RNA
<213>  Homo sapiens

<400>  950
ccuuggccau guaaaagugc uuacagugca gguagcuuuu ugagaucuac ugcaauguaa      60

gcacuucuua cauuaccaug g                                                81


<210>  951
<211>  82
<212>  RNA
<213>  Homo sapiens

<400>  951
ccugccgggg cuaaagugcu gacagugcag auaguggucc ucuccgugcu accgcacugu      60

ggguacuugc ugcuccagca gg                                               82


<210>  952
<211>  68
<212>  RNA
<213>  Homo sapiens

<400>  952
cuccggugcc uacugagcug auaucaguuc ucauuuuaca cacuggcuca guucagcagg      60

aacaggag                                                               68


<210>  953
<211>  73
<212>  RNA
<213>  Homo sapiens

<400>  953
cucugccucc cgugccuacu gagcugaaac acaguugguu uguguacacu ggcucaguuc      60

agcaggaaca ggg                                                         73


<210>  954
<211>  84
<212>  RNA
<213>  Homo sapiens

<400>  954
ggccaguguu gagaggcgga gacuugggca auugcuggac gcugcccugg gcauugcacu      60

ugucucgguc ugacagugcc ggcc                                             84


<210>  955
<211>  70

<212>  RNA
<213>  Homo sapiens

<400>  955
   ggagauauug cacauuacua aguugcaugu ugucacggcc ucaaugcaau uuagugugug      60
   ugauauuuuc                                                              70


<210>  956
<211>  77
<212>  RNA
<213>  Homo sapiens

<400>  956
guggccucgu ucaaguaauc caggauaggc ugugcagguc ccaaugggcc uauucuuggu      60

uacuugcacg gggacgc                                                     77


<210>  957
<211>  84
<212>  RNA
<213>  Homo sapiens

<400>  957
ggcuguggcu ggauucaagu aauccaggau aggcuguuuc caucugugag gccuauucuu      60

gauuacuugu uucuggaggc agcu                                             84


<210>  958
<211>  77
<212>  RNA
<213>  Homo sapiens

<400>  958
ccgggaccca guucaaguaa uucaggauag guugugugcu guccagccug uucuccauua      60

cuuggcucgg ggaccgg                                                     77


<210>  959
<211>  97
<212>  RNA
<213>  Homo sapiens

<400>  959
accucucuaa caaggugcag agcuuagcug auuggugaac agugauuggu uuccgcuuug      60

uucacagugg cuaaguucug caccugaaga gaaggug                               97


<210>  960
<211>  86
<212>  RNA
<213>  Homo sapiens

<400>  960
gguccuugcc cucaaggagc ucacagucua uugaguuacc uuucugacuu ucccacuaga      60

uugugagcuc cuggagggca ggcacu                                           86


<210>  961
<211>  64
<212>  RNA
<213>  Homo sapiens

<400>  961
augacugauu ucuuuuggug uucagaguca auauaauuuu cuagcaccau cugaaaucgg      60

uuau                                                                  64


<210>  962
<211>  88
<212>  RNA
<213>  Homo sapiens

<400>  962
aucucuuaca caggcugacc gauuucuccu gguguucaga gucuguuuuu gucuagcacc      60

auuugaaauc gguuaugaug uaggggga                                        88


<210>  963
<211>  88
<212>  RNA
<213>  Homo sapiens

<400>  963
accaaguuuc aguucaugua aacauccuac acucagcugu aauacaugga uuggcuggga      60

gguggauguu uacuucagcu gacuugga                                        88


<210>  964
<211>  89
<212>  RNA
<213>  Homo sapiens

<400>  964
accaugcugu agugugugua aacauccuac acucucagcu gugagcucaa gguggcuggg      60

agaggguugu uuacuccuuc ugccaugga                                       89


<210>  965
<211>  72
<212>  RNA
<213>  Homo sapiens

<400>  965
agauacugua aacauccuac acucucagcu guggaaagua agaaagcugg gagaaggcug      60

uuuacucuuu cu                                                         72


<210>  966
<211>  80
<212>  RNA
<213>  Homo sapiens

<400>  966
cugggggcuc caaagugcug uucgugcagg uagugugauu acccaaccua cugcugagcu      60

agcacuuccc gagcccccgg                                                 80


<210>  967
<211>  68
<212>  RNA
<213>  Homo sapiens

<400>  967

ccucuacuuu aacauggagg cacuugcugu gacaugacaa aaauaagugc uuccauguuu 60

gagugugg 68

<210> 968
<211> 81
<212> RNA
<213> Homo sapiens

<400> 968
aacacagugg gcacucaaua aaugucuguu gaauugaaau gcguuacauu caacggguau 60

uuauugagca cccacucugu g 81

<210> 969
<211> 119
<212> RNA
<213> Homo sapiens

<400> 969
aggauucugc ucaugccagg gugagguagu aaguuguauu guuguggggu agggauauua 60

ggccccaauu agaagauaac uauacaacuu acuacuuucc cuggugugug gcauauuca 119

<210> 970
<211> 89
<212> RNA
<213> Homo sapiens

<400> 970
ugcugcuggc cagagcucuu uucacauugu gcuacugucu gcaccuguca cuagcagugc 60

aauguuaaaa gggcauuggc cguguagug 89

<210> 971
<211> 82
<212> RNA
<213> Homo sapiens

<400> 971
ggccugcccg acacucuuuc ccuguugcac uacuauaggc cgcugggaag cagugcaaug 60

augaaagggc aucggucagg uc 82

<210> 972
<211> 86
<212> RNA
<213> Homo sapiens

<400> 972
acugcuaacg aaugcucuga cuuuauugca cuacuguacu uuacagcuag cagugcaaua 60

guauugucaa agcaucugaa agcagg 86

<210> 973
<211> 101
<212> RNA
<213> Homo sapiens

<400> 973
ccgcccccgc gucuccaggg caaccguggc uuucgauugu uacuguggga acuggaggua 60

acagucuaca gccauggucg ccccgcagca cgcccacgcg c 101

<210> 974
<211> 110
<212> RNA
<213> Homo sapiens

<400> 974
cggggcaccc cgcccggaca gcgcgccggc accuuggcuc uagacugcuu acugcccggg 60

ccgcccucag uaacagucuc cagucacggc caccgacgcc uggccccgcc 110

<210> 975
<211> 88
<212> RNA
<213> Homo sapiens

<400> 975
acaaugcuuu gcuagagcug guaaaaugga accaaaucgc cucuucaaug gauuuggucc 60

ccuucaacca gcuguagcua ugcauuga 88

<210> 976
<211> 102
<212> RNA
<213> Homo sapiens

<400> 976
gggagccaaa ugcuuugcua gagcugguaa aauggaacca aaucgacugu ccaauggauu 60

ugguccccuu caaccagcug uagcugugca uugauggcgc cg 102

<210> 977
<211> 119
<212> RNA
<213> Homo sapiens

<400> 977
ccucagaaga aagaugcccc cugcucuggc uggucaaacg gaaccaaguc cgucuuccug 60

agagguuugg uccccuucaa ccagcuacag cagggcuggc aaugcccagu ccuuggaga 119

<210> 978
<211> 73
<212> RNA
<213> Homo sapiens

<400> 978
cagggugugu gacugguuga ccagaggggc augcacugug uucacccugu gggccaccua 60

gucaccaacc cuc 73

<210> 979
<211> 102
<212> RNA
<213> Homo sapiens

<400> 979
gguccucuga cucucuucgg ugacggguau ucuugggugg auaauacgga uuacguuguu 60

auugcuuaag aauacgcgua gucgaggaga guaccagcgg ca 102

<210>  980
<211>  87
<212>  RNA
<213>  Homo sapiens

<400>  980
gacagugcag ucacccauaa aguagaaagc acuacuaaca gcacuggagg guguaguguu      60

uccuacuuua uggaugagug uacugug                                          87


<210>  981
<211>  99
<212>  RNA
<213>  Homo sapiens

<400>  981
ccgaugugua uccucagcuu ugagaacuga auuccauggg uugugucagu gucagaccuc      60

ugaaauucag uucuucagcu gggauaucuc ugucaucgu                             99


<210>  982
<211>  72
<212>  RNA
<213>  Homo sapiens

<400>  982
aaucuaaaga caacauuucu gcacacacac cagacuaugg aagccagugu guggaaaugc      60

uucugcuaga uu                                                          72


<210>  983
<211>  89
<212>  RNA
<213>  Homo sapiens

<400>  983
gccggcgccc gagcucuggc uccgugucuu cacucccgug cuuguccgag gagggaggga      60

gggacggggg cugugcuggg gcagcugga                                        89


<210>  984
<211>  110
<212>  RNA
<213>  Homo sapiens

<400>  984
cggaaaauuu gccaaggguu ugggggaaca uucaaccugu cggugaguuu gggcagcuca      60

ggcaaaccau cgaccguuga guggacccug aggccuggaa uugccauccu                110


<210>  985
<211>  137
<212>  RNA
<213>  Homo sapiens

<400>  985
guccccuccc cuaggccaca gccgagguca caaucaacau ucauuguugu cgguggguug      60

ugaggacuga ggccagaccc accgggggau gaaugucacu guggcugggc cagacacggc     120

uuaaggggaa uggggac                                                    137

<210>  986
<211>  110
<212>  RNA
<213>  Homo sapiens

<400>  986
gagcugcuug ccuccccccg uuuuuggcaa ugguagaacu cacacuggug agguaacagg      60

auccgguggu ucuagacuug ccaacuaugg ggcgaggacu cagccggcac      110


<210>  987
<211>  110
<212>  RNA
<213>  Homo sapiens

<400>  987
ccgcagagug ugacuccugu ucuguguaug gcacugguag aauucacugu gaacagucuc      60

agucagugaa uuaccgaagg gccauaaaca gagcagagac agauccacga      110


<210>  988
<211>  84
<212>  RNA
<213>  Homo sapiens

<400>  988
ccagucacgu ccccuuauca cuuuuccagc ccagcuuugu gacuguaagu guuggacgga      60

gaacugauaa ggguagguga uuga      84


<210>  989
<211>  110
<212>  RNA
<213>  Homo sapiens

<400>  989
gccgagaccg agugcacagg gcucugaccu augaauugac agccagugcu cucgucuccc      60

cucuggcugc caauuccaua ggucacaggu auguucgccu caaugccagc      110


<210>  990
<211>  110
<212>  RNA
<213>  Homo sapiens

<400>  990
aucauucaga aaugguauac aggaaaauga ccuaugaauu gacagacaau auagcugagu      60

uugucuguca uuucuuuagg ccaauauucu guaugacugu gcuacuucaa      110


<210>  991
<211>  88
<212>  RNA
<213>  Homo sapiens

<400>  991
cgaggauggg agcugagggc ugggucuuug cgggcgagau gagggugucg gaucaacugg      60

ccuacaaagu cccaguucuc ggcccccg      88


<210>  992

<211>  83
<212>  RNA
<213>  Homo sapiens

<400>  992
guggucucag aaucgggguu uugagggcga gaugaguuua uguuuuaucc aacuggcccu      60

caaagucccg cuuuuggggu cau                                              83


<210>  993
<211>  87
<212>  RNA
<213>  Homo sapiens

<400>  993
agcuucccug gcucuagcag cacagaaaua uuggcacagg gaagcgaguc ugccaauauu      60

ggcugugcug cuccaggcag gguggug                                          87


<210>  994
<211>  98
<212>  RNA
<213>  Homo sapiens

<400>  994
cgaggggaua cagcagcaau ucauguuuug aaguguucua aaugguucaa aacgugaggc      60

gcugcuauac ccccucgugg ggaagguaga aggugggg                              98


<210>  995
<211>  112
<212>  RNA
<213>  Homo sapiens

<400>  995
ccaccccggu ccugcucccg ccccagcagc acacuguggu uuguacggca cuguggccac      60

guccaaacca cacuguggug uuagagcgag ggugggggag gcaccgccga gg            112


<210>  996
<211>  70
<212>  RNA
<213>  Homo sapiens

<400>  996
gugaauuagg uaguuucaug uuguugggcc uggguuucug aacacaacaa cauuaaacca      60

cccgauucac                                                             70


<210>  997
<211>  110
<212>  RNA
<213>  Homo sapiens

<400>  997
ugcucgcuca gcugaucugu ggcuuaggua guuucauguu guugggauug aguuuugaac      60

ucggcaacaa gaaacugccu gaguuacauc agucgguuuu cgucgagggc                110


<210>  998
<211>  84
<212>  RNA

<213>  Homo sapiens

<400>  998
acuggucggu gauuuaggua guuuccuguu guugggaucc accuuucucu cgacagcacg      60

acacugccuu cauuacuuca guug                                              84


<210>  999
<211>  75
<212>  RNA
<213>  Homo sapiens

<400>  999
ggcugugccg gguagagagg gcagugggag guaagagcuc uucacccuuc accaccuucu      60

ccacccagca uggcc                                                        75


<210>  1000
<211>  71
<212>  RNA
<213>  Homo sapiens

<400>  1000
gccaacccag uguucagacu accuguucag gaggcucuca auguguacag uagucugcac      60

auugguuagg c                                                            71


<210>  1001
<211>  110
<212>  RNA
<213>  Homo sapiens

<400>  1001
aggaagcuuc uggagauccu gcuccgucgc cccaguguuc agacuaccug uucaggacaa      60

ugccguugua caguagucug cacauugguu agacugggca agggagagca                 110


<210>  1002
<211>  90
<212>  RNA
<213>  Homo sapiens

<400>  1002
ccgggccccu gugagcaucu uaccggacag ugcuggauuu cccagcuuga cucuaacacu      60

gucugguaac gauguucaaa ggugacccgc                                        90


<210>  1003
<211>  110
<212>  RNA
<213>  Homo sapiens

<400>  1003
cgccucagag ccgcccgccg uuccuuuuuc cuaugcauau acuucuuuga ggaucuggcc      60

uaaagaggua uagggcaugg gaaaacgggg cggucggguc cuccccagcg                 110


<210>  1004
<211>  110
<212>  RNA
<213>  Homo sapiens

<400>  1004
ggcuacaguc uuucuucaug ugacucgugg acuucccuuu gucauccuau gccugagaau      60

auaugaagga ggcugggaag gcaaagggac guucaauugu caucacuggc                110

<210>  1005
<211>  110
<212>  RNA
<213>  Homo sapiens

<400>  1005
ucaccuggcc augugacuug ugggcuuccc uuugucaucc uucgccuagg gcucugagca      60

gggcagggac agcaaagggg ugcucaguug ucacuuccca cagcacggag                110

<210>  1006
<211>  110
<212>  RNA
<213>  Homo sapiens

<400>  1006
aaagauccuc agacaaucca ugugcuucuc uuguccuuca uuccaccgga gucugucuca      60

uacccaacca gauuucagug gagugaaguu caggaggcau ggagcugaca                110

<210>  1007
<211>  71
<212>  RNA
<213>  Homo sapiens

<400>  1007
ugacgggcga gcuuuuggcc cggguuauac cugaugcuca cguauaagac gagcaaaaag      60

cuuguugguc a                                                           71

<210>  1008
<211>  110
<212>  RNA
<213>  Homo sapiens

<400>  1008
gcugcuggaa gguguaggua cccucaaugg cucaguagcc aguguagauc cugucuuucg      60

uaaucagcag cuacaucugg cuacuggguc ucugauggca ucuucuagcu                110

<210>  1009
<211>  63
<212>  RNA
<213>  Homo sapiens

<400>  1009
aagaaauggu uuaccguccc acauacauuu ugaauaugua ugugggaugg uaaaccgcuu      60

cuu                                                                    63

<210>  1010
<211>  69
<212>  RNA
<213>  Homo sapiens

<400>  1010
ccaccacuua aacguggaug uacuugcuuu gaaacuaaag aaguaagugc uuccauguuu      60

uggugaugg 69

<210> 1011
<211> 73
<212> RNA
<213> Homo sapiens

<400> 1011
gcucccuuca acuuuaacau ggaagugcuu ucugugacuu uaaaaguaag ugcuuccaug 60

uuuuaguagg agu 73

<210> 1012
<211> 83
<212> RNA
<213> Homo sapiens

<400> 1012
   cugacuaugc cuccccgcau ccccuagggc auugguguaa agcuggagac ccacugcccc 60
   aggugcugcu ggggguugua guc 83

<210> 1013
<211> 80
<212> RNA
<213> Homo sapiens

<400> 1013
gguaccugaa gagagguuuu cuggguuucu guuucuuuaa ugaggacgaa acacaccugg 60

uuaaccucuu uuccaguauc 80

<210> 1014
<211> 84
<212> RNA
<213> Homo sapiens

<400> 1014
gugguaccug aagagagguu uucuggguuu cuguuucuuu auugaggacg aaacacaccu 60

gguuaaccuc uuuuccagua ucaa 84

<210> 1015
<211> 94
<212> RNA
<213> Homo sapiens

<400> 1015
gaguuugguu uuguuugggu uuguucuagg uaugguccca gggaucccag aucaaaccag 60

gccccugggc cuauccuaga accaaccuaa gcuc 94

<210> 1016
<211> 93
<212> RNA
<213> Homo sapiens

<400> 1016
guagucagua guuggggggu gggaacggcu ucauacagga guugaugcac aguuauccag 60

cuccuauaug augccuuucu ucauccccuu caa 93

<210>  1017
<211>  95
<212>  RNA
<213>  Homo sapiens

<400>  1017
uuguaccugg ugugauuaua aagcaaugag acugauuguc auaugucguu ugugggaucc      60

gucucaguua cuuuauagcc auaccuggua ucuua                                 95


<210>  1018
<211>  99
<212>  RNA
<213>  Homo sapiens

<400>  1018
gaaacugggc ucaaggugag gggugcuauc ugugauugag ggacaugguu aauggaauug      60

ucucacacag aaaucgcacc cgucaccuug gccuacuua                             99


<210>  1019
<211>  86
<212>  RNA
<213>  Homo sapiens

<400>  1019
gucugucugc ccgcaugccu gccucucugu ugcucugaag gaggcagggg cugggccugc      60

agcugccugg gcagagcggc uccugc                                           86


<210>  1020
<211>  65
<212>  RNA
<213>  Homo sapiens

<400>  1020
cuugaauccu uggaaccuag gugugagugc uauuucagug caacacaccu auucaaggau      60

ucaaa                                                                  65


<210>  1021
<211>  66
<212>  RNA
<213>  Homo sapiens

<400>  1021
aaaaggugga uauuccuucu auguuuaugu uauuuauggu uaaacauaga ggaaauucca      60

cguuuu                                                                 66


<210>  1022
<211>  67
<212>  RNA
<213>  Homo sapiens

<400>  1022
guggcacuca aacugugggg gcacuuucug cucucuggug aaagugccgc caucuuuuga      60

guguuac                                                                67

<210>  1023
<211>  68
<212>  RNA
<213>  Homo sapiens

<400>  1023
uaaaagguag auucuccuuc uaugaguaca uuauuuauga uuaaucauag aggaaaaucc      60

acguuuuc                                                              68


<210>  1024
<211>  80
<212>  RNA
<213>  Homo sapiens

<400>  1024
gguauuuaaa agguagauuu uccuucuaug guuacguguu ugaugguuaa ucauagagga      60

aaauccacgu uuucaguauc                                                 80


<210>  1025
<211>  100
<212>  RNA
<213>  Homo sapiens

<400>  1025
caguccuucu uugguauuua aaacguggau auuccuucua uguuuacgug auuccugguu      60

aaucauagag gaaaauccau guuuucagua ucaaaugcug                          100


<210>  1026
<211>  69
<212>  RNA
<213>  Homo sapiens

<400>  1026
uugagcagag guugcccuug gugaauucgc uuuauuuaug uugaaucaca caaaggcaac      60

uuuuguuug                                                             69


<210>  1027
<211>  66
<212>  RNA
<213>  Homo sapiens

<400>  1027
agggcuccug acuccagguc cuguguguua ccuagaaaua gcacuggacu uggagucaga      60

aggccu                                                                66


<210>  1028
<211>  61
<212>  RNA
<213>  Homo sapiens

<400>  1028
aagaugguug accauagaac augcgcuauc ucugugucgu auguaauaug guccacaucu      60

u                                                                     61


<210>  1029
<211>  76

<212>  RNA
<213>  Homo sapiens

<400>  1029
uacuugaaga gaaguuguuc gugguggauu cgcuuuacuu augacgaauc auucacggac      60

aacacuuuuu ucagua                                                      76


<210>  1030
<211>  91
<212>  RNA
<213>  Homo sapiens

<400>  1030
cuggguuacg gggauggaug gucgaccagu uggaaaguaa uuguuucuaa uguacuucac      60

cugguccacu agccguccgu auccgcugca g                                     91


<210>  1031
<211>  90
<212>  RNA
<213>  Homo sapiens

<400>  1031
gagagaagca cuggacuuag ggucagaagg ccugagucuc ucugcugcag augggcucuc      60

ugucccugag ccaagcuuug uccucccugg                                       90


<210>  1032
<211>  94
<212>  RNA
<213>  Homo sapiens

<400>  1032
auaaaggaag uuaggcugag gggcagagag cgagacuuuu cuauuuucca aaagcucggu      60

cugaggcccc ucagucuugc uuccuaaccc gcgc                                  94


<210>  1033
<211>  114
<212>  RNA
<213>  Homo sapiens

<400>  1033
uccugcuugu ccugcgaggu gucuugcagg ccgucaugca ggccacacug acgguaacgu      60

ugcaggucgu cuugcagggc uucucgcaag acgacauccu caucaccaac gacg           114


<210>  1034
<211>  94
<212>  RNA
<213>  Homo sapiens

<400>  1034
ugacuccucc aggucuugga guaggucauu ggguggaucc ucuauuuccu uacgugggcc      60

acuggauggc uccuccaugu cuuggaguag auca                                  94


<210>  1035
<211>  93
<212>  RNA
<213>  Homo sapiens

<400>  1035
ccggggagaa guacggugag ccugucauua uucagagagg cuagauccuc uguguugaga      60

aggaucauga ugggcuccuc gguguucucc agg                                   93


<210>  1036
<211>  91
<212>  RNA
<213>  Homo sapiens

<400>  1036
cuguguguga ugagcuggca guguauuguu agcugguuga auaugugaau ggcaucggcu      60

aacaugcaac ugcugucuua uugcauauac a                                     91


<210>  1037
<211>  72
<212>  RNA
<213>  Homo sapiens

<400>  1037
cuugggaaug gcaaggaaac cguuaccauu acugaguuua guaaugguaa ugguucucuu      60

gcuauaccca ga                                                          72


<210>  1038
<211>  85
<212>  RNA
<213>  Homo sapiens

<400>  1038
gcuaagcacu uacaacuguu ugcagaggaa acugagacuu uguaacuaug ucucagucuc      60

aucugcaaag aaguaagugc uuugc                                            85


<210>  1039
<211>  96
<212>  RNA
<213>  Homo sapiens

<400>  1039
ucccuggcgu gaggguaugu gccuuuggac uacaucgugg aagccagcac caugcagucc      60

augggcauau acacuugccu caaggccuau gucauc                                96


<210>  1040
<211>  76
<212>  RNA
<213>  Homo sapiens

<400>  1040
gagggggaag acgggaggaa agaagggagu gguuccauca cgccuccuca cuccucuccu      60

cccgucuucu ccucuc                                                      76


<210>  1041
<211>  73
<212>  RNA
<213>  Homo sapiens

<400>  1041

acuuggagag aggcuggccg ugaugaauuc gauucaucaa agcgagucau acacggcucu 60

ccucucuuuu agu 73

<210> 1042
<211> 83
<212> RNA
<213> Homo sapiens

<400> 1042
gagaaucauc ucucccagau aauggcacuc ucaaacaagu uuccaaauug uuugaaaggc 60

uauuucuugg ucagaugacu cuc 83

<210> 1043
<211> 84
<212> RNA
<213> Homo sapiens

<400> 1043
guggcagcuu gguggucgua ugugugacgc cauuuacuug aaccuuuagg agugacauca 60

cauauacggc agcuaaacug cuac 84

<210> 1044
<211> 84
<212> RNA
<213> Homo sapiens

<400> 1044
uugacuuagc uggguagugg ggaacccuuc caugaggagu agaacacucc uuaugcaaga 60

uucccuucua ccuggcuggg uugg 84

<210> 1045
<211> 89
<212> RNA
<213> Homo sapiens

<400> 1045
cuggccucca gggcuuugua caugguaggc uuucauucau ucguuugcac auucggugaa 60

ggucuacugu gugccaggcc cugugccag 89

<210> 1046
<211> 81
<212> RNA
<213> Homo sapiens

<400> 1046
gauacucgaa ggagagguug uccguguugu cuucucuuua uuuaugauga aacauacacg 60

ggaaaccucu uuuuuaguau c 81

<210> 1047
<211> 84
<212> RNA
<213> Homo sapiens

<400> 1047
gcucuuccuc ucuaauccuu ugucccuggg ugagagugcu uucugaaugc aaugcacccg 60

ggcaaggauu cugagagggu gagc 84

<210> 1048
<211> 83
<212> RNA
<213> Homo sapiens

<400> 1048
gcugcuguug ggagacccug gucugcacuc uaucuguauu cuuacugaag ggagugcagg 60

gcaggguuuc ccauacagag ggc 83

<210> 1049
<211> 84
<212> RNA
<213> Homo sapiens

<400> 1049
gaugcaccca gugggggagc caggaaguau ugauguuucu gccaguuuag cgucaacacu 60

ugcugguuuc cucucuggag cauc 84

<210> 1050
<211> 124
<212> RNA
<213> Homo sapiens

<400> 1050
gccaccacca ucagccauac uauguguagu gccuuauuca ggaagguguu acuuaauaga 60

uuaauauuug uaaggcaccc uucugaguag aguaaugugc aacauggaca acauuugugg 120

uggc 124

<210> 1051
<211> 94
<212> RNA
<213> Homo sapiens

<400> 1051
gugcugugug uagugcuuca cuucaagaag ugccaugcau gugucuagaa auauguuuug 60

caccuuuugg agugaaauaa ugcacaacag auac 94

<210> 1052
<211> 94
<212> RNA
<213> Homo sapiens

<400> 1052
caugcugugu gugguacccu acugcagaca guggcaauca uguauaauua aaaaugauug 60

guacgucugu ggguagagua cugcaugaca caug 94

<210> 1053
<211> 74
<212> RNA
<213> Homo sapiens

<400> 1053
gugguguccu acucaggaga guggcaauca cauguaauua ggugugauug aaaccucuaa 60

gaguggagua acac                                                    74

<210>  1054
<211>  87
<212>  RNA
<213>  Homo sapiens

<400>  1054
caauagacac ccaucguguc uuuugcucug cagucaguaa auauuuuuuu gugaaugugu      60

agcaaaagac agaauggugg uccauug                                          87

<210>  1055
<211>  87
<212>  RNA
<213>  Homo sapiens

<400>  1055
caauagacac ccaucguguc uuuugcucug cagucaguaa auauuuuuuu gugaaugugu      60

agcaaaagac agaauggugg uccauug                                          87

<210>  1056
<211>  84
<212>  RNA
<213>  Homo sapiens

<400>  1056
ucucagucug uggcacucag ccuugagggc acuuucuggu gccagaauga aagugcuguc      60

auagcugagg uccaaugacu gagg                                             84

<210>  1057
<211>  98
<212>  RNA
<213>  Homo sapiens

<400>  1057
gguacuucuc agucuguggc acucagccuu gagggcacuu ucuggugcca gaaugaaagu      60

gcugucauag cugaggucca augacugagg cgagcacc                              98

<210>  1058
<211>  129
<212>  RNA
<213>  Homo sapiens

<400>  1058
   gggaugccac auucagccau ucagcguaca gugccuuuca cagggaggug ucauuuaugu      60
   gaacuaaaau auaaauuuca ccuuucugag aaggguaaug uacagcaugc acugcauaug     120
   uggugaccc                                                            129

<210>  1059
<211>  127
<212>  RNA
<213>  Homo sapiens

<400>  1059
ggaugccaca uucagccauu cagugugcag ugccuuucac agggaggugu cauuuaugug      60

aacuaaaaua uaaauuucac cuuucugaga aggguaaugu acagcaugca cugcauaugu    120

ggugucc                                                              127

<210>  1060
<211>  90
<212>  RNA
<213>  Homo sapiens

<400>  1060
ucucaggcug ugaccuucuc gaggaaagaa gcacuuucug uugucugaaa gaaaagaaag     60

ugcuuccuuu cagaggguua cgguuugaga                                      90

<210>  1061
<211>  90
<212>  RNA
<213>  Homo sapiens

<400>  1061
ucucagguug ugaccuucuc gaggaaagaa gcacuuucug uugucugaaa gaaaagaaag     60

ugcuuccuuu cagaggguua cgguuugaga                                      90

<210>  1062
<211>  85
<212>  RNA
<213>  Homo sapiens

<400>  1062
ucucaugaug ugaccaucug gagguaagaa gcacuuugug uuuugugaaa gaaagugcuu     60

ccuuucagag gguuacucuu ugaga                                           85

<210>  1063
<211>  90
<212>  RNA
<213>  Homo sapiens

<400>  1063
ucucaggcug ugaccaucug gagguaagaa gcacuuucug uuuugugaaa gaaaagaaag     60

ugcuuccuuu cagaggguua cucuuugaga                                      90

<210>  1064
<211>  87
<212>  RNA
<213>  Homo sapiens

<400>  1064
ucucaggcag ugacccucua gauggaagca cugucuguug uauaaaagaa aagaucgugc     60

aucccuuuag aguguuacug uuugaga                                         87

<210>  1065
<211>  67
<212>  RNA
<213>  Homo sapiens

<400>  1065
gugacccucu agauggaagc acugucuguu gucuaagaaa agaucgugca ucccuuuaga     60

guguuac                                                            67

<210>  1066
<211>  95
<212>  RNA
<213>  Homo sapiens

<400>  1066
gaagaucuca ggcagugacc cucuagaugg aagcacuguc uguugucuaa gaaaagaucg      60

ugcauccuuu uagaguguua cuguuugaga aaauc                                 95

<210>  1067
<211>  83
<212>  RNA
<213>  Homo sapiens

<400>  1067
ucaugcugug gcccuccaga gggaagcgcu uucuguuguc ugaaagaaaa caaagcgcuc      60

cccuuuagag guuuacgguu uga                                              83

<210>  1068
<211>  101
<212>  RNA
<213>  Homo sapiens

<400>  1068
gcgagaagau cucaugcugu gacucucugg agggaagcac uuucuguugu cugaaagaaa      60

acaaagcgcu ucucuuuaga guguuacggu uugagaaaag c                         101

<210>  1069
<211>  87
<212>  RNA
<213>  Homo sapiens

<400>  1069
ucccaugcug ugacccucua gagggaagca cuuucuguug ucugaaagaa accaaagcgc      60

uucccuuugg agcguuacgg uuugaga                                          87

<210>  1070
<211>  88
<212>  RNA
<213>  Homo sapiens

<400>  1070
ucucaggcug ugacccucua gagggaagcg cuuucuguug gcuaaaagaa aagaaagcgc      60

uucccuucag aguguuaacg cuuugaga                                         88

<210>  1071
<211>  87
<212>  RNA
<213>  Homo sapiens

<400>  1071
ucucaugcug ugacccucua gagggaagca cuuucucuug ucuaaaagaa aagaaagcgc      60

uucucuuuag aggauuacuc uuugaga                                          87

<210>  1072
<211>  88
<212>  RNA
<213>  Homo sapiens

<400>  1072
ucccaugcug ugacccucua gaggaagcac uuucuguuug uugucugaga aaaaacaaag      60

ugcuucccuu uagaguuacu guuuggga                                        88


<210>  1073
<211>  87
<212>  RNA
<213>  Homo sapiens

<400>  1073
ucucaugcug ugacccucua gagggaagcg cuuucuguug ucugaaagaa aagaacgcgc      60

uucccuauag aggguuaccc uuugaga                                         87


<210>  1074
<211>  87
<212>  RNA
<213>  Homo sapiens

<400>  1074
ucucaugcug ugacccuaca aagggaagca cuuucucuug uccaaaggaa aagaaggcgc      60

uucccuuugg aguguuacgg uuugaga                                         87


<210>  1075
<211>  85
<212>  RNA
<213>  Homo sapiens

<400>  1075
cucaagcugu gacucuccag agggaugcac uuucucuuau gugaaaaaaa agaaggcgcu      60

ucccuuuaga gcguuacggu uuggg                                           85


<210>  1076
<211>  85
<212>  RNA
<213>  Homo sapiens

<400>  1076
cucaggcugu gacccucuag agggaagcac uuucuguugc uugaaagaag agaaagcgcu      60

uccuuuuaga ggauuacucu uugag                                           85


<210>  1077
<211>  65
<212>  RNA
<213>  Homo sapiens

<400>  1077
gugacccucu agagggaagc acuuucuguu gaaagaaaag aacaugcauc cuuucagagg      60

guuac                                                                 65


<210>  1078

<211>  85
<212>  RNA
<213>  Homo sapiens

<400>  1078
ucucaagcug ugacugcaaa gggaagcccu uucuguuguc uaaaagaaaa gaaagugcuu      60

cccuuuggug aauuacgguu ugaga                                            85


<210>  1079
<211>  77
<212>  RNA
<213>  Mus musculus

<400>  1079
gcuugggaca cauacuucuu uauaugccca uaugaaccug cuaagcuaug gaauguaaag      60

aaguauguau uucaggc                                                     77


<210>  1080
<211>  72
<212>  RNA
<213>  Mus musculus

<400>  1080
ucagagcaca uacuucuuua uguacccaua ugaacauuca gugcuaugga auguaaagaa      60

guauguauuu ug                                                          72


<210>  1081
<211>  87
<212>  RNA
<213>  Rattus norvegicus

<400>  1081
ugccuacuca gagcacauac uucuuuaugu acccauauga acauagaaug cuauggaaug      60

uaaagaagug uguauuuugg guaggua                                          87


<210>  1082
<211>  85
<212>  RNA
<213>  Pan paniscus

<400>  1082
accuacucag aguacauacu ucuuuaugua cccauaugaa cauacaaugc uauggaaugu      60

aaagaaguau guauuuuugg uaggc                                            85


<210>  1083
<211>  86
<212>  RNA
<213>  Monodelphis domestica

<400>  1083
accuacucag aguacauacu ucuuuaugua cccauaugaa cauacaaugc uauggaaugu      60

aaagaaguau guauuuuugg uaggug                                           86


<210>  1084
<211>  71
<212>  RNA

<213>  Gallus gallus

<400>  1084
ugagagacau acuucuuuau augcccauau gaaccuggca aucuauggaa uguaaagaag      60

uauguauuuc a                                                           71


<210>  1085
<211>  85
<212>  RNA
<213>  Gallus gallus

<400>  1085
accugcucag agcacauacu ucuuuaugua cccauaugaa cauacaaugc uauggaaugu      60

aaagaaguau guauuuuugg caggc                                            85


<210>  1086
<211>  64
<212>  RNA
<213>  Gallus gallus

<400>  1086
acauacuucu ucauaugccc auauggaguc ggccggcguu auggaauguu aagaaguaug      60

uauc                                                                   64


<210>  1087
<211>  62
<212>  RNA
<213>  Xenopus tropicalis

<400>  1087
gcacauacuu cuuuauaugc ccauacugaa ugaaaaugcu auggaauguu aagaaguaug      60

ua                                                                     62


<210>  1088
<211>  106
<212>  RNA
<213>  Danio rerio

<400>  1088
gcccauauau cccgcuuggu agacauacuu cuuuauaugc ccauaugaac aagagcagcu      60

auggaaugua aagaaguaug uaucccaggu gagagaaaaa cggggc                    106


<210>  1089
<211>  87
<212>  RNA
<213>  Danio rerio

<400>  1089
gccuacuugg uguacauacu ucuuuaugug cccauaugaa cauauaaaag cuauggaaug      60

uaaagaagua uguauucuug gucaggu                                          87


<210>  1090
<211>  76
<212>  RNA
<213>  Fugu rubripes

<400>  1090
cuuaguguac auacuucuuu auguacccau auggacauau gauaacuaug gaauguaaag      60

aaguauguau ucuugg                                                      76


<210>  1091
<211>  92
<212>  RNA
<213>  Tetraodon nigroviridis

<400>  1091
auuaccuccu uaguguacau acuucuuuau guacccauau gaacauauga uaacuaugga      60

auguaaagaa guauguauuc uuggugaggu ga                                    92


<210>  1092
<211>  73
<212>  RNA
<213>  Mus musculus

<400>  1092
ccaggccaca ugcuucuuua uauccucaua gauaucucag cacuauggaa uguaaggaag      60

ugugugguuu ugg                                                         73


<210>  1093
<211>  84
<212>  RNA
<213>  Rattus norvegicus

<400>  1093
cuuccccagg ccacaugcuu cuuuauaucc ucauagauau cacugcgcua uggaauguaa      60

ggaagugugu gguuuuggca agug                                             84


<210>  1094
<211>  86
<212>  RNA
<213>  Macaca nemestrina

<400>  1094
ugcuucccga ggccacaugc uucuuuauau ccccauaugg auuacuuugc uauggaaugu      60

aaggaagugu gugguuucgg caagug                                           86


<210>  1095
<211>  86
<212>  RNA
<213>  Pongo pygmaeus

<400>  1095
ugcuucccga ggccacaugc uucuuuauau ccccauaugg auuacuuugc uauggaaugu      60

aaggaagugu gugguuucgg caagug                                           86


<210>  1096
<211>  76
<212>  RNA
<213>  Monodelphis domestica

<400>  1096
gaggcaacau gcuucuuuau auccccauau gaauuaugcu gcuauggaau guaaggaagu      60

gugugguuuc gggaag                                                    76

<210>  1097
<211>  76
<212>  RNA
<213>  Gallus gallus

<400>  1097
gagaugacau gcuucuuuau auccccauau ggauuaggcu gcuauggaau guaaggaagu      60

gugugguuuc agggag                                                    76

<210>  1098
<211>  70
<212>  RNA
<213>  Xenopus tropicalis

<400>  1098
gaggucacau gcuucuuuau auacccauau gaacuacauu guuauggaau guaaggaagu      60

guguggcuuc                                                           70

<210>  1099
<211>  99
<212>  RNA
<213>  Danio rerio

<400>  1099
gaauguugcc ucuugugaag acaugcuucc uuauaugccc auauuaaugc ucaaguuaug      60

gaauguaagg aagugugugg uuucaggggg aaauuuguc                            99

<210>  1100
<211>  109
<212>  RNA
<213>  Danio rerio

<400>  1100
gaguuggcug uugcuuauug agacgacaua cuuccuuaua uccccauauu cagaauuaau      60

cuauggaaug uaaggaagug uguggcuuca gugagaucuc aucaucauc                109

<210>  1101
<211>  111
<212>  RNA
<213>  Mus musculus

<400>  1101
aggagcggag uacgugagcc agugcuaugu ggaagacuug ugauuuuguu guucugauau      60

gauaugacaa caagucacag ccagccucau agcguggacu ccuaucaccu u             111

<210>  1102
<211>  97
<212>  RNA
<213>  Rattus norvegicus

<400>  1102
uguuggccua guucugugug gaagacuagu gauuuuguug uuuuuagaua acuaagacga      60

caacaaauca cagucugcca uauggcacag gccaccu                              97

<210>  1103
<211>  95
<212>  RNA
<213>  Rattus norvegicus

<400>  1103
ggacagacca gcccugucug gaagacuagu gauuuuguug uugugucugu guccaacaac      60

aagucccagu cugccacaug guguugguca cauca                                95


<210>  1104
<211>  110
<212>  RNA
<213>  Rattus norvegicus

<400>  1104
aggccagaac acaugagcca augcuaugug gaagacuugu gauuuuguug uucugauaug      60

auaugacaac aagucacagc cagccucaua gaguggacuc ccaucaccuu                110


<210>  1105
<211>  110
<212>  RNA
<213>  Lagothrix lagotricha

<400>  1105
uuggauguug gccuaguucu guguggaaga cuagugauuu uguuguuuuu agauaacuaa      60

aucgacaaca aaucacaguc ugccauaugg cacaggccau gccucuacag                110


<210>  1106
<211>  110
<212>  RNA
<213>  Saguinus labiatus

<400>  1106
uuggauauug gccuaguucu guguggaaga cuagugauuu uguuguuuuu agauaauuaa      60

aucgacaaca aaucacaguc ugccauaugg cacaggccau gccucuacag                110


<210>  1107
<211>  110
<212>  RNA
<213>  Macaca nemestrina

<400>  1107
uuggauguug gccuaguucu guguggaaga cuagugauuu uguuguuuuu agauaacuaa      60

auugacaaca aaucacaguc ugccauaugg cacaggccau gccucuacag                110


<210>  1108
<211>  110
<212>  RNA
<213>  Macaca nemestrina

<400>  1108
cuggauacag agugaagugg cuggccccgu cuggaagacu agugauuuug uuguugucuu      60

acugcgcuca acaacgaauc ccagucugcc gaauggugcc agccauugca                110

<210>  1109
<211>  110
<212>  RNA
<213>  Gorilla gorilla

<400>  1109
uuggauguug gccuaguucu guguggaaga cuagugauuu uguuguuuuu agauaacuaa      60

aucgacaaca aaucacaguc ugccauaugg cacaggccau gccucuacag                110


<210>  1110
<211>  110
<212>  RNA
<213>  Gorilla gorilla

<400>  1110
cugcauacag aguggaccgg cuggccccau cuggaagacu agugauuuug uuguugucuu      60

acugcgcuca acaacaaauc ccagucugcc uaauggugcc agccaucgca                110


<210>  1111
<211>  110
<212>  RNA
<213>  Gorilla gorilla

<400>  1111
agacuggagu ggcugugguc uagugcugug uggaagacua gugauuuugu uguucugaug      60

uacuacgaca acaagucaca gccggccuca cagcgcagac ucccuucgac                110


<210>  1112
<211>  110
<212>  RNA
<213>  Pan paniscus

<400>  1112
uuggauguug gccuaguucu guguggaaga cuagugauuu uguuguuuuu agauaacuaa      60

aucgacaaca aaucacaguc ugccauaugg cacaggccau gccucuacag                110


<210>  1113
<211>  110
<212>  RNA
<213>  Pan paniscus

<400>  1113
cuggauacag agcggaccag cuggccccau cuggaagacu agugauuuug uuguugucuu      60

acugcgcuca acaacaaauc ccagucuacc uaauggugcc agccaucaca                110


<210>  1114
<211>  110
<212>  RNA
<213>  Pan paniscus

<400>  1114
agauuagagu ggcugugguc uagugcugug uggaagacua gugauuuugu uguucugaug      60

uacuacgaca acaagucaca gccggccuca uagcgcagac ucccuucgac                110


<210>  1115
<211>  110

<212>  RNA
<213>  Pan troglodytes

<400>  1115
agauuagagu ggcugugguc uagugcugug uggaagacua gugauuuugu uguucugaug      60

uacuacgaca acaagucaca gccggccuca uagcgcagac ucccuucgac               110


<210>  1116
<211>  110
<212>  RNA
<213>  Pongo pygmaeus

<400>  1116
uuggauguug gccuaguucu guguggaaga cuagugauuu uguuguuuuu agauaacuaa      60

aucgacaaca aaucacaguc ugccauaugg cacaggccau gccucuacag               110


<210>  1117
<211>  110
<212>  RNA
<213>  Pongo pygmaeus

<400>  1117
cuggauacag aguggaccgg cuggccccgu cuggaagacu agugauuuug uuguugucuu      60

acugcgcuca acaacaaauc ccagucugcc uaauggugcc agccaucgca               110


<210>  1118
<211>  77
<212>  RNA
<213>  Monodelphis domestica

<400>  1118
uuggccuagu ucugugugga agacuaguga uuuuguuguu uuuagauaac uaaaacgaca      60

acaaaucaca gucugcc                                                    77


<210>  1119
<211>  109
<212>  RNA
<213>  Gallus gallus

<400>  1119
uggauguugg ucuaguucug uguggaagac uagugauuuu guuguuuuua gauaauuaaa      60

uugacaacaa aucacagucu gccauauggc acagaucaug ccucuacag                109


<210>  1120
<211>  99
<212>  RNA
<213>  Gallus gallus

<400>  1120
ggacggccgc ggugcccucu ggaagacuag ugauuuuguu guuguauggc ucaucccacc      60

acaacaaguc acagucugcc uuagggcgca cggccccgc                            99


<210>  1121
<211>  95
<212>  RNA
<213>  Gallus gallus

<400>  1121
cuguggucug gcucugugug gaagacuagu gauuuuguug uuaugauuua uaaaggugac      60

aacaaaucau agccugccau acagcacaga ucuuc                                 95


<210>  1122
<211>  110
<212>  RNA
<213>  Gallus gallus

<400>  1122
uggacauugg ucuaguucug uguggaagac uagugauuuu uguuguuuuu agauaacuaa      60

auugacaaca aaucacaguc uaccauaugg cacagacuau gccucuacag                110


<210>  1123
<211>  108
<212>  RNA
<213>  Xenopus tropicalis

<400>  1123
uggauguugg uuuaguucug uguggaagac uagugauuuu guuguuuuua gauaacaaca      60

uugacaacaa aucgcagucu gccauauggc acagaccaug ccucuaca                  108


<210>  1124
<211>  103
<212>  RNA
<213>  Xenopus tropicalis

<400>  1124
gaggugcagg cugacucuuu guggaagacu agugauuuug uuguuguaag ccuuauugca      60

ugacaacaag ucacagucug ccucacagug cccagcaaua uca                       103


<210>  1125
<211>  88
<212>  RNA
<213>  Xenopus tropicalis

<400>  1125
cugggucggu uuggaagacu agugauuuug uuguuuuuau aaaaguuugu cacaaacagc      60

aaaucguagu cuccacucug ccccagga                                         88


<210>  1126
<211>  82
<212>  RNA
<213>  Bos taurus

<400>  1126
agacuggagc gguugugguc uagugcugug uggaagacua gugauuuugu uguucugaug      60

uacuaugaca acaagucaca gc                                               82


<210>  1127
<211>  109
<212>  RNA
<213>  Sus scrofa

<400>  1127

uggauguugg ucuaguucug uguggaagac uagugauuuu guuguuuuua gauaacuaaa 60

ucgacaacaa aucacagucu gccauauggc acaggccaug ccucuacag 109

<210> 1128
<211> 79
<212> RNA
<213> Danio rerio

<400> 1128
uguggaagac uagugauuuu guuguuguuu cuucugcuuu cugacaacaa gucacagucu 60

accucagcga gcgggcccu 79

<210> 1129
<211> 97
<212> RNA
<213> Danio rerio

<400> 1129
uuuugggcau uauggaagac uagugauuuu guuguuguga cgucaucagu acugacaaca 60

agucacaguc ugccucagug accagaaucc acucguc 97

<210> 1130
<211> 120
<212> RNA
<213> Danio rerio

<400> 1130
ugugugugug uucugcccuc ugcggagugg aagacuagug auuuuguugu gaaaaugaac 60

aaaaaccaac aacaaaccgc agucguccuc ucagcacggg gcccacaccu gcagagcaca 120

<210> 1131
<211> 104
<212> RNA
<213> Danio rerio

<400> 1131
ugaacgcugg cuugcuucug uguggaagac uugugauuuu guuguuguua guuagaugaa 60

gugacaacaa aucacggucu gcccuacagc acaggcccag cauc 104

<210> 1132
<211> 91
<212> RNA
<213> Fugu rubripes

<400> 1132
cccugucugc ugcugugugg aagacuagug auuuuguugu uuuuaguuag aucaacugac 60

aacaaaucac agucugccaa acagcacagg c 91

<210> 1133
<211> 65
<212> RNA
<213> Tetraodon nigroviridis

<400> 1133
uggaagacua gugauuuugu uguuuggugu caaaauugac aacaaaucau ugucuuccuc 60

acugc                                                              65

<210>  1134
<211>  110
<212>  RNA
<213>  Mus musculus

<400>  1134
gaccugucug ucuucuguau auacccugua gauccgaauu uguguaagga auuuuguggu      60

cacaaauucg uaucuagggg aauauguagu ugacauaaac acuccgcuca                110

<210>  1135
<211>  110
<212>  RNA
<213>  Ateles geoffroyi

<400>  1135
gaucugucug ucuucuguau auacccugua gauccgaauu uguguaagga auuuuguggu      60

cacaaauucg uaucuagggg aauauguagu ugacauaaac acuccgcuca                110

<210>  1136
<211>  110
<212>  RNA
<213>  Saguinus labiatus

<400>  1136
gaucugucug ucuucuguau auacccugua gauccgaauu uguguaagga auuuuguggu      60

cacaaauucg uaucuagggg aauauguagu ugacauaaac acuccgcuca                110

<210>  1137
<211>  109
<212>  RNA
<213>  Macaca nemestrina

<400>  1137
cagagguugu aacguugucu auauauaccc uguagaaccg aauuugugug guauccauau      60

agucacagau ucgauucuag gggaauauau ggucgaugca aaaacuuca                 109

<210>  1138
<211>  110
<212>  RNA
<213>  Gorilla gorilla

<400>  1138
gaucugucug ucuucuguau auacccugua gauccgaauu uguguaagga auuuuguggu      60

cacaaauucg uaucuagggg aauauguagu ugacauaaac acuccgcucu                110

<210>  1139
<211>  110
<212>  RNA
<213>  Gorilla gorilla

<400>  1139
ccagacauug uaacguuguc uauauauacc cuguagaacc gaauuugugu gguauccaua      60

uagucacaga uucgauucua ggggaauaua uggucgaugc aaaaacuuca                110

<210> 1140
<211> 110
<212> RNA
<213> Pan paniscus

<400> 1140
gaucugucug ucuucuguau auacccugua gauccgaauu uguguaagga auuuuguggu 60

cacacauucg uaucuagggg aauauguagu ugacauacac acuccgcucu 110


<210> 1141
<211> 110
<212> RNA
<213> Pan paniscus

<400> 1141
ccagagguug uaacguuguc uauauauacc cuguagaacc gaauuugugu gguauccgua 60

uagucacaga uucgauucua ggggaauaua uggucgaugc aaaaacuuca 110


<210> 1142
<211> 90
<212> RNA
<213> Monodelphis domestica

<400> 1142
cugucuucug uauauacccu guagauccga auuuguguaa ggaauuuugu ggucacaaau 60

ucguaucuag gggaauaugu aguugacaua 90


<210> 1143
<211> 110
<212> RNA
<213> Gallus gallus

<400> 1143
cagaacguua uuacguuguc uauauauacc cuguagaacc gaauuugugu gauauucaua 60

uagucacaga uucgauucua ggggaauaua uggucgaugc aaaaacuuca 110


<210> 1144
<211> 100
<212> RNA
<213> Xenopus tropicalis

<400> 1144
gauuugccug uccucuguau guacccugua gauccgaauu ugugugagcg caaucauauc 60

acaaauucgu gucugggggg auaugcaguu gacacaaacg 100


<210> 1145
<211> 88
<212> RNA
<213> Xenopus tropicalis

<400> 1145
aacguugucu auauguaccc uguagaaccg aauuugugug guucguacag ucacagauuc 60

gauucuaggg ggauauaugg ucgaugca 88


<210> 1146

<211>  71
<212>  RNA
<213>  Xenopus tropicalis

<400>  1146
uauaugcacc cuguagaauc gaauuugugu gaguucugaa ccacagauuc gucucuaggg      60

ggguauaugg g                                                           71


<210>  1147
<211>  109
<212>  RNA
<213>  Bos taurus

<400>  1147
gaucugucug ucuucuguau auacccugua gauccgaauu uguguaagga auuuugugau      60

cacaaauucg uaucuagggg aauauguagu ugacauaaac acuccgcuc                 109


<210>  1148
<211>  99
<212>  RNA
<213>  Bos taurus

<400>  1148
cagugacguu gucuauauau acccuguaga accgaauuug ugugguaucc auguagucac      60

agauucgauu cuaggggaau auauggucga ugcaaaaac                             99


<210>  1149
<211>  99
<212>  RNA
<213>  Danio rerio

<400>  1149
ugucugucau cuauauauac ccuguagauc cgaauuugug ugaauauaca gucgcaaauu      60

cgugucuugg ggaauaugua guugacauaa acacaacgc                             99


<210>  1150
<211>  78
<212>  RNA
<213>  Danio rerio

<400>  1150
gucuauauau acccuguaga accgaauuug ugugaaaaaa uaacauucac agauucgauu      60

cuaggggagu auaugguc                                                    78


<210>  1151
<211>  92
<212>  RNA
<213>  Danio rerio

<400>  1151
guagucgucu auauguaccc uguagaaccg aauuuguguc caaaacauca aaaucgcaaa      60

uacgucucua caggaauaca ugggcgacgu aa                                    92


<210>  1152
<211>  95
<212>  RNA

<213>  Danio rerio

<400>  1152
ccugucaucu auauauaccc uguagauccg gauuugugua aacagacgca cagucacaaa      60

uucguaucua ggggaguaug uaguugaugu auagg                                 95


<210>  1153
<211>  118
<212>  RNA
<213>  Danio rerio

<400>  1153
uggaagcuuu guuccgucgu cuauauauac ccuguagaac cgaaugugug uuuacacagc      60

aaauucacag auucgguuuu aggggaguau auggacgaug caaaaacguc ugcuuuca       118


<210>  1154
<211>  118
<212>  RNA
<213>  Danio rerio

<400>  1154
uggaagcuuu guuccgucgu cuauauauac ccuguagaac cgaaugugug uuuacacagc      60

aaauucacag auucgguuuu aggggaguau auggacgaug caaaaacguc ugcuuuca       118


<210>  1155
<211>  70
<212>  RNA
<213>  Fugu rubripes

<400>  1155
auauauaccc uguagaaccg aauuugugug auggcgucaa agucacagau ucgauucuag      60

gggaguauau                                                             70


<210>  1156
<211>  84
<212>  RNA
<213>  Fugu rubripes

<400>  1156
guugucuaua uguacccugu agaaccgaau uugugugagu uccagacagu cgcaaguacg      60

ucucuacagg aauacauggg caac                                             84


<210>  1157
<211>  102
<212>  RNA
<213>  Fugu rubripes

<400>  1157
cugucuucua uaucuacccu guagauccgg auuuguguaa aaaucauuaa agcaaucaca      60

aauucgcuuc uaggggagua uauaguggau uuauacacga cg                        102


<210>  1158
<211>  112
<212>  RNA
<213>  Fugu rubripes

<400>  1158
ccggugaggu ggaucgucgu cuauaaauac ccuguagaac cgaaugugug ugcagcugac      60

uugaucacag auuggguucu aggggagucu augggcgaug aauaaucacu ga             112


<210>  1159
<211>  83
<212>  RNA
<213>  Tetraodon nigroviridis

<400>  1159
guugucuaua uguacccugu agaaccgaau uugugugagu ucagacaguc acaaguacgu      60

cucuacagga auacaugggc aac                                              83


<210>  1160
<211>  70
<212>  RNA
<213>  Tetraodon nigroviridis

<400>  1160
auauauaccc uguagaaccg aauuugugug aucaagucac agucacagau ucgauucuag      60

gggaguauau                                                             70


<210>  1161
<211>  108
<212>  RNA
<213>  Tetraodon nigroviridis

<400>  1161
gagccgcugu cuucuauauc uacccuguag auccggauuu guguaacgau cauuaaagca      60

aucacaaauu cgcuucuagg ggaguauaua guggauuuau acacgacg                  108


<210>  1162
<211>  120
<212>  RNA
<213>  Tetraodon nigroviridis

<400>  1162
gccggugagg ugcucgucgu cuauacauac ccuguagaac cgaaugugug ugcagcugac      60

uugaucacag auuggguucu aggggagucu augggcgcug aauaaucauc gaugaacggc     120


<210>  1163
<211>  96
<212>  RNA
<213>  Mus musculus

<400>  1163
ugcgugcuuu uuguucuaag gugcaucuag ugcagauagu gaaguagacu agcaucuacu      60

gcccuaagug cuccuucugg cauaagaagu uauguc                                96


<210>  1164
<211>  96
<212>  RNA
<213>  Rattus norvegicus

<400>  1164
ugcgugcuuu uuguucuaag gugcaucuag ugcagauagu gaaguagacu agcaucuacu      60

gcccuaagug cuccuucugg cauaagaagu uauguc                                96

<210>  1165
<211>  71
<212>  RNA
<213>  Ateles geoffroyi

<400>  1165
uguucuaagg ugcaucuagu gcagauagug aaguagauua gcaucuacug cccuaagugc      60

uccuucuggc a                                                           71

<210>  1166
<211>  71
<212>  RNA
<213>  Lagothrix lagotricha

<400>  1166
uguucuaagg ugcaucuagu gcagauagug aaguagauua gcaucuacug cccuaagugc      60

uccuucuggc a                                                           71

<210>  1167
<211>  71
<212>  RNA
<213>  Saguinus labiatus

<400>  1167
uguucuaagg ugcaucuagu gcagauagug aaguagauua gcaucuacug cccuaagugc      60

uccuucuggc a                                                           71

<210>  1168
<211>  71
<212>  RNA
<213>  Macaca mulatta

<400>  1168
uguucuaagg ugcaucuagu gcagauagug aaguagauua gcaucuacug cccuaagugc      60

uccuucuggc a                                                           71

<210>  1169
<211>  71
<212>  RNA
<213>  Macaca nemestrina

<400>  1169
uguucuaagg ugcaucuagu gcagauagug aaguagauua gcaucuacug cccuaagugc      60

uccuucuggc a                                                           71

<210>  1170
<211>  71
<212>  RNA
<213>  Gorilla gorilla

<400>  1170
uguucuaagg ugcaucuagu gcagauagug aaguagauua gcaucuacug cccuaagugc      60

uccuucuggc a                                                           71

<210>  1171
<211>  71
<212>  RNA
<213>  Pan paniscus

<400>  1171
uguucuaagg ugcaucuagu gcagauagug aaguagauua gcaucuacug cccuaagugc      60

uccuucuggc a                                                           71


<210>  1172
<211>  71
<212>  RNA
<213>  Pan troglodytes

<400>  1172
uguucuaagg ugcaucuagu gcagauagug aaguagauua gcaucuacug cccuaagugc      60

uccuucuggc a                                                           71


<210>  1173
<211>  71
<212>  RNA
<213>  Pongo pygmaeus

<400>  1173
uguucuaagg ugcaucuagu gcagauagug aaguagauua gcaucuacug cccuaagugc      60

uccuucuggc a                                                           71


<210>  1174
<211>  71
<212>  RNA
<213>  Lemur catta

<400>  1174
uguucuaagg ugcaucuagu gcagauagug aaguagauua gcaucuacug cccuaagugc      60

uccuucuggc a                                                           71


<210>  1175
<211>  72
<212>  RNA
<213>  Monodelphis domestica

<400>  1175
uuguucuaag gugcaucuag ugcagauagu gaaguagauu agcaucuacu gcccuaagug      60

cuccuucugg ca                                                          72


<210>  1176
<211>  93
<212>  RNA
<213>  Gallus gallus

<400>  1176
gugcuuuuug uacuaaggug caucuagugc agauagugaa guagauuagc aucuacugcc      60

cuaagugcuc cuucuggcau aagaaguuau guc                                   93

<210>  1177
<211>  87
<212>  RNA
<213>  Xenopus tropicalis

<400>  1177
gugcuuuuug uccuaaggug caucuagugc agauagugaa guagauuagc aucuacugcc      60

cuaagugcuc cuucuggcau aaaaagu                                          87


<210>  1178
<211>  71
<212>  RNA
<213>  Bos taurus

<400>  1178
uguucuaagg ugcaucuagu gcagauagug aaguagauua gcaucuacug cccuaagugc      60

uccuucuggc a                                                           71


<210>  1179
<211>  92
<212>  RNA
<213>  Sus scrofa

<400>  1179
gugcuuuuug uucuaaggug caucuagugc agauagugaa guagauuagc aucuacugcc      60

cuaagugcuc cuucuggcau aagaaguuau gu                                    92


<210>  1180
<211>  83
<212>  RNA
<213>  Danio rerio

<400>  1180
ggcuuugugc uaaggugcau cuagugcaga uagugaagua gacuagcacc uacugcccua      60

agugcuccuu cuggcacgag ggu                                              83


<210>  1181
<211>  85
<212>  RNA
<213>  Danio rerio

<400>  1181
gccuuccugc uaaggugcau cuuguguagu uagugaagua gucuaguauc uacugcgcua      60

gauguuccuu uuggcaggag uagcu                                            85


<210>  1182
<211>  76
<212>  RNA
<213>  Fugu rubripes

<400>  1182
uugugcuaag gugcaucuag ugcagauagu gaaauagacu agcaccuacu gcccuaagug      60

cuccuucugg cauaag                                                      76


<210>  1183
<211>  76

<212>  RNA
<213>  Tetraodon nigroviridis

<400>  1183
uugugcuaag gugcaucuag ugcagauagu gaaauagacu agcaccuacu gcccuaagug      60

cuccuucugg cauaag                                                      76


<210>  1184
<211>  83
<212>  RNA
<213>  Mus musculus

<400>  1184
ucucuugugu uaaggugcau cuagugcugu uagugaagca gcuuacaauc uacugcccua      60

aaugccccuu cucgcacagg cua                                              83


<210>  1185
<211>  84
<212>  RNA
<213>  Gallus gallus

<400>  1185
cgugguccuu guguuaaggu gcaucuagug caguuaguga cguagcguag aaucuacugc      60

ccuaaaugcu ccuucuggca caag                                             84


<210>  1186
<211>  78
<212>  RNA
<213>  Xenopus tropicalis

<400>  1186
ccuuguguua aggugcaucu agugcaguua gugacauagu guagcaucua cugcccuaaa      60

ugcuccuuuu ggcacagg                                                    78


<210>  1187
<211>  75
<212>  RNA
<213>  Bos taurus

<400>  1187
cuuguguuaa ggugcaucua gugcaguuag ugaagcagcu cagaaucuac ugcccuaaau      60

gcuccuucug gcaca                                                       75


<210>  1188
<211>  87
<212>  RNA
<213>  Danio rerio

<400>  1188
uacugcuuau gcuaaggugc auuuagugca gauagugaaa uagacuagca ccuacugccc      60

uaagugcccc uucuggcaua aggggcu                                          87


<210>  1189
<211>  80
<212>  RNA
<213>  Mus musculus

<400>  1189
ccuaguagug ccaaagugcu cauagugcag guaguuuuua uaccacucua cugcagugug      60

agcacuucua guacuccugg                                                  80


<210>  1190
<211>  71
<212>  RNA
<213>  Ateles geoffroyi

<400>  1190
guagcacuaa agugcuuaua gugcagguag uguuuaguua ucuacugcau uaugagcacu      60

uaaaguacug c                                                           71


<210>  1191
<211>  71
<212>  RNA
<213>  Lagothrix lagotricha

<400>  1191
guagcacuaa agugcuuaua gugcagguag uguuuaguua ucuacugcau uaugagcacu      60

uaaaguacug c                                                           71


<210>  1192
<211>  71
<212>  RNA
<213>  Saguinus labiatus

<400>  1192
guagcacuaa agugcuuaua gugcagguag uguuuaguua ucuacugcau uaugagcacu      60

uaaaguacug c                                                           71


<210>  1193
<211>  71
<212>  RNA
<213>  Macaca mulatta

<400>  1193
guagcacuaa agugcuuaua gugcagguag uguuuaguua ucuacugcau uaugagcacu      60

uaaaguacug c                                                           71


<210>  1194
<211>  71
<212>  RNA
<213>  Macaca nemestrina

<400>  1194
guagcacuaa agugcuuaua gugcagguag uguuuaguua ucuacugcau uaugagcacu      60

uaaaguacug c                                                           71


<210>  1195
<211>  71
<212>  RNA
<213>  Gorilla gorilla

<400>  1195

guagcacuaa agugcuuaua gugcagguag uguuuaguua ucuacugcau uaugagcacu 60

uaaaguacug c 71

<210> 1196
<211> 71
<212> RNA
<213> Pan paniscus

<400> 1196
guagcacuaa agugcuuaua gugcagguag uguuuaguua ucuacugcau uaugagcacu 60

uaaaguacug c 71

<210> 1197
<211> 71
<212> RNA
<213> Pan troglodytes

<400> 1197
guagcacuaa agugcuuaua gugcagguag uguuuaguua ucuacugcau uaugagcacu 60

uaaaguacug c 71

<210> 1198
<211> 71
<212> RNA
<213> Pongo pygmaeus

<400> 1198
guagcacuaa agugcuuaua gugcagguag uguuuaguua ucuacugcau uaugagcacu 60

uaaaguacug c 71

<210> 1199
<211> 71
<212> RNA
<213> Lemur catta

<400> 1199
guagcacuaa agugcuuaua gugcagguag uguuuaguua ucuacugcau caugagcacu 60

uaaaguacug c 71

<210> 1200
<211> 102
<212> RNA
<213> Monodelphis domestica

<400> 1200
ugaaaggaca gcuuuuguag cacuaaagug cuuauagugc agguaguguu uaguuaucua 60

cugcauuaug agcacuugaa guacugcuag cuguagaacu ac 102

<210> 1201
<211> 85
<212> RNA
<213> Gallus gallus

<400> 1201
cuagcaguau caaagugcuc auagugcagg uagcuuggca uuggaccuac uguaaugugg 60

gcacuuacag uacuguuaga uaaag                                              85

<210>  1202
<211>  74
<212>  RNA
<213>  Xenopus tropicalis

<400>  1202
gcaguuccaa agugcucaua gugcagguag uuguauugau guucuacugu aauaugggca      60

cuuacaguac ugcu                                                         74

<210>  1203
<211>  69
<212>  RNA
<213>  Bos taurus

<400>  1203
aguaccaaag ugcucacagu gcagguaguu uuggcagcgc ucuacuguag ugugggcacu      60

uccaguacu                                                               69

<210>  1204
<211>  71
<212>  RNA
<213>  Sus scrofa

<400>  1204
guagcacuaa agugcuuaua gugcagguag uguuuaguua ucuacugcau uaugagcacu      60

uaaaguacug c                                                            71

<210>  1205
<211>  94
<212>  RNA
<213>  Danio rerio

<400>  1205
gaguuugucc uggcaguucc aaagugcuca cagugcaggu agugccagug gaucuacugc      60

aaugucugca cuucaaguau ugccggacgc cuuc                                   94

<210>  1206
<211>  80
<212>  RNA
<213>  Fugu rubripes

<400>  1206
agcaguauua aagugcuuau agugcaggua guuacugaug gucuacugca gugugagcac      60

uucuaguacu ucuagaugca                                                   80

<210>  1207
<211>  73
<212>  RNA
<213>  Tetraodon nigroviridis

<400>  1207
gcaguauuaa agugcuuaua gugcagguag uuuucucagg gucuacugca guaugagcac      60

uucuaguacu ucu                                                          73

<210>  1208
<211>  65
<212>  RNA
<213>  Mus musculus

<400>  1208
augucaaagu gcuaacagug cagguagcuu uuugaguucu acugcagugc cagcacuucu      60

uacau                                                                  65


<210>  1209
<211>  81
<212>  RNA
<213>  Ateles geoffroyi

<400>  1209
ccuuggccau guaaaagugc uuacagugca gguagcuuuu ugagaucuac ugcaaugcaa      60

gcacuucuua cauuaccaug g                                                81


<210>  1210
<211>  81
<212>  RNA
<213>  Saguinus labiatus

<400>  1210
ccuuggccau guaaaagugc uuacagugca gguagcuuuu ugagaucuac ugcaaugcaa      60

gcacuucuua cauuaccaug g                                                81


<210>  1211
<211>  81
<212>  RNA
<213>  Macaca mulatta

<400>  1211
ccuuggccau guaaaagugc uuacagugca gguagcuuuu ugagaucuac ugcaaugcaa      60

gcacuucuua cauuaccaug g                                                81


<210>  1212
<211>  81
<212>  RNA
<213>  Macaca nemestrina

<400>  1212
ccuuggccau guaaaagugc uuacagugca gguagcuuuu ugagaucuac ugcaaugcaa      60

gcacuucuua cauuaccaug g                                                81


<210>  1213
<211>  81
<212>  RNA
<213>  Gorilla gorilla

<400>  1213
ccuuggccau guaaaagugc uuacagugca gguagcuuuu ugagaucuac ugcaaugcaa      60

gcacuucuua cauuaccaug g                                                81


<210>  1214

<211>  81
<212>  RNA
<213>  Pan paniscus

<400>  1214
ccuuggccau guaaaagugc uuacagugca gguagcuuuu ugagaucuac ugcaaugcaa      60

gcacuucuua cauuaccaug g                                                81


<210>  1215
<211>  81
<212>  RNA
<213>  Pan troglodytes

<400>  1215
ccuuggccau guaaaagugc uuacagugca gguagcuuuu ugagaucuac ugcaaugcaa      60

gcacuucuua cauuaccaug g                                                81


<210>  1216
<211>  81
<212>  RNA
<213>  Pongo pygmaeus

<400>  1216
ccuuggccau guaaaagugc uuacagugca gguagcuuuu ugagaucuac ugcaaugcaa      60

gcacuucuua cauuaccaug g                                                81


<210>  1217
<211>  81
<212>  RNA
<213>  Gallus gallus

<400>  1217
ccuugaguug ugcaaaagug cuuacagugc agguagagcu cagcaccuac ugcaguauaa      60

gcacuucugg caugaccgug g                                                81


<210>  1218
<211>  63
<212>  RNA
<213>  Xenopus tropicalis

<400>  1218
ugcaaaagug cuuauagugc agguagaauu uaaaccuacu gcaccauaag cacuuccugc      60

auc                                                                    63


<210>  1219
<211>  81
<212>  RNA
<213>  Bos taurus

<400>  1219
ccuuggccau guaaaagugc uuacagugca gguagcuuuu ugagaucuac ugcaaugcaa      60

gcacuucuua cauuaccaug g                                                81


<210>  1220
<211>  81
<212>  RNA

<213>  Sus scrofa

<400>  1220
ccuuggccgu guaaaagugc uuacagugca gguagcuuuu ugagaucuac ugcaaugcaa      60

gcacuucuua cauuaccaug g                                                81


<210>  1221
<211>  82
<212>  RNA
<213>  Mus musculus

<400>  1221
ccugcuggga cuaaagugcu gacagugcag auaguggucc ucucugugcu accgcacugu      60

ggguacuugc ugcuccagca gg                                               82


<210>  1222
<211>  82
<212>  RNA
<213>  Rattus norvegicus

<400>  1222
ccugcuggga cuaaagugcu gacagugcag auaguggucc ucucugugcu accgcacugu      60

ggguacuugc ugcuccagca gg                                               82


<210>  1223
<211>  82
<212>  RNA
<213>  Ateles geoffroyi

<400>  1223
ccugccgggg cuaaagugcu gacagugcag auaguggucc ucuccgugcu accgcacugu      60

ggguacuugc ugcuccagca gg                                               82


<210>  1224
<211>  81
<212>  RNA
<213>  Lagothrix lagotricha

<400>  1224
ccugccgggg cuaaagugcu gacagugcag auaguguccu cuccgugcua ccgcacugug      60

gguacuugcu gcuccagcag g                                                81


<210>  1225
<211>  82
<212>  RNA
<213>  Saguinus labiatus

<400>  1225
ccugccgggg cuaaagugcu gacagugcag auaguggucc ucuccgugcu accgcacugu      60

ggguacuugc ugcuccagca gg                                               82


<210>  1226
<211>  82
<212>  RNA
<213>  Macaca mulatta

<400>  1226
ccugcugggg cuaaagugcu gacagugcag auaguggucc ucuccgugcu accgcacugu      60

ggguacuugc ugcuccagca gg                                                82


<210>  1227
<211>  82
<212>  RNA
<213>  Macaca nemestrina

<400>  1227
ccugcugggg cuaaagugcu gacagugcag auaguggucc ucuccgugcu accgcacugu      60

ggguacuugc ugcuccagca gg                                                82


<210>  1228
<211>  82
<212>  RNA
<213>  Gorilla gorilla

<400>  1228
ccugccgggg cuaaagugcu gacagugcag auaguggucc ucuccgugcu accgcacugu      60

ggguacuugc ugcuccagca gg                                                82


<210>  1229
<211>  82
<212>  RNA
<213>  Pan paniscus

<400>  1229
ccugccgggg cuaaagugcu gacagugcag auaguggucc ucuccgugcu accgcacugu      60

ggguacuugc ugcuccagca gg                                                82


<210>  1230
<211>  82
<212>  RNA
<213>  Pan troglodytes

<400>  1230
ccugccgggg cuaaagugcu gacagugcag auaguggucc ucuccgugcu accgcacugu      60

ggguacuugc ugcuccagca gg                                                82


<210>  1231
<211>  82
<212>  RNA
<213>  Pongo pygmaeus

<400>  1231
ccugccgggg cuaaagugcu gacagugcag auaguggucc ucuccgugcu accgcacugu      60

ggguacuugc ugcuccagca gg                                                82


<210>  1232
<211>  68
<212>  RNA
<213>  Mus musculus

<400>  1232
cuccggugcc uacugagcug auaucaguuc ucauuucaca cacuggcuca guucagcagg      60

aacaggag                                                            68

<210>  1233
<211>  107
<212>  RNA
<213>  Mus musculus

<400>  1233
gccucucucc gggcuccgcc ucccgugccu acugagcuga aacaguugau uccagugcac      60

uggcucaguu cagcaggaac aggaguccag cccccuagga gcuggca                   107

<210>  1234
<211>  68
<212>  RNA
<213>  Rattus norvegicus

<400>  1234
cuccggugcc uacugagcug auaucaguuc ucauuucaca cacuggcuca guucagcagg      60

aacaggag                                                            68

<210>  1235
<211>  108
<212>  RNA
<213>  Rattus norvegicus

<400>  1235
gccucucccu gggcuccgcc uccugugccu acugagcuga aacaguugau uccagugcac      60

uggcucaguu cagcaggaac aggaguccag cccccauagg agcuggca                  108

<210>  1236
<211>  68
<212>  RNA
<213>  Macaca mulatta

<400>  1236
cuccggugcc uacugagcug auaucaguuc ucauuuuaca cacuggcuca guucagcagg      60

aacaggag                                                            68

<210>  1237
<211>  73
<212>  RNA
<213>  Macaca mulatta

<400>  1237
cucugccucc cgugccuacu gagcugaaac acaguugguu ugugcacacu ggcucaguuc      60

agcaggaaca gga                                                      73

<210>  1238
<211>  68
<212>  RNA
<213>  Macaca nemestrina

<400>  1238
cuccggugcc uacugagcug auaucaguuc ucauuuuaca cacuggcuca guucagcagg      60

aacaggag                                                            68

<210> 1239
<211> 73
<212> RNA
<213> Macaca nemestrina

<400> 1239
cucugccucc cgugccuacu gagcugaaac acaguugguu ugugcacacu ggcucaguuc 60

agcaggaaca gga 73

<210> 1240
<211> 73
<212> RNA
<213> Gorilla gorilla

<400> 1240
cucugccucc cgugccuacu gagcugaaac acaguugguu uguguacacu ggcucaguuc 60

agcaggaaca ggg 73

<210> 1241
<211> 68
<212> RNA
<213> Pan paniscus

<400> 1241
cuccggugcc uacugagcug auaucaguuc ucauuuuaca cacuggcuca guucagcagg 60

aacaggag 68

<210> 1242
<211> 73
<212> RNA
<213> Pan paniscus

<400> 1242
cucugccucc cgugccuacu gagcugaaac acaguugguu uguguacacu ggcucaguuc 60

agcaggaaca ggg 73

<210> 1243
<211> 73
<212> RNA
<213> Pan troglodytes

<400> 1243
cucugccucc cgugccuacu gagcugaaac acaguugguu uguguacacu ggcucaguuc 60

agcaggaaca ggg 73

<210> 1244
<211> 68
<212> RNA
<213> Pongo pygmaeus

<400> 1244
cuccggugcc uacugagcug auaucaguuc ucauuuuaca cacuggcuca guucagcagg 60

aacaggag 68

<210>  1245
<211>  73
<212>  RNA
<213>  Pongo pygmaeus

<400>  1245
cucugccucc cgugccuacu gagcugaaac acaguugguu uguguacacu ggcucaguuc      60

agcaggaaca ggg                                                          73


<210>  1246
<211>  57
<212>  RNA
<213>  Monodelphis domestica

<400>  1246
ggugccuacu gagcugauaa caguucugau uuuacacacu ggcucaguuc agcagga         57


<210>  1247
<211>  73
<212>  RNA
<213>  Monodelphis domestica

<400>  1247
gccuccugug ccuacugagc ugaaacacag uugcuuugga uaaacuggcu caguucagca      60

ggaacaggag ucu                                                          73


<210>  1248
<211>  68
<212>  RNA
<213>  Gallus gallus

<400>  1248
cuccggugcc uacugagcug auaucaguuc ugauuuuaca uacuggcuca guucagcagg      60

aacaggag                                                                68


<210>  1249
<211>  68
<212>  RNA
<213>  Xenopus tropicalis

<400>  1249
cucccguacc uacugggcug auaaucagug guuauaucuu ccccuggcuc aguucagcag      60

gacaggag                                                                68


<210>  1250
<211>  72
<212>  RNA
<213>  Bos taurus

<400>  1250
cucugccucc cgugccuacu gagcugaaac acaguugauu ugugcacacu ggcucaguuc      60

agcaggaaca gg                                                           72


<210>  1251
<211>  72
<212>  RNA
<213>  Sus scrofa

<400>  1251
cucugccucc cgugccuacu gagcugaaac acaguugauu ugugcagacu ggcucaguuc      60

agcaggaaca gg                                                          72


<210>  1252
<211>  80
<212>  RNA
<213>  Danio rerio

<400>  1252
   accugagcuc cggugccuuc ugagcugaua ucaguuguag uaaaucacug gcucaguuca      60
   gcaggaacag gaguguggcc                                                 80


<210>  1253
<211>  82
<212>  RNA
<213>  Danio rerio

<400>  1253
gggcuggucu ccugugccug cugugcugau aaucagugga cggcugugac uggcucaguu      60

cagcaggaac aggggccugg uc                                               82


<210>  1254
<211>  142
<212>  RNA
<213>  Danio rerio

<400>  1254
gcugaacggg agucauauga gagauagaug gagcuggccu cccgugccua cugagcugau      60

uacaguacug ugcaaacacu ggcucaguuc agcaggaaca ggagucuggu ccacaaacuc     120

caccaaacac ugcugcuuca gc                                              142


<210>  1255
<211>  90
<212>  RNA
<213>  Danio rerio

<400>  1255
gagggcuguu cccacuugug ccugcuaaac ugguaucagu auguugauuu agugcuggcu      60

caguucagca ggaacaggug ugaaguccuc                                       90


<210>  1256
<211>  79
<212>  RNA
<213>  Fugu rubripes

<400>  1256
ggguugugcc cuccugugcc uacugaacug guaucagugu cauacuggaa aacuggcuca      60

guucagcagg aacaggagu                                                   79


<210>  1257
<211>  82
<212>  RNA
<213>  Fugu rubripes

<400>  1257
gggucagucu ccugugccug cugugcugau aaucagugug ugacguuggc uggcucaguu      60

cagcaggaac aggggacugg uc                                                82


<210>  1258
<211>  70
<212>  RNA
<213>  Tetraodon nigroviridis

<400>  1258
cuuccugugc cuacugagcu gauaugcagu uguacagaac cacuggcuca guucagcagg      60

aacaggaguc                                                              70


<210>  1259
<211>  80
<212>  RNA
<213>  Tetraodon nigroviridis

<400>  1259
gggucagucu ccugugccug cugugcugau aaucagugug ugacguuggc uggcucaguu      60

cagcaggaac aggggacugg                                                   80


<210>  1260
<211>  84
<212>  RNA
<213>  Mus musculus

<400>  1260
ggccaguguu gagaggcgga gacuugggca auugcuggac gcugcccugg gcauugcacu      60

ugucucgguc ugacagugcc ggcc                                              84


<210>  1261
<211>  84
<212>  RNA
<213>  Rattus norvegicus

<400>  1261
ggccaguguu gagaggcgga gacacgggca auugcuggac gcugcccugg gcauugcacu      60

ugucucgguc ugacagugcc ggcc                                              84


<210>  1262
<211>  84
<212>  RNA
<213>  Lagothrix lagotricha

<400>  1262
ggccaguguu gagaggcgga gacuugggca auugcuggac gcugcccugg gcauugcacu      60

ugucucgguc ugacagugcc ggcc                                              84


<210>  1263
<211>  84
<212>  RNA
<213>  Macaca mulatta

<400>  1263

ggccaguguu gagaggcgga gacuugggca auugcuggac gcugcccugg gcauugcacu 60

ugucucgguc ugacagugcc ggcc 84

<210> 1264
<211> 84
<212> RNA
<213> Macaca nemestrina

<400> 1264
ggccaguguu gagaggcgga gacuugggca auugcuggac gcugcccugg gcauugcacu 60

ugucucgguc ugacagugcc ggcc 84

<210> 1265
<211> 84
<212> RNA
<213> Gorilla gorilla

<400> 1265
ggccaguguu gagaggcgga gacuugggca auugcuggac gcugcccugg gcauugcacu 60

ugucucgguc ugacagugcc ggcc 84

<210> 1266
<211> 84
<212> RNA
<213> Pan paniscus

<400> 1266
ggccaguguu gagaggcgga gacuugggca auugcuggac gcugcccugg gcauugcacu 60

ugucucgguc ugacagugcc ggcc 84

<210> 1267
<211> 84
<212> RNA
<213> Pan troglodytes

<400> 1267
ggccaguguu gagaggcgga gacuugggca auugcuggac gcugcccugg gcauugcacu 60

ugucucggcu gagacgcgcc cgcc 84

<210> 1268
<211> 84
<212> RNA
<213> Pongo pygmaeus

<400> 1268
ggccaguguu gagaggcgga gacuugggca auugcuggac gcugcccugg gcauugcacu 60

ugucucgguc ugacagugcc ggcc 84

<210> 1269
<211> 83
<212> RNA
<213> Monodelphis domestica

<400> 1269
ggccaguguu gagaggcgga gacuugggca auugcugaac ucugcccugg gcauugcacu 60

ugucucgguc ugacagugcu ggc 83

<210> 1270
<211> 82
<212> RNA
<213> Xenopus tropicalis

<400> 1270
gcugguguug acaggcagag acaggagcaa cugcuggugu gccuugguag cauugcacuu 60

gucucggucu gacagugucg gc 82

<210> 1271
<211> 82
<212> RNA
<213> Xenopus tropicalis

<400> 1271
gcugguguug acaggcagag acaggagcaa cugcuggugu gccuugguag cauugcacuu 60

gucucggucu gacagugucg gc 82

<210> 1272
<211> 84
<212> RNA
<213> Bos taurus

<400> 1272
ggccaguguu gagaggcgga gacuugggca auugcuggac gcugccccgg gcauugcacu 60

ugucucgguc ugacagugcc ggcc 84

<210> 1273
<211> 82
<212> RNA
<213> Danio rerio

<400> 1273
gccggcgcug agaggcggag acuugggcag cugccgucau ucccagaagg cauugcacuu 60

gucucggucu gacaguggcg gc 82

<210> 1274
<211> 82
<212> RNA
<213> Fugu rubripes

<400> 1274
gcugguguug agaggcggag acuugggcaa uugccggcca ucacagaggg cauugcacuu 60

gucucggucu gacagugccg gc 82

<210> 1275
<211> 82
<212> RNA
<213> Tetraodon nigroviridis

<400> 1275
gcugguguug agaggcggag acuugggcaa uugccggcca ucacagaggg cauugcacuu 60

gucucggucu gacagugccg gc 82

<210>  1276
<211>  70
<212>  RNA
<213>  Mus musculus

<400>  1276
ggagauauug cacauuacua aguugcaugu ugucacggcc ucaaugcaau uuagugugug      60

ugauauuuuc                                                              70


<210>  1277
<211>  70
<212>  RNA
<213>  Rattus norvegicus

<400>  1277
ggggauauug cacauuacua aguugcaugu ugucacggcc ucaaugcaau uuagugugug      60

ugauauucuc                                                              70


<210>  1278
<211>  70
<212>  RNA
<213>  Saguinus labiatus

<400>  1278
ggagauauug cacauuacua aguugcaugu ugucacggcc ucaaugcaau uuagugugug      60

ugauauuuuc                                                              70


<210>  1279
<211>  70
<212>  RNA
<213>  Macaca mulatta

<400>  1279
ggagauauug cacauuacua aguugcaugu ugucacggcc ucaaugcaau uuagugugug      60

ugauauuuuc                                                              70


<210>  1280
<211>  70
<212>  RNA
<213>  Macaca nemestrina

<400>  1280
ggagauauug cacauuacua aguugcaugu ugucacggcc ucaaugcaau uuagugugug      60

ugauauuuuc                                                              70


<210>  1281
<211>  70
<212>  RNA
<213>  Gorilla gorilla

<400>  1281
ggagauauug cacauuacua aguugcaugu ugucacggcc ucaaugcaau uuagugugug      60

ugauauuuuc                                                              70


<210>  1282

<211>  70
<212>  RNA
<213>  Pan paniscus

<400>  1282
ggagauauug cacauuacua aguugcaugu ugucacggcc ucaaugcaau uuagugugug      60

ugauauuuuc                                                              70


<210>  1283
<211>  70
<212>  RNA
<213>  Pan troglodytes

<400>  1283
ggagauauug cacauuacua aguugcaugu ugucacggcc ucaaugcaau uuagugugug      60

ugauauuuuc                                                              70


<210>  1284
<211>  70
<212>  RNA
<213>  Pongo pygmaeus

<400>  1284
ggagauauug cacauuacua aguugcaugu ugucacggcc ucaaugcaau uuagugugug      60

ugauauuuuc                                                              70


<210>  1285
<211>  69
<212>  RNA
<213>  Monodelphis domestica

<400>  1285
ggagauauug cacauuacua aguugcaugu ugucacggcu uuagugcaau uuaguaugug      60

ugauauuuu                                                               69


<210>  1286
<211>  70
<212>  RNA
<213>  Gallus gallus

<400>  1286
ggagauauug cacauuacua aguugcaugu ugucacggcc ucagugcaau uuagugugug      60

cgauacuuuc                                                              70


<210>  1287
<211>  70
<212>  RNA
<213>  Sus scrofa

<400>  1287
ggagauauug cacauuacua aguugcaugu ugucacggcc ucagugcaau uuagugugug      60

ugauauuuuc                                                              70


<210>  1288
<211>  90
<212>  RNA

<213>  Mus musculus

<400>  1288
aaggccgugg ccucguucaa guaauccagg auaggcugug caggucccaa ggggccuauu      60

cuugguuacu ugcacgggga cgcgggccug                                        90


<210>  1289
<211>  84
<212>  RNA
<213>  Mus musculus

<400>  1289
ggcugcggcu ggauucaagu aauccaggau aggcuguguc cguccaugag gccuguucuu      60

gauuacuugu uucuggaggc agcg                                              84


<210>  1290
<211>  90
<212>  RNA
<213>  Rattus norvegicus

<400>  1290
aaggccgugg ccuuguucaa guaauccagg auaggcugug caggucccaa ggggccuauu      60

cuugguuacu ugcacgggga cgcgggccug                                        90


<210>  1291
<211>  77
<212>  RNA
<213>  Lagothrix lagotricha

<400>  1291
guggccucgu ucaaguaauc caggauaggc ugugcagguc ccaaugggcc uauucuuggu      60

uacuugcacg gggacgc                                                      77


<210>  1292
<211>  77
<212>  RNA
<213>  Macaca mulatta

<400>  1292
guggccucgu ucaaguaauc caggauaggc ugugcagguc ccaaugggcc uauucuuggu      60

uacuugcacg gggacgc                                                      77


<210>  1293
<211>  77
<212>  RNA
<213>  Macaca nemestrina

<400>  1293
guggccucgu ucaaguaauc caggauaggc ugugcagguc ccaaugggcc uauucuugau      60

uacuugcacg gggacgc                                                      77


<210>  1294
<211>  77
<212>  RNA
<213>  Gorilla gorilla

<400>  1294
guggccucgu ucaaguaauc caggauaggc ugugcagguc ccaaugggcc uauucuuggu      60

uacuugcacg gggacgc                                                     77


<210>  1295
<211>  77
<212>  RNA
<213>  Pan paniscus

<400>  1295
guggccucgu ucaaguaauc caggauaggc ugugcagguc ccaaugggcc uauucuuggu      60

uacuugcacg gggacgc                                                     77


<210>  1296
<211>  77
<212>  RNA
<213>  Pan troglodytes

<400>  1296
guggccucgu ucaaguaauc caggauaggc ugugcagguc ccaaugggcc uauucuuggu      60

uacuugcacg gggacgc                                                     77


<210>  1297
<211>  77
<212>  RNA
<213>  Pongo pygmaeus

<400>  1297
guggccucgu ucaaguaauc caggauaggc ugugcagguc ccaaugggcc uauucuuggu      60

uacuugcacg gggacgc                                                     77


<210>  1298
<211>  77
<212>  RNA
<213>  Gallus gallus

<400>  1298
gucaccuggu ucaaguaauc caggauaggc uguauccauu ccugcuggcc uauucuuggu      60

uacuugcacu gggaggc                                                     77


<210>  1299
<211>  84
<212>  RNA
<213>  Bos taurus

<400>  1299
ggcuguggcu ggauucaagu aauccaggau aggcuguuuc caucugugag gccuauucuu      60

gauuacuugu uucuggaggc agcu                                             84


<210>  1300
<211>  84
<212>  RNA
<213>  Sus scrofa

<400>  1300
ggcuguggcu ggauucaagu aauccaggau aggcuguuuc caucugugag gccuauucuu      60

gauuacuugu uucuggaggc agcu                                          84

<210>  1301
<211>  78
<212>  RNA
<213>  Danio rerio

<400>  1301
uuuggccugg uucaaguaau ccaggauagg cuugugaugu ccggaaagcc uauucgggau      60

gacuugguuc aggaauga                                                    78

<210>  1302
<211>  128
<212>  RNA
<213>  Danio rerio

<400>  1302
guguggacuu gagugcugga agugguuguu cccuuguuca aguaauccag gauaggcugu      60

cuguccugga ggccuauuca ugauuacuug cacuaggugg cagccguugc ccuucaugga     120

acucaugc                                                              128

<210>  1303
<211>  137
<212>  RNA
<213>  Danio rerio

<400>  1303
cuaagcugau acugagucag uguguggcug caaccugguu caaguaaucc aggauaggcu      60

uuguggacua ggguuggccu guucuugguu acuugcacug gguugcagcu acuaaacaac     120

uaagaagauc agaagag                                                    137

<210>  1304
<211>  88
<212>  RNA
<213>  Fugu rubripes

<400>  1304
aggccucggc cugguucaag uaauccagga uaggcugguu aacccugcac ggccuauucu      60

ugauuacuug ugucaggaag uggccgug                                         88

<210>  1305
<211>  96
<212>  RNA
<213>  Tetraodon nigroviridis

<400>  1305
gcguuaggcc ucggccuggu ucaaguaauc caggauaggc ugguuaaccc ugcacggccu      60

auucuugauu acuuguguca ggaaguggcc gccagc                                96

<210>  1306
<211>  85
<212>  RNA
<213>  Mus musculus

<400>  1306
ugcccgggac ccaguucaag uaauucagga uagguugugg ugcugaccag ccuguucucc      60

auuacuuggc ucgggggccg gugcc                                            85


<210>  1307
<211>  85
<212>  RNA
<213>  Rattus norvegicus

<400>  1307
ugcccgggac ccaguucaag uaauucagga uagguugugg ugcuggccag ccuguucucc      60

auuacuuggc ucgggggccg gugcc                                            85


<210>  1308
<211>  85
<212>  RNA
<213>  Bos taurus

<400>  1308
ugcccgggac ccaguucaag uaauucagga uagguugugu gcuguccagc cuguucucca      60

uuacuuggcu cgggggccgg ugccc                                            85


<210>  1309
<211>  97
<212>  RNA
<213>  Danio rerio

<400>  1309
gcauuuggcc uuugccuggu ucaaguaauc caggauaggu uaguucccac uaguacggcc      60

uauucuuggu uacuuguuuc aggaggaggc uacgagc                               97


<210>  1310
<211>  73
<212>  RNA
<213>  Mus musculus

<400>  1310
aggugcagag cuuagcugau uggugaacag ugauugguuu ccgcuuuguu cacaguggcu      60

aaguucugca ccu                                                         73


<210>  1311
<211>  87
<212>  RNA
<213>  Rattus norvegicus

<400>  1311
uggccugugg agcagggcuu agcugcuugu gagcaagguc uacagcaaag ucguguucac      60

aguggcuaag uuccgccccc uggaccc                                          87


<210>  1312
<211>  97
<212>  RNA
<213>  Rattus norvegicus

<400>  1312
accucucuaa caaggugcag agcuuagcug auuggugaac agugauuggu uuccgcuuug      60

uucacagugg cuaaguucug caccugaaga gaaggug 97

<210> 1313
<211> 78
<212> RNA
<213> Ateles geoffroyi

<400> 1313
   cugaggagca gggcuuagcu gcuugugagc aggguucacu ccaagucaug uucacagugg 60
   cuaaguuccg ccccccag 78

<210> 1314
<211> 78
<212> RNA
<213> Saguinus labiatus

<400> 1314
   cugaggagca gggcuuagcu gcuugugagc aggguucacu ccaagucaug uucacagugg 60
   cuaaguuccg ccccccag 78

<210> 1315
<211> 78
<212> RNA
<213> Macaca mulatta

<400> 1315
   cugaggagca gggcuuagcu gcuugugagc aggguccaca ccaagucgug uucacagugg 60
   cuaaguuccg ccccccag 78

<210> 1316
<211> 79
<212> RNA
<213> Macaca nemestrina

<400> 1316
   cugaggagca gggcuuagcu gcuugugagc aggguccaca ccaagucgug uucacagugg 60
   cuaaguuccg cccccccag 79

<210> 1317
<211> 78
<212> RNA
<213> Gorilla gorilla

<400> 1317
   cugaggagca gggcuuagcu gcuugugagc aggguccaca ccaagucgug uucacagugg 60
   cuaaguuccg ccccccag 78

<210> 1318
<211> 78
<212> RNA
<213> Pan paniscus

<400> 1318
   cugaggagca gggcuuagcu gcuugugagc aggguccaca ccaagucgug uucacagugg 60
   cuaaguuccg ccccccag 78

<210>  1319
<211>  78
<212>  RNA
<213>  Pan troglodytes

<400>  1319
   cugaggagca gggcuuagcu gcuugugagc aggguccaca ccaagucgug uucacagugg      60
   cuaaguuccg ccccccag                                                     78


<210>  1320
<211>  78
<212>  RNA
<213>  Pongo pygmaeus

<400>  1320
   cugaggagca gggcuuagcu gcuugugagc aggguccaca ccaaguugug uucacagugg      60
   cuaaguuccg ccccccag                                                     78


<210>  1321
<211>  78
<212>  RNA
<213>  Lemur catta

<400>  1321
cugaggagca gggcuuagcu gcuugugagc aggguccaca ccaaaucaug uucacagugg      60

cuaaguuccg ccccccgg                                                    78


<210>  1322
<211>  86
<212>  RNA
<213>  Monodelphis domestica

<400>  1322
uggcuugagg agcaggacuu agcugccuug ugaacagagu cagcaucaua uuguguucac      60

aguggcuaag uuccgcuccc cuugcc                                           86


<210>  1323
<211>  97
<212>  RNA
<213>  Monodelphis domestica

<400>  1323
accucucuga caaggugcag agcuuagccg auuggugaac agucacugau uuccucuuug      60

uucacagugg cuaaguucug caccugaaga gaagggg                              97


<210>  1324
<211>  97
<212>  RNA
<213>  Gallus gallus

<400>  1324
accucucugg ugaggugcag agcuuagcug auuggugaac agugauuguu ucccucuuug      60

uucacagugg cuaaguucug caccugaaga gaaggug                              97

<210> 1325
<211> 76
<212> RNA
<213> Xenopus tropicalis

<400> 1325
cggcggccca gggcuuagcu guauugugag cacugucacu cucgcaccug uucacagugg 60

cuaaguuccg cgccuc 76

<210> 1326
<211> 96
<212> RNA
<213> Xenopus tropicalis

<400> 1326
accuuucuac caaggugcag agcuuagcug auuggugaac agugauugau ucccucuuug 60

uucacagugg cuaaguucug caccugaaga gagggc 96

<210> 1327
<211> 82
<212> RNA
<213> Xenopus tropicalis

<400> 1327
cucuagagag gcaggacuua gcuggcucug ugaacagguc uuguguguca auguucacag 60

uggcuaaguu ccaccccacu gg 82

<210> 1328
<211> 82
<212> RNA
<213> Xenopus tropicalis

<400> 1328
cucuagagag gcaggacuua gcuggcucug ugaacagguc uuguguguca auguucacag 60

uggcuaaguu ccaccccacu gg 82

<210> 1329
<211> 78
<212> RNA
<213> Bos taurus

<400> 1329
uggccugggg agcagggcuu agcugcuugu gagcaggucc acaucaaauc guguucacag 60

uggcuaaguu ccgccccc 78

<210> 1330
<211> 97
<212> RNA
<213> Bos taurus

<400> 1330
accucucuga cgaggugcag agcuuagcug auuggugaac agugacuggu uuccgcuuug 60

uucacagugg cuaaguucug caccugaaga gaaggug 97

<210> 1331
<211> 82

<212>  RNA
<213>  Sus scrofa

<400>  1331
uggccugggg agcagggcuu agcugcuugu gagcaggucc acagcaaguc guguucacag      60

uggcuaaguu ccgcccccug ga                                               82


<210>  1332
<211>  131
<212>  RNA
<213>  Danio rerio

<400>  1332
ucuggauaug augucugcug aaguuucgug aggugcagga cuuagcucac ucugugaaca      60

gaucucggau auccuauguu cacaguggcu aaguuccgcu ccucugaggc ccacacucga     120

aaucagccag g                                                          131


<210>  1333
<211>  91
<212>  RNA
<213>  Danio rerio

<400>  1333
ucuuuucuag caggugcaga gcuuagcuga uuggugaaca gugauugaac ucuuuguuca      60

caguggcuaa guucugcauc ugaggagagg a                                     91


<210>  1334
<211>  88
<212>  RNA
<213>  Danio rerio

<400>  1334
gguugugugg ugucaggacu uaacccacuu gugaacaaug caucgaacuu caauguucac      60

agugguuaag uucugccgcc ccuagacc                                         88


<210>  1335
<211>  89
<212>  RNA
<213>  Danio rerio

<400>  1335
uucugagcgg gugcagagcu uggcugauug gugaacgugc auggcuugug uuuuuguuca      60

caguggcuaa guucuucacc cgaaaagaa                                        89


<210>  1336
<211>  106
<212>  RNA
<213>  Danio rerio

<400>  1336
gucaucacuc gcucacggcg cagagcuuag cuaaucggug agcauugauc ccuuaagaaa      60

acuguucaca guggcuaagu ucagugucug gagugaacgg gaugac                    106


<210>  1337
<211>  74

<212>  RNA
<213>  Fugu rubripes

<400>  1337
aggcacagag cuuagcugau uggugaacag ugauugauuu ccucuuuguu cacaguggcu      60

aaguucugca ccug                                                        74


<210>  1338
<211>  78
<212>  RNA
<213>  Fugu rubripes

<400>  1338
ggcugugugg cggcaggacu uaacccacau gugagcagug agugugugcc auguucacag      60

ugguuaaguu cugccgcc                                                    78


<210>  1339
<211>  100
<212>  RNA
<213>  Fugu rubripes

<400>  1339
cacuugcuaa aggcaccgag cuuagcuaau uggugagcau ugaucccugc uauguguugu      60

ucacaguggc uaaguucagu gccugaggug aaauaguuga                           100


<210>  1340
<211>  74
<212>  RNA
<213>  Tetraodon nigroviridis

<400>  1340
aggcacagag cuuagcugau uggugaacag ugauugauuu ccucuuuguu cacaguggcu      60

aaguucugca ccug                                                        74


<210>  1341
<211>  76
<212>  RNA
<213>  Tetraodon nigroviridis

<400>  1341
ggcugugcgg cggcaggacu uaacccacau gugaacagug agugugccgu guucacagug      60

guuaaguucu gccgcc                                                      76


<210>  1342
<211>  83
<212>  RNA
<213>  Tetraodon nigroviridis

<400>  1342
cucuugcuga aggcacagag cuuagcuaau uggugagcau ugaucccugc uauguguugu      60

ucacaguggc uaaguucagu gcc                                              83


<210>  1343
<211>  86
<212>  RNA
<213>  Mus musculus

<400>  1343
ggucccuacc uucaaggagc ucacagucua uugaguugcc uuucugauuc ucccacuaga      60

uugugagcug cuggagggca ggcacu                                           86


<210>  1344
<211>  86
<212>  RNA
<213>  Rattus norvegicus

<400>  1344
ggucccuacc cgcaaggagc ucacagucua uugaguuccu uuucugauuc ucccacuaga      60

uugugagcuc cuggagggca ggcacu                                           86


<210>  1345
<211>  86
<212>  RNA
<213>  Ateles geoffroyi

<400>  1345
gguccuugcc cucaaggagc ucacagucua uugaguugcc uuucugacuu ucccacuaga      60

uugugagcuc cuggagggca ggcacu                                           86


<210>  1346
<211>  86
<212>  RNA
<213>  Lagothrix lagotricha

<400>  1346
gguccuugcc cucaaggagc ucacagucua uugaguugcc uuucugacuu ucccacuaga      60

uugugagcuc cuggagggca ggcacu                                           86


<210>  1347
<211>  86
<212>  RNA
<213>  Saguinus labiatus

<400>  1347
gguccuugcc cucaaggagc ucacagucua uugaguugcc uuucuaacuu ucccacuaga      60

uuguaagcuc cuggagggca ggcacu                                           86


<210>  1348
<211>  86
<212>  RNA
<213>  Macaca mulatta

<400>  1348
gguccuugcc cucaaggagc ucacagucua uugaguuucc uuugugacuu ucccacuaga      60

uugugagcuc cuggagggca ggcacu                                           86


<210>  1349
<211>  86
<212>  RNA
<213>  Macaca nemestrina

<400>  1349

gguccuugcc cucaaggagc ucacagucua uugaguuucc uuucugacuu ucccacuaga 60

uugugagcuc cuggagggca ggcacu 86

<210> 1350
<211> 86
<212> RNA
<213> Gorilla gorilla

<400> 1350
gguccuugcc cucaaggagc ucacagucua uugaguuacc uuucugacuu ucccacuaga 60

uugugagcuc cuggagggca ggcacu 86

<210> 1351
<211> 86
<212> RNA
<213> Pan paniscus

<400> 1351
gguccuugcc cucaaggagc ucacagucua uugaguuacc uuucugacuu ucccacuaga 60

uugugagcuc cuggagggca ggcacu 86

<210> 1352
<211> 86
<212> RNA
<213> Pan troglodytes

<400> 1352
gguccuugcc cucaaggagc ucacagucua uugaguuacc uuucugacuu ucccacuaga 60

uugugagcuc cuggagggca ggcacu 86

<210> 1353
<211> 86
<212> RNA
<213> Pongo pygmaeus

<400> 1353
gguccuugcc cucaaggagc ucacagucua uugaguuacc uuucugauuu ucccacuaga 60

uugugagcuc cuggagggca ggcacu 86

<210> 1354
<211> 86
<212> RNA
<213> Sus scrofa

<400> 1354
gguccuugcc cucaaggagc ucacacucua uugaguugcc uuucugucuu ccccacuaga 60

uugugagcuc cuggagggca ggcacu 86

<210> 1355
<211> 88
<212> RNA
<213> Mus musculus

<400> 1355
accccuuaga ggaugacuga uuucuuuugg uguucagagu caauagaauu uucuagcacc 60

aucugaaauc gguuauaaug auugggga                                          88

<210>  1356
<211>  88
<212>  RNA
<213>  Rattus norvegicus

<400>  1356
accccuuaga ggaugacuga uuucuuuugg uguucagagu caauagaauu uucuagcacc      60

aucugaaauc gguuauaaug auugggga                                          88

<210>  1357
<211>  81
<212>  RNA
<213>  Rattus norvegicus

<400>  1357
cuucaggaag cugguuucau auggugguuu agauuuaaau agugauuguc uagcaccauu      60

ugaaaucagu guucuuggug g                                                 81

<210>  1358
<211>  81
<212>  RNA
<213>  Rattus norvegicus

<400>  1358
cuucuggaag cugguuucac augguggcuu agauuuuucc aucuuuguau cuagcaccau      60

uugaaaucag uguuuuagga g                                                 81

<210>  1359
<211>  81
<212>  RNA
<213>  Ateles geoffroyi

<400>  1359
cuucaggaag cugguuucau auggugguuu agauuuaaau agugauuguc uagcaccauu      60

ugaaaucagu guucuugggg g                                                 81

<210>  1360
<211>  81
<212>  RNA
<213>  Lagothrix lagotricha

<400>  1360
cuucaggaag cugguuucau auggugguuu agauuuaaau agugauuguc uagcaccauu      60

ugaaaucagu guucuugggg g                                                 81

<210>  1361
<211>  81
<212>  RNA
<213>  Saguinus labiatus

<400>  1361
cuucuggaag cugguuucac augguggcuu agauuuuucc aucuuuguau cuagcaccau      60

uugaaaucag uguuuuagga g                                                 81

<210>  1362
<211>  81
<212>  RNA
<213>  Macaca nemestrina

<400>  1362
cuucuggaag cugguuucac augguggcuu agauuuuucc aucuuuguau cuagcaccau      60

uugaaaucag uguuuuagga g                                                81


<210>  1363
<211>  81
<212>  RNA
<213>  Gorilla gorilla

<400>  1363
cuucaggaag cugguuucau augguggguu agauuuaaau agugauuguc uagcaccauu      60

ugaaaucagu guucuugggg g                                                81


<210>  1364
<211>  81
<212>  RNA
<213>  Gorilla gorilla

<400>  1364
cuucuggaag cugguuucac augguggcuu agauuuuucc aucuuuguau cuagcaccau      60

uugaaaucag uguuuuagga g                                                81


<210>  1365
<211>  81
<212>  RNA
<213>  Pan paniscus

<400>  1365
cuucaggaag cugguuucau augguggguu agauuuaaau agugauuguc uagcaccauu      60

ugaaaucagu guucuugggg g                                                81


<210>  1366
<211>  81
<212>  RNA
<213>  Pan paniscus

<400>  1366
cuucuggaag cugguuucac augguggcuu agauuuuucc aucuuuguau cuagcaccau      60

uugaaaucag uguuuuagga g                                                81


<210>  1367
<211>  81
<212>  RNA
<213>  Pan troglodytes

<400>  1367
cuucaggaag cugguuucau augguggguu agauuuaaau agugauuguc uagcaccauu      60

ugaaaucagu guucuugggg g                                                81


<210>  1368

<211>  81
<212>  RNA
<213>  Pan troglodytes

<400>  1368
cuucuggaag cugguuucac augguggcuu agauuuuucc aucuuuguau cuagcaccau      60

uugaaaucag uguuuuagga g                                                81


<210>  1369
<211>  81
<212>  RNA
<213>  Pongo pygmaeus

<400>  1369
   cuucaggaag cugguuucau augguggguu agauuuaaau agugauuguc uagcaccauu      60
   ugaaaucagu guucuugggg g                                                81


<210>  1370
<211>  81
<212>  RNA
<213>  Pongo pygmaeus

<400>  1370
cuucuggaag cugguuucac augguggcuu agauuuuucc aucuuuguau cuagcaccau      60

uugaaaucag uguuuuagga g                                                81


<210>  1371
<211>  64
<212>  RNA
<213>  Monodelphis domestica

<400>  1371
augacugauu ucuuuuggug uucagaguca auaucauuuu cuagcaccau uugaaaucgg      60

uuau                                                                   64


<210>  1372
<211>  86
<212>  RNA
<213>  Monodelphis domestica

<400>  1372
gcuucuucag gaagcugguu ucauauggug guuuagauuu aacuacugag ugucuagcac      60

cauuugaaau caguguucuu ggagga                                           86


<210>  1373
<211>  89
<212>  RNA
<213>  Gallus gallus

<400>  1373
accccuuuag aggaugacug auuucuuuug guguucagag ucaauaauau uuucuagcac      60

cauuugaaau cgguuauagu gauuggga                                         89


<210>  1374
<211>  81
<212>  RNA

<213>  Gallus gallus

<400>  1374
ccucaggaag cugguuucau auggugguuu agauuuaacu auucauuguc uagcaccauu      60

ugaaaucagu guucuuggag g                                                81


<210>  1375
<211>  80
<212>  RNA
<213>  Gallus gallus

<400>  1375
ccucuggaag cugguuucac augguggcuu agauuuuccc acuuuguauc uagcaccauu      60

ugaaaucagu guucuaggag                                                  80


<210>  1376
<211>  87
<212>  RNA
<213>  Xenopus tropicalis

<400>  1376
acccuuuuaa aggaugacug auuucuucug guguucagag ucuuuuguuu ucuagcacca      60

uuugaaaucg guuauaauga uggggua                                          87


<210>  1377
<211>  80
<212>  RNA
<213>  Xenopus tropicalis

<400>  1377
ucccggaagc ugguuucuug uggugacuua gauuuaucca ucgcugcauc uagcaccauu      60

ugaaaucagu guucuaggag                                                  80


<210>  1378
<211>  75
<212>  RNA
<213>  Xenopus tropicalis

<400>  1378
ucaggaggcu gguuucaugu ggugguuuag auuuaacugc acaaugucua gcaccauaug      60

aaaucagugu ccuua                                                       75


<210>  1379
<211>  81
<212>  RNA
<213>  Bos taurus

<400>  1379
cuucuggaag cugguuucac augguggcuu agauuuuucc aucuuuguau cuagcaccau      60

uugaaaucag uguuuuagga g                                                81


<210>  1380
<211>  81
<212>  RNA
<213>  Sus scrofa

<400>  1380
cuucaggaag cugguuucau auggugguuu agauuuaaaa agugauuguc uagcaccauu      60

ugaaaucagu guucuugggg g                                                81


<210>  1381
<211>  126
<212>  RNA
<213>  Danio rerio

<400>  1381
ugaagacccu caucucucuc ucucucuccc caccaaacga ugacugauuu ccuuuggugc      60

uuagaguccc aucugucauc uagcaccauu ugaaaucggu uauaaugacu ggggaucaau     120

ucuuca                                                                126


<210>  1382
<211>  71
<212>  RNA
<213>  Danio rerio

<400>  1382
agguugaccg auuucuuuug guguucagag ucuuuugggg uuucuagcac cauuugaaau      60

cgguuacaau g                                                           71


<210>  1383
<211>  83
<212>  RNA
<213>  Danio rerio

<400>  1383
cugcuccugg aagcugaauu cagauggugc cauagaguau uuuauggcau cuagcaccau      60

uugaaaucag uguuccuggg ccg                                              83


<210>  1384
<211>  90
<212>  RNA
<213>  Danio rerio

<400>  1384
ucuccuccug gaagcugguu ucaaguggug uuuuagagug cugaucaaau gaaucuagca      60

ccauuugaaa ucaguguucu uggggugaga                                       90


<210>  1385
<211>  88
<212>  RNA
<213>  Danio rerio

<400>  1385
ucuuccucca gaugcugguu ucacauggug guuuagaugu guucuaccaa agucuagcac      60

cauuugaaau caguguucuu ggggaggg                                         88


<210>  1386
<211>  71
<212>  RNA
<213>  Fugu rubripes

<400>  1386
agguugacug auuucuuuug guguucagag uccaauucug uuucuagcac cauuugaaau      60

cgguuacagu g                                                           71


<210>  1387
<211>  126
<212>  RNA
<213>  Fugu rubripes

<400>  1387
gaagacaguc uuggacuccc uuccccgucc uccaccaaaa gaugacugau uucuucuggu      60

gcugagaguc ggcugcagcc uucuagcacc auuugaaauc gguuauaaaa cagugggucg    120

auacau                                                                126


<210>  1388
<211>  77
<212>  RNA
<213>  Fugu rubripes

<400>  1388
uccagaagcu gguuucaugu ggugguuuag augugucuuc ccucuugucu agcaccauuu      60

gaaaucagug uucuugg                                                     77


<210>  1389
<211>  74
<212>  RNA
<213>  Fugu rubripes

<400>  1389
uggaagcugg uuucaggugg uggcuuagag uauugucauc uaucuagcac cauuugaaau      60

caguguucuu gggg                                                        74


<210>  1390
<211>  72
<212>  RNA
<213>  Tetraodon nigroviridis

<400>  1390
agguugaccg auuucuuuug guguucagag uccaauucug uuucuagcac cauuugaaau      60

cgguuacagu ga                                                          72


<210>  1391
<211>  100
<212>  RNA
<213>  Tetraodon nigroviridis

<400>  1391
uguuucucca ccaaaagaug acugauuucc ucuggugcuu agagccgccu gcagccuucu      60

agcaccauuu gaaaucgguu auaaaacugu ggaucagugc                           100


<210>  1392
<211>  79
<212>  RNA
<213>  Tetraodon nigroviridis

<400>  1392
cuccagaagc ugguuucacg uggugguuua gaugugucuc cucaucgucu agcaccauuu      60

gaaaucagug uucuugggg                                                     79


<210>  1393
<211>  78
<212>  RNA
<213>  Tetraodon nigroviridis

<400>  1393
cucuuggaag cugguuucag gugguggcuu agaguauugu caucuaucua gcaccauuug      60

aaaucagugu ucuugggg                                                      78


<210>  1394
<211>  88
<212>  RNA
<213>  Mus musculus

<400>  1394
aucucuuaca caggcugacc gauuucuccu gguguucaga gucuguuuuu gucuagcacc      60

auuugaaauc gguuaugaug uaggggga                                           88


<210>  1395
<211>  88
<212>  RNA
<213>  Rattus norvegicus

<400>  1395
aucucuuaca caggcugacc gauuucuccu gguguucaga gucuguuuuu gucuagcacc      60

auuugaaauc gguuaugaug uaggggga                                           88


<210>  1396
<211>  89
<212>  RNA
<213>  Gallus gallus

<400>  1396
ucucuuacac aggcugaccg auuucucuug guguucagag ucucaguuuc ugucuagcac      60

cauuugaaau cgguuaugau guaggggga                                          89


<210>  1397
<211>  90
<212>  RNA
<213>  Xenopus tropicalis

<400>  1397
ucccuuacac aggaugaccg aucucucuug guguucagag gcucaggucu ucaucuagca      60

ccauuugaaa ucgguuauaa uguaagguga                                        90


<210>  1398
<211>  88
<212>  RNA
<213>  Bos taurus

<400>  1398
aucucuuaca caggcugacc gauuucuccu gguguucaga gucuguuuuu gucuagcacc      60

auuugaaauc gguuaugaug uaggggga                                          88

<210>  1399
<211>  88
<212>  RNA
<213>  Sus scrofa

<220>
<221>  misc_feature
<222>  (83)..(83)
<223>  n is a, c, g, or u

<400>  1399
aucucuuaca caggcugacc gauuucuccu gguguucaga gucuguuuuu gucuagcacc      60

auuugaaauc gguuaugaug uanggggga                                          88

<210>  1400
<211>  96
<212>  RNA
<213>  Mus musculus

<400>  1400
cuaagccaag uuucaguuca uguaaacauc cuacacucag cugucauaca ugcguuggcu      60

gggaugugga uguuuacguc agcugucuug gaguau                                96

<210>  1401
<211>  82
<212>  RNA
<213>  Mus musculus

<400>  1401
aagucugugu cuguaaacau ccccgacugg aagcuguaag ccacagccaa gcuuucaguc      60

agauguuugc ugcuacuggc uc                                                82

<210>  1402
<211>  92
<212>  RNA
<213>  Mus musculus

<400>  1402
gggcagucuu ugcuacugua aacauccuug acuggaagcu guaagguguu gagaggagcu      60

uucagucgga uguuuacagc ggcaggcugc ca                                    92

<210>  1403
<211>  71
<212>  RNA
<213>  Rattus norvegicus

<400>  1403
gcaacuguaa acauccucga cuggaagcug ugaagccaca aaugggcuuu cagucggaug      60

uuugcagcug c                                                            71

<210>  1404
<211>  82
<212>  RNA

<213>  Rattus norvegicus

<400>  1404
aagucugugu cuguaaacau ccccgacugg aagcuguaag ccacagccaa gcuuucaguc      60

agauguuugc ugcuacuggc uc                                                82


<210>  1405
<211>  92
<212>  RNA
<213>  Rattus norvegicus

<400>  1405
gggcagucuu ugcuacugua aacauccuug acuggaagcu guaagguguu gagaggagcu      60

uucagucgga uguuuacagc ggcaggcugc ca                                    92


<210>  1406
<211>  88
<212>  RNA
<213>  Ateles geoffroyi

<400>  1406
accgaguuuc aguucaugua aacauccuac acucagcugu aauacaugga uuggcugaga      60

gguggauguu uacuucagcu gacuugga                                          88


<210>  1407
<211>  88
<212>  RNA
<213>  Lagothrix lagotricha

<400>  1407
accgaguuuc aguucaugua aacauccuac acucagcugu aauacaugga uuggcugaga      60

gguggauguu uacuucagcu gacuugga                                          88


<210>  1408
<211>  71
<212>  RNA
<213>  Macaca mulatta

<400>  1408
gcgacuguaa acauccucga cuggaagcug ugaagccaua gaugggcuuu cagucggaug      60

uuugcagcug c                                                            71


<210>  1409
<211>  88
<212>  RNA
<213>  Macaca mulatta

<400>  1409
accaaguuuc aguucaugua aacauccuac acucagcugu aauacaugga uuggcuggga      60

gguggauguu uacuucagcu gacuugga                                          88


<210>  1410
<211>  88
<212>  RNA
<213>  Macaca nemestrina

<400>  1410
accaaguuuc aguucaugua aacauccuac acucagcugu aauacaugga uuggcuggga      60

gguggauguu uacuucagcu gacuugga                                          88


<210>  1411
<211>  70
<212>  RNA
<213>  Macaca nemestrina

<400>  1411
guuguuguaa acauccccga cuggaagcug uaagacacag cuaagcuuuc agucagaugu      60

uugcugcuac                                                              70


<210>  1412
<211>  71
<212>  RNA
<213>  Gorilla gorilla

<400>  1412
gcgacuguaa acauccucga cuggaagcug ugaagccaca gaugggcuuu cagucggaug      60

uuugcagcug c                                                            71


<210>  1413
<211>  88
<212>  RNA
<213>  Gorilla gorilla

<400>  1413
accaaguucc aguucaugua aacauccuac acucagcugu aauacaugga uuggcuggga      60

gguggauguu uacuucagcu gacuugga                                          88


<210>  1414
<211>  70
<212>  RNA
<213>  Gorilla gorilla

<400>  1414
guuguuguaa acauccccga cuggaagcug uaagacacag cuaagcuuuc agucagaugu      60

uugcugcuac                                                              70


<210>  1415
<211>  71
<212>  RNA
<213>  Pan paniscus

<400>  1415
gcgacuguaa acauccucga cuggaagcug ugaagccaca gaugggcuuu cagucggaug      60

uuugcagcug c                                                            71


<210>  1416
<211>  88
<212>  RNA
<213>  Pan paniscus

<400>  1416
accaaguuuc aguucaugua aacauccuac acucagcugu aauacaugga uuggcuggga      60

gguggauguu uacuucagcu gacuugga                                          88

<210>  1417
<211>  70
<212>  RNA
<213>  Pan paniscus

<400>  1417
guuguuguaa acauccccga cuggaagcug uaagacacag cuaagcuuuc agucagaugu      60

uugcugcuac                                                              70

<210>  1418
<211>  71
<212>  RNA
<213>  Pan troglodytes

<400>  1418
gcgacuguaa acauccucga cuggaagcug ugaagccaca gaugggcuuu cagucggaug      60

uuugcagcug c                                                            71

<210>  1419
<211>  88
<212>  RNA
<213>  Pan troglodytes

<400>  1419
accaaguuuc aguucaugua aacauccuac acucagcugu aauacaugga uuggcuggga      60

gguggauguu uacuucagcu gacuugga                                          88

<210>  1420
<211>  70
<212>  RNA
<213>  Pan troglodytes

<400>  1420
guuguuguaa acauccccga cuggaagcug uaagacacag cuaagcuuuc agucagaugu      60

uugcugcuac                                                              70

<210>  1421
<211>  71
<212>  RNA
<213>  Pongo pygmaeus

<400>  1421
gcgacuguaa acauccucga cuggaagcug ugaagccaca gaugggcuuu cagucggaug      60

uuugcagcug c                                                            71

<210>  1422
<211>  72
<212>  RNA
<213>  Monodelphis domestica

<400>  1422
gcggcuguaa acauccucga cuggaagcug ugaagcagca gauggggcuu ucagucggau      60

guuugcagcu gc                                                           72

<210>  1423
<211>  72
<212>  RNA
<213>  Gallus gallus

<400>  1423
gcgacuguaa acauccucga cuggaagcug ugaagcagca gauggggcuu ucagucggau      60

guuugcagcu gc                                                          72


<210>  1424
<211>  87
<212>  RNA
<213>  Gallus gallus

<400>  1424
cuaacuuuua guuccuguaa acauccuaca cucagcuaua acaaguggua gggcuggggg      60

guggauguuu acuucaacug acuugga                                          87


<210>  1425
<211>  64
<212>  RNA
<213>  Gallus gallus

<400>  1425
guugcuguaa acauccccga cuggaagcug uagcagcuug agcuuucagu cagauguuug      60

cugc                                                                   64


<210>  1426
<211>  96
<212>  RNA
<213>  Gallus gallus

<400>  1426
cggggcaguc aucgcugcug uaaacauccu ugacuggaag cugugaggug ucagcggggg      60

cuuucagucg gauguuuaca gcugcaggcu gcugca                                96


<210>  1427
<211>  71
<212>  RNA
<213>  Xenopus tropicalis

<400>  1427
gcgacuguaa acauccucga cuggaagcug ugaagcaguu gaagggcuuu cagucagaug      60

uuugcagcug c                                                           71


<210>  1428
<211>  80
<212>  RNA
<213>  Xenopus tropicalis

<400>  1428
guauuagucu auguaaacau ccuacacuca gcucguaugu auaaccugac uggguggggg      60

guguuugccu cgacugaucu                                                  80

<210>  1429
<211>  81
<212>  RNA
<213>  Xenopus tropicalis

<400>  1429
   aguuugucgc uguaaacauc cccgacugga agcugugagg cugcauuuga gcuuucaguc      60
   ugguguuugc ugcuaccggc u                                               81

<210>  1430
<211>  90
<212>  RNA
<213>  Xenopus tropicalis

<400>  1430
gggcaguuac agccucugua aacauccuug acuggaagcu gugaaauaau gauaguagcu      60

uucagucgga uguuuacagc ugcuagcugc                                       90

<210>  1431
<211>  67
<212>  RNA
<213>  Bos taurus

<400>  1431
cuguaaacau ccucgacugg aagcugugag gcugcagaaa ggcuuucagu cggauguuug      60

cagcugc                                                                67

<210>  1432
<211>  88
<212>  RNA
<213>  Bos taurus

<400>  1432
ccaaguuuuc aguucaugua aacauccuac acucagcugu aacacacgag ucggcuggga      60

gguggauguu uacuucagcu gacuugga                                         88

<210>  1433
<211>  70
<212>  RNA
<213>  Bos taurus

<400>  1433
guuguuguaa acauccccga cuggaagcug uaccacacag cuaagcuuuc agucagaugu      60

uugcugcuac                                                             70

<210>  1434
<211>  92
<212>  RNA
<213>  Bos taurus

<400>  1434
gggcagucuu ugcuacugua aacauccuug acuggaagcu guaaggcguu gcaaggagcu      60

uucagucgga uguuuacagc ggcaggcugc ca                                    92

<210>  1435
<211>  89

<212>  RNA
<213>  Danio rerio

<400>  1435
ggcuccuugc aguuguaaac auucccgacu ggaaguugua augcagaaaa ucucagcuuu      60

cagucugaug uuugcugcua cugguggcc                                        89


<210>  1436
<211>  88
<212>  RNA
<213>  Danio rerio

<400>  1436
uuccagugua gucgcuguaa acauccuaca cucagcugug agcugcagac gaggcugggc      60

ggaggguguu ugcugugacu gucuggag                                         88


<210>  1437
<211>  128
<212>  RNA
<213>  Danio rerio

<400>  1437
gugcuguaua uggguauaac ugguuguuca ugccuguaaa cauccccgac uggaagcugu      60

gcuacgcgga aaacgagcuu ucaguuggau guuugcuguc aucguccagu ucugucgccu     120

uguauuac                                                              128


<210>  1438
<211>  80
<212>  RNA
<213>  Danio rerio

<400>  1438
uacgggcuac uguaaacauc cuugacugga agcuggugca caugauggag cuuucagucg      60

gauguuugca gcagccaacu                                                  80


<210>  1439
<211>  90
<212>  RNA
<213>  Fugu rubripes

<400>  1439
uuccggcuca guugguguaa acauccuaca cucagcugug auccuuugcg gcagaggcug      60

agagaagguu guuuacuuga acuggcugga                                       90


<210>  1440
<211>  98
<212>  RNA
<213>  Fugu rubripes

<400>  1440
ugcacauggc aguuugaggc uguaaacauc cccgacugga agcugucacu cagaagagcu      60

uucagucuga uguuuaccac cuccgaccgc cuccugcu                              98


<210>  1441
<211>  81

<212>  RNA
<213>  Tetraodon nigroviridis

<400>  1441
agauggugua aacauccuac acucagcugu gaucuuuugc cgcagaggcu gagagaaggu      60

uguuuacuug aacuggcugg a                                                81


<210>  1442
<211>  86
<212>  RNA
<213>  Tetraodon nigroviridis

<400>  1442
uggcaguuug aggcuguaaa cauccccgac uggaagcugu cacucagcag agcuuucagu      60

cugauguuua cccccuccga ccgccu                                           86


<210>  1443
<211>  89
<212>  RNA
<213>  Mus musculus

<400>  1443
accauguugu agugugugua aacauccuac acucucagcu gugagcucaa gguggcuggg      60

agaggguugu uuacuccuuc ugccaugga                                        89


<210>  1444
<211>  84
<212>  RNA
<213>  Mus musculus

<400>  1444
gagugacaga uauuguaaac auccuacacu cucagcugug aaaaguaaga aagcugggag      60

aaggcuguuu acucucucug ccuu                                             84


<210>  1445
<211>  89
<212>  RNA
<213>  Rattus norvegicus

<400>  1445
accauguugu agugugugua aacauccuac acucucagcu gugagcucaa gguggcuggg      60

agaggguugu uuacuccuuc ugccaugga                                        89


<210>  1446
<211>  84
<212>  RNA
<213>  Rattus norvegicus

<400>  1446
gagugacaga uacuguaaac auccuacacu cucagcugug aaaaguaaga aagcugggag      60

aaggcuguuu acucucucug ccuu                                             84


<210>  1447
<211>  72
<212>  RNA
<213>  Lagothrix lagotricha

<400>  1447
agauacugua aacauccuac acucucagcu guggaaagua agaaagcugg gagaaggcug      60

uuuacucuuu cu                                                          72


<210>  1448
<211>  72
<212>  RNA
<213>  Macaca nemestrina

<400>  1448
agauacugua aacauccuac acucucagcu guggaaagua agaaagcugg gagaaggcug      60

uuuacucuuu cu                                                          72


<210>  1449
<211>  72
<212>  RNA
<213>  Pan troglodytes

<400>  1449
agauacugua aacauccuac acucucagcu guggaaagua agaaagcugg gagaaggcug      60

uuuacucuuu cu                                                          72


<210>  1450
<211>  89
<212>  RNA
<213>  Gallus gallus

<400>  1450
accaugcugu agcacgugua aacauccuac acucucagcu gugaacucga gguggcuggg      60

agaggauugu uuacgccuuc ugccaugga                                        89


<210>  1451
<211>  72
<212>  RNA
<213>  Gallus gallus

<400>  1451
agguacugua aacauccuac acucucagcu guggaaacua agaaagcugg gagaaggcug      60

uuuacucucc cu                                                          72


<210>  1452
<211>  89
<212>  RNA
<213>  Xenopus tropicalis

<400>  1452
accaugcagc agugauugua aacauccuac acucucagcu gugaacauaa gguggcuggg      60

agaagggugu uuacuccccc ugccaugga                                        89


<210>  1453
<211>  78
<212>  RNA
<213>  Xenopus tropicalis

<400>  1453

gagagacaga uacuguaaac auccuacacu cucagcugug gaaauacaaa gcugggggaa     60

ggcuguuuac ucucucug                                                   78


<210>  1454
<211>  105
<212>  RNA
<213>  Bos taurus

<400>  1454
cagacuguaa ccaugccgua guguguguaa acauccuaca cucucagcug ugagcucgag     60

guggcuggga gaggguuguu uacuccuucu gccauggaaa acguc                    105


<210>  1455
<211>  80
<212>  RNA
<213>  Sus scrofa

<400>  1455
gacagauacu guaaacaucc uacacucuca gcuguggaaa guaagaaagc ugggagaagg     60

cuguuuacuc ucucugccuu                                                 80


<210>  1456
<211>  79
<212>  RNA
<213>  Danio rerio

<400>  1456
cuucagggag uguaaacauc cuacacucuc agcuggagcg cagccgaggc cgggaguggg     60

auguuugcgc ucucuggcu                                                  79


<210>  1457
<211>  78
<212>  RNA
<213>  Fugu rubripes

<400>  1457
   ucaggaagug uaaacauccu acacucucgg cguccgcccc cugguggccg ggggugggac      60
   uguuugcacu gccugacu                                                   78


<210>  1458
<211>  79
<212>  RNA
<213>  Tetraodon nigroviridis

<400>  1458
   uucagggagu guaaacaucc uacacucucg gcguccgccc ccugguggcc ggggguggga      60
   cuguuugcac ugccuggcu                                                  79


<210>  1459
<211>  80
<212>  RNA
<213>  Ateles geoffroyi

<400>  1459
cugggggcuc caaagugcug uucgugcagg uagugugauu accugaccua cugcugagcu     60

agcacuuccc gagccccgg 80

<210> 1460
<211> 80
<212> RNA
<213> Lagothrix lagotricha

<400> 1460
cucggggcuc caaagugcug uucgugcagg uagugugauu accugaccua cugcugagcu 60

agcacuuccc gagccccgg 80

<210> 1461
<211> 80
<212> RNA
<213> Saguinus labiatus

<400> 1461
cugggggcuc caaagugcug uucgugcagg uagugugauu acccaaccua cugcugagcu 60

agcacuuccc gagccccgg 80

<210> 1462
<211> 80
<212> RNA
<213> Macaca mulatta

<400> 1462
cugggggcuc caaagugcug uucgugcagg uagugugauu aaccgaccua cugcugagcu 60

agcacuuccc gagccccag 80

<210> 1463
<211> 80
<212> RNA
<213> Macaca nemestrina

<400> 1463
cugggggcuc caaagugcug uucgugcagg uagugugauu aaccgaccua cugcugagcu 60

agcacuuccc gagccccag 80

<210> 1464
<211> 80
<212> RNA
<213> Gorilla gorilla

<400> 1464
cugggggcuc caaagugcug uucgugcagg uagugugauu acccgaccua cugcugagcu 60

agcacuuccc gagccccgg 80

<210> 1465
<211> 80
<212> RNA
<213> Pan paniscus

<400> 1465
cugggggcuc caaagugcug uucgugcagg uagugugauu acccgaccua cugcugagcu 60

agcacuuccc gagccccgg 80

<210>  1466
<211>  80
<212>  RNA
<213>  Pan troglodytes

<400>  1466
cugggggcuc caaagugcug uucgugcagg uagugugauu acccgaccua cugcugagcu      60

agcacuuccc gagcccccgg                                                  80


<210>  1467
<211>  80
<212>  RNA
<213>  Pongo pygmaeus

<400>  1467
cugggggcuc caaagugcug uucgugcagg uagugugauu acccgaccua cugcugagcu      60

agcacuuccc gagcccccgg                                                  80


<210>  1468
<211>  89
<212>  RNA
<213>  Monodelphis domestica

<400>  1468
agucuugggg ggcuccaaag ugcuguucgu gcagguagug ugauaaccug accuacugcu      60

gagcuagcac uuccagagcc ccugggaca                                        89


<210>  1469
<211>  74
<212>  RNA
<213>  Mus musculus

<400>  1469
guucccuuca acuuuaacau gggaaugcuu ucugucucau cgaagaguaa gugcuuccau      60

guuuuaguag aagu                                                        74


<210>  1470
<211>  66
<212>  RNA
<213>  Mus musculus

<400>  1470
ccuuuacuuu aacauggagg cacuugcugu gcauuuaaaa auaagugcuu ccauguuuga      60

gugugg                                                                 66


<210>  1471
<211>  70
<212>  RNA
<213>  Monodelphis domestica

<400>  1471
cccacuacuu aaacguggau uuacuugcuu uguuucuaaa aaaguaagug cuuccauguu      60

uuagugaugg                                                             70


<210>  1472

<211>  71
<212>  RNA
<213>  Monodelphis domestica

<400>  1472
ccccuucuac uuuaacaugg agguacuuuc uguguuuaaa aagguaagug cuuccauguu      60

uugguaggag u                                                           71


<210>  1473
<211>  59
<212>  RNA
<213>  Monodelphis domestica

<400>  1473
gcuuuaacau gggguaccu gcuacguaau aaaaaguaag ugcuuccaug uuucagugg      59


<210>  1474
<211>  71
<212>  RNA
<213>  Monodelphis domestica

<400>  1474
ccucugcuuu aacauggaag ugcuugcugu gauuuuaaaa aauuaagugc uuccauguuu      60

gaguuguggu g                                                           71


<210>  1475
<211>  72
<212>  RNA
<213>  Gallus gallus

<400>  1475
cccuuuuacu uuaacaugga ggugcuuucu gugauuuuac aaaaguaagu gcuuccaugu      60

uuuaguagag gu                                                          72


<210>  1476
<211>  65
<212>  RNA
<213>  Gallus gallus

<400>  1476
ccuccgcuuu aacauggagg uaccugcugc cuacaaaagu aagugcuucc auguuucagu      60

ggugg                                                                  65


<210>  1477
<211>  69
<212>  RNA
<213>  Gallus gallus

<400>  1477
ccucaacuuu aacauggagg uacuugcugg acaccugaaa aaguaagugc uuccauguuu      60

uaguugugg                                                              69


<210>  1478
<211>  69
<212>  RNA
<213>  Xenopus tropicalis

<400>  1478
ccccacuacu uuaacauugg uguacuuucu augucuuuaa aaaggguaag ugcuccaaug      60

uuuuagugg                                                              69


<210>  1479
<211>  81
<212>  RNA
<213>  Lagothrix lagotricha

<400>  1479
aacacagugg gcacucaaua aaugucuguu gaauugaaau gcguuacauu caacggguau      60

uuauugagca cccacucugu g                                                81


<210>  1480
<211>  81
<212>  RNA
<213>  Saguinus labiatus

<400>  1480
aacacagugg gcacucaaua aaugucuguu gaauugaaau gcguuacauu caacggguau      60

uuauugagca cccacucugu g                                                81


<210>  1481
<211>  81
<212>  RNA
<213>  Gorilla gorilla

<400>  1481
aacacagugg gcacucaaua aaugucuguu gaauugaaau gcguuacauu caacggguau      60

uuauugagca cccacucugu g                                                81


<210>  1482
<211>  81
<212>  RNA
<213>  Pan paniscus

<400>  1482
aacacagugg gcacucaaua aaugucuguu gaauugaaau gcauuacauu caacggguau      60

uuauugagca cccacucugu g                                                81


<210>  1483
<211>  81
<212>  RNA
<213>  Pan troglodytes

<400>  1483
aacacagugg gcacucaaua aaugucuguu gaauugaaau gcguuacauu caacggguau      60

uuauugagca cccacucugu g                                                81


<210>  1484
<211>  81
<212>  RNA
<213>  Pongo pygmaeus

<400>  1484
aacacagugg gcacucaaua aaugucuguu gaauugaaau gcguuacauu caacggguau      60

uuauugagca cccacucugu g                                              81

<210>  1485
<211>  81
<212>  RNA
<213>  Sus scrofa

<400>  1485
aacacagugg gcgcucaaua aauguuuguu gaauugagau gcguuaaauu caacgguau      60

uuauugagca cccacucugu g                                              81

<210>  1486
<211>  108
<212>  RNA
<213>  Mus musculus

<400>  1486
cugcacaugc uggggugagg uaguaaguug uauuguugug ggguagggau uuuaggcccc      60

aguaagaaga uaacuauaca acuuacuacu uuccuuggug uguggcau                108

<210>  1487
<211>  108
<212>  RNA
<213>  Rattus norvegicus

<400>  1487
cugcacaugc uggggugagg uaguaaguug uauuguugug ggguagggau uuuaggcccc      60

aauaagaaga uaacuauaca acuuacuacu uucccuggug uguggcau                108

<210>  1488
<211>  80
<212>  RNA
<213>  Ateles geoffroyi

<400>  1488
gugagguagu aaguuguauu guuguggggu agggauauua ggccccaauu agaagauaac      60

uauacaacuu acuacuuucc                                                80

<210>  1489
<211>  80
<212>  RNA
<213>  Macaca mulatta

<400>  1489
gugagguagu aaguuguauu guuguggggu agggauauua ggccccaauu agaagauaac      60

uauacaacuu acuacuuucc                                                80

<210>  1490
<211>  80
<212>  RNA
<213>  Gorilla gorilla

<400>  1490
gugagguagu aaguuguauu guuguggggu agggauauua ggccccaauu agaagauaac      60

uauacaacuu acuacuuucc                                                80

<210> 1491
<211> 80
<212> RNA
<213> Pan paniscus

<400> 1491
gugagguagu aaguuguauu guuguggggu agggauauua ggccccaauu agaagauaac 60

uauacaacuu acuacuuucc 80


<210> 1492
<211> 119
<212> RNA
<213> Pan troglodytes

<400> 1492
aggauucugc ucaugccagg gugagguagu aaguuguauu guuguggggu agggauauua 60

ggccccaauu agaagauaac uauacaacuu acuacuuucc cuggugugug gcauauuca 119


<210> 1493
<211> 80
<212> RNA
<213> Pongo pygmaeus

<400> 1493
gugagguagu aaguuguauu guuguggggu agggauauua ggccccaauu agaagauaac 60

uauacaacuu acuacuuucc 80


<210> 1494
<211> 108
<212> RNA
<213> Xenopus tropicalis

<400> 1494
ucugcccucu ucaggaugag guaguaaguu guauuguugu ggggguuuuu auuuccccaa 60

auuaggagau aacuauacag cuuacugccu ucccugcagc augguaau 108


<210> 1495
<211> 119
<212> RNA
<213> Bos taurus

<400> 1495
aggacucugc ucaugcuggg gugagguagu aaguuguauu guuguggggu agggauuuua 60

ggccccaauu ugaagauaac uauacaacuu acuacuuucc cuggugugua gcacauuca 119


<210> 1496
<211> 64
<212> RNA
<213> Mus musculus

<400> 1496
gagcucuuuu cacauugugc uacugucuaa cguguaccga gcagugcaau guuaaaaggg 60

cauc 64

<210>  1497
<211>  88
<212>  RNA
<213>  Rattus norvegicus

<400>  1497
ugcugcuggc cggagcucuu uucacauugu gcuacugucu acacguguac cgagcagugc      60

aauguuaaaa gggcaucggc cuuguagu                                        88


<210>  1498
<211>  89
<212>  RNA
<213>  Macaca mulatta

<400>  1498
ugcugcuggc cagagcucuu uucacauugu gcuacugucu gcaccuguca cuagcagugc      60

aauguuaaaa gggcauuggc cccccagua                                       89


<210>  1499
<211>  89
<212>  RNA
<213>  Macaca nemestrina

<400>  1499
ugcugcuggc cagagcucuu uucacauugu gcuacugucu gcaccuguca cuagcagugc      60

aauguuaaaa gggcauuggc cguguagug                                       89


<210>  1500
<211>  89
<212>  RNA
<213>  Gorilla gorilla

<400>  1500
ugcugcuggc cagagcucuu uucacauugu gcuacugucu gcaccuguca cuagcagugc      60

aauguuaaaa gggcauuggc cguguagug                                       89


<210>  1501
<211>  89
<212>  RNA
<213>  Pan paniscus

<400>  1501
ugcugcuggc cagagcucuu uucacauugu gcuacugucu gcaccuguca cuagcagugc      60

aauguuaaaa gggcauuggc cguguagug                                       89


<210>  1502
<211>  76
<212>  RNA
<213>  Monodelphis domestica

<400>  1502
uggccagagc ucuuuucaca uugugcuacu gucugcaccu aucacuagca gugcaaugua      60

aaaagggcau uggcug                                                      76


<210>  1503
<211>  83

<212>  RNA
<213>  Gallus gallus

<400>  1503
ugcuguuguc cagagcccuu uuucuguugu acuacuggca auuaugauga gcagugcaau      60

auuaaaaggg cauuggcugg cag                                              83


<210>  1504
<211>  83
<212>  RNA
<213>  Xenopus tropicalis

<400>  1504
cuguccagug cccuuuuuau guuguacuac uaguggucac acacaaaaaa agcagugcaa      60

uguuaaaagg gcauuggcca ggg                                              83


<210>  1505
<211>  85
<212>  RNA
<213>  Danio rerio

<400>  1505
ugucugucca gugccccuuu uauauuguac uacugauaac ccaguuauua aagcagugca      60

auguuaaaag ggcauuggcc aggga                                            85


<210>  1506
<211>  85
<212>  RNA
<213>  Danio rerio

<400>  1506
ugucugucca gugccccuuu uauauuguac uacugauaac ccaguuauua aagcagugca      60

auguuaaaag ggcauuggcc aggga                                            85


<210>  1507
<211>  141
<212>  RNA
<213>  Danio rerio

<400>  1507
gggagagguc uguacgguga uauugaccau guuuugucca uugcccuuuu ucuguuguac      60

uacuggccaa ucagaagagc agugcaauau uaaaagggca uuggcugaua gaacagaguc     120

cuucaccucu cacccucucc u                                               141


<210>  1508
<211>  81
<212>  RNA
<213>  Danio rerio

<400>  1508
uuguugacca gggcccuuuu ucuguuguac uacugugcag ucagaugagc agugcaauau      60

uaaaagggca uuggcugaca a                                                81


<210>  1509
<211>  79

<212>  RNA
<213>  Fugu rubripes

<400>  1509
uguuguuguc cucugcccuu uuucuguugc accacuggac acugagauga gcagugcaau      60

auuaaaaggg cauuggcug                                                   79


<210>  1510
<211>  74
<212>  RNA
<213>  Tetraodon nigroviridis

<400>  1510
uguuguccuc ugcccuuuuu cuguugcacc acuggacacu gagaugagca gugcaauauu      60

aaaagggcau uggc                                                        74


<210>  1511
<211>  82
<212>  RNA
<213>  Mus musculus

<400>  1511
ggcuuguugg acacucuuuc ccuguugcac uacugugggc cucugggaag cagugcaaug      60

augaaagggc aucugucggg cc                                               82


<210>  1512
<211>  82
<212>  RNA
<213>  Rattus norvegicus

<400>  1512
ggcuugcugg acacucuuuc ccuguugcac uacugugggc cucugggaag cagugcaaug      60

augaaagggc auccgucagg cc                                               82


<210>  1513
<211>  77
<212>  RNA
<213>  Gallus gallus

<400>  1513
gggcgggcug ccccucuuuc ccuguugcac uacugucacg gucgcagcga gcagugcaau      60

aaugaaaggg cgucagu                                                     77


<210>  1514
<211>  76
<212>  RNA
<213>  Xenopus tropicalis

<400>  1514
ccgccugaca cucuuucccu guugcacuac uguggcagug aauaaagcag ugcaaugaug      60

aaagggcauc agucug                                                      76


<210>  1515
<211>  84
<212>  RNA
<213>  Danio rerio

<400>  1515
cuguugccug acacucuuuc ccuguugcac uacuguggga gcugcagcaa agcagugcaa      60

uaaugaaagg gcaucagucc acug                                              84


<210>  1516
<211>  86
<212>  RNA
<213>  Mus musculus

<400>  1516
ccugcuaacg gcugcucuga cuuuauugca cuacuguacu uuacagcgag cagugcaaua      60

guauugucaa agcauccgcg agcagg                                            86


<210>  1517
<211>  97
<212>  RNA
<213>  Mus musculus

<400>  1517
uuuccugcug gcugcgggug cucugacuag guugcacuac ugugcuguga gaagcagugc      60

aaugguauug ucaaagcauc ugggaccagc cucgaag                               97


<210>  1518
<211>  100
<212>  RNA
<213>  Rattus norvegicus

<400>  1518
ccugcuggcu acugcugacg acugcucuga cuuuauugca cuacuguacu guacagcuag      60

cagugcaaua guauugucaa agcauccggg agcaggcuac                           100


<210>  1519
<211>  78
<212>  RNA
<213>  Rattus norvegicus

<400>  1519
gcugcgggug cucugacuag guugcacuac ugugcugugg aaagcagugc aaugguauug      60

ucaaagcauc ugggacca                                                     78


<210>  1520
<211>  93
<212>  RNA
<213>  Gallus gallus

<400>  1520
uggcugcugg uaucgcuggc ucugacaaug uugcacuacu gucugcacaa auaaagcagu      60

gcaauaauau ugucaaagca uuugguucca guc                                   93


<210>  1521
<211>  84
<212>  RNA
<213>  Xenopus tropicalis

<400>  1521

cugcuaaugu gugcucugac uuuauugcac uacuguacua uacagcuagc agugcaauag 60

uauugucaaa gcaucugcaa gcag 84

<210> 1522
<211> 73
<212> RNA
<213> Xenopus tropicalis

<400> 1522
gaauugcucu gacaguguug cacuacugug aucucaaaug aagcagugca auaguauugu 60

caaagcauuu cau 73

<210> 1523
<211> 86
<212> RNA
<213> Danio rerio

<400> 1523
gcuguuaaca ggugcucuga cuucauugca cuacuguauu ggacagcuag cagugcaaua 60

guauugucaa agcgucugag agcagc 86

<210> 1524
<211> 86
<212> RNA
<213> Danio rerio

<400> 1524
gcuguuaaca ggugcucuga cuucauugca cuacuguauu ggacagcuag cagugcaaua 60

guauugucaa agcgucugag agcagc 86

<210> 1525
<211> 81
<212> RNA
<213> Danio rerio

<400> 1525
aaggucuguu gcuuugacga uguugcacua cugaaccauc uaaucaagca gugcaauagu 60

auugucauug cauucggcuu u 81

<210> 1526
<211> 83
<212> RNA
<213> Danio rerio

<400> 1526
ugaggucagc ugcucugacg auguugcacu acuguaccau ccauucuagc agugcaauag 60

uauugucaua gcauuuagcc ucu 83

<210> 1527
<211> 68
<212> RNA
<213> Fugu rubripes

<400> 1527
cagcugcuuu gacaauguug cacuacugua ccauccauuc uagcagugca auaguauugu 60

cauagcau                                                            68

<210>  1528
<211>  79
<212>  RNA
<213>  Tetraodon nigroviridis

<400>  1528
aggucagcug cucugacaau guugcacuac uguaccaucc auucuagcag ugcaauagua      60

uugucauagc auuuggccu                                                79

<210>  1529
<211>  66
<212>  RNA
<213>  Mus musculus

<400>  1529
gggcaaccgu ggcuuucgau uguuacugug ggaaccggag guaacagucu acagccaugg      60

ucgccc                                                              66

<210>  1530
<211>  101
<212>  RNA
<213>  Rattus norvegicus

<400>  1530
ccgcccccgc gucuccaggg caaccguggc uuucgauugu uacuguggga accggaggua      60

acagucuaca gccauggucg ccccgcagca cgcccacgcu c                      101

<210>  1531
<211>  66
<212>  RNA
<213>  Monodelphis domestica

<400>  1531
gggcaaccgu ggcuuucgau uguuacugug ggaaccaggg guaacagucu acagccaugg      60

ucgccc                                                              66

<210>  1532
<211>  87
<212>  RNA
<213>  Xenopus tropicalis

<400>  1532
cugucuccag ggcaaccgug gcuuuagauu guuacuguag uucugcauug guaacagucu      60

acagccaugg ucgcucgggc aagaugc                                       87

<210>  1533
<211>  101
<212>  RNA
<213>  Bos taurus

<400>  1533
ccgcccccgc gucuccaggg caaccguggc uuucgauugu uacuguggga accggaggua      60

acagucuaca gccauggucg ccccgcagca cgcccacgcg c                      101

<210>  1534
<211>  85
<212>  RNA
<213>  Danio rerio

<400>  1534
gucuccaugg cgaccguggc auuagauugu uacuguagga acagaauuuu ugguaacagu      60

cuacagccau ggucgcuagu gggca                                            85


<210>  1535
<211>  119
<212>  RNA
<213>  Danio rerio

<400>  1535
ucccuuccuc ugcugucucc augcugaccg uggcuuuaga uuguuacugu agcguuggca      60

cgugguaaca gucuacagcc auggucgcca aggggcaggu guuacagcga cuacuggga       119


<210>  1536
<211>  85
<212>  RNA
<213>  Fugu rubripes

<400>  1536
gucuccaugg uuaccguggc auuagauugu uacuguagca acugcaccag ugguaacagu      60

cuacagccau ggucgcuagg gggca                                            85


<210>  1537
<211>  85
<212>  RNA
<213>  Tetraodon nigroviridis

<400>  1537
gucuccaugg uuaccguggc auuagauugu uacuguagca acugcaccag cgguaacagu      60

cuacagccau ggucgcuagg gggca                                            85


<210>  1538
<211>  91
<212>  RNA
<213>  Mus musculus

<400>  1538
gggcagcgcg ccggcaccuu ggcucuagac ugcuuacugc ccgggccgcc uucaguaaca      60

gucuccaguc acggccaccg acgccuggcc c                                     91


<210>  1539
<211>  111
<212>  RNA
<213>  Rattus norvegicus

<400>  1539
cgggauaucc ccgcccgggc agcgcgccgg caccuuggcu cuagacugcu uacugcccgg      60

gccgcccuca guaacagucu ccagucacgg ccaccgacgc cuggccccgc c              111


<210>  1540

<211>  91
<212>  RNA
<213>  Monodelphis domestica

<400>  1540
gggcagcgcg ccggcaccuu ggcucuagac ugcuuacugc ccgggccacc cucaguaaca      60

gucuccaguc acggccaccg acgccuggcc c                                     91


<210>  1541
<211>  96
<212>  RNA
<213>  Xenopus tropicalis

<400>  1541
uggucauugc aucggcaccu uggcucuaga cugcuuacug ugaaacugug cuauaggaac      60

aguaacaguc uacagucaug gcuacugacg uaugac                                96


<210>  1542
<211>  94
<212>  RNA
<213>  Danio rerio

<400>  1542
gucagugcau caauaccuug gcucuagacu gcuuacugcu aaaugugucu gaaaguacag      60

uaacagucua cagucauggc uacugacguc uggc                                  94


<210>  1543
<211>  95
<212>  RNA
<213>  Fugu rubripes

<400>  1543
ggucagcgca ucaacaccuu ggcucuagac ugcuuacugc uaaaaccgcu cauaaguaca      60

guaacagucu acagucaugg cuacugacgc cugac                                 95


<210>  1544
<211>  95
<212>  RNA
<213>  Tetraodon nigroviridis

<400>  1544
ggucagcgca ucaacaccuu ggcucuagac ugcuuacugc uaacaccgcu cugaaguaca      60

guaacagucu acagucaugg cuacugacgc cuggc                                 95


<210>  1545
<211>  88
<212>  RNA
<213>  Ateles geoffroyi

<400>  1545
acaaugcuuu gcuagagcug guaaaaugga accaaaucgc cucuucaaug gauuuggucc      60

ccuucaacca gcuguagcua ugcauuga                                         88


<210>  1546
<211>  88
<212>  RNA

<213>  Lagothrix lagotricha

<400>  1546
acaaugcuuu gcuagagcug guaaaaugga accaaaucgc cucuucaaug gauuuggucc      60

ccuucaacca gcuguagcua ugcauuga                                          88


<210>  1547
<211>  88
<212>  RNA
<213>  Saguinus labiatus

<400>  1547
acaaugcuuu gcuagagcug guaaaaugga accaaaucgc cucuucaaug gauuuggucc      60

ccuucaacca gcuguagcua ugcauuga                                          88


<210>  1548
<211>  88
<212>  RNA
<213>  Macaca nemestrina

<400>  1548
acaaugcuuu gcuagagcug guaaaaugga accaaaucgc cucuucaaug gauuuggucc      60

ccuucaacca gcuguagcua ugcauuga                                          88


<210>  1549
<211>  88
<212>  RNA
<213>  Gorilla gorilla

<400>  1549
acaaugcuuu gcuagagcug guaaaaugga accaaaucgc cucuucaaug gauuuggucc      60

ccuucaacca gcuguagcua ugcauuga                                          88


<210>  1550
<211>  88
<212>  RNA
<213>  Pan paniscus

<400>  1550
acaaugcuuu gcuagagcug guaaaaugga accaaaucgc cucuucaaug gauuuggucc      60

ccuucaacca gcuguagcua ugcauuga                                          88


<210>  1551
<211>  88
<212>  RNA
<213>  Pan troglodytes

<400>  1551
acaaugcuuu gcuagagcug guaaaaugga accaaaucgc cucuucaaug gauuuggucc      60

ccuucaacca gcuguagcua ugcauuga                                          88


<210>  1552
<211>  87
<212>  RNA
<213>  Monodelphis domestica

<400>  1552
caaugcuuug cuaaagcugg uaaaauggaa ccaaaucacc uauucaaugg auuugguccc     60

cuucaaccag cuguagcuau gcauuga                                          87


<210>  1553
<211>  87
<212>  RNA
<213>  Gallus gallus

<400>  1553
caaugcuuug cuaaagcugg uaaaauggaa ccaaaucacc ucuucaaugg auuggucccc     60

cuucaaccag cuguagcuau gcauuga                                          87


<210>  1554
<211>  96
<212>  RNA
<213>  Gallus gallus

<400>  1554
gugaggccaa augcuuugcu aaagcuggua aaauggaacc aaaucaacug uucaauggau     60

uugguccccu ucaaccagcu guagcugugc auugau                                96


<210>  1555
<211>  79
<212>  RNA
<213>  Gallus gallus

<400>  1555
ugccuuccug gggcugguaa aaaggaacca gaucaacuac aacuggauuu gguccccuuc     60

aaccagcugc aguggggca                                                   79


<210>  1556
<211>  86
<212>  RNA
<213>  Xenopus laevis

<400>  1556
cagugcuuug cuaaagcugg uaaaauggaa ccaaaucacc ucuucaaugg auuggucccc     60

cuucaaccag cuguaggauu gcauug                                           86


<210>  1557
<211>  86
<212>  RNA
<213>  Xenopus tropicalis

<400>  1557
caaugcuuug cuaaagcugg uaaaauggaa ccaaaucacc ucuucaaugg auuugguccc     60

cuucaaccag cuguaggauu gcauug                                           86


<210>  1558
<211>  76
<212>  RNA
<213>  Xenopus tropicalis

<400>  1558
cugguugcgg cuggugaaaa ggaaccacau caacccagaa aaaggauuug guccccuuca     60

accagccgca acuggu                                                    76

<210>  1559
<211>  84
<212>  RNA
<213>  Gallus gallus

<400>  1559
cucugcucug gcuggucaaa cggaaccaag cccgucuucu ucggagguuu gguccccuuc      60

aaccagcuau agcaguguug aaaa                                            84

<210>  1560
<211>  75
<212>  RNA
<213>  Xenopus tropicalis

<400>  1560
cugcuauggc uggucaaacg gaaccaaguc cgucuuccuu agagguuugg uccccuucaa      60

ccagcuauug cagua                                                      75

<210>  1561
<211>  71
<212>  RNA
<213>  Mus musculus

<400>  1561
agggugugug acugguugac cagaggggcg ugcacucugu ucacccugug ggccaccuag      60

ucaccaaccc u                                                          71

<210>  1562
<211>  73
<212>  RNA
<213>  Rattus norvegicus

<400>  1562
cagggugugu gacugguuga ccagaggggc gugcacuuug uucacccugu gggccaccua      60

gucaccaacc cuc                                                        73

<210>  1563
<211>  73
<212>  RNA
<213>  Macaca nemestrina

<400>  1563
cagggugugu gacugguuga ccagaggggc augcacugug uucacccugu gggccaccua      60

gucaccaacc cuc                                                        73

<210>  1564
<211>  73
<212>  RNA
<213>  Gorilla gorilla

<400>  1564
uagggugugu gacugguuga ccagaggggc augcacugug uucacccugu gggccaccua      60

gucaccaacc cuc                                                        73

<210>  1565
<211>  73
<212>  RNA
<213>  Pan paniscus

<400>  1565
cagggugugu gacugguuga ccagaggggc augcacugug uucacccugu gggccaccua      60

gucaccaacc cuc                                                          73


<210>  1566
<211>  73
<212>  RNA
<213>  Pongo pygmaeus

<400>  1566
cagggugugu gacugguuga ccagaggggc augcacugug uucacccugu gggccaccua      60

gucaccaacc cuc                                                          73


<210>  1567
<211>  56
<212>  RNA
<213>  Monodelphis domestica

<400>  1567
cuugggugga uaauacggau uacguuguua uugcuuaaga auacgcguag ucgagg          56


<210>  1568
<211>  96
<212>  RNA
<213>  Gallus gallus

<400>  1568
ucugacucuc uucggugacg gguauucuug gguggauaau acggauuacg uuguuauugc      60

uuaagaauac gcguagucga ggagaguacc ggcggc                                96


<210>  1569
<211>  101
<212>  RNA
<213>  Xenopus tropicalis

<400>  1569
gcucuguggc ucucuucggu gacggguauu cuugggugga uaauacggau uacguuguua      60

uugcuuaaga auacgcguag uugaggagag uaucuucagc a                         101


<210>  1570
<211>  101
<212>  RNA
<213>  Xenopus tropicalis

<400>  1570
gcucuguggc ucucuucggu gacggguauu cuugggugga uaauacggau uacguuguua      60

uugcuuaaga auacgcguag uugaggagag uaucuucagc a                         101


<210>  1571
<211>  101

<212>  RNA
<213>  Xenopus tropicalis

<400>  1571
gcucuguggc ucucuucggu gacggguauu cuugggugga uaauacggau uacguuguua      60

uugcuuaaga auacgcguag uugaggagag uaucuucagc a                         101


<210>  1572
<211>  64
<212>  RNA
<213>  Mus musculus

<400>  1572
acccauaaag uagaaagcac uacuaacagc acuggagggu guaguguuuc cuacuuuaug      60

gaug                                                                   64


<210>  1573
<211>  87
<212>  RNA
<213>  Rattus norvegicus

<400>  1573
gacagugcag ucacccauaa aguagaaagc acuacuaaca gcacuggagg guguaguguu      60

uccuacuuua uggaugagug uacugug                                          87


<210>  1574
<211>  88
<212>  RNA
<213>  Gallus gallus

<400>  1574
gacagugcag ucacccauaa aguagaaagc acuacuaaac agcacugcag gguguagugu      60

uuccuacuuu auggaugagu guacugug                                         88


<210>  1575
<211>  86
<212>  RNA
<213>  Xenopus tropicalis

<400>  1575
cagugcagcc acccauaaag uagaaagcac uacuagacag gacugaacgc uguaguguuu      60

ccuacuuuau ggaugagugu acuggg                                           86


<210>  1576
<211>  86
<212>  RNA
<213>  Xenopus tropicalis

<400>  1576
cagugcagcc acccauaaag uagaaagcac uacuagacag gacugaacgc uguaguguuu      60

ccuacuuuau ggaugagugu acuggg                                           86


<210>  1577
<211>  65
<212>  RNA
<213>  Mus musculus

<400>  1577
agcucugaga acugaauucc auggguuaua ucaaugucag accugugaaa uucaguucuu      60

cagcu                                                                 65


<210>  1578
<211>  95
<212>  RNA
<213>  Rattus norvegicus

<400>  1578
uguguauccu cagcucugag aacugaauuc cauggguuau agcaauguca gaccugugaa      60

guucaguucu uuagcuggga uagcucuauc gucau                                95


<210>  1579
<211>  99
<212>  RNA
<213>  Gallus gallus

<400>  1579
guguauccuc agcuuugaga acugaauucc auggguugua auugaauccu uugucagacc      60

cauggggcuc aguucuucag cuuggauauu ucugucuuc                            99


<210>  1580
<211>  105
<212>  RNA
<213>  Gallus gallus

<400>  1580
ggacuggcug cuuggcuuug agaacugaau uccauaggcg uuaaagcauc caaaaaugcc      60

cuauggauuc aguucugcag uugggcagca aauaaaccuc cagcc                    105


<210>  1581
<211>  90
<212>  RNA
<213>  Xenopus tropicalis

<400>  1581
caaugaguuc uuagcuuuga gaacugaauu ccauagguug uuagagguua gaccuauggu      60

gcuuaguucc auagcuugga ucccacuggc                                      90


<210>  1582
<211>  114
<212>  RNA
<213>  Danio rerio

<400>  1582
gaguuuguuu ugagcacuuu ucccugagaa cugaauucca uagauggugu ucaugaaaag      60

uucaucuaug ggcucaguuc uucuggcaau cuguuuaaug ucugcuacaa auuc          114


<210>  1583
<211>  80
<212>  RNA
<213>  Danio rerio

<400>  1583

gcucuuggcu uugagaacug aauuccaagg gugucugcuu uauauucagc ccacggaguu     60

caguucuuaa guuuggaugc                                                80


<210>  1584
<211>  80
<212>  RNA
<213>  Danio rerio

<400>  1584
gcucuuggcu uugagaacug aauuccaagg gugucugcuu uauauucagc ccacggaguu     60

caguucuuaa guuuggaugc                                                80


<210>  1585
<211>  72
<212>  RNA
<213>  Saguinus labiatus

<400>  1585
aaucuaaaga aaacauuucu gcacacacac cagacuauug aagccagugu guggaaaugc     60

uucugccaca uu                                                        72


<210>  1586
<211>  72
<212>  RNA
<213>  Macaca nemestrina

<400>  1586
aaucuaaaga aaacauuucu gcacacacac cagacuauug aagccagugu guggaaaugc     60

uucugcuaca uu                                                        72


<210>  1587
<211>  72
<212>  RNA
<213>  Pan paniscus

<400>  1587
aaucuaaaga aaacauuucu gcacacacac cagacuaugg aagccagugu guggaaaugc     60

uucugcuaga uu                                                        72


<210>  1588
<211>  72
<212>  RNA
<213>  Pan troglodytes

<400>  1588
aaucuaaaga aaacauuucu gcacacacac cagacuaugg aagccagugu guggaaaugc     60

uucugcuaga uu                                                        72


<210>  1589
<211>  72
<212>  RNA
<213>  Pongo pygmaeus

<400>  1589
aaucuaaaga aaacauuucu gcacacacac cagacuaugg aagccagugu guggaaaugc     60

uucugcuaga uu                                                          72

<210>  1590
<211>  66
<212>  RNA
<213>  Mus musculus

<400>  1590
ggcucuggcu ccgugucuuc acucccgugu uuguccgagg agggagggag ggacgggggc      60

ggugcu                                                                66

<210>  1591
<211>  89
<212>  RNA
<213>  Mus musculus

<400>  1591
gccaaggguu ugggggaaca uucaaccugu cggugaguuu gggcagcuca gacaaaccau      60

cgaccguuga guggaccccg aggccugga                                       89

<210>  1592
<211>  106
<212>  RNA
<213>  Rattus norvegicus

<400>  1592
agaacuugcc aaggguuugg gggaacauuc aaccugucgg ugaguuuggg cagcucagac      60

aaaccaucga ccguugagug gaccccgagg ccuggaacug ccaccc                   106

<210>  1593
<211>  110
<212>  RNA
<213>  Macaca mulatta

<400>  1593
cggagaauuu gccaaggguu ugggggaaca uucaaccugu cggugaguuu gggcagcuca      60

ggcaaaccau cgaccguuga guggacccug agaccuggac uugccauccu               110

<210>  1594
<211>  110
<212>  RNA
<213>  Gorilla gorilla

<400>  1594
cggaaaauuu gccaaggguu ugggggaaca uucaaccugu cggugaguuu gggcagcuca      60

ggcaaaccau cgaccguuga guggacccug aggccuggaa uugccauccu               110

<210>  1595
<211>  110
<212>  RNA
<213>  Pan paniscus

<400>  1595
cggaaaauuu gccaaggguu ugggggaaca uucaaccugu cggugaguuu gggcagcuca      60

ggcaaaccau cgaccguuga guggacccug aggccuggaa uugccauccu               110

<210>  1596
<211>  110
<212>  RNA
<213>  Pan troglodytes

<400>  1596
cggaaaauuu gccaaggguu ugggggaaca uucaaccugu cggugaguuu gggcagcuca      60

ggcaaaccau cgaccguuga guggacccug aggccuggaa uugccauccu                110


<210>  1597
<211>  72
<212>  RNA
<213>  Monodelphis domestica

<400>  1597
ugggggaaca uucaacgcug ucggugaguu ugagcagcug aaggcaaacc aucgaccguu      60

gaguggaccc cg                                                          72


<210>  1598
<211>  97
<212>  RNA
<213>  Bos taurus

<400>  1598
uugccaaggg uuugggggaa cauucaaccu gucggugagu uugggcagcu caggcaaacc      60

aucgaccguu gaguggaccc cgaggccugg aacugcc                               97


<210>  1599
<211>  87
<212>  RNA
<213>  Sus scrofa

<400>  1599
caaggguuug ggggaacauu caaccugucg gugaguuugg gcagcucagg caaaccaucg      60

accguugagu ggaccccgag gccugga                                          87


<210>  1600
<211>  78
<212>  RNA
<213>  Danio rerio

<400>  1600
ggguccugau ucacauucau ugcugucggu ggguuuuauc ucuucgacuc gccggacaau      60

gaaugagaac uacggcuc                                                    78


<210>  1601
<211>  72
<212>  RNA
<213>  Mus musculus

<400>  1601
acaauuaaca uucauuguug ucgguggguu gugaggaggc agccagaccc accgggggau      60

gaaugucacu gu                                                          72


<210>  1602

<211>  83
<212>  RNA
<213>  Rattus norvegicus

<400>  1602
ggucacaauu aacauucauu guugucggug gguugugagg agaagaccag acccaccggg      60

ggaugaaugu cacuguggcu ggg                                              83


<210>  1603
<211>  105
<212>  RNA
<213>  Xenopus tropicalis

<400>  1603
aguggcuucu cuggcagugu uuggcaaugg uagaacucac acuggugagc uaugaagauc      60

cggugguucu agacuugcca acuauggccu gggaauaaag cagca                    105


<210>  1604
<211>  68
<212>  RNA
<213>  Danio rerio

<400>  1604
guauuuggca augguagaac ucacacuggu gagguaguca gauccggugg uucuagacuu      60

gccaacua                                                               68


<210>  1605
<211>  70
<212>  RNA
<213>  Mus musculus

<400>  1605
cuguguaugg cacugguaga auucacugug aacagucuca gucagugaau uaccgaaggg      60

ccauaaacag                                                             70


<210>  1606
<211>  110
<212>  RNA
<213>  Rattus norvegicus

<400>  1606
ccagagagug ugacuccugu ccuguguaug gcacugguag aauucacugu gaacagucuc      60

ggucagugaa uuaccgaagg gccauaaaca gagcagagac agauccgcga                110


<210>  1607
<211>  110
<212>  RNA
<213>  Saguinus labiatus

<400>  1607
ccgcagaacg ugacuccugu ucuguguaug gcacugguag aauucacugu gaacagucuc      60

ggucagugaa uuaccgaagg gccauaaaca gagcagagac agauccucga                110


<210>  1608
<211>  110
<212>  RNA

<213>  Macaca mulatta

<400>  1608
ccgcagaacg ugacuccugu ucuguguaug gcacugguag aauucacugu gaacagucuc      60

ggucagugaa uuaccgaagg gccauaaaca gagcagagac agauccacga                110


<210>  1609
<211>  110
<212>  RNA
<213>  Macaca nemestrina

<400>  1609
ccgcagaacg ugacuccugu ucuguguaug gcacugguag aauucacugu gaacagucuc      60

ggucagugaa uuaccgaagg gccauaaaca gagcagagac agauccacga                110


<210>  1610
<211>  110
<212>  RNA
<213>  Gorilla gorilla

<400>  1610
ccgcagagug ugacuccugu ucuguguaug gcacugguag aauucacugu gaacagucuc      60

agucagugaa uuaccgaagg gccauaaaca gagcagagac agauccacga                110


<210>  1611
<211>  110
<212>  RNA
<213>  Pan paniscus

<400>  1611
ccgcagagug ugacuccugu ucuguguaug gcacugguag aauucacugu gaacagucuc      60

agucagugaa uuaccgaagg gccauaaaca gagcagagac agauccacga                110


<210>  1612
<211>  110
<212>  RNA
<213>  Pan troglodytes

<400>  1612
ccgcagagug ugacuccugu ucuguguaug gcacugguag aauucacugu gaacagucuc      60

agucagugaa uuaccgaagg gccauaaaca gagcagagac agauccacga                110


<210>  1613
<211>  88
<212>  RNA
<213>  Monodelphis domestica

<400>  1613
acuccuguuc uguguauggc acugguagaa uucacuguga aaacacacua ucagugaauu      60

accaaagggc cauaaacaga gcagagaa                                         88


<210>  1614
<211>  98
<212>  RNA
<213>  Gallus gallus

<400>  1614
acuccuguuc uguguauggc acugguagaa uucacugugc aaccccgcgg ucagugaauu      60

accauagggc cauaaacaaa gcagagaaag acccgcga                              98


<210>  1615
<211>  97
<212>  RNA
<213>  Xenopus tropicalis

<400>  1615
caucccuccu guucugugua uggcacuggu agaauucacu gugaaaacac aaaaucagug      60

aauuaccaua gggccauaaa cagagcagag aaagaac                               97


<210>  1616
<211>  70
<212>  RNA
<213>  Sus scrofa

<400>  1616
cuguguaugg cacugguaga auucacugug aacagucucg gucagugaau uaccgaaggg      60

ccauaaacag                                                             70


<210>  1617
<211>  98
<212>  RNA
<213>  Danio rerio

<400>  1617
gacuccuguu cuguguaugg cacugguaga auucacugug aaagcacacu aucagugaau      60

uaccaaaggg ccauaaacag agcagagaaa gaaccacg                              98


<210>  1618
<211>  70
<212>  RNA
<213>  Fugu rubripes

<400>  1618
cuguguaugg cacugguaga auucacugug agagcucacu aucagugaau uaccauaggg      60

ccauaaacag                                                             70


<210>  1619
<211>  70
<212>  RNA
<213>  Tetraodon nigroviridis

<400>  1619
cuguguaugg cacugguaga auucacugug agagcucacu aucagugaau uaccauaggg      60

ccauaaacag                                                             70


<210>  1620
<211>  69
<212>  RNA
<213>  Mus musculus

<400>  1620
   ccuuuccuua ucacuuuucc agccagcuuu gugacucuaa guguuggacg gagaacugau      60
   aagggu agg                                                    69

<210>  1621
<211>  77
<212>  RNA
<213>  Rattus norvegicus

<400>  1621
cacuuucccu uaucaguuuu ccagccagcu uugugacugu aaauguugga cggagaacug      60

auaaggguaa gugacug                                                    77

<210>  1622
<211>  84
<212>  RNA
<213>  Macaca nemestrina

<400>  1622
ucagucacgu ccccuuauca cuuuuccagc ccagcuuuau gacuguaagu guuggacgga      60

gaacugauaa ggguagguga uuga                                            84

<210>  1623
<211>  84
<212>  RNA
<213>  Pan troglodytes

<400>  1623
ccagucacgu ccccuuauca cuuuuccagc ccagcuuugu gacuguaagu guuggacgga      60

gaacugauaa ggguagguga uuga                                            84

<210>  1624
<211>  84
<212>  RNA
<213>  Pongo pygmaeus

<400>  1624
ccagucacgu ccccuuauca cuuuuccagc ccagcuuugu gacuguaagu guuggacgga      60

gaacugauaa ggguagguga uuga                                            84

<210>  1625
<211>  84
<212>  RNA
<213>  Monodelphis domestica

<400>  1625
ccagucacau ccccuuauca cuuuuccagc ccagcuuucu aaugcuaauu guuggacgga      60

gaacugauaa ggguagguga uuga                                            84

<210>  1626
<211>  74
<212>  RNA
<213>  Gallus gallus

<400>  1626
ccgcucucac ccccuuauca cuuuuccagc ccagcuucuu cgcucugacu guuggacgga      60

gaacugauaa gggu                                                       74

<210>  1627
<211>  83
<212>  RNA
<213>  Xenopus tropicalis

<400>  1627
ccaguaucac uuccuuauca cuuuuccagc ccagcuuuuc augacaaacu guuggacgga      60

gaacugauaa ggcuguguga cug                                              83


<210>  1628
<211>  83
<212>  RNA
<213>  Sus scrofa

<400>  1628
ccagucacau ccccuuauca cuuuuccagc cagcuuugug acucuaaaug uuggacggag      60

aacugauaag gguaggugau uga                                              83


<210>  1629
<211>  79
<212>  RNA
<213>  Danio rerio

<400>  1629
cacaucuccu uaucacuuuu ccagcccagc uaacgauggu gaauccguug gacggagaac      60

ugauaagggc auguggcug                                                   79


<210>  1630
<211>  87
<212>  RNA
<213>  Danio rerio

<400>  1630
gucgaacacg ucuccuuauc acuuuuccag cccagcuauc cauuuaguau ucguuggacg      60

gagaacugau aagggcaugu gcccgau                                          87


<210>  1631
<211>  73
<212>  RNA
<213>  Fugu rubripes

<400>  1631
cgcucacauc uccuuaucac uuuuccagcc cagcuauagg uuuugaaucc guuggacgga      60

gaacugauaa ggg                                                         73


<210>  1632
<211>  73
<212>  RNA
<213>  Tetraodon nigroviridis

<400>  1632
cgcucacauc uccuuaucac uuuuccagcc cagcuauagc uucugaaucc guuggacgga      60

gaacugauaa ggg                                                         73

<210>  1633
<211>  89
<212>  RNA
<213>  Mus musculus

<400>  1633
cgugcacagg gcucugaccu augaauugac agccaguacu cuuuucucuc cucuggcugc      60

caauuccaua ggucacaggu auguucacc                                        89


<210>  1634
<211>  110
<212>  RNA
<213>  Rattus norvegicus

<400>  1634
gucaagaugg agugcacagg gcucugaccu augaauugac agccaguacu cugaucucgc      60

cucuggcugc caguuccaua ggucacaggu auguucgccu caaugccagc                110


<210>  1635
<211>  87
<212>  RNA
<213>  Xenopus tropicalis

<400>  1635
gaguguacgg gccuaugacc uaugaauuga cagccagugg augugaaguc ugccugucaa      60

uucuguaggc cacagguucg uccaccu                                          87


<210>  1636
<211>  106
<212>  RNA
<213>  Bos taurus

<400>  1636
agaccgagug cacagggcuc ugaccuauga auugacagcc agugcucuug uguccccucu      60

ggcugccaau uccauagguc acagguaugu ucgccucaau gccagc                    106


<210>  1637
<211>  85
<212>  RNA
<213>  Danio rerio

<400>  1637
cuaggacaca gggugaugac cuaugaauug acagccagug uuugcagucc agcugccugu      60

caguucugua ggccacugcc cuguu                                            85


<210>  1638
<211>  82
<212>  RNA
<213>  Fugu rubripes

<400>  1638
ugggacguga ggugaugacc uaugaauuga cagccaguaa cuggagccuc ugccugucag      60

uucuguaggc cacugcuacg uu                                               82


<210>  1639
<211>  83

<212>  RNA
<213>  Tetraodon nigroviridis

<400>  1639
cacgagguga ugaccuauga auugacagcc aguaacugga gccucugccu gucaguucug      60

uaggccacug cugcguccgu ccc                                              83


<210>  1640
<211>  112
<212>  RNA
<213>  Mus musculus

<400>  1640
agcucucagc aucaacggug uacaggagaa ugaccuauga uuugacagac cgugcagcug      60

uguaugucug ucauucugua ggccaauauu cuguauguca cugcuacuua aa             112


<210>  1641
<211>  97
<212>  RNA
<213>  Rattus norvegicus

<400>  1641
gguguacagg acaaugaccu augauuugac agacagugug gcugcgugug ucugucauuc      60

uguaggccaa uauucuguau gucucuccuc cuuacaa                               97


<210>  1642
<211>  110
<212>  RNA
<213>  Macaca mulatta

<400>  1642
aucauuaaga aaugguauac aggaaaauga ccuaugaauu gacagacacu auagcugagu      60

uugucuguca uuucuuuagg ccaauauucu guaugacugu gcuacuucaa                110


<210>  1643
<211>  110
<212>  RNA
<213>  Macaca nemestrina

<400>  1643
aucauuaaga aaugguauac aggaaaauga ccuaugaauu gacagacacu auagcugagu      60

uugucuguca uuucuuuagg ccaauauucu guaugacugu gcuacuucaa                110


<210>  1644
<211>  110
<212>  RNA
<213>  Gorilla gorilla

<400>  1644
aucauucaga aaugguauac gggaaaauga ccuaugaauu gacagacaau auagcugagu      60

uugucuguca uuucuuuaga ccaauauucu guaugacugu gcuacuucaa                110


<210>  1645
<211>  110
<212>  RNA
<213>  Pan troglodytes

<400>  1645
aucauucaga aaugguauac gggaaaauga ccuaugaauu gacagacaau auagcugagu      60

uugucuguca uuucuuuagg ccaauauucu guaugacugu gcuacuucaa                110

<210>  1646
<211>  110
<212>  RNA
<213>  Pongo pygmaeus

<400>  1646
aucauucaga aaugguauac aggaaaauga ccuaugaauu gacagacaau acagcugagu      60

uugucuguca uuucuuuagg ccaauauucu guacaacugu gcuacuucaa                110

<210>  1647
<211>  105
<212>  RNA
<213>  Gallus gallus

<400>  1647
ucaguaagaa cuggugucca ggaaaaugac cuaugaauug acagacugcu uucaaaaugu      60

gccugucauu ucuauaggcc aauauucugu gcacuuuucc uacuu                     105

<210>  1648
<211>  97
<212>  RNA
<213>  Xenopus tropicalis

<400>  1648
aacugguaac caggaggaug accuaugaaa ugacagccac uuccauacca aacaugucug      60

ucauuucugu aggccaauau ucugauugcu uuguuga                               97

<210>  1649
<211>  79
<212>  RNA
<213>  Bos taurus

<400>  1649
uguacaggaa aaugaccuau gaauugacag acaacgugac uaagucuguc ugucauuucu      60

guaggccaau guucuguau                                                   79

<210>  1650
<211>  86
<212>  RNA
<213>  Rattus norvegicus

<400>  1650
gcggacggga gcugagagcu gggucuuugc gggcaagaug agggugucag uucaacuggc      60

cuacaaaguc ccaguccucg gcuccc                                          86

<210>  1651
<211>  66
<212>  RNA
<213>  Monodelphis domestica

<400>  1651

gaggauuggg ucuuuguggg cgagaugagg gugucaguuc aacuggccua caaaguccca 60

guucuc 66

<210> 1652
<211> 83
<212> RNA
<213> Gallus gallus

<400> 1652
gugguuccag agucgggguu uugggggcga gaugagcuua uguuuuaucc aacuggccca 60

caaagucccg cuuuuggugg uca 83

<210> 1653
<211> 83
<212> RNA
<213> Xenopus tropicalis

<400> 1653
gugguuucgg agucgggauu uugggggcga gaugagcuaa ugauuuaucc aacuggcccg 60

caaagucccg cuucuggaag uca 83

<210> 1654
<211> 81
<212> RNA
<213> Bos taurus

<400> 1654
ugggagcuga gagcuggguc uuugcgggcg agaugaaggu gucgguucaa cuggccuaca 60

aagucccagu ccucggcccc c 81

<210> 1655
<211> 99
<212> RNA
<213> Danio rerio

<400> 1655
gacauuaaug uguuagaggu ugggucuuug cgggcaaggu gaguaguuaa auuuacucuc 60

aacuggccua caaaguccca guuucuggcu cauguuauc 99

<210> 1656
<211> 98
<212> RNA
<213> Danio rerio

<400> 1656
gaaagugugu cagaggcugg gucuuugcgg gcaaggugag uuuuccuuuu auucaacugg 60

ccuacaaagu cccaguuuuc ggcccaugug accaucuc 98

<210> 1657
<211> 98
<212> RNA
<213> Danio rerio

<400> 1657
gaaagugugu cagaggcugg gucuuugcgg gcaaggugag uuuuccuuuu auucaacugg 60

ccuacaaagu cccaguuuuc ggcccaugug accaucuc 98

<210> 1658
<211> 88
<212> RNA
<213> Fugu rubripes

<400> 1658
ugugucagaa gcugggucuu ugcgggcaag gugaguccuc aguuuguuca acuggccuac 60

aaagucccag ucucuggcuc acguaacc 88

<210> 1659
<211> 83
<212> RNA
<213> Tetraodon nigroviridis

<400> 1659
cagaagcugg gucuuugcgg gcaaggugag uccucaguuu guucaacugg ccuacaaagu 60

cccagugucu ggcucacgug acc 83

<210> 1660
<211> 79
<212> RNA
<213> Mus musculus

<400> 1660
   ggcccagaau cgggguuuug agggcgagau gaguuugugu uuuauccaac uggcccacaa 60
   agucccgcuu uugggguca 79

<210> 1661
<211> 85
<212> RNA
<213> Danio rerio

<400> 1661
gugauuucag ugacgggacu uugggggcga gaugaguauu gaucucuauc caacuggccc 60

gcaaaguccc gcuucuggga cucac 85

<210> 1662
<211> 94
<212> RNA
<213> Mus musculus

<400> 1662
acacccaacu cuccuggcuc uagcagcaca gaaauauugg cauggggaag ugagucugcc 60

aauauuggcu gugcugcucc aggcagggug guga 94

<210> 1663
<211> 87
<212> RNA
<213> Rattus norvegicus

<400> 1663
aacucuccug gcucuagcag cacagaaaua uuggcacggg uaagugaguc ugccaauauu 60

ggcugugcug cuccaggcag gguggug 87

<210> 1664
<211> 87
<212> RNA
<213> Gorilla gorilla

<400> 1664
agcuuccugg gcucuagcag cacagaaaua uuggcacagg gaagcgaguc ugccaauauu 60

ggcugugcug cuccaggcag gguggug 87


<210> 1665
<211> 87
<212> RNA
<213> Pan paniscus

<400> 1665
agcuucccug gcucuagcag cacagaaaua uuggcacagg gaagcgaguc ugccaauauu 60

ggcugugcug cuccaggcag gguggug 87


<210> 1666
<211> 87
<212> RNA
<213> Bos taurus

<400> 1666
agcuccccug gcucuagcag cacagaaaua uuggcacugg gaagaaagcc ugccaauauu 60

ggcugugcug cuccaggcag gguggug 87


<210> 1667
<211> 84
<212> RNA
<213> Mus musculus

<400> 1667
   ccugcccccg ccccagcagc acacuguggu uuguacggca cuguggccac guccaaacca 60
   cacuguggug uuagagcgag ggua 84


<210> 1668
<211> 69
<212> RNA
<213> Rattus norvegicus

<400> 1668
ccagcagcac acugugguuu guacggcacu guggccacgu ccaaaccaca cugugguguu 60

agagcgagg 69


<210> 1669
<211> 112
<212> RNA
<213> Bos taurus

<400> 1669
ccaccccagu ccugcucccg ccccagcagc acacuguggu uuguacggca cuguggccac 60

guccaaacca cacuguggug uuagagcgag ggugggggag gcaccgcuga gg 112


<210> 1670

<211>  102
<212>  RNA
<213>  Mus musculus

<400>  1670
ugagccggga cuguugagug aaguagguag uuucauguug uugggccugg cuuucugaac      60

acaacgacau caaaccaccu gauucauggc aguuacugcu uc                        102


<210>  1671
<211>  85
<212>  RNA
<213>  Mus musculus

<400>  1671
agcugaucug uggcuuaggu aguuucaugu uguugggauu gaguuuugaa cucggcaaca      60

agaaacugcc ugaguuacau caguc                                            85


<210>  1672
<211>  110
<212>  RNA
<213>  Rattus norvegicus

<400>  1672
uguuugcuca gcugaucugu ggcuuaggua guuucauguu guugggauug aguuuugaac      60

ucggcaacaa gaaacugccu gaguuacauc agucgguuuu cgucgagggc                110


<210>  1673
<211>  110
<212>  RNA
<213>  Ateles geoffroyi

<400>  1673
ugcuugcuca gcugaucugu ggcuuaggua guuucauguu guugggauug aguuuugaac      60

ucggcaacaa gaaacugccu gaguuacauc agucgguuuu cgucgagggg                110


<210>  1674
<211>  110
<212>  RNA
<213>  Lagothrix lagotricha

<400>  1674
ugcuugcuca gcugaucugu ggcuuaggua guuucauguu guugggauug aguuuugaac      60

ucggcaacaa gaaacugccu gaguuacauc agucgguuuu cgucgagggc                110


<210>  1675
<211>  70
<212>  RNA
<213>  Macaca mulatta

<400>  1675
gugaauuagg uaguuucaug uuguugggcc uggguuucug aacacaacaa cauuaaacca      60

cccgauucac                                                             70


<210>  1676
<211>  70
<212>  RNA

<213>  Gorilla gorilla

<400>  1676
gugaauuagg uaguuucaug uuguugggcc uggguuucug aacacaacaa cauuaaacca      60

cccgauucac                                                            70


<210>  1677
<211>  110
<212>  RNA
<213>  Gorilla gorilla

<400>  1677
   ugcucgcuca gcugaucugu ggcuuaggua guuucauguu guugggauug aguuuugaac      60
   ucggcaacaa gaaacugccu gaguuacauc agucgguuuu cgucgagggc               110


<210>  1678
<211>  110
<212>  RNA
<213>  Pan paniscus

<400>  1678
   ugcucgcuca gcugaucugu ggcuuaggua guuucauguu guugggauug aguuuugaac      60
   ucggcaacaa gaaacugccu gaguuacauc agucgguuuu cgucgagggc               110


<210>  1679
<211>  110
<212>  RNA
<213>  Pan troglodytes

<400>  1679
   ugcucgcuca gcugaucugu ggcuuaggua guuucauguu guugggauug aguuuugaac      60
   ucggcaacaa gaaacugccu gaguuacauc agucgguuuu cgucgagggc               110


<210>  1680
<211>  70
<212>  RNA
<213>  Pongo pygmaeus

<400>  1680
gugaauuagg uaguuucaug uuguugggcc uggguuucug aacacaacaa cauuaaacca      60

cccgauucac                                                            70


<210>  1681
<211>  110
<212>  RNA
<213>  Pongo pygmaeus

<400>  1681
   ugcucgcuca gcugaucugu ggcuuaggua guuucauguu guugggauug aguuuugaac      60
   ucggcaacaa gaaacugccu gaguuacauc agucgguuuu cgucgagggc               110


<210>  1682
<211>  95
<212>  RNA
<213>  Gallus gallus

<400>  1682
ugaacuagaa cugcucugug aauuagguag uuucauguug uugggcuuua aauuuuaaac      60

acaagaacau caaacuaccu gauuuacucc aguua                                 95


<210>  1683
<211>  94
<212>  RNA
<213>  Gallus gallus

<400>  1683
aacugcucug ugguuuaggu aguuucaugu uguuggggcu ccaccuuucu cucuacagca      60

cgaaacugcc uuaauuacuu caguugauau cauc                                  94


<210>  1684
<211>  108
<212>  RNA
<213>  Gallus gallus

<400>  1684
uuucaugcag cugaucugug guuuagguag uuucauguug uugggauugg cuuuuagcuc      60

ggcaacaaga aacugccuua auuacgucag uuagucuuca ucaagggc                  108


<210>  1685
<211>  106
<212>  RNA
<213>  Xenopus tropicalis

<400>  1685
uuuguaucca gcugaucugu gguuuaggua guuucauguu guugggauug cuuuuucuua      60

acgcggcaac aagaaacugc cuuaauuacg ucaguucguc uucauc                    106


<210>  1686
<211>  107
<212>  RNA
<213>  Sus scrofa

<400>  1686
uugcucagcu gaucuguggc uuagguaguu ucauguuguu gggauugagu uuugaacucg      60

gcaacaagaa acugccugag uuacaucagu cgguuaucgu cgagggc                   107


<210>  1687
<211>  106
<212>  RNA
<213>  Danio rerio

<400>  1687
cgcgcggcug gugcgugguu uagguaguuu cauguuguug ggauuggcuu ccuggcucga      60

caacaagaaa cugccuugau uacgucaguu cgucuucauc aagggc                    106


<210>  1688
<211>  76
<212>  RNA
<213>  Danio rerio

<400>  1688
ucgagugguu uagguaguuu cauguuguug ggauuacauu caaacucugc aacgugaaac      60

ugucuuaauu gcccca                                                    76

<210>  1689
<211>  85
<212>  RNA
<213>  Fugu rubripes

<400>  1689
agcuggagcg ugguuuaggu aguuucaugu uguuggggau ggcuuccugg cucggcaaca      60

agaaacugcc uugauuacgu caguu                                            85

<210>  1690
<211>  76
<212>  RNA
<213>  Fugu rubripes

<400>  1690
ucgagugguu uagguaguuu cauguuguug ggguccauuu caaacucugc aacaugaaac      60

ugucuuaauu gcccca                                                    76

<210>  1691
<211>  101
<212>  RNA
<213>  Tetraodon nigroviridis

<400>  1691
agcuggagcg ugguuuaggu aguuucaugu uguuggggau ggcuuccugg cucggcaaca      60

agaaacugcc uugauuacgu caguucgucu ucaucaaggg c                         101

<210>  1692
<211>  76
<212>  RNA
<213>  Tetraodon nigroviridis

<400>  1692
ucgagugguu uagguaguuu cauguuguug ggguccauuu caaacucugc aacaugaaac      60

ugucuuaauu gcccca                                                    76

<210>  1693
<211>  85
<212>  RNA
<213>  Mus musculus

<400>  1693
aacuggucgg ugauuuaggu aguuuccugu uguugggauc caccuuucuc ucgacagcac      60

gacacugccu ucauuacuuc aguug                                            85

<210>  1694
<211>  85
<212>  RNA
<213>  Rattus norvegicus

<400>  1694
aacuggucgg ugauuuaggu aguuuccugu uguugggauc caccuuucuc ucgacagcac      60

gacacugccu ucauuacuuc aguug                                            85

<210> 1695
<211> 86
<212> RNA
<213> Monodelphis domestica

<400> 1695
aacuggucug ugauuuaggu aguuuccugu uguuggggcu ccaccuuucu cucgacagca 60

cgauacugcc uucauuacuu caguug 86

<210> 1696
<211> 87
<212> RNA
<213> Xenopus tropicalis

<400> 1696
agccgcugug ugguuuaggu aguuuuaugu uguugggcau ucaccuuucu cucuacaaca 60

agaaacugcc uuaauuacau caguggg 87

<210> 1697
<211> 79
<212> RNA
<213> Danio rerio

<400> 1697
gucugugauu uagguaguuu caaguuguug ggcuggacgu uuaauuucac aggaaccuga 60

aacugccuga auugcucca 79

<210> 1698
<211> 69
<212> RNA
<213> Fugu rubripes

<400> 1698
ugugauauag guaguuucaa guuguugggc ugaacucuug ugaucacagg aaccugaaac 60

ugccugagu 69

<210> 1699
<211> 69
<212> RNA
<213> Tetraodon nigroviridis

<400> 1699
ugugauguag guaguuucaa guuguugggc uaaacuuugg ugaucacagg aaccugaaac 60

ugcccgauu 69

<210> 1700
<211> 81
<212> RNA
<213> Mus musculus

<400> 1700
ggggcugugc cggguagaga gggcaguggg agguaagagc ucuucacccu ucaccaccuu 60

cuccacccag cauggccggc a 81

<210> 1701
<211> 75
<212> RNA
<213> Ateles geoffroyi

<400> 1701
ggcugugccg gguagagagg gcagugggag guaagagcuc uucacccuuc accaccuucu 60

ccacccagca uggcc 75

<210> 1702
<211> 75
<212> RNA
<213> Macaca nemestrina

<400> 1702
ggcugugccg gguagagagg gcagugggag guaagagcuc uucacccuuc accaccuucu 60

ccacccggca uggcc 75

<210> 1703
<211> 75
<212> RNA
<213> Pan paniscus

<400> 1703
ggcugugccg gguagagagg gcagugggag guaagagcuc uucacccuuc accaccuucu 60

ccacccagca uggcc 75

<210> 1704
<211> 75
<212> RNA
<213> Pan troglodytes

<400> 1704
ggcugugccg gguagagagg gcagugggag guaagagcuc uucacccuuc accaccuucu 60

ccacccagca uggcc 75

<210> 1705
<211> 75
<212> RNA
<213> Pongo pygmaeus

<400> 1705
ggcugugccg gguagagagg gcagugggag guaagagcuc uucacccuuc accaccuucu 60

ccacccagca uggcc 75

<210> 1706
<211> 96
<212> RNA
<213> Xenopus tropicalis

<400> 1706
aagaaccugc uccgucgccc caguguucag acuaccuguu caggacaaug cuguuguaca 60

guagucugca cauugguuag acugggcaug ggacag 96

<210> 1707
<211> 87

<212>  RNA
<213>  Danio rerio

<400>  1707
uccugcuccg ucaucccagu guucagacua ccuguucagg aucauacugg uguacaguag      60

ucugcacauu gguuagacug ugcaugg                                          87


<210>  1708
<211>  96
<212>  RNA
<213>  Danio rerio

<400>  1708
ggaguuuuug uggacgcccg ucccgccugc ccaguguuca gacuaccugu ucaggaauua      60

guguuuguac aguagucugc acauugguua ggcugg                                96


<210>  1709
<211>  98
<212>  RNA
<213>  Danio rerio

<400>  1709
ccucccccuc gccugcccag uguucagacu accuguucau caugcugcag cugaacagua      60

guccgcacau ugguuaggcu gggcugggac acacacac                              98


<210>  1710
<211>  90
<212>  RNA
<213>  Mus musculus

<400>  1710
cugggccucu gugggcaucu uaccggacag ugcuggauuu cuuggcuuga cucuaacacu      60

gucugguaac gauguucaaa ggugacccac                                       90


<210>  1711
<211>  89
<212>  RNA
<213>  Rattus norvegicus

<400>  1711
cugggccucu gugggcaucu uaccggacag ugcuggauuu cuuggcuuga cucuaacacu      60

gucugguaac gauguucaaa ggugaccca                                        89


<210>  1712
<211>  86
<212>  RNA
<213>  Monodelphis domestica

<400>  1712
gggccucugu gggcaucuua cuagacagug cuggauuuuu ggauguacuc uaacacuguc      60

ugguaacgau guuuaaagag ggaacc                                           86


<210>  1713
<211>  87
<212>  RNA
<213>  Gallus gallus

<400>  1713
gguccucugu gggcaucuua cuagacagug cuggauuucu uggaucuauu cuaacacugu      60

cugguaacga uguuuaaagg gugaacc                                          87


<210>  1714
<211>  89
<212>  RNA
<213>  Xenopus tropicalis

<400>  1714
ugguccucua uggacaucuu acuagacagu gcuggauuua uuuuaucuuu ucuaacacug      60

ucugguaacg auguuuaaag agugagcca                                        89


<210>  1715
<211>  86
<212>  RNA
<213>  Bos taurus

<400>  1715
gggccucugu ggacaucuua ccggacagug cuggauuucu cggcucgacu cuaacacugu      60

cugguaacga uguucaaagg ugaccc                                           86


<210>  1716
<211>  86
<212>  RNA
<213>  Danio rerio

<400>  1716
ggcacuuagc agccaucuua ccggacagug cuggacugua uaacuguuuu cuaacacugu      60

cugguaacga uguuuguugg gugacc                                           86


<210>  1717
<211>  83
<212>  RNA
<213>  Fugu rubripes

<400>  1717
ucucaggauc caucuuaccc gacagugcug gauuguacua cuguuguucu aacacugucu      60

gguaacgaug uuuucugggu gac                                              83


<210>  1718
<211>  62
<212>  RNA
<213>  Tetraodon nigroviridis

<400>  1718
caucuuaccu gacagugcug gauuauacua cuguuguucu aacacugucu gguaacgaug      60

uu                                                                     62


<210>  1719
<211>  68
<212>  RNA
<213>  Mus musculus

<400>  1719

uggacuuccc uuugucaucc uaugccugag aauauaugaa ggaggcuggg aaggcaaagg     60

gacguuca                                                              68


<210>  1720
<211>  110
<212>  RNA
<213>  Rattus norvegicus

<400>  1720
ggcuacagcc cuucuucaug ugacucgugg acuucccuuu gucauccuau gccugagaau     60

auaugaagga ggcugggaag gcaaagggac guucaauugu caucacuggc               110


<210>  1721
<211>  110
<212>  RNA
<213>  Saguinus labiatus

<400>  1721
ggcuacaguc uuucuucaug ugacucgugg acuucccuuu gucauccuau gccugagaau     60

auaugaagga ggcugggaag gcaaagggac guucaauugu caucacuggc               110


<210>  1722
<211>  110
<212>  RNA
<213>  Macaca nemestrina

<400>  1722
ggcuacaguc uuucuucaug ugacucgugg acuucccuuu gucauccuau gccugagaau     60

auaugaagga ggcugggaag gcaaagggac guucaauugu caucacuggc               110


<210>  1723
<211>  110
<212>  RNA
<213>  Gorilla gorilla

<400>  1723
ggcuacaguc uuucuucaug ugacucgugg acuucccuuu gucauccuau gccugagaau     60

auaugaagga ggcugggaag gcaaagggac guucaauugu caucacuggc               110


<210>  1724
<211>  110
<212>  RNA
<213>  Pan paniscus

<400>  1724
ggcuacaguc uuucuucaug ugacucgugg acuucccuuu gucauccuau gccugagaau     60

auaugaagga ggcugggaag gcaaagggac guucaauugu caucacuggc               110


<210>  1725
<211>  110
<212>  RNA
<213>  Pan troglodytes

<400>  1725
ggcuacaguc uuucuucaug ugacucgugg acuucccuuu gucauccuau gccugagaau     60

auaugaagga ggcugggaag gcaaagggac guucaauugu caucacuggc 110

<210> 1726
<211> 110
<212> RNA
<213> Pongo pygmaeus

<400> 1726
ggcuacaguc uuucuucaug ugacucgugg acuucccuuu gucauccuau gccugagaau 60

auaugaagga ggcugggaag gcaaagggac guucaauugu caucacuggc 110

<210> 1727
<211> 97
<212> RNA
<213> Monodelphis domestica

<400> 1727
caacugacua ugugaccugu gggcuucccu uugucauccu augccuggaa aucagagugg 60

ggcagggaca gcaaagggau gcucagcugu cgucuuc 97

<210> 1728
<211> 104
<212> RNA
<213> Gallus gallus

<400> 1728
gucaacagug ucuguucaug ugacccgugg acuucccuuu gucauccuau gccugaaaau 60

auaugaaggg ggcggggaag gcaaagggac guucaacugu cauc 104

<210> 1729
<211> 101
<212> RNA
<213> Gallus gallus

<400> 1729
cuacugagac ugugugaccu gugggcuucc cuuugucauc cuaugccugg agaucacagu 60

gaggcaggga cagcaaaggg augcucagcu gucgucucuu c 101

<210> 1730
<211> 105
<212> RNA
<213> Xenopus tropicalis

<400> 1730
uagaggacuu cgacauugug acccaugggc uucccuuugu cauccuaugc cugagaaugc 60

uggagaggca gggacagcaa agggaugcuc agauguuacc ucuug 105

<210> 1731
<211> 88
<212> RNA
<213> Xenopus tropicalis

<400> 1731
augugaccug ugggcuuccc uuugucaucc uaugccugag aauauaugaa gggggcuggg 60

aaggcaaagg gacguucagu ugucauca 88

<210>  1732
<211>  96
<212>  RNA
<213>  Bos taurus

<400>  1732
uuucuucaug ugacucgugg acuucccuuu gucauccuau gccugagaau auaugaagga      60

ggcugggaag gcaaagggac guucaauugu caucac                                96


<210>  1733
<211>  109
<212>  RNA
<213>  Sus scrofa

<400>  1733
ggcuacaguc cuucucaugu gacucgugga cuucccuuug ucauccuaug ccugagaaua      60

uaugaaggag gcugggaagg caaagggacg uucaauuguc aucacuggc                 109


<210>  1734
<211>  93
<212>  RNA
<213>  Danio rerio

<400>  1734
ucaugugacc uguggacuuc ccuuugucau ccuaugccug gaguaauaga gggggcuggg      60

aagucaaagg gacgcucagg cgucaucauu cgc                                   93


<210>  1735
<211>  124
<212>  RNA
<213>  Danio rerio

<400>  1735
guguugcucu agugaccagu uugugaccuc cuggguuucc cuuugucauc cuaugccugc      60

aguuccugau gaggcuggga cagcaaaggg agguucagau gucgaccugu acuacaguca     120

auac                                                                  124


<210>  1736
<211>  124
<212>  RNA
<213>  Danio rerio

<400>  1736
guguugcucu agugaccagu uugugaccuc cuggguuucc cuuugucauc cuaugccugc      60

aguuccugau gaggcuggga cagcaaaggg agguucagau gucgaccugu acuacaguca     120

auac                                                                  124


<210>  1737
<211>  92
<212>  RNA
<213>  Fugu rubripes

<400>  1737
ugugaccugu gggcuucccu uugucauccu augccuggag cucggauaag gcagggacag      60

caaagggaug cucagcuguc accacugaca uc 92

<210> 1738
<211> 93
<212> RNA
<213> Tetraodon nigroviridis

<400> 1738
ugugaccugu gggcuucccu uugucauccu augccuggag cucggauaag gcagggacag 60

caaagggaug cucagccguc accacugacu uca 93

<210> 1739
<211> 106
<212> RNA
<213> Mus musculus

<400> 1739
cugcuuggac cugugaccug ugggcuuccc uuugucaucc uuugccuagg ccucugagug 60

aggcaaggac agcaaagggg ggcucagugg ucaccucuac ugcaga 106

<210> 1740
<211> 106
<212> RNA
<213> Rattus norvegicus

<400> 1740
cagcuuggac cugugaccuc ugggcuuccc uuugucaucc uuugccuagg ccucugagug 60

gggcaaggac agcaaagggg ggcucagugg ucaccucuac ugcaga 106

<210> 1741
<211> 109
<212> RNA
<213> Macaca mulatta

<400> 1741
ucacuggcca ugugacuugu gggcuucccu uugucauccu uugccuaggg cucugagcag 60

ggcagggaca gcaaaggggu gcucaguugu cacuucccac agcacagag 109

<210> 1742
<211> 109
<212> RNA
<213> Macaca nemestrina

<400> 1742
ucacuggcca ugugacuugu gggcuucccu uugucauccu ucgccuaggg cucugagcag 60

ggcagggaca gcaaaggggu gcucaguugu cacuucccac agcacagag 109

<210> 1743
<211> 110
<212> RNA
<213> Pongo pygmaeus

<400> 1743
ucacccagcc augugacuug uggacuuccc uuugucaucc uucgccuagg gcucugagca 60

gggcagggac agcaaagggg ugcucaguug ucacuuccca cagcacggag 110

<210> 1744
<211> 74
<212> RNA
<213> Gallus gallus

<400> 1744
ccugugagcu ucccuuuguc auccuaugcc ugagcgaugc cuggaggcug ggacggugaa 60

gggaggccca cggg 74


<210> 1745
<211> 68
<212> RNA
<213> Mus musculus

<400> 1745
cucuuguccu ucauuccacc ggagucuguc uuaugccaac cagauuucag uggagugaag 60

cucaggag 68


<210> 1746
<211> 108
<212> RNA
<213> Rattus norvegicus

<400> 1746
aaacagcccc agacaaucca ugggucccuc uguccuucau uccaccggag ucugucuuau 60

gccaaccaga uuucagugga gugaagcuca ggaggcaugg agcugcca 108


<210> 1747
<211> 109
<212> RNA
<213> Ateles geoffroyi

<400> 1747
aaagauccuc gggcaaucca ugugcuucuc uuguccuuca uuccaccgga gucugucuca 60

uacccaacca gauuucagug gagugaaguc aggaggcaug gagcugacg 109


<210> 1748
<211> 110
<212> RNA
<213> Lagothrix lagotricha

<400> 1748
aaagauccuc gggcaaucca ugugcuucuc uuguccuuca uuccaccgga gucugucuca 60

uacccaacca gauuucagug gagugaaguu caggaggcau ggagcugacg 110


<210> 1749
<211> 110
<212> RNA
<213> Macaca nemestrina

<400> 1749
aaagauccuc aggcaaucca ugugcuucuc uuguccuuca uuccaccgga gucugucuca 60

uacccaacca gauuucagug gagugaaguu caggaggcau ggagcugacg 110


<210> 1750

<211>   110
<212>   RNA
<213>   Gorilla gorilla

<400>   1750
aaagauccuc agacaaucca ugugcuucuc uuguccuuca uuccaccgga gucugucuca      60

uacccaacca gauuucagug gagugaaguu caggaggcau ggagcugaca                110


<210>   1751
<211>   110
<212>   RNA
<213>   Pan paniscus

<400>   1751
aaagauccuc agacaaucca ugugcuucuc uuguccuuca uuccaccgga gucugucuca      60

uacccaacca gauuucagug gagugaaguu caggaggcau ggagcugaca                110


<210>   1752
<211>   110
<212>   RNA
<213>   Pan troglodytes

<400>   1752
aaagauccuc agacaaucca ugugcuucuc uuguccuuca uuccaccgga gucugucuca      60

uacccaacca gauuucagug gagugaaguu caggaggcau ggagcugaca                110


<210>   1753
<211>   96
<212>   RNA
<213>   Gallus gallus

<400>   1753
gacaauccau ggguucuguu guccuucauu ccaccggagu cugucucgua ccuaaccaga      60

uuucagugga gugaagcaca agagacaugg agauga                                96


<210>   1754
<211>   98
<212>   RNA
<213>   Gallus gallus

<400>   1754
auacacuacu agggccuucc ugaugcccuu cauuccaccg gaaucuguca gugcagaaac      60

cagauuucag ugaaaugaag cccaucagag aggcagcu                              98


<210>   1755
<211>   86
<212>   RNA
<213>   Xenopus tropicalis

<400>   1755
ccacgugucc ugcuguccuu cauuccaccg gagucugucu cauacauaau cagauuucag      60

uggagugaag cacaagaggc auguag                                           86


<210>   1756
<211>   69
<212>   RNA

<213>  Bos taurus

<400>  1756
cucuuguccu ucauuccacc ggagucuguc ucguacccaa ccagauuuca guggagugaa      60

guucaggag                                                              69


<210>  1757
<211>  69
<212>  RNA
<213>  Sus scrofa

<400>  1757
cucuuguccu ucauuccacc ggagucuguc ucauacccaa ccagauuuca guggagugaa      60

gcucaggag                                                              69


<210>  1758
<211>  89
<212>  RNA
<213>  Danio rerio

<400>  1758
aaacuacugu gcauucuauc cuucauucca ccggagucug uguaguuguu caaucagauu      60

ucaguggugu gaaguguagg aaacacgga                                        89


<210>  1759
<211>  79
<212>  RNA
<213>  Fugu rubripes

<400>  1759
uguguucuau ccuucauucc accggagucu guauguguau uuaaccagau uucaguggug      60

ugaaguguaa gagacaugg                                                   79


<210>  1760
<211>  66
<212>  RNA
<213>  Tetraodon nigroviridis

<400>  1760
uauccuucau uccaccggag ucuguaacug uuuuuaacca gauuucagug gugugaagug      60

uaagag                                                                 66


<210>  1761
<211>  83
<212>  RNA
<213>  Mus musculus

<400>  1761
uuccuuugac gggugagcuu uuggcccggg uuauaccuga cacucacgua uaagacgagc      60

aaaaagcuug uuggucagag gag                                              83


<210>  1762
<211>  83
<212>  RNA
<213>  Rattus norvegicus

<400>  1762
uuccuuugac gggugagcuu uuggcccggg uuauaccuga cucucacgua uaagacgagc      60

aaaaagcuug uuggucagag gag                                              83


<210>  1763
<211>  77
<212>  RNA
<213>  Monodelphis domestica

<400>  1763
uuccugugac aggugagcuu uugguccggg uuauaccuga ugcaugugua uaagacgagc      60

aaaaagcucg uuggucg                                                     77


<210>  1764
<211>  81
<212>  RNA
<213>  Xenopus tropicalis

<400>  1764
uccuuucaac aggcaagcuu uuugcuugga uuauguuuuc uguuguugua uaagacgagc      60

auaaagcuug uuuguuagaa g                                                81


<210>  1765
<211>  79
<212>  RNA
<213>  Mus musculus

<400>  1765
cccucagugg cucaguagcc aguguagauc cugucuuugg uaaucagcag cuacaucugg      60

cuacuggguc ucugguggc                                                   79


<210>  1766
<211>  103
<212>  RNA
<213>  Rattus norvegicus

<400>  1766
aaggauuagg gugcccucag uggcucagua gccaguguag auccugucuu ugguaaucag      60

cagcuacauc uggcuacugg gucucugaug gcaucaucua gcu                       103


<210>  1767
<211>  110
<212>  RNA
<213>  Ateles geoffroyi

<400>  1767
ucugccggaa ggugugggua cccucagugg cucaguagcc aguguagauc cugucuuuug      60

uaaucaguag cuacaucugg cuacuggguc ucugauggca ucuuuuagcu                110


<210>  1768
<211>  98
<212>  RNA
<213>  Gallus gallus

<400>  1768
uguaguugcc caucaaucgc ucaguaguca guguagauuc ugucuuuaca aucagcagcu      60

acaucuggcu acugggucuc ugaugacauc ucauaucu 98

<210> 1769
<211> 64
<212> RNA
<213> Xenopus tropicalis

<400> 1769
cgcucaguaa ucaguguaga uccuguauau cugucagcag cuacaucugg cuacuggguc 60

ucua 64

<210> 1770
<211> 110
<212> RNA
<213> Bos taurus

<400> 1770
gcugcuggaa uguguaggua cccucaaugg cucaguagcc aguguagauc cugucuuuug 60

uaaucaguag cuacaucugg cuacuggguc ucugauggca ucuucuaccu 110

<210> 1771
<211> 108
<212> RNA
<213> Danio rerio

<400> 1771
gccgguggcu uguucgggug cucaugagau gcucaguagu caguguagau ccugugucac 60

aaucagcagc uacaucuggc uacugggucu cugauggcau uuucugcu 108

<210> 1772
<211> 69
<212> RNA
<213> Fugu rubripes

<400> 1772
ucaguugcuc aguaggcagu guagauccug uguagcaauc agcagcuaca ucuggcuacu 60

gggucucug 69

<210> 1773
<211> 68
<212> RNA
<213> Tetraodon nigroviridis

<400> 1773
caguugcuca guaggcagug uagauccugu guagcaauca gcagcuacau cuggcuacug 60

ggucucug 68

<210> 1774
<211> 89
<212> RNA
<213> Mus musculus

<400> 1774
   aacugacuau gccuccucgc auccccuagg gcauuggugu aaagcuggag acccacugcc 60
   ccaggugcug cuggggguug uagucugac 89

<210>  1775
<211>  83
<212>  RNA
<213>  Rattus norvegicus

<400>  1775
cugacuaugc cuccucgcau ccccuagggc auugguguaa agcuggagac ccacugcccc      60

aggugcugcu gggguugua guc                                              83


<210>  1776
<211>  97
<212>  RNA
<213>  Mus musculus

<400>  1776
uguucgcuuc ugguaccgga agagagguuu ucugggucuc uguuucuuug augagaauga      60

aacacaccca gcuaaccuuu uuuucaguau caaaucc                              97


<210>  1777
<211>  97
<212>  RNA
<213>  Rattus norvegicus

<400>  1777
uguucgcuuc ugguaccgga agagagguuu ucugggucuc uguuucuuug augagaauga      60

aacacaccca gcuaaccuuu uuuucaguau caaaucc                              97


<210>  1778
<211>  96
<212>  RNA
<213>  Rattus norvegicus

<400>  1778
gagucugguc uuguuugggu uuguucuagg uaugguccca gggaucccag aucaaaccag      60

gccccugggc cuauccuaga accaaccuaa acccau                               96


<210>  1779
<211>  90
<212>  RNA
<213>  Bos taurus

<400>  1779
gaguuugguu uuguuugggu uuguucuagg uaugguccca gggaucccag aucaaaccag      60

gccccugggc cuauccuaga accaaccuaa                                      90


<210>  1780
<211>  94
<212>  RNA
<213>  Bos taurus

<400>  1780
uggaagcggg ugcgaggcga ggggugcuau cugugguuga ggacacggca aaugaaacug      60

ucucacacag aaaucgcacc caucuccucg gccc                                 94

<210>  1781
<211>  98
<212>  RNA
<213>  Mus musculus

<400>  1781
ucuguguugg gcgucugucu gcccgagugc cugccucucu guugcucuga aggaggcagg      60

ggcugggccu gcagcugccu gggcagagcu gcuccuuc                              98


<210>  1782
<211>  98
<212>  RNA
<213>  Rattus norvegicus

<400>  1782
ucuguguugg gcaucugucu gccugagugc cugccucucu guugcucuga aggaggcagg      60

ggcugggccu gcagcugccu gggcagagcu gcuccuuc                              98


<210>  1783
<211>  82
<212>  RNA
<213>  Mus musculus

<400>  1783
ugguauuuaa aagguggaua uuccuucuau gguuacgugc uuccuggaua aucauagagg      60

aacauccacu uuuucaguau ca                                               82


<210>  1784
<211>  68
<212>  RNA
<213>  Mus musculus

<400>  1784
ugagcagagg uugcccuugg ugaauucgcu uuauugaugu ugaaucacac aaaggcaacu      60

uuuguuug                                                               68


<210>  1785
<211>  72
<212>  RNA
<213>  Rattus norvegicus

<400>  1785
cuugagcaga gguugcccuu ggugaauucg cuuuauugau guugaaucac acaaaggcaa      60

cuuuugguug ag                                                          72


<210>  1786
<211>  61
<212>  RNA
<213>  Bos taurus

<400>  1786
aagaugguug accauagaac augcgcuguc ucuaugucgu auguaaugug guccacgucu      60

u                                                                      61


<210>  1787
<211>  76

<212>  RNA
<213>  Mus musculus

<400>  1787
uacuugaaga gaaguuguuc gugguggauu cgcuuuacuu gugacgaauc auucacggac      60

aacacuuuuu ucagua                                                      76


<210>  1788
<211>  80
<212>  RNA
<213>  Rattus norvegicus

<400>  1788
gguacuugaa gagaaguugu ucguggugga uucgcuuuac uugugacgaa ucauucacgg      60

acaacacuuu uuucaguacc                                                  80


<210>  1789
<211>  70
<212>  RNA
<213>  Rattus norvegicus

<400>  1789
gggacggaug gucgaccagc uggaaaguaa uuguuucuaa uguacuucac cugguccacu      60

agccgucggu                                                             70


<210>  1790
<211>  79
<212>  RNA
<213>  Rattus norvegicus

<400>  1790
gaaguuaggc ugaggggcag agagcgagac uuuucuauuu uccaaaagcu cggucugagg      60

ccccucaguc uugcuuccu                                                   79


<210>  1791
<211>  91
<212>  RNA
<213>  Mus musculus

<400>  1791
cguccugcga ggugucuugc aggccgucau gcaggccaca cugacgguaa cguugcaggu      60

cgucuugcag ggcuucucgc aagacgacau c                                     91


<210>  1792
<211>  114
<212>  RNA
<213>  Rattus norvegicus

<400>  1792
uccugcgcgu ccugcgaggu gucuugcagg ccgucaugca ggccacacug acgguaacgu      60

ugcaggucgu cuugcagggc uucucgcaag acgacaucuu caucgccaac gacg           114


<210>  1793
<211>  91
<212>  RNA
<213>  Ovis aries

<400>  1793
cguccugcga ggugucuugc aggccgucau gcaggccaca cugacggaaa cguugcaggu      60

cgucuugcag ggcgucucgc aagacgacac a                                      91


<210>  1794
<211>  124
<212>  RNA
<213>  Mus musculus

<400>  1794
ugcccgggga gaaguacggu gagccuguca uuauucagag aggcuagauc cucuguguug      60

agaaggauca ugaugggcuc cucgguguuc uccagguagc ggcaccacac caugaaggca     120

gccc                                                                   124


<210>  1795
<211>  93
<212>  RNA
<213>  Rattus norvegicus

<400>  1795
ccggggagaa guacggugag ccugucauua uucagagagg cuagauccuc uguguugaga      60

aggaucauga ugggcuccuc gguguucucc agg                                    93


<210>  1796
<211>  80
<212>  RNA
<213>  Mus musculus

<400>  1796
agacucgggu aggcaguguu guuagcuggc ugcguugggu caggccagca gccacagcua      60

cccugccacu uccuucuggc                                                   80


<210>  1797
<211>  109
<212>  RNA
<213>  Mus musculus

<400>  1797
aggaugaagu gugggugugu caggcaggca gugcauugcu agcuggcugu uagaacuuua      60

ucccaacagu ugcuagcugc acuacccucu gcugcacuca gaagcaugc                  109


<210>  1798
<211>  91
<212>  RNA
<213>  Rattus norvegicus

<400>  1798
cugugugcga uggguuggca guguauuguu agcugguuga guauguaaaa ggcaccagcu      60

aacaugcaac ugcucuccua uugcacauac a                                      91


<210>  1799
<211>  91
<212>  RNA
<213>  Canis familiaris

<400>  1799
ccguguguga uggguuggca guguauuguu agcugguuga auauaugaau ggcaucagcu      60

aacaugcaac ugcuaucuua uugcauauac a                                    91


<210>  1800
<211>  79
<212>  RNA
<213>  Monodelphis domestica

<400>  1800
ugugauggga uggcagugua uuguuagcug guugacuauc ugaacgugcc agcuaacaug      60

caacugcuau cccauugca                                                  79


<210>  1801
<211>  86
<212>  RNA
<213>  Gallus gallus

<400>  1801
cugugugcgg ugggguggca guguauguua gcugguugaa acucuugaca ucagcuaaca      60

cgcaguugcu aaccugcucc acauac                                          86


<210>  1802
<211>  87
<212>  RNA
<213>  Xenopus tropicalis

<400>  1802
cuguguuuag aaggaaggca guguaauguu agcugguugg aaaauagcag acacugguua      60

acuuacaccu gcccccuuc uuuacau                                          87


<210>  1803
<211>  72
<212>  RNA
<213>  Mus musculus

<400>  1803
cuugggaaug gcgaggaaac cguuaccauu acugaguuua guaaugguaa cgguucucuu      60

gcugcuccca ca                                                         72


<210>  1804
<211>  72
<212>  RNA
<213>  Rattus norvegicus

<400>  1804
uuugggaaug gcgaggaaac cguuaccauu acugaguuua guaaugguaa ugguucucuu      60

gcugcuccca ca                                                         72


<210>  1805
<211>  62
<212>  RNA
<213>  Monodelphis domestica

<400>  1805

cuggcgggga aaccguuacc auuacugugu uuaguaaugg uaaggguucu cccgcugcgc 60

ug 62

<210> 1806
<211> 69
<212> RNA
<213> Gallus gallus

<400> 1806
acacauggcu ggcagggaaa ccguuaccau uacugaguuu aguaauggua acgguucugc 60

ugacagcca 69

<210> 1807
<211> 62
<212> RNA
<213> Xenopus tropicalis

<400> 1807
gaguggcaau gaaaccguua ccauuacuga guuuaguaau gguaaggguu cuguugcugc 60

uc 62

<210> 1808
<211> 60
<212> RNA
<213> Danio rerio

<400> 1808
agaggcggcg aaaccguuac cauuacugag uuuaguaaug guaaggguuc ugcugccuuu 60

<210> 1809
<211> 85
<212> RNA
<213> Mus musculus

<400> 1809
gcuaagcagu uacaacuguu ugcagaggaa acugagacuu uauaacuaug ucucagucuc 60

aucugcaaag agguaagugc uuugc 85

<210> 1810
<211> 78
<212> RNA
<213> Rattus norvegicus

<400> 1810
ggugugagcg uaugugccuu uggacuacau cguggacgca gcaccaugca guccacgggc 60

auauacacuu gccucaag 78

<210> 1811
<211> 86
<212> RNA
<213> Gallus gallus

<400> 1811
ucccuggugu gaggguaugu gcccuuggac uacaucgugg aagccagcac caugcagucc 60

augggcauau acacuugccu caaggu 86

<210>  1812
<211>  87
<212>  RNA
<213>  Xenopus tropicalis

<400>  1812
ucccuggcgu gaggguaugu gcccuuggac uacaucgugg aagccagcac caugcagucc      60

augggcauau acacuugccu caagguu                                          87


<210>  1813
<211>  89
<212>  RNA
<213>  Bos taurus

<400>  1813
ucccuggcgu gaggguaugu gccuuuggac uacaucgugg aagccagcac caugcagucc      60

augggcauau acacuugccu caaggccua                                        89


<210>  1814
<211>  96
<212>  RNA
<213>  Danio rerio

<400>  1814
ucccuggugu gaggguaugu gcccuuggac uacaucgugg aagccagcac caugcagucc      60

augggcauau acacuugccu caaggccuag aucuuc                                96


<210>  1815
<211>  84
<212>  RNA
<213>  Fugu rubripes

<400>  1815
cccuggugug aggguaugug cccuuggacu acaucgugga agccagcacc augcagucca      60

ugggcauaua cacuugccuc augg                                             84


<210>  1816
<211>  84
<212>  RNA
<213>  Tetraodon nigroviridis

<400>  1816
cccugaugug aggguaugug cccuuggacu acaucgugga agccagcacc augcagucca      60

ugggcauaua cacuuuccuc aagg                                             84


<210>  1817
<211>  73
<212>  RNA
<213>  Rattus norvegicus

<400>  1817
gagggggaag acgggagaag agaagggagu gguuuuuggg ugccucacuc cuccccuccc      60

gucuuguucu cuc                                                         73


<210>  1818

<211>  84
<212>  RNA
<213>  Gallus gallus

<400>  1818
gugguggcuu gguggucgua uguaugacgu cauuuacuug gacuuuuagg agugacauca      60

uauguacggc ugcuaaacug cugc                                              84


<210>  1819
<211>  84
<212>  RNA
<213>  Xenopus tropicalis

<400>  1819
gugguggcuu aguggucgua uguaugacgu cauuuacugg auuuuuuagg agugacauca      60

uauguacggc ugcuaaacug cugc                                              84


<210>  1820
<211>  104
<212>  RNA
<213>  Danio rerio

<400>  1820
acugacuuga gugguggccu gguggucgua uguaugacgu cauuuacuuc aaaguuugga      60

gugacaucau auguacggcu gcuaaacugc uacauggcuc auca                      104


<210>  1821
<211>  102
<212>  RNA
<213>  Fugu rubripes

<400>  1821
cuucugauac uggugguggc uugguggucg uaugcaugac gucauuuacu ucgaugauug      60

gagugacauc auauguacgg cugcuaaacu gcuacaagag ac                        102


<210>  1822
<211>  92
<212>  RNA
<213>  Tetraodon nigroviridis

<400>  1822
uggugguggc cugguggucg uaugcaugac gucauuuacu ucgaugauug gagugacauc      60

auauguacgg cugcuaaacc gcuacaagag ac                                    92


<210>  1823
<211>  86
<212>  RNA
<213>  Mus musculus

<400>  1823
aauugacuua gcugggaagu ggggaacccu uccaugagga guagaacacu ccuuaugcaa      60

gauucccuuc uaccugacug aguuga                                            86


<210>  1824
<211>  85
<212>  RNA

<213>  Mus musculus

<400>  1824
uugauacuug aaggagaggu uguccguguu gucuucucuu uauuuaugau gaaacauaca      60

cgggaaaccu cuuuuuuagu aucaa                                            85


<210>  1825
<211>  83
<212>  RNA
<213>  Rattus norvegicus

<400>  1825
ugauacuuga aggagagguu guccguguug ucuucucuuu auuuaugaug aaacauacac      60

gggaaaccuc uuuuuuagua uca                                              83


<210>  1826
<211>  84
<212>  RNA
<213>  Rattus norvegicus

<400>  1826
gcucguccuc ucuaauccuu ugucccuggg ugaaaaugcu auuuguaugc aaugcacccg      60

ggcaaggauu uggggaaggu aagc                                             84


<210>  1827
<211>  79
<212>  RNA
<213>  Mus musculus

<400>  1827
uccuguuggg agacccuggu cugcacucua ucuguaaacu uacugaaggg agagcagggc      60

aggguuuccc auacagagg                                                   79


<210>  1828
<211>  67
<212>  RNA
<213>  Rattus norvegicus

<400>  1828
agugggggag ccaggaagua uugauguuuc ugccagguua gcgucaacac uugcugguuu      60

ccucucu                                                                67


<210>  1829
<211>  95
<212>  RNA
<213>  Homo sapiens

<400>  1829
ggugagugcg uuuccaagug ugaagggacc cuuccuguag ugucuuauau acaauacagu      60

aggaauguuc cuucuuugcc acucauacac cuuua                                 95


<210>  1830
<211>  110
<212>  RNA
<213>  Homo sapiens

<400>  1830
ccagagguug uaacguuguc uauauauacc cuguagaacc gaauuugugu gguauccgua      60

uagucacaga uucgauucua ggggaauaua uggucgaugc aaaaacuuca                110


<210>  1831
<211>  84
<212>  RNA
<213>  Homo sapiens

<400>  1831
gucagaauaa ugucaaagug cuuacagugc agguagugau augugcaucu acugcaguga      60

aggcacuugu agcauuaugg ugac                                             84


<210>  1832
<211>  88
<212>  RNA
<213>  Homo sapiens

<400>  1832
ucccaugcug ugacccucca aagggaagcg cuuucuguuu guuuucucuu aaacaaagug      60

ccucccuuua gaguguuacc guuuggga                                         88


<210>  1833
<211>  83
<212>  RNA
<213>  Homo sapiens

<400>  1833
ucaggcugug acccucuuga gggaagcacu uucuguuguc ugaaagaaga gaaagugcuu      60

ccuuuuagag gcuuacuguc uga                                              83


<210>  1834
<211>  84
<212>  RNA
<213>  Mus musculus

<400>  1834
gucagaauaa ugucaaagug cuuacagugc agguagugau gugugcaucu acugcaguga      60

gggcacuugu agcauuaugc ugac                                             84


<210>  1835
<211>  88
<212>  RNA
<213>  Mus musculus

<400>  1835
agucaugggg gcuccaaagu gcuguucgug cagguagugu aauuaccuga ccuacugcug      60

agcuagcacu ucccgagccc ccaggaca                                         88


<210>  1836
<211>  84
<212>  RNA
<213>  Rattus norvegicus

<400>  1836
gucaggauaa ugucaaagug cuuacagugc agguaguggu gugugcaucu acugcaguga      60

aggcacuugu ggcauugugc ugac 84

<210> 1837
<211> 78
<212> RNA
<213> Rattus norvegicus

<400> 1837
uuggccaugu caaagugcuu acagugcagg uagcuucuug agaucuacug cacugcaagc 60

acuucuuaca uuaccaug 78

<210> 1838
<211> 87
<212> RNA
<213> Rattus norvegicus

<400> 1838
agucaugggg gcuccaaagu gcuguucgug cagguagugc auugccugac cuacugcuga 60

gcuagcacuu cccgagcccc caggaca 87

<210> 1839
<211> 73
<212> RNA
<213> Xenopus tropicalis

<400> 1839
augggcuuca aagugcuguu cgugcaggua gcuuaauaac agaccuacug caugggcggc 60

acuucccaag ccc 73

<210> 1840
<211> 77
<212> RNA
<213> Bos taurus

<400> 1840
cugggggcuc caaagugcug uucgugcagg uagugugauc accugaccua cugcugagcc 60

agcacuuccc gagcccc 77

<210> 1841
<211> 138
<212> RNA
<213> Danio rerio

<400> 1841
ggacuuucuu gaguggacuu gguugguguc aauguauugu caaagugcuu acagugcagg 60

uaguauuaug gaauaucuac ugcaguggag gcacuucuag caauacacuu gaccauuuua 120

accuuccucc aggcaucc 138

<210> 1842
<211> 83
<212> RNA
<213> Danio rerio

<400> 1842
gucacuguag ugucaaagug cuuacagugc agguaguuca auauaaucua cugcagugga 60

ggcacuucaa gcuuuaccgu gac                                          83

<210>  1843
<211>  74
<212>  RNA
<213>  Danio rerio

<400>  1843
guguguguua aaagugcugu uugugcaggu aguguguuuc cucuacugua ggagcagcac      60

uucacaacac acac                                                       74

<210>  1844
<211>  86
<212>  RNA
<213>  Fugu rubripes

<400>  1844
gucuguguau ugccaaagug cuuacagugc agguaguucu augugacacc uacugcaaug      60

gaggcacuua cagcaguacu cuugac                                          86

<210>  1845
<211>  82
<212>  RNA
<213>  Fugu rubripes

<400>  1845
gucaaaguag ugucaaagug cuuacagugc agguaguuug augacaucua cugcagugaa      60

ggcacuuuca gcgcuauucu ga                                              82

<210>  1846
<211>  86
<212>  RNA
<213>  Tetraodon nigroviridis

<400>  1846
gucuguguau ugccaaagug cuuacagugc agguaguacu auguaacacc uacugcaaug      60

gaggcacuua cagcaguacu cuugac                                          86

<210>  1847
<211>  82
<212>  RNA
<213>  Tetraodon nigroviridis

<400>  1847
gucaccguag ugucaaagug cuuacagugc agguaguuaa aagaaaucua cugcagugaa      60

ggcacuuuca gcacuauucu ga                                              82

<210>  1848
<211>  78
<212>  RNA
<213>  Homo sapiens

<400>  1848
cuuucuacac agguugggau cgguugcaau gcuguguuuc uguaugguau ugcacuuguc      60

ccggccuguu gaguuugg                                                   78

<210> 1849
<211> 75
<212> RNA
<213> Homo sapiens

<400> 1849
ucaucccugg guggggauuu guugcauuac uuguguucua uauaaaguau ugcacuuguc 60

ccggccugug gaaga 75


<210> 1850
<211> 96
<212> RNA
<213> Homo sapiens

<400> 1850
cgggccccgg gcgggcggga gggacgggac gcggugcagu guuguuuuuu cccccgccaa 60

uauugcacuc gucccggccu ccggcccccc cggccc 96


<210> 1851
<211> 75
<212> RNA
<213> Homo sapiens

<400> 1851
uguugucggg uggaucacga ugcaauuuug augaguauca uaggagaaaa auugcacggu 60

auccaucugu aaacc 75


<210> 1852
<211> 68
<212> RNA
<213> Homo sapiens

<400> 1852
ccauuacugu ugcuaauaug caacucuguu gaauauaaau uggaauugca cuuuagcaau 60

ggugaugg 68


<210> 1853
<211> 80
<212> RNA
<213> Mus musculus

<400> 1853
cuuucuacac agguugggau uugucgcaau gcuguguuuc ucuguauggu auugcacuug 60

ucccggccug uugaguuugg 80


<210> 1854
<211> 91
<212> RNA
<213> Mus musculus

<400> 1854
ugcccauuca uccacaggug gggauuggug gcauuacuug uguuagauau aaaguauugc 60

acuugucccg gccugaggaa gaaagagggu u 91

<210>  1855
<211>  83
<212>  RNA
<213>  Mus musculus

<400>  1855
ggugggcggg agggacggga cguggugcag uguuguucuu uccccugcca auauugcacu      60

cgucccggcc uccggccccc ucg                                              83


<210>  1856
<211>  75
<212>  RNA
<213>  Mus musculus

<400>  1856
uguuaucagg uggaacacga ugcaauuuug guugguguaa uaggaggaaa auugcacggu      60

auccaucugu aaacc                                                       75


<210>  1857
<211>  75
<212>  RNA
<213>  Mus musculus

<400>  1857
aggccgucac uguugcuaac augcaacucu guugaauaga aauuggaauu gcacuuuagc      60

aauggugaug gaccu                                                       75


<210>  1858
<211>  78
<212>  RNA
<213>  Rattus norvegicus

<400>  1858
cuuucuacac agguugggau uugucgcaau gcuguguuuc uguauaguau ugcacuuguc      60

ccggccuguu gaguuugg                                                    78


<210>  1859
<211>  92
<212>  RNA
<213>  Rattus norvegicus

<400>  1859
ugcccauuca uccacaggug gggauuagug ccauuacuug uguuagauaa aaaguauugc      60

acuugucccg gccugaggaa gaaaagaggg uu                                    92


<210>  1860
<211>  83
<212>  RNA
<213>  Rattus norvegicus

<400>  1860
ggugggcagg agggacggga cgcggugcag uguuguucuu uccccugcca auauugcacu      60

cgucccggcc uccggccccc ucg                                              83


<210>  1861
<211>  87

<212>   RNA
<213>   Rattus norvegicus

<400>   1861
uuuugcuguu aucgggugga ucacgaugca auuuugauua gaguaauggg agagaaauug      60

cacgguaucc aucuguaaac cgcagga                                          87


<210>   1862
<211>   78
<212>   RNA
<213>   Ateles geoffroyi

<400>   1862
cuuucuacac agguugggau cgguugcaau gcuguguuuc uguaugguau ugcacuuguc      60

ccggccuguu gaguuugg                                                    78


<210>   1863
<211>   75
<212>   RNA
<213>   Ateles geoffroyi

<400>   1863
ucaucccugg guggggauuu guugcauuac uuguguucua uauaaaguau ugcacuuguc      60

ccggccugug gaaga                                                       75


<210>   1864
<211>   78
<212>   RNA
<213>   Lagothrix lagotricha

<400>   1864
cuuucuacac agguugggau cgguugcaau gcuguguuuc uguaugguau ugcacuuguc      60

ccggccuguu gaguuugg                                                    78


<210>   1865
<211>   75
<212>   RNA
<213>   Lagothrix lagotricha

<400>   1865
ucaucccugg guggggauuu guugcauuac uuguguucua uauaaaguau ugcacuuguc      60

ccggccugug gaaga                                                       75


<210>   1866
<211>   78
<212>   RNA
<213>   Saguinus labiatus

<400>   1866
cuuucuacac agguugggau cgguugcaau gcuguguuuc uguaugguau ugcacuuguc      60

ccggccuguu gaguuugg                                                    78


<210>   1867
<211>   75
<212>   RNA
<213>   Saguinus labiatus

<400>  1867
ucaucccugg guggggauuu guugcauuac uuguguucua uauaaaguau ugcacuuguc      60

ccggccugug gaaga                                                        75


<210>  1868
<211>  78
<212>  RNA
<213>  Macaca mulatta

<400>  1868
cuuucuacac agguugggau cgguugcaau gcuguguuuc uguaugguau ugcacuuguc      60

ccggccuguu gaguuugg                                                     78


<210>  1869
<211>  75
<212>  RNA
<213>  Macaca nemestrina

<400>  1869
ucaucccugg guggggauuu guugcauuac uuguguucua uauaaaguau ugcacuuguc      60

ccggccugug gaaga                                                        75


<210>  1870
<211>  78
<212>  RNA
<213>  Gorilla gorilla

<400>  1870
cuuucuacac agguugggau cgguugcaau gcuguguuuc uguaugguau ugcacuuguc      60

ccggccuguu gaguuugg                                                     78


<210>  1871
<211>  75
<212>  RNA
<213>  Gorilla gorilla

<400>  1871
ucaucccugg guggggauuu guugcauuac uuguguucua uauaaaguau ugcacuuguc      60

ccggccugug gaaga                                                        75


<210>  1872
<211>  78
<212>  RNA
<213>  Pan paniscus

<400>  1872
cuuucuacac agguugggau cgguugcaau gcuguguuuc uguaugguau ugcacuuguc      60

ccggccuguu gaguuugg                                                     78


<210>  1873
<211>  75
<212>  RNA
<213>  Pan paniscus

<400>  1873

ucaucccugg guggggauuu guugcauuac uuguguucua uauaaaguau ugcacuuguc 60

ccggccugug gaaga 75

<210> 1874
<211> 78
<212> RNA
<213> Pan troglodytes

<400> 1874
cuuucuacac agguugggau cgguugcaau gcuguguuuc uguaugguau ugcacuuguc 60

ccggccuguu gaguuugg 78

<210> 1875
<211> 74
<212> RNA
<213> Pan troglodytes

<400> 1875
ucaucccugg guggggauuu guugcauuac uuguguucca caaaaguauu gcacuugucc 60

cggccuguga gaga 74

<210> 1876
<211> 78
<212> RNA
<213> Pongo pygmaeus

<400> 1876
cuuucuacac agguugggau cgguugcaau gcuguguuuc uguaugguau ugcacuuguc 60

ccggccuguu gaguuugg 78

<210> 1877
<211> 75
<212> RNA
<213> Pongo pygmaeus

<400> 1877
ucaucccugg guggggauuu guugcauuac uuguguucua uauaaaguau ugcacuuguc 60

ccggccugug gaaga 75

<210> 1878
<211> 78
<212> RNA
<213> Lemur catta

<400> 1878
cuuucuacac agguugggau cgguugcaau gcuguguuuc uguaugguau ugcacuuguc 60

ccggccuguu gaguuugg 78

<210> 1879
<211> 75
<212> RNA
<213> Lemur catta

<400> 1879
ucauccgugg guggggauuu guugcauuac uuguguuaua uguaaaguau ugcacuuguc 60

ccggccugug gaaga                                                    75

<210>  1880
<211>  78
<212>  RNA
<213>  Monodelphis domestica

<400>  1880
cuuucuacac agguugggau caguugcaau gcugugucug ucuguaguau ugcacuuguc      60

ccggccuguu gaguuugg                                                    78

<210>  1881
<211>  78
<212>  RNA
<213>  Gallus gallus

<400>  1881
cuuucuacac agguugggau caguugcaau gcugugcguu ucugugguau ugcacuuguc      60

ccggccuguu gagguugg                                                    78

<210>  1882
<211>  73
<212>  RNA
<213>  Gallus gallus

<400>  1882
aggcuaauac uguugcuaac augcaacucu guuguauaaa aauuggaauu gcacuuuagc      60

aauggugaug gac                                                         73

<210>  1883
<211>  78
<212>  RNA
<213>  Xenopus tropicalis

<400>  1883
cuuucuguau agguugggau ugguugcaau gcuguacuau uuauguagua uugcacuugu      60

cccggccugu uuaggaug                                                    78

<210>  1884
<211>  73
<212>  RNA
<213>  Xenopus tropicalis

<400>  1884
uccucugugg guggggauuu guugcacuac uguauguaug aaaaguauug cacuuguccc      60

ggccuguggg uga                                                         73

<210>  1885
<211>  73
<212>  RNA
<213>  Xenopus tropicalis

<400>  1885
uacagaagga ucgggauguu gugcacuguu guccuuucuc cugccaauau ugcacucguc      60

ccggccuccu gcg                                                         73

<210>  1886
<211>  87
<212>  RNA
<213>  Xenopus tropicalis

<400>  1886
uuuuguuguu cgcgggugga ucacgaugca auuuuauuua guuugguagg agaaaaauug      60

cacgguaucc aucuguaaac cgcagaa                                          87


<210>  1887
<211>  63
<212>  RNA
<213>  Xenopus tropicalis

<400>  1887
uuacuguugc cuaugugcaa acucugugcu auauugucua gaauugcacu guagcaaugg      60

uga                                                                    63


<210>  1888
<211>  68
<212>  RNA
<213>  Bos taurus

<400>  1888
ugggugggga uuuguugcac ugcuuguguu auauauaaag uauugcacuu gucccggccu      60

guggaaga                                                               68


<210>  1889
<211>  85
<212>  RNA
<213>  Danio rerio

<400>  1889
uggucccuuu cugcgcaggu ugggauuggu agcaaugcug uguguuuuga agguauugca      60

cuugucccgg ccuguaaagg auugu                                            85


<210>  1890
<211>  95
<212>  RNA
<213>  Danio rerio

<400>  1890
acagcauccc uuucuuugca gguugggauc ggccgcaaug cucugugcug gaaguauugc      60

acuugucccg gccugugaag agcaugggaa auugu                                 95


<210>  1891
<211>  82
<212>  RNA
<213>  Danio rerio

<400>  1891
uccuacgggc agggaggugu gggauguugu gcaguguugu ucaaucuccc gccaauauug      60

cacucguccc ggccucccug ac                                               82


<210>  1892

<211>  127
<212>  RNA
<213>  Danio rerio

<400>  1892
uaaaugcaaa uauuugucuu gcuguuuucg gguggaugac ucugcaauuu uauuagugau      60

ggaaaaacuu caauaaaaau ugcacgguau ccaucuguaa uccgcuggau uccauuaccu     120

gcguuug                                                               127


<210>  1893
<211>  79
<212>  RNA
<213>  Fugu rubripes

<400>  1893
cuuuccugug cagguuggga gagguagcaa ugcucagaga uuaugcggua uugcacuugu      60

cccggccugu ggaggaagg                                                   79


<210>  1894
<211>  63
<212>  RNA
<213>  Fugu rubripes

<400>  1894
caggugggga ucaguagcaa ugcugugcac uggaagguau ugcacuuguc ccggccugug      60

uag                                                                    63


<210>  1895
<211>  74
<212>  RNA
<213>  Tetraodon nigroviridis

<400>  1895
cuuuccugua cagguuggga gagguagcaa ugcucggagu ucauguggua uugcacuugu      60

cccggccugu ggag                                                        74


<210>  1896
<211>  72
<212>  RNA
<213>  Tetraodon nigroviridis

<400>  1896
cuucccauac agguggggau gaguagcaau gcuguguacu aggagguauu gcacuugucc      60

cggccugugu ag                                                          72


<210>  1897
<211>  78
<212>  RNA
<213>  Homo sapiens

<400>  1897
cugaggagca gggcuuagcu gcuugugagc aggguccaca ccaagucgug uucacagugg      60

cuaaguuccg cccccccag                                                   78


<210>  1898

<211>  88
<212>  RNA
<213>  Homo sapiens

<400>  1898
aacugcccuc aaggagcuua caaucuagcu ggggguaaau gacuugcaca ugaacacaac      60

uagacuguga gcuucuagag ggcaggga                                          88


<210>  1899
<211>  109
<212>  RNA
<213>  Mus musculus

<400>  1899
cuguguuuga aauggggacu gcccucaagg agcuuacaau cuagcugggg guagaugacu      60

ugcacuugaa cacaacuaga cugugagcuu cuagagggca ggggccuua                  109


<210>  1900
<211>  88
<212>  RNA
<213>  Rattus norvegicus

<400>  1900
gacugcccuc aaggagcuua caaucuagcu ggggguagau gacuugcacu ugaacacaac      60

uagacuguga gcuucuagag ggcagggg                                          88


<210>  1901
<211>  81
<212>  RNA
<213>  Homo sapiens

<400>  1901
cuucaggaag cugguuucau auggugguuu agauuuaaau agugauuguc uagcaccauu      60

ugaaaucagu guucuugggg g                                                 81


<210>  1902
<211>  81
<212>  RNA
<213>  Homo sapiens

<400>  1902
cuucuggaag cugguuucac augguggcuu agauuuuucc aucuuuguau cuagcaccau      60

uugaaaucag uguuuuagga g                                                 81


<210>  1903
<211>  71
<212>  RNA
<213>  Homo sapiens

<400>  1903
gcgacuguaa acauccucga cuggaagcug ugaagccaca gaugggcuuu cagucggaug      60

uuugcagcug c                                                            71


<210>  1904
<211>  70
<212>  RNA

<213>  Homo sapiens

<400>  1904
guuguuguaa acauccccga cuggaagcug uaagacacag cuaagcuuuc agucagaugu      60

uugcugcuac                                                              70


<210>  1905
<211>  92
<212>  RNA
<213>  Homo sapiens

<400>  1905
gggcagucuu ugcuacugua aacauccuug acuggaagcu guaagguguu cagaggagcu      60

uucagucgga uguuuacagc ggcaggcugc ca                                     92


<210>  1906
<211>  88
<212>  RNA
<213>  Mus musculus

<400>  1906
uguuaaauca ggaauuguaa acaauuccua ggcaaugugu auaauguugg uaagucauuc      60

cuagaaauug uucacaaugc cuguaaca                                          88


<210>  1907
<211>  83
<212>  RNA
<213>  Rattus norvegicus

<400>  1907
aaucaggaau uguaaacaau uccuaggcaa uguguauaau guugguaagu cauuccuaga      60

aauuguucac aaugccugua aca                                               83


<210>  1908
<211>  68
<212>  RNA
<213>  Homo sapiens

<400>  1908
ccuuugcuuu aacauggggg uaccugcugu gugaaacaaa aguaagugcu uccauguuuc      60

aguggagg                                                                68


<210>  1909
<211>  85
<212>  RNA
<213>  Homo sapiens

<400>  1909
cucaggcugu gacccuccag agggaaguac uuucuguugu cugagagaaa agaaagugcu      60

ucccuuugga cuguuucggu uugag                                             85


<210>  1910
<211>  61
<212>  RNA
<213>  Homo sapiens

<400>  1910
cccucuacag ggaagcgcuu ucuguugucu gaaagaaaag aaagugcuuc cuuuuagagg      60

g                                                                      61


<210>  1911
<211>  87
<212>  RNA
<213>  Homo sapiens

<400>  1911
ucucaggcug ucguccucua gagggaagca cuuucuguug ucugaaagaa aagaaagugc      60

uuccuuuuag aggguuaccg uuugaga                                          87


<210>  1912
<211>  87
<212>  RNA
<213>  Homo sapiens

<400>  1912
ucucaagcug ugagucuaca aagggaagcc cuuucuguug ucuaaaagaa aagaaagugc      60

uucucuuugg ugguuacgg uuugaga                                           87


<210>  1913
<211>  87
<212>  RNA
<213>  Homo sapiens

<400>  1913
ucuccugcug ugacccucaa gauggaagca guuucuguug ucugaaagga aagaaagugc      60

uuccuuuuug aggguuacug uuugaga                                          87


<210>  1914
<211>  82
<212>  RNA
<213>  Mus musculus

<400>  1914
ccuauguagc ggccaucaaa guggaggccc ucucuugagc cugaaugaga aagugcuucc      60

acuuugugug ccacugcaug gg                                               82


<210>  1915
<211>  84
<212>  RNA
<213>  Mus musculus

<400>  1915
uuccauauag ccauacucaa aauggaggcc cuaucuaagc uuuuaagugg aaagugcuuc      60

ccuuuugugu guugccaugu ggag                                             84


<210>  1916
<211>  69
<212>  RNA
<213>  Mus musculus

<400>  1916
ggugagacuc aaaugugggg cacacuucug gacuguacau agaaagugcu acuacuuuug      60

agucucucc                                                          69

<210>  1917
<211>  82
<212>  RNA
<213>  Rattus norvegicus

<400>  1917
ccgguguagu agccaucaaa guggaggccc ucucuugggc ccgagcuaga aagugcuucc      60

acuuugugug ccacugcaug gg                                              82

<210>  1918
<211>  68
<212>  RNA
<213>  Xenopus laevis

<400>  1918
agcacccaaa acggggcucu cucuugugac ugaauuauga ugagaaagug cuuucuguuu      60

ugggcguu                                                              68

<210>  1919
<211>  71
<212>  RNA
<213>  Xenopus laevis

<400>  1919
cacgucggcc uaacuggagc ccugucucau ugcagcugug aguaagugcu cucuaguucg      60

guugcugagu g                                                          71

<210>  1920
<211>  71
<212>  RNA
<213>  Xenopus tropicalis

<400>  1920
cacgucggcc uaacuggagc ccugucucau ugcagcugug aguaagugcu cucuaguucg      60

guugcugagu g                                                          71

<210>  1921
<211>  101
<212>  RNA
<213>  Danio rerio

<400>  1921
cugcuacugu ugucacuauc ggugcccuca caaaggcacu gacuuggaug cugcaugugg      60

uaagugcuau uuguuggggu aguuucaagu gaccugugcu a                        101

<210>  1922
<211>  82
<212>  RNA
<213>  Danio rerio

<400>  1922
gucacuaucg guacccucac aaaggcacug acuuggaugc uguaauuggu aagugcuauu      60

uguuggggua guuucaagug ac                                              82

<210>  1923
<211>  82
<212>  RNA
<213>  Danio rerio

<400>  1923
gucacuaucg guacccucac aaaggcacug acuuggaugc uguaauuggu aagugcuauu      60

uguuggggua guuucaagug ac                                               82


<210>  1924
<211>  77
<212>  RNA
<213>  Danio rerio

<400>  1924
cuaucgguac ccucacaaaa acacugacuu ugaugcugua uuugguaagu gcuauuuguu      60

gggguaguuu caaguga                                                     77


<210>  1925
<211>  132
<212>  RNA
<213>  Danio rerio

<400>  1925
ggacaagguu uuaaucugcu acuauuguca cuaucgguac ccucacaaag gcacugacuu      60

ggaugcugua auugguaagu gcuauuuguu gggguaguuu caagugaccu gugcuacaag     120

agaauguugu cu                                                         132


<210>  1926
<211>  132
<212>  RNA
<213>  Danio rerio

<400>  1926
ggacaagguu uuaaucugcu acuauuguca cuaucgguac ccucacaaag gcacugacuu      60

ggaugcugua auugguaagu gcuauuuguu gggguaguuu caagugaccu gugcuacaag     120

agaauguugu cu                                                         132


<210>  1927
<211>  132
<212>  RNA
<213>  Danio rerio

<400>  1927
ggacaagguu uuaaucugcu acuauuguca cuaucgguac ccucacaaag gcacugacuu      60

ggaugcugua auugguaagu gcuauuuguu gggguaguuu caagugaccu gugcuacaag     120

agaauguugu cu                                                         132


<210>  1928
<211>  132
<212>  RNA
<213>  Danio rerio

<400>  1928
ggacaagguu uuaaucugcu acuauuguca cuaucgguac ccucacaaag gcacugacuu      60

ggaugcugua auugguaagu gcuauuuguu gggguaguuu caagugaccu gugcuacaag     120

agaauguugu cu                                                         132


<210>  1929
<211>  132
<212>  RNA
<213>  Danio rerio

<400>  1929
ggacaagguu uuaaucugcu acuauuguca cuaucgguac ccucacaaag gcacugacuu      60

ggaugcugua auugguaagu gcuauuuguu gggguaguuu caagugaccu gugcuacaag     120

agaauguugu cu                                                         132


<210>  1930
<211>  132
<212>  RNA
<213>  Danio rerio

<400>  1930
ggacaagguu uuaaucugcu acuauuguca cuaucgguac ccucacaaag gcacugacuu      60

ggaugcugua auugguaagu gcuauuuguu gggguaguuu caagugaccu gugcuacaag     120

agaauguugu cu                                                         132


<210>  1931
<211>  132
<212>  RNA
<213>  Danio rerio

<400>  1931
ggacaagguu uuaaucugcu acuauuguca cuaucgguac ccucacaaag gcacugacuu      60

ggaugcugua auugguaagu gcuauuuguu gggguaguuu caagugaccu gugcuacaag     120

agaauguugu cu                                                         132


<210>  1932
<211>  82
<212>  RNA
<213>  Danio rerio

<400>  1932
gucucuaucg guacccucac aaaagcacug acuuugaugc uguauuuggu aagugcuauu      60

uguuggggua guuucaagug ac                                               82


<210>  1933
<211>  82
<212>  RNA
<213>  Danio rerio

<400>  1933
gucucuaucg guacccucac aaaagcacug acuuugaugc uguauuuggu aagugcuauu      60

uguuggggua guuucaagug ac                                               82

<210>  1934
<211>  82
<212>  RNA
<213>  Danio rerio

<400>  1934
gucucuaucg guacccucac aaaagcacug acuuugaugc uguauuuggu aagugcuauu      60

uguuggggua guuucaagug ac                                                82


<210>  1935
<211>  82
<212>  RNA
<213>  Danio rerio

<400>  1935
gucucuaucg guacccucac aaaagcacug acuuugaugc uguauuuggu aagugcuauu      60

uguuggggua guuucaagug ac                                                82


<210>  1936
<211>  82
<212>  RNA
<213>  Danio rerio

<400>  1936
gucucuaucg guacccucac aaaagcacug acuuugaugc uguauuuggu aagugcuauu      60

uguuggggua guuucaagug ac                                                82


<210>  1937
<211>  82
<212>  RNA
<213>  Danio rerio

<400>  1937
gucucuaucg guacccucac aaaagcacug acuuugaugc uguauuuggu aagugcuauu      60

uguuggggua guuucaagug ac                                                82


<210>  1938
<211>  94
<212>  RNA
<213>  Danio rerio

<400>  1938
caccauuguc ucuaucggua cccucacaaa agcacugacu uugaugcugu auuugguaag      60

ugcuauuugu ugggguaguu uuaagugaca ugug                                    94


<210>  1939
<211>  94
<212>  RNA
<213>  Danio rerio

<400>  1939
caccauuguc ucuaucggua cccucacaaa agcacugacu uugaugcugu auuugguaag      60

ugcuauuugu ugggguaguu uuaagugaca ugug                                    94


<210>  1940

<211>  132
<212>  RNA
<213>  Danio rerio

<400>  1940
ggacaagguu uuaaucugcu gcuauuguca cuaucgguac ccucacaaag gcacugacuu      60

ggaugcugua auugguaagu gcuauuuguu gggguaguuu caagugaccu gugcuacaag     120

agaauguugu cu                                                         132


<210>  1941
<211>  132
<212>  RNA
<213>  Danio rerio

<400>  1941
ggacaagguu uuaaucugcu gcuauuguca cuaucgguac ccucacaaag gcacugacuu      60

ggaugcugua auugguaagu gcuauuuguu gggguaguuu caagugaccu gugcuacaag     120

agaauguugu cu                                                         132


<210>  1942
<211>  130
<212>  RNA
<213>  Danio rerio

<400>  1942
gacaagauuu uagucugcca ccauugucuc uaucgguacc cucacaaaag cacugacuuu      60

gaugcuguaa uugguaagug cuauuuguug ggguaguuuc aagugaccug ugcuacaaga     120

gaauguuguc                                                            130


<210>  1943
<211>  108
<212>  RNA
<213>  Danio rerio

<400>  1943
uuuaaauaaa cuauuaccgu caggcuuuca caagccagcc ucaaaguuug uucucaaacu      60

uuaagaaucu aguugguaag ugcuauuugu ugggguaguu ucaaguga                   108


<210>  1944
<211>  91
<212>  RNA
<213>  Danio rerio

<400>  1944
auugauagaa accaguugag gucaacucua acuuuagcau cuuucuuuua agcaaaguag      60

aaagugcuau caaguugggg uagauguuug c                                     91


<210>  1945
<211>  81
<212>  RNA
<213>  Danio rerio

<400>  1945
caguugaggu caacucuaac uuuagcaucu uucuuuuaag caaaguagaa agugcuauca      60

aguuggggua gauguuugcu g                                                81

<210>  1946
<211>  81
<212>  RNA
<213>  Danio rerio

<400>  1946
caguugaggu caacucuaac uuuagcaucu uucuuuuaag caaaguagaa agugcuauca      60

aguuggggua gauguuugcu g                                                81

<210>  1947
<211>  81
<212>  RNA
<213>  Danio rerio

<400>  1947
caguugaggu caacucuaac uuuagcaucu uucuuuuaag caaaguagaa agugcuauca      60

aguuggggua gauguuugcu g                                                81

<210>  1948
<211>  81
<212>  RNA
<213>  Danio rerio

<400>  1948
caguugacau caacucuaac uuuagcaucu uucuuuuaag caaaguaaaa agugcuauca      60

aguuggggua gauguuugcu g                                                81

<210>  1949
<211>  81
<212>  RNA
<213>  Danio rerio

<400>  1949
caguugacgu caacucuaac uuuagcaucu uucuuuuaag cagaguagaa agugcuauca      60

aguuggggua gauguuugcu g                                                81

<210>  1950
<211>  81
<212>  RNA
<213>  Danio rerio

<400>  1950
caguugacgu caacucuaac uuuagcaucu uucuuuuaag cagaguagaa agugcuauca      60

aguuggggua gauguuugcu g                                                81

<210>  1951
<211>  81
<212>  RNA
<213>  Danio rerio

<400>  1951
caguugacgu caacucuaac uuuagcaucu uucuuuuaag cagaguagaa agugcuauca      60

aguuggggua gauguuugcu g                                                81

<210>  1952
<211>  81
<212>  RNA
<213>  Danio rerio

<400>  1952
caguugacgu caacucuaac uuuagcaucu uucuuuuaag cagaguagaa agugcuauca      60

aguuggggua gauguuugcu g                                                81


<210>  1953
<211>  81
<212>  RNA
<213>  Danio rerio

<400>  1953
caguugacau caacucuaac uuuagcaucu uucuuuuaag caaaguagaa agugcuauca      60

aguuggggua gauguuugcu g                                                81


<210>  1954
<211>  81
<212>  RNA
<213>  Danio rerio

<400>  1954
caguugacau caacucuaac uuuagcaucu uucuuuuaag caaaguagaa agugcuauca      60

aguuggggua gauguuugcu g                                                81


<210>  1955
<211>  81
<212>  RNA
<213>  Danio rerio

<400>  1955
caguugacau caacucuaac uuuagcaucu uucuuuuaag caaaguagaa agugcuauca      60

aguuggggua gauguuugcu g                                                81


<210>  1956
<211>  81
<212>  RNA
<213>  Danio rerio

<400>  1956
caguugacau caacucuaac uuuagcaucu uucuuuuaag caaaguagaa agugcuauca      60

aguuggggua gauguuugcu g                                                81


<210>  1957
<211>  81
<212>  RNA
<213>  Danio rerio

<400>  1957
caguugacau caacucuaac uuuagcaucu uucuuuuaag caaaguagaa agugcuauca      60

aguuggggua gauguuugcu g                                                81


<210>  1958

<211>  81
<212>  RNA
<213>  Danio rerio

<400>  1958
caguugacau caacucuaac uuuagcaucu uucuuuuaag caaaguagaa agugcuauca      60

aguuggggua gauguuugcu g                                                81


<210>  1959
<211>  81
<212>  RNA
<213>  Danio rerio

<400>  1959
caguugacau caacucuaac uuuagcaucu uucuuuuaag caaaguagaa agugcuauca      60

aguuggggua gauguuugcu g                                                81


<210>  1960
<211>  81
<212>  RNA
<213>  Danio rerio

<400>  1960
caguugacau caacucuaac uuuagcaucu uucuuuuaag caaaguagaa agugcuauca      60

aguuggggua gauguuugcu g                                                81


<210>  1961
<211>  81
<212>  RNA
<213>  Danio rerio

<400>  1961
caguugacau caacucuaac uuuagcaucu uucuuuuaag caaaguagaa agugcuauca      60

aguuggggua gauguuugcu g                                                81


<210>  1962
<211>  81
<212>  RNA
<213>  Danio rerio

<400>  1962
caguugacau caacucuaac uuuagcaucu uucuuuuaag cagagcagaa agugcuauca      60

aguuggggua gauguuugcu g                                                81


<210>  1963
<211>  81
<212>  RNA
<213>  Danio rerio

<400>  1963
caguugaggu caacucuaac uuuagcaucu uucuuuuaag cauaguagaa agugcuauca      60

aguuggggua gauguuuacu g                                                81


<210>  1964
<211>  81
<212>  RNA

<213>  Danio rerio

<400>  1964
caguugaggu caacucuaac uuuagcaucu uucuuuuaag cauaguagaa agugcuauca      60

aguuggggua gauguuuacu g                                                81


<210>  1965
<211>  81
<212>  RNA
<213>  Danio rerio

<400>  1965
caguugaggu caacucuaac uuuagcaucu uucuuuuaag caaaguagaa agugcuauca      60

aguuggggua gauguuuguu g                                                81


<210>  1966
<211>  81
<212>  RNA
<213>  Danio rerio

<400>  1966
caguugaggu caacucuaac uuuagcaucu uucuuuuaag caaaguagaa agugcuauca      60

aguuggggua gauguuuguu g                                                81


<210>  1967
<211>  96
<212>  RNA
<213>  Danio rerio

<400>  1967
uucaaucuaa agcacauuaa gaucacuuca aacaggagca uugauuuguc cuuuguucau      60

aagugcuucu cuuuggggua guuuuaauaa ggacag                                96


<210>  1968
<211>  79
<212>  RNA
<213>  Danio rerio

<400>  1968
   cacauuaaga ucacuucaaa caggagcauu gauuuguccu uuguucauaa gugcuucucu      60
   uugggguagu uuuaauaag                                                  79


<210>  1969
<211>  79
<212>  RNA
<213>  Danio rerio

<400>  1969
   cacauuaaga ucacuucaaa caggagcauu gauuuguccu uuguucauaa gugcuucucu      60
   uugggguagu uuuaauaag                                                  79


<210>  1970
<211>  79
<212>  RNA
<213>  Danio rerio

<400>  1970
cacauuaaga ucacuucaaa caggagcauu gauuuguccu uuguucauaa gugcuucucu      60

uugggguagu uuuaauaag                                                  79


<210>  1971
<211>  79
<212>  RNA
<213>  Danio rerio

<400>  1971
cacauuaaga ucacuucaaa caggagcauu gauuuguccu uuguucauaa gugcuucucu      60

uugggguagu uuuaauaag                                                  79


<210>  1972
<211>  82
<212>  RNA
<213>  Danio rerio

<400>  1972
auuaagauca cuacaaacag gagcauugau uuguccuuug uucauaagug cuucucuuug      60

ggguaguuuu aauaaggaca ag                                              82


<210>  1973
<211>  73
<212>  RNA
<213>  Danio rerio

<400>  1973
auuaagauca cuucaaacag gagcauugau uuguccuuug uucauaagug cuucucuuug      60

ggguaguuuu aau                                                        73


<210>  1974
<211>  82
<212>  RNA
<213>  Danio rerio

<400>  1974
auuaagauca cuucaaacag gagcauugau uuguccuuug uucauaagug cuucucuuug      60

ggguaguuuu aauaaggaca ag                                              82


<210>  1975
<211>  82
<212>  RNA
<213>  Danio rerio

<400>  1975
auuaagauca cuucaaacag gagcauugau uuguccuuug uucauaagug cuucucuuug      60

ggguaguuuu aauaaggaca ag                                              82


<210>  1976
<211>  82
<212>  RNA
<213>  Danio rerio

<400>  1976
auuaagauca cuucaaacag gagcauugau uuguccuuug uucauaagug cuucucuuug      60

ggguaguuuu aauaaggaca ag                                              82

<210>  1977
<211>  82
<212>  RNA
<213>  Danio rerio

<400>  1977
auuaagauca cuucaaacag gagcauugau uuguccuuug uucauaagug cuucucuuug      60

ggguaguuuu aauaaggaca ag                                              82

<210>  1978
<211>  82
<212>  RNA
<213>  Danio rerio

<400>  1978
auuaagauca cuucaaacag gagcauugau uuguccuuug uucauaagug cuucucuuug      60

ggguaguuuu aauaaggaca ag                                              82

<210>  1979
<211>  82
<212>  RNA
<213>  Danio rerio

<400>  1979
auuaagauca cuucaaacag gagcauugau uuguccuuug uucauaagug cuucucuuug      60

ggguaguuuu aauaaggaca ag                                              82

<210>  1980
<211>  82
<212>  RNA
<213>  Danio rerio

<400>  1980
auuaagauca cuucaaacag gagcauugau uuguccuuug uucauaagug cuucucuuug      60

ggguaguuuu aauaaggaca ag                                              82

<210>  1981
<211>  82
<212>  RNA
<213>  Danio rerio

<400>  1981
auuaagauca cuucaaacag gagcauugau uuguccuuug uucauaagug cuucucuuug      60

ggguaguuuu aauaaggaca ag                                              82

<210>  1982
<211>  82
<212>  RNA
<213>  Danio rerio

<400>  1982
auuaagauca cuucaaacag gagcauugau uuguccuuug uucauaagug cuucucuuug      60

ggguaguuuu aauaaggaca ag                                              82

<210>  1983
<211>  82
<212>  RNA
<213>  Danio rerio

<400>  1983
auuaagauca cuucaaacag gagcauugau uuguccuuug uucauaagug cuucucuuug      60

ggguaguuuu aauaaggaca ag                                               82


<210>  1984
<211>  82
<212>  RNA
<213>  Danio rerio

<400>  1984
auuaagauca cuucaaacag gagcauugau uuguccuuug uucauaagug cuucucuuug      60

ggguaguuuu aauaaggaca ag                                               82


<210>  1985
<211>  82
<212>  RNA
<213>  Danio rerio

<400>  1985
auuaagauca cuucaaacag gagcauugau uuguccuuug uucauaagug cuucucuuug      60

ggguaguuuu aauaaggaca ag                                               82


<210>  1986
<211>  82
<212>  RNA
<213>  Danio rerio

<400>  1986
auuaagauca cuucaaacag gagcauugau uuguccuuug uucauaagug cuucucuuug      60

ggguaguuuu aauaaggaca ag                                               82


<210>  1987
<211>  82
<212>  RNA
<213>  Danio rerio

<400>  1987
auuaagauca cuucaaacag gagcauugau uuguccuuug uucauaagug cuucucuuug      60

ggguaguuuu aauaaggaca ag                                               82


<210>  1988
<211>  82
<212>  RNA
<213>  Danio rerio

<400>  1988
gucucuaucg guacccucac aaaagcacug acuuugaugc uguauuuggu aagugcuauu      60

uguuggcgua guuucaagug ac                                               82

<210>  1989
<211>  82
<212>  RNA
<213>  Danio rerio

<400>  1989
gucucuaucg guacccucac aaaagcacug acuuugaugc uguauuuggu aagugcuauu      60

uguuggcgua guuucaagug ac                                               82


<210>  1990
<211>  82
<212>  RNA
<213>  Danio rerio

<400>  1990
gucucuaucg guacccucac aaaagcacug acuuugaugc uguauuuggu aagugcuauu      60

uguuggcgua guuucaagug ac                                               82


<210>  1991
<211>  81
<212>  RNA
<213>  Danio rerio

<400>  1991
caguugagau caacucuaac uuuagcaucu uucuuuuaag caaaguagaa agugcuauca      60

aauuggggua gauguuugcu g                                                81


<210>  1992
<211>  78
<212>  RNA
<213>  Homo sapiens

<400>  1992
gccgcaggug cucugacgag guugcacuac ugugcucuga gaagcagugc aaugauauug      60

ucaaagcauc ugggacca                                                    78


<210>  1993
<211>  115
<212>  RNA
<213>  Homo sapiens

<400>  1993
ucuguuuauc accagauccu agaacccuau caauauuguc ucugcugugu aaauaguucu      60

gaguagugca auauugcuua uaggguuuug guguuuggaa agaacaaugg gcagg          115


<210>  1994
<211>  88
<212>  RNA
<213>  Mus musculus

<400>  1994
ggaagacagu gcaauuaaaa gggggaaaaa aguaccuggg auguucugag aauuucauuu      60

uucuuguuau ugccacuccu gcuuggaa                                         88


<210>  1995
<211>  124

<212>  RNA
<213>  Danio rerio

<400>  1995
uugcaggucg uuuaagaauu aauccuaauu cuugggaccc uaucaguauu gccucugcug      60

uccacugugu ucagaguagu gcaauauugc uaauaggguu uuagguuuua gguuguaccu     120

ucag                                                                  124


<210>  1996
<211>  116
<212>  RNA
<213>  Danio rerio

<400>  1996
gauaaaugca ucuugcaggc gagacccuau caauauugcc ucugcuuuuc ucacuguuua      60

uggaguagug caauauugcu uauagggucu ugacuuuaag gcuaaucugc aauauc         116


<210>  1997
<211>  73
<212>  RNA
<213>  Homo sapiens

<400>  1997
ccuggcacug agaacugaau uccauaggcu gugagcucua gcaaugcccu guggacucag      60

uucuggugcc cgg                                                         73


<210>  1998
<211>  95
<212>  RNA
<213>  Mus musculus

<400>  1998
ccucguugac uccgaagggc ugcagcagca auucauguuu uggaguauug ccaagguuca      60

aaacaugaag cgcugcaaca ccccuucgug gggaa                                 95


<210>  1999
<211>  95
<212>  RNA
<213>  Rattus norvegicus

<400>  1999
ccucgcugac uccgaaggga ugcagcagca auucauguuu uggaguauug ccaagguuca      60

aaacaugaag cgcugcaaca ccccuucgug ggaaa                                 95


<210>  2000
<211>  83
<212>  RNA
<213>  Danio rerio

<400>  2000
ugccugacag aagcagcaca ucaauauugg cagcugcccu cucucugggu ugccaguaug      60

guuugugcug cucccgucag aca                                              83


<210>  2001
<211>  78

<212>  RNA
<213>  Danio rerio

<400>  2001
gaauguacua aagcagcaca uaaauacugg aggugauugu gguguuaucc aguauugcug      60

uucugcugua guaagacc                                                    78


<210>  2002
<211>  95
<212>  RNA
<213>  Homo sapiens

<400>  2002
cggccggccc uggguccauc uuccaguaca guguuggaug gucuaauugu gaagcuccua      60

acacugucug guaaagaugg cucccgggug gguuc                                 95


<210>  2003
<211>  72
<212>  RNA
<213>  Mus musculus

<400>  2003
ggguccaucu uccagugcag uguuggaugg uugaaguaug aagcuccuaa cacugucugg      60

uaaagauggc cc                                                          72


<210>  2004
<211>  94
<212>  RNA
<213>  Rattus norvegicus

<400>  2004
ggcugacucu gaguccaucu uccagugcag uguuggaugg uugaaguacg aagcuccuaa      60

cacugucugg uaaagauggc ccccggguca guuc                                  94


<210>  2005
<211>  64
<212>  RNA
<213>  Monodelphis domestica

<400>  2005
uggggccauc uuccaguaca gugguggaug gugaagcuuc uaacacuguc ugguaaagau      60

gccc                                                                   64


<210>  2006
<211>  83
<212>  RNA
<213>  Danio rerio

<400>  2006
gucucuaggg uacaucuuac cugacagugc uuggcuguuc acugauguuc uaacacuguc      60

ugguaacgau gcacucuggu gac                                              83


<210>  2007
<211>  77
<212>  RNA
<213>  Homo sapiens

<400>  2007
ccucucaggg aagcuuuuug cucgaauuau guuucugauc cgaauauaag acgaacaaaa      60

gguuugucug agggcag                                                     77


<210>  2008
<211>  80
<212>  RNA
<213>  Danio rerio

<400>  2008
ucuacugaca gagaagcuuu uuguuugugu uauguuuauu uucaaaugua agacgaacaa      60

aaaguuuuuc uguuaguagg                                                  80


<210>  2009
<211>  110
<212>  RNA
<213>  Homo sapiens

<400>  2009
ugaacaucca ggucuggggc augaaccugg cauacaaugu agauuucugu guucguuagg      60

caacagcuac auugucugcu ggguuucagg cuaccuggaa acauguucuc                110


<210>  2010
<211>  95
<212>  RNA
<213>  Mus musculus

<400>  2010
auccaggucu ggggcaugaa ccuggcauac aauguagauu ucuguguuug uuaggcaaca      60

gcuacauugu cugcuggguu ucaggcuacc uggaa                                 95


<210>  2011
<211>  109
<212>  RNA
<213>  Rattus norvegicus

<400>  2011
ugaauaucca ggucuggggc augaaccugg cauacaaugu agauuucugu guuuguuagg      60

caacagcuac auugucugcu ggguuucagg cuaccuggaa gcauuucuc                 109


<210>  2012
<211>  110
<212>  RNA
<213>  Macaca mulatta

<400>  2012
ugaacaucca ggucuggggc augaaccugg cauacaaugu agauuucugu guucguuagg      60

caacagcuac auugucugcu ggguuucagg cuaccuggaa acauguucuc                110


<210>  2013
<211>  110
<212>  RNA
<213>  Gorilla gorilla

<400>  2013

ugaacaucca ggucuggggc augaaccugg cauacaaugu agauuucugu guucguuagg 60

caacagcuac auugucugcu ggguuucagg cuaccuggaa acauguucuc 110

<210> 2014
<211> 110
<212> RNA
<213> Pan paniscus

<400> 2014
ugaacaucca ggucuggggc augaaccugg cauacaaugu agauuucugu guucguuagg 60

caacagcuac auugucugcu ggguuucagg cuaccuggaa acauguucuc 110

<210> 2015
<211> 110
<212> RNA
<213> Pongo pygmaeus

<400> 2015
ugaacaucca ggucuggggc augaaccugg cauacaaugu agauuucugu guucguuagg 60

caacagcuac auugucugcu ggguuucagg cuaccuggaa acauguucuc 110

<210> 2016
<211> 74
<212> RNA
<213> Monodelphis domestica

<400> 2016
gcaugaaccu ggcauacaau guagaauucu guguuuauua aguaacagcu acauugucug 60

cuggguuuca ggcu 74

<210> 2017
<211> 99
<212> RNA
<213> Gallus gallus

<400> 2017
gacuguccag guuuggggca ugaaccuggc auacaaugua gauuucugug uuuguuaagc 60

aacagcuaca uugucugcug gguuuccagc ugccuggaa 99

<210> 2018
<211> 93
<212> RNA
<213> Xenopus tropicalis

<400> 2018
acccagacuu uggcgugcac cuggcauaca auguagaaaa cuguguuugc aaagcaacag 60

cuacauuguc ugcuggguuu caugcugaau gga 93

<210> 2019
<211> 110
<212> RNA
<213> Bos taurus

<400> 2019
ccaacaucca ggucuagggc augaaccugg cauacaaugu agauuucugu guuuguugag 60

caacagcuac auugucugcu ggguuucagg cuaccuggaa acacguucuu 110

<210> 2020
<211> 93
<212> RNA
<213> Danio rerio

<400> 2020
gucgugaacc uggcauacaa uguagauuuc ugugugguac uaucuacagc uacauugucu 60

gcuggguuuc aggccagcag aauaauucug cuc 93

<210> 2021
<211> 77
<212> RNA
<213> Fugu rubripes

<400> 2021
ccugaaccug gcauacaaug uagauuucug ugugcuugga uucuacagcu acauugucug 60

cuggguuucc ggauagc 77

<210> 2022
<211> 69
<212> RNA
<213> Tetraodon nigroviridis

<400> 2022
ccugaaccug gcauacaaug uagauuucug ugugcuugga uucuacagcu acauugucug 60

cuggguuuc 69

<210> 2023
<211> 65
<212> RNA
<213> Mus musculus

<400> 2023
cucgaauccu uggaaccuag gugugaaugc ugcuucagug caacacaccu guucaaggau 60

ucaaa 65

<210> 2024
<211> 79
<212> RNA
<213> Homo sapiens

<400> 2024
agcaaagaag uguguugccc ucuaggaaau guguguugcu cugauguaau uagguugaca 60

uacguuuccc ugguagcca 79

<210> 2025
<211> 97
<212> RNA
<213> Homo sapiens

<400> 2025
ugaccugaau cagguaggca guguauuguu agcuggcugc uugggucaag ucagcagcca 60

caacuacccu gccacuugcu ucuggauaaa uucuucu 97

<210>  2026
<211>  63
<212>  RNA
<213>  Mus musculus

<400>  2026
cgugcagcgg ggcgucaucc gucagcucac auagugacgc aggcagugcg accuggcucg      60

cac                                                                    63


<210>  2027
<211>  85
<212>  RNA
<213>  Homo sapiens

<400>  2027
aggccucucu cuccguguuc acagcggacc uugauuuaaa uguccauaca auuaaggcac      60

gcggugaaug ccaagaaugg ggcug                                            85


<210>  2028
<211>  109
<212>  RNA
<213>  Homo sapiens

<400>  2028
aucaagauua gaggcucugc ucuccguguu cacagcggac cuugauuuaa ugucauacaa      60

uuaaggcacg cggugaaugc caagagcgga gccuacggcu gcacuugaa                 109


<210>  2029
<211>  87
<212>  RNA
<213>  Homo sapiens

<400>  2029
ugagggcccc ucugcguguu cacagcggac cuugauuuaa ugucuauaca auuaaggcac      60

gcggugaaug ccaagagagg cgccucc                                          87


<210>  2030
<211>  85
<212>  RNA
<213>  Mus musculus

<400>  2030
aggccucucu cuccguguuc acagcggacc uugauuuaaa uguccauaca auuaaggcac      60

gcggugaaug ccaagaaugg ggcug                                            85


<210>  2031
<211>  109
<212>  RNA
<213>  Mus musculus

<400>  2031
aucaagauca gagacucugc ucuccguguu cacagcggac cuugauuuaa ugucauacaa      60

uuaaggcacg cggugaaugc caagagcgga gccuacggcu gcacuugaa                 109


<210>  2032

<211>  68
<212>  RNA
<213>  Mus musculus

<400>  2032
cucugcgugu ucacagcgga ccuugauuua augucuauac aauuaaggca cgcggugaau      60

gccaagag                                                                68


<210>  2033
<211>  85
<212>  RNA
<213>  Rattus norvegicus

<400>  2033
aggccucucu cuccguguuc acagcggacc uugauuuaaa uguccauaca auuaaggcac      60

gcggugaaug ccaagaaugg ggcug                                             85


<210>  2034
<211>  109
<212>  RNA
<213>  Rattus norvegicus

<400>  2034
aucaagauca gagacucugc ucuccguguu cacagcggac cuugauuuaa ugucauacaa      60

uuaaggcacg cggugaaugc caagagcgga gccuacggcu gcacuugaa                  109


<210>  2035
<211>  87
<212>  RNA
<213>  Rattus norvegicus

<400>  2035
ugagggcccc ucugcguguu cacagcggac cuugauuuaa ugucuauaca auuaaggcac      60

gcggugaaug ccaagagagg cgccucc                                           87


<210>  2036
<211>  82
<212>  RNA
<213>  Danio rerio

<400>  2036
ggcucucgcu guacguguuc acaguggacc uugauuuauu guauuucaau uaaggcacgc      60

ggugaaugcc aacagcacag cc                                                82


<210>  2037
<211>  83
<212>  RNA
<213>  Danio rerio

<400>  2037
ccugcuuuuc uucguguuca cagcggaccu ugauuuaaau guccauacaa uuaaggcacg      60

cggugaaugc caagagagau ggc                                               83


<210>  2038
<211>  117
<212>  RNA

<213>  Danio rerio

<400>  2038
ggcucugugg gauuucagac ucuggcuuuc cguguucaca gcggaccuug auuuaauguc      60

uuacaauuaa ggcacgcggu gaaugccaag agcggagccu uuuaacauca gcaggcc        117


<210>  2039
<211>  83
<212>  RNA
<213>  Danio rerio

<400>  2039
gguuuuugcu cuuuguguuc acaguggacc uugauuuaau uucaauacaa uuaaggcacg      60

cggugaaugc caagagagaa gcc                                              83


<210>  2040
<211>  129
<212>  RNA
<213>  Danio rerio

<400>  2040
ggguuuugcu cgugcguucu uuuugaguuc ucgcucugcg uguucacagc ggaccuugau      60

uuaaugucca uacaauuaag gcacgcggug aaugccaaga gaagaaucuc uccagcaacg     120

aguuugcgc                                                             129


<210>  2041
<211>  113
<212>  RNA
<213>  Danio rerio

<400>  2041
ggguggugac acaggcccgc cacucugcgu guucacggcg gaccuugauu uaauauccau      60

acaauuaagg cacgcgguga augccaagag aggggucuua aaacgacaaa ccc            113


<210>  2042
<211>  77
<212>  RNA
<213>  Fugu rubripes

<400>  2042
gguuguguc u cuccguguuc acagcggacc uugauuuaau gucuuacaau uaaggcacgc      60

ggugaaugcc aagagau                                                     77


<210>  2043
<211>  78
<212>  RNA
<213>  Fugu rubripes

<400>  2043
cuggucucuc cucguguuca cagcggaccu ugauuuaaau guccauacaa uuaaggcacg      60

cggugaaugc caagagag                                                    78


<210>  2044
<211>  83
<212>  RNA

<213>  Fugu rubripes

<400>  2044
gguuugagcu cuuuguguuc acaguggacc uugauuuaau uucaauacaa uuaaggcacg      60

cggugaaugc caagagagaa gcc                                              83


<210>  2045
<211>  67
<212>  RNA
<213>  Tetraodon nigroviridis

<400>  2045
cucuccgugu ucacagcgga ccuugauuua augucuuaca auuaaggcac gcggugaaug      60

ccaagag                                                                67


<210>  2046
<211>  75
<212>  RNA
<213>  Tetraodon nigroviridis

<400>  2046
gccucuccuc guguucacag cggaccuuga uuuaaauguc cauacaauua aggcacgcgg      60

ugaaugccaa gagag                                                       75


<210>  2047
<211>  80
<212>  RNA
<213>  Tetraodon nigroviridis

<400>  2047
gguuugagcu cuuuguguuc acaguggacc uugauuuaau uucaauacaa uuaaggcacg      60

cggugaaugc caagagagaa                                                  80


<210>  2048
<211>  98
<212>  RNA
<213>  Homo sapiens

<400>  2048
agaaugggca aaugaacagu aaauuuggag gccuggggcc cucccugcug cuggagaagu      60

guuugcacgg gugggccuug ucuuugaaag gaggugga                              98


<210>  2049
<211>  85
<212>  RNA
<213>  Homo sapiens

<400>  2049
ucucaagcug ugacugcaaa gggaagcccu uucuguuguc ugaaagaaga gaaagcgcuu      60

cccuuugcug gauuacgguu ugaga                                            85


<210>  2050
<211>  87
<212>  RNA
<213>  Homo sapiens

<400>  2050
ucucaagcug ugggucugca aagggaagcc cuuucuguug ucuaaaagaa gagaaagcgc      60

uucccuuugc uggauuacgg uuugaga                                          87


<210>  2051
<211>  90
<212>  RNA
<213>  Homo sapiens

<400>  2051
ucccaugcug ugacccucua gaggaagcac uuucuguuug uugucugaga aaaaacaaag      60

ugcuucccuu uagaguguua ccguuuggga                                       90


<210>  2052
<211>  85
<212>  RNA
<213>  Homo sapiens

<400>  2052
cucaggcugu gacacucuag agggaagcgc uuucuguugu cugaaagaaa ggaaagugca      60

uccuuuuaga guguuacugu uugag                                            85


<210>  2053
<211>  87
<212>  RNA
<213>  Homo sapiens

<400>  2053
ucucaggcug ugucccucua cagggaagcg cuuucuguug ucugaaagaa aggaaagugc      60

auccuuuuag aguguuacug uuugaga                                          87


<210>  2054
<211>  81
<212>  RNA
<213>  Homo sapiens

<400>  2054
caugcuguga cccucuagag ggaagcgcuu ucuguugucu gaaagaaaag aaagugcauc      60

cuuuuagagg uuuacuguuu g                                                81


<210>  2055
<211>  87
<212>  RNA
<213>  Homo sapiens

<400>  2055
ucucagccug ugacccucua gagggaagcg cuuucuguug ucugaaagaa aagaaagugc      60

aucuuuuuag aggauuacag uuugaga                                          87


<210>  2056
<211>  87
<212>  RNA
<213>  Homo sapiens

<400>  2056
ucucaggcug ugucccucua gagggaagcg cuuucuguug ucugaaagaa aagaaaaugg      60

uucccuuuag aguguuacgc uuugaga                                          87

<210>  2057
<211>  21
<212>  RNA
<213>  Homo sapiens

<400>  2057
uaguagaccg uauagcguac g                                                21

<210>  2058
<211>  23
<212>  RNA
<213>  Homo sapiens

<400>  2058
aucaacagac auuaauuggg cgc                                              23

<210>  2059
<211>  23
<212>  RNA
<213>  Homo sapiens

<400>  2059
aaugacacga ucacucccgu uga                                              23

<210>  2060
<211>  22
<212>  RNA
<213>  Homo sapiens

<400>  2060
acccuaucaa uauugucucu gc                                               22

<210>  2061
<211>  21
<212>  RNA
<213>  Homo sapiens

<400>  2061
ugaaggucua cugugugcca g                                                21

<210>  2062
<211>  22
<212>  RNA
<213>  Homo sapiens

<400>  2062
caugccuuga guguaggacc gu                                               22

<210>  2063
<211>  22
<212>  RNA
<213>  Homo sapiens

<400>  2063
ugugacagau ugauaacuga aa                                               22

<210>  2064

<211>  22
<212>  RNA
<213>  Homo sapiens

<400>  2064
aucagcaaac auuuauugug ug                                                22


<210>  2065
<211>  22
<212>  RNA
<213>  Homo sapiens

<400>  2065
caaaacuggc aauuacuuuu gc                                                22


<210>  2066
<211>  22
<212>  RNA
<213>  Homo sapiens

<400>  2066
caagaaccuc aguugcuuuu gu                                                22


<210>  2067
<211>  22
<212>  RNA
<213>  Homo sapiens

<400>  2067
caaaaaccac aguuucuuuu gc                                                22


<210>  2068
<211>  22
<212>  RNA
<213>  Homo sapiens

<400>  2068
ugucuuacuc ccucaggcac au                                                22


<210>  2069
<211>  21
<212>  RNA
<213>  Homo sapiens

<400>  2069
gcgacccaua cuugguuuca g                                                 21


<210>  2070
<211>  21
<212>  RNA
<213>  Homo sapiens

<400>  2070
aacaggugac ugguuagaca a                                                 21


<210>  2071
<211>  21
<212>  RNA
<213>  Homo sapiens

<400>  2071

aaaacgguga gauuuuguuu u                                                21

<210>  2072
<211>  19
<212>  RNA
<213>  Homo sapiens

<400>  2072
ugagcugcug uaccaaaau                                                   19

<210>  2073
<211>  22
<212>  RNA
<213>  Homo sapiens

<400>  2073
caaaguuuaa gauccuugaa gu                                               22

<210>  2074
<211>  22
<212>  RNA
<213>  Homo sapiens

<400>  2074
ggcuggcucg cgaugucugu uu                                               22

<210>  2075
<211>  22
<212>  RNA
<213>  Homo sapiens

<400>  2075
gaaaacagca auuaccuuug ca                                               22

<210>  2076
<211>  21
<212>  RNA
<213>  Homo sapiens

<400>  2076
ugaguuggcc aucugaguga g                                                21

<210>  2077
<211>  23
<212>  RNA
<213>  Homo sapiens

<400>  2077
auucauuugg uauaaaccgc gau                                              23

<210>  2078
<211>  22
<212>  RNA
<213>  Homo sapiens

<400>  2078
uuaugguuug ccugggacug ag                                               22

<210>  2079
<211>  24

<212>  RNA
<213>  Homo sapiens

<400>  2079
ucagaacaaa ugccgguucc caga                                              24


<210>  2080
<211>  20
<212>  RNA
<213>  Homo sapiens

<400>  2080
agaccauggg uucucauugu                                                   20


<210>  2081
<211>  25
<212>  RNA
<213>  Homo sapiens

<400>  2081
aggcaccagc caggcauugc ucagc                                             25


<210>  2082
<211>  21
<212>  RNA
<213>  Homo sapiens

<400>  2082
gaagugugcc gugguguguc u                                                 21


<210>  2083
<211>  20
<212>  RNA
<213>  Homo sapiens

<400>  2083
guugugucag uuuaucaaac                                                   20


<210>  2084
<211>  23
<212>  RNA
<213>  Homo sapiens

<400>  2084
acuuacagac aagagccuug cuc                                               23


<210>  2085
<211>  22
<212>  RNA
<213>  Homo sapiens

<400>  2085
uggucuagga uuguuggagg ag                                                22


<210>  2086
<211>  19
<212>  RNA
<213>  Homo sapiens

<400>  2086
aggcugcgga auucaggac                                                    19

<210>  2087
<211>  23
<212>  RNA
<213>  Homo sapiens

<400>  2087
uaaaucccau ggugccuucu ccu                                              23


<210>  2088
<211>  21
<212>  RNA
<213>  Homo sapiens

<400>  2088
aaacuacuga aaaucaaaga u                                                21


<210>  2089
<211>  20
<212>  RNA
<213>  Homo sapiens

<400>  2089
aggguguuuc ucucaucucu                                                  20


<210>  2090
<211>  21
<212>  RNA
<213>  Homo sapiens

<400>  2090
ugagcuaaau gugugcuggg a                                                21


<210>  2091
<211>  23
<212>  RNA
<213>  Homo sapiens

<400>  2091
   gcgaggaccc cucggggucu gac                                               23


<210>  2092
<211>  23
<212>  RNA
<213>  Homo sapiens

<400>  2092
gaacgccugu ucuugccagg ugg                                              23


<210>  2093
<211>  21
<212>  RNA
<213>  Homo sapiens

<400>  2093
uccgagccug ggucucccuc u                                                21


<210>  2094
<211>  22
<212>  RNA

<213>  Homo sapiens

<400>  2094
acucaaaacc cuucagugac uu                                              22


<210>  2095
<211>  22
<212>  RNA
<213>  Homo sapiens

<400>  2095
agacuuccca uuugaaggug gc                                              22


<210>  2096
<211>  23
<212>  RNA
<213>  Homo sapiens

<400>  2096
aaacucuacu uguccuucug agu                                             23


<210>  2097
<211>  21
<212>  RNA
<213>  Homo sapiens

<400>  2097
ggcuagcaac agcgcuuacc u                                               21


<210>  2098
<211>  22
<212>  RNA
<213>  Homo sapiens

<400>  2098
uaguaccagu accuuguguu ca                                              22


<210>  2099
<211>  22
<212>  RNA
<213>  Homo sapiens

<400>  2099
agggggaaag uucuauaguc cu                                              22


<210>  2100
<211>  22
<212>  RNA
<213>  Homo sapiens

<400>  2100
gugagucucu aagaaaagag ga                                              22


<210>  2101
<211>  22
<212>  RNA
<213>  Homo sapiens

<400>  2101
aguauucugu accagggaag gu                                              22

<210>  2102
<211>  21
<212>  RNA
<213>  Homo sapiens

<400>  2102
agaccuggcc cagaccucag c                                                21


<210>  2103
<211>  22
<212>  RNA
<213>  Homo sapiens

<400>  2103
aaccagcacc ccaacuuugg ac                                               22


<210>  2104
<211>  24
<212>  RNA
<213>  Homo sapiens

<400>  2104
acugggggcu uucgggcucu gcgu                                             24


<210>  2105
<211>  19
<212>  RNA
<213>  Homo sapiens

<400>  2105
aagcagcugc cucugaggc                                                   19


<210>  2106
<211>  21
<212>  RNA
<213>  Homo sapiens

<400>  2106
guggcugcac ucacuuccuu c                                                21


<210>  2107
<211>  19
<212>  RNA
<213>  Homo sapiens

<400>  2107
aagugugcag ggcacuggu                                                   19


<210>  2108
<211>  21
<212>  RNA
<213>  Homo sapiens

<400>  2108
aggaggcagc gcucucagga c                                                21


<210>  2109
<211>  22
<212>  RNA
<213>  Homo sapiens

<400>  2109
uuuaggauaa gcuugacuuu ug                                                22

<210>  2110
<211>  21
<212>  RNA
<213>  Homo sapiens

<400>  2110
uugaaacaau cucuacugaa c                                                 21

<210>  2111
<211>  22
<212>  RNA
<213>  Homo sapiens

<400>  2111
uggugggccg cagaacaugu gc                                                22

<210>  2112
<211>  23
<212>  RNA
<213>  Homo sapiens

<400>  2112
ggcagguucu cacccucucu agg                                               23

<210>  2113
<211>  24
<212>  RNA
<213>  Homo sapiens

<400>  2113
   ugccuggguc ucuggccugc gcgu                                             24

<210>  2114
<211>  22
<212>  RNA
<213>  Homo sapiens

<400>  2114
ugagaccucu ggguucugag cu                                                22

<210>  2115
<211>  21
<212>  RNA
<213>  Mus musculus

<400>  2115
uaguagaccg uauagcguac g                                                 21

<210>  2116
<211>  21
<212>  RNA
<213>  Rattus norvegicus

<400>  2116
uaguagaccg uauagcguac g                                                 21

<210>  2117
<211>  23
<212>  RNA
<213>  Mus musculus

<400>  2117
aucaacagac auuaauuggg cgc                                              23


<210>  2118
<211>  23
<212>  RNA
<213>  Xenopus tropicalis

<400>  2118
aaugacacga ucacucccgu uga                                              23


<210>  2119
<211>  22
<212>  RNA
<213>  Bos taurus

<400>  2119
caugccuuga guguaggacc gu                                               22


<210>  2120
<211>  22
<212>  RNA
<213>  Mus musculus

<400>  2120
ugugacagau ugauaacuga aa                                               22


<210>  2121
<211>  22
<212>  RNA
<213>  Rattus norvegicus

<400>  2121
ugugacagau ugauaacuga aa                                               22


<210>  2122
<211>  21
<212>  RNA
<213>  Mus musculus

<400>  2122
gcgacccaua cuugguuuca g                                                21


<210>  2123
<211>  23
<212>  RNA
<213>  Mus musculus

<400>  2123
aaugacacca cauauauggc agc                                              23


<210>  2124
<211>  24
<212>  RNA
<213>  Danio rerio

<400>  2124
aaugacacgu uuucucccgg aucg                                              24


<210>  2125
<211>  22
<212>  RNA
<213>  Homo sapiens

<400>  2125
ugaaggucua cugugugcca gg                                                22


<210>  2126
<211>  22
<212>  RNA
<213>  Homo sapiens

<400>  2126
cgucuuaccc agcaguguuu gg                                                22


<210>  2127
<211>  22
<212>  RNA
<213>  Mus musculus

<400>  2127
cgucuuaccc agcaguguuu gg                                                22


<210>  2128
<211>  21
<212>  RNA
<213>  Homo sapiens

<400>  2128
gcgacccacu cuugguuucc a                                                 21


<210>  2129
<211>  23
<212>  RNA
<213>  Mus musculus

<400>  2129
auugugucaa uaugcgauga ugu                                               23


<210>  2130
<211>  22
<212>  RNA
<213>  Homo sapiens

<400>  2130
acucaaaaug ggggcgcuuu cc                                                22


<210>  2131
<211>  22
<212>  RNA
<213>  Mus musculus

<400>  2131
acucaaaaug gaggcccuau cu                                                22


<210>  2132

<211>  22
<212>  RNA
<213>  Homo sapiens

<400>  2132
uggugggcac agaaucugga cu                                              22


<210>  2133
<211>  96
<212>  RNA
<213>  Homo sapiens

<400>  2133
ugguacuugg agagauagua gaccguauag cguacgcuuu aucugugacg uauguaacac      60

gguccacuaa cccucaguau caaauccauc cccgag                                96


<210>  2134
<211>  85
<212>  RNA
<213>  Homo sapiens

<400>  2134
gcacauugua ggccucauua aauguuuguu gaaugaaaaa augaaucauc aacagacauu      60

aauugggcgc cugcucugug aucuc                                            85


<210>  2135
<211>  87
<212>  RNA
<213>  Homo sapiens

<400>  2135
gaaagcgcuu uggaaugaca cgaucacucc cguugagugg gcacccgaga agccaucggg      60

aaugucgugu ccgcccagug cucuuuc                                          87


<210>  2136
<211>  115
<212>  RNA
<213>  Homo sapiens

<400>  2136
ucuguuuauc accagauccu agaacccuau caauauuguc ucugcugugu aaauaguucu      60

gaguagugca auauugcuua uaggguuuug guguuuggaa agaacaaugg gcagg          115


<210>  2137
<211>  89
<212>  RNA
<213>  Homo sapiens

<400>  2137
cuggccucca gggcuuugua caugguaggc uuucauucau ucguuugcac auucggugaa      60

ggucuacugu gugccaggcc cugugccag                                        89


<210>  2138
<211>  91
<212>  RNA
<213>  Homo sapiens

<400>  2138
cgacuugcuu ucucuccucc augccuugag uguaggaccg uuggcaucuu aauuacccuc      60

ccacacccaa ggcuugcaaa aaagcgagcc u                                     91


<210>  2139
<211>  97
<212>  RNA
<213>  Homo sapiens

<400>  2139
cagaucucag acaucucggg gaucaucaug ucacgagaua ccagugugca cuugugacag      60

auugauaacu gaaaggucug ggagccacuc aucuuca                               97


<210>  2140
<211>  106
<212>  RNA
<213>  Homo sapiens

<400>  2140
cccagccugg cacauuagua ggccucagua aauguuuauu agaugaauaa augaaugacu      60

caucagcaaa cauuuauugu gugccugcua aagugagcuc cacagg                    106


<210>  2141
<211>  97
<212>  RNA
<213>  Homo sapiens

<400>  2141
ugcagggagg uauuaaguug gugcaaaagu aauugugauu uuugccauua aaaguaacga      60

caaaacuggc aauuacuuuu gcaccaaacc ugguauu                               97


<210>  2142
<211>  97
<212>  RNA
<213>  Homo sapiens

<400>  2142
ugugaugugu auuagguuug ugcaaaagua auuggguuuu uuugccguua aaaguaaugg      60

caaaacuggc aauuacuuuu gcaccaaacu aauauaa                               97


<210>  2143
<211>  97
<212>  RNA
<213>  Homo sapiens

<400>  2143
ccuagaaugu uauuaggucg gugcaaaagu aauugcgagu uuuaccauua cuuucaaugg      60

caaaacuggc aauuacuuuu gcaccaacgu aauacuu                               97


<210>  2144
<211>  97
<212>  RNA
<213>  Homo sapiens

<400>  2144
cagacuauau auuuagguug gcgcaaaagu aauugugguu uuggccuuua uuuucaaugg      60

caagaaccuc aguugcuuuu gugccaaccu aauacuu 97

<210> 2145
<211> 97
<212> RNA
<213> Homo sapiens

<400> 2145
aaacaaguua uauuagguug gugcaaaagu aauugugguu uuugccugua aaaguaaugg 60

caaaaaccac aguuucuuuu gcaccagacu aauaaag 97

<210> 2146
<211> 97
<212> RNA
<213> Homo sapiens

<400> 2146
gagagggaag auuuagguug gugcaaaagu aauugugguu uuugccauug aaaguaaugg 60

caaaaaccac aguuucuuuu gcaccaaccu aauaaaa 97

<210> 2147
<211> 97
<212> RNA
<213> Homo sapiens

<400> 2147
ugaugcuuug cuggcuggug cagugccuga gggaguaaga gcccuguugu uguaagauag 60

ugucuuacuc ccucaggcac aucuccaaca agucucu 97

<210> 2148
<211> 97
<212> RNA
<213> Homo sapiens

<400> 2148
ugaugcuuug cuggcuggug cagugccuga gggaguaaga gcccuguugu ugucagauag 60

ugucuuacuc ccucaggcac aucuccagcg agucucu 97

<210> 2149
<211> 96
<212> RNA
<213> Homo sapiens

<400> 2149
agaugugcuc uccuggccca ugaaaucaag cgugggugag accuggugca gaacgggaag 60

gcgacccaua cuugguuuca gaggcuguga gaauaa 96

<210> 2150
<211> 96
<212> RNA
<213> Homo sapiens

<400> 2150
aaccauucaa auauaccaca guuuguuuaa ccuuuugccu guugguugaa gaugccuuuc 60

aacaggugac ugguuagaca aacuguggua uauaca 96

<210> 2151
<211> 68
<212> RNA
<213> Homo sapiens

<400> 2151
cuucaauuuu auuuuaaaac ggugagauuu uguuuugucu gagaaaaucu cgcuguuuua 60

gacugagg 68


<210> 2152
<211> 94
<212> RNA
<213> Homo sapiens

<400> 2152
gugugugugu gugugugugg uuauuuuggu auaguagcuc uagacucuau uauaguuucc 60

ugagcugcug uaccaaaaua ccacaaacgg gcug 94


<210> 2153
<211> 97
<212> RNA
<213> Homo sapiens

<400> 2153
cuucauccac caguccucca ggaacaucaa ggaucuuaaa cuuugccaga gcuacaaagg 60

caaaguuuaa gauccuugaa guuccugggg gaaccau 97


<210> 2154
<211> 97
<212> RNA
<213> Homo sapiens

<400> 2154
ccaguggcgc aauggauaac gcgucugacu acggaucaga agauucuagg uucgacuccu 60

ggcuggcucg cgaugucugu uuugccacac uugaccc 97


<210> 2155
<211> 97
<212> RNA
<213> Homo sapiens

<400> 2155
cuagauaagu uauuaggugg gugcaaaggu aauugcaguu uuucccauua uuuuaauugc 60

gaaaacagca auuaccuuug caccaaccug auggagu 97


<210> 2156
<211> 96
<212> RNA
<213> Homo sapiens

<400> 2156
ccucaguaag accaagcuca gugugccauu uccuugucug uagccauguc uaugggcucu 60

ugaguuggcc aucugaguga gggccugcuu auucua 96

<210>  2157
<211>  98
<212>  RNA
<213>  Homo sapiens

<400>  2157
cauauuaggu uaaugcaaaa guaaucgcgg uuugugccag augacgauuu gaauuaauaa      60

auucauuugg uauaaaccgc gauuauuuuu gcaucaac                              98


<210>  2158
<211>  97
<212>  RNA
<213>  Homo sapiens

<400>  2158
uagggugacc agccauuaug guuugccugg gacugaggaa uuugcuggga uaugucaguu      60

ccaggccaac caggcugguu ggucucccug aagcaac                               97


<210>  2159
<211>  99
<212>  RNA
<213>  Homo sapiens

<400>  2159
uccagccugu gcccagcagc cccugagaac cacgucugcu cugagcuggg uacugccugu      60

ucagaacaaa ugccgguucc cagacgcugc cagcuggcc                             99


<210>  2160
<211>  95
<212>  RNA
<213>  Homo sapiens

<400>  2160
ucuuaucaau gagguagacc auggguucuc auuguaauag uguagaaugu ugguuaacug      60

uggacucccu ggcucugucu caaaucuacu gauuc                                 95


<210>  2161
<211>  100
<212>  RNA
<213>  Homo sapiens

<400>  2161
cccccagaau cugucaggca ccagccaggc auugcucagc ccguuucccu cugggggagc      60

aaggaguggu gcuggguuug ucucugcugg gguuucuccu                           100


<210>  2162
<211>  96
<212>  RNA
<213>  Homo sapiens

<400>  2162
acggaagccu gcacgcauuu aacaccagca cgcucaaugu agucuuguaa ggaacagguu      60

gaagugugcc gugguguguc uggaggaagc gccugu                               96


<210>  2163
<211>  95

<212>  RNA
<213>  Homo sapiens

<400>  2163
aaagacaugc uguccacagu guguuugaua agcugacaug ggacagggau ucuuuucacu      60

guugugucag uuuaucaaac ccauacuugg augac                                 95


<210>  2164
<211>  98
<212>  RNA
<213>  Homo sapiens

<400>  2164
aagucacgug cuguggcucc agcuucauag gaaggcucuu gucugucagg caguggaguu      60

acuuacagac aagagccuug cucaggccag cccugccc                              98


<210>  2165
<211>  79
<212>  RNA
<213>  Homo sapiens

<400>  2165
ugcaugaguu cgucuugguc uaggauuguu ggaggaguca gaaaaacuac cccagggauc      60

cugaaguccu uugggugga                                                   79


<210>  2166
<211>  94
<212>  RNA
<213>  Homo sapiens

<400>  2166
agagcaucgu gcuugaccuu ccacgcucuc guguccacua gcaggcaggu uuucugacac      60

aggcugcgga auucaggaca gugcaucaug gaga                                  94


<210>  2167
<211>  83
<212>  RNA
<213>  Homo sapiens

<400>  2167
gcccuagcuu gguucuaaau cccauggugc cuucuccuug ggaaaaacag agaaggcacu      60

augagauuua gaaucaaguu agg                                              83


<210>  2168
<211>  96
<212>  RNA
<213>  Homo sapiens

<400>  2168
uguauccuug guuuuuagua guuuuacuau gaugaggugu gccauccacc ccaucauagu      60

aaacuacuga aaaucaaaga uacaagugcc ugacca                                96


<210>  2169
<211>  95
<212>  RNA
<213>  Homo sapiens

<400>  2169
ugcucggcug uuccuagggu guuucucuca ucucuggucu auaauggguu aaauaguaga      60

gaugagggca acacccuagg aacagcagag gaacc                                95


<210>  2170
<211>  96
<212>  RNA
<213>  Homo sapiens

<400>  2170
ucuauuuguc uuaggugagc uaaaugugug cugggacaca uuugagccaa augucccagc      60

acacauuuag cucacauaag aaaaauggac ucuagu                               96


<210>  2171
<211>  67
<212>  RNA
<213>  Homo sapiens

<400>  2171
aaaaugguga gagcguugag gggaguucca gacggagaug cgaggacccc ucggggucug      60

acccaca                                                               67


<210>  2172
<211>  90
<212>  RNA
<213>  Homo sapiens

<400>  2172
ucuaagaaac gcaguggucu cugaagccug caggggcagg ccagcccugc acugaacgcc      60

uguucuugcc agguggcaga agguugcugc                                      90


<210>  2173
<211>  96
<212>  RNA
<213>  Homo sapiens

<400>  2173
cucgggaggg gcgggagggg gguccccggu gcucggaucu cgagggugcu uauuguucgg      60

uccgagccug ggucucccuc uuccccccaa cccccc                                96


<210>  2174
<211>  97
<212>  RNA
<213>  Homo sapiens

<400>  2174
uuagguaauu ccuccacuca aaacccuuca gugacuucca ugacaugaaa uaggaaguca      60

uuggaggguu ugagcagagg aaugaccugu uuuaaaa                              97


<210>  2175
<211>  97
<212>  RNA
<213>  Homo sapiens

<400>  2175

caucauaagg agccuagacu ucccauuuga agguggccau uuccuaccac cuucaaaugg 60

uaaguccagg cuccuucuga uucaauaaau gaggagc 97

<210> 2176
<211> 98
<212> RNA
<213> Homo sapiens

<400> 2176
cucuuguuca cagccaaacu cuacuugucc uucugagugu aauuacguac augcaguagc 60

ucaggagaca agcagguuua cccuguggau gagucuga 98

<210> 2177
<211> 96
<212> RNA
<213> Homo sapiens

<400> 2177
   uagauugagg aaggggcuga gugguaggcg gugcugcugu gcucugauga agacccaugu 60
   ggcuagcaac agcgcuuacc uuuugucucu ggg ucc 96

<210> 2178
<211> 97
<212> RNA
<213> Homo sapiens

<400> 2178
aaugcuguuu caagguagua ccaguaccuu guguucagug gaaccaaggu aaacacaagg 60

uauugguauu accuugagau agcauuacac cuaagug 97

<210> 2179
<211> 85
<212> RNA
<213> Homo sapiens

<400> 2179
aggguagagg gaugaggggg aaaguucuau aguccuguaa uuagaucuca ggacuauaga 60

acuuuccccc ucaucccucu gcccu 85

<210> 2180
<211> 97
<212> RNA
<213> Homo sapiens

<400> 2180
uacuuauuac ugguagugag ucucuaagaa aagaggaggu gguuguuuuc cuccucuuuu 60

cuuugagacu cacuaccaau aauaagaaau acuacua 97

<210> 2181
<211> 97
<212> RNA
<213> Homo sapiens

<400> 2181
aacuuaacau caugcuaccu cuuuguauca uauuuuguua uucuggucac agaaugaccu 60

aguauucugu accagggaag guaguucuua acuauau 97

<210> 2182
<211> 75
<212> RNA
<213> Homo sapiens

<400> 2182
guggggagcc ugguuagacc uggcccagac cucagcuaca caagcugaug gacugaguca 60

ggggccacac ucucc 75

<210> 2183
<211> 97
<212> RNA
<213> Homo sapiens

<400> 2183
aaacccacac cacugcauuu uggccaucga ggguuggggc uuggugucau gccccaagau 60

aaccagcacc ccaacuuugg acagcaugga uuagucu 97

<210> 2184
<211> 99
<212> RNA
<213> Homo sapiens

<400> 2184
uggcuaaggu guuggcucgg gcuccccacu gcaguuaccc uccccucggc guuacugagc 60

acugggggcu uucgggcucu gcgucugcac agauacuuc 99

<210> 2185
<211> 94
<212> RNA
<213> Homo sapiens

<400> 2185
gaucaggagu cugccagugg agucagcaca ccugcuuuuc accugugauc ccaggagagg 60

aagcagcugc cucugaggcc ucaggcucag uggc 94

<210> 2186
<211> 96
<212> RNA
<213> Homo sapiens

<400> 2186
aggaaguguu ggccuguggc ugcacucacu uccuucagcc ccaggaagcc uuggucgggg 60

gcaggaggga gggucaggca gggcuggggg ccugac 96

<210> 2187
<211> 94
<212> RNA
<213> Homo sapiens

<400> 2187
aucacagaca ccuccaagug ugcagggcac uggugggggc cggggcaggc ccagcgaaag 60

ugcaggaccu ggcacuuagu cggaagugag ggug 94

<210>  2188
<211>  96
<212>  RNA
<213>  Homo sapiens

<400>  2188
cagugcuggg gucucaggag gcagcgcucu caggacguca ccaccauggc cugggcucug      60

cuccuccuca cccuccucac ucagggcaca ggugau                                96


<210>  2189
<211>  97
<212>  RNA
<213>  Homo sapiens

<400>  2189
aaucuaucac ugcuuuuuag gauaagcuug acuuuuguuc aaauaaaaau gcaaaaggaa      60

aguguauccu aaaaggcaau gacaguuuaa uguguuu                               97


<210>  2190
<211>  96
<212>  RNA
<213>  Homo sapiens

<400>  2190
uucauuccuu caguguugaa acaaucucua cugaaccagc uucaaacaag uucacuggag      60

uuuguuucaa uauugcaaga augauaagau ggaagc                                96


<210>  2191
<211>  81
<212>  RNA
<213>  Homo sapiens

<400>  2191
ggguaagugg aaagauggug ggccgcagaa caugugcuga guucgugcca uaugucugcu      60

gaccaucacc uuuagaagcc c                                                81


<210>  2192
<211>  98
<212>  RNA
<213>  Homo sapiens

<400>  2192
guguaguaga gcuaggagga gaggguccug gagaagcgug gaccgguccg gguggguucc      60

ggcagguucu cacccucucu aggccccauu cuccucug                              98


<210>  2193
<211>  89
<212>  RNA
<213>  Homo sapiens

<400>  2193
   ggagaggcug ugcugugggg caggcgcagg ccugagcccu gguuucgggc ugccuggguc      60
   ucuggccugc gcgugacuuu gggguggcu                                       89


<210>  2194

<211>  118
<212>  RNA
<213>  Homo sapiens

<400>  2194
gccuuggugc ugauuccugg gcucugaccu gagaccucug gguucugagc ugugauguug      60

cucucgagcu gggaucuccg gggucuuggu ucagggccgg ggccucuggg uuccaagc      118


<210>  2195
<211>  82
<212>  RNA
<213>  Mus musculus

<400>  2195
ugguacuugg agagauagua gaccguauag cguacgcuuu aucugugacg uauguaacac      60

gguccacuaa cccucaguau ca                                               82


<210>  2196
<211>  76
<212>  RNA
<213>  Rattus norvegicus

<400>  2196
cuuggagaga uaguagaccg uauagcguac gcuuuaucug ugacguaugu aacacggucc      60

acuaacccuc aguauc                                                      76


<210>  2197
<211>  76
<212>  RNA
<213>  Mus musculus

<400>  2197
cauuguaggc cucauuaaau guuuguugaa ugaaaaaaug aaucaucaac agacauuaau      60

ugggcgccug cucugu                                                      76


<210>  2198
<211>  77
<212>  RNA
<213>  Xenopus tropicalis

<400>  2198
gccuuggaau gacacgauca cucccguuga gccgaaacuc acagcgccau cgggaauauc      60

gugucgcucc aaagcuc                                                     77


<210>  2199
<211>  86
<212>  RNA
<213>  Bos taurus

<400>  2199
gacuugcuuu cucucuuaca ugccuugagu guaggaccgu uggcaucuua auuacccucc      60

cacacccaag gcuugcagga gagcca                                           86


<210>  2200
<211>  85
<212>  RNA

<213>  Mus musculus

<400>  2200
ggaucucaga cgucucgggg aucaucaugu cacgagauac cacugugccc uugugacaga      60

uugauaacug aaaggucugg gagcc                                            85


<210>  2201
<211>  79
<212>  RNA
<213>  Rattus norvegicus

<400>  2201
ucucagacgu cucggggauc aucaugucac gagauaccac ugcgcacuug ugacagauug      60

auaacugaaa ggucuggga                                                   79


<210>  2202
<211>  98
<212>  RNA
<213>  Mus musculus

<400>  2202
gugcucuugu ggcccaugaa aucaagcuug ggugagaccu ggugcagaac aggaaggcga      60

cccauacuug guuucagugg cugcaagaau gacugcau                              98


<210>  2203
<211>  107
<212>  RNA
<213>  Mus musculus

<400>  2203
acugcugcag uggcagcuug guugucauau gugugaugac acuuucuaaa gucuuccaga      60

augacaccac auauauggca gcuaaacugu uacauggaac aacaagu                   107


<210>  2204
<211>  90
<212>  RNA
<213>  Danio rerio

<400>  2204
ugaugcugau cuggaaugac acguuuucuc ccggaucgcc agaaauaugu uucgccaccg      60

ggaacuucgu gucagccaag auuggcauca                                       90


<210>  2205
<211>  68
<212>  RNA
<213>  Homo sapiens

<400>  2205
cccucgucuu acccagcagu guuugggugc gguugggagu cucuaauacu gccggguaau      60

gauggagg                                                               68


<210>  2206
<211>  69
<212>  RNA
<213>  Mus musculus

<400>  2206
cccucgucuu acccagcagu guuugggugc ugguugggag ucucuaauac ugccggguaa      60

ugauggagg                                                              69


<210>  2207
<211>  96
<212>  RNA
<213>  Homo sapiens

<400>  2207
ggggacugcc gggugacccu ggaaauccag aguggguggg gccagucuga ccguuucuag      60

gcgacccacu cuugguuucc aggguugccc uggaaa                                96


<210>  2208
<211>  96
<212>  RNA
<213>  Mus musculus

<400>  2208
ugauauuaug ccaugacauu gugucaauau gcgaugaugu guugugaugg cacagcguca      60

ucacguggug acgcaacauc augacguaag acauca                                96


<210>  2209
<211>  69
<212>  RNA
<213>  Homo sapiens

<400>  2209
   gggauacuca aaaugggggc gcuuuccuuu uugucuguac ugggaagugc uucgauuuug      60
   ggguguccc                                                             69


<210>  2210
<211>  84
<212>  RNA
<213>  Mus musculus

<400>  2210
uuccauauag ccauacucaa aauggaggcc cuaucuaagc uuuuaagugg aaagugcuuc      60

ccuuuugugu guugccaugu ggag                                             84


<210>  2211
<211>  84
<212>  RNA
<213>  Homo sapiens

<400>  2211
acgucaggga aaggauucug cugucggucc cacuccaaag uucacagaau gggugguggg      60

cacagaaucu ggacucugcu ugug                                             84


<210>  2212
<211>  21
<212>  RNA
<213>  Xenopus tropicalis

<400>  2212
uggaauguaa agaaguaugu a                                                21

<210>  2213
<211>  23
<212>  RNA
<213>  Mus musculus

<400>  2213
uggaagacua gugauuuugu ugu                                              23


<210>  2214
<211>  23
<212>  RNA
<213>  Mus musculus

<400>  2214
uacccuguag aaccgaauuu gug                                              23


<210>  2215
<211>  23
<212>  RNA
<213>  Rattus norvegicus

<400>  2215
uacccuguag auccgaauuu gug                                              23


<210>  2216
<211>  23
<212>  RNA
<213>  Xenopus laevis

<400>  2216
uaaggugcau cuagugcagu uag                                              23


<210>  2217
<211>  21
<212>  RNA
<213>  Rattus norvegicus

<400>  2217
caaagugcuc auagugcagg u                                                21


<210>  2218
<211>  23
<212>  RNA
<213>  Xenopus laevis

<400>  2218
caaagugcuc auagugcagg uag                                              23


<210>  2219
<211>  22
<212>  RNA
<213>  Xenopus tropicalis

<400>  2219
uggcucaguu cagcaggaac ag                                               22


<210>  2220
<211>  21
<212>  RNA

<213>  Xenopus tropicalis

<400>  2220
uucaaguaau ccaggauagg c                                                21


<210>  2221
<211>  21
<212>  RNA
<213>  Mus musculus

<400>  2221
uucacagugg cuaaguuccg c                                                21


<210>  2222
<211>  23
<212>  RNA
<213>  Mus musculus

<400>  2222
uagcaccauu ugaaaucagu guu                                              23


<210>  2223
<211>  22
<212>  RNA
<213>  Mus musculus

<400>  2223
uguaaacauc cucgacugga ag                                               22


<210>  2224
<211>  22
<212>  RNA
<213>  Rattus norvegicus

<400>  2224
uguaaacauc cuacacucag cu                                               22


<210>  2225
<211>  23
<212>  RNA
<213>  Mus musculus

<400>  2225
uaagugcuuc cauguuuugg uga                                              23


<210>  2226
<211>  22
<212>  RNA
<213>  Xenopus tropicalis

<400>  2226
cagugcaaua uuaaaagggc au                                               22


<210>  2227
<211>  22
<212>  RNA
<213>  Xenopus tropicalis

<400>  2227
uugguccccu ucaaccagcu gc                                               22

<210>  2228
<211>  23
<212>  RNA
<213>  Monodelphis domestica

<400>  2228
uguaguguuu ccuacuuuau gga                                                23


<210>  2229
<211>  23
<212>  RNA
<213>  Danio rerio

<400>  2229
uguaguguuu ccuacuuuau gga                                                23


<210>  2230
<211>  22
<212>  RNA
<213>  Mus musculus

<400>  2230
ugagaacuga auuccauagg cu                                                 22


<210>  2231
<211>  21
<212>  RNA
<213>  Fugu rubripes

<400>  2231
ugguucuaga cuugccaacu a                                                  21


<210>  2232
<211>  21
<212>  RNA
<213>  Tetraodon nigroviridis

<400>  2232
ugguucuaga cuugccaacu a                                                  21


<210>  2233
<211>  22
<212>  RNA
<213>  Mus musculus

<400>  2233
aacuggccua caaaguccca gu                                                 22


<210>  2234
<211>  22
<212>  RNA
<213>  Rattus norvegicus

<400>  2234
uagguaguuu cguguuguug gg                                                 22


<210>  2235
<211>  20
<212>  RNA
<213>  Gallus gallus

<400>  2235
uacaguaguc ugcacauugg                                                    20

<210>  2236
<211>  22
<212>  RNA
<213>  Mus musculus

<400>  2236
agagguauag cgcaugggaa ga                                                 22

<210>  2237
<211>  22
<212>  RNA
<213>  Tetraodon nigroviridis

<400>  2237
uucccuuugu uauccuaugc cu                                                 22

<210>  2238
<211>  22
<212>  RNA
<213>  Xenopus tropicalis

<400>  2238
uccuucauuc caccggaucc ug                                                 22

<210>  2239
<211>  22
<212>  RNA
<213>  Mus musculus

<400>  2239
auaagacgaa caaaagguuu gu                                                 22

<210>  2240
<211>  24
<212>  RNA
<213>  Monodelphis domestica

<400>  2240
agcuacaucu ggcuacuggg ucuc                                               24

<210>  2241
<211>  22
<212>  RNA
<213>  Mus musculus

<400>  2241
uaugugggac gguaaaccgc uu                                                 22

<210>  2242
<211>  22
<212>  RNA
<213>  Mus musculus

<400>  2242
ugguuuaccg ucccacauac au                                                 22

<210>  2243
<211>  22
<212>  RNA
<213>  Rattus norvegicus

<400>  2243
ugguuuaccg ucccacauac au                                                22


<210>  2244
<211>  22
<212>  RNA
<213>  Mus musculus

<400>  2244
gcuuuaacau gggguuaccu gc                                                22


<210>  2245
<211>  21
<212>  RNA
<213>  Mus musculus

<400>  2245
gccccugggc cuauccuaga a                                                 21


<210>  2246
<211>  22
<212>  RNA
<213>  Mus musculus

<400>  2246
uccgucucag uuacuuuaua gc                                                22


<210>  2247
<211>  23
<212>  RNA
<213>  Rattus norvegicus

<400>  2247
uccgucucag uuacuuuaua gcc                                               23


<210>  2248
<211>  23
<212>  RNA
<213>  Mus musculus

<400>  2248
ucucacacag aaaucgcacc cgu                                               23


<210>  2249
<211>  23
<212>  RNA
<213>  Rattus norvegicus

<400>  2249
ucucacacag aaaucgcacc cgu                                               23


<210>  2250
<211>  24
<212>  RNA
<213>  Mus musculus

<400>  2250
aauccuugga accuaggugu gaau                                              24

<210>  2251
<211>  21
<212>  RNA
<213>  Rattus norvegicus

<400>  2251
aucauagagg aacauccacu u                                                 21

<210>  2252
<211>  22
<212>  RNA
<213>  Mus musculus

<400>  2252
cuccugacuc cagguccugu gu                                                22

<210>  2253
<211>  22
<212>  RNA
<213>  Rattus norvegicus

<400>  2253
cuccugacuc cagguccugu gu                                                22

<210>  2254
<211>  23
<212>  RNA
<213>  Mus musculus

<400>  2254
agcucggucu gaggccccuc agu                                               23

<210>  2255
<211>  22
<212>  RNA
<213>  Mus musculus

<400>  2255
uggcagugua uuguuagcug gu                                                22

<210>  2256
<211>  22
<212>  RNA
<213>  Mus musculus

<400>  2256
uaugugccuu uggacuacau cg                                                22

<210>  2257
<211>  21
<212>  RNA
<213>  Mus musculus

<400>  2257
ucacuccucc ccucccgucu u                                                 21

<210>  2258

<211>  22
<212>  RNA
<213>  Mus musculus

<400>  2258
agaggcuggc cgugaugaau uc                                                22


<210>  2259
<211>  22
<212>  RNA
<213>  Rattus norvegicus

<400>  2259
agaggcuggc cgugaugaau uc                                                22


<210>  2260
<211>  21
<212>  RNA
<213>  Mus musculus

<400>  2260
cccagauaau agcacucuca a                                                 21


<210>  2261
<211>  22
<212>  RNA
<213>  Mus musculus

<400>  2261
aauccuuugu cccuggguga aa                                                22


<210>  2262
<211>  84
<212>  RNA
<213>  Xenopus tropicalis

<400>  2262
accuguuugg aguacauacu ucuuuaugua cccauaugaa cauacaaugc uauggaaugu      60

aaagaaguau guauuuuuau cagg                                              84


<210>  2263
<211>  71
<212>  RNA
<213>  Xenopus tropicalis

<400>  2263
ugggagacau acuucuuuau auguccauau ggaagugcca augcuaugga auguaaagaa      60

guauguaucu c                                                            71


<210>  2264
<211>  108
<212>  RNA
<213>  Mus musculus

<400>  2264
uuggauguug gccuaguucu guguggaaga cuagugauuu uguuguuuuu agauaacuaa      60

aacgacaaca aaucacaguc ugccauaugg cacaggccac cucuacag                   108

<210>  2265
<211>  97
<212>  RNA
<213>  Mus musculus

<400>  2265
ggucgggcca gccccguuug gaagacuagu gauuuuguug uugugucucu guauccaaca      60

acaaguccca gucugccaca uggugcuggu cauuuca                               97


<210>  2266
<211>  68
<212>  RNA
<213>  Mus musculus

<400>  2266
uauauacccu guagaaccga auuugugugg uacccacaua gucacagauu cgauucuagg      60

ggaauaua                                                               68


<210>  2267
<211>  110
<212>  RNA
<213>  Rattus norvegicus

<400>  2267
gaccugucug ucuucuguau auacccugua gauccgaauu uguguaagga auuuuguggu      60

cacaaauucg uaucuagggg aauauguagu ugacauaaac acuccgcuca                110


<210>  2268
<211>  79
<212>  RNA
<213>  Xenopus laevis

<400>  2268
ccuuguguua aggugcaucu agugcaguua gugacauagc guagcaucua cugcccuaaa      60

ugcuccuuuu ggcacaggg                                                   79


<210>  2269
<211>  72
<212>  RNA
<213>  Rattus norvegicus

<400>  2269
guagugccaa agugcucaua gugcagguag guuuugcugc acucuacugc agugugagca      60

cuucugguac uc                                                          72


<210>  2270
<211>  74
<212>  RNA
<213>  Xenopus laevis

<400>  2270
gcaguuccaa agugcucaua gugcagguag uuguauucau guucuacugu aauaugggca      60

cuuacaguac ugcu                                                        74


<210>  2271
<211>  67

<212>  RNA
<213>  Xenopus tropicalis

<400>  2271
cucuugugcc uacugaacug auaucaguuc uauuucacac acuggcucag uucagcagga      60

acaggag                                                                67


<210>  2272
<211>  85
<212>  RNA
<213>  Xenopus tropicalis

<400>  2272
ggcugcugcc ugguucaagu aauccaggau aggcuguuuc cucaaagcac ggccuacucu      60

ugauuacuug uuucaggaag uagcu                                            85


<210>  2273
<211>  80
<212>  RNA
<213>  Xenopus tropicalis

<400>  2273
ugggcgcucg cuucaaguaa uccaggauag gcuguaaugu cuccugucag ccuuuucucg      60

auuacuuggg accagagcca                                                  80


<210>  2274
<211>  87
<212>  RNA
<213>  Mus musculus

<400>  2274
uggccugagg agcagggcuu agcugcuugu gagcaagguc cacagcaaag ucguguucac      60

aguggcuaag uuccgccccc uggaccc                                          87


<210>  2275
<211>  71
<212>  RNA
<213>  Mus musculus

<400>  2275
aggaagcugg uuucauaugg ugguuuagau uuaaauagug auugucuagc accauuugaa      60

aucaguguuc u                                                           71


<210>  2276
<211>  81
<212>  RNA
<213>  Mus musculus

<400>  2276
cuucuggaag cugguuucac augguggcuu agauuuuucc aucuuuguau cuagcaccau      60

uugaaaucag uguuuuagga g                                                81


<210>  2277
<211>  71
<212>  RNA
<213>  Mus musculus

<400>  2277
gcgacuguaa acauccucga cuggaagcug ugaagccaca aaugggcuuu cagucggaug      60

uuugcagcug c                                                          71


<210>  2278
<211>  87
<212>  RNA
<213>  Rattus norvegicus

<400>  2278
ccgaguuuca guucauguaa acauccuaca cucagcuguc auacaugagu uggcugggau      60

guggauguuu acgucagcug ucuugga                                         87


<210>  2279
<211>  69
<212>  RNA
<213>  Mus musculus

<400>  2279
ccaccacuua aacgugguug uacuugcuuu agaccuaaga aaguaagugc uuccauguuu      60

uggugaugg                                                             69


<210>  2280
<211>  71
<212>  RNA
<213>  Xenopus tropicalis

<400>  2280
auccguggcc cuuuuucugu uguacuacug gaauuuguaa uuagcagugc aauauuaaaa      60

gggcauuggc u                                                          71


<210>  2281
<211>  86
<212>  RNA
<213>  Xenopus tropicalis

<400>  2281
aaugcuuugc uaaagcuggu aaaauggaac caaaucaacu guugaaugga uuuggucccc      60

uucaaccagc ugcagcugug cauuga                                          86


<210>  2282
<211>  74
<212>  RNA
<213>  Monodelphis domestica

<400>  2282
gcagucaccc auaaaguaga aagcacuacu aacagcaaug uaggguguag uguuuccuac      60

uuuauggaug agug                                                       74


<210>  2283
<211>  91
<212>  RNA
<213>  Danio rerio

<400>  2283

cguacagugc agucauccau aaaguagaaa gcacuacuaa accccucgcc acaguguagu 60

guuuccuacu uuauggauga guguacuguu g 91

<210> 2284
<211> 109
<212> RNA
<213> Mus musculus

<400> 2284
gacugagaga acuuuggcca ccuggcucug agaacugaau uccauaggcu gugagcucua 60

gcagacgccc uagggacuca guucuggugc ccggcugugc uauaccauc 109

<210> 2285
<211> 72
<212> RNA
<213> Fugu rubripes

<400> 2285
uguuuggcaa ugguagaacu cacacuggug agguagaugg auccgguggu ucuagacuug 60

ccaacuacug cu 72

<210> 2286
<211> 67
<212> RNA
<213> Tetraodon nigroviridis

<400> 2286
uguuuggcaa ugguagaacu cacacuggug agguagaugg auccgguggu ucuagacuug 60

ccaacua 67

<210> 2287
<211> 66
<212> RNA
<213> Mus musculus

<400> 2287
gagagcuggg ucuuugcggg caagaugaga gugucaguuc aacuggccua caaaguccca 60

guccuc 66

<210> 2288
<211> 80
<212> RNA
<213> Rattus norvegicus

<400> 2288
cugagugaag uagguaguuu cguguuguug ggccuggcuu ucugaacaca acaacaccaa 60

accaccugau ucacugcagu 80

<210> 2289
<211> 94
<212> RNA
<213> Gallus gallus

<400> 2289
cuccacuccg ucugcccagu guucagacua ccuguucagg acuacgagau uguacaguag 60

ucugcacauu gguuaggcug ugcugggaua cacc                                    94

<210>  2290
<211>  108
<212>  RNA
<213>  Gallus gallus

<400>  2290
gaagcguccg gagauccugc uccgucgccc caguguucag acuaccuguu caggacaaug      60

cuguuguaca guagucugca cauugguuag acugggcaag ggaaagca                  108

<210>  2291
<211>  72
<212>  RNA
<213>  Mus musculus

<400>  2291
guuccuuuuu ccuaugcaua uacuucuuug uggaucuggu cuaaagaggu auagcgcaug      60

ggaagaugga gc                                                          72

<210>  2292
<211>  86
<212>  RNA
<213>  Tetraodon nigroviridis

<400>  2292
augugaccua uggacuuccc uuuguuaucc uaugccugga cucaaaaaag gggcugggag      60

ggcaaagggu ugcccaguug ucauac                                           86

<210>  2293
<211>  72
<212>  RNA
<213>  Xenopus tropicalis

<400>  2293
ugcugcuguc cuucauucca ccggauccug uguaaauaua auccagauuc ccaguggcau      60

gaagugcauu ag                                                          72

<210>  2294
<211>  77
<212>  RNA
<213>  Mus musculus

<400>  2294
ccucucaggg aagcuuuuug cucgcguuau guuucucauc cgaauauaag acgaacaaaa      60

gguuugucug agggcug                                                     77

<210>  2295
<211>  68
<212>  RNA
<213>  Monodelphis domestica

<400>  2295
uguagauacu gucucuuucc aucagcagcu acaucuggcu acugggucuc ugauggcauc      60

uuagagcu                                                               68

<210>  2296
<211>  63
<212>  RNA
<213>  Mus musculus

<400>  2296
aagaaauggu uuaccguccc acauacauuu ugaguaugua ugugggacgg uaaaccgcuu      60

cuu                                                                   63


<210>  2297
<211>  63
<212>  RNA
<213>  Rattus norvegicus

<400>  2297
aagaaauggu uuaccguccc acauacauuu ugaguaugua ugugggacgg uaaaccgcuu      60

cuu                                                                   63


<210>  2298
<211>  68
<212>  RNA
<213>  Mus musculus

<400>  2298
ccucugcuuu aacauggggu uaccugcugu guuaaacaaa agcaagugcu uccauguuuc      60

aguggggg                                                              68


<210>  2299
<211>  96
<212>  RNA
<213>  Mus musculus

<400>  2299
gagucugguu uuguuugggu uuguucuagg uaugguccca gggaucccag aucaaaccag      60

gccccugggc cuauccuaga accaaccuaa acccgu                               96


<210>  2300
<211>  98
<212>  RNA
<213>  Mus musculus

<400>  2300
caauuguacu uggugugauu auaaagcaau gagacugauu gucauauguc guuuguggga      60

uccgucucag uuacuuuaua gccauaccug guaucuua                             98


<210>  2301
<211>  98
<212>  RNA
<213>  Rattus norvegicus

<400>  2301
cacuuguacu cggugugauu auaaagcaau gagacugauu gucaugugucguuuguggga      60

uccgucucag uuacuuuaua gccauaccug guaucuua                             98


<210>  2302

<211>  99
<212>  RNA
<213>  Mus musculus

<400>  2302
gaaaaugggc ucaaggugag gggugcuauc ugugauugag ggacaugguc aauggaauug      60

ucucacacag aaaucgcacc cgucaccuug gccugcuga                            99


<210>  2303
<211>  99
<212>  RNA
<213>  Rattus norvegicus

<400>  2303
gaaaaugggc ucaaggugag gggugcuauc ugugauugag ggacaugguc aauggaauug      60

ucucacacag aaaucgcacc cgucaccuug gccugcuga                            99


<210>  2304
<211>  86
<212>  RNA
<213>  Rattus norvegicus

<400>  2304
uuugguauuu aaaaggugga uauuccuucu augguuacgu gcuuccugga uaaucauaga      60

ggaacaucca cuuuuucagu aucaaa                                          86


<210>  2305
<211>  66
<212>  RNA
<213>  Mus musculus

<400>  2305
agggcuccug acuccagguc cuguguguua ccucgaaaua gcacuggacu uggagucaga      60

aggccu                                                                66


<210>  2306
<211>  65
<212>  RNA
<213>  Rattus norvegicus

<400>  2306
gggcuccuga cuccaggucc ugugugguac cucgaaauag cacuggacuu ggagucagaa      60

ggccu                                                                 65


<210>  2307
<211>  109
<212>  RNA
<213>  Mus musculus

<400>  2307
acuugugagg aaauaaagga aguuaggcug aggggcagag agcgagacuu uucuauuuuc      60

caaaagcucg gucugaggcc ccucagucuu gcuuccuacc ccgcgcuug                109


<210>  2308
<211>  91
<212>  RNA

<213>  Mus musculus

<400>  2308
cuguguguga uggcuuggca guguauuguu agcugguuga guaugugagc ggcaccagcu      60

aacaugcgac ugcucuccua uugcacacac a                                     91


<210>  2309
<211>  82
<212>  RNA
<213>  Mus musculus

<400>  2309
cucccuggug ugagcguaug ugccuuugga cuacaucgug aacgcagcac caugcagucc      60

acgggcauau acacuugccu ca                                               82


<210>  2310
<211>  73
<212>  RNA
<213>  Mus musculus

<400>  2310
gagggggaag acgggagaag agaagggagu gguuuuuggg ugccucacuc cuccccuccc      60

gucuuguucu cuc                                                         73


<210>  2311
<211>  73
<212>  RNA
<213>  Mus musculus

<400>  2311
acuuggagag aggcuggccg ugaugaauuc gauucaucua aacgagucau acacggcucu      60

ccucucuucu agu                                                         73


<210>  2312
<211>  79
<212>  RNA
<213>  Rattus norvegicus

<400>  2312
   gauacuugga gagaggcugg ccgugaugaa uucgauucau cuaaacgagu cauacacggc      60
   ucuccucucu ucuaguguc                                                 79


<210>  2313
<211>  109
<212>  RNA
<213>  Mus musculus

<400>  2313
uucgggugag agugagaauc cucucuccca gauaauagca cucucaaaca aguuuccaug      60

uuguuugaaa ggcuguuucu uggucagaag acucucaauu ucuucugga                 109


<210>  2314
<211>  109
<212>  RNA
<213>  Mus musculus

<400>  2314
cuucugcucu gcucauccuc ucuaauccuu ugucccuggg ugaaaaugcu auuuguaugc      60

aaugcacccg ggcaaggauu uggggaaggu gagccugauc ugcauggag                 109


<210>  2315
<211>  23
<212>  RNA
<213>  Mus musculus

<400>  2315
aaugacacga ucacucccgu uga                                              23


<210>  2316
<211>  23
<212>  RNA
<213>  Rattus norvegicus

<400>  2316
aaugacacga ucacucccgu uga                                              23


<210>  2317
<211>  23
<212>  RNA
<213>  Mus musculus

<400>  2317
ugaagguccu acugugugcc agg                                              23


<210>  2318
<211>  21
<212>  RNA
<213>  Rattus norvegicus

<400>  2318
ugaaggucua cugugugcca g                                                21


<210>  2319
<211>  22
<212>  RNA
<213>  Mus musculus

<400>  2319
caugccuuga guguaggacc gu                                               22


<210>  2320
<211>  22
<212>  RNA
<213>  Rattus norvegicus

<400>  2320
caugccuuga guguaggacu gu                                               22


<210>  2321
<211>  22
<212>  RNA
<213>  Mus musculus

<400>  2321
uccgagccug ggucucccuc uu                                               22

<210>  2322
<211>  85
<212>  RNA
<213>  Mus musculus

<400>  2322
aaagugcuuu ggaaugacac gaucacuccc guugaguggg cacccaagaa gccaucggga      60

augucguguc cgcccagugc ucuuu                                            85


<210>  2323
<211>  83
<212>  RNA
<213>  Rattus norvegicus

<400>  2323
agugcuuugg aaugacacga ucacucccgu ugagugggca cccaagaagc caucgggaau      60

aucguguccg cccagugcuc uuu                                              83


<210>  2324
<211>  83
<212>  RNA
<213>  Mus musculus

<400>  2324
cgccagggcc uuguacaugg uaggcuuuca uucauuuuuu gcacauucgg ugaagguccu      60

acugugugcc aggcccugug cca                                              83


<210>  2325
<211>  84
<212>  RNA
<213>  Rattus norvegicus

<400>  2325
ggccuccagg gccuuguaca ugguaggcuu ucauucauug uuugcacauu cggugaaggu      60

cuacugugug ccaggcccug ugcc                                             84


<210>  2326
<211>  96
<212>  RNA
<213>  Mus musculus

<400>  2326
cagauuugcu uuuucucuuc caugccuuga guguaggacc guugacaucu uaauuacccu      60

cccacaccca aggcuugcag gagagcaagc cuucuc                                96


<210>  2327
<211>  79
<212>  RNA
<213>  Rattus norvegicus

<400>  2327
uuuucucuuc caugccuuga guguaggacu guugacaucu uaauuacccu cccacaccca      60

aggcuugcag gagagcaag                                                   79


<210>  2328

<211>  92
<212>  RNA
<213>  Mus musculus

<400>  2328
ucgggagggg cgggaggggg guccccggug cucggaucuc gagggugcuu auuguucggu      60

ccgagccugg gucucccucu uccccccauc cc                                    92